(19) Europäisches Patentamt
European Patent Office
Office européen des brevets

(11) EP 3 256 583 B1

(12) **FASCICULE DE BREVET EUROPEEN**

(45) Date de publication et mention de la délivrance du brevet:
**23.12.2020 Bulletin 2020/52**

(21) Numéro de dépôt: **16707896.3**

(22) Date de dépôt: **10.02.2016**

(51) Int Cl.:
***C12N 15/10*** *(2006.01)* ***C12N 15/64*** *(2006.01)*
***C12N 15/66*** *(2006.01)*

(86) Numéro de dépôt international:
**PCT/FR2016/050305**

(87) Numéro de publication internationale:
**WO 2016/128679 (18.08.2016 Gazette 2016/33)**

(54) **PROCÉDÉ DE FABRICATION DE VECTEURS D'ADN À PARTIR DE BRIQUES MOLÉCULAIRES CONTENANT DES SÉQUENCES D'INTÉRÊT**

VERFAHREN ZUR HERSTELLUNG VON DNA-VEKTOREN AUS MOLEKULAREN BAUSTEINEN MIT INTERESSIERENDEN SEQUENZEN

METHOD FOR PRODUCING DNA VECTORS FROM MOLECULAR BRICKS CONTAINING SEQUENCES OF INTEREST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.02.2015 FR 1551075**

(43) Date de publication de la demande:
**20.12.2017 Bulletin 2017/51**

(73) Titulaires:
- **Université de Lille**
  **59800 Lille (FR)**
- **Centre National de la Recherche Scientifique**
  **75794 Paris Cedex 16 (FR)**

(72) Inventeurs:
- **DE BETTIGNIES, Geoffroy**
  **59263 Houplin-Ancoisne (FR)**
- **DE BETTIGNIES, Carine**
  **59263 Houplin-Ancoisne (FR)**
- **JULIEN, Sylvain**
  **59170 Croix (FR)**

(74) Mandataire: **Plasseraud IP**
**66, rue de la Chaussée d'Antin**
**75440 Paris Cedex 09 (FR)**

(56) Documents cités:
**WO-A1-98/38326 WO-A1-2008/095927**

- **ERNST WEBER ET AL: "A Modular Cloning System for Standardized Assembly of Multigene Constructs", PLOS ONE, vol. 6, no. 2, 18 février 2011 (2011-02-18), page e16765, XP055110994, ISSN: 1932-6203, DOI: 10.1371/journal.pone.0016765**
- **WERNER S ET AL: "Fast track assembly of multigene constructs using Golden Gate cloning and the MoClo system", BIOENGINEERED BUGS, LANDES BIOSCIENCE, US, vol. 3, no. 1, 1 janvier 2012 (2012-01-01) , pages 38-43, XP002722591, ISSN: 1949-1018, DOI: 10.1371/JOURNAL.PONE.0016765 [extrait le 2012-01-01]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 256 583 B1

## Description

### DOMAINE TECHNIQUE

La présente divulgation porte sur un procédé de fabrication de vecteurs « sur mesure » d'ADN double brin, à partir de briques moléculaires comportant des séquences d'intérêt.

### ETAT DE LA TECHNIQUE

A l'heure actuelle, les manipulations d'ADN sont largement toujours basées sur l'utilisation de vecteurs de clonage ou d'expression. L'insertion ou l'extraction de fragments d'ADN correspondants à des éléments fonctionnels (gènes de résistance aux antibiotiques, sites de clonage, étiquettes moléculaires, promoteurs, origines de réplication dans d'autres organismes, cassettes de sélection, etc...) dans des plasmides, molécules d'ADN circulaire naturellement présentes chez certaines bactéries, a permis le développement d'une multitude de vecteurs différents, adaptés à diverses utilisations, chaque vecteur étant développé pour une application particulière et spécifique. L'utilisateur final choisit donc un vecteur adapté à ses besoins et introduit dans celui-ci son propre fragment d'ADN d'intérêt, par des méthodes de clonage de l'ADN. Ces méthodes sont traditionnellement basées sur l'utilisation d'une part d'enzymes de restriction, le plus souvent de type IIp (c'est-à-dire des enzymes qui reconnaissent et coupent l'ADN au niveau de courtes séquences palindromiques) et d'autre part d'ADN ligases, qui sont capables de recoller des fragments d'ADN générés par les enzymes de restriction. Ces méthodes nécessitent des étapes multiples, dont le nombre augmente le risque d'exposition à des contaminants exogènes susceptibles de dégrader l'ADN, le risque d'auto appariement ou d'appariements erronés. De même, ces méthodes nécessitent l'utilisation d'enzymes multiples et plus ou moins performantes, ce qui constitue un inconvénient difficile à contourner. Des difficultés peuvent aussi être rencontrées selon la compatibilité des plasmides donneurs et accepteurs. En effet, de très nombreux vecteurs différant par leurs composants fonctionnels sont commercialement disponibles pour répondre à la grande variété d'utilisations possibles. Cependant chaque vecteur n'est pas nécessairement compatible avec les autres et en particulier l'existence ou l'absence de sites de restriction dans la séquence plasmidique peut rendre les transferts de séquence d'un vecteur à un autre relativement complexe. Le transfert de fragments d'ADN d'un plasmide à un autre plasmide présente un certain nombre d'inconvénients et engendre une forte réduction des rendements de réaction et un coût important en temps et en réactifs.

Les activités de clonages basées sur les méthodes de restriction ligation et les méthodes en dérivant sont basées sur l'utilisation d'un vecteur d'arrivée, le plus souvent d'origine commerciale, ce qui entraine un manque de contrôle sur la nature et le nombre de composants fonctionnels des vecteurs d'arrivée. De plus, l'utilisation de la méthode de restriction ligation nécessite de disposer de sites de restriction utilisables de part et d'autre du fragment à insérer dans le vecteur ainsi que de ces mêmes sites dans le vecteur lui-même. Il faut donc s'assurer pour pouvoir introduire un fragment d'ADN par restriction-ligation qu'aucune des enzymes utilisées ne coupe à l'intérieur du fragment d'ADN d'intérêt et que chacune de ces enzymes ne coupe qu'a un seul endroit dans le vecteur qui est l'endroit auquel le fragment doit être inséré. C'est pour cette raison que les vecteurs développés contiennent des sites de clonage multiples (MCS). La présence et l'utilisation de ces MCS pour insérer des fragments d'ADN dans des vecteurs laissent des traces dans la séquence d'ADN hybride obtenue, il s'agit de séquences pouvant aller de quelques nucléotides à plusieurs dizaines de nucléotides avant et après l'insert. Ces 'cicatrices' nucléotidiques ne sont pas nécessaires à la fonction du plasmide et sont parfois même délétères (par exemple dans le cas de deux séquences contenant des protéines que l'on souhaite fusionner).

Le système Gateway est présenté comme une solution aux problèmes de transfert d'insert d'un plasmide à l'autre. Cependant, le système Gateway peut également être perçu comme un système clos, peu compatible avec les autres outils moléculaires disponibles. De plus, dans le système Gateway, la séquence de recombinaison est fixe et laissera toujours des traces dans le vecteur final. L'assemblage Golden Gate est également basé sur un plasmide donneur et un plasmide receveur, très comparable au système Gateway.

Certaines des méthodes décrites dans l'art antérieur sont proposées comme des solutions pour pallier ces limitations. Par exemple, les méthodes de clonage indépendante de la ligation ou indépendante de la séquence et de la ligation (LIC/SLIC) ou de Gibson Assembly permettent aux utilisateurs de ne pas avoir à utiliser de multiples enzymes de restriction et de lever ainsi un certain nombre de limitations techniques liées à ces dernières (compatibilité, présence de sites dans les séquences ou plasmides d'intérêt). Elles ne permettent pas cependant un plus grand contrôle des fonctionnalités du vecteur final qui dépend toujours des outils moléculaires commercialement disponibles.

Pour s'affranchir d'un vecteur de départ et d'un vecteur final, Wang T. et al. (2012), Appl Microbiol Biotechnol 93: 1853-1863, ou encore Weber E. (2011), PLoS ONE 6 (2): e16765 et EP2395087, et Sarrion-Perdigones A. (2012), PLoS ONE 6 (7): e21622) ont proposé de nouvelles méthodes de clonage modulaire comme le GoldenBraid (ou le Golden Gate). a méthode Golden Gate est décrite dans le document WO 2008/095927 ainsi que dans l'article d'Engler et al. PLoS ONE 4 (2009) e5553. Cette méthode est compatible avec de nombreux outils moléculaires (plasmides) commercialement disponibles et repose sur l'utilisation d'un type d'enzyme de restriction disponible chez de nombreux fournisseurs. Les utilisateurs de cette méthode ne sont donc pas captifs d'une gamme de produit dédiés. Le revers de cette versatilité est le travail à réaliser pour vérifier que tous les éléments des constructions souhaitées soient compatibles avec la méthode. Autrement dit, ils doivent être naturellement dépourvus de site de restriction d'enzymes de type IIs, ou être modifiés pour éliminer les sites potentiellement présents.

Pour fabriquer un vecteur selon les besoins, un squelette plasmidique 'modulaire' a été développé par la société Oxford Genetics (http://oxfordgenetics.com/). Ce squelette, baptisé SnapFast®, contient des sites de restriction introduits dans la séquence de sorte à flanquer les composants fonctionnels du plasmide. Ainsi, chaque composant peut être remplacé par un autre de la même catégorie (ex : promoteur, étiquette) en appliquant la méthode de restriction-ligation pour chaque modification souhaitée par l'expérimentateur. Cependant cette technique nécessite une relativement grande quantité d'ADN. Même si le système SnapFast® est probablement l'outil de biologie moléculaire qui propose aux utilisateurs la plus grande marge de manœuvre concernant le contrôle des fonctionnalités du vecteur d'arrivée, des défauts subsistent. Cette alternative présente les inconvénients inhérents à l'usage des enzymes de restriction multiples (ex : présence éventuelle des sites dans les séquences d'intérêt, cicatrices nucléotidiques) et un coût de prestation élevé. Autre limitation, la modularité demeure restreinte aux substitutions rendues possibles par le squelette défini de ce vecteur.

D'autres approches ont été développées pour surmonter cette limitation. Il s'agit notamment de clonage basé sur l'activité recombinase. Cette technique réduit les problèmes provenant de la présence de plusieurs sites de restriction dans les grandes constructions, mais est limitée par le fait que les sites de recombinaison sont laissés dans le produit d'assemblage final, ce qui empêche l'assemblage sans soudure de séquences codant pour des protéines (Weber E. (2011), PLoS ONE 6 (2): e16765 et EP2395087). De plus, seul un petit nombre de fragments peuvent être assemblés dans une construction selon cette méthode. En outre, le contrôle total de la composition du vecteur final (séquence d'intérêt et modules fonctionnels) n'est pas atteint.

La technologie de synthèse de gène le permettrait. Cependant cette technologie nécessite de son utilisateur qu'il effectue le design complet de son vecteur avant sa préparation, la moindre erreur étant synonyme de perte totale et irréversible de l'investissement. De plus, cette fabrication à façon est très coûteuse et un processus long.

Le document US 2014 0038240 décrit un procédé d'assemblage réalisé en plusieurs étapes successives. Cette méthode nécessite l'utilisation soit d'une agrafe d'ADN simple brin soit d'un adaptateur. Les inconvénients liés aux agrafes sont qu'elles peuvent adopter une structure secondaire, s'auto apparier ou encore être masquées par des protéines. Cela peut entrainer des difficultés d'assemblage et des rendements faibles. Selon un autre mode de réalisation, le document US2014 0038240 décrit un procédé d'assemblage réalisé à l'aide d'un adaptateur. Ce mode de réalisation requiert l'utilisation de plusieurs enzymes de restriction pour générer des extrémités simple brin.

Le document WO9838326 décrit un procédé d'auto-assemblage d'un vecteur de gènes dans lequel les fragments amplifiés sont digérés par une enzyme de restriction de type IIs, puis purifiés, de préférence par électrophorèse en gel d'agarose, pour éliminer les extrémités des amorces libérées par l'enzyme de type IIs; les fragments purifiés sont ensuite combinés en présence de ligase pour produire le vecteur circulaire.

De manière générale, toutes les techniques de clonage par recombinaison laissent des séquences qui ne sont pas voulues dans le vecteur final, qui sont les séquences utilisées pour la recombinaison.

Aucune des méthodes ou technologie existantes ne permet aux utilisateurs de générer facilement, (en une seule étape) à faible coût (en présence d'une seule enzyme) et en peu de temps, des vecteurs d'expression entièrement modulaires à façon.

Il existe donc un réel besoin de proposer une méthode permettant d'assembler des molécules d'ADN complexes et d'obtenir facilement, avec un bon rendement, et sans erreur, une construction que l'on aura choisie entièrement, c'est-à-dire dont on pourra contrôler la nature et le nombre des composants, en particulier de composants fonctionnels.

Il est donc nécessaire de mettre au point un nouveau procédé permettant de résoudre tous ces problèmes qui soit plus efficace, moins coûteux, et plus rapide.

La présente invention propose de résoudre ces problèmes.

## EXPOSE DE L'INVENTION

La présente divulgation porte sur un procédé de fabrication d'un vecteur circulaire d'ADN double brin comportant au moins deux séquences d'intérêt, comprenant :

a) une étape de mise en contact simultanée d'au moins deux briques moléculaires, différentes l'une de l'autre, en présence d'une seule enzyme de restriction, ladite seule enzyme de restriction étant une enzyme de restriction de type IIs,
chaque brique moléculaire étant une molécule d'ADN linéaire double brin, et contenant :

(i) une séquence d'intérêt dépourvue de site de reconnaissance spécifique de la sus-dite enzyme de restriction

de type IIs et comprenant au moins une unité, ladite unité étant une unité fonctionnelle ou une unité non fonctionnelle, ladite unité comprenant au moins un module, ledit module étant un module fonctionnel ou un module non fonctionnel,
(ii) deux adaptateurs d'ADN double brin, flanqués en amont et en aval de ladite séquence d'intérêt, chaque adaptateur d'ADN double brin consistant en une séquence d'au moins 12 nucléotides, laquelle contient un seul et unique site de reconnaissance de la susdite enzyme de restriction de type IIs,

le site de reconnaissance de la susdite enzyme de restriction de type IIs de l'adaptateur en amont de ladite séquence d'intérêt et le site de reconnaissance de la susdite enzyme de restriction de type IIs de l'adaptateur en aval de ladite séquence spécifique étant convergents,
laquelle étape conduisant :

- à l'élimination *par coupure* des sites de reconnaissance de l'enzyme de restriction de type IIs utilisée,
- la formation d'une suture monobrin cohésive d'au moins 2 nucléotides à chacune des extrémités de ladite séquence d'intérêt,

ladite suture monobrin cohésive d'au moins 2 nucléotides en amont d'une des au moins deux séquences d'intérêt étant complémentaire à ladite suture monobrin cohésive d'au moins 2 nucléotides en aval d'une autre séquence d'intérêt,
ladite étape du procédé conduisant :

- à l'appariement par complémentarité nucléotidique des susdites sutures monobrins cohésives d'au moins 2 nucléotides et
- au positionnement des séquences d'intérêt, de façon contigüe les unes aux autres, selon un ordre et un sens unique et déterminé,

ledit procédé comprenant en outre,
b) une étape de ligation des susdites sutures monobrins cohésives d'au moins 2 nucléotides,

pour obtenir un vecteur circulaire d'ADN double brin.
Selon un mode de réalisation, le procédé porte sur un procédé de fabrication d'un vecteur circulaire d'ADN double brin comportant au moins deux séquences d'intérêt, comprenant :

a) une étape de mise en contact simultanée d'au moins deux briques moléculaires, différentes l'une de l'autre, en présence d'une seule enzyme de restriction, ladite seule enzyme de restriction étant une enzyme de restriction de type IIs,
chaque brique moléculaire étant une molécule d'ADN linéaire double brin à extrémités non cohésives, et contenant :

(i) une séquence d'intérêt dépourvue de site de reconnaissance spécifique de la sus-dite enzyme de restriction de type IIs et comprenant au moins une unité, ladite unité étant une unité fonctionnelle ou une unité non fonctionnelle, ladite unité comprenant au moins un module, ledit module étant un module fonctionnel ou un module non fonctionnel,
(ii) deux adaptateurs d'ADN double brin, flanqués en amont et en aval de ladite séquence d'intérêt, chaque adaptateur d'ADN double brin consistant en une séquence d'au moins 12 nucléotides, laquelle contient un seul et unique site de reconnaissance de la sus dite enzyme de restriction de type IIs,

le site de reconnaissance de la sus dite enzyme de restriction de type IIs de l'adaptateur en amont de ladite séquence d'intérêt et le site de reconnaissance de la sus dite enzyme de restriction de type IIs de l'adaptateur en aval de ladite séquence spécifique étant convergents,
laquelle étape conduisant :

- à l'élimination *par coupure* des sites de reconnaissance de l'enzyme de restriction de type IIs utilisée,
- la formation d'une suture monobrin cohésive d'au moins 2 nucléotides à chacune des extrémités de ladite séquence d'intérêt,

ladite suture monobrin cohésive d'au moins 2 nucléotides en amont d'une des au moins deux séquences d'intérêt étant complémentaire à ladite suture monobrin cohésive d'au moins 2 nucléotides en aval d'une autre séquence d'intérêt,

- à l'appariement par complémentarité nucléotidique des susdites sutures monobrins cohésives d'au moins 2 nucléotides et
- au positionnement des séquences d'intérêt, de façon contigüe les unes aux autres, selon un ordre et un sens unique et déterminé,

b) une étape de ligation des susdites sutures monobrins cohésives d'au moins 2 nucléotides,

pour obtenir un vecteur circulaire d'ADN double brin.

Le procédé selon l'invention ne requiert l'utilisation que d'une seule enzyme de restriction, qui est une enzyme de restriction de type IIs.

Selon un aspect particulier de la divulgation, les vecteurs selon l'invention, obtenus en particulier selon le procédé de l'invention, sont dépourvus de site de clonage multiple.

Les conventions selon lesquelles un ADN double brin est lu de 5' en 3' sont respectées dans la présente invention.

L'invention décrite permet d'abolir totalement l'usage des enzymes de restriction classiques dans la fabrication de vecteurs et donc de l'ensemble de leurs inconvénients. Il en résulte un temps de réalisation des vecteurs beaucoup plus court et une diminution dramatique des risques d'erreur. Le coût de fabrication donc devrait être fortement réduit ; la constitution et le maintien d'un stock d'enzymes de restriction variées dans chaque laboratoire devenant aussi moins nécessaire.

On entend par enzyme de restriction classique une enzyme de restriction de type IIp.

L'invention permet de s'affranchir d'utiliser des vecteurs d'arrivée pour les techniques de sous-clonage. Cela veut dire qu'il n'est plus besoin d'acquérir ou d'entretenir une collection de multiples vecteurs pour procéder à des activités de sous-clonage. Cela représente une économie en temps (culture bactérienne et purification de plasmides) conséquente.

Par ailleurs, la méthode selon l'invention permet de concevoir des vecteurs comprenant des cassettes d'expression multiples. Dans un contexte de modification génétique de cellules ou d'organisme cela permet aux utilisateurs de n'utiliser qu'un seul vecteur, et donc une seule étape de sélection pour transférer plusieurs gènes d'intérêt simultanément dans leur système d'intérêt.

L'invention permet aussi de restreindre le contenu des vecteurs aux seules séquences d'intérêt choisies par le demandeur. Il n'y a aucune séquence plasmidique résiduelle, ou cicatrice nucléotidique, due à l'utilisation des enzymes de restriction classiques et à la nécessité d'utiliser des sites de clonage multiples. L'invention permet donc aux utilisateurs d'exercer un contrôle total sur les composants du vecteur produit.

L'invention est appropriée pour générer des gènes chimères par appariements combinatoires, et est tout à fait compatible avec des applications basées sur ces approches (ex : étiquetage intramoléculaire ou analyse de promoteur). Dans ces contextes, l'invention permet la création simultanée de nombreux vecteurs d'expression différents en une seule étape. Elle permet donc de produire rapidement (en parallèle) une gamme de vecteurs se différenciant par l'un ou l'autre des composants choisis (ex : gène de résistance, marqueur moléculaire), sans modifier l'ensemble de l'architecture du vecteur.

Pour l'ensemble de ces raisons, l'invention décrite constitue une méthode techniquement et économiquement attractive comparée aux méthodes les plus performantes connues.

On entend par vecteur, une molécule d'ADN comportant des informations génétiques, susceptible de transmettre lesdites informations génétiques. Un vecteur peut également être une molécule d'origine plasmidique, ou un plasmide, modifié par génie génétique et destiné à transférer des séquences d'ADN dans une cellule ou un organisme de choix.

Selon la divulgation, un plasmide est une molécule d'ADN, distincte de l'ADN chromosomique, capable de réplication autonome. Les plasmides sont généralement circulaires et bicaténaires (d'ADN double brin).

Une molécule d'origine plasmidique est une molécule constituée au moins en partie d'acide nucléique provenant d'un plasmide.

On entend par vecteur d'expression tout vecteur utilisé pour comprendre, et/ou permettre l'expression de l'information génétique d'un gène dans une cellule ou un organisme de choix.

Selon la divulgation, la fonctionnalité de chaque vecteur est définie selon la combinaison des fonctions basiques portées par le ou les module(s) constituant le vecteur et/ou l'utilisation qui en est faite.

En général on entend par séquence d'intérêt une séquence d'acides nucléiques que l'expérimentateur souhaite utiliser et/ou assembler à une autre séquence d'intérêt.

On entend aussi par séquence d'intérêt, une séquence d'acides nucléiques qui contient l'information génétique correspondant à un ou plusieurs modules fonctionels.

Le terme **unité** définit l'ensemble des informations contenues dans une séquence d'ADN qui confère à cette séquence une fonctionnalité génétique **intégrée** correspondant à l'une des fonctions primordiales d'un vecteur. Par définition, une unité est composée d'un ensemble de modules dont la combinaison produit la fonction de cette unité.

Une unité **bactérienne ou unité de maintien en bactérie** a pour fonction d'assurer la réplication et la sélection du vecteur dans un système procaryote.

L' unité **d'expression** a pour fonction de permettre l'expression d'un produit génétique dans un système d'intérêt (eucaryote ou procaryote).

L'unité d'**intégration** en cellule eucaryote ou procaryote a pour fonction de permettre le maintien du ou des transgènes dans le génome d'un système d'intérêt.

Un vecteur doit contenir à minima une unité fonctionnelle bactérienne et peut en outre contenir une ou plusieurs unité d'expression et/ ou une ou plusieurs unités d'intégration.

Un vecteur d'expression doit donc contenir une unité bactérienne et une unité d'expression. Un vecteur d'expression peut contenir en outre une ou plusieurs autres unités d'expression et une ou plusieurs unités d'intégration.

Du point de vue physique, une unité peut être apportée au vecteur par une ou plusieurs séquences d'intérêt. Si l'unité est contenue dans une seule séquence, l'unité est, de facto, fonctionnelle.

Selon la divulgation, une unité fonctionnelle (bactérienne, d'expression, d'intégration) en général, comprend un ensemble de modules (au moins un module) nécessaires à une fonction décrite.

Une unité fonctionnelle se suffit à elle-même pour conférer une fonction biologique au vecteur.

Selon la divulgation, une unité fonctionnelle comprend au moins un module, ledit module étant un module fonctionnel ou un module non fonctionnel.

En général, on entend par module fonctionnel une séquence d'acides nucléiques qui confère une fonction au vecteur en participant à la fonction d'une unité fonctionnelle. Un module fonctionnel est une entité physique, représentée par une séquence qui participe, en combinaison avec d'autres modules, à la fonction de l'unité.

Le terme **"module"** définit ainsi une information contenue dans une séquence d'ADN qui confère à cette séquence une fonctionnalité génétique **minimale** utile à la fonction d'un vecteur. Les informations répondant à ce critère minimal sont par exemple :

- les promoteurs,
- les séquences codantes,
- les terminateurs,
- les séquences non codantes correspondant à des ARN fonctionnels (introns, ARN en épingle à cheveux, longs ARN non codant),
- les enhancers,
- les insulateurs,
- les séquences IRES,
- les séquences cibles de recombinases,
- les origines de réplication,
- les séquences homologues à des loci génétiques.

Selon la divulgation, un module fonctionnel est une séquence qui contient une information génétique nécessaire et suffisante à la réalisation d'une fonction basique impliquée dans la fonctionnalité du vecteur, en particulier une origine de réplication, un promoteur, un terminateur, une séquence de recombinaison, une séquence codante ou non codante, une séquence régulatrice de traduction, etc...

Un module non fonctionnel n'a pas de fonction dans un contexte (dans une cellule hôte particulière par exemple) déterminé, mais peut en avoir s'il est combiné à d'autres modules fonctionnels ou non fonctionnels ou s'il est utilisé dans un contexte approprié.

Par exemple, dans une unité d'expression (ou cassette d'expression) fonctionnelle, les modules minimum nécessaires sont un promoteur, une séquence codant un produit d'expression et un terminateur. D'autres modules peuvent intervenir dans cette unité afin d'en modifier la fonction.

En d'autres termes, lorsque l'information définie par le module se suffit à elle-même pour supporter la fonctionnalité, le module est dit fonctionnel : Par exemple une séquence codante entière (de l'ATG au codon stop) ou un promoteur défini (site de reconnaissance de facteur de transcription et présence d'une initiation de transcription) constituent des modules fonctionnels. Si l'information présente ne permet pas cette fonctionnalité minimale le module sera dit non fonctionnel. Par exemple une séquence codante incomplète (absence d'un ATG ou d'un codon stop), ou un promoteur tronqué (absence ou mutation de site de reconnaissance de facteur de transcription) sont par définitions des modules "non fonctionnels".

Les modules ont donc des fonctions singulières et définies qui, par combinaison produisent les fonctions biologiques des unités.

Un vecteur d'expression peut contenir plusieurs unités, dont au moins une unité fonctionnelle bactérienne et une unité fonctionnelle d'expression.

On entend par brique moléculaire une séquence d'ADN double brin linéaire. Selon un mode de réalisation avantageux, une brique moléculaire est une séquence d'ADN double brin linéaire ayant un nucléotide (Adénine) débordant en 3' sur chacun des deux brins d'ADN. Selon un mode de réalisation plus avantageux, une brique moléculaire est une séquence d'ADN double brin linéaire à bout franc.

Une brique moléculaire selon l'invention est composée d'une séquence d'intérêt flanquée de part et d'autre d'un adaptateur.

Selon la divulgation, une brique est incapable de former un vecteur *per se*. En d'autres termes, une brique seule ne peut constituer un vecteur.

De manière non limitative, la figure 30 présente un exemple de construction selon l'invention. Dans cet exemple, l'assemblage ordonné des séquences d'intérêt (SI) produit un vecteur contenant 4 unités fonctionnelles distinctes :

- L'unité bactérienne est composée de deux SI : A et G. La SI A contient un module fonctionnel (1) et la SI G contient deux modules fonctionnels (9 et 10). Par définition, les SI A et G contiennent chacune une unité (bactérienne) non fonctionnelle.

- L'unité d'intégration est composée de trois SI : B, E et F. Les SI B et F contiennent chacune un module non fonctionnel (respectivement 2a et 2b). La présence simultanée de ces deux modules non fonctionnels dans le même vecteur constitue un module fonctionnel. Dans le cas présent ce module fonctionnel reconstitué permet l'intégration de la séquence d'ADN C+D+E à un locus ciblé par la séquence B+F. La SI E contient trois modules fonctionnels (6, 7 et 8) qui codent un gène de sélection positive. Dans le cas présent ce gène permet de sélectionner les cellules cibles qui auront intégré la séquence C+D+E de façon stable dans leur génome par traitement avec l'antiobiotique correspondant au gène de sélection choisi. Par définition, et dans le contexte décrit dans l'exemple, les SI B, E et F contiennent chacune une unité d'intégration non fonctionnelle.

- L'unité d'expression 1 est composée de deux SI : C et D. Les SI C et D contiennent chacune un module fonctionnel et un module non fonctionnel : 3 et 4a pour la SI C, 5 et 4b pour la SI D. Dans cet exemple, l'assemblage produit un module fonctionnel à partir des modules 4a et 4b qui, une fois reliés constituent une séquence codante complète. Par définition, les SI C et D contiennent chacune une unité d'expression non fonctionnelle.

- L'unité d'expression 2 est contenue par la SI H. la SI H contient trois modules fonctionnels : 11, 12 et 13. Par définition la SI H contient une unité (d'expression) fonctionnelle.

De manière non limitative, le tableau 1 présente une liste d'unités et de modules.

**Tableau** 1. Exemples d'unités et de modules

| UNITES | TYPES DE MODULES | | EXEMPLES |
|---|---|---|---|
| Unité fonctionnelle bactérienne : | Origine de réplication bactérienne | | Origine de réplication des plasmides pMB1, pUC, ColE1, p15A, pSC101, R1, RK2, R6K, F1, M13, Lambda , pA81, pRAS3.1, pTi, pBPS1, pUO1, pKH9, pWKS1, pCD1, pMAK3, pBL63.1, pTA1060, p4M, pHT926, pCD6, pJB01, pIME300, pMD5057, pTE44, pDP1, pT38 |
| | Marqueur de sélection bactérien | gène de résistance à un antibiotique | gène de résitance à l'ampicilline, à la néomycine, à la kanamycine, au chloramphénicol, à la streptomycine, à la gentamicine, à la tétracycline, à l'érythromycine, à la vancomycine |
| | | gène de criblage | lacZ$\alpha$ |

(suite)

| UNITES | TYPES DE MODULES | | EXEMPLES |
|---|---|---|---|
| Unité fonctionnelle d'expression: | Promoteur | Promoteur de l'ARN-polymerase II: | pCMV, pEF1α, pβ-actin, Ubiquitine |
| | | Promoteur ARN-Polymerase II inductible: | promoteurs inductibles par la tétracycline (ex : pTRE3G), pGAL1, pGAL10 |
| | | promoteur de l'ARN-polymérase III: | U6, H1 |
| | Séquence codant un produit d'expression | séquence codante ou non codante décrite dans un génome | |
| | | gène rapporteur | Luciférase, β-galactosidase |
| | Amplificateur | | HACNS1/CENTG2, GADD45g |
| | Terminateur | Séquence de polyadénylation | BGHpolyA, HSV TKterm, SV40polyA |
| | Tag | tag | HA, Myc, Flag |
| | | protéine d'affinité | GST, MBP, TAPTag |
| | | protéine fluorescentes | GFP, Mcherry et variantes |
| | Séquence IRES | | (PPT19)4, KMI1, KMI1, KMI2, KMI2, KMIX, X1, X2. |
| Unité fonctionnelle d'intégration en cellule eucaryote | Gène de sélection | gène de sélection positive | Gène de résistance à l'Hygromycine B , au G418, à la tétracycline, à la puromycine, ou à la zeocine... |
| | | gène de sélection négative | Gène codant la thymidine kinase |
| | Séquence de maintien en cellule eucaryote | | Origine de réplication levure (ARS), séquence de centromère |
| | Séquence d'intégration homologue | | locus Rosa26 ou HRPT (cellules de souris) séquences d'intégration au génome de levure |
| | Séquences impliquées dans l'édition d'ADN (recombinaison homologue ciblée) | séquence reconnue par une recombinsase | séquence LoxP ou FRT. |

Selon un mode de réalisation particulier, la présente divulgation porte sur une brique moléculaire comprenant une séquence d'intérêt flanquée de part et d'autre d'une suture monobrin.

Selon l'information contenue dans la séquence d'intérêt, une brique moléculaire utilisée dans l'assemblage selon l'invention apporte une ou plusieurs fonctions au vecteur final, par exemple ;

- Plusieurs unités fonctionnelles
- Une unité fonctionnelle
- Une unité non fonctionnelle
- Un module fonctionnel

- Un module non fonctionnel
- Plusieurs modules fonctionnels

On entend par enzyme de restriction, une protéine pouvant se lier à, et couper un acide nucléique.
En général, les enzymes de restriction de type IIs sont des enzymes qui se lient de façon spécifique à l'ADN double brin, au niveau d'un site de reconnaissance non palindromique, donc orienté, et clivent les deux brins de l'ADN double brin à une distance fixe du site de reconnaissance. Les séquences nucléotidiques du site de reconnaissance et du site de clivage sont donc différentes.
Par convention, le site d'une enzyme de type IIs est orienté de sorte que son site de coupure soit situé après son site de reconnaissance.
Selon l'invention, une enzyme de type IIs se lie à l'ADN et coupe en aval du site de liaison.
La longueur de l'extrémité cohésive générée -ou la distance entre le site de reconnaissance de l'enzyme de type IIs et le site de clivage- dépend de l'enzyme de type IIs utilisée.
Les nucléotides du site de clivage ne faisant pas partie du site de reconnaissance, ils peuvent être choisis parmi les 4 bases nucléotidiques qui composent l'ADN.
L'enzyme de type IIs utilisée dans le procédé selon l'invention est choisie parmi l'une quelconque des enzymes de type IIs référencées dans http://rebase.neb.com/rebase/rebase.html.
Avantageusement, des enzymes de type IIs ayant un site de reconnaissance à une distance de 2, 3, 4, 5, 6, 7, 8, 9, 10, 11, 12, 13, 14, 15, 16, 17, ou 18 nucléotides du site de clivage de l'un des brins d'ADN telles que décrites dans Lippow et al, 2009, Nucleic Acides Res, 37:. 3061-3073 sont utilisées. Le site de clivage est défini comme la séquence comprise entre les coupures réalisées sur chacun des deux brins d'ADN.
Selon l'invention, les séquences d'intérêt sont entièrement définies et ne contiennent aucun site de restriction (site de reconnaissance) reconnu par l'enzyme de type IIs utilisée dans l'étape (a) du procédé selon l'invention.
Selon l'invention, on entend par sites convergents, des sites permettant à l'enzyme de type IIs de générer une suture monobrin en amont et en aval de la séquence d'intérêt.
Selon un mode de réalisation avantageux, on entend par sites de reconnaissance de l'enzyme de type IIs convergents, deux sites de reconnaissance de l'enzyme de type IIs convergents et localisés sur l'un et l'autre des deux brins complémentaires d'ADN de telle façon que l'enzyme de type IIs se lie de part et d'autre de l'ADN et coupe l'ADN en amont et en aval de la séquence d'intérêt (voir figure 1) pour générer une extrémité simple brin en amont et en aval.
On entend par adaptateur une séquence d'ADN d'au moins 8 nucléotides ou plus, en particulier de 8 à 100 nucléotides, de préférence 12 nucléotides, flanquant de part et d'autre une séquence d'intérêt dans une brique moléculaire.
En particulier, un adaptateur selon l'invention est une séquence d'ADN d'au moins 12 nucléotides ou plus, en particulier de 12 à 100 nucléotides, de préférence 12 nucléotides, flanquant de part et d'autre une séquence d'intérêt dans une brique moléculaire.
Un adaptateur contient au moins :

- une séquence de 5,6, 7 ou plus nucléotides correspondant au site de fixation de l'enzyme à activité de type IIs utilisée pour l'assemblage selon l'invention,
- une séquence de 1 à 8 et plus nucléotides correspondant à l'espacement entre le site de fixation et le site de coupure de l'enzyme utilisée. La longueur de cette séquence dépend des caractéristiques intrinsèques de l'enzyme de type IIs utilisée,
- une séquence de 2 à 5 ou plus nucléotides correspondant à l'extrémité simple brin générée par l'action de l'enzyme de type IIs utilisée. Cette séquence permettant l'appariement des fragments à assembler, elle correspond *de facto* à la définition de suture.

Selon l'invention, les adaptateurs contiennent un seul site de restriction (site de reconnaissance) reconnu par l'enzyme de type IIs utilisée dans l'étape (a) du procédé selon l'invention.
On entend par suture une séquence simple brin (ou monobrin) de 2 à 5, ou plus, nucléotides.
Selon l'invention, une suture est une séquence de 2 à 5, ou plus, nucléotides correspondant à l'extrémité simple brin générée par l'action de l'enzyme de type IIs utilisée pour l'assemblage, ladite séquence simple brin étant générée en amont et aval de la séquence d'intérêt.
Cette séquence de 2 à 5, ou plus, nucléotides, appariée à sa séquence complémentaire, est présente dans les adaptateurs, et donc dans les briques moléculaires, ainsi que dans le produit de réaction final (le vecteur).
Complémentaire ou complémentarité signifie que 100%, des bases nucélotidiques de deux séquences s'apparient. Selon l'invention, 100% de la séquence de la suture monobrin en aval de la brique n-1 s'apparie avec 100% de la séquence de la suture monobrin en amont de la brique n et les sutures sont donc complémentaires.
L'appariement des deux séquences de sutures monobrins va constituer une suture double brin.

On entend aussi par suture une séquence monobrin en aval de la brique n-1 appariée à la séquence monobrin en amont de la brique n.

Selon la divulgation une suture est une séquence d'ADN double brin dans le vecteur obtenu par le procédé.

La séquence des sutures générées au cours de l'invention est entièrement définie pour permettre d'une part le bon ordonnancement des séquences d'intérêt lors de l'assemblage et leur fonctionnement, et d'autre part un rendement optimum. Par conséquent, la séquence des sutures générées est une caractéristique de chaque brique moléculaire (choix intra-brique de la séquence des sutures) et dépend également de l'ordre dans lequel chacune des briques moléculaires sont agencées les unes par rapport aux autres (choix inter-brique de la séquence des sutures).

Les sutures ne sont pas des cicatrices, en particulier des cicatrices introduisant des dysfonctionnements dans le vecteur.

Selon la divulgation, les sutures font donc partie intégrante de chacune des briques moléculaires et peuvent, en outre entièrement ou partiellement faire partie de l'information génétique constitutive d'un module.

Les sutures sont choisies de façon raisonnée, avec une assistance informatique, pour assurer le bon ordonnancement des séquences d'intérêt lors de l'assemblage et un rendement optimum.

Selon l'invention, la suture monobrin cohésive d'au moins 2 nucléotides générée à chacune des extrémités amont et aval de la séquence d'intérêt, comprend une séquence choisie parmi « $4^z$ combinaisons possibles excluant les combinaisons qui résultent en un palindrome d'ADN dans laquelle z est compris de 2 à 10 et z est le nombre de nucléotides de la suture mono brin».

Le procédé selon l'invention comprend une réaction enzymatique combinant l'action d'une enzyme de restriction de type IIs définie selon l'invention et d'une ligase définie selon l'invention. Selon un mode de réalisation, le procédé selon l'invention est un procédé d'assemblage. Le produit de la réaction est une molécule d'ADN, de préférence circulaire contenant le nombre de briques moléculaires voulu, assemblées de façon ordonnée, sur la base des complémentarités des sutures. Le bon ordonnancement des briques assure *de facto* le bon ordonnancement des modules quel que soit le nombre de modules contenus dans chaque brique.

Selon un mode avantageux de réalisation, le procédé selon l'invention est un procédé d'assemblage sans cicatrice.

Selon un mode de réalisation, l'adaptateur comprend au moins un autre site de reconnaissance d'une enzyme de type IIs, ledit site est reconnu par une enzyme de type IIs différente de celle utilisée dans le procédé selon l'invention et n'est pas reconnu par l'enzyme de type IIs utilisée dans le procédé selon l'invention.

Selon un mode avantageux, l'adaptateur selon l'invention comprend, en outre, au moins un site de restriction, et plus avantageusement au moins un site de restriction choisi parmi les sites rares de restriction, et plus avantageusement encore, un site de restriction parmi les sites rares de restriction choisis parmi NotI, PacI, PmeI, SwaI, SmiI, SgsI, SgrDI, SgrAI, SbfI, FseI, AscI, AsiSI, MreI, MssI.

Dans tous les modes de réalisation selon l'invention, le site de reconnaissance de l'enzyme de type IIs qui est utilisée dans le procédé selon l'invention, présent dans chaque adaptateur, consiste en un site unique et se localise uniquement dans les adaptateurs, les séquences d'intérêt selon l'invention n'en comprenant aucun.

Le procédé selon l'invention ne met en œuvre qu'une seule enzyme de type IIs, ladite enzyme de type IIs reconnaissant une séquence d'ADN, en particulier une séquence d'ADN unique.

La divulgation ne saurait se limiter à une seule enzyme de type IIs reconnaissant une séquence unique.

Toute enzyme de type IIs présentant l'activité de restriction voulue, c'est-à-dire pouvant se lier à ladite séquence unique et générer les séquences d'ADN monobrin attendues, fait partie de la présente invention.

De même, toute séquence présentant une variation n'affectant pas ou peu la reconnaissance et /ou l'activité de l'enzyme de type IIs fait partie de l'invention.

Selon un mode de réalisation, la divulgation concerne un procédé de fabrication d'un vecteur circulaire d'ADN double brin tel que défini précédemment comprenant une étape de mise en contact simultanée des briques moléculaires Ori-AmpR BsaI B (SEQ ID NO : 36), pCMV BsaI B (SEQ ID NO : 37), hFUT3 BsaI A (SEQ ID NO : 38), BGHpA BsaI B (SEQ ID NO : 39), et shB3Galt6 BsaI A (SEQ ID NO : 40), pour obtenir le vecteur V1 (SEQ ID NO : 41).

Selon un mode de réalisation, la divulgation concerne un procédé de fabrication d'un vecteur circulaire d'ADN double brin tel que défini précédemment comprenant une étape de mise en contact simultanée des briques moléculaires Ori-AmpR BsaI B (SEQ ID NO :36), pCMV BsaI B (SEQ ID NO : 37), hFUT3 BsaI A (SEQ ID NO : 38), BGHpA BsaI B (SEQ ID NO : 39), shB3GALT6 BsaI B (SEQ ID NO : 46), et HygroR BsaI B (SEQ ID NO : 47), pour obtenir le vecteur V1.1 (SEQ ID NO : 48).

Selon un mode de réalisation, la divulgation concerne un procédé de fabrication d'un vecteur circulaire d'ADN double brin tel que défini précédemment comprenant une étape de mise en contact simultanée des briques moléculaires Ori-AmpR BsaI B (SEQ ID NO : 36), rosa26-5' BsaI A B (SEQ ID NO : 39), shB3GALT6 BsaI B (SEQ ID NO : 46), HygroR BsaI C (SEQ ID NO : 51), et rosa26-3' BsaI A (SEQ ID NO : 52), pour obtenir le vecteur V1.2 (SEQ ID NO : 53).

Selon un mode de réalisation, la divulgation concerne un procédé de fabrication d'un vecteur circulaire d'ADN double brin tel que défini précédemment comprenant une étape de mise en contact simultanée des briques moléculaires Ori-AmpR BsaI B (SEQ ID NO : 36), rosa26-5' BsaI A (SEQ ID NO : 49), pCMV BsaI C (SEQ ID NO : 50), hFUT3 BsaI A (SEQ ID NO : 38), BGHpA BsaI B(SEQ ID NO : 39), shB3GALT6 BsaI B (SEQ ID NO : 46), HygroR BsaI C (SEQ ID NO : 51), rosa26-3' BsaI B (SEQ ID NO : 54), pEF1a BsaI A (SEQ ID NO : 55), TK BsaI A (SEQ ID NO : 56), et Tkter BsaI A (SEQ ID NO : 57), pour obtenir le vecteur V1.3 (SEQ ID NO : 58).

Selon un mode de réalisation, la divulgation concerne un procédé de fabrication d'un vecteur circulaire d'ADN double brin tel que défini précédemment comprenant une étape de mise en contact simultanée des briques moléculaires Ori-AmpR BsaI B (SEQ ID NO : 36), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B(SEQ ID NO : 63), et Tkter BsaI A (SEQ ID NO : 57), pour obtenir le vecteur V2 (SEQ ID NO : 62).

Selon un mode de réalisation, la divulgation concerne un procédé de fabrication d'un vecteur circulaire d'ADN double brin tel que défini précédemment comprenant une étape de mise en contact simultanée des briques moléculaires Ori-AmpR BsaI B (SEQ ID NO : 36), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mb3GALT6 BsaI B (SEQ ID NO :63), Tkter BsaI B (SEQ ID NO :64), et shB3Galt6 BsaI C (SEQ ID NO :65), pour obtenir le vecteur V3 (SEQ ID NO : 66).

Selon un mode de réalisation, la divulgation concerne un procédé de fabrication d'un vecteur circulaire d'ADN double brin tel que défini précédemment comprenant une étape de mise en contact simultanée des briques moléculaires Ori BsaI A (SEQ ID NO : 104), AmpR BsaI A (SEQ ID NO : 105), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B (SEQ ID NO : 63), et Tkter BsaI A (SEQ ID NO : 57), pour obtenir le vecteur V2b (SEQ ID NO : 149).

Selon un mode de réalisation, la divulgation concerne un procédé de fabrication d'un vecteur circulaire d'ADN double brin tel que défini précédemment comprenant une étape de mise en contact simultanée des briques moléculaires Ori BsaI A (SEQ ID NO : 104), AmpR BsaI A (SEQ ID NO : 105), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mb3GALT6 BsaI B (SEQ ID NO :63), Tkter BsaI B (SEQ ID NO : 64), et shB3Galt6 BsaI C (SEQ ID NO : 65), pour obtenir le vecteur V3b (SEQ ID NO : 150).

Selon un mode de réalisation, concerne un procédé de fabrication d'un vecteur circulaire d'ADN double brin tel que défini précédemment comprenant une étape de mise en contact simultanée des briques moléculaires Ori BsaI B (SEQ ID NO : 106), AmpR BsaI B (SEQ ID NO : 107), pEF1aL BsaI B (SEQ ID NO : 108), EGFP-CAAX BsaI A (SEQ ID NO : 109), BGHpA BsaI C (SEQ ID NO : 110), pCMV BsaI D (SEQ ID NO : 111), SiaT BsaI B (SEQ ID NO : 112), mCherry BsaI B (SEQ ID NO : 113), TKter BsaI B (SEQ ID NO : 64) et HygroR BsaI D (SEQ ID NO : 114), pour obtenir le vecteur V1.1b (SEQ ID NO : 151).

Selon un mode de réalisation, la divulgation concerne un procédé de fabrication d'un vecteur circulaire d'ADN double brin tel que défini précédemment comprenant une étape de mise en contact simultanée des briques moléculaires Ori-2 BsaI C (SEQ ID NO : 115), AmpR BsaI C (SEQ ID NO : 116), MNN10-Lrec BsaI A (SEQ ID NO : 117), KanMX BsaI A (SEQ ID NO : 119), MNN10-Rrec BsaI A (SEQ ID NO : 118), pour obtenir le vecteur V4 (SEQ ID NO : 152).

Selon un mode de réalisation, la divulgation concerne un procédé de fabrication d'un vecteur circulaire d'ADN double brin tel que défini précédemment pour obtenir un vecteur choisi parmi le groupe de vecteurs de séquence SEQ ID NO : 30, 31, 32, 33, 34 et 35.

Selon un mode de réalisation, la divulgation concerne un procédé de fabrication d'un vecteur circulaire d'ADN double brin tel que défini précédemment pour obtenir un vecteur choisi parmi le groupe de vecteurs de séquence SEQ ID NO : 41, 48, 53, 58, 62 et 66.

Selon un mode de réalisation, la divulgation concerne un procédé de fabrication d'un vecteur circulaire d'ADN double brin tel que défini précédemment pour obtenir un vecteur choisi parmi le groupe de vecteurs de séquence SEQ ID NO : 149, 150, 151 et 152.

Selon un mode de réalisation, la divulgation concerne un procédé de fabrication d'un vecteur circulaire d'ADN double brin tel que défini précédemment pour obtenir un vecteur choisi parmi le groupe de vecteurs de séquence SEQ ID NO : 30, 31, 32, 33, 34, 35, 41, 48, 53, 58, 62, 66, 149, 150, 151 et 152.

Selon un aspect particulier, la présente divulgation porte sur un procédé tel que celui décrit ci-dessus comprenant :

a) une étape de mise en contact simultanée de n briques moléculaires, chaque brique moléculaire étant une molécule d'ADN linéaire double brin, en particulier une molécule d'ADN linéaire double brin à extrémités non cohésives, et contenant : (i) Une séquence d'intérêt (SI)i dépourvue de site de reconnaissance spécifique de la susdite enzyme de restriction de type IIs et comprenant au moins une unité, ladite unité étant une unité fonctionnelle ou une unité non fonctionnelle, ladite unité fonctionnelle comprenant au moins un module, ledit module étant un module fonctionnel ou un module non fonctionnel, et (ii) deux adaptateurs d'ADN double brin A(i-1,i) et A(i,i+1), différents l'un de l'autre, flanqués respectivement en amont et en aval de ladite séquence d'intérêt (SI)i, chaque adaptateur d'ADN double brin consistant en une séquence d'au moins 12 nucléotides,

- la séquence d'au moins 12 nucléotides de l'adaptateur d'ADN double brin A(i-1,i) contenant un seul et unique site de reconnaissance de la susdite enzyme de restriction de type IIs, et une suture d'au moins 2 nucléotides, s(i-1, i) en aval du site de reconnaissance de ladite enzyme de restriction de type IIs,
- la séquence d'au moins 12 nucléotides de l'adaptateur d'ADN double brin A(i,i+1) contenant un seul et unique site de reconnaissance de la susdite enzyme de restriction de type IIs, et une suture d'au moins 2 nucléotides, s(i,i+1), en amont du site de reconnaissance de ladite enzyme de restriction de type IIs,

le site de reconnaissance de la susdite enzyme de restriction de type IIs de l'adaptateur d'ADN double brin A(i-1,i) en amont de ladite séquence d'intérêt et le site de reconnaissance de la susdite enzyme de restriction de type IIs de l'adaptateur d'ADN double brin A(i,i+1) en aval de ladite séquence spécifique étant convergents,

(SI)1 étant la séquence d'intérêt (SI)i dans laquelle i=1
(SI)n étant la séquence d'intérêt (SI)i dans laquelle i=n

n étant un nombre entier allant de 2 à 100, i allant de 1 à n, i étant différent de n quand i=1
et quand i=n alors i+1 est 1
et lorsque i=1, alors i-1 =n, de sorte que la suture monobrin cohésive d'au moins 2 nucléotides générée en amont de (SI)1, si-1, est la séquence complémentaire de la suture monobrin cohésive d'au moins 2 nucléotides générée en aval de (SI)n, sn +1,
et lorsque i=n alors la suture monobrin cohésive d'au moins 2 nucléotides générée en aval de (SI)n, sn+1, est la séquence complémentaire de la suture monobrin cohésive d'au moins 2 nucléotides générée en amont de (SI)1, si-1, laquelle étape conduit :

- à l'élimination des sites de reconnaissance de l'enzyme de restriction de type IIs utilisée,
- à la formation d'une extrémité cohésive constituée par une suture monobrin d'au moins 2 nucléotides, à chacune des extrémités amont et aval de chacune des (SI)i et
- à l'appariement par complémentarité nucléotidique de la susdite suture monobrin cohésive d'au moins 2 nucléotides en aval de (SI)i avec la suture monobrin cohésive d'au moins 2 nucléotides en amont de la (SI)i+1, et de la susdite suture monobrin cohésive d'au moins 2 nucléotides en amont de la (SI)i avec la susdite suture monobrin cohésive d'au moins 2 nucléotides en aval de (SI)i-1
- et au positionnement des susdites (SI)i de façon contigüe les unes aux autres, selon un ordre et un sens unique et déterminé,

b) une étape de ligation des susdites sutures monobrins cohésives d'au moins 2 nucléotides pour obtenir un vecteur circulaire d'ADN double brin.

Selon l'invention, le site de reconnaissance de l'enzyme de type IIs de l'adaptateur en aval de la séquence d'intérêt, est localisé et orienté de sorte que l'enzyme coupe l'ADN de telle façon que le site de reconnaissance est éliminé, et une suture monobrin est générée (figure 1).
Selon l'invention le procédé est mis en œuvre en présence d'une ligase.
On entend par ligase, une enzyme de la classe des ligases (EC6) qui lie les brins d'acides nucléiques, en particulier les ADN ligase (EC 6.5.1.1). Une ligase selon l'invention lie des extrémités d'ADN, d'oligonucléotides, d'ARN et d'hybride ARN-ADN. De préférence, une ligase selon l'invention lie des molécules d'acides nucléiques à extrémités cohésives.
La ligase utilisée dans le procédé selon l'invention est une ligase choisie parmi une ligase T3, T4 T7 ou Taq ligase, préférentiellement une ligase T3 et plus préférentiellement une ligase T7 (T7 DNA ligase) et encore plus avantageusement, une T4 ligase.
Selon un mode de réalisation avantageux, le procédé est un procédé dans lequel l'étape (a) de mise en contact simultanée d'au moins deux briques moléculaires, différentes l'une de l'autre, en présence d'une seule enzyme de restriction de type IIs, est effectuée à une température allant de 20°C à une température de 55°C, pendant une période allant de 2 minutes à une période de 30 minutes,
l'étape (b) de ligation est effectuée à une température allant de 10°C à une température de 40°C pendant une période allant de 2 min à un période de 30 min,

(a) et (b) pouvant être répétées de 1 à 49 fois,
ledit procédé comprenant en outre,
(c) au moins une étape d'incubation à une température de 41 à 60°C pendant une période allant de 0.5 à 15 min, et éventuellement,

(d) une étape d'incubation à une température de 61 à 90°C pendant une période allant de 0.5 à 15 minutes.

Selon un mode de réalisation avantageux, le procédé est un procédé dans lequel l'étape (a) de mise en contact simultanée d'au moins deux briques moléculaires, différentes l'une de l'autre, en présence d'une seule enzyme de restriction de type IIs, est effectuée à une température allant de 20°C à une température de 55°C, pendant une période allant de 2 minutes à une période de 30 minutes,
l'étape (b) de ligation est effectuée à une température allant de 10°C à une température de 40°C pendant une période allant de 2 min à un période de 30 min,

(a) et (b) pouvant être répétées de 1 à 49 fois,
ledit procédé comprenant en outre,
(c) au moins une étape d'incubation à une température de 41 à 60°C pendant une période allant de 0.5 à 15 min et
(d) une étape d'incubation à une température de 61 à 90°C pendant une période allant de 0.5 à 15 minutes.

Selon un mode de réalisation particulier, le procédé est un procédé dans lequel l'étape

(a) de mise en contact simultanée d'au moins deux briques moléculaires, différentes l'une de l'autre, en présence d'une seule enzyme de restriction de type IIs, est effectuée à une température allant de 20°C à une température de 55°C, pendant une période allant de 2 minutes à une période de 30 minutes, l'étape
(b) de ligation s'effectue à une température supérieure à 40°C en présence de TAq Ligase. Selon ce mode de réalisation particulier, l'étape (b) de ligation s'effectue à une température inférieure à 95°C, de préférence inférieure à 65 °C.
L'étape (b) de ligation est effectuée pendant une période allant de 2 min à un période de 30 min, et (b) pouvant être répétées de 1 à 49 fois,
ledit procédé comprenant en outre,
(c) au moins une étape d'incubation à une température de 41 à 60°C pendant une période allant de 0,5 à 15 min, et éventuellement,
(d) une étape d'incubation à une température de 61 à 90°C pendant une période allant de 0.5 à 15 minutes.

Selon un mode de réalisation particulier, le procédé est un procédé dans lequel l'étape

(a) de mise en contact simultanée d'au moins deux briques moléculaires, différentes l'une de l'autre, en présence d'une seule enzyme de restriction de type IIs, est effectuée à une température allant de 20°C à une température de 55°C, pendant une période allant de 2 minutes à une période de 30 minutes, l'étape
(b) de ligation s'effectue à une température supérieure à 40°C en présence de TAq Ligase. Selon ce mode de réalisation particulier, l'étape (b) de ligation s'effectue à une température inférieure à 95°C, de préférence inférieure à 65 °C.
L'étape (b) de ligation est effectuée pendant une période allant de 2 min à un période de 30 min, et (b) pouvant être répétées de 1 à 49 fois,
ledit procédé comprenant en outre,
(c) au moins une étape d'incubation à une température de 41 à 60°C pendant une période allant de 0,5 à 15 min et
(d) une étape d'incubation à une température de 61 à 90°C pendant une période allant de 0.5 à 15 minutes.

Selon un autre mode de réalisation, le procédé de fabrication d'un vecteur circulaire d'ADN double brin est un procédé dans lequel l'une des au moins deux séquences d'intérêt comprend au moins une unité non fonctionnelle.

Selon un autre mode de réalisation, le procédé de fabrication d'un vecteur circulaire d'ADN double brin est un procédé comportant au moins deux séquences d'intérêt, dans lequel lesdites au moins deux séquences d'intérêt sont constituées d'une unité non fonctionnelle et dans lequel le positionnement desdites séquences d'intérêt de façon contigüe les unes aux autres conduit à une entité fonctionnelle d'ADN double brin.

Selon un mode de réalisation, le procédé de fabrication d'un vecteur circulaire d'ADN double brin est un procédé de fabrication d'un vecteur circulaire d'ADN double brin dans lequel l'une des au moins deux séquences d'intérêt comprend au moins une unité fonctionnelle.

Selon la divulgation, une unité fonctionnelle peut être une unité fonctionnelle d'expression ou un gène, une unité fonctionnelle d'intégration en cellule eucaryote ou procaryote, une unité fonctionnelle bactérienne.

Selon la divulgation, une unité fonctionnelle d'expression ou un gène comprend une séquence codante et des éléments non codants tels qu'un promoteur, un terminateur, chacun pouvant être considéré individuellement comme un module fonctionnel.

Un gène est une séquence d'acide désoxyribonucléique (ADN) qui spécifie la synthèse d'une chaîne de polypeptides ou d'un acide ribonucléique (ARN). On peut également définir un gène comme une unité d'information génétique. Un gène comprend une séquence de nucléotides appelée promoteur, dont le rôle est de permettre l'initiation mais surtout la régulation de la transcription de l'ADN en ARN. Dans le cas d'ARN dit codant, la molécule d'ARN ainsi produite peut être traduite en protéine. La séquence d'ADN correspondant à l'information qui sera traduite en protéine est appelée cadre ouvert de lecture. Un ARN non traduit peut également être fonctionnel (ex : ARN ribosomaux, ARN de transfert, ARN interférant). Un gène peut se terminer par une séquence terminatrice appelée terminateur, qui marque la fin de la transcription.

Selon un mode de réalisation le vecteur permet d'apporter au moins une information génétique à la cellule hôte, en permettant l'expression ou l'inhibition d'au moins un gène, la fabrication ou le blocage d'au moins un ARN, d'au moins une protéine.

Selon la divulgation, le procédé de fabrication d'un vecteur circulaire d'ADN double brin est un procédé dans lequel au moins une unité fonctionnelle est une unité fonctionnelle choisie parmi les éléments suivants :

(i) une unité fonctionnelle bactérienne,
(ii) une unité fonctionnelle d'expression,
(iii) une unité fonctionnelle d'intégration en cellule eucaryote ou procaryote,
(iv) ou une combinaison.

Selon la divulgation, un vecteur d'expression peut contenir au moins trois éléments fonctionnels, 1. Origine de réplication bactérienne, 2. Un marqueur de sélection bactérien, 3. Une cassette d'expression.

Le procédé de fabrication d'un vecteur circulaire d'ADN double brin est un procédé dans lequel l'une des au moins deux séquences d'intérêt comprend au moins une unité non fonctionnelle.

On entend par unité fonctionnelle bactérienne, une unité fonctionnelle comprenant une origine de réplication bactérienne et un marqueur de sélection bactérien.

Une origine de réplication peut être d'origine bactérienne ou artificielle (aussi appelée « ori »). C'est une séquence unique d'ADN permettant l'initiation de la réplication unidirectionnelle ou bidirectionnelle, en particulier dans une cellule bactérienne. La réplication est le processus au cours duquel l'ADN est synthétisé grâce à l'ADN polymérase. Ce mécanisme permet d'obtenir, à partir d'une molécule d'ADN, deux molécules d'ADN identiques à la molécule initiale, sauf erreur de l'enzyme. La structure de l'origine de réplication varie d'une espèce à l'autre. Dans l'unité fonctionnelle de réplication bactérienne, l'origine de réplication est d'origine bactérienne.

Des exemples d'origine de réplication sont ceux décrits dans la Table 1 page 49 de l'article de Wang et al., 2009 (Zhijun Wang a,, Li Jin a,b, Zhenghong Yuan c, Grzegorz We,grzyn d, Alicja We,grzyn. Classification of plasmid vectors using replication origin, selection marker and promoter as criteria. Plasmid, 61 (2009) 47-51).

Selon un aspect de la divulgation, le procédé de fabrication d'un vecteur circulaire d'ADN double brin est un procédé dans lequel l'unité fonctionnelle bactérienne contient au moins une origine de réplication bactérienne choisie parmi les éléments figurant dans la publication de Wang et al., 2009.

Selon un autre aspect avantageux de la divulgation, le procédé de fabrication d'un vecteur circulaire d'ADN double brin est un procédé dans lequel l'unité fonctionnelle bactérienne contient au moins une origine de réplication choisie parmi toutes les origines de réplication procaryotes de la Table 1 de Wang et al., 2009. De préférence les plasmides sont pMB1, pUC, ColE1, p15A, pSC101, R1, RK2, R6K, pA81, pRAS3.1, pTi, pBPS1, pUO1, pKH9, pWKS1, pCD1, pMAK3, pBL63.1, pTA1060, p4M, pHT926, pCD6, pJB01, pIME300, pMD5057, pTE44, pDP1, ou pT38.

Un marqueur de sélection est un gène dont l'expression confère à son hôte une propriété mesurable comme par exemple la capacité à fabriquer un pigment ou à résister à un antibiotique.

Un marqueur de sélection bactérien est un gène dont l'expression confère à des bactéries transformées (qui ont incorporé le vecteur permettant l'expression du marqueur d'intérêt) une propriété mesurable.

Un marqueur de sélection bactérien permet par exemple de sélectionner des bactéries selon un crible défini. Il peut s'agir d'un gène de résistance à un antibiotique, d'un gène permettant la complémentation d'une auxotrophie, d'un gène codant l'expression d'une molécule détectable optiquement (colorant, marqueur chimio-luminescent, fluorochrome) ou de tout autre gène dont le produit permettrait de discriminer les colonies bactériennes.

Par définition, un marqueur de sélection bactérien est donc par exemple un gène de résistance à un antibiotique, ou un gène de criblage.

Selon encore un autre aspect de la divulgation, le procédé de fabrication d'un vecteur circulaire d'ADN double brin est un procédé dans lequel l'unité fonctionnelle bactérienne comporte au moins un module fonctionnel, le module fonctionnel étant un marqueur, en particulier un marqueur de sélection, et de préférence un marqueur de sélection qui est un gène de résistance à un antibiotique ou un gène de criblage.

Des exemples de marqueurs de sélection bactériens sont des gènes codant des éléments permettant une résistance à un antibiotique tels que:

- gène *bla* (AmpR) permettant la résistance à l'ampicilline,
- gène neo permettant la résistance à la néomycine,
- gène *aph* (kanR) permettant la résistance à la kanamycine,
- gène *cat* permettant la résistance au chloramphénicol,
- gène *aad*A7 permettant la résistance à la spectinomycine,
- gène *aac*C1 permettant la résistance à la gentamicine,
- gène tetA permettant la résistance à la tétracycline,
- gène erm permettant la résistance à l'érythromycine,
- gène van permettant la résistance à la vancomycine

Selon la divulgation, le procédé de fabrication d'un vecteur circulaire d'ADN double brin est un procédé dans lequel le gène de résistance à un antibiotique est choisi parmi les gènes Ampicillin bla, Ampicillin blaA, Ampicillin blaZ, Kanamycin aph, Neomycin neo, Chloramphenicol cat, Chloramphenicol cmIA, Chloramphenicol catAIII, Chloramphenicol catB2, Chloramphenicol cmx, Gentamycin aacC1, Gentamycin aacC2, Tetracycline tetA(A), Tetracycline tetA(C), Tetracycline tetA(D), Tetracycline tetA(E), Tetracycline tetA(G), Tetracycline tetA(H), Tetracycline tetA(L), Tetracycline tetA(Q), Tetracycline tetA(S), Tetracycline tetA(Y)Tetracycline tetA(Z), Erythromycin erm, Vancomycin van, Spectinomycin aadA7, Streptomycin str. (Table 2, Wang et al., 2009).

De préférence, le procédé de fabrication d'un vecteur circulaire d'ADN double brin est un procédé dans lequel le gène de résistance à un antibiotique est choisi parmi les gènes permettant une résistance à l'ampicilline, à la kanamycine, à la néomycine, à la gentamycine, à la spectinomycine.

Selon la divulgation, le procédé de fabrication d'un vecteur circulaire d'ADN double brin est un procédé dans lequel le gène de criblage est le gène lac Z$\alpha$.

Une unité fonctionnelle d'expression selon le procédé de l'invention est une unité qui comprend au moins un module fonctionnel, ledit module fonctionnel étant constitué par l'un des éléments suivants.

(i) une séquence régulatrice de l'expression des gènes ou séquence régulatrice de l'activité des séquences régulatrices des gènes, choisie parmi :
un promoteur, un terminateur, une séquence « Internal ribosome entry site » (IRES),
(ii) une séquence nucléotidique codant un produit, en particulier une séquence nucléotidique codant un produit d'expression, et plus particulièrement une séquence nucléotidique codant une protéine,
(iii) une séquence codant une étiquette moléculaire ou
(iv) une combinaison de ces éléments.

Selon la divulgation, un promoteur, ou séquence promotrice, est une région d'ADN constituante d'un gène et indispensable à la transcription de l'ADN en ARN. Le promoteur est la zone de l'ADN sur laquelle se fixent initialement les facteurs de transcriptions et l'ARN polymérase, avant de démarrer la synthèse de l'ARN. Les séquences promotrices sont en général situées en amont du site de démarrage de la transcription. Les promoteurs utilisés dans le procédé selon l'invention sont constitutifs ou inductibles.

Selon la divulgation une séquence codante ou cadre de lecture est une séquence d'ADN qui, lorsqu'elle est transcrite par une enzyme qui est une ARN polymérase, correspond à un ARN, en particulier un ARN messager (ARNm). Ladite séquence codante se situe en aval du promoteur et en amont du terminateur dans le sens de lecture de la molécule.

L'ARNm transcrit peut aussi correspondre, sans être limité à une ou plusieurs phases ouvertes de lecture (ARN traductible en peptide ou protéine), à un ou plusieurs ARN non codant (ex : petit ARN interférant, micro-ARN, ARN catalytique).

Selon la divulgation un terminateur ou terminateur de transcription est une séquence d'ADN qui marque la fin de la transcription d'un ARN par l'enzyme responsable de la transcription. Selon la divulgation un terminateur est un terminateur procaryote ou eucaryote.

Selon la divulgation une séquence IRES (de l'anglais Internal ribosome entry site), est une séquence qui, dans les cellules eucaryotes, permet le démarrage de la traduction d'un ARN messager de manière interne. Le processus conventionnel de la traduction des ARNm eucaryotes repose sur un mécanisme de balayage par le ribosome à partir de la coiffe située à l'extrémité 5', qui scrute l'ARNm jusqu'au premier codon de démarrage. Les IRES permettent le recrutement direct du ribosome au niveau de ce codon de démarrage, indépendamment de la présence de la coiffe et du mécanisme de balayage. Les IRES sont des régions structurées de l'ARNm qui interagissent directement avec le ribosome ou avec les facteurs d'initiation de la traduction.

Selon la divulgation une étiquette moléculaire est une séquence d'ADN, en particulier codant un peptide ou une protéine qui sera fusionné à une protéine d'intérêt.

La séquence de l'étiquette est insérée, ou assemblée selon l'invention, en phase, en amont du premier codon de la protéine d'intérêt ou bien en aval du dernier codon de la protéine d'intérêt, ou à l'intérieur du cadre ouvert de lecture de la protéine d'intérêt. Les propriétés intrinsèques de l'étiquette permettent de visualiser et ou de purifier la protéine d'intérêt soit directement (fluorochrome) soit indirectement (épitope reconnu par un anticorps ou par une autre protéine, activité enzymatique,...).

Selon la divulgation, le procédé de fabrication d'un vecteur circulaire d'ADN double brin est un procédé dans lequel l'unité fonctionnelle d'expression comprend au moins un module fonctionnel, ledit module fonctionnel comprenant un promoteur, une séquence nucléotidique codant une protéine et un terminateur.

Selon la divulgation, le procédé de fabrication d'un vecteur circulaire d'ADN double brin est un procédé dans lequel l'unité fonctionnelle d'expression comprend au moins un module fonctionnel, ledit module fonctionnel comprenant un promoteur, une séquence nucléotidique codant une protéine ou un terminateur.

Selon la divulgation, le procédé de fabrication d'un vecteur circulaire d'ADN double brin est un procédé dans lequel l'unité fonctionnelle d'expression comprend au moins un module fonctionnel d'expression, ledit module fonctionnel comprenant un promoteur, une séquence nucléotidique codant une protéine, une étiquette moléculaire et un terminateur.

Selon la divulgation, le procédé de fabrication d'un vecteur circulaire d'ADN double brin est un procédé dans lequel l'unité fonctionnelle d'expression comprend au moins un module fonctionnel d'expression, ledit module fonctionnel comprenant un promoteur, une séquence nucléotidique codant une protéine, une étiquette moléculaire ou un terminateur.

Selon la divulgation, le procédé de fabrication d'un vecteur circulaire d'ADN double brin est un procédé dans lequel l'unité fonctionnelle d'expression comprend au moins un module fonctionnel d'expression, ledit module fonctionnel d'expression comprenant un promoteur, une séquence nucléotidique codant une protéine, une séquence IRES, une seconde séquence nucléotidique codant une protéine et un terminateur.

Selon la divulgation, le procédé de fabrication d'un vecteur circulaire d'ADN double brin est un procédé dans lequel l'unité fonctionnelle d'expression comprend au moins un module fonctionnel d'expression, ledit module fonctionnel d'expression comprenant un promoteur, une séquence nucléotidique codant une protéine, une séquence IRES, une seconde séquence nucléotidique codant une protéine ou un terminateur.

Selon la divulgation, le procédé de fabrication d'un vecteur circulaire d'ADN double brin est un procédé dans lequel au moins un module fonctionnel est un module fonctionnel d'expression contenant une séquence nucléotidique codant une protéine de fusion.

Selon la divulgation, le procédé de fabrication d'un vecteur circulaire d'ADN double brin est un procédé dans lequel un module fonctionnel est un promoteur.

Selon la divulgation, le procédé de fabrication d'un vecteur circulaire d'ADN double brin est un procédé dans lequel un module fonctionnel est un promoteur et ledit promoteur est un promoteur du cytomégalovirus (CMV), un promoteur EF1$\alpha$, un promoteur du virus SV40, un promoteur de la béta-actine ou un promoteur de l'ubiquitine C. De préférence, le promoteur selon l'invention est un promoteur choisi parmi les promoteurs décrits table 3 de Wang et al., 2009.

Selon la divulgation, le procédé de fabrication d'un vecteur circulaire d'ADN double brin est un procédé dans lequel un module fonctionnel est un gène codant un produit d'expression. Selon l'invention, le procédé de fabrication d'un vecteur circulaire d'ADN double brin est un procédé dans lequel un module fonctionnel est un gène codant un produit d'expression choisi parmi les gènes référencés dans la base de données « Gene » du NCBI (National Center for Biotechnology Information) (http://www.ncbi.nlm.nih.gov/gene/) et ledit gène codant un produit d'expression est un gène dont la séquence peut appartenir aux espèces, *Homo sapiens, Mus musculus, Rattus norvegicus, Danio rerio, Caenorhabditis elegans, Saccharomyces Cerevisiae, Arabidopsis thaliana, rosophila melanogaster,* ou à toute autre espèce référencée.

Selon la divulgation, le procédé de fabrication d'un vecteur circulaire d'ADN double brin est un procédé dans lequel un module fonctionnel est un terminateur.

Selon la divulgation, le procédé de fabrication d'un vecteur circulaire d'ADN double brin est un procédé dans lequel un module fonctionnel est un terminateur et ledit terminateur est une séquence de polyadénylation.

Selon la divulgation, le procédé de fabrication d'un vecteur circulaire d'ADN double brin est un procédé dans lequel au moins un module fonctionnel est un marqueur ou étiquette moléculaire choisi parmi la séquence AviTag, Calmodulin-tag, polyglutamate tag, E-tag, FLAG-tag, HA-tag, His-tag, Mc-tag, S-tag, SBP-tag, Softag 1, Softag 3, Strep-tag, TC tag, V5 tag, VSV-tag, Xpress tag, Isopeptag , SpyTag, BCCP (Biotin Carboxyl Carrier Protein), Glutathione-S-transferase-tag, Green fluorescent protein-tag, Maltose binding protein-tag, Nus-tag, Thioredoxin-tag, Fc-tag, Designed Intrinsically Disordered tags containing disorder promoting amino acids (P,E,S,T,A,Q,G,...) and Ty tag.

Selon la divulgation, le procédé de fabrication d'un vecteur circulaire d'ADN double brin est un procédé dans lequel au moins un module fonctionnel est un marqueur moléculaire (ou étiquette moéluculaire) ledit marqueur moléculaire étant une protéine d'affinité, choisie parmi la Maltose Binding Protein (MBP), la Glutathione-S-Transferase (GST), la protéine Tandem Affinity Purification (TAP)-Tag, TAP-Tag, ou une séquence codant une protéine fluorescente, de préférence la GFP ou l'un de ses nombreux variants (BFP, CFP, YFP, mCherry...).

Selon la divulgation, le procédé de fabrication d'un vecteur circulaire d'ADN double brin est en particulier un procédé dans lequel au moins une unité fonctionnelle est une unité fonctionnelle d'intégration en cellule eucaryote.

Selon la divulgation, le procédé de fabrication d'un vecteur circulaire d'ADN double brin est un procédé dans lequel ladite unité fonctionnelle d'intégration en cellule eucaryote comprend au moins un module fonctionnel comprenant au moins un élément choisi parmi :

- un gène de sélection,
- une séquence de maintien en cellule eucaryote,
- une séquence d'intégration, en particulier une séquence d'intégration homologue
- une ou plusieurs séquences impliquées dans l'édition d'ADN et/ou une ou plusieurs séquences impliquées dans la recombinaison homologue ciblée,
- ou une combinaison de ces éléments.

Selon la divulgation, le procédé de fabrication d'un vecteur circulaire d'ADN double brin est un procédé dans lequel le gène de sélection est un gène de sélection positive ou un gène de sélection négative.

Selon un mode avantageux, un gène de sélection est un gène de sélection positive ou un gène de sélection négative et peut dépendre ou non de la présence de substrats externes.

Selon la divulgation, un gène de sélection positive est un gène qui permet la survie voire la croissance de la cellule ou de l'hôte dont le génome a été génétiquement modifié en présence d'agents normalement toxiques pour la cellule ou l'hôte (par exemple un antibiotique, un herbicide ou un médicament). Certains gènes de sélection positive ne sont pas conditionnés à des substrats extérieurs, mais modifient des processus physiologiques régissant le développement des cellules (bactéries, champignons, animaux ou plantes le cas échéant).

Selon la divulgation, un gène de sélection négative provoque la mort des cellules ou de l'hôte génétiquement modifiés dans certaines conditions, contrôlables et connues de l'expérimentateur. Selon l'invention une séquence de maintien en cellule eucaryote est une séquence d'ADN utilisable dans le cas où le vecteur est destiné à se maintenir dans la descendance cellulaire de la cellule récipiendaire, en absence d'intégration au génome hôte. Il peut s'agir d'origine de réplication propre à l'espèce de la cellule modifiée (exemple séquence Autonomous Replicating Sequence (ARS) pour la levure), de séquence de centromère permettant la ségrégation des molécules d'ADN dupliquées dans chacune des cellules filles.

Selon la divulgation, une séquence homologue au génome eucaryote est une séquence permettant l'intégration par recombinaison homologue. Le vecteur d'expression peut contenir des séquences d'ADN correspondant à l'ADN génomique de l'organisme ou de la cellule ciblée. Ces séquences sont déterminées expérimentalement et peuvent correspondre à des loci connus pour leur susceptibilité à la recombinaison homologue.

Selon la divulgation, une séquence impliquée dans l'édition d'ADN est une séquence d'ADN spécifiquement reconnue par une recombinase qui permet la modification ciblée de la molécule d'ADN. Par exemple la séquence LoxP1 reconnues par la recombinase Cre, ou la séquence FRT reconnues par la recombinase Flp.

Selon la divulgation, le procédé de fabrication d'un vecteur circulaire d'ADN double brin est un procédé dans lequel le gène de sélection et un gène de sélection positive, en particulier un gène de sélection positive en cellule eucaryote.

Selon un mode de réalisation, un gène de sélection positive est un gène de résistance aux antibiotiques choisi parmi : les gènes de résistance à l'hygromycine B ou à ses dérivés, les gènes de résistance au G418 ou à ses dérivés, les gènes de résistance à l'ampicilline ou à ses dérivés , les gènes de résistance à la tétracycline ou à ses dérivés, les gènes de résistance à la puromycine ou à ses dérivés, ou les gènes de résistance à la zéocine ou à ses dérivés.

Selon un autre mode de réalisation, un gène de sélection positive en cellule eucaryote est un gène de résistance aux antibiotiques choisi parmi les gènes de résistance à l'hygromycine B ou à ses dérivés, les gènes de résistance au G418 ou à ses dérivés, les gènes de résistance à la puromycine ou à ses dérivés, ou les gènes de résistance à la zéocine ou à ses dérivés.

Selon la divulgation, le procédé de fabrication d'un vecteur circulaire d'ADN double brin est un procédé dans lequel le gène de sélection négative est un gène de sélection négative codant la thymidine kinase dans la levure.

Selon la divulgation, le procédé de fabrication d'un vecteur circulaire d'ADN double brin est un procédé dans lequel la séquence de maintien en cellule eucaryote est une séquence de réplication autonome (Autonomous Replicating Sequence- ARS) ou une séquence de centromère telle que définie pour la levure.

Selon la divulgation, le procédé de fabrication d'un vecteur circulaire d'ADN double brin est un procédé dans lequel la séquence d'intégration homologue est un locus Rosa 26 ou un locus hypoxanthine phosphoribosyltransferase (HPRT).

Selon le procédé de fabrication d'un vecteur circulaire d'ADN double brin est un procédé dans lequel la séquence impliquée dans l'édition d'ADN et/ou la séquence impliquée dans la recombinaison homologue ciblée est une séquence de 34 nucléotides du bactériophage P1 « locus of X over P1 » (LoxP1) de séquence générique ATAACTTCGTATA -NNNTANNN-TATACGAAGTTAT (SEQ ID NO : 67) dans laquelle N est A, T, G ou C, de préférence une séquence Cre recombinase-LoxP.

Selon la divulgation, le procédé de fabrication d'un vecteur circulaire d'ADN double brin est un procédé dans lequel la séquence impliquée dans l'édition d'ADN et/ou la séquence impliquée dans la recombinaison homologue ciblée est une séquence FRT Flp-FRT en particulier une séquence GAAGTTCCTATTCtctagaaaGtATAGGAACTTC (SEQ ID NO : 68).

Selon la divulgation, le procédé de fabrication d'un vecteur circulaire d'ADN double brin est un procédé dans lequel l'enzyme de restriction de type IIs est une enzyme de restriction de type IIs choisie parmi BsaI, Eco31I, BbsI, BpiI, BsmBI, Esp3I, BspMI, BfuAI et BveI.

De préférence, la seule enzyme de restriction de type IIs utilisée dans le procédé selon l'invention est BbsI et plus préférentiellement la seule enzyme de type IIs utilisée dans le procédé selon l'invention est BsaI.

Selon l'invention, le procédé de fabrication d'un vecteur circulaire d'ADN double brin est un procédé dans lequel l'adaptateur d'ADN double brin en aval ou en amont de ladite séquence d'intérêt, comprend en outre au moins un site de reconnaissance d'une enzyme de restriction de type IIp, avantageusement au moins un site de reconnaissance d'une enzyme de restriction rare telles que NotI, PacI, PmeI, SwaI, SmiI, SgsI, SgrDI, SgrAI, SbfI, FseI, AscI, AsiSI, MreI, MssI et plus avantageusement deux sites de reconnaissance d'enzymes de restriction choisis parmi KpnI, AgeI, EcoRI et BstBI, SalI et MluI.

En général, le procédé de fabrication d'un vecteur circulaire d'ADN double brin est un procédé dans lequel les adaptateurs d'ADN double brin en amont et en aval de ladite séquence d'intérêt comprennent au moins un site de reconnaissance d'une enzyme de restriction de type IIs dont un seul et unique site de reconnaissance de l'enzyme de restriction de type IIs présente dans l'étape (a) de mise en contact simultanée d'au moins deux briques moléculaires, enzyme qui coupe l'ADN des adaptateurs et génère des extrémités simple brin d'au moins deux nucléotides de part et d'autre des séquences d'intérêt, (ou l'enzyme de type IIs utilisée dans le procédé à l'étape a).

Selon un mode de réalisation particulier, le procédé est un procédé dans lequel les adaptateurs d'ADN double brin en amont et en aval de ladite séquence d'intérêt ne comprennent aucun site de reconnaissance d'une enzyme de restriction de type IIs autre que celui de l'enzyme de restriction de type IIs, présente dans l'étape de mise en contact simultanée d'au moins deux briques moléculaires différentes l'une de l'autre.

Selon un mode de réalisation avantageux, le procédé de fabrication d'un vecteur circulaire d'ADN double brin est un procédé dans lequel chaque adaptateur d'ADN double brin, en amont et en aval de ladite séquence d'intérêt, ne comprend qu'un seul (et unique) site de reconnaissance de l'enzyme de restriction de type IIs présente dans l'étape (a) de mise en contact simultanée d'au moins deux briques moléculaires, différentes l'une de l'autre, et chaque adaptateur ne comprend aucun autre site de reconnaissance d'une enzyme de restriction de type IIs.

Plus précisément, le procédé de fabrication d'un vecteur circulaire d'ADN double brin est un procédé dans lequel chaque adaptateur d'ADN double brin en amont et en aval de ladite séquence d'intérêt, ne comprend qu'un seul (et unique) site de reconnaissance d'une seule enzyme de restriction de type IIs qui est l'enzyme de restriction de type IIs présente dans l'étape (a) de mise en contact simultanée d'au moins deux briques moléculaires, différentes l'une de l'autre, enzyme qui coupe l'ADN des adaptateurs et génère des extrémités simple brin d'au moins deux nucléotides de part et d'autre des séquences d'intérêt ou encore l'enzyme de type IIs utilisée dans le procédé selon l'invention.

Selon un mode de réalisation avantageux, le procédé de fabrication d'un vecteur circulaire d'ADN double brin selon l'invention est un procédé dans lequel le site de reconnaissance d'une enzyme de restriction de type IIs présent dans chaque adaptateur consiste en un site unique de reconnaissance de l'enzyme de type IIs utilisée dans le procédé selon l'invention.

En particulier, le site unique de reconnaissance de l'enzyme de type IIs utilisé dans le procédé selon l'invention, est un site reconnu par BbsI et plus préférentiellement par BsaI.

Selon un mode de réalisation particulier, le procédé de fabrication d'un vecteur circulaire d'ADN double brin est un procédé dans lequel les adaptateurs d'ADN double brin en amont et en aval de ladite séquence d'intérêt comprennent un seul (et unique) site de reconnaissance de l'enzyme de restriction de type IIs présente dans l'étape (a) de mise en contact simultanée d'au moins deux briques moléculaires, différentes l'une de l'autre, et au moins un site de reconnaissance d'une enzyme de restriction de type IIs, ladite enzyme de type IIs étant une enzyme, autre que celle présente dans l'étape a) de mise en contact simultanée d'au moins deux briques moléculaires différentes l'une de l'autre.

Selon l'invention, la suture monobrin cohésive d'au moins 2 nucléotides à chacune des extrémités amont et aval de la séquence d'intérêt, comprend 2 à 10 nucléotides, de préférence 2 à 5 nucléotides, et plus particulièrement 4 nucléotides.

Le procédé selon l'invention est un procédé dans lequel la suture monobrin cohésive d'au moins 2 nucléotides à chacune des extrémités amont et aval de la séquence d'intérêt comprend 2 à 10 nucléotides, de préférence 2 à 5 nucléotides, et plus particulièrement 4 nucléotides.

Selon un mode de réalisation particulier, le procédé de fabrication d'un vecteur circulaire d'ADN double brin est un procédé dans lequel les sutures monobrin cohésives d'au moins 2 nucléotides à chacune des extrémités amont et aval de la séquence d'intérêt comprennent indépendamment l'une de l'autre, 2, 3, 4, 5, 6, 7, 8, 9, 10, 11, ou 12 nucléotides.

Dans le procédé selon l'invention, chaque suture monobrin cohésive d'au moins 2 nucléotides générée à partir d'une brique moléculaire s'apparie avec une seule suture monobrin cohésive d'au moins 2 nucléotides générée à partir d'une autre brique moléculaire.

Selon encore un autre aspect de l'invention, le procédé de fabrication d'un vecteur circulaire d'ADN double brin est un procédé dans lequel la suture monobrin cohésive d'au moins 2 nucléotides ne peut s'apparier qu'avec une seule autre suture monobrin cohésive d'au moins 2 nucléotides présente dans le mélange réactionnel.

Selon la divulgation, le milieu réactionnel est un milieu dans lequel le procédé selon l'invention est mis en œuvre.

Selon un mode de réalisation particulier, le procédé de fabrication d'un vecteur circulaire d'ADN double brin est un procédé dans lequel la suture mono brin cohésive d'au moins 2 nucléotides, en amont et en aval de la séquence d'intérêt, est conçue à l'aide d'une matrice de score.

Selon l'invention, le procédé de fabrication d'un vecteur circulaire d'ADN double brin est un procédé dans lequel ladite enzyme de restriction de type IIs coupe l'ADN à une distance allant de 2 à 15 nucléotides, 2 à 14, 2 à 13, 2 à 12, 2 à 11, 2 à 10, 2 à 9, 2 à 8, 2 à 7, 2 à 6, 2 à 5, 2 à 4, 2 à 3 nucléotides du site de reconnaissance spécifique de ladite enzyme de type IIs.

Avantageusement, ladite enzyme de restriction de type IIs coupe l'un des deux brins d'ADN à une distance de 2 nucléotides, 3, 4, 5, 6, 7, 8, 9, 10, 11, 12, 13, 14, ou 15 nucléotides du site de reconnaissance spécifique de ladite enzyme de type IIs.

Selon le procédé de l'invention, la séquence complémentaire de la suture monobrin cohésive d'au moins 2 nucléotides générée en amont n'est pas complémentaire de la suture monobrin cohésive d'au moins 2 nucléotides générée en aval d'une même brique, la séquence complémentaire de la suture monobrin cohésive d'au moins 2 nucléotides générée en amont de la (SI)1, (si-1), (de la première brique) est complémentaire de la suture monobrin cohésive d'au moins 2 nucléotides en aval de la séquence (SI)n, (sn+1) (de la dernière brique).

Selon la divulgation, le procédé comprend, avant l'étape de mise en contact simultanée d'au moins deux briques moléculaires, une étape de préparation de chacune des briques moléculaires, par synthèse chimique ou par une étape d'amplification par PCR de la séquence d'intérêt contenue dans une brique à l'aide d'une amorce sens comprenant, de 5' en 3', une séquence correspondant à la séquence de l'adaptateur, et au moins 14 nucléotides de la séquence d'intérêt et d'une amorce anti-sens comprenant de 5' en 3' au moins 14 nucléotides de la séquence d'intérêt et au moins une séquence correspondant à la séquence de l'adaptateur.

Dans un mode de réalisation, le procédé comprend, avant l'étape de mise en contact simultanée d'au moins deux briques moléculaires, une étape de préparation de chacune des briques moléculaires, par synthèse chimique ou par une étape d'amplification par PCR de la séquence d'intérêt contenue dans une brique à l'aide d'une amorce sens comprenant, de 5' en 3', une séquence correspondant à la séquence de l'adaptateur, et au moins 14 à 20 nucléotides de la séquence d'intérêt et d'une amorce anti-sens comprenant de 5' en 3' au moins 14 à 20 nucléotides de la séquence d'intérêt et au moins une séquence correspondant à la séquence de l'adaptateur.

Dans un mode de réalisation, le procédé comprend, avant l'étape de mise en contact simultanée d'au moins deux briques moléculaires, une étape de préparation de chacune des briques moléculaires, par synthèse chimique ou par une étape d'amplification par PCR de la séquence d'intérêt contenue dans une brique à l'aide d'une amorce sens comprenant, de 5' en 3', une séquence correspondant à la séquence de l'adaptateur, et au moins 14, 15, 16, 17, 18, 19 ou 20 nucléotides de la séquence d'intérêt et d'une amorce anti-sens comprenant de 5' en 3' au moins 14, 15, 16, 17, 18, 19 ou 20 nucléotides de la séquence d'intérêt et au moins une séquence correspondant à la séquence de l'adaptateur.

La PCR est une réaction en chaine par polymérase et permet de reproduire l'ADN en quantité.

La présente divulgation porte également sur un procédé de fabrication d'un vecteur circulaire d'ADN double brin comprenant une étape de préparation de chacune des briques moléculaires, laquelle étape étant constituée par une étape d'amplification par réaction en chaine par polymérase (PCR) d'une séquence d'intérêt contenue dans une matrice à l'aide d'une amorce sens comprenant de 5' en 3', au moins une séquence correspondant à un adaptateur et au moins 14 nucléotides de la séquence d'intérêt et d'une amorce anti-sens comprenant de 5' en 3' au moins une séquence correspondant à un adaptateur et au moins 14 nucléotides de la séquence d'intérêt , une étape de mise en contact simultanée d'au moins deux briques moléculaires, différentes l'une de l'autre, en présence d'une seule enzyme de restriction, ladite seule enzyme de restriction étant une enzyme de restriction de type IIs, chaque brique moléculaire étant une molécule d'ADN linéaire double brin à extrémités non cohésives, et contenant :

(i) une séquence d'intérêt dépourvue de site de reconnaissance spécifique de la susdite enzyme de restriction de type IIs et comprenant au moins une unité, ladite unité étant une unité fonctionnelle ou une unité non fonctionnelle, ladite unité comprenant au moins un module, ledit module étant un module fonctionnel ou un module non fonctionnel,
(ii) deux adaptateurs d'ADN double brin, flanqués en amont et en aval de ladite séquence d'intérêt, chaque adaptateur d'ADN double brin consistant en une séquence d'au moins 12 nucléotides, laquelle contient:
un seul et unique site de reconnaissance de la susdite enzyme de restriction de type IIs,

- le site de reconnaissance de la susdite enzyme de restriction de type IIs de l'adaptateur en amont de ladite séquence d'intérêt et le site de reconnaissance de la susdite enzyme de restriction de type IIs de l'adaptateur en aval de ladite séquence d'intérêt étant convergents,
  laquelle étape conduit :

  - à l'élimination *par coupure* des sites de reconnaissance de l'enzyme de restriction de type IIs utilisée,
  - la formation d'une suture monobrin cohésive d'au moins 2 nucléotides à chacune des extrémités de ladite séquence d'intérêt,
    ladite suture monobrin cohésive d'au moins 2 nucléotides en amont d'une des au moins deux séquences d'intérêt étant capable de s'apparier à ladite suture monobrin cohésive d'au moins 2 nucléotides en aval d'une autre séquence d'intérêt,
  - à l'appariement par complémentarité nucléotidique des susdites sutures monobrins cohésives d'au moins 2 nucléotides et
  - au positionnement des séquences d'intérêt, de façon contigüe les unes aux autres, selon un ordre et un sens unique et déterminé,

b) une étape de ligation des susdites sutures monobrins cohésives d'au moins 2 nucléotides, pour obtenir un vecteur circulaire d'ADN double brin.

La PCR est une réaction en chaîne par polymérase et permet de reproduire l'ADN en quantité.

## MODES DE REALISATION PARTICULIERS DE L'INVENTION

Plusieurs enzymes de restriction peuvent être utilisées pour réaliser l'assemblage. Cela inclut des enzymes de restriction de type IIs comme BsaI, BpiI, BsmBI, Esp3I, BspMI. En fait n'importe quelle enzyme de type IIs qui possède un site de coupure éloigné de son site de reconnaissance et qui génère une séquence débordante de 2 ou 3 nucléotides ou plus peut être utilisée pour l'assemblage. Selon l'enzyme utilisée, les conditions d'incubation pour l'assemblage (tampon, température, temps, nature de l'ADN ligase) sont optimisables. Pour chaque enzyme utilisée dans l'assemblage, une collection de fragments d'ADN et de séquences d'intérêt dépourvus de sites de reconnaissance de cette enzyme est générée.

### 1. Préparation des briques d'ADN.

La préparation de briques d'ADN à partir d'une matrice est faite par la technique de Polymerase Chain Reaction (PCR), en utilisant des amorces oligonucléotidiques soigneusement choisies. La synthèse de gène (synthèse chimique) peut également être utilisée comme méthode alternative d'obtention de brique.

Par PCR, les amorces permettent l'amplification du fragment d'ADN par une polymérase à haute-fidélité, afin de limiter au maximum le nombre de mutations qui pourraient être introduites aléatoirement par l'ADN polymérase. D'autre part, ces amorces permettent d'introduire les adaptateurs contenant notamment le(s) site(s) de reconnaissance de l'enzyme de type IIs utilisée pour l'assemblage ainsi que les sutures qui permettront d'obtenir un assemblage orienté. Une brique contiendra donc une séquence d'intérêt flanquée de deux adaptateurs (en 5' et 3' de ladite séquence d'intérêt). Les sites de reconnaissance de l'enzyme de restriction de type IIs utilisée pour l'assemblage, potentiellement présents dans la séquence d'intérêt, sont indésirables et doivent au préalable être éliminés par mutagenèse dirigée ou par tout autre méthode appropriée.

### 1.1. Définition des amorces

Les amorces oligonucléotidiques utilisées pour la création de briques, contiennent 2 parties essentielles.

En partie 5' ces amorces contiennent la séquence d'un adaptateur. L'adaptateur contient une séquence comprenant :

- une séquence de 2 nucléotides minimum, pouvant contenir ou participer à 1 ou plusieurs sites de restriction d'endonucléase de type II,
- une séquence de 5, 6, 7 ou plus nucléotides correspondant au site de fixation de l'enzyme à activité de type IIs utilisée pour l'assemblage,
- une séquence de 1 à 8 et plus nucléotides correspondant à l'espacement entre le site de fixation et le site de coupure de l'enzyme utilisée. La longueur de cette séquence dépend des caractéristiques intrinsèques de l'enzyme de type IIs utilisée,
- une séquence de 2 à 5 ou plus, nucléotides correspondant à l'extrémité simple brin générée par l'action de l'enzyme

de type IIs utilisée. Cette séquence permettant l'appariement des fragments à assembler, elle correspond de facto à la définition de suture.

En partie 3', les amorces contiennent une séquence de 14 à 100 nucléotides correspondant aux extrémités 5' de la séquence d'intérêt, qui permettra l'hybridation des amorces à des zones complémentaires de la matrice et l'amplification de la séquence d'intérêt. Cette séquence est de taille variable mais supérieure ou égale à 14 nucléotides. De plus elle est choisie de manière à i) respecter des Tm (température de fusion) assez proches pour deux amorces conçues pour la fabrication d'une brique donnée et ii) dans la mesure du possible finir l'amorce sur au moins un G ou un C et pas plus de 2 G ou C consécutifs.

On entend par matrice une molécule d'ADN contenant la séquence d'intérêt à amplifier. Il peut s'agir par exemple d'ADN génomique, d'ADN complémentaire obtenu par transcription inverse d'un ARNm ou d'un plasmide.

Selon un autre mode de réalisation, des séquences supplémentaires peuvent être insérées entre la séquence de l'adaptateur et la séquence complémentaire de la matrice. Il peut s'agir par exemple d'une séquence codant des acides aminés supplémentaires qui seront fusionnés au produit protéique codé par la séquence d'intérêt.

**1.2. PCR (haute fidélité) avec protocole et contrôle des produits obtenus**

Chaque brique est amplifiée avec une ADN polymérase à haute-fidélité. La phusion taq DNA polymérase (Thermo Scientific), est utilisée selon le protocole du fournisseur, mais n'importe quelle DNA polymérase à haute-fidélité pourrait être utilisée. La quantité de matrice utilisée est réduite au minimum (10pg à 2ng/ μl, selon la brique).

Les produits de PCR sont purifiés à l'aide d'un kit (ex : Macherey Nagel PCR and gel cleanup kit ®)soit directement (PCR cleanup), soit en passant par une étape de dépôt sur un gel d'agarose en Tampon TAE (Tris 40mM pH8, Acétate 20mM, EDTA 1mM) où après migration, les morceaux de gel d'agarose contenant les produits de PCR sont découpés puis purifiés (gel cleanup) L'ADN est alors quantifié en utilisant un Nanospectrophotomètre (ex : nanodrop®)

**1.3.Stratégie d'élimination de sites d'enzymes de type IIs (exemple BsaI) potentiellement présents** :

Pour que l'assemblage des briques puisse être mis en œuvre, et que le procédé selon l'invention soit effectif, il ne faut pas qu'il y ait de site de l'enzyme de restriction de type IIs utilisée dans le procédé selon l'invention, (pour l'assemblage) à l'intérieur des séquences d'intérêt. Pour cela la technique de mutagenèse par Golden Gate a été utilisée telle qu'elle a été décrite par Engler et al., 2008 mais n'importe quelle technique de mutagenèse dirigée peut être employée. (Cormack, B. 2001. Directed Mutagenesis Using the Polymerase Chain Reaction. Current Protocols in Molecular Biology. 37:8.5:8.5.1-8.5.10.) Le cas échéant, les mutations nécessaires sont introduites de sorte à conserver la(les) fonction(s) biologique(s) de la séquence d'intérêt (ex : sites de fixation à l'ADN, structures secondaires, produit d'expression).

**1.4 Méthode de choix des sutures**

Grâce à l'utilisation des adaptateurs selon la divulgation, l'assemblage des briques moléculaires peut se faire à la base nucléotidique près. Ce niveau de précision permet d'éliminer toute cicatrice nucléotidique.

On entend par cicatrice, tout nucléotide ou groupe de nucléotides dont la présence dans le vecteur final serait rendue obligatoire de par l'utilisation de la technique d'assemblage sans toutefois assurer de fonction au sein du vecteur lui-même.

Le choix des sutures est crucial pour favoriser un assemblage correct. Il doit respecter plusieurs critères simples. Tout d'abord une suture ne doit pas être palindromique : en effet une suture palindromique peut se lier avec ou sur elle-même ce qui pourrait conduire à des difficultés d'assemblages, à la formation de dimères de briques liés tête bêche.

Selon l'invention, la suture ne doit pas être palindromique (ex : ACGT dont la séquence anti-parallèle ACGT est identique) pour éviter un auto-appariement correspondant à un assemblage de plusieurs exemplaires de la même brique, tête-bêche, pour former des chaînes.

On appelle une séquence palindromique une séquence qui se lit de la même manière de 5' vers 3' sur chacun des deux brins d'ADN.

Dans le procédé selon l'invention, il ne peut y avoir « d'auto-circularisation » puisque les sutures en 5' et 3' sont choisies pour ne pas être complémentaires entre elles. La séquence anti-parallèle résultant d'un clivage est l'extrémité non cohésive de l'adaptateur libéré.

Ensuite le choix d'une suture à une position de l'assemblage élimine la possibilité de l'utiliser à une autre position. Enfin le choix de sutures trop similaires (ne différant que d'un seul nucléotide) à deux positions dans un assemblage est évité car pourrait conduire à des assemblages et ligations illégitimes. En effet un appariement partiellement complémentaire (3/4 nucléotides seulement interagissant) ou 'mismatch' ou mésappariement peut être suffisant pour l'activité de certaines ADN ligases et donc conduire à l'apparition de constructions aberrantes.

Ce critère diminue la fréquence d'obtention de constructions aberrantes.

Afin de respecter ces consignes, un programme simple a été développé qui, en fonction des sutures déjà choisies, élimine des sutures jugées incompatibles. Le programme est basé sur l'utilisation d'une matrice de combinaisons de sutures : pour chacune des 240 combinaisons non palindromiques possibles, un score de compatibilité est calculé avec chacune des 239 autres. Le mode de calcul de ce score est indiqué dans le schéma (figure. 2) :
Par conséquent chaque appariement de deux séquences de 4 nucléotides non palindromiques (soit 57 600 combinaisons) se voit attribué un score allant de 0 à 10 où 0 correspond à une absence totale de complémentarité (0%) et 10 indique une complémentarité totale (100%). Complémentarité signifie complémentarités inter-sutures. Ces scores sont intégrés à une matrice illustrée (figure. 3). Pour un vecteur donné, l'ensemble des sutures nécessaire est choisi de sorte à ce que chaque suture ait un score de complémentarité minimale avec chacune des autres sutures nécessaires à l'assemblage.

## 2. Protocole général d'assemblage

Afin d'assembler les briques entre elles pour obtenir le vecteur désiré, il faut les mélanger à des ratios équimolaires en présence d'une seule enzyme de restriction de type IIs et d'une ligase.

Avantageusement, les briques sont mélangées à des ratios équimolaires en présence d'une seule enzyme de restriction de type IIs et d'une ligase, dans un tampon approprié.

La quantité en ng de chaque brique moléculaire est calculée comme suit : $Q_{(ng)} = taille_{(pb)} \times 649_{(ng.nmol-1.pb-1)} \times (20 \text{ à } 100).10\text{-}6_{(nmol)}$ et le volume est calculé comme suit: $V_{(\mu l)} = Q_{(ng)} / concentration_{(ng.\mu l\text{-}1).}$

Le mélange s'effectue à une température allant de 0 à 6°C, de préférence à 4°C.

Le mélange est ensuite soumis à une température de 37°C pendant une période allant de 30' à 6 heures, pour un nombre de briques moléculaires à assembler inférieur à 5,

Le mélange est soumis à des cycles d'incubation de 2' à 37°C puis 3' à 16°C (25 à 50 cycles) si le nombre de briques moléculaires est supérieur à 4.

Le mélange est ensuite incubé à 50°C pendant 5' (coupure des sites d'endonucléase de type IIs subsistants (non coupés) puis à 80°C pendant 5' (inactivation des enzymes).

Le mélange réactionnel est ensuite utilisé pour transformer des bactéries compétentes qui sont ensuite sélectionnées.

## 3. Vérifications des constructions

Deux niveaux de vérifications peuvent être considérés :

1)- la vérification de la molécule produite en termes de séquence

2)- la vérification du vecteur en termes de fonctionnalité

1)- Parallèlement à l'assemblage *in vitro,* un assemblage virtuel des fragments est effectué informatiquement pour reconstituer la séquence du vecteur souhaité. Ceci permet l'établissement d'une carte de restriction du vecteur. Afin de vérifier les clones obtenus, les mini préparations d'ADN circulaire sont digérées par une ou plusieurs enzymes choisies de sorte à générer au moins trois fragments de taille distincte. De préférence le choix des enzymes se fait de sorte qu'il y ait au moins un site présent dans chacun des fragments assemblés. L'analyse du profil de restriction après électrophorèse en gel d'agarose permet de vérifier que les fragments ont tous été assemblés dans l'ordre souhaité.

D'autres procédés de vérification des clones obtenus peuvent être envisagés, notamment une vérification par PCR pour vérifier, par des mesures de taille de fragments, les constructions. De plus l'intégralité du vecteur ou une partie de celui-ci peut être séquencée (Prober JM, Trainor GL, Dam RJ, Hobbs FW, Robertson CW, Zagursky RJ et al. (16 Oct 1987). "A system for rapid DNA sequencing with fluorescent chain-terminating dideoxynucleotides". Science 238 (4825): 336-4.).

2)- Selon les séquences d'inétrêt intégrées dans les vecteurs selon la divulgation, il est possible également de mesurer et ou quantifier directement ou indirectement la fonctionnalité des unités et/ou modules composant le vecteur. Par exemple, l'extraction d'un vecteur à partir d'une culture de bactéries transformées réalisée en présence d'un antibiotique approprié (ex : kanamycine, ampicilline, chloramphénicol) permet de valider la fonctionnalité de modules composant l'unité bactérienne. De même, l'obtention de clones eucaryotes issus d'une sélection effectuée par une drogue appropriée (ex : G418, hygromycine, puromycine) permet de valider la fonctionnalité de modules composant une unité d'intégration en cellule eucaryote. Enfin, la présence d'un produit d'expression (ARNm, micro-ARN, long ARN non codant) peut être détectée à partir des ARN totaux extraits de la cellule cible ayant reçu le vecteur, par PCR ou par toute méthode permettant de mesurer la présence d'ARN dans une cellule comme le RNA-Fish (Langer-Safer PR, Levine M, Ward DC (July 1982). "Immunological method for mapping genes on Drosophila polytene chromosomes". Proc. Natl. Acad. Sci. U.S.A. 79 (14): 4381-5. doi:10.1073/pnas.79.14.4381) ou le SmartFlare (Prigodich, A. E.; Randeria, P. S.; Briley, W. ; Kim, N.; Daniel, W. L.; Giljohann, D. A.; Mirkin, C. A. "Multiplexed Nanoflares: mRNA Detection in Live Cells," Anal. Chem. 2012, 84, 2062-2066, doi: 10.1021/ac202648w). Le cas échéant l'expression d'un ARN interférant peut être détectée en mesurant la diminution de l'expression (ARNm) du gène endogène ciblé par ledit ARN interférant.

Selon un aspect avantageux, la divulgation porte sur un vecteur sélectionné parmi un vecteur V1, V1.1, V1.2, V1.3, V2 et V3.

Selon un aspect avantageux, la divulgation porte sur un vecteur sélectionné parmi un vecteur V1, V1.1, V1.2, V1.3, V2, V3, V2b, V3b, V1.1b, V4.

Avantageusement, la divulgation porte sur un vecteur sans site de clonage multiple et sans cicatrice, et encore plus avantageusement un vecteur sans cicatrice et sans site de restriction de l'enzyme de type IIs ayant servi à son assemblage.

Selon un aspect avantageux, la divulgation porte sur un groupe de vecteurs V1, en particulier un groupe de vecteurs V1 obtenu selon le procédé de l'invention.

Selon un aspect avantageux, la divulgation porte sur un groupe de vecteurs V1.1, en particulier un groupe de vecteurs V1.1 obtenu selon le procédé de l'invention.

Selon un aspect avantageux, la divulgation porte sur un groupe de vecteurs V1.2, en particulier un groupe de vecteurs V1.2 obtenu selon le procédé de l'invention.

Selon un aspect avantageux, la divulgation porte sur un groupe de vecteurs V1.3, en particulier un groupe de vecteurs V1.3 obtenu selon le procédé de l'invention.

Selon un aspect avantageux, la divulgation porte sur un groupe de vecteurs V2, en particulier un groupe de vecteurs V2 obtenu selon le procédé de l'invention.

Selon un aspect avantageux, la divulgation porte sur un groupe de vecteurs V3, en particulier un groupe de vecteurs V3 obtenu selon le procédé de l'invention.

Selon un aspect avantageux, la divulgation porte sur un groupe de vecteurs V4, en particulier un groupe de vecteurs V4 obtenu selon le procédé de l'invention.

Un vecteur V1 (SEQ ID NO: 41) est construit à partir d'une combinaison des briques Ori AmpR BsaI B (SEQ ID NO : 36), pCMV BsaI B (SEQ ID NO : 37), hFUT3 BsaI A (SEQ ID NO : 38), BGHpA BsaI B (SEQ ID NO : 39), et shB3Galt6 BsaI A (SEQ ID NO : 40).

Un vecteur V1.1 (SEQ ID NO : 48) est construit à partir d'une combinaison des briques Ori-AmpR BsaI B (SEQ ID NO :36), pCMV BsaI B (SEQ ID NO : 37), hFUT3 BsaI A (SEQ ID NO : 38), BGHpA BsaI B (SEQ ID NO : 39), shB3GALT6 BsaI B (SEQ ID NO : 46), etHygroR BsaI B (SEQ ID NO : 47).

Un vecteur V1.2 (SEQ ID NO : 53) est construit à partir d'une combinaison des briques Ori-AmpR BsaI B (SEQ ID NO : 36), rosa26-5' BsaI A (SEQ ID NO : 49), pCMV BsaI C (SEQ ID NO : 50), hFUT3 BsaI A (SEQ ID NO : 38), BGHpA BsaI B (SEQ ID NO : 39), shB3GALT6 BsaI B (SEQ ID NO : 46), HygroR BsaI C (SEQ ID NO : 51), et rosa26-3' BsaI A (SEQ ID NO : 52).

Un vecteur V1.3 (SEQ ID NO : 58) est construit à partir d'une combinaison des briques Ori-AmpR BsaI B (SEQ ID NO : 36), rosa26-5' BsaI A (SEQ ID NO : 49), pCMV BsaI C (SEQ ID NO : 50), hFUT3 BsaI A (SEQ ID NO : 38), BGHpA BsaI B (SEQ ID NO : 39), shB3GALT6 BsaI B (SEQ ID NO : 46), HygroR BsaI C (SEQ ID NO : 51), rosa26-3' BsaI B (SEQ ID NO : 54), pEF1a BsaI A (SEQ ID NO : 55), TK BsaI A (SEQ ID NO : 56), et Tkter BsaI A (SEQ ID NO : 57).

Un vecteur V2 (SEQ ID NO : 62) est construit à partir d'une combinaison des briques Ori-AmpR BsaI B (SEQ ID NO : 36), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B (SEQ ID NO : 63), et Tkter BsaI A (SEQ ID NO : 57).

Un vecteur V3 (SEQ ID NO: 66) est construit à partir d'une combinaison des briques Ori-AmpR BsaI B (SEQ ID NO : 36), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mb3GALT6 BsaI B (SEQ ID NO :63), Tkter BsaI B (SEQ ID NO :64), et shB3Galt6 BsaI C (SEQ ID NO :65).

Un vecteur V2b (SEQ ID NO : 149) est construit à partir d'une combinaison des briques Ori BsaI A (SEQ ID NO : 104), AmpR BsaI A (SEQ ID NO : 105), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B (SEQ ID NO : 63), et Tkter Bsa IA (SEQ ID NO : 57).

Un vecteur V3b (SEQ ID NO : 150) est construit à partir d'une combinaison des briques Ori BsaI A (SEQ ID NO : 104), AmpR BsaI A (SEQ ID NO : 105), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mb3GALT6 BsaI B (SEQ ID NO :63), Tkter BsaI B (SEQ ID NO : 64), et shB3Galt6 BsaI C (SEQ ID NO : 65).

Un vecteur V1.1b (SEQ ID NO : 151) est construit à partir d'une combinaison des briques Ori BsaI B (SEQ ID NO : 106), AmpR BsaI B (SEQ ID NO : 107), pEF1aL BsaI B (SEQ ID NO : 108), EGFP-CAAX BsaI A (SEQ ID NO :

109), BGHpA BsaI C (SEQ ID NO : 110), pCMV BsaI D (SEQ ID NO : 111), SiaT BsaI B (SEQ ID NO : 112), mCherry BsaI B (SEQ ID NO : 113), TKter BsaI B (SEQ ID NO : 64) et HygroR BsaI D (SEQ ID NO : 114).

Un vecteur V4 (SEQ ID NO : 152) est construit à partir d'une combinaison des briques Ori-2 BsaI C (SEQ ID NO : 115), AmpR BsaI C (SEQ ID NO : 116), MNN10-Lrec BsaI A (SEQ ID NO : 117), KanMX BsaI A (SEQ ID NO : 119), MNN10-Rrec BsaI A (SEQ ID NO : 118).

Un vecteur est un vecteur ayant une séquence choisie parmi les séquences SEQ ID NO : 41, 48, 53, 58, 62 et 66.

Un vecteur est un vecteur ayant une séquence choisie parmi les séquences SEQ ID NO : 149, 150, 151 et 152.

Un vecteur est un vecteur ayant une séquence choisie parmi les séquences SEQ ID NO : 41, 48, 53, 58, 62, 66, 149, 150, 151 et 152.

Un autre vecteur est un vecteur ayant une séquence choisie parmi les séquences SEQ ID NO : 30, 31, 32, 33, 34 et 35.

Les vecteur obtenus selon le procédé de l'invention sont les vecteurs de séquence SEQ ID NO : 30 31, 32, 33, 34 et 35.

Les vecteurs obtenus selon le procédé de l'invention sont les vecteurs de séquence SEQ ID NO : 30, 31, 32, 33, 34, 35, 41, 48, 53, 58, 62, 66, 149, 150, 151 et 152.

Selon un autre mode de réalisation, la divulgation a pour objet un vecteur circulaire d'ADN double brin tel qu'obtenu par mise en œuvre selon le procédé de l'invention.

La divulgation a aussi pour objet un vecteur d'ADN double brin, dépourvu de site de clonage multiple et permettant l'expression simultanée de multiples transgènes et consistant en une séquence comprenant les unités fonctionnelles suivantes : U1, nxU2a et mxU2b,
U1 représentant une unité fonctionnelle bactérienne,
U2a représentant une unité fonctionnelle d'expression dont le promoteur est dépendant de l'ARN polymerase II et dont le produit d'expression est une protéine,
U2b représentant une unité fonctionnelle d'expression dont le promoteur est dépendant de l'ARN polymerase III et dont le produit d'expression est un ARN non codant,
n étant supérieur ou égal à 0,
m étant supérieur ou égal à 0, sous réserve que n+m≥2,
l'unité U1 pouvant être contiguë aux unités nxU2a, elles-mêmes pouvant être contiguës aux unités mxU2b;
l'unité U1 étant de préférence contiguë aux unités nxU2a, elles-mêmes étant de préférence contiguës aux unités mxU2b.
Ces vecteurs constituent un groupe de vecteurs V1.
Ce groupe de vecteurs V1 peut etre préparé selon le procédé de l'invention.

Selon encore un autre aspect, la divulgation a pour objet un groupe de vecteurs V1 tels qu'obtenus par mise en oeuvre du procédé de l'invention.

la divulgation a également pour objet un vecteur d'ADN double brin, dépourvu de site de clonage multiple et permettant de sélectionner l'intégration des transgènes par recombinaison non homologue dans le génome cible et consistant en une séquence comprenant les unités fonctionnelles suivantes : U1, nxU2a, mxU2b et U3a,
U1 représentant une unité fonctionnelle bactérienne,
U2a représentant une unité fonctionnelle d'expression dont le promoteur est dépendant de l'ARN polymerase II et dont le produit d'expression est une protéine,
U2b représentant une unité fonctionnelle d'expression dont le promoteur est dépendant de l'ARN polymerase III et dont le produit d'expression est un ARN non codant,
n étant supérieur ou égal à 0,
m étant supérieur ou égal à 0, sous réserve que n+m≥2,
U3a représentant une cassette de sélection positive,
l'unité U1 pouvant être contiguë aux unités nxU2a, elles-mêmes pouvant être contiguës aux unités mxU2b, elles-mêmes pouvant être contiguës à U3a;
l'unité U1 étant de préférence contiguë aux unités nxU2a, elles-mêmes étant de préférence contiguës aux unités mxU2b, elles-mêmes étant de préférence contiguës à U3a.

Ces vecteurs constituent un groupe de vecteurs V1.1.

Ce groupe de vecteurs V1.1 peut etre préparé selon le procédé de l'invention.

Selon encore un autre aspect, la divulgation a pour objet un groupe de vecteurs V1.1 tels qu'obtenus par mise en oeuvre du procédé de l'invention.

la divulgation a également pour objet un vecteur d'ADN double brin, dépourvu de site de clonage multiple et permettant de sélectionner l'intégration simultanée de multiples transgènes par recombinaison homologue dans le génome cible et consistant en une séquence comprenant les unités fonctionnelles suivantes : U1, U3b, nxU2a, mxU2b, U3a et U3c,
U1 représentant une unité fonctionnelle bactérienne,
U3b représentant un motif 5' d'une séquence de recombinaison homologue X

U2a représentant une unité fonctionnelle d'expression dont le promoteur est dépendant de l'ARN polymerase II et dont le produit d'expression est une protéine,
U2b représentant une unité fonctionnelle d'expression dont le promoteur est dépendant de l'ARN polymerase III et dont le produit d'expression est un ARN non codant,
n étant supérieur ou égal à 0,
m étant supérieur ou égal à 0, sous réserve que n+m≥2,
U3a représentant une cassette de sélection positive,
U3c représentant motif 3' d'une séquence de recombinaison homologue X,
l'unité U1 étant contiguë à U3b, elle-même étant contiguë aux unités nxU2a, elles-mêmes étant contiguës aux unités mxU2b, elles-mêmes étant contiguës à l'unité U3a, elle-même étant contiguë à l'unité U3c.

Ces vecteurs constituent un groupe de vecteurs V1.2.

Ce groupe de vecteurs V1.2 peut etre préparé selon le procédé de l'invention.

Selon encore un autre aspect, la divulgation a pour objet un groupe de vecteurs V1.2 tels qu'obtenus par mise en oeuvre du procédé de l'invention.

la divulgation a également pour objet un vecteur d'ADN double brin, dépourvu de site de clonage multiple et permettant d'éliminer les cellules hôtes ayant intégré un ou des transgènes par recombinaison non-homologue et consistant en une séquence comprenant les unités fonctionnelles suivantes : U1, U3b, nxU2a, mxU2b, U3a, U3c et U3d,
U1 représentant une unité fonctionnelle bactérienne,
U3b représentant motif 5' d'une séquence de recombinaison homologue X
U2a représentant une unité fonctionnelle d'expression dont le promoteur est dépendant de l'ARN polymerase II et dont le produit d'expression est une protéine,
U2b représentant une unité fonctionnelle d'expression dont le promoteur est dépendant de l'ARN polymerase III et dont le produit d'expression est un ARN non codant,
n étant supérieur ou égal à 0,
m étant supérieur ou égal à 0, sous réserve que n+m≥2,
U3a représentant une cassette de sélection positive,
U3c représentant un motif 3' d'une séquence de recombinaison homologue X,
U3d représentant une cassette de sélection négative, l'unité U1 étant contiguë à l'unité U3b, elle-même étant contiguë aux unités nxU2a, elles-mêmes étant contiguës aux unités mxU2b, elles-mêmes étant contiguës à l'unité U3a, elle-même étant contiguë à l'unité U3c, elle-même étant contiguë à l'unité U3d.

Ces vecteurs constituent un groupe de vecteurs V1.3.

Ce groupe de vecteurs V1.3 peut etre préparé selon le procédé de l'invention.

Selon encore un autre aspect, la divulgation a pour objet un groupe de vecteurs V1.3 tels qu'obtenus par mise en oeuvre du procédé de l'invention.

la divulgation a également pour objet un vecteur d'ADN double brin, dépourvu de site de clonage multiple et permettant d'exprimer un ou plusieurs transgènes de façon inductible et consistant en une séquence comprenant les unités fonctionnelles suivantes : U1, U2c, nxU2d, et mxU2e,
U1 représentant une unité fonctionnelle bactérienne,
U2c représentant un gène codant un transactivateur transcriptionel,
U2d représentant un gène dont le promoteur est dépendant du transactivateur codé par le gène U2c,
U2e représentant un gène dont le promoteur n'est pas dépendant du transactivateur codé par le gène U2c,
n étant supérieur ou égal à 1,
m étant supérieur ou égal à 0,
l'unité U1 pouvant être contiguë à l'unité U2c, elle-même pouvant être contiguë aux unités nxU2d, elles-mêmes pouvant être contiguës aux unités mxU2e;
l'unité U1 étant de préférence contiguë à l'unité U2c, elle-même étant de préférence contiguë aux unités nxU2d, elles-mêmes étant de préférence contiguës aux unités mxU2e.

Ces vecteurs constituent un groupe de vecteurs V2.

Ce groupe de vecteurs V2 peut etre préparé selon le procédé de l'invention.

Selon encore un autre aspect, la divulgation a pour objet un groupe de vecteurs V2 tels qu'obtenus par mise en oeuvre du procédé de l'invention.

la divulgation a également pour objet un vecteur d'ADN double brin, dépourvu de site de clonage multiple et permettant de réaliser de la complémentation génétique sous contrôle inductible et consistant en une séquence comprenant les unités fonctionnelles suivantes : U1, U2f, U2c, et U2g,
U1 représentant une unité fonctionnelle bactérienne,
U2c représentant un gène codant un transactivateur transcriptionel,
U2f représentant un gène dont le promoteur est un promoteur à ARN polymérase III et dont le produit d'expression est un petit ARN en épingle à cheveux (shRNA) précurseur d'un petit ARN interférant ciblant un gène X,

U2g représentant un gène dont le promoteur est dépendant du transactivateur codé par le gène
U2c et dont le produit d'expression est une version mutée du produit du gène X de sorte à être insensible au produit du gène U2f ;
l'unité U1 pouvant être contiguë à l'unité U2f, elle-même pouvant être contiguë à l'unité U2c, elle-même pouvant être contiguë à l'unité U2g;
l'unité U1 étant de préférence contiguë à l'unité U2f, elle-même étant de préférence contiguë à l'unité U2c, elle-même étant de préférence contiguë à l'unité U2g.

Ces vecteurs constituent un groupe de vecteurs V3.

Ce groupe de vecteurs V3 peut etre préparé selon le procédé de l'invention.

Selon encore un autre aspect, la divulgation a pour objet un groupe de vecteurs V3 tels qu'obtenus par mise en oeuvre du procédé de l'invention.

la divulgation a également pour objet un vecteur d'ADN double brin, dépourvu de site de clonage multiple et permettant de sélectionner des cellules dont le génome a été édité par recombinaison homologue ciblée et consistant en une séquence comprenant les unités fonctionnelles suivantes :

U1, U3b, U3a, et U3c,
U1 représentant une unité fonctionnelle bactérienne,
U3a représentant une cassette de sélection positive,
U3b représentant un motif 5' d'une séquence de recombinaison homologue X
U3c représentant un motif 3' d'une séquence de recombinaison homologue X,
l'unité U1 étant contiguë à l'unité U3b, elle-même étant contiguë à l'unité U3a, elle-même étant contiguë à l'unité U3c.

Ces vecteurs constituent un groupe de vecteurs V4.

Ce groupe de vecteurs V4 peut être préparé selon le procédé de l'invention.

Selon encore un autre aspect, la divulgation a pour objet un groupe de vecteurs V4 tels qu'obtenus par mise en œuvre du procédé de l'invention.

**LEGENDES DES FIGURES**

**Figure 1**

A : Schéma représentant une brique moléculaire sur laquelle une enzyme de type IIs (rond) liée à un adaptateur (A) de part et d'autre d'une séquence d'intérêt (SI) est liée à l'ADN au niveau de son site de reconnaissance et coupe l'ADN à distance du site de reconnaissance (flèche) pour générer une SI ayant deux extrémités simple brin d'au moins 2 nucléotides (sutures) qui permettront un assemblage ordonné.
B. Exemple de coupure induite par une enzyme de type IIs produisant une extrémité monobrin pouvant s'apparier avec une autre extrémité monobrin et s'assembler sans cicatrice.

**Figure 2 :** Schéma représentant le mode de calcul de score de compatibilité de choix des sutures (choix intersutures).

**Figure 3 :** Matrice des scores obtenus pour l'ensemble des combinaisons possibles

**Figure 4** : Schéma de représentation des plasmides réalisés (réalisation 1) et des sutures d'assemblage des briques

**Figure 5** : Carte de restriction du plasmide pHCsiaT-EGFP de la réalisation 1 et vérification de l'assemblage

**Figure 6** : Schéma du vecteur V1 (SEQ ID NO : 41) et des sutures permettant l'assemblage des brique Ori-AmpR BsaI B (SEQ ID NO : 36), pCMV BsaI B (SEQ ID NO : 37), hFUT3 BsaI A (SEQ ID NO : 38), BGHpA BsaI B (SEQ ID NO : 39), et shB3Galt6 BsaI A (SEQ ID NO : 40). Où 1= Ori-AmpR BsaI B (SEQ ID NO : 36), 2 = pCMV BsaI B (SEQ ID NO : 37), 3= hFUT3 BsaI A (SEQ ID NO : 38), 4= BGHpA BsaI B (SEQ ID NO : 39) et 5 = shB3Galt6 BsaI A (SEQ ID NO : 40).

**Figure 7** : Empreinte de restriction par triple digestion EcoRV/PvuI/SalI du vecteur V1. La digestion produit 5 fragments de 1705, 992, 602, 561 et 357 paires de bases respectivement.

1 : 1 705 pb, de SalI [3821] à PvuI [1305]

2 : 992 pb, de EcoRV [2829] à SalI [3821]
3 : 606 pb, de SalI [1866] à SalI [2472]
4 : 561 pb, de PvuI [1305] à SalI [1866]
5 : 357 pb, de SalI [2472] à EcoRV [2829]

**Figure 8 :** Schéma du vecteur V1.1 (SEQ ID NO : 48) et des sutures permettant l'assemblage des briques Ori-AmpR BsaI B (SEQ ID NO :36), pCMV BsaI B (SEQ ID NO : 37), hFUT3 BsaI A (SEQ ID NO : 38), BGHpA BsaI B (SEQ ID NO : 39), shB3Galt6 BsaI B (SEQ ID NO : 46), et HygroR BsaI B (SEQ ID NO : 47).
Où 1= Ori-AmpR BsaI B (SEQ ID NO : 36), 2 = pCMV BsaI B (SEQ ID NO : 37), 3= hFUT3 BsaI A (SEQ ID NO : 38), 4= BGHpA BsaI B (SEQ ID NO : 39), 5 = shB3Galt6 BsaI B (SEQ ID NO : 46) et 6= HygroR BsaI B (SEQ ID NO : 47)

**Figure 9 :** Empreinte de restriction par triple digestion EcoRV/PvuI/SalI du vecteur V1.1.
La digestion produit 6 fragments de 2217, 1104, 992, 606, 561 et 357 paires de bases respectivement.

1: 2 217 pb, de PvuI [4925] à PvuI [1305]
2 : 1104 pb, de SalI [3821] à PvuI [4925]
3 : 992 pb, de EcoRV [2829] à SalI [3821]
4 : 606 pb, de SalI [1866] à SalI [2472]
5 : 561 pb, de PvuI [1305] à SalI [1866]
6 : 357 pb, de SalI [2472] à EcoRV [2829]

**Figure 10** : Vérification fonctionnelle du vecteur V1.1 (SEQ ID NO : 48): profil d'expression de hFUT3 et mB3GALT6 mesurés par qRT-PCR à partir d'ARN totaux extraits d'un clone de cellules de souris 4T1 stablement transfectées par le vecteur V1.1.

**Figure 11** : Schéma du vecteur V1.2 (SEQ ID NO : 53) et des sutures permettant l'assemblage des briques Ori-AmpR BsaI B (SEQ ID NO : 36), rosa26-5' BsaI A (SEQ ID NO : 49), pCMV BsaI C (SEQ ID NO : 50), hFUT3 BsaI A (SEQ ID NO : 38), BGHpA BsaI B (SEQ ID NO : 39), shB3Galt6 BsaI B (SEQ ID NO : 46), Hygro BsaI C (SEQ ID NO : 51), et rosa26-3' BsaI A (SEQ ID NO : 52).
Où 1= Ori-AmpR BsaI B (SEQ ID NO : 36), 2 = rosa26-5' BsaI A (SEQ ID NO : 49), 3 = pCMV BsaI C (SEQ ID NO : 50), 4 = hFUT3 BsaI A (SEQ ID NO : 38), 5 = BGHpA BsaI B (SEQ ID NO : 39), 6 = shB3Galt6 BsaI B (SEQ ID NO : 46), 7 = HygroR BsaI C (SEQ ID NO : 51) et 8 = rosa26-3' BsaI A (SEQ ID NO : 52).

**Figure 12** : Empreinte de restriction par triple digestion EcoRV/PvuI/SalI du vecteur V1.2.
La digestion produit 6 fragments de 6492, 1649, 1104, 992, 606 et 357 paires de bases respectivement.

1 6 492 pb, de PvuI [6009] à PvuI [1301]
2 : 1 649 pb, de PvuI [1301] à SalI [2950]
3 : 1104 pb, de SalI [4905] à PvuI [6009]
4 : 992 pb, de EcoRV [3913] à SalI [4905]
5 : 606 pb, de SalI [2950] à SalI [3556]
6 : 357 pb, de SalI [3556] à EcoRV [3913]

**Figure 13** : Schéma du vecteur V1.3 (SEQ ID NO: 58) et des sutures permettant l'assemblage des briques Ori-AmpR BsaI B (SEQ ID NO : 36), rosa26-5' BsaI A (SEQ ID NO : 49), pCMV BsaI C (SEQ ID NO : 50), hFUT3 BsaI A (SEQ ID NO : 38), BGHpA BsaI B (SEQ ID NO : 39), shB3Galt6 BsaI B (SEQ ID NO : 46), HygroR BsaI C (SEQ ID NO : 51), rosa26-3' BsaI B (SEQ ID NO : 54), pEF1a BsaI A (SEQ ID NO : 55), TK BsaI A (SEQ ID NO : 56), et Tkter BsaI A (SEQ ID NO : 57).
Où 1= Ori-AmpR BsaI B (SEQ ID NO : 36), 2 = rosa26-5' BsaI A (SEQ ID NO : 49), 3 = pCMV BsaI C (SEQ ID NO : 50), 4 = hFUT3 BsaI A (SEQ ID NO : 38), 5 = BGHpA BsaI B (SEQ ID NO : 39), 6 = shB3Galt6 BsaI B (SEQ ID NO : 46), 7 = HygroR BsaI C (SEQ ID NO : 51), 8 = rosa26-3' BsaI B (SEQ ID NO : 54), 9 = pEF1a Bsa I A (SEQ ID NO : 55), 10 = TK BsaI A (SEQ ID NO : 56) et 11 = Tkter BsaI A (SEQ ID NO : 57).

**Figure 14** : Empreinte de restriction par triple digestion EcoRV/PvuI/SalI du vecteur V1.3.
La digestion produit 10 fragments de 5206, 2379, 1649, 1104, 992, 606, 357, 302, 252 et 104 paires de bases respectivement.

1 5 206 pb, de PvuI [6009] à SalI [11215]
2 : 2 379 pb, de EcoRV [11873] à PvuI [1301]
3 : 1 649 pb, de PvuI [1301] à SalI [2950]
4 : 1104 pb, de SalI [4905] à PvuI [6009]
5 : 992 pb, de EcoRV [3913] à SalI [4905]
6 : 606 pb, de SalI [2950] à SalI [3556]
7 : 357 pb, de SalI [3556] à EcoRV [3913]
8 : 302 pb, de SalI [11215] à SalI [11517]
9 : 252 pb, de SalI [11517] à EcoRV [11769]
10 : 104 pb, de EcoRV [11769] à EcoRV [11873]

**Figure 15 :** Schéma du vecteur V2 (SEQ ID NO : 62) et des sutures permettant l'assemblage des briques Ori-AmpR BsaI B (SEQ ID NO : 36), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B (SEQ ID NO : 63), et Tkter BsaI A (SEQ ID NO : 57). Où 1= Ori-AmpR BsaI B (SEQ ID NO : 36), 2 = pCMV BsaI B (SEQ ID NO : 37), 3= TO3G BsaI A (SEQ ID NO : 59), 4= BGHpA BsaI B (SEQ ID NO : 39), 5 = pTRE3G BsaI A (SEQ ID NO : 60), 6 = mB3Galt6 BsaI B (SEQ ID NO : 63) et 7 = Tkter BsaI A (SEQ ID NO : 57).

**Figure 16** : Empreinte de restriction par triple digestion NdeI/SalI/XhoI du vecteur V2.
La digestion produit 6 fragments de 2334, 1132, 602, 472, 361 et 245 paires de bases respectivement.

1 : 2 334 pb, de XhoI [4678] à SalI [1866]
2 : 1 132 pb, de NdeI [3074] à XhoI [4206]
3 : 602 pb, de SalI [2472] à NdeI [3074]
4 : 472 pb, de XhoI [4206] à XhoI [4678]
5 : 361 pb, de NdeI [2111] à SalI [2472]
6 : 245 pb, de SalI [1866] à NdeI [2111]

**Figure 17 :** Schéma du vecteur V3 (SEQ ID NO : 66) et des sutures permettant l'assemblage des briques Ori-AmpR BsaI B (SEQ ID NO : 36), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B (SEQ ID NO :63), Tkter BsaI B (SEQ ID NO :64), et shB3Galt6 BsaI C (SEQ ID NO :65).
Où 1= Ori-AmpR BsaI B (SEQ ID NO : 36), 2 = pCMV BsaI B (SEQ ID NO : 37), 3= TO3G BsaI A (SEQ ID NO : 59), 4= BGHpA BsaI B (SEQ ID NO : 39), 5 = pTRE3G BsaI A (SEQ ID NO : 60), 6 = mB3Galt6 BsaI B (SEQ ID NO : 63), 7 = Tkter BsaI B (SEQ ID NO : 64) et 8 = shB3Galt6 BsaI C (SEQ ID NO :65).

**Figure 18 :** Empreinte de restriction par triple digestion NdeI/SalI/XhoI du vecteur V3.
La digestion produit 8 fragments de 2110, 1107, 602, 478, 472, 361, 245 et 146 paires de bases respectivement.

1 : 2 110 pb, de NdeI [5308] à SalI [1862]
2 : 1 132 pb, de NdeI [3070] à XhoI [4202]
3 : 602 pb, de SalI [2468] à NdeI [3070]
4 : 478 pb, de XhoI [4674] à SalI [5152]
5 : 472 pb, de XhoI [4202] à XhoI [4674]
6 : 361 pb, de NdeI [2107] à SalI [2468]
7 : 245 pb, de SalI [1862] à NdeI [2107]
8 : 156 pb, de SalI [5152] à NdeI [5308]

**Figure 19:** Schéma du vecteur V2b (SEQ ID NO: 149) et des sutures permettant l'assemblage des briques Ori BsaI A (SEQ ID NO : 104), AmpR BsaI A (SEQ ID NO : 105), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mB3Galt6 BsaI B (SEQ ID NO : 63), et Tkter BsaI A (SEQ ID NO : 57)

**Figure 20 :** Empreinte de restriction par triple digestion NdeI/SalI/XhoI du vecteur V2b.
La digestion produit 6 fragments de 2334, 1132, 602, 472, 361 et 245 paires de bases respectivement.

1 : 2 334 pb, de XhoI [4678] à SalI [1866]
2 : 1 132 pb, de NdeI [3074] à XhoI [4206]

3 : 602 pb, de SalI [2472] à NdeI [3074]
4 : 472 pb, de XhoI [4206] à XhoI [4678]
5 : 361 pb, de NdeI [2111] à SalI [2472]
6 : 245 pb, de SalI [1866] à NdeI [2111]

**Figure 21 :** Expression relative de mB3GALT6 mesurée par PCR quantitative après transfert du vecteur V2b en cellule 4T1 par électroporation. 1 : cellules électroporées en absence de vecteur. 2: cellules électroporées en présence de V2.b non traitées à la doxocycline. 3 : cellules électroporées, puis traitées 24 heures à la doxocycline. L'expérience montre que l'expression du transcrit mB3Galt6, codé par le vecteur V2b, est augmentée en cellule 4T1 uniquement en présence de doxocycline, démontrant la présence concomitante d'un transgène inductible et de son co-activateur dans le vecteur V2b.

**Figure 22 ;** Schéma du vecteur V3b (SEQ ID NO: 150) et des sutures permettant l'assemblage des briques Ori BsaI A (SEQ ID NO : 104), AmpR BsaI A (SEQ ID NO : 105), pCMV BsaI B (SEQ ID NO : 37), TO3G BsaI A (SEQ ID NO : 59), BGHpA BsaI B (SEQ ID NO : 39), pTRE3G BsaI A (SEQ ID NO : 60), mb3Galt6 BsaI B (SEQ ID NO :63), Tkter BsaI B (SEQ ID NO : 64), et shB3Galt6 BsaI C (SEQ ID NO : 65).

**Figure 23 :** Empreinte de restriction par triple digestion NdeI/SalI/XhoI du vecteur V3b.
La digestion produit 8 fragments de 2110, 1132, 602, 478, 472, 361, 245 et 156 paires de bases respectivement.

1 : 2 110 pb, de NdeI [5312] à SalI [1866]
2 : 1132 pb, de NdeI [3074] à XhoI [4206]
3 : 602 pb, de SalI [2472] à NdeI [3074]
4 : 478 pb, de XhoI [4678] à SalI [5156]
5 : 472 pb, de XhoI [4206] à XhoI [4678]
6 : 361 pb, de NdeI [2111] à SalI [2472]
7 : 245 pb, de SalI [1866] à NdeI [2111]
8 : 156 pb, de SalI [5156] à NdeI [5312]

**Figure 24 :** Schéma du vecteur V1.1b (SEQ ID NO: 151) et des sutures permettant l'assemblage des briques Ori BsaI B (SEQ ID NO : 106), AmpR BsaI B (SEQ ID NO : 107), pEF1aL BsaI B (SEQ ID NO : 108), EGFP-CAAX BsaI A (SEQ ID NO : 109), BGHpA BsaI C (SEQ ID NO : 110), pCMV BsaI D (SEQ ID NO : 111), SiaT BsaI B (SEQ ID NO : 112), mCherry BsaI B (SEQ ID NO : 113), TKter BsaI B (SEQ ID NO : 64) et HygroR BsaI D (SEQ ID NO : 114).

**Figure 25 :** Empreinte de restriction par triple digestion EcoRV/PstI/ScaI du vecteur V1.1b.
La digestion produit 7 fragments de 2281, 1723, 1309, 757, 635, 505 et 360 paires de bases respectivement.

1 : 2 281 pb, de EcoRV [3036] à PstI [5317]
2 : 1 723 pb, de ScaI [7261] à ScaI [1414]
3 : 1 309 pb, de PstI [5317] à PstI [6626]
4 : 757 pb, de ScaI [1414] à PstI [2171]
5 : 635 pb, de PstI [6626] à ScaI [7261]
6 : 505 pb, de PstI [2171] à PstI [2676]
7 : 360 pb, de PstI [2676] à EcoRV [3036]

**Figure 26 :** Observation microscopique de cellules 4T1 24 heures après électroporation avec le vecteur V1.1b.

**A** : groupe de cellules visibles en microscopie en lumière blanche.
**B :** même champs optique que A observé en microscopie à fluorescence verte (visualisation GFP).
**C** : même champs optique que A observé en microscopie à fluorescence rouge (visualisation mCherry).
**D** : superposition des champs observés en B et C.

**Figure 27 :** Schéma du vecteur V4 (SEQ ID NO : 152) et des sutures permettant l'assemblage des briques Ori-2 BsaI C (SEQ ID NO : 115), AmpR BsaI C (SEQ ID NO : 116), MNN10-Lrec BsaI A (SEQ ID NO : 117), KanMX BsaI A (SEQ ID NO : 119) et MNN10-Rrec BsaI A (SEQ ID NO : 118).

**Figure 28** : Empreinte de restriction par double digestion PmeI/HindIII du vecteur V4.
La digestion produit 5 fragments de 1280, 1012, 856, 631 et 567 paires de bases respectivement.

1 : 1280 pb, de PmeI [1857] à HindIII [3137]
2 : 1 012 pb, de PmeI [845] à PmeI [1857]
3 : 856 pb, de PmeI [4335] à PmeI [845]
4 : 631 pb, de HindIII [3137] à HindIII [3768]
5 : 567 pb, de HindIII [3768] à PmeI [4335]

**Figure 29 :** Vérification de l'invalidation du gène MNN10 de levure par analyse du profil de glycosylation de l'invertase.

A : profil de migration d'une souche inactivée pour le gène Pmr1, gène dont la mutation entraîne un net défaut de glycosylation de l'invertase.
B : profil de migration de l'invertase d'une souche sauvage.
C :profil de glycosylation de l'invertase d'une souche dont le gène MNN10 a été invalidé par recombinaison homologue avec la cassette de délétion du plasmide V4.

**Figure 30 :** Schéma représentant la construction d'un vecteur d'expression par la méthode décrite. **A :** Représentation de 8 briques d'ADN. Chaque brique contient une séquence d'intérêt (SI) annotées de A à H (en gris) qui elle-même peut contenir plusieurs modules (nuances de gris) numérotés de 1 à 13. Les modules non fonctionnels sont annotés a et b en sus de la numérotation. Les briques contiennent en outre des adaptateurs en amont et aval des SI, dont la partie 'suture' est représentée en blanc et la partie la plus externe en noir. Sur ce schéma, les sutures complémentaires les unes des autres sont reliées par des lignées pointillées. B : Représentation linéaire d'un vecteur circulaire contenant les SI illustrées en A (x étant contigu à y) obtenu selon la méthode décrite. Les lignes en gras délimitent les SI qui sont maintenant contiguës et reliées par les sutures (blanc) dont chacun des deux brins cohésifs a été apporté par une SI voisine. La structuration du vecteur selon les unités fonctionnelles telles que définies dans le texte est illustrée sous forme de rectangles en dessous de la molécule schématisée. Lorsque les combinaisons de modules constituant les unités correspondent à une séquence d'ADN destinée à être transcrite, le sens de transcription est représenté sous forme de flèche.

Dans l'exemple représenté par les figures A et B, les modules sont définis comme suit :

Module 1 : origine de réplication
Module 2a : séquence bipartite d'intégration génomique, partie gauche
Module 3 : terminateur de transcription
Module 4a : séquence codante incomplète (partie 3' qui contient un codon stop)
Module 4b : séquence codante incomplète (partie 5' qui contient un ATG)
Module 5 : promoteur eucaryote
Module 6 : promoteur eucaryote
Module 7 : séquence codant un marqueur de sélection positive eucaryote
Module 8 : terminateur de transcription
Module 2b : séquence bipartite d'intégration génomique, partie droite
Module 9 : séquence codante d'un marqueur de sélection procaryote
Module 10 : promoteur bactérien
Module 11 : promoteur eucaryote
Module 12 : séquence codante complète
Module 13 : terminateur de transcription

## EXEMPLES

*1. Exemples de briques fabriquées*

Tableau **2 :** Exemple de briques fabriquées, les matrices initiales ont été génétiquement modifiées afin de pouvoir être utilisées selon le procédé décrit par la présente invention (non représenté dans le tableau).

| Brique | Matrice initiale | fournisseur |
|---|---|---|
| OriAmp | pCDNA3.1(+)Hygro | Invitrogen-Life technologies |
| LacZ$\alpha$ | pUC19 | Invitrogen-Life technologies |

(suite)

| Brique | Matrice initiale | fournisseur |
|---|---|---|
| ZeocinR | pCDNA3.1(-)Zeo | Invitrogen-Life technologies |
| HygromycinR | pCDNA3.1(+)Hygro | Invitrogen-Life technologies |
| CMVp | pEN_CmiRc2 | ATCC (MBA-284) |
| EF1p | pEN_EmiRc3 | ATCC (MBA-286) |
| Ubi-Cp | pEN_UbmiRc3 | ATCC (MBA-288) |
| EYFP-Ctag | pEYFP-C1 | Clontech disc[d] |
| ECFP-Ctag | pECFP-C1 | Clontech disc[d] |
| EGFP-Ctag | pEGFP-C1 | Clontech disc[d] |
| E1GFP-Ctag | pEGFP-C1 | Clontech disc[d] |
| mCherry-Ctag | pmCherry-C1 | M. Coppey-Moisan |
| TagBFP-Ctag | pTagBFP-C | Evrogen |
| BGHpolyA | pCDNA3.1(+)Hygro | In vitrogen-Life technologies |
| BGH-PolyA signal : bovine growth hormone polyadenylation signal<br>CMV: Cytomegalovirus<br>EGFP: enhanced green fluorescent protein<br>EYFP : enhanced yellow fluorescent protein | | |

**Tableau 3.** Briques construites selon l'invention.

| BRIQUE | | MATRICE | | AMORCE SENS | | AMORCE ANTI-SENS | |
|---|---|---|---|---|---|---|---|
| NOM | ID | NOM | ID | NOM | ID | NOM | ID |
| AmpR BsaI A | **SEQ ID NO : 105** | eZ-Ori-AmpR | **SEQ ID NO : 153** | AmpR TTTA BsaI CW | **SEQ ID NO : 121** | AmpR-2 TCCT BsaI CCW | **SEQ ID NO : 72** |
| AmpR BsaI B | **SEQ ID NO : 107** | eZ-Ori-AmpR | **SEQ ID NO : 153** | AmpR AAAC BsaI CW | **SEQ ID NO : 125** | AmpR-2 GCAT BsaI CCW | **SEQ ID NO : 124** |
| AmpR BsaI C | **SEQ ID NO : 116** | eZ-Ori-AmpR | **SEQ ID NO : 153** | AmpR AAAC BsaI CW | **SEQ ID NO : 125** | AmpR-2 TCAC BsaI CCW | **SEQ ID NO : 142** |
| BGHpA BsaI A | **SEQ ID NO : 26** | BGH polyA | **SEQ ID NO : 154** | BGHpA TGAT BsaI CW | **SEQ ID NO : 1** | BGHpA AGAA BsaI CCW | **SEQ ID NO : 2** |
| BGHpA BsaI B | **SEQ ID NO: 39** | BGH polyA | **SEQ ID NO : 154** | BGHpA TGAT BsaI CW | **SEQ ID NO : 1** | BGHpA CGAA BsaI CCW 2 | **SEQ ID NO : 77** |
| BGHpA BsaI C | **SEQ ID NO : 110** | BGH polyA | **SEQ ID NO : 154** | BGHpA GAGT BsaI CW | **SEQ ID NO : 131** | BGHpA CTAC BsaI CCW 2 | **SEQ ID NO : 130** |
| E1GFP BsaI A | **SEQ ID NO : 20** | eZ-E1GFP | **SEQ ID NO: 155** | XFP-Ctag GGGG BsaI CW | **SEQ ID NO: 15** | XFP-Ctag ATCA BsaI CCW | **SEQ ID NO : 16** |
| ECFP BsaI A | **SEQ ID NO : 22** | ECFP | **SEQ ID NO : 157** | XFP-Ctag GGGG BsaI CW | **SEQ ID NO: 15** | XFP-Ctag ATCA BsaI CCW | **SEQ ID NO : 16** |

(suite)

| BRIQUE | | MATRICE | | AMORCE SENS | | AMORCE ANTI-SENS | |
|---|---|---|---|---|---|---|---|
| NOM | ID | NOM | ID | NOM | ID | NOM | ID |
| EGFP BsaI A | **SEQ ID NO : 21** | EGFP | **SEQ ID NO : 156** | XFP-Ctag GGGG BsaI CW | **SEQ ID NO: 15** | XFP-Ctag ATCA BsaI CCW | **SEQ ID NO : 16** |
| EGFP-CAAX BsaI A | **SEQ ID NO : 109** | EGFP | **SEQ ID NO : 156** | EGFP TATC BsaI CW | **SEQ ID NO : 129** | EGFP-CAAX ACTC BsaI CCW | **SEQ ID NO : 128** |
| EYFP BsaI A | **SEQ ID NO : 23** | EYFP | **SEQ ID NO : 158** | XFP-Ctag GGGG BsaI CW | **SEQ ID NO: 15** | XFP-Ctag ATCA BsaI CCW | **SEQ ID NO : 16** |
| hFUT3 BsaI A | **SEQ ID NO: 38** | hFUT3 cDNA | **SEQ ID NO : 160** | hFUT3 CACC BsaI CW | **SEQ ID NO: 75** | hFUT3 ATCA BsaI CCW | **SEQ ID NO : 76** |
| HygroR BsaI A | **SEQ ID NO : 29** | eZ-HygromycinR K7 | **SEQ ID NO : 161** | SV40pori CCAG BsaI CW | **SEQ ID NO : 7** | SV40term ATAA BsaI CCW | **SEQ ID NO : 8** |
| HygroR BsaI B | **SEQ ID NO : 47** | eZ-HygromycinR K7 | **SEQ ID NO : 161** | Hygro CCAG BsaI CW | **SEQ ID NO : 81** | Hygro AATA BsaI CCW | **SEQ ID NO : 82** |
| HygroR BsaI C | **SEQ ID NO : 51** | eZ-HygromycinR K7 | **SEQ ID NO : 161** | Hygro CCAG BsaI CW | **SEQ ID NO : 81** | Hygro CTAC BsaI CCW | **SEQ ID NO : 86** |
| HygroR BsaI D | **SEQ ID NO : 114** | eZ-HygromycinR K7 | **SEQ ID NO : 161** | Hygro ACAA BsaI CW | **SEQ ID NO : 139** | Hygro TAAT BsaI CCW | **SEQ ID NO : 138** |
| KanMX BsaI A | **SEQ ID NO : 119** | KanMX4 K7 | **SEQ ID NO : 162** | KanMX TGCG BsaI CW | **SEQ ID NO : 148** | KanMX ACGA BsaI CCW | **SEQ ID NO : 147** |
| LacZα-down BsaI A | **SEQ ID NO : 28** | LacZα | **SEQ ID NO : 163** | lacZ-down GACA BsaI CW | **SEQ ID NO : 5** | lacZ-down CTGG BsaI CCW | **SEQ ID NO : 6** |
| LacZα-up BsaI A | **SEQ ID NO : 27** | LacZα | **SEQ ID NO: 163** | lacZ-up TTCT BsaI CW | **SEQ ID NO : 3** | lacZ-up TGTC BsaI CCW | **SEQ ID NO : 4** |
| mB3Galt6 BsaI A | **SEQ ID NO : 61** | mB3Galt6 cDNA | **SEQ ID NO : 164** | mB3Galt6 CCAG BsaI CW | **SEQ ID NO : 100** | mB3Galt6 GCGG BsaI CCW | **SEQ ID NO : 101** |
| mB3Galt6 BsaI B | **SEQ ID NO : 63** | eZ-mB3Galt6 cDNA shins | **SEQ ID NO : 165** | mB3Galt6 CCAG BsaI CW | **SEQ ID NO : 100** | mB3Galt6 GCGG BsaI CCW | **SEQ ID NO : 101** |
| mCherry BsaI A | **SEQ ID NO : 24** | mCherry | **SEQ ID NO : 159** | XFP-Ctag GGGG BsaI CW | **SEQ ID NO : 15** | XFP-Ctag ATCA BsaI CCW | **SEQ ID NO : 16** |
| mCherry BsaI B | **SEQ ID NO : 113** | mCherry | **SEQ ID NO : 159** | XFP-Ctag GTGA BsaI CW | **SEQ ID NO : 137** | XFP-Ctag GCGG BsaI CCW | **SEQ ID NO : 136** |
| MNN10-Lrec BsaI A | **SEQ ID NO : 117** | Yeast MNN10 gene | **SEQ ID NO : 166** | MNN10Lrec GTGA BsaI CW | **SEQ ID NO : 144** | MNN10Lrec CGCA BsaI CCW | **SEQ ID NO : 143** |

(suite)

| BRIQUE | | MATRICE | | AMORCE SENS | | AMORCE ANTI-SENS | |
|---|---|---|---|---|---|---|---|
| NOM | ID | NOM | ID | NOM | ID | NOM | ID |
| MNN10-Rrec BsaI A | **SEQ ID NO : 118** | Yeast MNN10 gene | **SEQ ID NO : 166** | MNN10Rrec TCGT BsaI CW | **SEQ ID NO : 146** | MNN10Rrec CCCC BsaI CCW | **SEQ ID NO : 145** |
| Ori BsaI A | **SEQ ID NO : 104** | eZ-Ori-AmpR | **SEQ ID NO : 153** | ColE1-ori 2 TATT BsaI CW | **SEQ ID NO : 71** | ColE1-ori TAAA BsaI CCW | **SEQ ID NO : 120** |
| Ori BsaI B | **SEQ ID NO : 106** | eZ-Ori-AmpR | **SEQ ID NO : 153** | ColE1-or1-2 ATTA BsaI CW | **SEQ ID NO : 123** | ColE1-ori GTTT BsaI CCW | **SEQ ID NO : 122** |
| Ori-2 BsaI C | **SEQ ID NO : 115** | eZ-Ori-AmpR | **SEQ ID NO : 153** | ColE1-or1-2 GGGG BsaI CW | **SEQ ID NO : 141** | ColE1-or1-2 GTTT BsaI CCW | **SEQ ID NO : 140** |
| Ori-AmpR BsaI A | **SEQ ID NO : 17** | eZ-Ori-AmpR | **SEQ ID NO : 153** | ColE1-ori TTAT BsaI CW | **SEQ ID NO : 9** | AmpR TCCT BsaI CCW | **SEQ ID NO :10** |
| Ori-AmpR BsaI B | **SEQ ID NO : 36** | eZ-Ori-AmpR | **SEQ ID NO : 153** | ColE1-or1-2 TATT BsaI CW | **SEQ ID NO : 71** | AmpR-2 TCCT BsaI CCW | **SEQ ID NO : 72** |
| pCMV BsaI A | **SEQ ID NO : 18** | promCMV | **SEQ ID NO : 167** | pENprom AGGA BsaI CW | **SEQ ID NO :11** | pENprom GGTG BsaI CCW | **SEQ ID NO :12** |
| pCMV BsaI B | **SEQ ID NO : 37** | promCMV | **SEQ ID NO : 167** | CMVp AGGA BsaI CW | **SEQ ID NO : 73** | CMVp GGTG BsaI CCW | **SEQ ID NO : 74** |
| pCMV BsaI C | **SEQ ID NO : 50** | promCMV | **SEQ ID NO : 167** | CMVp ACAA BsaI CW | **SEQ ID NO : 85** | CMVp GGTG BsaI CCW | **SEQ ID NO : 74** |
| pCMV BsaI D | **SEQ ID NO : 111** | promCMV | **SEQ ID NO : 167** | CMVp GTAG BsaI CW | **SEQ ID NO : 133** | CMVp CGAA BsaI CCW | **SEQ ID NO : 132** |
| pEF1a BsaI A | **SEQ ID NO : 55** | promEF1alpha court | **SEQ ID NO : 168** | EF1ap ATGC BsaI CW | **SEQ ID NO : 90** | EF1ap GGGC BsaI CCW | **SEQ ID NO : 91** |
| pEF1aL BsaI B | **SEQ ID NO : 108** | promEF1alpha | **SEQ ID NO :169** | EF1apL ATGC BsaI CW | **SEQ ID NO : 127** | EF1ap GATA BsaI CCW | **SEQ ID NO : 126** |
| pTRE3G BsaI A | **SEQ ID NO : 60** | promTRE3G | **SEQ ID NO : 170** | pTet3G TTCG BsaI CW | **SEQ ID NO: 98** | pTet3G CTGG BsaI CCW | **SEQ ID NO : 99** |
| rosa26-3' BsaI A | **SEQ ID NO : 52** | eZ-Rosa26-3' | **SEQ ID NO : 171** | rosa26-3' GTAG BsaI CW | **SEQ ID NO : 87** | rosa26-3' AATA BsaI CCW | **SEQ ID NO : 88** |
| rosa26-3' BsaI B | **SEQ ID NO : 54** | eZ-Rosa26-3' | **SEQ ID NO : 171** | rosa26-3' GTAG BsaI CW | **SEQ ID NO : 87** | rosa26-3' GCAT BsaI CCW | **SEQ ID NO : 89** |
| rosa26-5' BsaI A | **SEQ ID NO : 49** | eZ-Rosa26-5' | **SEQ ID NO : 172** | rosa26-5' AGGA BsaI CW | **SEQ ID NO : 83** | rosa26-5' TTGT BsaI CCW | **SEQ ID NO : 84** |
| shB3Galt6 BsaI A | **SEQ ID NO : 40** | mB3Galt6 shRNA TR506016D | **SEQ ID NO : 173** | shB3Galt6 TTCG BsaI CW | **SEQ ID NO : 78** | shB3Galt6 AATA BsaI CCW | **SEQ ID NO : 79** |
| shB3Galt6 BsaI B | **SEQ ID NO : 46** | mB3Galt6 shRNA TR506016D | **SEQ ID NO :** 173 | shB3Galt6 TTCG BsaI CW | **SEQ ID NO : 78** | shB3Galt6 CTGG BsaI CCW | **SEQ ID NO : 80** |

(suite)

| BRIQUE | | MATRICE | | AMORCE SENS | | AMORCE ANTI-SENS | |
|---|---|---|---|---|---|---|---|
| NOM | ID | NOM | ID | NOM | ID | NOM | ID |
| shB3Galt6 BsaI C | **SEQ ID NO : 65** | mB3Galt6 shRNA TR506016D | **SEQ ID NO : 173** | shB3Galt6 ACAA BsaI CW | **SEQ ID NO : 103** | shB3Galt6 AATA BsaI CCW | **SEQ ID NO : 79** |
| SiaT BsaI A | **SEQ ID NO : 19** | eZ-SiaT-TGS-Hook | **SEQ ID NO : 174** | SiaT CACC BsaI CW | **SEQ ID NO : 13** | SiaT CCCC BsaI CCW | **SEQ ID NO : 14** |
| SiaT BsaI B | **SEQ ID NO** : 112 | eZ-SiaT-TGS-Hook | **SEQ ID NO :174** | SiaT TTCG BsaI CW | **SEQ ID NO : 135** | SiaT TCAC BsaI CCW | **SEQ ID NO : 134** |
| TagBFP BsaI A | **SEQ ID NO** : 25 | TagBFP | **SEQ ID NO :175** | TagBFP-Ctag GGGG BsaI CW | **SEQ ID NO :69** | TagBFP-Ctag ATCA BsaI CCW | **SEQ ID NO :70** |
| TK BsaI A | **SEQ ID NO** : 56 | Thymidine Kinase cDNA | **SEQ ID NO :176** | TK GCCC BsaI CW | **SEQ ID NO : 92** | TK GCGG BsaI CCW | **SEQ ID NO : 93** |
| Tkter BsaI A | **SEQ ID NO** : 57 | TK term | **SEQ ID NO :177** | HSVTKterm CCGC BsaI CW | **SEQ ID NO : 94** | HSVTKterm AATA BsaI CCW | **SEQ ID NO : 95** |
| Tkter BsaI B | **SEQ ID** NO : 64 | TK term | **SEQ ID NO :177** | HSVTKterm CCGC BsaI CW | **SEQ ID NO : 94** | HSVTKterm TTGT BsaI CCW | **SEQ ID NO : 102** |
| TO3G BsaI A | **SEQ ID NO** : 59 | TetON-3G cDNA | **SEQ ID NO :178** | TetOn3G CACC BsaI CW | **SEQ ID NO : 96** | TetOn3G ATCA BsaI CCW | **SEQ ID NO : 97** |

Les exemples suivants illustreront mieux l'invention.

**Exemple de protocole d'assemblage selon l'invention :**

20 à 100 fmol de chaque brique moléculaire sélectionnée pour réaliser un vecteur sont mélangés dans un volume de 20 µl d'une solution comprenant:

- 2 µl de tampon de ligation 10X,
- 10U (1 µl) d'enzyme de restriction de type IIs e BsaI,
- 3U (1 µl) de ligase si le nombre de fragments est inférieur ou égal à 4 ou de 20U (1 µl) de ligase Haute Concentration (HC) si le nombre de fragments est supérieur à 4.
  qsp : eau distillée ultrapure

Le mélange est réalisé sur glace, c'est à dire à une température de 4°C environ.
Le mélange est ensuite soit incubé à 37°C (30' à 6h), si le nombre de briques à assembler est inférieur à 5, soit soumis à des cycles d'incubation de 2' à 37°C et de 3' à 16°C (25 à 50 cycles) si le nombre de fragments est supérieur à 4.
A la fin de cette période d'incubation, les réactions sont incubées à 50°C pendant 5' (coupure des sites BsaI subsistants) puis à 80°C pendant 5' (inactivation des enzymes).
2 à 10 µl de chaque assemblage sont ensuite utilisés pour transformer 50 à 100 µl de bactéries compétentes, et l'intégralité des bactéries transformées sont étalées sur une ou deux boîtes de pétri contenant une gélose LB complémentée de l'antibiotique de sélection (correspondant au module de résistance antiobiotique de l'unité bactérienne).

**Réalisation 1**

Dans ce premier exemple, le but est de réaliser 6 constructions permettant d'exprimer des protéines fluorescentes de différentes couleurs dans le compartiment Golgien de cellules de mammifères (figure 4). Les vecteurs doivent pouvoir être utilisés pour créer des lignées stables, ce qui impose l'introduction d'un module de sélection dans l'unité d'intégration. Afin de repérer visuellement les plasmides où l'assemblage est correct et d'estimer la proportion de contaminants ou de clones incorrects, une cassette bactérienne exprimant lac Z est également ajoutée.

Les vecteurs souhaités contiennent :

- Une unité de réplication bactérienne :
  - un module contenant une origine de réplication suivie du gène de résistance *bla* (AmpR)
  - un module (en deux fragments) permettant le criblage bleu blanc par l'activité du gène lacZα
- Une cassette d'expression des protéines de fusion contenant :
  - un module promoteur (CMV),
  - un module d'adressage au compartiment Golgien (première partie de l'ORF) constitué des 111 premiers acides aminés codés par le cDNA du gène humain ST6GAL1,
  - un module codant une protéine fluorescente fusionnée à la séquence d'adressage en C-terminal (six modules interchangeables),
  - un module correspondant à un terminateur de transcription (BGHpolyA)
- Une unité d'intégration en cellule eucaryote :
  - Un module contenant le gène de résistance à l'Hygromycine B sous le contrôle du promoteur SV40.

Le choix de chaque suture a été fait selon l'invention, et le plasmide reconstitué est représenté figure. 4.

**Préparation des briques moléculaires**

**Tableau 4 :** Liste des amorces utilisées pour amplifier les briques indiquées (site de reconnaissance BsaI souligné et sutures en gras)

| NOM | SEQUENCE | ID |
|---|---|---|
| BGHpA TGAT BsaI CW | GAGGTACC<u>GGTCTC</u>A**TGAT**CGACTGTGCCTTCTAGTTGCC | SEQ ID NO : 1 |
| BGHpA AGAA BsaI CCW | GAGGTACC<u>GGTCTC</u>C**AGAA**GCCATAGAGCCCACCGC | SEQ ID NO : 2 |
| lacZ-up TTCT BsaI CW | GAGGTACC<u>GGTCTC</u>G**TTCT**CCCTGCAGGTGCGCCCAATACGCAAACCGCC | SEQ ID NO : 3 |
| lacZ-up TGTC BsaI CCW | GAGGTACC<u>GGTCTC</u>C**TGTC**CGTAATCATGGTCATAGCTGTTTCC | SEQ ID NO : 4 |
| lacZ-down GACA BsaI CW | GAGGTACC<u>GGTCTC</u>G**GACA**GCCTGGCCGTCGTTTTACAACG | SEQ ID NO: 5 |
| lacZ-down CTGG BsaI CCW | GAGGTACC<u>GGTCTC</u>A**CTGG**CCCTGCAGGTCTATGCGGCATCAGAGCAGATTGTAC | SEQ ID NO: 6 |
| SV40pori CCAG BsaI CW | GAGGTACC<u>GGTCTC</u>C**CCAG**CAGGCAGAAGTATGCAAAGC | SEQ ID NO : 7 |
| SV40term ATAA BsaI CCW | GAGGTACC<u>GGTCTC</u>G**ATAA**GATACATTGATGAGTTTGGAC | SEQ ID NO : 8 |
| ColE1-ori TTAT BsaI CW | GAGGTACC<u>GGTCTC</u>A**TTAT**GCGTCTTCTAGGGTTAAGGTTAGTGTAGAGAAGCAACCG | SEQ ID NO : 9 |

(suite)

| NOM | SEQUENCE | ID |
|---|---|---|
| AmpR TCCT BsaI CCW | GAGGTACCGGTCTCG**TCCT**TGAGACGCTAGTCCTCGTTCCCGATGCTCTCGTCCTATCC | SEQ ID NO : 10 |
| pENprom AGGA BsaI CW | GAGGTACCGGTCTCA**AGGA**ACCAATTCAGTCGACTGG | SEQ ID NO : 11 |
| pENprom GGTG BsaI CCW | GAGGTACCGGTCTCA**GGTG**GCGGCCCTGTTATCCCTAGTCGACTAG | SEQ ID NO : 12 |
| SiaT CACC BsaI CW | GAGGTACCGGTCTCC**CACC**ATGATTCACACCAACCTGAAG | SEQ ID NO: 13 |
| SiaT CCCC BsaI CCW | GAGGTACCGGTCTCA**CCCC**TTTTGCAGCCTAGGGATAAGG | SEQ ID NO: 14 |
| XFP-Ctag GGGG BsaI CW | GAGGTACCGGTCTCC**GGGG**TCGGGGGTGAGCAAGGGCGAGGAG | SEQ ID NO : 15 |
| XFP-Ctag ATCA BsaI CCW | GAGGTACCGGTCTCC**ATCA**CTTGTACAGCTCGTCCATGC | SEQ ID NO: 16 |
| tagBFP-Ctag GGGG BsaI CW | GAGGTACCGGTCTCC**GGGG**TCGGGGAGCGAGCTGATTAAGGAGAACATGC | SEQ ID NO : 69 |
| tagBFP-Ctag ATCA BsaI CCW | GAGGTACCGGTCTCC**ATCA**TCCGGAATTAAGCTTGTGCCCCAG | SEQ ID NO : 70 |
| ColE1-or1-2 TATT BsaI CW | GAGGTACCGGTCTCG**TATT**GTAATACGGTTATCCACAGAATCAGG | SEQ ID NO : 71 |
| AmpR-2 TCCT BsaI CCW | GAGGTACCGGTCTCG**TCCT**TGGCACTTTTCGGGGAAATGTGC | SEQ ID NO : 72 |
| CMVp AGGA BsaI CW | GAGGTACCGGTCTCAAGGAACCA**ATTC**AGTCGACTGG | SEQ ID NO : 73 |
| CMVp GGTG BsaI CCW | GAGGTACCGGTCTCG**GGTG**CCCTGTTATCCCTAGTCGACTAG | SEQ ID NO : 74 |
| hFUT3 CACC BsaI CW | GAGGTACCGGTCTCA**CACC**ATGGATCCCCTGGGTGCAGC | SEQ ID NO: 75 |
| hFUT3 ATCA BsaI CCW | GAGGTACCGGTCTCG**ATCA**GGTGAACCAAGCCGCTATGCTG | SEQ ID NO: 76 |
| BGH polyA CGAA BsaI CCW | GAGGTACCGGTCTCG**CGAA**GCCATAGAGCCCACCGC | SEQ ID NO : 77 |
| shB3Galt6 TTCG BsaI CW | GAGGTACCGGTCTCA**TTCG**ACAGGGTCGACAAGCTTTTCC | SEQ ID NO : 78 |
| shB3Galt6 AATA BsaI CCW | GAGGTACCGGTCTCG**AATA**CAAAACGCACCACGTGACG | SEQ ID NO : 79 |
| shB3Galt6 CTGG BsaI CCW | GAGGTACCGGTCTCG**CTGG**CAAAACGCACCACGTGACG | SEQ ID NO : 80 |
| Hygro CCAG BsaI CW | GAGGTACCGGTCTCA**CCAG**CAGGCAGAAGTATGCAAAGC | SEQ ID NO : 81 |
| Hygro AATA BsaI CCW | GAGGTACCGGTCTCG**AATA**GATACATTGATGAGTTTGGACAAACCAC | SEQ ID NO : 82 |

(suite)

| NOM | SEQUENCE | ID |
|---|---|---|
| rosa26-5' AGGA BsaI CW | GAGGTACCGGTCTCA**AGGA**CCCCGCGGCAGGCCCTCC | SEQ ID NO: 83 |
| rosa26-5' TTGT BsaI CCW | GAGGTACCGGTCTCG**TTGT**AAGACTGGAGTTGCAGATCACGAG | SEQ ID NO: 84 |
| CMVp ACAA BsaI CW | GAGGTACCGGTCTCA**ACAA**ACCAATTCAGTCGACTGG | SEQ ID NO : 85 |
| Hygro CTAC BsaI CCW | GAGGTACCGGTCTCG**CTAC**GATACATTGATGAGTTTGGACAAACC AC | SEQ ID NO: 86 |
| rosa26-3' GTAG BsaI CW | GAGGTACCGGTCTCA**GTAG**AGATGGGCGGGAGTCTTCTG | SEQ ID NO : 87 |
| rosa26-3' AATA BsaI CCW | GAGGTACCGGTCTCG**AATA**GATAAGCTAGATGTCCTAAATATTTC TATC | SEQ ID NO : 88 |
| rosa26-3' GCAT BsaI CCW | GAGGTACCGGTCTCG**GCAT**GATAAGCTAGATGTCCTAAATATTTC TATC | SEQ ID NO : 89 |
| EF1a ATGC BsaI CW | GAGGTACCGGTCTCA**ATGC**AAGGAACCAATTCAGTCGACTGGAT C | SEQ ID NO : 90 |
| EF1a GGGC BsaI CCW | GAGGTACCGGTCTCG**GGGC**CCCTGTTATCCCTAGTCGACTAG | SEQ ID NO : 91 |
| TK GCCC BsaI CW | GAGGTACCGGTCTCA**GCCC**ATGGCTTCGTACCCCTGC | SEQ ID NO : 92 |
| TK GCGG BsaI CCW | GAGGTACCGGTCTCG**GCGG**TCAGTTAGCCTCCCCCATCTCC | SEQ ID NO: 93 |
| HSVTKterm CCGC BsaI CW | GAGGTACCGGTCTCA**CCGC**GGGGGAGGCTAACTGAAACAC | SEQ ID NO: 94 |
| HSVTKterm AATA BsaI CCW | GAGGTACCGGTCTCG**AATA**GGCTATGGCAGGGCCTGC | SEQ ID NO : 95 |
| TetOn3G CACC BsaI CW | GAGGTACCGGTCTCA**CACC**ATGTCTAGACTGGACAAGAGCAAAG | SEQ ID NO : 96 |
| TetOn3G ATCA BsaI CCW | GAGGTACCGGTCTCA**ATCA**TTACCCGGGGAGCATGTCAAG | SEQ ID NO : 97 |
| pTet3G TTCG BsaI CW | GAGGTACCGGTCTCA**TTCG**TCTTCAAGAATTCCTCGAGTTTACTCC | SEQ ID NO : 98 |
| pTet3G CTGG BsaI CCW | GAGGTACCGGTCTCG**CTGG**TTTACGAGGGTAGGAAGTGGTACG | SEQ ID NO : 99 |
| mB3Galt6 CCAG BsaI CW | GAGGTACCGGTCTCA**CCAG**AGCATGAAGGTATTCCGGCGCGCTT G | SEQ ID NO : 100 |
| mB3Galt6 GCGG BsaI CCW | GAGGTACCGGTCTCG**GCGG**TGACATCAGGGAACGCCCTCCTTG | SEQ ID NO: 101 |
| HSVTKterm TTGT BsaI CCW | GAGGTACCGGTCTCG**TTGT**GGCTATGGCAGGGCCTGC | SEQ ID NO: 102 |
| shB3Galt6 ACAA BsaI CW | GAGGTACCGGTCTCA**ACAA**ACAGGGTCGACAAGCTTTTCC | SEQ ID NO : 103 |

(suite)

| NOM | SEQUENCE | ID |
|---|---|---|
| ColE1-ori TAAA BsaI CCW | GAGGTACCGGTCTCA**TAAA**ACTCATATATACTTTAGATTGATTTAAAAC | SEQ ID NO: 120 |
| AmpR TTTA BsaI CW | GAGGTACCGGTCTCT**TTTA**TTGGTCTGACAGTTACCAATGCTTAATC | SEQ ID NO : 121 |
| ColE1-ori GTTT BsaI CCW | GAGGTACCGGTCTCG**GTTT**ACTCATATATACTTTAGATTGATTTAAAAC | SEQ ID NO : 122 |
| ColE1-ori 2 ATTA BsaI CW | GAGGTACCGGTCTCT**ATTA**CGGTAATACGGTTATCCACAG | SEQ ID NO : 123 |
| AmpR 2 GCAT BsaI CCW | GAGGTACCGGTCTCA**GCAT**TGGCACTTTTCGGGGAAATGTGC | SEQ ID NO : 124 |
| AmpR AAAC BsaI CW | GAGGTACCGGTCTCA**AAAC**TTGGTCTGACAGTTACCAATGCTTAATC | SEQ ID NO : 125 |
| EF1ap GATA BsaI CCW | GAGGTACCGGTCTCG**GATA**TCACGACACCTGAAATGGAAG | SEQ ID NO : 126 |
| EF1apL ATGC BsaI CW | GAGGTACCGGTCTCA**ATGC**GTGAGGCTCCGGTGCCCGTC | SEQ ID NO: 127 |
| EGFP-CAAX ACTC BsaI CCW | GAGGTACCGGTCTCC**ACTC**TTACATAATTACACACTTTGTCTTTGACTTCTTTTTCTTCTTCTTGTACAGCTCGTCCATGC | SEQ ID NO: 128 |
| EGFP TATC BsaI CW | GAGGTACCGGTCTCC**TATC**ATGGTGAGCAAGGGCGAGG | SEQ ID NO : 129 |
| BGHpA CTAC BsaI CCW 2 | GAGGTACCGGTCTCG**CTAC**CCATAGAGCCCACCGCATCC | SEQ ID NO : 130 |
| BGHpA GAGT BsaI CW | GAGGTACCGGTCTCA**GAGT**CGACTGTGCCTTCTAGTTGCC | SEQ ID NO : 131 |
| CMVp CGAA BsaI CCW | GAGGTACCGGTCTCG**CGAA**GATCTGACGGTTCACTAAACCAG | SEQ ID NO : 132 |
| CMVp GTAG BsaI CW | GAGGTACCGGTCTCA**GTAG**TTATTAATAGTAATCAATTACGGGGTC | SEQ ID NO : 133 |
| SiaT TCAC BsaI CCW | GAGGTACCGGTCTCA**TCAC**CCCCGACCCCTTTTGCAG | SEQ ID NO : 134 |
| SiaT TTCG BsaI CW | GAGGTACCGGTCTCC**TTCG**ATGATTCACACCAACCTGAAGAAAAAG | SEQ ID NO: 135 |
| XFP-Ctag GCGG BsaI CCW | GAGGTACCGGTCTCA**GCGG**TTACTTGTACAGCTCGTCCATGC | SEQ ID NO: 136 |
| XFP-Ctag GTGA BsaI CW | GAGGTACCGGTCTCA**GTGA**GCAAGGGCGAGGAG | SEQ ID NO : 137 |
| Hygro TAAT BsaI CCW | GAGGTACCGGTCTCG**TAAT**GATACATTGATGAGTTTGGACAAACCAC | SEQ ID NO: 138 |
| Hygro ACAA BsaI CW | GAGGTACCGGTCTCA**ACAA**CAGGCAGAAGTATGCAAAGC | SEQ ID NO : 139 |
| ColE1-ori 2 GTTT BsaI CCW | GAGGTACCGGTCTCA**GTTT**AAACTCATATATACTTTAGATTGATTTAAAAC | SEQ ID NO : 140 |

(suite)

| NOM | SEQUENCE | ID |
|---|---|---|
| ColE1-ori 2 GGGG BsaI CW | GAGGTACCGGTCTCT**GGGG**CGGTAATACGGTTATCCACAG | SEQ ID NO : 141 |
| AmpR 2 TCAC BsaI CCW | GAGGTACCGGTCTCA**TCAC**TGGCACTTTTCGGGGAAATGTGC | SEQ ID NO : 142 |
| MNN10Lrec CGCA BsaI CCW | GAGGTACCGGTCTCA**CGCA**ATTGTATAGTTGTACATGCACAATTA TTCC | SEQ ID NO : 143 |
| MNN10Lrec GTGA BsaI CW | GAGGTACCGGTCTCT**GTGA**GTTTAAACATGCATTCAAAGGTCATA ATTGCTG | SEQ ID NO : 144 |
| MNN10Rrec CCCC BsaI CCW | GAGGTACCGGTCTCT**CCCC**GTTTAAACTGCCCAGTTTTTCATTATT AGTGTG | SEQ ID NO: 145 |
| MNN10Rrec TCGT BsaI CW | GAGGTACCGGTCTCT**TCGT**AATGGAAGTTATCAATATTGTAAAGA GAAGC | SEQ ID NO: 146 |
| KanMX ACGA BsaI CCW | GAGGTACCGGTCTCT**ACGA**CACTAGTGGATCTGATATCACC | SEQ ID NO : 147 |
| KanMX TGCG BsaI CW | GAGGTACCGGTCTCG**TGCG**GTACGCTGCAGGTCGACAACC | SEQ ID NO : 148 |

**Tableau 5:** Exemple de composition du mix réactionnel pour la réalisation des PCR

| Composé | volume |
|---|---|
| Matrice (2ng/μl) | 1 μl |
| 5X HF buffer | 10 μl |
| dNTP (25mM) | 0,5 μl |
| Oligo_CW_F | 1,25 μl |
| Oligo_CCW_F | 1,25 μl |
| Phusion Taq pol. | 0,5 μl |
| $H_2O$ | 35,5 μl |

**Tableau 6:** Cycles d'Amplification

| Température | Durée | Cycle |
|---|---|---|
| 95°C | 30" | - |
| 95°C | 10" | X25 |
| ≈Tm+3°C | 30" | |
| 72°C | ≈30"/ kb | |
| 72°C | 5' | - |
| 4°C | ∞ | - |
| * Les modules fluorescents ont tous été ajustés à la même concentration | | |

**Tableau 7** : Caractéristiques des briques et des conditions de PCR

| Nom de la brique | Taille (pb) | Température d'hybridation | Temps d'élongation | Concentration après purification (ng/μl) |
|---|---|---|---|---|
| Ori-AmpR Bsal A (SEQ ID NO : 17) | 2005 | 60 | 1' | 42 |
| pCMV Bsal A (SEQ ID NO : 18) | 677 | 65 | 20" | 24 |
| SiaT Bsal A (SEQ ID NO : 19) | 370 | 61 | 10" | 104 |
| E1GFP Bsal A (SEQ ID NO : 20) ou EGFP Bsal A (SEQ ID NO : 21) ou ECFP Bsal A (SEQ ID No: 22) ou EYFP Bsal A (SEQ ID NO : 23) ou mCherry Bsal A (SEQ ID NO: 24) ou TagBFP Bsal A (SEQ ID NO: 25) | 758 | 63 | 20" | 75* |
| BGHpA BsaI A (SEQ ID NO : 26) | 267 | 63 | 8" | 52 |
| LacZα-up Bsal A (SEQ ID NO : 27) | 278 | 67 | 8" | 38 |
| LacZα-down Bsal A (SEQ ID NO : 28) | 299 | 65 | 8" | 36 |
| HygroR Bsal A (SEQ ID NO : 29) | 1650 | 63 | 45" | 60 |

A la fin de la PCR les produits sont soumis à une électrophorèse en gel d'agarose et les bandesdécoupées du gel sont purifiées et quantifiées, selon les techniques bien connues de l'homme du métier.

**Protocole d'assemblage des différentes briques moléculaires / construction du vecteur**

**Tableau 8 :** *Composition d'un mix d'assemblage :*

| Brique | Volume (μl) |
|---|---|
| Ori-AmpR Bsal A (SEQ ID NO : 17) | 2,4 |
| pCMV Bsal A (SEQ ID NO : 18) | 1,4 |
| SiaT Bsal A (SEQ ID NO : 19) | 0,2 |
| BGHpA BsaI A (SEQ ID NO : 26) | 0,25 |
| LacZα-up Bsal A (SEQ ID NO : 27) | 0,4 |
| LacZα-down Bsal A (SEQ ID NO : 28) | 0,4 |
| HygroR Bsal A (SEQ ID NO : 29) | 1,4 |
| E1GFP Bsal A (SEQ ID NO : 20) ou EGFP Bsal A (SEQ ID NO : 21) ou ECFP Bsal A (SEQ ID No: 22) ou EYFP Bsal A (SEQ ID NO: 23) ou mCherry Bsal A (SEQ ID NO: 24) ou TagBFP Bsal A (SEQ ID NO: 25) | 0,5 |
| Ligase HC | 1 |
| Bsal | 1 |
| LigationlOX Br | 2 |
| H2O | 9,05 |

L'assemblage a été réalisé par 50 cycles d'incubation à 37°C pendant 2' puis 16°C pendant 3', suivis d'une incubation à 50°C pendant 5' et enfin d'une incubation à 80°C pendant 5'.

Pour chaque construction, l'ADN de 3 colonies obtenues après transformation a été extrait et analysé par restriction par les enzymes PvuIHF et ScaIHF (figure. 5).

Le procédé selon l'invention permet d'assembler en une seule étape et en présence d'une seule enzyme au moins 6 briques moléculaires (et plus préférentiellement au moins 8 briques) sans aucune erreur.

Le procédé selon l'invention permet d'assembler en une seule étape et en présence d'une seule enzyme au moins 6 briques moléculaires avec un rendement de 100%
Le procédé selon l'invention permet d'assembler en une seule étape et en présence d'une seule enzyme au moins 6 briques et jusqu'à 30 briques.

La présente invention permet donc, selon un procédé, la fabrication d'un vecteur circulaire d'ADN double brin à partir de modules fonctionnels linéaires d'ADN double brin comprenant une seule étape d'assemblage des dits modules, en présence d'une seule enzyme de restriction, ladite seule enzyme de restriction étant une enzyme de type IIs.

**Séquences des briques utilisées pour les différentes constructions de la réalisation 1** :

**Brique Ori-AmpR BsaI A (2005 bp) (SEQ ID NO : 17)**

GAGGTACCGGTCTCATTATGCGTCTTCTAGGGTTAAGGTTAGTGTAGAGAAGCAACCGAAGATTGAGAAGACATG
GCGGTAATACGGTTATCCACAGAATCAGGGGATAACGCAGGAAAGAACATGTGAGCAAAAGGCCAGCAAAAGGC
CAGGAACCGTAAAAAGGCCGCGTTGCTGGCGTTTTTCCATAGGCTCCGCCCCCCTGACGAGCATCACAAAAATCGA
CGCTCAAGTCAGAGGTGGCGAAACCCGACAGGACTATAAAGATACCAGGCGTTTCCCCCTGGAAGCTCCCTCGTGC
GCTCTCCTGTTCCGACCCTGCCGCTTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACACGACTTATCGCCACTGGCA
GCAGCCACTGGTAACAGGATTAGCAGAGCGAGGTATGTAGGCGGTGCTACAGAGTTCTTGAAGTGGTGGCCTAAC
TACGGCTACACTAGAAGAACAGTATTTGGTATCTGCGCTCTGCTGAAGCCAGTTACCTTCGGAAAAAGAGTTGGTA
GCTCTTGATCCGGCAAACAAACCACCGCTGGTAGCGGTTTTTTTGTTTGCAAGCAGCAGATTACGCGCAGAAAAAA
AGGATCTCAAGAAGATCCTTTGATCTTTTCTACGGGGTCTGACGCTCAGTGGAACGAAAACTCACGTTAAGGGATT
TTGGTCATGAGATTATCAAAAAGGATCTTCACCTAGATCCTTTTAAATTAAAAATGAAGTTTTAAATCAATCTAAAGT
ATATATGAGTAAACTTGGTCTGACAGTTACCAATGCTTAATCAGTGAGGCACCTATCTCAGCGATCTGTCTATTTCG
TTCATCCATAGTTGCCTGACTCCCCGTCGTGTAGATAACTACGATACGGGAGGGCTTACCATCTGGCCCCAGTGCTG
CAATGATACCGCGTGACCCACGCTCACCGGCTCCAGATTTATCAGCAATAAACCAGCCAGCCGGAAGGGCCGAGC
GCAGAAGTGGTCCTGCAACTTTATCCGCCTCCATCCAGTCTATTAATTGTTGCCGGGAAGCTAGAGTAAGTAGTTCG
CCAGTTAATAGTTTGCGCAACGTTGTTGCCATTGCTACAGGCATCGTGGTGTCACGCTCGTCGTTTGGTATGGCTTC

ATTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCACCTATGAGACGTGAGGCTAGGGATAGGACGAGAGCATCGGGAACGAGGACTAGCG
TCTCAAGGACGAGACCGGTACCTC

**Brique pCMV BsaI A (677pb) (SEQ ID NO : 18)**

GAGGTACCGGTCTCAAGGAACCAATTCAGTCGACTGGATCCTAGTTATTAATAGTAATCAATTACGGGGTCATTAG
TTCATAGCCCATATATGGAGTTCCGCGTTACATAACTTACGGTAAATGGCCCGCCTGGCTGACCGCCCAACGACCCC
CGCCCATTGACGTCAATAATGACGTATGTTCCCATAGTAACGCCAATAGGGACTTTCCATTGACGTCAATGGGTGG
AGTATTTACGGTAAACTGCCCACTTGGCAGTACATCAAGTGTATCATATGCCAAGTACGCCCCCTATTGACGTCAAT
GACGGTAAATGGCCCGCCTGGCATTATGCCCAGTACATGACCTTATGGGACTTTCCTACTTGGCAGTACATCTACGT
ATTAGTCATCGCTATTACCATGGTGATGCGGTTTTGGCAGTACATCAATGGGCGTGGATAGCGGTTTGACTCACGG
GGATTTCCAAGTCTCCACCCCATTGACGTCAATGGGAGTTTGTTTTGGCACCAAAATCAACGGGACTTTCCAAAATG
TCGTAACAACTCCGCCCCATTGACGCAAATGGGCGGTAGGCGTGTACGGTGGGAGGTCTATATAAGCAGAGCTGG
TTTAGTGAACCGTCAGATCACTAGTCGACTAGGGATAACAGGGCCGCCACCTGAGACCGGTACCTC

**Brique SiaT BsaI A (370pb) (SEQ ID NO : 19)**

GAGGTACCGGTCTCCCACCATGATTCACACCAACCTGAAGAAAAAGTTCAGCTGCTGCGTCCTGGTCTTTCTTCTGT
TTGCAGTCATCTGTGTGTGGAAGGAAAAGAAGAAAGGGAGTTACTATGATTCCTTTAAATTGCAAACCAAGGAATT
CCAGGTGTTAAAGAGTCTGGGGAAATTGGCCATGGGGTCTGATTCCCAGTCTGTATCCTCAAGCAGCACCCAGGAC
CCCCACAGGGGCCGCCAGACCCTCGGCAGTCTCAGAGGCCTAGCCAAGGCCAAACCAGAGGCCTCCTTCCAGGTG
TGGAACAAGGACAGCTCTTCCAAAAACCTTATCCCTAGGCTGCAAAAGGGGTGAGACCGGTACCTC

**Brique EGFP BsaI A (758pb) (SEQ ID NO : 20)**

GAGGTACCGGTCTCCGGGGTCGGGGGTGAGCAAGGGCGAGGAGCTGTTCACCGGGGTGGTGCCCATCCTGGTCG
AGCTGGACGGCGACGTAAACGGCCACAAGTTCAGCGTGTCCGGCGAGGGCGAGGGCGATGCCACCTACGGCAAG

CTGACCCTGAAGTTCATCTGCACCACCGGCAAGCTGCCCGTGCCCTGGCCCACCCTCGTGACCACCCTgtccTACGGC
GTGCAGTGCTTCAGCCGCTACCCCGACCACATGAAGCAGCACGACTTCTTCAAGTCCGCCATGCCCGAAGGCTACG
TCCAGGAGCGCACCATCTTCTTCAAGGACGACGGCAACTACAAGACCCGCGCCGAGGTGAAGTTCGAGGGCGACA
CCCTGGTGAACCGCATCGAGCTGAAGGGCATCGACTTCAAGGAGGACGGCAACATCCTGGGGCACAAGCTGGAG
TACAACTACAACAGCCACAACGTCTATATCATGGCCGACAAGCAGAAGAACGGCATCAAGGTGAACTTCAAGATCC
GCCACAACATCGAGGACGGCAGCGTGCAGCTCGCCGACCACTACCAGCAGAACACCCCCATCGGCGACGGCCCCG
TGCTGCTGCCCGACAACCACTACCTGAGctacCAGTCCGCCCTGAGCAAAGACCCCAACGAGAAGCGCGATCACATG
GTCCTGCTGGAGTTCGTGACCGCCGCCGGGATCACTCTCGGCATGGACGAGCTGTACAAGTGATGGAGACCGGTA
CCTC

**Brique EGFP BsaI A (758pb) (SEQ ID NO : 21)**

GAGGTACCGGTCTCCGGGGTCGGGGGTGAGCAAGGGCGAGGAGCTGTTCACCGGGGTGGTGCCCATCCTGGTCG
AGCTGGACGGCGACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGACCACCCTGACCTACGG
CGTGCAGTGCTTCAGCCGCTACCCCGACCACATGAAGCAGCACGACTTCTTCAAGTCCGCCATGCCCGAAGGCTAC
GTCCAGGAGCGCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCACAAGCTGGA
GTACAACTACAACAGCCACAACGTCTATATCATGGCCGACAAGCAGAAGAACGGCATCAAGGTGAACTTCAAGAT
CCGCCACAACATCGAGGACGGCAGCGTGCAGCTCGCCGACCACTACCAGCAGAACACCCCCATCGGCGACGGCCC
CGTGCTGCTGCCCGACAACCACTACCTGAGCACCCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGTACAAGTGATGGAGACCG
GTACCTC

**Brique ECFP BsaI A (758pb) (SEQ ID NO : 22)**

GAGGTACCGGTCTCCGGGGTCGGGGGTGAGCAAGGGCGAGGAGCTGTTCACCGGGGTGGTGCCCATCCTGGTCG
AGCTGGACGGCGACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGACCACCCTGACCTGGG
GCGTGCAGTGCTTCAGCCGCTACCCCGACCACATGAAGCAGCACGACTTCTTCAAGTCCGCCATGCCCGAAGGCTA
CGTCCAGGAGCGCACCATCTTCTTCAAGGACGACGGCAACTACAAGACCCGCGCCGAGGTGAAGTTCGAGGGCGA
CACCCTGGTGAACCGCATCGAGCTGAAGGGCATCGACTTCAAGGAGGACGGCAACATCCTGGGGCACAAGCTGG
AGTACAACTACATCAGCCACAACGTCTATATCACCGCCGACAAGCAGAAGAACGGCATCAAGGCCAACTTCAAGAT
CCGCCACAACATCGAGGACGGCAGCGTGCAGCTCGCCGACCACTACCAGCAGAACACCCCCATCGGCGACGGCCC

CGTGCTGCTGCCCGACAACCACTACCTGAGCACCCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGTACAAGTGATGGAGACCG
GTACCTC

**Brique EYFP BsaI A (758pb) (SEQ ID NO : 23)**

GAGGTACCGGTCTCCGGGGTCGGGGGTGAGCAAGGGCGAGGAGCTGTTCACCGGGGTGGTGCCCATCCTGGTCG
AGCTGGACGGCGACGTAAACGGCCACAAGTTCAGCGTGTCCGGCGAGGGCGAGGGCGATGCCACCTACGGCAAG
CTGACCCTGAAGTTCATCTGCACCACCGGCAAGCTGCCCGTGCCCTGGCCCACCCTCGTGACCACCTTCGGCTACGG
CCTGCAGTGCTTCGCCCGCTACCCCGACCACATGAAGCAGCACGACTTCTTCAAGTCCGCCATGCCCGAAGGCTAC
GTCCAGGAGCGCACCATCTTCTTCAAGGACGACGGCAACTACAAGACCCGCGCCGAGGTGAAGTTCGAGGGCGAC
ACCCTGGTGAACCGCATCGAGCTGAAGGGCATCGACTTCAAGGAGGACGGCAACATCCTGGGGCACAAGCTGGA
GTACAACTACAACAGCCACAACGTCTATATCATGGCCGACAAGCAGAAGAACGGCATCAAGGTGAACTTCAAGAT
CCGCCACAACATCGAGGACGGCAGCGTGCAGCTCGCCGACCACTACCAGCAGAACACCCCCATCGGCGACGGCCC
CGTGCTGCTGCCCGACAACCACTACCTGAGCTACCAGTCCGCCCTGAGCAAAGACCCCAACGAGAAGCGCGATCAC
ATGGTCCTGCTGGAGTTCGTGACCGCCGCCGGGATCACTCTCGGCATGGACGAGCTGTACAAGTGATGGAGACCG
GTACCTC

**Brique mCherry BsaI A (749pb) (SEQ ID NO : 24)**

GAGGTACCGGTCTCCGGGGTCGGGGGTGAGCAAGGGCGAGGAGGATAACATGGCCATCATCAAGGAGTTCATGC
GCTTCAAGGTGCACATGGAGGGCTCCGTGAACGGCCACGAGTTCGAGATCGAGGGCGAGGGCGAGGGCCGCCCC
TACGAGGGCACCCAGACCGCCAAGCTGAAGGTGACCAAGGGTGGCCCCCTGCCCTTCGCCTGGGACATCCTGTCC
CCTCAGTTCATGTACGGCTCCAAGGCCTACGTGAAGCACCCCGCCGACATCCCCGACTACTTGAAGCTGTCCTTCCC
CGAGGGCTTCAAGTGGGAGCGCGTGATGAACTTCGAGGACGGCGGCGTGGTGACCGTGACCCAGGACTCCTCCCT
GCAGGACGGCGAGTTCATCTACAAGGTGAAGCTGCGCGGCACCAACTTCCCCTCCGACGGCCCCGTAATGCAGAA
GAAAACCATGGGCTGGGAGGCCTCCTCCGAGCGGATGTACCCCGAGGACGGCGCCCTGAAGGGCGAGATCAAGC
AGAGGCTGAAGCTGAAGGACGGCGGCCACTACGACGCTGAGGTCAAGACCACCTACAAGGCCAAGAAGCCCGTG
CAGCTGCCCGGCGCCTACAACGTCAACATCAAGTTGGACATCACCTCCCACAACGAGGACTACACCATCGTGGAAC
AGTACGAACGCGCCGAGGGCCGCCACTCCACCGGCGGCATGGACGAGCTGTACAAGTGATGGAGACCGGTACCT
C

**Brique TagBFP BsaI A (746pb) (SEQ ID NO : 25)**

GAGGTACCGGTCTCCGGGGTCGGGGAGCGAGCTGATTAAGGAGAACATGCACATGAAGCTGTACATGGAGGGCA
CCGTGGACAACCATCACTTCAAGTGCACATCCGAGGGCGAAGGCAAGCCCTACGAGGGCACCCAGACCATGAGAA
TCAAGGTGGTCGAGGGCGGCCCTCTCCCCTTCGCCTTCGACATCCTGGCTACTAGCTTCCTCTACGGCAGCAAGACC
TTCATCAACCACACCCAGGGCATCCCCGACTTCTTCAAGCAGTCCTTCCCTGAGGGCTTCACATGGGAGAGAGTCAC
CACATACGAGGACGGGGGCGTGCTGACCGCTACCCAGGACACCAGCCTCCAGGACGGCTGCCTCATCTACAACGT
CAAGATCAGAGGGGTGAACTTCACATCCAACGGCCCTGTGATGCAGAAGAAAACACTCGGCTGGGAGGCCTTCAC
CGAAACGCTGTACCCCGCTGACGGCGGCCTGGAAGGCAGAAACGACATGGCCCTGAAGCTCGTGGGCGGGAGCC
ATCTGATCGCAAACATCAAGACCACATATAGATCCAAGAAACCCGCTAAGAACCTCAAGATGCCTGGCGTCTACTA
TGTGGACTACAGACTGGAAAGAATCAAGGAGGCCAACAACGAAACCTACGTCGAGCAGCACGAGGTGGCAGTGG
CCAGATACTGCGACCTCCCTAGCAAACTGGGGCACAAGCTTAATTCCGGATGATGGAGACCGGTACCTC

**Brique BGHpA BsaI A (267pb) (SEQ ID NO : 26)**

GAGGTACCGGTCTCATGATCGACTGTGCCTTCTAGTTGCCAGCCATCTGTTGTTTGCCCCTCCCCCGTGCCTTCCTTG
ACCCTGGAAGGTGCCACTCCCACTGTCCTTTCCTAATAAAATGAGGAAATTGCATCGCATTGTCTGAGTAGGTGTCA
TTCTATTCTGGGGGGTGGGGTGGGGCAGGACAGCAAGGGGGAGGATTGGGAGGACAATAGCAGGCATGCTGGG
GATGCGGTGGGCTCTATGGCTTCTGGAGACCGGTACCTC

**Brique LacZα-up BsaI A (278) (SEQ ID NO : 27)**

GAGGTACCGGTCTCGTTCTCCCTGCAGGTGCGCCCAATACGCAAACCGCCTCTCCCCGCGCGTTGGCCGATTCATTA
ATGCAGCTGGCACGACAGGTTTCCCGACTGGAAAGCGGGCAGTGAGCGCAACGCAATTAATGTGAGTTAGCTCAC
TCATTAGGCACCCCAGGCTTTACACTTTATGCTTCCGGCTCGTATGTTGTGTGGAATTGTGAGCGGATAACAATTTC
ACACAGGAAACAGCTATGACCATGATTACGGACAGGAGACCGGTACCTC

**Brique LacZα-down BsaI A (299pb) (SEQ ID NO : 28)**

GAGGTACCGGTCTCGGACAGCCTGGCCGTCGTTTTACAACGTCGTGACTGGGAAAACCCTGGCGTTACCCAACTTA
ATCGCCTTGCAGCACATCCCCCTTTCGCCAGCTGGCGTAATAGCGAAGAGGCCCGCACCGATCGCCCTTCCCAACA
GTTGCGCAGCCTGAATGGCGAATGGCGCCTGATGCGGTATTTTCTCCTTACGCATCTGTGCGGTATTTCACACCGCA
TATGGTGCACTCTCAGTACAATCTGCTCTGATGCCGCATAGACCTGCAGGGCCAGTGAGACCGGTACCTC

**Brique HygroR BsaI A (1650pb) (SEQ ID NO : 29)**

GAGGTACCGGTCTCCCCAGCAGGCAGAAGTATGCAAAGCATGCATCTCAATTAGTCAGCAACCAGGTGTGGAAAG
TCCCCAGGCTCCCCAGCAGGCAGAAGTATGCAAAGCATGCATCTCAATTAGTCAGCAACCATAGTCCCGCCCCTAA

CTCCGCCCATCCCGCCCCTAACTCCGCCCAGTTCCGCCCATTCTCCGCCCCATGGCTGACTAATTTTTTTTATTTATGC
AGAGGCCGAGGCCGCCTCTGCCTCTGAGCTATTCCAGAAGTAGTGAGGAGGCTTTTTTGGAGGCCTAGGCTTTTGC
AAAAAGCTCCCGGGAGCTTGTATATCCATTTTCGGATCTGATCAGCACGTGATGAAAAAGCCTGAACTCACCGCGA
CGTCTGTCGAGAAGTTTCTGATCGAAAAGTTCGACAGCGTGTCCGACCTGATGCAGCTCTCGGAGGGCGAAGAAT
CTCGTGCTTTCAGCTTCGATGTAGGAGGGCGTGGATATGTCCTGCGGGTAAATAGCTGCGCCGATGGTTTCTACAA
AGATCGTTATGTTTATCGGCACTTTGCATCGGCCGCGCTCCCGATTCCGGAAGTGCTTGACATTGGGGAATTCAGC
GAGAGCCTGACCTATTGCATCTCCCGCCGTGCACAGGGTGTCACGTTGCAAGACTTGCCTGAAACCGAACTGCCCG
CTGTTCTGCAGCCGGTCGCGGAGGCCATGGATGCGATCGCTGCGGCCGATCTTAGCCAGACGAGCGGGTTCGGCC
CATTCGGACCGCAAGGAATCGGTCAATACACTACATGGCGTGATTTCATATGCGCGATTGCTGATCCCCATGTGTAT
CACTGGCAAACTGTGATGGACGACACCGTCAGTGCGTCCGTCGCGCAGGCTCTCGATGAGCTGATGCTTTGGGCC
GAGGACTGCCCCGAAGTCCGGCACCTCGTGCACGCGGATTTCGGCTCCAACAATGTCCTGACGGACAATGGCCGC
ATAACAGCGGTCATTGACTGGAGCGAGGCGATGTTCGGGGATTCCCAATACGAGGTCGCCAACATCTTCTTCTGGA
GGCCGTGGTTGGCTTGTATGGAGCAGCAGACGCGCTACTTCGAGCGGAGGCATCCGGAGCTTGCAGGATCGCCGC
GGCTCCGGGCGTATATGCTCCGCATTGGTCTTGACCAACTCTATCAGAGCTTGGTTGACGGCAATTTCGATGATGC
AGCTTGGGCGCAGGGTCGATGCGACGCAATCGTCCGATCCGGAGCCGGGACTGTCGGGCGTACACAAATCGCCC
GCAGAAGCGCGGCCGTCTGGACCGATGGCTGTGTAGAAGTACTCGCCGATAGTGGAAACCGACGCCCCAGCACTC
GTCCGAGGGCAAAGGAATAGCACGTGCTACGAGATTTCGATTCCACCGCCGCCTTCTATGAAAGGTTGGGCTTCG
GAATCGTTTTCCGGGACGCCGGCTGGATGATCCTCCAGCGCGGGGATCTCATGCTGGAGTTCTTCGCCCACCCCAA
CTTGTTTATTGCAGCTTATAATGGTTACAAATAAAGCAATAGCATCACAAATTTCACAAATAAAGCATTTTTTTCACT
GCATTCTAGTTGTGGTTTGTCCAAACTCATCAATGTATCTTATCGAGACCGGTACCTC

**Séquence des vecteurs obtenus dans la réalisation 1**

Un vecteur pHCsiaT-$E_1$GFP (SEQ ID NO : 30) selon l'invention est construit à partir d'une combinaison des briques Ori-AmpR BsaI A (SEQ ID NO : 17), pCMV BsaI A (SEQ ID NO : 18), SiaT BsaI A (SEQ ID NO : 19), BGHpA BsaI A (SEQ ID NO : 26), LacZa-up BsaI A (SEQ ID NO : 27), LacZa-down BsaI A (SEQ ID NO : 28), HygroR BsaI A (SEQ ID NO : 29) et E1GFP BsaI A (SEQ ID NO : 20).

**Vecteur pHCsiaT-$E_1$GFP (SEQ ID NO : 30)**

TGAGGCTAGGGATAGGACGAGAGCATCGGGAACGAGGACTAGCGTCTCAAGGAACCAATTCAGTCGACTGGATC
CTAGTTATTAATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATGTTCCCATAGTAA

CGCCAATAGGGACTTTCCATTGACGTCAATGGGTGGAGTATTTACGGTAAACTGCCCACTTGGCAGTACATCAAGT
GTATCATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTTGGCA
GTACATCAATGGGCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGCAAATGGGCGGTA
GGCGTGTACGGTGGGAGGTCTATATAAGCAGAGCTGGTTTAGTGAACCGTCAGATCACTAGTCGACTAGGGATAA
CAGGGCCGCCACCATGATTCACACCAACCTGAAGAAAAAGTTCAGCTGCTGCGTCCTGGTCTTTCTTCTGTTTGCAG
TCATCTGTGTGTGGAAGGAAAAGAAGAAAGGGAGTTACTATGATTCCTTTAAATTGCAAACCAAGGAGTTCCAGGT
GTTAAAGAGTCTGGGGAAATTGGCCATGGGGTCTGATTCCCAGTCTGTATCCTCAAGCAGCACCCAGGACCCCCAC
AGGGGCCGCCAGACCCTCGGCAGTCTCAGAGGCCTAGCCAAGGCCAAACCAGAGGCCTCCTTCCAGGTGTGGAAC
AAGGACAGCTCTTCCAAAAACCTTATCCCTAGGCTGCAAAAGGGGTCGGGGGTGAGCAAGGGCGAGGAGCTGTTC
ACCGGGGTGGTGCCCATCCTGGTCGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACCGGCAAGCTGCCCGTGCCCTGGCCC
ACCCTCGTGACCACCCTGTCCTACGGCGTGCAGTGCTTCAGCCGCTACCCCGACCACATGAAGCAGCACGACTTCTT
CAAGTCCGCCATGCCCGAAGGCTACGTCCAGGAGCGCACCATCTTCTTCAAGGACGACGGCAACTACAAGACCCGC
GCCGAGGTGAAGTTCGAGGGCGACACCCTGGTGAACCGCATCGAGCTGAAGGGCATCGACTTCAAGGAGGACGG
CAACATCCTGGGGCACAAGCTGGAGTACAACTACAACAGCCACAACGTCTATATCATGGCCGACAAGCAGAAGAA
CGGCATCAAGGTGAACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCTACCAGTCCGCCCTGAGCAAA
GACCCCAACGAGAAGCGCGATCACATGGTCCTGCTGGAGTTCGTGACCGCCGCCGGGATCACTCTCGGCATGGAC
GAGCTGTACAAGTGATCGACTGTGCCTTCTAGTTGCCAGCCATCTGTTGTTTGCCCCTCCCCCGTGCCTTCCTTGACC
CTGGAAGGTGCCACTCCCACTGTCCTTTCCTAATAAAATGAGGAAATTGCATCGCATTGTCTGAGTAGGTGTCATTC
TATTCTGGGGGGTGGGGTGGGGCAGGACAGCAAGGGGGAGGATTGGGAGGACAATAGCAGGCATGCTGGGGAT
GCGGTGGGCTCTATGGCTTCTCCCTGCAGGTGCGCCCAATACGCAAACCGCCTCTCCCCGCGCGTTGGCCGATTCA
TTAATGCAGCTGGCACGACAGGTTTCCCGACTGGAAAGCGGGCAGTGAGCGCAACGCAATTAATGTGAGTTAGCT
CACTCATTAGGCACCCCAGGCTTTACACTTTATGCTTCCGGCTCGTATGTTGTGTGGAATTGTGAGCGGATAACAAT
TTCACACAGGAAACAGCTATGACCATGATTACGGACAGCCTGGCCGTCGTTTTACAACGTCGTGACTGGGAAAACC
CTGGCGTTACCCAACTTAATCGCCTTGCAGCACATCCCCCTTTCGCCAGCTGGCGTAATAGCGAAGAGGCCCGCAC
CGATCGCCCTTCCCAACAGTTGCGCAGCCTGAATGGCGAATGGCGCCTGATGCGGTATTTTCTCCTTACGCATCTGT
GCGGTATTTCACACCGCATATGGTGCACTCTCAGTACAATCTGCTCTGATGCCGCATAGACCTGCAGGGCCAGCAG
GCAGAAGTATGCAAAGCATGCATCTCAATTAGTCAGCAACCAGGTGTGGAAAGTCCCCAGGCTCCCCAGCAGGCA
GAAGTATGCAAAGCATGCATCTCAATTAGTCAGCAACCATAGTCCCGCCCCTAACTCCGCCCATCCCGCCCCTAACT

CCGCCCAGTTCCGCCCATTCTCCGCCCCATGGCTGACTAATTTTTTTTATTTATGCAGAGGCCGAGGCCGCCTCTGCC
TCTGAGCTATTCCAGAAGTAGTGAGGAGGCTTTTTTGGAGGCCTAGGCTTTTGCAAAAAGCTCCCGGGAGCTTGTA
TATCCATTTTCGGATCTGATCAGCACGTGATGAAAAAGCCTGAACTCACCGCGACGTCTGTCGAGAAGTTTCTGATC
GAAAAGTTCGACAGCGTGTCCGACCTGATGCAGCTCTCGGAGGGCGAAGAATCTCGTGCTTTCAGCTTCGATGTAG
GAGGGCGTGGATATGTCCTGCGGGTAAATAGCTGCGCCGATGGTTTCTACAAAGATCGTTATGTTTATCGGCACTT
TGCATCGGCCGCGCTCCCGATTCCGGAAGTGCTTGACATTGGGGAATTCAGCGAGAGCCTGACCTATTGCATCTCC
CGCCGTGCACAGGGTGTCACGTTGCAAGACTTGCCTGAAACCGAACTGCCCGCTGTTCTGCAGCCGGTCGCGGAG
GCCATGGATGCGATCGCTGCGGCCGATCTTAGCCAGACGAGCGGGTTCGGCCCATTCGGACCGCAAGGAATCGGT
CAATACACTACATGGCGTGATTTCATATGCGCGATTGCTGATCCCCATGTGTATCACTGGCAAACTGTGATGGACG
ACACCGTCAGTGCGTCCGTCGCGCAGGCTCTCGATGAGCTGATGCTTTGGGCCGAGGACTGCCCCGAAGTCCGGC
ACCTCGTGCACGCGGATTTCGGCTCCAACAATGTCCTGACGGACAATGGCCGCATAACAGCGGTCATTGACTGGAG
CGAGGCGATGTTCGGGGATTCCCAATACGAGGTCGCCAACATCTTCTTCTGGAGGCCGTGGTTGGCTTGTATGGAG
CAGCAGACGCGCTACTTCGAGCGGAGGCATCCGGAGCTTGCAGGATCGCCGCGGCTCCGGGCGTATATGCTCCGC
ATTGGTCTTGACCAACTCTATCAGAGCTTGGTTGACGGCAATTTCGATGATGCAGCTTGGGCGCAGGGTCGATGCG
ACGCAATCGTCCGATCCGGAGCCGGGACTGTCGGGCGTACACAAATCGCCCGCAGAAGCGCGGCCGTCTGGACCG
ATGGCTGTGTAGAAGTACTCGCCGATAGTGGAAACCGACGCCCCAGCACTCGTCCGAGGGCAAAGGAATAGCACG
TGCTACGAGATTTCGATTCCACCGCCGCCTTCTATGAAAGGTTGGGCTTCGGAATCGTTTTCCGGGACGCCGGCTG
GATGATCCTCCAGCGCGGGGATCTCATGCTGGAGTTCTTCGCCCACCCCAACTTGTTTATTGCAGCTTATAATGGTT
ACAAATAAAGCAATAGCATCACAAATTTCACAAATAAAGCATTTTTTTCACTGCATTCTAGTTGTGGTTTGTCCAAAC
TCATCAATGTATCTTATGCGTCTTCTAGGGTTAAGGTTAGTGTAGAGAAGCAACCGAAGATTGAGAAGACATGGCG
GTAATACGGTTATCCACAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGCATCACAAAAATCGACGC
TCAAGTCAGAGGTGGCGAAACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTTCTTGAAGTGGTGGCCTAACTAC
GGCTACACTAGAAGAACAGTATTTGGTATCTGCGCTCTGCTGAAGCCAGTTACCTTCGGAAAAAGAGTTGGTAGCT
CTTGATCCGGCAAACAAACCACCGCTGGTAGCGGTTTTTTTGTTTGCAAGCAGCAGATTACGCGCAGAAAAAAAGG
ATCTCAAGAAGATCCTTTGATCTTTTCTACGGGGTCTGACGCTCAGTGGAACGAAAACTCACGTTAAGGGATTTTG
GTCATGAGATTATCAAAAAGGATCTTCACCTAGATCCTTTTAAATTAAAAATGAAGTTTTAAATCAATCTAAAGTAT
ATATGAGTAAACTTGGTCTGACAGTTACCAATGCTTAATCAGTGAGGCACCTATCTCAGCGATCTGTCTATTTCGTT

CATCCATAGTTGCCTGACTCCCCGTCGTGTAGATAACTACGATACGGGAGGGCTTACCATCTGGCCCCAGTGCTGC
AATGATACCGCGTGACCCACGCTCACCGGCTCCAGATTTATCAGCAATAAACCAGCCAGCCGGAAGGGCCGAGCG
CAGAAGTGGTCCTGCAACTTTATCCGCCTCCATCCAGTCTATTAATTGTTGCCGGGAAGCTAGAGTAAGTAGTTCGC
CAGTTAATAGTTTGCGCAACGTTGTTGCCATTGCTACAGGCATCGTGGTGTCACGCTCGTCGTTTGGTATGGCTTCA
TTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCACCTATGAGACG

Un vecteur pHCsiaT-EGFP (SEQ ID NO : 31) selon l'invention est construit à partir d'une combinaison des briques Ori-AmpR BsaI A (SEQ ID NO : 17), pCMV BsaI A (SEQ ID NO : 18), SiaT BsaI A (SEQ ID NO : 19), BGHpA BsaI A (SEQ ID NO : 26), LacZa-up BsaI A (SEQ ID NO : 27), LacZa-down BsaI A (SEQ ID NO : 28), HygroR BsaI A (SEQ ID NO : 29) et EGFP BsaI A (SEQ ID NO : 21).

**Vecteur pHCsiaT-EGFP (SEQ ID NO : 31)**

TGAGGCTAGGGATAGGACGAGAGCATCGGGAACGAGGACTAGCGTCTCAAGGAACCAATTCAGTCGACTGGATC
CTAGTTATTAATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATGTTCCCATAGTAA
CGCCAATAGGGACTTTCCATTGACGTCAATGGGTGGAGTATTTACGGTAAACTGCCCACTTGGCAGTACATCAAGT
GTATCATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTTGGCA
GTACATCAATGGGCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGCAAATGGGCGGTA
GGCGTGTACGGTGGGAGGTCTATATAAGCAGAGCTGGTTTAGTGAACCGTCAGATCACTAGTCGACTAGGGATAA
CAGGGCCGCCACCATGATTCACACCAACCTGAAGAAAAAGTTCAGCTGCTGCGTCCTGGTCTTTCTTCTGTTTGCAG
TCATCTGTGTGTGGAAGGAAAAGAAGAAAGGGAGTTACTATGATTCCTTTAAATTGCAAACCAAGGAATTCCAGGT
GTTAAAGAGTCTGGGGAAATTGGCCATGGGGTCTGATTCCCAGTCTGTATCCTCAAGCAGCACCCAGGACCCCCAC

AGGGGCCGCCAGACCCTCGGCAGTCTCAGAGGCCTAGCCAAGGCCAAACCAGAGGCCTCCTTCCAGGTGTGGAAC
AAGGACAGCTCTTCCAAAAACCTTATCCCTAGGCTGCAAAAGGGGTCGGGGGTGAGCAAGGGCGAGGAGCTGTTC
ACCGGGGTGGTGCCCATCCTGGTCGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACCGGCAAGCTGCCCGTGCCCTGGCCC
ACCCTCGTGACCACCCTGACCTACGGCGTGCAGTGCTTCAGCCGCTACCCCGACCACATGAAGCAGCACGACTTCTT
CAAGTCCGCCATGCCCGAAGGCTACGTCCAGGAGCGCACCATCTTCTTCAAGGACGACGGCAACTACAAGACCCGC
GCCGAGGTGAAGTTCGAGGGCGACACCCTGGTGAACCGCATCGAGCTGAAGGGCATCGACTTCAAGGAGGACGG
CAACATCCTGGGGCACAAGCTGGAGTACAACTACAACAGCCACAACGTCTATATCATGGCCGACAAGCAGAAGAA
CGGCATCAAGGTGAACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCACCCAGTCCGCCCTGAGCAAA
GACCCCAACGAGAAGCGCGATCACATGGTCCTGCTGGAGTTCGTGACCGCCGCCGGGATCACTCTCGGCATGGAC
GAGCTGTACAAGTGATCGACTGTGCCTTCTAGTTGCCAGCCATCTGTTGTTTGCCCCTCCCCCGTGCCTTCCTTGACC
CTGGAAGGTGCCACTCCCACTGTCCTTTCCTAATAAAATGAGGAAATTGCATCGCATTGTCTGAGTAGGTGTCATTC
TATTCTGGGGGGTGGGGTGGGGCAGGACAGCAAGGGGGAGGATTGGGAGGACAATAGCAGGCATGCTGGGGAT
GCGGTGGGCTCTATGGCTTCTCCCTGCAGGTGCGCCCAATACGCAAACCGCCTCTCCCCGCGCGTTGGCCGATTCA
TTAATGCAGCTGGCACGACAGGTTTCCCGACTGGAAAGCGGGCAGTGAGCGCAACGCAATTAATGTGAGTTAGCT
CACTCATTAGGCACCCCAGGCTTTACACTTTATGCTTCCGGCTCGTATGTTGTGTGGAATTGTGAGCGGATAACAAT
TTCACACAGGAAACAGCTATGACCATGATTACGGACAGCCTGGCCGTCGTTTTACAACGTCGTGACTGGGAAAACC
CTGGCGTTACCCAACTTAATCGCCTTGCAGCACATCCCCCTTTCGCCAGCTGGCGTAATAGCGAAGAGGCCCGCAC
CGATCGCCCTTCCCAACAGTTGCGCAGCCTGAATGGCGAATGGCGCCTGATGCGGTATTTTCTCCTTACGCATCTGT
GCGGTATTTCACACCGCATATGGTGCACTCTCAGTACAATCTGCTCTGATGCCGCATAGACCTGCAGGGCCAGCAG
GCAGAAGTATGCAAAGCATGCATCTCAATTAGTCAGCAACCAGGTGTGGAAAGTCCCCAGGCTCCCCAGCAGGCA
GAAGTATGCAAAGCATGCATCTCAATTAGTCAGCAACCATAGTCCCGCCCCTAACTCCGCCCATCCCGCCCCTAACT
CCGCCCAGTTCCGCCCATTCTCCGCCCCATGGCTGACTAATTTTTTTTATTTATGCAGAGGCCGAGGCCGCCTCTGCC
TCTGAGCTATTCCAGAAGTAGTGAGGAGGCTTTTTTGGAGGCCTAGGCTTTTGCAAAAAGCTCCCGGGAGCTTGTA
TATCCATTTTCGGATCTGATCAGCACGTGATGAAAAAGCCTGAACTCACCGCGACGTCTGTCGAGAAGTTTCTGATC
GAAAAGTTCGACAGCGTGTCCGACCTGATGCAGCTCTCGGAGGGCGAAGAATCTCGTGCTTTCAGCTTCGATGTAG
GAGGGCGTGGATATGTCCTGCGGGTAAATAGCTGCGCCGATGGTTTCTACAAAGATCGTTATGTTTATCGGCACTT
TGCATCGGCCGCGCTCCCGATTCCGGAAGTGCTTGACATTGGGGAATTCAGCGAGAGCCTGACCTATTGCATCTCC
CGCCGTGCACAGGGTGTCACGTTGCAAGACTTGCCTGAAACCGAACTGCCCGCTGTTCTGCAGCCGGTCGCGGAG
GCCATGGATGCGATCGCTGCGGCCGATCTTAGCCAGACGAGCGGGTTCGGCCCATTCGGACCGCAAGGAATCGGT
CAATACACTACATGGCGTGATTTCATATGCGCGATTGCTGATCCCCATGTGTATCACTGGCAAACTGTGATGGACG

ACACCGTCAGTGCGTCCGTCGCGCAGGCTCTCGATGAGCTGATGCTTTGGGCCGAGGACTGCCCCGAAGTCCGGC
ACCTCGTGCACGCGGATTTCGGCTCCAACAATGTCCTGACGGACAATGGCCGCATAACAGCGGTCATTGACTGGAG
CGAGGCGATGTTCGGGGATTCCCAATACGAGGTCGCCAACATCTTCTTCTGGAGGCCGTGGTTGGCTTGTATGGAG
CAGCAGACGCGCTACTTCGAGCGGAGGCATCCGGAGCTTGCAGGATCGCCGCGGCTCCGGGCGTATATGCTCCGC
ATTGGTCTTGACCAACTCTATCAGAGCTTGGTTGACGGCAATTTCGATGATGCAGCTTGGGCGCAGGGTCGATGCG
ACGCAATCGTCCGATCCGGAGCCGGGACTGTCGGGCGTACACAAATCGCCCGCAGAAGCGCGGCCGTCTGGACCG
ATGGCTGTGTAGAAGTACTCGCCGATAGTGGAAACCGACGCCCCAGCACTCGTCCGAGGGCAAAGGAATAGCACG
TGCTACGAGATTTCGATTCCACCGCCGCCTTCTATGAAAGGTTGGGCTTCGGAATCGTTTTCCGGGACGCCGGCTG
GATGATCCTCCAGCGCGGGGATCTCATGCTGGAGTTCTTCGCCCACCCCAACTTGTTTATTGCAGCTTATAATGGTT
ACAAATAAAGCAATAGCATCACAAATTTCACAAATAAAGCATTTTTTTCACTGCATTCTAGTTGTGGTTTGTCCAAAC
TCATCAATGTATCTTATGCGTCTTCTAGGGTTAAGGTTAGTGTAGAGAAGCAACCGAAGATTGAGAAGACATGGCG
GTAATACGGTTATCCACAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGCATCACAAAAATCGACGC
TCAAGTCAGAGGTGGCGAAACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTTCTTGAAGTGGTGGCCTAACTAC
GGCTACACTAGAAGAACAGTATTTGGTATCTGCGCTCTGCTGAAGCCAGTTACCTTCGGAAAAAGAGTTGGTAGCT
CTTGATCCGGCAAACAAACCACCGCTGGTAGCGGTTTTTTTGTTTGCAAGCAGCAGATTACGCGCAGAAAAAAAGG
ATCTCAAGAAGATCCTTTGATCTTTTCTACGGGGTCTGACGCTCAGTGGAACGAAAACTCACGTTAAGGGATTTTG
GTCATGAGATTATCAAAAAGGATCTTCACCTAGATCCTTTTAAATTAAAAATGAAGTTTTAAATCAATCTAAAGTAT
ATATGAGTAAACTTGGTCTGACAGTTACCAATGCTTAATCAGTGAGGCACCTATCTCAGCGATCTGTCTATTTCGTT
CATCCATAGTTGCCTGACTCCCCGTCGTGTAGATAACTACGATACGGGAGGGCTTACCATCTGGCCCCAGTGCTGC
AATGATACCGCGTGACCCACGCTCACCGGCTCCAGATTTATCAGCAATAAACCAGCCAGCCGGAAGGGCCGAGCG
CAGAAGTGGTCCTGCAACTTTATCCGCCTCCATCCAGTCTATTAATTGTTGCCGGGAAGCTAGAGTAAGTAGTTCGC
CAGTTAATAGTTTGCGCAACGTTGTTGCCATTGCTACAGGCATCGTGGTGTCACGCTCGTCGTTTGGTATGGCTTCA
TTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC

GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCACCTATGAGACG

Un vecteur pHCsiaT-ECFP (SEQ ID NO : 32) est construit à partir d'une combinaison des briques Ori-AmpR BsaI A (SEQ ID NO : 17), pCMV BsaI A (SEQ ID NO : 18), SiaT BsaI A (SEQ ID NO : 19), BGHpA BsaI A (SEQ ID NO : 26), LacZa-up BsaI A (SEQ ID NO : 27), LacZa-down BsaI A (SEQ ID NO : 28), HygroR BsaI A (SEQ ID NO : 29) et ECFP BsaI A (SEQ ID NO : 22).

**Vecteur pHCsiaT-ECFP (SEQ ID NO : 32)**

TGAGGCTAGGGATAGGACGAGAGCATCGGGAACGAGGACTAGCGTCTCAAGGAACCAATTCAGTCGACTGGATC
CTAGTTATTAATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATGTTCCCATAGTAA
CGCCAATAGGGACTTTCCATTGACGTCAATGGGTGGAGTATTTACGGTAAACTGCCCACTTGGCAGTACATCAAGT
GTATCATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTTGGCA
GTACATCAATGGGCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGCAAATGGGCGGTA
GGCGTGTACGGTGGGAGGTCTATATAAGCAGAGCTGGTTTAGTGAACCGTCAGATCACTAGTCGACTAGGGATAA
CAGGGCCGCCACCATGATTCACACCAACCTGAAGAAAAAGTTCAGCTGCTGCGTCCTGGTCTTTCTTCTGTTTGCAG
TCATCTGTGTGTGGAAGGAAAAGAAGAAAGGGAGTTACTATGATTCCTTTAAATTGCAAACCAAGGAATTCCAGGT
GTTAAAGAGTCTGGGGAAATTGGCCATGGGGTCTGATTCCCAGTCTGTATCCTCAAGCAGCACCCAGGACCCCCAC
AGGGGCCGCCAGACCCTCGGCAGTCTCAGAGGCCTAGCCAAGGCCAAACCAGAGGCCTCCTTCCAGGTGTGGAAC
AAGGACAGCTCTTCCAAAAACCTTATCCCTAGGCTGCAAAAGGGGTCGGGGGTGAGCAAGGGCGAGGAGCTGTTC
ACCGGGGTGGTGCCCATCCTGGTCGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACCGGCAAGCTGCCCGTGCCCTGGCCC
ACCCTCGTGACCACCCTGACCTGGGGCGTGCAGTGCTTCAGCCGCTACCCCGACCACATGAAGCAGCACGACTTCT
TCAAGTCCGCCATGCCCGAAGGCTACGTCCAGGAGCGCACCATCTTCTTCAAGGACGACGGCAACTACAAGACCCG
CGCCGAGGTGAAGTTCGAGGGCGACACCCTGGTGAACCGCATCGAGCTGAAGGGCATCGACTTCAAGGAGGACG
GCAACATCCTGGGGCACAAGCTGGAGTACAACTACATCAGCCACAACGTCTATATCACCGCCGACAAGCAGAAGA
ACGGCATCAAGGCCAACTTCAAGATCCGCCACAACATCGAGGACGGCAGCGTGCAGCTCGCCGACCACTACCAGC

AGAACACCCCCATCGGCGACGGCCCCGTGCTGCTGCCCGACAACCACTACCTGAGCACCCAGTCCGCCCTGAGCAA
AGACCCCAACGAGAAGCGCGATCACATGGTCCTGCTGGAGTTCGTGACCGCCGCCGGGATCACTCTCGGCATGGA
CGAGCTGTACAAGTGATCGACTGTGCCTTCTAGTTGCCAGCCATCTGTTGTTTGCCCCTCCCCCGTGCCTTCCTTGAC
CCTGGAAGGTGCCACTCCCACTGTCCTTTCCTAATAAAATGAGGAAATTGCATCGCATTGTCTGAGTAGGTGTCATT
CTATTCTGGGGGGTGGGGTGGGGCAGGACAGCAAGGGGGAGGATTGGGAGGACAATAGCAGGCATGCTGGGG
ATGCGGTGGGCTCTATGGCTTCTCCCTGCAGGTGCGCCCAATACGCAAACCGCCTCTCCCCGCGCGTTGGCCGATT
CATTAATGCAGCTGGCACGACAGGTTTCCCGACTGGAAAGCGGGCAGTGAGCGCAACGCAATTAATGTGAGTTAG
CTCACTCATTAGGCACCCCAGGCTTTACACTTTATGCTTCCGGCTCGTATGTTGTGTGGAATTGTGAGCGGATAACA
ATTTCACACAGGAAACAGCTATGACCATGATTACGGACAGCCTGGCCGTCGTTTTACAACGTCGTGACTGGGAAAA
CCCTGGCGTTACCCAACTTAATCGCCTTGCAGCACATCCCCCTTTCGCCAGCTGGCGTAATAGCGAAGAGGCCCGC
ACCGATCGCCCTTCCCAACAGTTGCGCAGCCTGAATGGCGAATGGCGCCTGATGCGGTATTTTCTCCTTACGCATCT
GTGCGGTATTTCACACCGCATATGGTGCACTCTCAGTACAATCTGCTCTGATGCCGCATAGACCTGCAGGGCCAGC
AGGCAGAAGTATGCAAAGCATGCATCTCAATTAGTCAGCAACCAGGTGTGGAAAGTCCCCAGGCTCCCCAGCAGG
CAGAAGTATGCAAAGCATGCATCTCAATTAGTCAGCAACCATAGTCCCGCCCCTAACTCCGCCCATCCCGCCCCTAA
CTCCGCCCAGTTCCGCCCATTCTCCGCCCCATGGCTGACTAATTTTTTTTATTTATGCAGAGGCCGAGGCCGCCTCTG
CCTCTGAGCTATTCCAGAAGTAGTGAGGAGGCTTTTTTGGAGGCCTAGGCTTTTGCAAAAAGCTCCCGGGAGCTTG
TATATCCATTTTCGGATCTGATCAGCACGTGATGAAAAAGCCTGAACTCACCGCGACGTCTGTCGAGAAGTTTCTGA
TCGAAAAGTTCGACAGCGTGTCCGACCTGATGCAGCTCTCGGAGGGCGAAGAATCTCGTGCTTTCAGCTTCGATGT
AGGAGGGCGTGGATATGTCCTGCGGGTAAATAGCTGCGCCGATGGTTTCTACAAAGATCGTTATGTTTATCGGCAC
TTTGCATCGGCCGCGCTCCCGATTCCGGAAGTGCTTGACATTGGGGAATTCAGCGAGAGCCTGACCTATTGCATCT
CCCGCCGTGCACAGGGTGTCACGTTGCAAGACTTGCCTGAAACCGAACTGCCCGCTGTTCTGCAGCCGGTCGCGGA
GGCCATGGATGCGATCGCTGCGGCCGATCTTAGCCAGACGAGCGGGTTCGGCCCATTCGGACCGCAAGGAATCGG
TCAATACACTACATGGCGTGATTTCATATGCGCGATTGCTGATCCCCATGTGTATCACTGGCAAACTGTGATGGACG
ACACCGTCAGTGCGTCCGTCGCGCAGGCTCTCGATGAGCTGATGCTTTGGGCCGAGGACTGCCCCGAAGTCCGGC
ACCTCGTGCACGCGGATTTCGGCTCCAACAATGTCCTGACGGACAATGGCCGCATAACAGCGGTCATTGACTGGAG
CGAGGCGATGTTCGGGGATTCCCAATACGAGGTCGCCAACATCTTCTTCTGGAGGCCGTGGTTGGCTTGTATGGAG
CAGCAGACGCGCTACTTCGAGCGGAGGCATCCGGAGCTTGCAGGATCGCCGCGGCTCCGGGCGTATATGCTCCGC
ATTGGTCTTGACCAACTCTATCAGAGCTTGGTTGACGGCAATTTCGATGATGCAGCTTGGGCGCAGGGTCGATGCG
ACGCAATCGTCCGATCCGGAGCCGGGACTGTCGGGCGTACACAAATCGCCCGCAGAAGCGCGGCCGTCTGGACCG
ATGGCTGTGTAGAAGTACTCGCCGATAGTGGAAACCGACGCCCCAGCACTCGTCCGAGGGCAAAGGAATAGCACG
TGCTACGAGATTTCGATTCCACCGCCGCCTTCTATGAAAGGTTGGGCTTCGGAATCGTTTTCCGGGACGCCGGCTG
GATGATCCTCCAGCGCGGGGATCTCATGCTGGAGTTCTTCGCCCACCCCAACTTGTTTATTGCAGCTTATAATGGTT

ACAAATAAAGCAATAGCATCACAAATTTCACAAATAAAGCATTTTTTTCACTGCATTCTAGTTGTGGTTTGTCCAAAC
TCATCAATGTATCTTATGCGTCTTCTAGGGTTAAGGTTAGTGTAGAGAAGCAACCGAAGATTGAGAAGACATGGCG
GTAATACGGTTATCCACAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGCATCACAAAAATCGACGC
TCAAGTCAGAGGTGGCGAAACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTTCTTGAAGTGGTGGCCTAACTAC
GGCTACACTAGAAGAACAGTATTTGGTATCTGCGCTCTGCTGAAGCCAGTTACCTTCGGAAAAAGAGTTGGTAGCT
CTTGATCCGGCAAACAAACCACCGCTGGTAGCGGTTTTTTTGTTTGCAAGCAGCAGATTACGCGCAGAAAAAAAGG
ATCTCAAGAAGATCCTTTGATCTTTTCTACGGGGTCTGACGCTCAGTGGAACGAAAACTCACGTTAAGGGATTTTG
GTCATGAGATTATCAAAAAGGATCTTCACCTAGATCCTTTTAAATTAAAAATGAAGTTTTAAATCAATCTAAAGTAT
ATATGAGTAAACTTGGTCTGACAGTTACCAATGCTTAATCAGTGAGGCACCTATCTCAGCGATCTGTCTATTTCGTT
CATCCATAGTTGCCTGACTCCCCGTCGTGTAGATAACTACGATACGGGAGGGCTTACCATCTGGCCCCAGTGCTGC
AATGATACCGCGTGACCCACGCTCACCGGCTCCAGATTTATCAGCAATAAACCAGCCAGCCGGAAGGGCCGAGCG
CAGAAGTGGTCCTGCAACTTTATCCGCCTCCATCCAGTCTATTAATTGTTGCCGGGAAGCTAGAGTAAGTAGTTCGC
CAGTTAATAGTTTGCGCAACGTTGTTGCCATTGCTACAGGCATCGTGGTGTCACGCTCGTCGTTTGGTATGGCTTCA
TTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCACCTATGAGACG

Un vecteur pHCsiaT-EYGFP (SEQ ID NO : 33) est construit à partir d'une combinaison des briques Ori-AmpR BsaI A (SEQ ID NO : 17), pCMV BsaI A (SEQ ID NO : 18), SiaT BsaI A (SEQ ID NO : 19), BGHpA BsaI A (SEQ ID NO : 26), LacZa-up BsaI A (SEQ ID NO : 27), LacZa-down BsaI A (SEQ ID NO : 28), HygroR BsaI A (SEQ ID NO : 29) et EYFP BsaI A (SEQ ID NO : 23).

**Vecteur pHCsiaT-EYFP (SEQ ID NO : 33)**

TGAGGCTAGGGATAGGACGAGAGCATCGGGAACGAGGACTAGCGTCTCAAGGAACCAATTCAGTCGACTGGATC
CTAGTTATTAATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATGTTCCCATAGTAA
CGCCAATAGGGACTTTCCATTGACGTCAATGGGTGGAGTATTTACGGTAAACTGCCCACTTGGCAGTACATCAAGT
GTATCATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTTGGCA
GTACATCAATGGGCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGCAAATGGGCGGTA
GGCGTGTACGGTGGGAGGTCTATATAAGCAGAGCTGGTTTAGTGAACCGTCAGATCACTAGTCGACTAGGGATAA
CAGGGCCGCCACCATGATTCACACCAACCTGAAGAAAAAGTTCAGCTGCTGCGTCCTGGTCTTTCTTCTGTTTGCAG
TCATCTGTGTGTGGAAGGAAAAGAAGAAAGGGAGTTACTATGATTCCTTTAAATTGCAAACCAAGGAATTCCAGGT
GTTAAAGAGTCTGGGGAAATTGGCCATGGGGTCTGATTCCCAGTCTGTATCCTCAAGCAGCACCCAGGACCCCCAC
AGGGGCCGCCAGACCCTCGGCAGTCTCAGAGGCCTAGCCAAGGCCAAACCAGAGGCCTCCTTCCAGGTGTGGAAC
AAGGACAGCTCTTCCAAAAACCTTATCCCTAGGCTGCAAAAGGGGTCGGGGGTGAGCAAGGGCGAGGAGCTGTTC
ACCGGGGTGGTGCCCATCCTGGTCGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACCGGCAAGCTGCCCGTGCCCTGGCCC
ACCCTCGTGACCACCTTCGGCTACGGCCTGCAGTGCTTCGCCCGCTACCCCGACCACATGAAGCAGCACGACTTCTT
CAAGTCCGCCATGCCCGAAGGCTACGTCCAGGAGCGCACCATCTTCTTCAAGGACGACGGCAACTACAAGACCCGC
GCCGAGGTGAAGTTCGAGGGCGACACCCTGGTGAACCGCATCGAGCTGAAGGGCATCGACTTCAAGGAGGACGG
CAACATCCTGGGGCACAAGCTGGAGTACAACTACAACAGCCACAACGTCTATATCATGGCCGACAAGCAGAAGAA
CGGCATCAAGGTGAACTTCAAGATCCGCCACAACATCGAGGACGGCAGCGTGCAGCTCGCCGACCACTACCAGCA
GAACACCCCCATCGGCGACGGCCCCGTGCTGCTGCCCGACAACCACTACCTGAGCTACCAGTCCGCCCTGAGCAAA
GACCCCAACGAGAAGCGCGATCACATGGTCCTGCTGGAGTTCGTGACCGCCGCCGGGATCACTCTCGGCATGGAC
GAGCTGTACAAGTGATCGACTGTGCCTTCTAGTTGCCAGCCATCTGTTGTTTGCCCCTCCCCCGTGCCTTCCTTGACC
CTGGAAGGTGCCACTCCCACTGTCCTTTCCTAATAAAATGAGGAAATTGCATCGCATTGTCTGAGTAGGTGTCATTC
TATTCTGGGGGGTGGGGTGGGGCAGGACAGCAAGGGGGAGGATTGGGAGGACAATAGCAGGCATGCTGGGGAT
GCGGTGGGCTCTATGGCTTCTCCCTGCAGGTGCGCCCAATACGCAAACCGCCTCTCCCCGCGCGTTGGCCGATTCA
TTAATGCAGCTGGCACGACAGGTTTCCCGACTGGAAAGCGGGCAGTGAGCGCAACGCAATTAATGTGAGTTAGCT
CACTCATTAGGCACCCCAGGCTTTACACTTTATGCTTCCGGCTCGTATGTTGTGTGGAATTGTGAGCGGATAACAAT
TTCACACAGGAAACAGCTATGACCATGATTACGGACAGCCTGGCCGTCGTTTTACAACGTCGTGACTGGGAAAACC

CTGGCGTTACCCAACTTAATCGCCTTGCAGCACATCCCCCTTTCGCCAGCTGGCGTAATAGCGAAGAGGCCCGCAC
CGATCGCCCTTCCCAACAGTTGCGCAGCCTGAATGGCGAATGGCGCCTGATGCGGTATTTTCTCCTTACGCATCTGT
GCGGTATTTCACACCGCATATGGTGCACTCTCAGTACAATCTGCTCTGATGCCGCATAGACCTGCAGGGCCAGCAG
GCAGAAGTATGCAAAGCATGCATCTCAATTAGTCAGCAACCAGGTGTGGAAAGTCCCCAGGCTCCCCAGCAGGCA
GAAGTATGCAAAGCATGCATCTCAATTAGTCAGCAACCATAGTCCCGCCCCTAACTCCGCCCATCCCGCCCCTAACT
CCGCCCAGTTCCGCCCATTCTCCGCCCCATGGCTGACTAATTTTTTTTATTTATGCAGAGGCCGAGGCCGCCTCTGCC
TCTGAGCTATTCCAGAAGTAGTGAGGAGGCTTTTTTGGAGGCCTAGGCTTTTGCAAAAAGCTCCCGGGAGCTTGTA
TATCCATTTTCGGATCTGATCAGCACGTGATGAAAAAGCCTGAACTCACCGCGACGTCTGTCGAGAAGTTTCTGATC
GAAAAGTTCGACAGCGTGTCCGACCTGATGCAGCTCTCGGAGGGCGAAGAATCTCGTGCTTTCAGCTTCGATGTAG
GAGGGCGTGGATATGTCCTGCGGGTAAATAGCTGCGCCGATGGTTTCTACAAAGATCGTTATGTTTATCGGCACTT
TGCATCGGCCGCGCTCCCGATTCCGGAAGTGCTTGACATTGGGGAATTCAGCGAGAGCCTGACCTATTGCATCTCC
CGCCGTGCACAGGGTGTCACGTTGCAAGACTTGCCTGAAACCGAACTGCCCGCTGTTCTGCAGCCGGTCGCGGAG
GCCATGGATGCGATCGCTGCGGCCGATCTTAGCCAGACGAGCGGGTTCGGCCCATTCGGACCGCAAGGAATCGGT
CAATACACTACATGGCGTGATTTCATATGCGCGATTGCTGATCCCCATGTGTATCACTGGCAAACTGTGATGGACG
ACACCGTCAGTGCGTCCGTCGCGCAGGCTCTCGATGAGCTGATGCTTTGGGCCGAGGACTGCCCCGAAGTCCGGC
ACCTCGTGCACGCGGATTTCGGCTCCAACAATGTCCTGACGGACAATGGCCGCATAACAGCGGTCATTGACTGGAG
CGAGGCGATGTTCGGGGATTCCCAATACGAGGTCGCCAACATCTTCTTCTGGAGGCCGTGGTTGGCTTGTATGGAG
CAGCAGACGCGCTACTTCGAGCGGAGGCATCCGGAGCTTGCAGGATCGCCGCGGCTCCGGGCGTATATGCTCCGC
ATTGGTCTTGACCAACTCTATCAGAGCTTGGTTGACGGCAATTTCGATGATGCAGCTTGGGCGCAGGGTCGATGCG
ACGCAATCGTCCGATCCGGAGCCGGGACTGTCGGGCGTACACAAATCGCCCGCAGAAGCGCGGCCGTCTGGACCG
ATGGCTGTGTAGAAGTACTCGCCGATAGTGGAAACCGACGCCCCAGCACTCGTCCGAGGGCAAAGGAATAGCACG
TGCTACGAGATTTCGATTCCACCGCCGCCTTCTATGAAAGGTTGGGCTTCGGAATCGTTTTCCGGGACGCCGGCTG
GATGATCCTCCAGCGCGGGGATCTCATGCTGGAGTTCTTCGCCCACCCCAACTTGTTTATTGCAGCTTATAATGGTT
ACAAATAAAGCAATAGCATCACAAATTTCACAAATAAAGCATTTTTTTCACTGCATTCTAGTTGTGGTTTGTCCAAAC
TCATCAATGTATCTTATGCGTCTTCTAGGGTTAAGGTTAGTGTAGAGAAGCAACCGAAGATTGAGAAGACATGGCG
GTAATACGGTTATCCACAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGCATCACAAAAATCGACGC
TCAAGTCAGAGGTGGCGAAACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTTCTTGAAGTGGTGGCCTAACTAC

GGCTACACTAGAAGAACAGTATTTGGTATCTGCGCTCTGCTGAAGCCAGTTACCTTCGGAAAAAGAGTTGGTAGCT
CTTGATCCGGCAAACAAACCACCGCTGGTAGCGGTTTTTTTGTTTGCAAGCAGCAGATTACGCGCAGAAAAAAAGG
ATCTCAAGAAGATCCTTTGATCTTTTCTACGGGGTCTGACGCTCAGTGGAACGAAAACTCACGTTAAGGGATTTTG
GTCATGAGATTATCAAAAAGGATCTTCACCTAGATCCTTTTAAATTAAAAATGAAGTTTTAAATCAATCTAAAGTAT
ATATGAGTAAACTTGGTCTGACAGTTACCAATGCTTAATCAGTGAGGCACCTATCTCAGCGATCTGTCTATTTCGTT
CATCCATAGTTGCCTGACTCCCCGTCGTGTAGATAACTACGATACGGGAGGGCTTACCATCTGGCCCCAGTGCTGC
AATGATACCGCGTGACCCACGCTCACCGGCTCCAGATTTATCAGCAATAAACCAGCCAGCCGGAAGGGCCGAGCG
CAGAAGTGGTCCTGCAACTTTATCCGCCTCCATCCAGTCTATTAATTGTTGCCGGGAAGCTAGAGTAAGTAGTTCGC
CAGTTAATAGTTTGCGCAACGTTGTTGCCATTGCTACAGGCATCGTGGTGTCACGCTCGTCGTTTGGTATGGCTTCA
TTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCACCTATGAGACG

Un vecteur pHCsiaT-mCherry (SEQ ID NO : 34) est construit à partir d'une combinaison des briques Ori-AmpR BsaI A (SEQ ID NO : 17), pCMV BsaI A (SEQ ID NO : 18), SiaT BsaI A (SEQ ID NO : 19), BGHpA BsaI A (SEQ ID NO : 26), LacZa-up BsaI A (SEQ ID NO : 27), LacZa-down BsaI A (SEQ ID NO : 28), HygroR BsaI A (SEQ ID NO : 29) et mCherry BsaI A (SEQ ID NO : 24).

**Vecteur pHCsiaT-mCherry (SEQ ID NO : 34)**

TGAGGCTAGGGATAGGACGAGAGCATCGGGAACGAGGACTAGCGTCTCAAGGAACCAATTCAGTCGACTGGATC
CTAGTTATTAATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATGTTCCCATAGTAA
CGCCAATAGGGACTTTCCATTGACGTCAATGGGTGGAGTATTTACGGTAAACTGCCCACTTGGCAGTACATCAAGT
GTATCATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTTGGCA
GTACATCAATGGGCGTGGATAGCGGTTTGACTCACGGGGATTTCCAAGTCTCCACCCCATTGACGTCAATGGGAGT

TTGTTTTGGCACCAAAATCAACGGGACTTTCCAAAATGTCGTAACAACTCCGCCCCATTGACGCAAATGGGCGGTA
GGCGTGTACGGTGGGAGGTCTATATAAGCAGAGCTGGTTTAGTGAACCGTCAGATCACTAGTCGACTAGGGATAA
CAGGGCCGCCACCATGATTCACACCAACCTGAAGAAAAAGTTCAGCTGCTGCGTCCTGGTCTTTCTTCTGTTTGCAG
TCATCTGTGTGTGGAAGGAAAAGAAGAAAGGGAGTTACTATGATTCCTTTAAATTGCAAACCAAGGAATTCCAGGT
GTTAAAGAGTCTGGGGAAATTGGCCATGGGGTCTGATTCCCAGTCTGTATCCTCAAGCAGCACCCAGGACCCCCAC
AGGGGCCGCCAGACCCTCGGCAGTCTCAGAGGCCTAGCCAAGGCCAAACCAGAGGCCTCCTTCCAGGTGTGGAAC
AAGGACAGCTCTTCCAAAAACCTTATCCCTAGGCTGCAAAAGGGGTCGGGGGTGAGCAAGGGCGAGGAGGATAA
CATGGCCATCATCAAGGAGTTCATGCGCTTCAAGGTGCACATGGAGGGCTCCGTGAACGGCCACGAGTTCGAGAT
CGAGGGCGAGGGCGAGGGCCGCCCCTACGAGGGCACCCAGACCGCCAAGCTGAAGGTGACCAAGGGTGGCCCC
CTGCCCTTCGCCTGGGACATCCTGTCCCCTCAGTTCATGTACGGCTCCAAGGCCTACGTGAAGCACCCCGCCGACAT
CCCCGACTACTTGAAGCTGTCCTTCCCCGAGGGCTTCAAGTGGGAGCGCGTGATGAACTTCGAGGACGGCGGCGT
GGTGACCGTGACCCAGGACTCCTCCCTGCAGGACGGCGAGTTCATCTACAAGGTGAAGCTGCGCGGCACCAACTT
CCCCTCCGACGGCCCCGTAATGCAGAAGAAAACCATGGGCTGGGAGGCCTCCTCCGAGCGGATGTACCCCGAGGA
CGGCGCCCTGAAGGGCGAGATCAAGCAGAGGCTGAAGCTGAAGGACGGCGGCCACTACGACGCTGAGGTCAAG
ACCACCTACAAGGCCAAGAAGCCCGTGCAGCTGCCCGGCGCCTACAACGTCAACATCAAGTTGGACATCACCTCCC
ACAACGAGGACTACACCATCGTGGAACAGTACGAACGCGCCGAGGGCCGCCACTCCACCGGCGGCATGGACGAG
CTGTACAAGTGATCGACTGTGCCTTCTAGTTGCCAGCCATCTGTTGTTTGCCCCTCCCCCGTGCCTTCCTTGACCCTG
GAAGGTGCCACTCCCACTGTCCTTTCCTAATAAAATGAGGAAATTGCATCGCATTGTCTGAGTAGGTGTCATTCTAT
TCTGGGGGGTGGGGTGGGGCAGGACAGCAAGGGGGAGGATTGGGAGGACAATAGCAGGCATGCTGGGGATGC
GGTGGGCTCTATGGCTTCTCCCTGCAGGTGCGCCCAATACGCAAACCGCCTCTCCCCGCGCGTTGGCCGATTCATTA
ATGCAGCTGGCACGACAGGTTTCCCGACTGGAAAGCGGGCAGTGAGCGCAACGCAATTAATGTGAGTTAGCTCAC
TCATTAGGCACCCCAGGCTTTACACTTTATGCTTCCGGCTCGTATGTTGTGTGGAATTGTGAGCGGATAACAATTTC
ACACAGGAAACAGCTATGACCATGATTACGGACAGCCTGGCCGTCGTTTTACAACGTCGTGACTGGGAAAACCCTG
GCGTTACCCAACTTAATCGCCTTGCAGCACATCCCCCTTTCGCCAGCTGGCGTAATAGCGAAGAGGCCCGCACCGA
TCGCCCTTCCCAACAGTTGCGCAGCCTGAATGGCGAATGGCGCCTGATGCGGTATTTTCTCCTTACGCATCTGTGCG
GTATTTCACACCGCATATGGTGCACTCTCAGTACAATCTGCTCTGATGCCGCATAGACCTGCAGGGCCAGCAGGCA
GAAGTATGCAAAGCATGCATCTCAATTAGTCAGCAACCAGGTGTGGAAAGTCCCCAGGCTCCCCAGCAGGCAGAA
GTATGCAAAGCATGCATCTCAATTAGTCAGCAACCATAGTCCCGCCCCTAACTCCGCCCATCCCGCCCCTAACTCCG
CCCAGTTCCGCCCATTCTCCGCCCCATGGCTGACTAATTTTTTTTATTTATGCAGAGGCCGAGGCCGCCTCTGCCTCT
GAGCTATTCCAGAAGTAGTGAGGAGGCTTTTTTGGAGGCCTAGGCTTTTGCAAAAAGCTCCCGGGAGCTTGTATAT
CCATTTTCGGATCTGATCAGCACGTGATGAAAAAGCCTGAACTCACCGCGACGTCTGTCGAGAAGTTTCTGATCGA
AAAGTTCGACAGCGTGTCCGACCTGATGCAGCTCTCGGAGGGCGAAGAATCTCGTGCTTTCAGCTTCGATGTAGGA

GGGCGTGGATATGTCCTGCGGGTAAATAGCTGCGCCGATGGTTTCTACAAAGATCGTTATGTTTATCGGCACTTTG
CATCGGCCGCGCTCCCGATTCCGGAAGTGCTTGACATTGGGGAATTCAGCGAGAGCCTGACCTATTGCATCTCCCG
CCGTGCACAGGGTGTCACGTTGCAAGACTTGCCTGAAACCGAACTGCCCGCTGTTCTGCAGCCGGTCGCGGAGGC
CATGGATGCGATCGCTGCGGCCGATCTTAGCCAGACGAGCGGGTTCGGCCCATTCGGACCGCAAGGAATCGGTCA
ATACACTACATGGCGTGATTTCATATGCGCGATTGCTGATCCCCATGTGTATCACTGGCAAACTGTGATGGACGAC
ACCGTCAGTGCGTCCGTCGCGCAGGCTCTCGATGAGCTGATGCTTTGGGCCGAGGACTGCCCCGAAGTCCGGCAC
CTCGTGCACGCGGATTTCGGCTCCAACAATGTCCTGACGGACAATGGCCGCATAACAGCGGTCATTGACTGGAGC
GAGGCGATGTTCGGGGATTCCCAATACGAGGTCGCCAACATCTTCTTCTGGAGGCCGTGGTTGGCTTGTATGGAGC
AGCAGACGCGCTACTTCGAGCGGAGGCATCCGGAGCTTGCAGGATCGCCGCGGCTCCGGGCGTATATGCTCCGCA
TTGGTCTTGACCAACTCTATCAGAGCTTGGTTGACGGCAATTTCGATGATGCAGCTTGGGCGCAGGGTCGATGCGA
CGCAATCGTCCGATCCGGAGCCGGGACTGTCGGGCGTACACAAATCGCCCGCAGAAGCGCGGCCGTCTGGACCGA
TGGCTGTGTAGAAGTACTCGCCGATAGTGGAAACCGACGCCCCAGCACTCGTCCGAGGGCAAAGGAATAGCACGT
GCTACGAGATTTCGATTCCACCGCCGCCTTCTATGAAAGGTTGGGCTTCGGAATCGTTTTCCGGGACGCCGGCTGG
ATGATCCTCCAGCGCGGGGATCTCATGCTGGAGTTCTTCGCCCACCCCAACTTGTTTATTGCAGCTTATAATGGTTA
CAAATAAAGCAATAGCATCACAAATTTCACAAATAAAGCATTTTTTTCACTGCATTCTAGTTGTGGTTTGTCCAAACT
CATCAATGTATCTTATGCGTCTTCTAGGGTTAAGGTTAGTGTAGAGAAGCAACCGAAGATTGAGAAGACATGGCG
GTAATACGGTTATCCACAGAATCAGGGGATAACGCAGGAAAGAACATGTGAGCAAAAGGCCAGCAAAAGGCCAG
GAACCGTAAAAAGGCCGCGTTGCTGGCGTTTTTCCATAGGCTCCGCCCCCCTGACGAGCATCACAAAAATCGACGC
TCAAGTCAGAGGTGGCGAAACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAGCGTGGCGCTTTCTCATAGCT
CACGCTGTAGGTATCTCAGTTCGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTTCTTGAAGTGGTGGCCTAACTAC
GGCTACACTAGAAGAACAGTATTTGGTATCTGCGCTCTGCTGAAGCCAGTTACCTTCGGAAAAAGAGTTGGTAGCT
CTTGATCCGGCAAACAAACCACCGCTGGTAGCGGTTTTTTTGTTTGCAAGCAGCAGATTACGCGCAGAAAAAAAGG
ATCTCAAGAAGATCCTTTGATCTTTTCTACGGGGTCTGACGCTCAGTGGAACGAAAACTCACGTTAAGGGATTTTG
GTCATGAGATTATCAAAAAGGATCTTCACCTAGATCCTTTTAAATTAAAAATGAAGTTTTAAATCAATCTAAAGTAT
ATATGAGTAAACTTGGTCTGACAGTTACCAATGCTTAATCAGTGAGGCACCTATCTCAGCGATCTGTCTATTTCGTT
CATCCATAGTTGCCTGACTCCCCGTCGTGTAGATAACTACGATACGGGAGGGCTTACCATCTGGCCCCAGTGCTGC
AATGATACCGCGTGACCCACGCTCACCGGCTCCAGATTTATCAGCAATAAACCAGCCAGCCGGAAGGGCCGAGCG
CAGAAGTGGTCCTGCAACTTTATCCGCCTCCATCCAGTCTATTAATTGTTGCCGGGAAGCTAGAGTAAGTAGTTCGC
CAGTTAATAGTTTGCGCAACGTTGTTGCCATTGCTACAGGCATCGTGGTGTCACGCTCGTCGTTTGGTATGGCTTCA

TTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCACCTATGAGACG

Un vecteur pHCsiaT-TagBFP (SEQ ID NO : 35) est construit à partir d'une combinaison des briques Ori-AmpR BsaI A (SEQ ID NO : 17), pCMV BsaI A (SEQ ID NO : 18), SiaT BsaI A (SEQ ID NO : 19), BGHpA BsaI A (SEQ ID NO : 26), LacZa-up BsaI A (SEQ ID NO : 27), LacZa-down BsaI A (SEQ ID NO : 28), HygroR BsaI A (SEQ ID NO : 29) et TagBFP BsaI A (SEQ ID NO : 25).

**Vecteur pHCsiaT-TagBFP (SEQ ID NO : 35)**

TGAGGCTAGGGATAGGACGAGAGCATCGGGAACGAGGACTAGCGTCTCAAGGAACCAATTCAGTCGACTGGATC
CTAGTTATTAATAGTAATCAATTACGGGGTCATTAGTTCATAGCCCATATATGGAGTTCCGCGTTACATAACTTACG
GTAAATGGCCCGCCTGGCTGACCGCCCAACGACCCCCGCCCATTGACGTCAATAATGACGTATGTTCCCATAGTAA
CGCCAATAGGGACTTTCCATTGACGTCAATGGGTGGAGTATTTACGGTAAACTGCCCACTTGGCAGTACATCAAGT
GTATCATATGCCAAGTACGCCCCCTATTGACGTCAATGACGGTAAATGGCCCGCCTGGCATTATGCCCAGTACATG
ACCTTATGGGACTTTCCTACTTGGCAGTACATCTACGTATTAGTCATCGCTATTACCATGGTGATGCGGTTTTGGCA
GTACATCAATGGGCGTGGATAGCGGTTTGACTCACGGGGATTTCCAAGTCTCCACCCCATTGACGTCAATGGGAGT
TTGTTTTGGCACCAAAATCAACGGGACTTTCCAAAATGTCGTAACAACTCCGCCCCATTGACGCAAATGGGCGGTA
GGCGTGTACGGTGGGAGGTCTATATAAGCAGAGCTGGTTTAGTGAACCGTCAGATCACTAGTCGACTAGGGATAA
CAGGGCCGCCACCATGATTCACACCAACCTGAAGAAAAAGTTCAGCTGCTGCGTCCTGGTCTTTCTTCTGTTTGCAG
TCATCTGTGTGTGGAAGGAAAAGAAGAAAGGGAGTTACTATGATTCCTTTAAATTGCAAACCAAGGAATTCCAGGT
GTTAAAGAGTCTGGGGAAATTGGCCATGGGGTCTGATTCCCAGTCTGTATCCTCAAGCAGCACCCAGGACCCCCAC
AGGGGCCGCCAGACCCTCGGCAGTCTCAGAGGCCTAGCCAAGGCCAAACCAGAGGCCTCCTTCCAGGTGTGGAAC
AAGGACAGCTCTTCCAAAAACCTTATCCCTAGGCTGCAAAAGGGGTCGGGGAGCGAGCTGATTAAGGAGAACATG
CACATGAAGCTGTACATGGAGGGCACCGTGGACAACCATCACTTCAAGTGCACATCCGAGGGCGAAGGCAAGCCC
TACGAGGGCACCCAGACCATGAGAATCAAGGTGGTCGAGGGCGGCCCTCTCCCCTTCGCCTTCGACATCCTGGCTA

CTAGCTTCCTCTACGGCAGCAAGACCTTCATCAACCACACCCAGGGCATCCCCGACTTCTTCAAGCAGTCCTTCCCT
GAGGGCTTCACATGGGAGAGAGTCACCACATACGAGGACGGGGGCGTGCTGACCGCTACCCAGGACACCAGCCT
CCAGGACGGCTGCCTCATCTACAACGTCAAGATCAGAGGGGTGAACTTCACATCCAACGGCCCTGTGATGCAGAA
GAAAACACTCGGCTGGGAGGCCTTCACCGAAACGCTGTACCCCGCTGACGGCGGCCTGGAAGGCAGAAACGACAT
GGCCCTGAAGCTCGTGGGCGGGAGCCATCTGATCGCAAACATCAAGACCACATATAGATCCAAGAAACCCGCTAA
GAACCTCAAGATGCCTGGCGTCTACTATGTGGACTACAGACTGGAAAGAATCAAGGAGGCCAACAACGAAACCTA
CGTCGAGCAGCACGAGGTGGCAGTGGCCAGATACTGCGACCTCCCTAGCAAACTGGGGCACAAGCTTAATTCCGG
ATGATCGACTGTGCCTTCTAGTTGCCAGCCATCTGTTGTTTGCCCCTCCCCCGTGCCTTCCTTGACCCTGGAAGGTGC
CACTCCCACTGTCCTTTCCTAATAAAATGAGGAAATTGCATCGCATTGTCTGAGTAGGTGTCATTCTATTCTGGGGG
GTGGGGTGGGGCAGGACAGCAAGGGGGAGGATTGGGAGGACAATAGCAGGCATGCTGGGGATGCGGTGGGCT
CTATGGCTTCTCCCTGCAGGTGCGCCCAATACGCAAACCGCCTCTCCCCGCGCGTTGGCCGATTCATTAATGCAGCT
GGCACGACAGGTTTCCCGACTGGAAAGCGGGCAGTGAGCGCAACGCAATTAATGTGAGTTAGCTCACTCATTAGG
CACCCCAGGCTTTACACTTTATGCTTCCGGCTCGTATGTTGTGTGGAATTGTGAGCGGATAACAATTTCACACAGGA
AACAGCTATGACCATGATTACGGACAGCCTGGCCGTCGTTTTACAACGTCGTGACTGGGAAAACCCTGGCGTTACC
CAACTTAATCGCCTTGCAGCACATCCCCCTTTCGCCAGCTGGCGTAATAGCGAAGAGGCCCGCACCGATCGCCCTTC
CCAACAGTTGCGCAGCCTGAATGGCGAATGGCGCCTGATGCGGTATTTTCTCCTTACGCATCTGTGCGGTATTTCAC
ACCGCATATGGTGCACTCTCAGTACAATCTGCTCTGATGCCGCATAGACCTGCAGGGCCAGCAGGCAGAAGTATGC
AAAGCATGCATCTCAATTAGTCAGCAACCAGGTGTGGAAAGTCCCCAGGCTCCCCAGCAGGCAGAAGTATGCAAA
GCATGCATCTCAATTAGTCAGCAACCATAGTCCCGCCCCTAACTCCGCCCATCCCGCCCCTAACTCCGCCCAGTTCCG
CCCATTCTCCGCCCCATGGCTGACTAATTTTTTTTATTTATGCAGAGGCCGAGGCCGCCTCTGCCTCTGAGCTATTCC
AGAAGTAGTGAGGAGGCTTTTTTGGAGGCCTAGGCTTTTGCAAAAAGCTCCCGGGAGCTTGTATATCCATTTTCGG
ATCTGATCAGCACGTGATGAAAAAGCCTGAACTCACCGCGACGTCTGTCGAGAAGTTTCTGATCGAAAAGTTCGAC
AGCGTGTCCGACCTGATGCAGCTCTCGGAGGGCGAAGAATCTCGTGCTTTCAGCTTCGATGTAGGAGGGCGTGGA
TATGTCCTGCGGGTAAATAGCTGCGCCGATGGTTTCTACAAAGATCGTTATGTTTATCGGCACTTTGCATCGGCCGC
GCTCCCGATTCCGGAAGTGCTTGACATTGGGGAATTCAGCGAGAGCCTGACCTATTGCATCTCCCGCCGTGCACAG
GGTGTCACGTTGCAAGACTTGCCTGAAACCGAACTGCCCGCTGTTCTGCAGCCGGTCGCGGAGGCCATGGATGCG
ATCGCTGCGGCCGATCTTAGCCAGACGAGCGGGTTCGGCCCATTCGGACCGCAAGGAATCGGTCAATACACTACA
TGGCGTGATTTCATATGCGCGATTGCTGATCCCCATGTGTATCACTGGCAAACTGTGATGGACGACACCGTCAGTG
CGTCCGTCGCGCAGGCTCTCGATGAGCTGATGCTTTGGGCCGAGGACTGCCCCGAAGTCCGGCACCTCGTGCACG
CGGATTTCGGCTCCAACAATGTCCTGACGGACAATGGCCGCATAACAGCGGTCATTGACTGGAGCGAGGCGATGT
TCGGGGATTCCCAATACGAGGTCGCCAACATCTTCTTCTGGAGGCCGTGGTTGGCTTGTATGGAGCAGCAGACGC
GCTACTTCGAGCGGAGGCATCCGGAGCTTGCAGGATCGCCGCGGCTCCGGGCGTATATGCTCCGCATTGGTCTTG

ACCAACTCTATCAGAGCTTGGTTGACGGCAATTTCGATGATGCAGCTTGGGCGCAGGGTCGATGCGACGCAATCGT
CCGATCCGGAGCCGGGACTGTCGGGCGTACACAAATCGCCCGCAGAAGCGCGGCCGTCTGGACCGATGGCTGTGT
AGAAGTACTCGCCGATAGTGGAAACCGACGCCCCAGCACTCGTCCGAGGGCAAAGGAATAGCACGTGCTACGAG
ATTTCGATTCCACCGCCGCCTTCTATGAAAGGTTGGGCTTCGGAATCGTTTTCCGGGACGCCGGCTGGATGATCCTC
CAGCGCGGGGATCTCATGCTGGAGTTCTTCGCCCACCCCAACTTGTTTATTGCAGCTTATAATGGTTACAAATAAAG
CAATAGCATCACAAATTTCACAAATAAAGCATTTTTTTCACTGCATTCTAGTTGTGGTTTGTCCAAACTCATCAATGT
ATCTTATGCGTCTTCTAGGGTTAAGGTTAGTGTAGAGAAGCAACCGAAGATTGAGAAGACATGGCGGTAATACGG
TTATCCACAGAATCAGGGGATAACGCAGGAAAGAACATGTGAGCAAAAGGCCAGCAAAAGGCCAGGAACCGTAA
AAAGGCCGCGTTGCTGGCGTTTTTCCATAGGCTCCGCCCCCCTGACGAGCATCACAAAAATCGACGCTCAAGTCAG
AGGTGGCGAAACCCGACAGGACTATAAAGATACCAGGCGTTTCCCCCTGGAAGCTCCCTCGTGCGCTCTCCTGTTC
CGACCCTGCCGCTTACCGGATACCTGTCCGCCTTTCTCCCTTCGGGAAGCGTGGCGCTTTCTCATAGCTCACGCTGT
AGGTATCTCAGTTCGGTGTAGGTCGTTCGCTCCAAGCTGGGCTGTGTGCACGAACCCCCCGTTCAGCCCGACCGCT
GCGCCTTATCCGGTAACTATCGTCTTGAGTCCAACCCGGTAAGACACGACTTATCGCCACTGGCAGCAGCCACTGG
TAACAGGATTAGCAGAGCGAGGTATGTAGGCGGTGCTACAGAGTTCTTGAAGTGGTGGCCTAACTACGGCTACAC
TAGAAGAACAGTATTTGGTATCTGCGCTCTGCTGAAGCCAGTTACCTTCGGAAAAAGAGTTGGTAGCTCTTGATCC
GGCAAACAAACCACCGCTGGTAGCGGTTTTTTTGTTTGCAAGCAGCAGATTACGCGCAGAAAAAAAGGATCTCAA
GAAGATCCTTTGATCTTTTCTACGGGGTCTGACGCTCAGTGGAACGAAAACTCACGTTAAGGGATTTTGGTCATGA
GATTATCAAAAAGGATCTTCACCTAGATCCTTTTAAATTAAAAATGAAGTTTTAAATCAATCTAAAGTATATATGAGT
AAACTTGGTCTGACAGTTACCAATGCTTAATCAGTGAGGCACCTATCTCAGCGATCTGTCTATTTCGTTCATCCATAG
TTGCCTGACTCCCCGTCGTGTAGATAACTACGATACGGGAGGGCTTACCATCTGGCCCCAGTGCTGCAATGATACC
GCGTGACCCACGCTCACCGGCTCCAGATTTATCAGCAATAAACCAGCCAGCCGGAAGGGCCGAGCGCAGAAGTGG
TCCTGCAACTTTATCCGCCTCCATCCAGTCTATTAATTGTTGCCGGGAAGCTAGAGTAAGTAGTTCGCCAGTTAATA
GTTTGCGCAACGTTGTTGCCATTGCTACAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAAGCGGTTAGCTCCTTCGGTCCTCCGA
TCGTTGTCAGAAGTAAGTTGGCCGCAGTGTTATCACTCATGGTTATGGCAGCACTGCATAATTCTCTTACTGTCATG
CCATCCGTAAGATGCTTTTCTGTGACTGGTGAGTACTCAACCAAGTCATTCTGAGAATAGTGTATGCGGCGACCGA
GTTGCTCTTGCCCGGCGTCAATACGGGATAATACCGCGCCACATAGCAGAACTTTAAAAGTGCTCATCATTGGAAA
ACGTTCTTCGGGGCGAAAACTCTCAAGGATCTTACCGCTGTTGAGATCCAGTTCGATGTAACCCACTCGTGCACCCA
ACTGATCTTCAGCATCTTTTACTTTCACCAGCGTTTCTGGGTGAGCAAAAACAGGAAGGCAAAATGCCGCAAAAAA
GGGAATAAGGGCGACACGGAAATGTTGAATACTCATACTCTTCCTTTTTCAATATTATTGAAGCATTTATCAGGGTT
ATTGTCTCATGAGCGGATACATATTTGAATGTATTTAGAAAAATAAACAAATAGGGGTTCCGCGCACATTTCCCCGA
AAAGTGCCACCTATGAGACG

Dans les exemples suivants l’objectif est de créer un vecteur permettant d’exprimer un transgène humain (cDNA) de façon stable dans une lignée cancéreuse de souris et/ou d’inhiber l’expression d’un second gène (shRNA) de souris, toujours de façon stable. La série de vecteurs décrite ci-dessous illustre l’implémentation de différents modules fonctionnels permettant de développer les fonctionnalités d’intégration à partir d’une architecture princeps (vecteur V1) qui se caractérise par la présence des deux unités d'expression décrites ci-dessus et par l'absence d'un site de clonage multiple (inhérente au procédé décrit).

Les briques décrites ci-dessous ont été obtenues par PCR selon un protocole usuel tel que celui décrit pour la réalisation 1. Les assemblages ont été réalisés à l'aide de l'enzyme Bsal (NEB) et de la ligase T4 HC (Promega) dans le tampon de la ligase T4 HC, conformément au protocole décrit plus haut.

## Réalisation 2

Groupe de vecteurs **V1** : **vecteurs permettant l'expression simultanée de multiples transgènes (et ne contenant pas de site de clonage multiple)**

$$U1 + nxU2a + mxU2b$$

U1 : Unité fonctionnelle bactérienne
U2a : Unité fonctionnelle d'expression dont le promoteur est dépendant de l'ARN polymerase II et dont le produit d'expression est une protéine
U2b : Unité fonctionnelle d'expression dont le promoteur est dépendant de l'ARN polymerase III et dont le produit d'expression est un ARN non codant
$n \geq 0$, $m \geq 0$ et $n+m \geq 2$

Exemple : Vecteur permettant d'exprimer l'enzyme hFUT3 tout en réprimant l'expression de mB3Galt6

| | |
|---|---|
| U1 | ori-Amp |
| U2a | promoteur CMV |
| | hFUT3 cDNA |
| | Terminateur BPA |
| U2b | cassette shRNA mB3Galt6 |

Liste des briques utilisées pour la construction du vecteur V1 (figure 6)

**Brique Ori-AmpR Bsal B (SEQ ID NO : 36)**

GAGGTACCGGTCTCATATTGTAATACGGTTATCCACAGAATCAGGGGATAACGCAGGAAAGAACATGTGAGCAAAAGGCCAGCAAAAGGCCAGGAACCGTAAAAAGGCCGCGTTGCTGGCGTTTTTCCATAGGCTCCGCCCCCCTGACGAGCATCACAAAAATCGACGCTCAAGTCAGAGGTGGCGAAACCCGACAGGACTATAAAGATACCAGGCGTTTCCCCCTGGAAGCTCCCTCGTGCGCTCTCCTGTTCCGACCCTGCCGCTTACCGGATACCTGTCCGCCTTTCTCCCTTCGGGAAGCGTGGCGCTTTCTCATAGCTCACGCTGTAGGTATCTCAGTTCGGTGTAGGTCGTTCGCTCCAAGCTGGGCTGTGTGCACGAACCCCCCGTTCAGCCCGACCGCTGCGCCTTATCCGGTAACTATCGTCTTGAGTCCAACCCGGTAAGACACGACTTATCGCCACTGGCAGCAGCCACTGGTAACAGGATTAGCAGAGCGAGGTATGTAGGCGGTGCTACAGAGTTCTTGAAGTGGTGGCCTAACTACGGCTACACTAGAAGAACAGTATTTGGTATCTGCGCTCTGCTGAAGCCAGTTACCTTCGGAAAAAGAGTTGGTAGCTCTTGATCCGGCAAACAAACCACCGCTGGTAGCGGTTTTTTTGTTTGCAAGCAGCAGATTACGCGCAGAAAAAAAGGATCTCAAGAAGATCCTTTGATCTTTTCTACGGGGTCTGACGCTCAGTGGAACGAAAACTCACGTTAAGGGATTTTGGTCATGAGATTATCAAAAAGGATCTTCACCTAGATCCTTTTAAATTAAAAATGAAGTTTTAAATCAATCTAAAGTATATATGAGTAAACTTGGTCTGACAGTTACCAATGCTTAATCAGTGAGGCACCTATCTCAGCGATCTGTCTATTTCGTTCATCCATAGTTGCCTGACTCCCCGTCGTGTAGATAACTACGATACGGGAGGGCTTACCATCTGGCCCCAGTGCTGCAATGATACCGCGTGACCCACGCTCACCGGCTCCAGATTTATCAGCAATAAACCAGCCAGCCGGAAGGGCCGAGCGCAGAAGTGGTCCTGCAACTTTATCCGCCTCCATCCAGTCTATTAATTGTTGCCGGGAAGCTAGAGTAAGTAGTTCGCCAGTTAATAGTTTGCGCAACGTTGTTGCCATTGCTACAGGCATCGTGGTGTCACGCTCGTCGTTTGGTATGGCTTCATTCAGCTCCGGTTCCCAACGATCAAGGCGAGTTACATGATCCCCCATGTTGTGCAAAAAAGCGGTTAGCTCCTTCGGTCCTCCGATCGTTGTCAGAAGTAAGTTGGCCGCAGTGTTATCACTCATGGTTATGGCAGCACTGCATAATTCTCTTACTGTCATGCCATCCGTAAGATGCTTTTCTGTGACTGGTGAGTACTCAACCAAGTCATTCTGAGAATAGTGTATGCGGCGACCGAGTTGCTCTTGCCCGGCGTCAATACGGGATAATACCGCGCCACATAGCAGAACTTTAAAAGTGCTCATCATTGGAAAACGTTCTTCGGGGCGAAAACTCTCAAGGATCTTACCGCTGTTGAGATCCAGTTCGATGTAACCCACTCGTGCACCCAACTGATCTTCAGCATCTTTTACTTTCACCAGCGTTTCTGGGTGAGCAAAAACAGGAAGGCAAAATGCCGCAAAAAAGGGAATAAGGGCGACACGGAAATGTTGAATACTCATACTCTTCCTTTTTCAATATTATTGAAGCATTTATCAGGGTTATTGTCTCATGAGCGGATACATATTTGAATGTATTTAGAAAAATAAACAAATAGGGGTTCCGCGCACATTTCCCCGAAAAGTGCCAAGGACGAGACCGGTACCTC

**Brique pCMV BsaI B (SEQ ID NO : 37)**

GAGGTACCGGTCTCAAGGAACCAATTCAGTCGACTGGATCCTAGTTATTAATAGTAATCAATTACGGGGTCATTAGTTCATAGCCCATATATGGAGTTCCGCGTTACATAACTTACGGTAAATGGCCCGCCTGGCTGACCGCCCAACGACCCC

CGCCCATTGACGTCAATAATGACGTATGTTCCCATAGTAACGCCAATAGGGACTTTCCATTGACGTCAATGGGTGG AGTATTTACGGTAAACTGCCCACTTGGCAGTACATCAAGTGTATCATATGCCAAGTACGCCCCCTATTGACGTCAAT GACGGTAAATGGCCCGCCTGGCATTATGCCCAGTACATGACCTTATGGGACTTTCCTACTTGGCAGTACATCTACGT ATTAGTCATCGCTATTACCATGGTGATGCGGTTTTGGCAGTACATCAATGGGCGTGGATAGCGGTTTGACTCACGG GGATTTCCAAGTCTCCACCCCATTGACGTCAATGGGAGTTTGTTTTGGCACCAAAATCAACGGGACTTTCCAAAATG TCGTAACAACTCCGCCCCATTGACGCAAATGGGCGGTAGGCGTGTACGGTGGGAGGTCTATATAAGCAGAGCTGG TTTAGTGAACCGTCAGATCACTAGTCGACTAGGGATAACAGGGCACCCGAGACCGGTACCTC

**Brique hFUT3 BsaI A (SEQ ID NO : 38)**

GAGGTACCGGTCTCACACCATGGATCCCCTGGGTGCAGCCAAGCCACAATGGCCATGGCGCCGCTGTCTGGCCGC ACTGCTATTTCAGCTGCTGGTGGCTGTGTGTTTCTTCTCCTACCTGCGTGTGTCCCGAGACGATGCCACTGGATCCC CTAGGGCTCCCAGTGGGTCCTCCCGACAGGACACCACTCCCACCCGCCCCACCCTCCTGATCCTGCTATGGACATGG CCTTTCCACATCCCTGTGGCTCTGTCCCGCTGTTCAGAGATGGTGCCCGGCACAGCCGACTGCCACATCACTGCCGA CCGCAAGGTGTACCCACAGGCAGACACGGTCATCGTGCACCACTGGGATATCATGTCCAACCCTAAGTCACGCCTC CCACCTTCCCCGAGGCCGCAGGGGCAGCGCTGGATCTGGTTCAACTTGGAGCCACCCCCTAACTGCCAGCACCTGG AAGCCCTGGACAGATACTTCAATCTCACCATGTCCTACCGCAGCGACTCCGACATCTTCACGCCCTACGGCTGGCTG GAGCCGTGGTCCGGCCAGCCTGCCCACCCACCGCTCAACCTCTCGGCCAAGACCGAGCTGGTGGCCTGGGCGGTG TCCAACTGGAAGCCGGACTCAGCCAGGGTGCGCTACTACCAGAGCCTGCAGGCTCATCTCAAGGTGGACGTGTAC GGACGCTCCCACAAGCCCCTGCCCAAGGGGACCATGATGGAGACGCTGTCCCGGTACAAGTTCTACCTGGCCTTCG AGAACTCCTTGCACCCCGACTACATCACCGAGAAGCTGTGGAGGAACGCCCTGGAGGCCTGGGCCGTGCCCGTGG TGCTGGGCCCCAGCAGAAGCAACTACGAGAGGTTCCTGCCACCCGACGCCTTCATCCACGTGGACGACTTCCAGAG CCCCAAGGACCTGGCCCGGTACCTGCAGGAGCTGGACAAGGACCACGCCCGCTACCTGAGCTACTTTCGCTGGCG GGAGACGCTGCGGCCTCGCTCCTTCAGCTGGGCACTGGATTTCTGCAAGGCCTGCTGGAAACTGCAGCAGGAATC CAGGTACCAGACGGTGCGCAGCATAGCGGCTTGGTTCACCTGATCGAGACCGGTACCTC

**Brique BGHpA BsaI B (SEQ ID NO : 39)**

GAGGTACCGGTCTCATGATCGACTGTGCCTTCTAGTTGCCAGCCATCTGTTGTTTGCCCCTCCCCCGTGCCTTCCTTG ACCCTGGAAGGTGCCACTCCCACTGTCCTTTCCTAATAAAATGAGGAAATTGCATCGCATTGTCTGAGTAGGTGTCA TTCTATTCTGGGGGGTGGGGTGGGGCAGGACAGCAAGGGGGAGGATTGGGAGGACAATAGCAGGCATGCTGGG GATGCGGTGGGCTCTATGGCTTCGCGAGACCGGTACCTC

**Brique shB3Galt6 BsaI A (SEQ ID NO : 40)**

GAGGTACCGGTCTCATTCGACAGGGTCGACAAGCTTTTCCAAAAAAAAAGCATGAGGTGCAGTTGCGCCTTTCCTA
TCTCTTGAATAGGAAAGGCGCAACTGCACCTCATGCTGGATCCCGCGTCCTTTCCACAAGATATATAAACCCAAGAA
ATCGAAATACTTTCAAGTTACGGTAAGCATATGATAGTCCATTTTAAAACATAATTTTAAAACTGCAAACTACCCAA
GAAATTATTACTTTCTACGTCACGTATTTTGTACTAATATCTTTGTGTTTACAGTCAAATTAATTCTAATTATCTCTCTA
ACAGCCTTGTATCGTATATGCAAATATGAAGGAATCATGGGAAATAGGCCCTCTTCCTGCCCGACCTTGGCGCGCG
CTCGGCGCGCGGTCACGCTCCGTCACGTGGTGCGTTTTGTATTCGAGACCGGTACCTC

**Vecteur V1 (SEQ ID NO : 41, exemple de vecteur du groupe V1)**

TATTGTAATACGGTTATCCACAGAATCAGGGGATAACGCAGGAAAGAACATGTGAGCAAAAGGCCAGCAAAAGG
CCAGGAACCGTAAAAAGGCCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGAAGCGTGGCGCTTTCTCA
TAGCTCACGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCCACTGGC
AGCAGCCACTGGTAACAGGATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTTTTTTTGTTTGCAAGCAGCAGATTACGCGCAGAAAAA
AAGGATCTCAAGAAGATCCTTTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGAAGTTTTAAATCAATCTAAAG
TATATATGAGTAAACTTGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACCATCTGGCCCCAGTGCT
GCAATGATACCGCGTGACCCACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACGCTCGTCGTTTGGTATGGCTT
CATTCAGCTCCGGTTCCCAACGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATGGCAGCACTGCATAATTCTCT
TACTGTCATGCCATCCGTAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC

ATTTCCCCGAAAAGTGCCAAGGAACCAATTCAGTCGACTGGATCCTAGTTATTAATAGTAATCAATTACGGGGTCAT
TAGTTCATAGCCCATATATGGAGTTCCGCGTTACATAACTTACGGTAAATGGCCCGCCTGGCTGACCGCCCAACGA
CCCCCGCCCATTGACGTCAATAATGACGTATGTTCCCATAGTAACGCCAATAGGGACTTTCCATTGACGTCAATGGG
TGGAGTATTTACGGTAAACTGCCCACTTGGCAGTACATCAAGTGTATCATATGCCAAGTACGCCCCCTATTGACGTC
AATGACGGTAAATGGCCCGCCTGGCATTATGCCCAGTACATGACCTTATGGGACTTTCCTACTTGGCAGTACATCTA
CGTATTAGTCATCGCTATTACCATGGTGATGCGGTTTTGGCAGTACATCAATGGGCGTGGATAGCGGTTTGACTCA
CGGGGATTTCCAAGTCTCCACCCCATTGACGTCAATGGGAGTTTGTTTTGGCACCAAAATCAACGGGACTTTCCAAA
ATGTCGTAACAACTCCGCCCCATTGACGCAAATGGGCGGTAGGCGTGTACGGTGGGAGGTCTATATAAGCAGAGC
TGGTTTAGTGAACCGTCAGATCACTAGTCGACTAGGGATAACAGGGCACCATGGATCCCCTGGGTGCAGCCAAGC
CACAATGGCCATGGCGCCGCTGTCTGGCCGCACTGCTATTTCAGCTGCTGGTGGCTGTGTGTTTCTTCTCCTACCTG
CGTGTGTCCCGAGACGATGCCACTGGATCCCCTAGGGCTCCCAGTGGGTCCTCCCGACAGGACACCACTCCCACCC
GCCCCACCCTCCTGATCCTGCTATGGACATGGCCTTTCCACATCCCTGTGGCTCTGTCCCGCTGTTCAGAGATGGTG
CCCGGCACAGCCGACTGCCACATCACTGCCGACCGCAAGGTGTACCCACAGGCAGACACGGTCATCGTGCACCACT
GGGATATCATGTCCAACCCTAAGTCACGCCTCCCACCTTCCCCGAGGCCGCAGGGGCAGCGCTGGATCTGGTTCAA
CTTGGAGCCACCCCCTAACTGCCAGCACCTGGAAGCCCTGGACAGATACTTCAATCTCACCATGTCCTACCGCAGCG
ACTCCGACATCTTCACGCCCTACGGCTGGCTGGAGCCGTGGTCCGGCCAGCCTGCCCACCCACCGCTCAACCTCTCG
GCCAAGACCGAGCTGGTGGCCTGGGCGGTGTCCAACTGGAAGCCGGACTCAGCCAGGGTGCGCTACTACCAGAG
CCTGCAGGCTCATCTCAAGGTGGACGTGTACGGACGCTCCCACAAGCCCCTGCCCAAGGGGACCATGATGGAGAC
GCTGTCCCGGTACAAGTTCTACCTGGCCTTCGAGAACTCCTTGCACCCCGACTACATCACCGAGAAGCTGTGGAGG
AACGCCCTGGAGGCCTGGGCCGTGCCCGTGGTGCTGGGCCCCAGCAGAAGCAACTACGAGAGGTTCCTGCCACCC
GACGCCTTCATCCACGTGGACGACTTCCAGAGCCCCAAGGACCTGGCCCGGTACCTGCAGGAGCTGGACAAGGAC
CACGCCCGCTACCTGAGCTACTTTCGCTGGCGGGAGACGCTGCGGCCTCGCTCCTTCAGCTGGGCACTGGATTTCT
GCAAGGCCTGCTGGAAACTGCAGCAGGAATCCAGGTACCAGACGGTGCGCAGCATAGCGGCTTGGTTCACCTGAT
CGACTGTGCCTTCTAGTTGCCAGCCATCTGTTGTTTGCCCCTCCCCCGTGCCTTCCTTGACCCTGGAAGGTGCCACTC
CCACTGTCCTTTCCTAATAAAATGAGGAAATTGCATCGCATTGTCTGAGTAGGTGTCATTCTATTCTGGGGGGTGGG
GTGGGGCAGGACAGCAAGGGGGAGGATTGGGAGGACAATAGCAGGCATGCTGGGGATGCGGTGGGCTCTATGG
CTTCGACAGGGTCGACAAGCTTTTCCAAAAAAAAAGCATGAGGTGCAGTTGCGCCTTTCCTATCTCTTGAATAGGA
AAGGCGCAACTGCACCTCATGCTGGATCCCGCGTCCTTTCCACAAGATATATAAACCCAAGAAATCGAAATACTTTC
AAGTTACGGTAAGCATATGATAGTCCATTTTAAAACATAATTTTAAAACTGCAAACTACCCAAGAAATTATTACTTTC
TACGTCACGTATTTTGTACTAATATCTTTGTGTTTACAGTCAAATTAATTCTAATTATCTCTCTAACAGCCTTGTATCG
TATATGCAAATATGAAGGAATCATGGGAAATAGGCCCTCTTCCTGCCCGACCTTGGCGCGCGCTCGGCGCGCGGTC
ACGCTCCGTCACGTGGTGCGTTTTG.

L'analyse de restriction par triple digestion (validation structurelle), EcoRV, PvuI, SalI de la réalisation 2 est illustrée Figure 7.

Vérification fonctionnelle de la réalisation 2 : Des cellules eucaryotes (ex : cellules cancéreuses mammaires de souris 4T1) sont transfectées avec le vecteur V1 selon un protocole usuel (exemple lipotransfection ou électroporation).

Les cellules sont collectées 24 à 48h après transfection et lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel.

**Conditions spécifiques de la PCR quantitative:**

40 cycles des trois étapes suivantes sont réalisés: dénaturation 94°C-30s ; hybridation 60°C-30s ; extension 72°C-30s.

Amorces utilisées:

**mB3Galt6**

BETA3Galt6ms2-s ACCACTCTGTTGTACCTGGC (SEQ ID NO : 42).
BETA3Galt6ms2-as CACACGTCCTCGGGTCC (SEQ ID NO : 43).

**HFUT3**

FUT35 : CACTAGTCGACTAGGGATAACAGG (SEQ ID NO :44).
FUT33 : ATGTCCATAGCAGGATCAGGAG (SEQ ID NO :45).

**Réalisation 3**

**Groupe de vecteurs V1.1** : **Vecteurs V1 permettant de sélectionner l'intégration des transgènes par recombinaison non homologue dans le génome cible**

$$U1 + nxU2a + mxU2b + U3a$$

U1 : Unité fonctionnelle bactérienne
U2a : Unité fonctionnelle d'expression dont le promoteur est dépendant de l'ARN polymerase II et dont le produit d'expression est une protéine
U2b : Unité fonctionnelle d'expression dont le promoteur est dépendant de l'ARN polymerase III et dont le produit d'expression est un ARN non codant
U3a est une cassette de sélection positive
n≥0, m≥0 et n+m≥2

Exemple : Vecteur permettant d'exprimer l'enzyme hFUT3 tout en réprimant l'expression de mB3Galt6

| | |
|---|---|
| U1 | ori-Amp |
| U2a | promoteur CMV<br>hFUT3 cDNA<br>Terminateur BPA |
| U2b | cassette shRNA mB3Galt6 |
| U3a | Hygomicin resistance |

Listes des briques utilisées pour construire le vecteur V1.1 (Figure 8)

**Brique Ori-AmpR BsaI B** (SEQ ID NO : 36)
**Brique pCMV BsaI B** (SEQ ID NO : 37)
**Brique hFUT3 BsaI A** (SEQ ID NO : 38)
**Brique BGHpA BsaI B** (SEQ ID NO : 39)

**Brique shB3Galt6 BsaI B (SEQ ID NO : 46)**

GAGGTACCGGTCTCATTCGACAGGGTCGACAAGCTTTTCCAAAAAAAAAGCATGAGGTGCAGTTGCGCCTTTCCTA
TCTCTTGAATAGGAAAGGCGCAACTGCACCTCATGCTGGATCCCGCGTCCTTTCCACAAGATATATAAACCCAAGAA
ATCGAAATACTTTCAAGTTACGGTAAGCATATGATAGTCCATTTTAAAACATAATTTTAAAACTGCAAACTACCCAA
GAAATTATTACTTTCTACGTCACGTATTTTGTACTAATATCTTTGTGTTTACAGTCAAATTAATTCTAATTATCTCTCTA
ACAGCCTTGTATCGTATATGCAAATATGAAGGAATCATGGGAAATAGGCCCTCTTCCTGCCCGACCTTGGCGCGCG
CTCGGCGCGCGGTCACGCTCCGTCACGTGGTGCGTTTTGCCAGCGAGACCGGTACCTC

**Brique HygroR BsaI B (SEQ ID NO : 47)**

GAGGTACCGGTCTCACCAGCAGGCAGAAGTATGCAAAGCATGCATCTCAATTAGTCAGCAACCAGGTGTGGAAAG
TCCCCAGGCTCCCCAGCAGGCAGAAGTATGCAAAGCATGCATCTCAATTAGTCAGCAACCATAGTCCCGCCCCTAA

CTCCGCCCATCCCGCCCCTAACTCCGCCCAGTTCCGCCCATTCTCCGCCCCATGGCTGACTAATTTTTTTTATTTATGC
AGAGGCCGAGGCCGCCTCTGCCTCTGAGCTATTCCAGAAGTAGTGAGGAGGCTTTTTTGGAGGCCTAGGCTTTTGC
AAAAAGCTCCCGGGAGCTTGTATATCCATTTTCGGATCTGATCAGCACGTGATGAAAAAGCCTGAACTCACCGCGA
CGTCTGTCGAGAAGTTTCTGATCGAAAAGTTCGACAGCGTGTCCGACCTGATGCAGCTCTCGGAGGGCGAAGAAT
CTCGTGCTTTCAGCTTCGATGTAGGAGGGCGTGGATATGTCCTGCGGGTAAATAGCTGCGCCGATGGTTTCTACAA
AGATCGTTATGTTTATCGGCACTTTGCATCGGCCGCGCTCCCGATTCCGGAAGTGCTTGACATTGGGGAATTCAGC
GAGAGCCTGACCTATTGCATCTCCCGCCGTGCACAGGGTGTCACGTTGCAAGACTTGCCTGAAACCGAACTGCCCG
CTGTTCTGCAGCCGGTCGCGGAGGCCATGGATGCGATCGCTGCGGCCGATCTTAGCCAGACGAGCGGGTTCGGCC
CATTCGGACCGCAAGGAATCGGTCAATACACTACATGGCGTGATTTCATATGCGCGATTGCTGATCCCCATGTGTAT
CACTGGCAAACTGTGATGGACGACACCGTCAGTGCGTCCGTCGCGCAGGCTCTCGATGAGCTGATGCTTTGGGCC
GAGGACTGCCCCGAAGTCCGGCACCTCGTGCACGCGGATTTCGGCTCCAACAATGTCCTGACGGACAATGGCCGC
ATAACAGCGGTCATTGACTGGAGCGAGGCGATGTTCGGGGATTCCCAATACGAGGTCGCCAACATCTTCTTCTGGA
GGCCGTGGTTGGCTTGTATGGAGCAGCAGACGCGCTACTTCGAGCGGAGGCATCCGGAGCTTGCAGGATCGCCGC
GGCTCCGGGCGTATATGCTCCGCATTGGTCTTGACCAACTCTATCAGAGCTTGGTTGACGGCAATTTCGATGATGC
AGCTTGGGCGCAGGGTCGATGCGACGCAATCGTCCGATCCGGAGCCGGGACTGTCGGGCGTACACAAATCGCCC
GCAGAAGCGCGGCCGTCTGGACCGATGGCTGTGTAGAAGTACTCGCCGATAGTGGAAACCGACGCCCCAGCACTC
GTCCGAGGGCAAAGGAATAGCACGTGCTACGAGATTTCGATTCCACCGCCGCCTTCTATGAAAGGTTGGGCTTCG
GAATCGTTTTCCGGGACGCCGGCTGGATGATCCTCCAGCGCGGGGATCTCATGCTGGAGTTCTTCGCCCACCCCAA
CTTGTTTATTGCAGCTTATAATGGTTACAAATAAAGCAATAGCATCACAAATTTCACAAATAAAGCATTTTTTTCACT
GCATTCTAGTTGTGGTTTGTCCAAACTCATCAATGTATCTATTCGAGACCGGTACCTC

**V1.1 (SEQ ID NO : 48, exemple de vecteur du groupe V1.1)**

TATTGTAATACGGTTATCCACAGAATCAGGGGATAACGCAGGAAAGAACATGTGAGCAAAAGGCCAGCAAAAGG
CCAGGAACCGTAAAAAGGCCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGAAGCGTGGCGCTTTCTCA
TAGCTCACGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCCACTGGC
AGCAGCCACTGGTAACAGGATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTTTTTTTGTTTGCAAGCAGCAGATTACGCGCAGAAAAA
AAGGATCTCAAGAAGATCCTTTGATCTTTTCTACGGGGTCTGACGCTCAGTGGAACGAAAACTCACGTTAAGGGAT

TTTGGTCATGAGATTATCAAAAAGGATCTTCACCTAGATCCTTTTAAATTAAAAATGAAGTTTTAAATCAATCTAAAG
TATATATGAGTAAACTTGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACCATCTGGCCCCAGTGCT
GCAATGATACCGCGTGACCCACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACGCTCGTCGTTTGGTATGGCTT
CATTCAGCTCCGGTTCCCAACGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATGGCAGCACTGCATAATTCTCT
TACTGTCATGCCATCCGTAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCAAGGAACCAATTCAGTCGACTGGATCCTAGTTATTAATAGTAATCAATTACGGGGTCAT
TAGTTCATAGCCCATATATGGAGTTCCGCGTTACATAACTTACGGTAAATGGCCCGCCTGGCTGACCGCCCAACGA
CCCCCGCCCATTGACGTCAATAATGACGTATGTTCCCATAGTAACGCCAATAGGGACTTTCCATTGACGTCAATGGG
TGGAGTATTTACGGTAAACTGCCCACTTGGCAGTACATCAAGTGTATCATATGCCAAGTACGCCCCCTATTGACGTC
AATGACGGTAAATGGCCCGCCTGGCATTATGCCCAGTACATGACCTTATGGGACTTTCCTACTTGGCAGTACATCTA
CGTATTAGTCATCGCTATTACCATGGTGATGCGGTTTTGGCAGTACATCAATGGGCGTGGATAGCGGTTTGACTCA
CGGGGATTTCCAAGTCTCCACCCCATTGACGTCAATGGGAGTTTGTTTTGGCACCAAAATCAACGGGACTTTCCAAA
ATGTCGTAACAACTCCGCCCCATTGACGCAAATGGGCGGTAGGCGTGTACGGTGGGAGGTCTATATAAGCAGAGC
TGGTTTAGTGAACCGTCAGATCACTAGTCGACTAGGGATAACAGGGCACCATGGATCCCCTGGGTGCAGCCAAGC
CACAATGGCCATGGCGCCGCTGTCTGGCCGCACTGCTATTTCAGCTGCTGGTGGCTGTGTGTTTCTTCTCCTACCTG
CGTGTGTCCCGAGACGATGCCACTGGATCCCCTAGGGCTCCCAGTGGGTCCTCCCGACAGGACACCACTCCCACCC
GCCCCACCCTCCTGATCCTGCTATGGACATGGCCTTTCCACATCCCTGTGGCTCTGTCCCGCTGTTCAGAGATGGTG
CCCGGCACAGCCGACTGCCACATCACTGCCGACCGCAAGGTGTACCCACAGGCAGACACGGTCATCGTGCACCACT
GGGATATCATGTCCAACCCTAAGTCACGCCTCCCACCTTCCCCGAGGCCGCAGGGGCAGCGCTGGATCTGGTTCAA
CTTGGAGCCACCCCCTAACTGCCAGCACCTGGAAGCCCTGGACAGATACTTCAATCTCACCATGTCCTACCGCAGCG
ACTCCGACATCTTCACGCCCTACGGCTGGCTGGAGCCGTGGTCCGGCCAGCCTGCCCACCCACCGCTCAACCTCTCG
GCCAAGACCGAGCTGGTGGCCTGGGCGGTGTCCAACTGGAAGCCGGACTCAGCCAGGGTGCGCTACTACCAGAG
CCTGCAGGCTCATCTCAAGGTGGACGTGTACGGACGCTCCCACAAGCCCCTGCCCAAGGGGACCATGATGGAGAC

GCTGTCCCGGTACAAGTTCTACCTGGCCTTCGAGAACTCCTTGCACCCCGACTACATCACCGAGAAGCTGTGGAGG
AACGCCCTGGAGGCCTGGGCCGTGCCCGTGGTGCTGGGCCCCAGCAGAAGCAACTACGAGAGGTTCCTGCCACCC
GACGCCTTCATCCACGTGGACGACTTCCAGAGCCCCAAGGACCTGGCCCGGTACCTGCAGGAGCTGGACAAGGAC
CACGCCCGCTACCTGAGCTACTTTCGCTGGCGGGAGACGCTGCGGCCTCGCTCCTTCAGCTGGGCACTGGATTTCT
GCAAGGCCTGCTGGAAACTGCAGCAGGAATCCAGGTACCAGACGGTGCGCAGCATAGCGGCTTGGTTCACCTGAT
CGACTGTGCCTTCTAGTTGCCAGCCATCTGTTGTTTGCCCCTCCCCCGTGCCTTCCTTGACCCTGGAAGGTGCCACTC
CCACTGTCCTTTCCTAATAAAATGAGGAAATTGCATCGCATTGTCTGAGTAGGTGTCATTCTATTCTGGGGGGTGGG
GTGGGGCAGGACAGCAAGGGGGAGGATTGGGAGGACAATAGCAGGCATGCTGGGGATGCGGTGGGCTCTATGG
CTTCGACAGGGTCGACAAGCTTTTCCAAAAAAAAAAGCATGAGGTGCAGTTGCGCCTTTCCTATCTCTTGAATAGGA
AAGGCGCAACTGCACCTCATGCTGGATCCCGCGTCCTTTCCACAAGATATATAAACCCAAGAAATCGAAATACTTTC
AAGTTACGGTAAGCATATGATAGTCCATTTTAAAACATAATTTTAAAACTGCAAACTACCCAAGAAATTATTACTTTC
TACGTCACGTATTTTGTACTAATATCTTTGTGTTTACAGTCAAATTAATTCTAATTATCTCTCTAACAGCCTTGTATCG
TATATGCAAATATGAAGGAATCATGGGAAATAGGCCCTCTTCCTGCCCGACCTTGGCGCGCGCTCGGCGCGCGGTC
ACGCTCCGTCACGTGGTGCGTTTTGCCAGCAGGCAGAAGTATGCAAAGCATGCATCTCAATTAGTCAGCAACCAGG
TGTGGAAAGTCCCCAGGCTCCCCAGCAGGCAGAAGTATGCAAAGCATGCATCTCAATTAGTCAGCAACCATAGTCC
CGCCCCTAACTCCGCCCATCCCGCCCCTAACTCCGCCCAGTTCCGCCCATTCTCCGCCCCATGGCTGACTAATTTTTTT
TATTTATGCAGAGGCCGAGGCCGCCTCTGCCTCTGAGCTATTCCAGAAGTAGTGAGGAGGCTTTTTTGGAGGCCTA
GGCTTTTGCAAAAAGCTCCCGGGAGCTTGTATATCCATTTTCGGATCTGATCAGCACGTGATGAAAAAGCCTGAAC
TCACCGCGACGTCTGTCGAGAAGTTTCTGATCGAAAAGTTCGACAGCGTGTCCGACCTGATGCAGCTCTCGGAGGG
CGAAGAATCTCGTGCTTTCAGCTTCGATGTAGGAGGGCGTGGATATGTCCTGCGGGTAAATAGCTGCGCCGATGG
TTTCTACAAAGATCGTTATGTTTATCGGCACTTTGCATCGGCCGCGCTCCCGATTCCGGAAGTGCTTGACATTGGGG
AATTCAGCGAGAGCCTGACCTATTGCATCTCCCGCCGTGCACAGGGTGTCACGTTGCAAGACTTGCCTGAAACCGA
ACTGCCCGCTGTTCTGCAGCCGGTCGCGGAGGCCATGGATGCGATCGCTGCGGCCGATCTTAGCCAGACGAGCGG
GTTCGGCCCATTCGGACCGCAAGGAATCGGTCAATACACTACATGGCGTGATTTCATATGCGCGATTGCTGATCCC
CATGTGTATCACTGGCAAACTGTGATGGACGACACCGTCAGTGCGTCCGTCGCGCAGGCTCTCGATGAGCTGATGC
TTTGGGCCGAGGACTGCCCCGAAGTCCGGCACCTCGTGCACGCGGATTTCGGCTCCAACAATGTCCTGACGGACAA
TGGCCGCATAACAGCGGTCATTGACTGGAGCGAGGCGATGTTCGGGGATTCCCAATACGAGGTCGCCAACATCTT
CTTCTGGAGGCCGTGGTTGGCTTGTATGGAGCAGCAGACGCGCTACTTCGAGCGGAGGCATCCGGAGCTTGCAGG
ATCGCCGCGGCTCCGGGCGTATATGCTCCGCATTGGTCTTGACCAACTCTATCAGAGCTTGGTTGACGGCAATTTCG
ATGATGCAGCTTGGGCGCAGGGTCGATGCGACGCAATCGTCCGATCCGGAGCCGGGACTGTCGGGCGTACACAA
ATCGCCCGCAGAAGCGCGGCCGTCTGGACCGATGGCTGTGTAGAAGTACTCGCCGATAGTGGAAACCGACGCCCC
AGCACTCGTCCGAGGGCAAAGGAATAGCACGTGCTACGAGATTTCGATTCCACCGCCGCCTTCTATGAAAGGTTGG

GCTTCGGAATCGTTTTCCGGGACGCCGGCTGGATGATCCTCCAGCGCGGGGATCTCATGCTGGAGTTCTTCGCCCA
CCCCAACTTGTTTATTGCAGCTTATAATGGTTACAAATAAAGCAATAGCATCACAAATTTCACAAATAAAGCATTTTT
TTCACTGCATTCTAGTTGTGGTTTGTCCAAACTCATCAATGTATC

L'analyse de restriction par triple digestion, EcoRV, PvuI, SalI (1 μg de D'ADN digéré par 10 unités de chaque enzyme 15 minutes à 37°C) de la réalisation 3 est illustrée figure 9.

Vérification fonctionnelle de la réalisation 3: des cellules eucaryotes (ex : cellules cancéreuses mammaires de souris 4T1) sont transfectées avec le vecteur V1.1 selon un protocole usuel (exemple lipotransfection ou électroporation). Après transfection les cellules sont traitées avec de l'hygromycine (50μg-mL-1 pendant 7 jours). Les cellules sont ensuite collectées et lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel (Figure 10).

Conditions spécifiques de la PCR quantitative:

40 cycles des trois étapes suivantes sont réalisés: dénaturation 94°C-30s ; hybridation 60°C-30s ; extension 72°C-30s.

Amorces utilisées

**mB3Galt6**

BETA3Galt6ms2-s ACCACTCTGTTGTACCTGGC (SEQ ID NO : 42).
BETA3Galt6ms2-as CACACGTCCTCGGGTCC (SEQ ID NO : 43).

**hFUT3**

FUT35 CACTAGTCGACTAGGGATAACAGG (SEQ ID NO : 44).
FUT33 ATGTCCATAGCAGGATCAGGAG (SEQ ID NO : 45).

**Réalisation 4**

**Groupe de vecteurs V1.2: Vecteurs V1.1 permettant de sélectionner l'intégration simultanée de multiples de transgènes par recombinaison homologue dans le génome cible**

U1 + U3b + U2 + U3a + U3c

U1 : Unité fonctionnelle bactérienne

U2 : nxU2a + mxU2b, n≥0, m≥0 et n+m≥2

U2a : Unité fonctionnelle d'expression dont le promoteur est dépendant de l'ARN polymerase II et dont le produit d'expression est une protéine

U2b : Unité fonctionnelle d'expression dont le promoteur est dépendant de l'ARN polymerase III et dont le produit d'expression est un ARN non codant

U3a = cassette de sélection positive

U3b = motif 5' d'une séquence de recombinaison homologue X

U3c = motif 3' de la séquence de recombinaison homologue X

Exemple : Vecteur permettant d'exprimer l'enzyme hFUT3 tout en réprimant l'expression de mB3Galt6

| | |
|---|---|
| U1 | ori-Amp |
| U3b | Rosa26-5' |
| U2a | promoteur CMV |
| | hFUT3 cDNA |
| | Terminateur BPA |
| U2b | cassette shRNA mB3Galt6 |
| U3a | Hygomicin resistance |
| U3c | Rosa26-3' |

Listes des briques utilisées pour la construction du vecteur V1.2 (figure 11)

**Brique Ori-AmpR BsaI B (SEQ ID NO : 36)**

**Brique rosa26-5' BsaI A (SEQ ID NO : 49)**

GAGGTACCGGTCTCAAGGACCCCGCGGCAGGCCCTCCGAGCGTGGTGGAGCCGTTCTGTGAGACAGCCGGGTAC
GAGTCGTGACGCTGGAAGGGGCAAGCGGGTGGTGGGCAGGAATGCGGTCCGCCCTGCAGCAACCGGAGGGGGA
GGGAGAAGGGAGCGGAAAAGTCTCCACCGGACGCGGCCATGGCTCGGGGGGGGGGGGGCAGCGGAGGAGCGC
TTCCGGCCGACGTCTCGTCGCTGATTGGCTTCTTTTCCTCCCGCCGTGTGTGAAAACACAAATGGCGTGTTTTGGTT
GGCGTAAGGCGCCTGTCAGTTAACGGCAGCCGGAGTGCGCAGCCGCCGGCAGCCTCGCTCTGCCCACTGGGTGG
GGCGGGAGGTAGGTGGGGTGAGGCGAGCTGGACGTGCGGGCGCGGTCGGCCTCTGGCGGGGCGGGGGAGGGG
AGGGAGGGTCAGCGAAAGTAGCTCGCGCGCGAGCGGCCGCCCACCCTCCCCTTCCTCTGGGGGAGTCGTTTTACC
CGCCGCCGGCCGGGCCTCGTCGTCTGATTGGCTCTCGGGGCCCAGAAAACTGGCCCTTGCCATTGGCTCGTGTTCG

TGCAAGTTGAGTCCATCCGCCGGCCAGCGGGGGCGGCGAGGAGGCGCTCCCAGGTTCCGGCCCTCCCCTCGGCCC
CGCGCCGCAGAGTCTGGCCGCGCGCCCCTGCGCAACGTGGCAGGAAGCGCGCGCTGGGGGCGGGGACGGGCAG
TAGGGCTGAGCGGCTGCGGGGCGGGTGCAAGCACGTTTCCGACTTGAGTTGCCTCAAGAGGGGCGTGCTGAGCC
AGACCTCCATCGCGCACTCCGGGGAGTGGAGGGAAGGAGCGAGGGCTCAGTTGGGCTGTTTTGGAGGCAGGAAG
CACTTGCTCTCCCAAAGTCGCTCTGAGTTGTTATCAGTAAGGGAGCTGCAGTGGAGTAGGCGGGGAGAAGGCCGC
ACCCTTCTCCGGAGGGGGGAGGGGAGTGTTGCAATACCTTTCTGGGAGTTCTCTGCTGCCTCCTGGCTTCTGAGGA
CCGCCCTGGGCCTGGGAGAATCCCTTGCCCCCTCTTCCCCTCGTGATCTGCAACTCCAGTCTTACAACGAGACCGGT
ACCTC

**Brique pCMV BsaI C (SEQ ID NO : 50)**

GAGGTACCGGTCTCAACAAACCAATTCAGTCGACTGGATCCTAGTTATTAATAGTAATCAATTACGGGGTCATTAGTTCATAGCCCATATATGGAGTTCCGCGTTACATAACTTACGGTAAATGGCCCGCCTGGCTGACCGCCCAACGACCCCCGCCCATTGACGTCAATAATGACGTATGTTCCCATAGTAACGCCAATAGGGACTTTCCATTGACGTCAATGGGTGGAGTATTTACGGTAAACTGCCCACTTGGCAGTACATCAAGTGTATCATATGCCAAGTACGCCCCCTATTGACGTCAATGACGGTAAATGGCCCGCCTGGCATTATGCCCAGTACATGACCTTATGGGACTTTCCTACTTGGCAGTACATCTACGTATTAGTCATCGCTATTACCATGGTGATGCGGTTTTGGCAGTACATCAATGGGCGTGGATAGCGGTTTGACTCACGGGGATTTCCAAGTCTCCACCCCATTGACGTCAATGGGAGTTTGTTTTGGCACCAAAATCAACGGGACTTTCCAAAATGTCGTAACAACTCCGCCCCATTGACGCAAATGGGCGGTAGGCGTGTACGGTGGGAGGTCTATATAAGCAGAGCTGGTTTAGTGAACCGTCAGATCACTAGTCGACTAGGGATAACAGGGCACCCGAGACCGGTACCTC

**Brique hFUT3 BsaI A (SEQ ID NO : 38)**
**Brique BGHpA BsaI B (SEQ ID NO : 39)**
**Brique shB3Galt6 BsaI B (SEQ ID NO : 46)**

**Brique HygroR BsaI C (SEQ ID NO : 51)**

GAGGTACCGGTCTCACCAGCAGGCAGAAGTATGCAAAGCATGCATCTCAATTAGTCAGCAACCAGGTGTGGAAAGTCCCCAGGCTCCCCAGCAGGCAGAAGTATGCAAAGCATGCATCTCAATTAGTCAGCAACCATAGTCCCGCCCCTAACTCCGCCCATCCCGCCCCTAACTCCGCCCAGTTCCGCCCATTCTCCGCCCCATGGCTGACTAATTTTTTTTATTTATGCAGAGGCCGAGGCCGCCTCTGCCTCTGAGCTATTCCAGAAGTAGTGAGGAGGCTTTTTTGGAGGCCTAGGCTTTTGCAAAAAGCTCCCGGGAGCTTGTATATCCATTTTCGGATCTGATCAGCACGTGATGAAAAAGCCTGAACTCACCGCGACGTCTGTCGAGAAGTTTCTGATCGAAAAGTTCGACAGCGTGTCCGACCTGATGCAGCTCTCGGAGGGCGAAGAATCTCGTGCTTTCAGCTTCGATGTAGGAGGGCGTGGATATGTCCTGCGGGTAAATAGCTGCGCCGATGGTTTCTACAA

AGATCGTTATGTTTATCGGCACTTTGCATCGGCCGCGCTCCCGATTCCGGAAGTGCTTGACATTGGGGAATTCAGC
GAGAGCCTGACCTATTGCATCTCCCGCCGTGCACAGGGTGTCACGTTGCAAGACTTGCCTGAAACCGAACTGCCCG
CTGTTCTGCAGCCGGTCGCGGAGGCCATGGATGCGATCGCTGCGGCCGATCTTAGCCAGACGAGCGGGTTCGGCC
CATTCGGACCGCAAGGAATCGGTCAATACACTACATGGCGTGATTTCATATGCGCGATTGCTGATCCCCATGTGTAT
CACTGGCAAACTGTGATGGACGACACCGTCAGTGCGTCCGTCGCGCAGGCTCTCGATGAGCTGATGCTTTGGGCC
GAGGACTGCCCCGAAGTCCGGCACCTCGTGCACGCGGATTTCGGCTCCAACAATGTCCTGACGGACAATGGCCGC
ATAACAGCGGTCATTGACTGGAGCGAGGCGATGTTCGGGGATTCCCAATACGAGGTCGCCAACATCTTCTTCTGGA
GGCCGTGGTTGGCTTGTATGGAGCAGCAGACGCGCTACTTCGAGCGGAGGCATCCGGAGCTTGCAGGATCGCCGC
GGCTCCGGGCGTATATGCTCCGCATTGGTCTTGACCAACTCTATCAGAGCTTGGTTGACGGCAATTTCGATGATGC
AGCTTGGGCGCAGGGTCGATGCGACGCAATCGTCCGATCCGGAGCCGGGACTGTCGGGCGTACACAAATCGCCC
GCAGAAGCGCGGCCGTCTGGACCGATGGCTGTGTAGAAGTACTCGCCGATAGTGGAAACCGACGCCCCAGCACTC
GTCCGAGGGCAAAGGAATAGCACGTGCTACGAGATTTCGATTCCACCGCCGCCTTCTATGAAAGGTTGGGCTTCG
GAATCGTTTTCCGGGACGCCGGCTGGATGATCCTCCAGCGCGGGGATCTCATGCTGGAGTTCTTCGCCCACCCCAA
CTTGTTTATTGCAGCTTATAATGGTTACAAATAAAGCAATAGCATCACAAATTTCACAAATAAAGCATTTTTTTCACT
GCATTCTAGTTGTGGTTTGTCCAAACTCATCAATGTATCGTAGCGAGACCGGTACCTC

**Brique rosa26-3' BsaI A (SEQ ID NO : 52)**

GAGGTACCGGTCTCAGTAGAGATGGGCGGGAGTCTTCTGGGCAGGCTTAAAGGCTAACCTGGTGTGTGGGCGTT
GTCCTGCAGGGGAATTGAACAGGTGTAAAATTGGAGGGACAAGACTTCCCACAGATTTTCGGTTTTGTCGGGAAG
TTTTTTAATAGGGGCAAATAGGAAAATGGAGGATAGGAGTCATCTGGGGTTTATGCAGCAAAACTACAGGTATATT
GCTTGTATCCGCCTCGGAGATTTCCATGAGGAGATAAAGACATGTCACCCGAGTTTATACTCTCCTGCTTAGATCCT
ACTACAGTATGAAATACAGTGTCGCGAGGTAGACTATGTAAGCAGATTTAATCATTTTAAAGAGCCCAGTACTTCAT
ATCCATTTCTCCCGCTCCTTCTGCAGCCTTATCAAAAGGTATTTAGAACACTCATTTTAGCCCCATTTTCATTTATTAT
ACTGGCTTATCCAACCCCTAGACAGAGCATTGGCATTTTCCCTTTCCTGATCTTAGAAGTCTGATGACTCATGAAACC
AGACAGATTAGTTACATACACCACAAATCGAGGCTGTAGCTGGGGCCTCAACACTGCAGTTCTTTTATAACTCCTTA
GTACACTTTTTGTTGATCCTTTGCCTTGATCCTTAATTTTCAGTGTCTATCACCTCTCCCGTCAGGTGGTGTTCCACAT
TTGGGCCTATTCTCAGTCCAGGGAGTTTTACAACAATAGATGTATTGAGAATCCAACCTAAAGCTTAACTTTCCACT
CCCATGAATGCCTCTCTCCTTTTTCTCCATTATAACTGAGCTATAACCATTAATGGTTTCAGGTGGATGTCTCCTCCCC
CAATATACCTGATGTATCTACATATTGCCAGGCTGATATTTTAAGACATAAAAGGTATATTTCATTATTGAGCCACAT
GGTATTGATTACTGCTACTAAAATTTTGTCATTGTACACATCTGTAAAAGGTGGTTCCTTTTGGAATGCAAAGTTCA
GGTGTTTGTTGTCTTTCCTGACCTAAGGTCTTGTGAGCTTGTATTTTTTCTATTTAAGCAGTGCTTTCTCTTGGACTGG
CTTGACTCATGGCATTCTACACGTTATTGCTGGTCTAAATGTGATTTTGCCAAGCTTCTTCAGGACCTATAATTTTGC

TTGACTTGTAGCCAAACACAAGTAAAATGATTAAGCAACAAATGTATTTGTGAAGCTTGGTTTTTAGGTTGTTGTGT
TGTGTGTGCTTGTGCTCTATAATAATACTATCCAGGGGCTGGAGAGGTGGCTCGGAGTTCAAGAGCACAGACTGCT
CTTCCAGAAGTCCTGAGTTCAATTCCCAGCAACCACATGGTGGCTCACAACCATCTGTAATGGGATCTGATGCCCTC
TTCTGGTGTGTCTGAAGACCACAAGTGTATTCACATTAAATAAATAATCCTCCTTCTTCTTCTTTTTTTTTTTTTAAAG
AGAATACTGTCTCCAGTAGAATTACTGAAGTAATGAAATACTTTGTGTTTGTTCCAATATGGAAGCCAATAATCAAA
TACTCTTAAGCACTGGAAATGTACCAAGGAACTATTTTATTTAAGTGAACTGTGGACAGAGGAGCCATAACTGCAG
ACTTGTGGGATACAGAAGACCAATGCAGACTTAATGTCTTTTCTCTTACACTAAGCAATAAAGAAATAAAAATTGAA
CTTCTAGTATCCTATTTGTTAAACTGCTAGCTTTACTAACTTTTGTGCTTCATCTATACAAAGCTGAAAGCTAAGTCTG
CAGCCATTACTAAACATGAAAGCAAGTAATGATAATTTTGGATTTCAAAAATGTAGGGCCAGAGTTTAGCCAGCCA
GTGGTGGTGCTTGCCTTTATGCCTTAATCCCAGCACTCTGGAGGCAGAGACAGGCAGATCTCTGAGTTTGAGCCCA
GCCTGGTCTACACATCAAGTTCTATCTAGGATAGCCAGGAATACACACAGAAACCCTGTTGGGGAGGGGGGCTCT
GAGATTTCATAAAATTATAATTGAAGCATTCCCTAATGAGCCACTATGGATGTGGCTAAATCCGTCTACCTTTCTGA
TGAGATTTGGGTATTATTTTTTCTGTCTCTGCTGTTGGTTGGGTCTTTTGACACTGTGGGCTTTCTTAAAGCCTCCTTC
CCTGCCATGTGGACTCTTGTTTGCTACTAACTTCCCATGGCTTAAATGGCATGGCTTTTTGCCTTCTAAGGGCAGCTG
CTGAGATTTGCAGCCTGATTTCCAGGGTGGGGTTGGGAAATCTTTCAAACACTAAAATTGTCCTTTAATTTTTTTTTA
AAAAATGGGTTATATAATAAACCTCATAAAATAGTTATGAGGAGTGAGGTGGACTAATATTAATGAGTCCCTCCCC
TATAAAAGAGCTATTAAGGCTTTTTGTCTTATACTAACTTTTTTTTTAAATGTGGTATCTTTAGAACCAAGGGTCTTA
GAGTTTTAGTATACAGAAACTGTTGCATCGCTTAATCAGATTTTCTAGTTTCAAATCCAGAGAATCCAAATTCTTCAC
AGCCAAAGTCAAATTAAGAATTTCTGACTTTAATGTTATTTGCTACTGTGAATATAAAATGATAGCTTTTCCTGAGG
CAGGGTATCACTATGTATCTCTGCCTGATCTGCAACAAGATATGTAGACTAAAGTTCTGCCTGCTTTTGTCTCCTGAA
TACTAAGGTTAAAATGTAGTAATACTTTTGGAACTTGCAGGTCAGATTCTTTTATAGGGGACACACTAAGGGAGCT
TGGGTGATAGTTGGTAAATGTGTTTAAGTGATGAAAACTTGAATTATTATCACCGCAACCTACTTTTTAAAAAAAAA
AGCCAGGCCTGTTAGAGCATGCTAAGGGATCCCTAGGACTTGCTGAGCACACAAGAGTAGTACTTGGCAGGCTCC
TGGTGAGAGCATATTTCAAAAAACAAGGCAGACAACCAAGAAACTACAGTAAGGTTACCTGTCTTTAACCATCTGC
ATATACACAGGGATATTAAAATATTCCAAATAATATTTCATTCAAGTTTTCCCCCATCAAATTGGGACATGGATTTCT
CCGGTGAATAGGCAGAGTTGGAAACTAAACAAATGTTGGTTTTGTGATTTGTGAAATTGTTTTCAAGTGATAGTTA
AAGCCCATGAGATACAGAACAAAGCTGCTATTTCGAGGTCACTTGGTTATACTCAGAAGCACTTCTTTGGGTTTCCC
TGCACTATCCTGATCATGTGCTAGGCCTACCTTAGGCTGATTGTTGTTCAAATAACTTAAGTTTCCTGTCAGGTGATG
TCATATGATTTCATATATCAAGGCAAAACATGTTATATATGTTAAACATTTGGACTTAATGTGAAAGTTAGGTCTTTG
TGGGTTTTGATTTTAATTTCAAAACCTGAGCTAAATAAGTCATTTTACATGTCTTACATTTGGTGAATTGTATATTGT
GGTTTGCAGGCAAGACTCTCTGACCTAGTAACCCTCCTATAGAGCACTTTGCTGGGTCACAAGTCTAGGAGTCAAG
CATTTCACCTTGAAGTTGAGACGTTTTGTTAGTGTATACTAGTTATATGTTGGAGGACATGTTTATCCAGAAGATAT

TCAGGACTATTTTTGACTGGGCTAAGGAATTGATTCTGATTAGCACTGTTAGTGAGCATTGAGTGGCCTTTAGGCTT
GAATTGGAGTCACTTGTATATCTCAAATAATGCTGGCCTTTTTTAAAAAGCCCTTGTTCTTTATCACCCTGTTTTCTAC
ATAATTTTTGTTCAAAGAAATACTTGTTTGGATCTCCTTTTGACAACAATAGCATGTTTTCAAGCCATATTTTTTTTCC
TTTTTTTTTTTTTTTTTGGTTTTTCGAGACAGGGTTTCTCTGTATAGCCCTGGCTGTCCTGGAACTCACTTTGTAGACC
AGGCTGGCCTCGAACTCAGAAATCCGCCTGCCTCTGCCTCCTGAGTGCCGGGATTAAAGGCGTGCACCACCACGCC
TGGCTAAGTTGGATATTTTGTATATAACTATAACCAATACTAACTCCACTGGGTGGATTTTTAATTCAGTCAGTAGTC
TTAAGTGGTCTTTATTGGCCCTTATTAAAATCTACTGTTCACTCTAACAGAGGCTGTTGGACTAGTGGGACTAAGCA
ACTTCCTACGGATATACTAGCAGATAAGGGTCAGGGATAGAAACTAGTCTAGCGTTTTGTATACCTACCAGCTTATA
CTACCTTGTTCTGATAGAAATATTTAGGACATCTAGCTTATCTATTCGAGACCGGTACCTC

**V1.2 (SEQ ID NO : 53, exemple de vecteur du groupe V1.2)**

TATTGTAATACGGTTATCCACAGAATCAGGGGATAACGCAGGAAAGAACATGTGAGCAAAAGGCCAGCAAAAGG
CCAGGAACCGTAAAAAGGCCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGAAGCGTGGCGCTTTCTCA
TAGCTCACGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCCACTGGC
AGCAGCCACTGGTAACAGGATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTTTTTTTGTTTGCAAGCAGCAGATTACGCGCAGAAAAA
AAGGATCTCAAGAAGATCCTTTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGAAGTTTTAAATCAATCTAAAG
TATATATGAGTAAACTTGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACCATCTGGCCCCAGTGCT
GCAATGATACCGCGTGACCCACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACGCTCGTCGTTTGGTATGGCTT
CATTCAGCTCCGGTTCCCAACGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATGGCAGCACTGCATAATTCTCT
TACTGTCATGCCATCCGTAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCCACTC

GTGCACCCAACTGATCTTCAGCATCTTTTACTTTCACCAGCGTTTCTGGGTGAGCAAAAACAGGAAGGCAAAATGC CGCAAAAAAGGGAATAAGGGCGACACGGAAATGTTGAATACTCATACTCTTCCTTTTTCAATATTATTGAAGCATTT ATCAGGGTTATTGTCTCATGAGCGGATACATATTTGAATGTATTTAGAAAAATAAACAAATAGGGGTTCCGCGCAC ATTTCCCCGAAAAGTGCCAAGGACCCCGCGGCAGGCCCTCCGAGCGTGGTGGAGCCGTTCTGTGAGACAGCCGGG TACGAGTCGTGACGCTGGAAGGGGCAAGCGGGTGGTGGGCAGGAATGCGGTCCGCCCTGCAGCAACCGGAGGG GGAGGGAGAAGGGAGCGGAAAAGTCTCCACCGGACGCGGCCATGGCTCGGGGGGGGGGGGGCAGCGGAGGAG CGCTTCCGGCCGACGTCTCGTCGCTGATTGGCTTCTTTTCCTCCCGCCGTGTGTGAAAACACAAATGGCGTGTTTTG GTTGGCGTAAGGCGCCTGTCAGTTAACGGCAGCCGGAGTGCGCAGCCGCCGGCAGCCTCGCTCTGCCCACTGGGT GGGGCGGGAGGTAGGTGGGGTGAGGCGAGCTGGACGTGCGGGCGCGGTCGGCCTCTGGCGGGGCGGGGGAGG GGAGGGAGGGTCAGCGAAAGTAGCTCGCGCGCGAGCGGCCGCCCACCCTCCCCTTCCTCTGGGGGAGTCGTTTTA CCCGCCGCCGGCCGGGCCTCGTCGTCTGATTGGCTCTCGGGGCCCAGAAAACTGGCCCTTGCCATTGGCTCGTGTT CGTGCAAGTTGAGTCCATCCGCCGGCCAGCGGGGGCGGCGAGGAGGCGCTCCCAGGTTCCGGCCCTCCCCTCGGC CCCGCGCCGCAGAGTCTGGCCGCGCGCCCCTGCGCAACGTGGCAGGAAGCGCGCGCTGGGGGCGGGGACGGGC AGTAGGGCTGAGCGGCTGCGGGGCGGGTGCAAGCACGTTTCCGACTTGAGTTGCCTCAAGAGGGGCGTGCTGAG CCAGACCTCCATCGCGCACTCCGGGGAGTGGAGGGAAGGAGCGAGGGCTCAGTTGGGCTGTTTTGGAGGCAGGA AGCACTTGCTCTCCCAAAGTCGCTCTGAGTTGTTATCAGTAAGGGAGCTGCAGTGGAGTAGGCGGGGAGAAGGCC GCACCCTTCTCCGGAGGGGGGAGGGGAGTGTTGCAATACCTTTCTGGGAGTTCTCTGCTGCCTCCTGGCTTCTGAG GACCGCCCTGGGCCTGGGAGAATCCCTTGCCCCCTCTTCCCCTCGTGATCTGCAACTCCAGTCTTACAAACCAATTC AGTCGACTGGATCCTAGTTATTAATAGTAATCAATTACGGGGTCATTAGTTCATAGCCCATATATGGAGTTCCGCGT TACATAACTTACGGTAAATGGCCCGCCTGGCTGACCGCCCAACGACCCCCGCCCATTGACGTCAATAATGACGTAT GTTCCCATAGTAACGCCAATAGGGACTTTCCATTGACGTCAATGGGTGGAGTATTTACGGTAAACTGCCCACTTGG CAGTACATCAAGTGTATCATATGCCAAGTACGCCCCCTATTGACGTCAATGACGGTAAATGGCCCGCCTGGCATTAT GCCCAGTACATGACCTTATGGGACTTTCCTACTTGGCAGTACATCTACGTATTAGTCATCGCTATTACCATGGTGAT GCGGTTTTGGCAGTACATCAATGGGCGTGGATAGCGGTTTGACTCACGGGGATTTCCAAGTCTCCACCCCATTGAC GTCAATGGGAGTTTGTTTTGGCACCAAAATCAACGGGACTTTCCAAAATGTCGTAACAACTCCGCCCCATTGACGC AAATGGGCGGTAGGCGTGTACGGTGGGAGGTCTATATAAGCAGAGCTGGTTTAGTGAACCGTCAGATCACTAGTC GACTAGGGATAACAGGGCACCATGGATCCCCTGGGTGCAGCCAAGCCACAATGGCCATGGCGCCGCTGTCTGGCC GCACTGCTATTTCAGCTGCTGGTGGCTGTGTGTTTCTTCTCCTACCTGCGTGTGTCCCGAGACGATGCCACTGGATC CCCTAGGGCTCCCAGTGGGTCCTCCCGACAGGACACCACTCCCACCCGCCCCACCCTCCTGATCCTGCTATGGACAT GGCCTTTCCACATCCCTGTGGCTCTGTCCCGCTGTTCAGAGATGGTGCCCGGCACAGCCGACTGCCACATCACTGCC GACCGCAAGGTGTACCCACAGGCAGACACGGTCATCGTGCACCACTGGGATATCATGTCCAACCCTAAGTCACGCC TCCCACCTTCCCCGAGGCCGCAGGGGCAGCGCTGGATCTGGTTCAACTTGGAGCCACCCCCTAACTGCCAGCACCT

GGAAGCCCTGGACAGATACTTCAATCTCACCATGTCCTACCGCAGCGACTCCGACATCTTCACGCCCTACGGCTGGC
TGGAGCCGTGGTCCGGCCAGCCTGCCCACCCACCGCTCAACCTCTCGGCCAAGACCGAGCTGGTGGCCTGGGCGG
TGTCCAACTGGAAGCCGGACTCAGCCAGGGTGCGCTACTACCAGAGCCTGCAGGCTCATCTCAAGGTGGACGTGT
ACGGACGCTCCCACAAGCCCCTGCCCAAGGGGACCATGATGGAGACGCTGTCCCGGTACAAGTTCTACCTGGCCTT
CGAGAACTCCTTGCACCCCGACTACATCACCGAGAAGCTGTGGAGGAACGCCCTGGAGGCCTGGGCCGTGCCCGT
GGTGCTGGGCCCCAGCAGAAGCAACTACGAGAGGTTCCTGCCACCCGACGCCTTCATCCACGTGGACGACTTCCA
GAGCCCCAAGGACCTGGCCCGGTACCTGCAGGAGCTGGACAAGGACCACGCCCGCTACCTGAGCTACTTTCGCTG
GCGGGAGACGCTGCGGCCTCGCTCCTTCAGCTGGGCACTGGATTTCTGCAAGGCCTGCTGGAAACTGCAGCAGGA
ATCCAGGTACCAGACGGTGCGCAGCATAGCGGCTTGGTTCACCTGATCGACTGTGCCTTCTAGTTGCCAGCCATCT
GTTGTTTGCCCCTCCCCCGTGCCTTCCTTGACCCTGGAAGGTGCCACTCCCACTGTCCTTTCCTAATAAAATGAGGAA
ATTGCATCGCATTGTCTGAGTAGGTGTCATTCTATTCTGGGGGGTGGGGTGGGGCAGGACAGCAAGGGGGAGGA
TTGGGAGGACAATAGCAGGCATGCTGGGGATGCGGTGGGCTCTATGGCTTCGACAGGGTCGACAAGCTTTTCCAA
AAAAAAAGCATGAGGTGCAGTTGCGCCTTTCCTATCTCTTGAATAGGAAAGGCGCAACTGCACCTCATGCTGGATC
CCGCGTCCTTTCCACAAGATATATAAACCCAAGAAATCGAAATACTTTCAAGTTACGGTAAGCATATGATAGTCCAT
TTTAAAACATAATTTTAAAACTGCAAACTACCCAAGAAATTATTACTTTCTACGTCACGTATTTTGTACTAATATCTTT
GTGTTTACAGTCAAATTAATTCTAATTATCTCTCTAACAGCCTTGTATCGTATATGCAAATATGAAGGAATCATGGG
AAATAGGCCCTCTTCCTGCCCGACCTTGGCGCGCGCTCGGCGCGCGGTCACGCTCCGTCACGTGGTGCGTTTTGCC
AGCAGGCAGAAGTATGCAAAGCATGCATCTCAATTAGTCAGCAACCAGGTGTGGAAAGTCCCCAGGCTCCCCAGC
AGGCAGAAGTATGCAAAGCATGCATCTCAATTAGTCAGCAACCATAGTCCCGCCCCTAACTCCGCCCATCCCGCCCC
TAACTCCGCCCAGTTCCGCCCATTCTCCGCCCCATGGCTGACTAATTTTTTTTATTTATGCAGAGGCCGAGGCCGCCT
CTGCCTCTGAGCTATTCCAGAAGTAGTGAGGAGGCTTTTTTGGAGGCCTAGGCTTTTGCAAAAAGCTCCCGGGAGC
TTGTATATCCATTTTCGGATCTGATCAGCACGTGATGAAAAAGCCTGAACTCACCGCGACGTCTGTCGAGAAGTTTC
TGATCGAAAAGTTCGACAGCGTGTCCGACCTGATGCAGCTCTCGGAGGGCGAAGAATCTCGTGCTTTCAGCTTCGA
TGTAGGAGGGCGTGGATATGTCCTGCGGGTAAATAGCTGCGCCGATGGTTTCTACAAAGATCGTTATGTTTATCGG
CACTTTGCATCGGCCGCGCTCCCGATTCCGGAAGTGCTTGACATTGGGGAATTCAGCGAGAGCCTGACCTATTGCA
TCTCCCGCCGTGCACAGGGTGTCACGTTGCAAGACTTGCCTGAAACCGAACTGCCCGCTGTTCTGCAGCCGGTCGC
GGAGGCCATGGATGCGATCGCTGCGGCCGATCTTAGCCAGACGAGCGGGTTCGGCCCATTCGGACCGCAAGGAA
TCGGTCAATACACTACATGGCGTGATTTCATATGCGCGATTGCTGATCCCCATGTGTATCACTGGCAAACTGTGATG
GACGACACCGTCAGTGCGTCCGTCGCGCAGGCTCTCGATGAGCTGATGCTTTGGGCCGAGGACTGCCCCGAAGTC
CGGCACCTCGTGCACGCGGATTTCGGCTCCAACAATGTCCTGACGGACAATGGCCGCATAACAGCGGTCATTGACT
GGAGCGAGGCGATGTTCGGGGATTCCCAATACGAGGTCGCCAACATCTTCTTCTGGAGGCCGTGGTTGGCTTGTAT
GGAGCAGCAGACGCGCTACTTCGAGCGGAGGCATCCGGAGCTTGCAGGATCGCCGCGGCTCCGGGCGTATATGC

TCCGCATTGGTCTTGACCAACTCTATCAGAGCTTGGTTGACGGCAATTTCGATGATGCAGCTTGGGCGCAGGGTCG
ATGCGACGCAATCGTCCGATCCGGAGCCGGGACTGTCGGGCGTACACAAATCGCCCGCAGAAGCGCGGCCGTCTG
GACCGATGGCTGTGTAGAAGTACTCGCCGATAGTGGAAACCGACGCCCCAGCACTCGTCCGAGGGCAAAGGAATA
GCACGTGCTACGAGATTTCGATTCCACCGCCGCCTTCTATGAAAGGTTGGGCTTCGGAATCGTTTTCCGGGACGCC
GGCTGGATGATCCTCCAGCGCGGGGATCTCATGCTGGAGTTCTTCGCCCACCCCAACTTGTTTATTGCAGCTTATAA
TGGTTACAAATAAAGCAATAGCATCACAAATTTCACAAATAAAGCATTTTTTTCACTGCATTCTAGTTGTGGTTTGTC
CAAACTCATCAATGTATCGTAGAGATGGGCGGGAGTCTTCTGGGCAGGCTTAAAGGCTAACCTGGTGTGTGGGCG
TTGTCCTGCAGGGGAATTGAACAGGTGTAAAATTGGAGGGACAAGACTTCCCACAGATTTTCGGTTTTGTCGGGAA
GTTTTTTAATAGGGGCAAATAGGAAAATGGAGGATAGGAGTCATCTGGGGTTTATGCAGCAAAACTACAGGTATA
TTGCTTGTATCCGCCTCGGAGATTTCCATGAGGAGATAAAGACATGTCACCCGAGTTTATACTCTCCTGCTTAGATC
CTACTACAGTATGAAATACAGTGTCGCGAGGTAGACTATGTAAGCAGATTTAATCATTTTAAAGAGCCCAGTACTTC
ATATCCATTTCTCCCGCTCCTTCTGCAGCCTTATCAAAAGGTATTTAGAACACTCATTTTAGCCCCATTTTCATTTATT
ATACTGGCTTATCCAACCCCTAGACAGAGCATTGGCATTTTCCCTTTCCTGATCTTAGAAGTCTGATGACTCATGAAA
CCAGACAGATTAGTTACATACACCACAAATCGAGGCTGTAGCTGGGGCCTCAACACTGCAGTTCTTTTATAACTCCT
TAGTACACTTTTTGTTGATCCTTTGCCTTGATCCTTAATTTTCAGTGTCTATCACCTCTCCCGTCAGGTGGTGTTCCAC
ATTTGGGCCTATTCTCAGTCCAGGGAGTTTTACAACAATAGATGTATTGAGAATCCAACCTAAAGCTTAACTTTCCA
CTCCCATGAATGCCTCTCTCCTTTTTCTCCATTATAACTGAGCTATAACCATTAATGGTTTCAGGTGGATGTCTCCTCC
CCCAATATACCTGATGTATCTACATATTGCCAGGCTGATATTTTAAGACATAAAAGGTATATTTCATTATTGAGCCAC
ATGGTATTGATTACTGCTACTAAAATTTTGTCATTGTACACATCTGTAAAAGGTGGTTCCTTTTGGAATGCAAAGTTC
AGGTGTTTGTTGTCTTTCCTGACCTAAGGTCTTGTGAGCTTGTATTTTTTCTATTTAAGCAGTGCTTTCTCTTGGACTG
GCTTGACTCATGGCATTCTACACGTTATTGCTGGTCTAAATGTGATTTTGCCAAGCTTCTTCAGGACCTATAATTTTG
CTTGACTTGTAGCCAAACACAAGTAAAATGATTAAGCAACAAATGTATTTGTGAAGCTTGGTTTTTAGGTTGTTGTG
TTGTGTGTGCTTGTGCTCTATAATAATACTATCCAGGGGCTGGAGAGGTGGCTCGGAGTTCAAGAGCACAGACTGC
TCTTCCAGAAGTCCTGAGTTCAATTCCCAGCAACCACATGGTGGCTCACAACCATCTGTAATGGGATCTGATGCCCT
CTTCTGGTGTGTCTGAAGACCACAAGTGTATTCACATTAAATAAATAATCCTCCTTCTTCTTCTTTTTTTTTTTTTAAA
GAGAATACTGTCTCCAGTAGAATTACTGAAGTAATGAAATACTTTGTGTTTGTTCCAATATGGAAGCCAATAATCAA
ATACTCTTAAGCACTGGAAATGTACCAAGGAACTATTTTATTTAAGTGAACTGTGGACAGAGGAGCCATAACTGCA
GACTTGTGGGATACAGAAGACCAATGCAGACTTAATGTCTTTTCTCTTACACTAAGCAATAAAGAAATAAAAATTG
AACTTCTAGTATCCTATTTGTTAAACTGCTAGCTTTACTAACTTTTGTGCTTCATCTATACAAAGCTGAAAGCTAAGT
CTGCAGCCATTACTAAACATGAAAGCAAGTAATGATAATTTTGGATTTCAAAAATGTAGGGCCAGAGTTTAGCCAG
CCAGTGGTGGTGCTTGCCTTTATGCCTTAATCCCAGCACTCTGGAGGCAGAGACAGGCAGATCTCTGAGTTTGAGC
CCAGCCTGGTCTACACATCAAGTTCTATCTAGGATAGCCAGGAATACACACAGAAACCCTGTTGGGGAGGGGGGC

TCTGAGATTTCATAAAATTATAATTGAAGCATTCCCTAATGAGCCACTATGGATGTGGCTAAATCCGTCTACCTTTCT

GATGAGATTTGGGTATTATTTTTTCTGTCTCTGCTGTTGGTTGGGTCTTTTGACACTGTGGGCTTTCTTAAAGCCTCC

TTCCCTGCCATGTGGACTCTTGTTTGCTACTAACTTCCCATGGCTTAAATGGCATGGCTTTTTGCCTTCTAAGGGCAG

CTGCTGAGATTTGCAGCCTGATTTCCAGGGTGGGGTTGGGAAATCTTTCAAACACTAAAATTGTCCTTTAATTTTTTT

TTAAAAAATGGGTTATATAATAAACCTCATAAAATAGTTATGAGGAGTGAGGTGGACTAATATTAATGAGTCCCTC

CCCTATAAAAGAGCTATTAAGGCTTTTTGTCTTATACTAACTTTTTTTTTAAATGTGGTATCTTTAGAACCAAGGGTC

TTAGAGTTTTAGTATACAGAAACTGTTGCATCGCTTAATCAGATTTTCTAGTTTCAAATCCAGAGAATCCAAATTCTT

CACAGCCAAAGTCAAATTAAGAATTTCTGACTTTAATGTTATTTGCTACTGTGAATATAAAATGATAGCTTTTCCTGA

GGCAGGGTATCACTATGTATCTCTGCCTGATCTGCAACAAGATATGTAGACTAAAGTTCTGCCTGCTTTTGTCTCCT

GAATACTAAGGTTAAAATGTAGTAATACTTTTGGAACTTGCAGGTCAGATTCTTTTATAGGGGACACACTAAGGGA

GCTTGGGTGATAGTTGGTAAATGTGTTTAAGTGATGAAAACTTGAATTATTATCACCGCAACCTACTTTTTAAAAAA

AAAAGCCAGGCCTGTTAGAGCATGCTAAGGGATCCCTAGGACTTGCTGAGCACACAAGAGTAGTACTTGGCAGGC

TCCTGGTGAGAGCATATTTCAAAAAACAAGGCAGACAACCAAGAAACTACAGTAAGGTTACCTGTCTTTAACCATC

TGCATATACACAGGGATATTAAAATATTCCAAATAATATTTCATTCAAGTTTTCCCCCATCAAATTGGGACATGGATT

TCTCCGGTGAATAGGCAGAGTTGGAAACTAAACAAATGTTGGTTTTGTGATTTGTGAAATTGTTTTCAAGTGATAGT

TAAAGCCCATGAGATACAGAACAAAGCTGCTATTTCGAGGTCACTTGGTTATACTCAGAAGCACTTCTTTGGGTTTC

CCTGCACTATCCTGATCATGTGCTAGGCCTACCTTAGGCTGATTGTTGTTCAAATAACTTAAGTTTCCTGTCAGGTGA

TGTCATATGATTTCATATATCAAGGCAAAACATGTTATATATGTTAAACATTTGGACTTAATGTGAAAGTTAGGTCTT

TGTGGGTTTTGATTTTAATTTCAAAACCTGAGCTAAATAAGTCATTTTACATGTCTTACATTTGGTGAATTGTATATT

GTGGTTTGCAGGCAAGACTCTCTGACCTAGTAACCCTCCTATAGAGCACTTTGCTGGGTCACAAGTCTAGGAGTCA

AGCATTTCACCTTGAAGTTGAGACGTTTTGTTAGTGTATACTAGTTATATGTTGGAGGACATGTTTATCCAGAAGAT

ATTCAGGACTATTTTTGACTGGGCTAAGGAATTGATTCTGATTAGCACTGTTAGTGAGCATTGAGTGGCCTTTAGGC

TTGAATTGGAGTCACTTGTATATCTCAAATAATGCTGGCCTTTTTTAAAAAGCCCTTGTTCTTTATCACCCTGTTTTCT

ACATAATTTTTGTTCAAAGAAATACTTGTTTGGATCTCCTTTTGACAACAATAGCATGTTTTCAAGCCATATTTTTTTT

CCTTTTTTTTTTTTTTTTTTGGTTTTTCGAGACAGGGTTTCTCTGTATAGCCCTGGCTGTCCTGGAACTCACTTTGTAGA

CCAGGCTGGCCTCGAACTCAGAAATCCGCCTGCCTCTGCCTCCTGAGTGCCGGGATTAAAGGCGTGCACCACCACG

CCTGGCTAAGTTGGATATTTTGTATATAACTATAACCAATACTAACTCCACTGGGTGGATTTTTAATTCAGTCAGTAG

TCTTAAGTGGTCTTTATTGGCCCTTATTAAAATCTACTGTTCACTCTAACAGAGGCTGTTGGACTAGTGGGACTAAG

CAACTTCCTACGGATATACTAGCAGATAAGGGTCAGGGATAGAAACTAGTCTAGCGTTTTGTATACCTACCAGCTTA

TACTACCTTGTTCTGATAGAAATATTTAGGACATCTAGCTTATC

L’analyse de restriction par triple digestion EcoRV, PvuI, SalI de la réalisation 4 est illustrée figure 12.

Vérification fonctionnelle de la réalisation 4 : des cellules eucaryotes (ex : cellules cancéreuses mammaires de souris 4T1) sont transfectées avec le vecteur V1.2 selon un protocole usuel (exemple lipotransfection ou électroporation). Après transfection les cellules sont traitées avec de l’hygromycine (50µg-mL-1 pendant 7-14 jours). Les cellules sont ensuite collectées et lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel.

Conditions spécifiques de la PCR quantitative:

40 cycles des trois étapes suivantes sont réalisés: dénaturation 94°C-30s; hybridation 60°C-30s; extension 72°C-30s.

Amorces utilisées :

**mB3Galt6**

BETA3Galt6ms2-s ACCACTCTGTTGTACCTGGC (SEQ ID NO : 42).
BETA3Galt6ms2-as CACACGTCCTCGGGTCC (SEQ ID NO : 43).

**HFUT3**

FUT35 CACTAGTCGACTAGGGATAACAGG (SEQ ID NO : 44).
FUT33 ATGTCCATAGCAGGATCAGGAG (SEQ ID NO : 45).

**Réalisation 5**

**Groupe de vecteurs V1.3** : **Vecteurs V1.2 permettant d'éliminer les cellules hôtes ayant intégré un ou des transgènes par recombinaisons non-homologue**

$$U1 + U3b + U2 + U3a + U3c + U3d$$

U1 : Unité fonctionnelle bactérienne
U2 : nxU2a + mxU2b, n≥0, m≥0 et n+m≥2
U2a : Unité fonctionnelle d'expression dont le promoteur est dépendant de l'ARN polymerase II et dont le produit d'expression est une protéine
U2b : Unité fonctionnelle d'expression dont le promoteur est dépendant de l'ARN polymerase III et dont le produit d'expression est un ARN non codant
U3a = cassette de sélection positive
U3b = motif 5' d'une séquence de recombinaison homologue X
U3c = motif 3' de la séquence de recombinaison homologue X
U3d est un cassette de sélection négative.

Exemple : Vecteur permettant d'exprimer l'enzyme hFUT3 tout en réprimant l'expression de mB3Galt6.

| | |
|---|---|
| U1 | ori-Amp |
| U3b | Rosa26 5' |
| U2a | promoteur CMV<br>hFUT3 cDNA<br>Terminateur BPA |
| U2b | cassette shRNA mB3Galt6 |
| U3a | Hygomicin resistance |
| U3c | Rosa26 3' |

(suite)

| | |
|---|---|
| U3d | promotor ef1a<br>Thimidine kinase<br>HSV Tk terminator |

Liste des briques utilisées pour construire le vecteur V1.3 (Figure 13)

**Brique Ori-AmpR Bsal B (SEQ ID NO : 36)**
**Brique rosa26-5' Bsal A (SEQ ID NO : 49)**
**Brique pCMV Bsal C (SEQ ID NO : 50)**
**Brique hFUT3 Bsal A (SEQ ID NO : 38)**
**Brique BGHpA Bsal B (SEQ ID NO : 39)**
**Brique shB3Galt6 Bsal B (SEQ ID NO : 46)**
**Brique HygroR Bsal C (SEQ ID NO : 51)**

**Brique rosa26-3' Bsal B (SEQ ID NO : 54)**

GAGGTACCGGTCTCAGTAGAGATGGGCGGGAGTCTTCTGGGCAGGCTTAAAGGCTAACCTGGTGTGTGGGCGTT

GTCCTGCAGGGGAATTGAACAGGTGTAAAATTGGAGGGACAAGACTTCCCACAGATTTTCGGTTTTGTCGGGAAG

TTTTTTAATAGGGGCAAATAGGAAAATGGAGGATAGGAGTCATCTGGGGTTTATGCAGCAAAACTACAGGTATATT

GCTTGTATCCGCCTCGGAGATTTCCATGAGGAGATAAAGACATGTCACCCGAGTTTATACTCTCCTGCTTAGATCCT
ACTACAGTATGAAATACAGTGTCGCGAGGTAGACTATGTAAGCAGATTTAATCATTTTAAAGAGCCCAGTACTTCAT
ATCCATTTCTCCCGCTCCTTCTGCAGCCTTATCAAAAGGTATTTAGAACACTCATTTTAGCCCCATTTTCATTTATTAT
ACTGGCTTATCCAACCCCTAGACAGAGCATTGGCATTTTCCCTTTCCTGATCTTAGAAGTCTGATGACTCATGAAACC
AGACAGATTAGTTACATACACCACAAATCGAGGCTGTAGCTGGGGCCTCAACACTGCAGTTCTTTTATAACTCCTTA
GTACACTTTTTGTTGATCCTTTGCCTTGATCCTTAATTTTCAGTGTCTATCACCTCTCCCGTCAGGTGGTGTTCCACAT
TTGGGCCTATTCTCAGTCCAGGGAGTTTTACAACAATAGATGTATTGAGAATCCAACCTAAAGCTTAACTTTCCACT
CCCATGAATGCCTCTCTCCTTTTTCTCCATTATAACTGAGCTATAACCATTAATGGTTTCAGGTGGATGTCTCCTCCCC
CAATATACCTGATGTATCTACATATTGCCAGGCTGATATTTTAAGACATAAAAGGTATATTTCATTATTGAGCCACAT
GGTATTGATTACTGCTACTAAAATTTTGTCATTGTACACATCTGTAAAAGGTGGTTCCTTTTGGAATGCAAAGTTCA
GGTGTTTGTTGTCTTTCCTGACCTAAGGTCTTGTGAGCTTGTATTTTTTCTATTTAAGCAGTGCTTTCTCTTGGACTGG
CTTGACTCATGGCATTCTACACGTTATTGCTGGTCTAAATGTGATTTTGCCAAGCTTCTTCAGGACCTATAATTTTGC
TTGACTTGTAGCCAAACACAAGTAAAATGATTAAGCAACAAATGTATTTGTGAAGCTTGGTTTTTAGGTTGTTGTGT
TGTGTGTGCTTGTGCTCTATAATAATACTATCCAGGGGCTGGAGAGGTGGCTCGGAGTTCAAGAGCACAGACTGCT
CTTCCAGAAGTCCTGAGTTCAATTCCCAGCAACCACATGGTGGCTCACAACCATCTGTAATGGGATCTGATGCCCTC
TTCTGGTGTGTCTGAAGACCACAAGTGTATTCACATTAAATAAATAATCCTCCTTCTTCTTCTTTTTTTTTTTTTAAAG
AGAATACTGTCTCCAGTAGAATTACTGAAGTAATGAAATACTTTGTGTTTGTTCCAATATGGAAGCCAATAATCAAA
TACTCTTAAGCACTGGAAATGTACCAAGGAACTATTTTATTTAAGTGAACTGTGGACAGAGGAGCCATAACTGCAG
ACTTGTGGGATACAGAAGACCAATGCAGACTTAATGTCTTTTCTCTTACACTAAGCAATAAAGAAATAAAAATTGAA
CTTCTAGTATCCTATTTGTTAAACTGCTAGCTTTACTAACTTTTGTGCTTCATCTATACAAAGCTGAAAGCTAAGTCTG
CAGCCATTACTAAACATGAAAGCAAGTAATGATAATTTTGGATTTCAAAAATGTAGGGCCAGAGTTTAGCCAGCCA
GTGGTGGTGCTTGCCTTTATGCCTTAATCCCAGCACTCTGGAGGCAGAGACAGGCAGATCTCTGAGTTTGAGCCCA
GCCTGGTCTACACATCAAGTTCTATCTAGGATAGCCAGGAATACACACAGAAACCCTGTTGGGGAGGGGGGCTCT
GAGATTTCATAAAATTATAATTGAAGCATTCCCTAATGAGCCACTATGGATGTGGCTAAATCCGTCTACCTTTCTGA
TGAGATTTGGGTATTATTTTTTCTGTCTCTGCTGTTGGTTGGGTCTTTTGACACTGTGGGCTTTCTTAAAGCCTCCTTC
CCTGCCATGTGGACTCTTGTTTGCTACTAACTTCCCATGGCTTAAATGGCATGGCTTTTTGCCTTCTAAGGGCAGCTG
CTGAGATTTGCAGCCTGATTTCCAGGGTGGGGTTGGGAAATCTTTCAAACACTAAAATTGTCCTTTAATTTTTTTTTA
AAAAATGGGTTATATAATAAACCTCATAAAATAGTTATGAGGAGTGAGGTGGACTAATATTAATGAGTCCCTCCCC
TATAAAAGAGCTATTAAGGCTTTTTGTCTTATACTAACTTTTTTTTTAAATGTGGTATCTTTAGAACCAAGGGTCTTA
GAGTTTTAGTATACAGAAACTGTTGCATCGCTTAATCAGATTTTCTAGTTTCAAATCCAGAGAATCCAAATTCTTCAC
AGCCAAAGTCAAATTAAGAATTTCTGACTTTAATGTTATTTGCTACTGTGAATATAAAATGATAGCTTTTCCTGAGG
CAGGGTATCACTATGTATCTCTGCCTGATCTGCAACAAGATATGTAGACTAAAGTTCTGCCTGCTTTTGTCTCCTGAA

TACTAAGGTTAAAATGTAGTAATACTTTTGGAACTTGCAGGTCAGATTCTTTTATAGGGGACACACTAAGGGAGCT
TGGGTGATAGTTGGTAAATGTGTTTAAGTGATGAAAACTTGAATTATTATCACCGCAACCTACTTTTTAAAAAAAAA
AGCCAGGCCTGTTAGAGCATGCTAAGGGATCCCTAGGACTTGCTGAGCACACAAGAGTAGTACTTGGCAGGCTCC
TGGTGAGAGCATATTTCAAAAAACAAGGCAGACAACCAAGAAACTACAGTAAGGTTACCTGTCTTTAACCATCTGC
ATATACACAGGGATATTAAAATATTCCAAATAATATTTCATTCAAGTTTTCCCCCATCAAATTGGGACATGGATTTCT
CCGGTGAATAGGCAGAGTTGGAAACTAAACAAATGTTGGTTTTGTGATTTGTGAAATTGTTTTCAAGTGATAGTTA
AAGCCCATGAGATACAGAACAAAGCTGCTATTTCGAGGTCACTTGGTTATACTCAGAAGCACTTCTTTGGGTTTCCC
TGCACTATCCTGATCATGTGCTAGGCCTACCTTAGGCTGATTGTTGTTCAAATAACTTAAGTTTCCTGTCAGGTGATG
TCATATGATTTCATATATCAAGGCAAAACATGTTATATATGTTAAACATTTGGACTTAATGTGAAAGTTAGGTCTTTG
TGGGTTTTGATTTTAATTTCAAAACCTGAGCTAAATAAGTCATTTTACATGTCTTACATTTGGTGAATTGTATATTGT
GGTTTGCAGGCAAGACTCTCTGACCTAGTAACCCTCCTATAGAGCACTTTGCTGGGTCACAAGTCTAGGAGTCAAG
CATTTCACCTTGAAGTTGAGACGTTTTGTTAGTGTATACTAGTTATATGTTGGAGGACATGTTTATCCAGAAGATAT
TCAGGACTATTTTTGACTGGGCTAAGGAATTGATTCTGATTAGCACTGTTAGTGAGCATTGAGTGGCCTTTAGGCTT
GAATTGGAGTCACTTGTATATCTCAAATAATGCTGGCCTTTTTTAAAAAGCCCTTGTTCTTTATCACCCTGTTTTCTAC
ATAATTTTTGTTCAAAGAAATACTTGTTTGGATCTCCTTTTGACAACAATAGCATGTTTTCAAGCCATATTTTTTTTCC
TTTTTTTTTTTTTTTTTGGTTTTTCGAGACAGGGTTTCTCTGTATAGCCCTGGCTGTCCTGGAACTCACTTTGTAGACC
AGGCTGGCCTCGAACTCAGAAATCCGCCTGCCTCTGCCTCCTGAGTGCCGGGATTAAAGGCGTGCACCACCACGCC
TGGCTAAGTTGGATATTTTGTATATAACTATAACCAATACTAACTCCACTGGGTGGATTTTTAATTCAGTCAGTAGTC
TTAAGTGGTCTTTATTGGCCCTTATTAAAATCTACTGTTCACTCTAACAGAGGCTGTTGGACTAGTGGGACTAAGCA
ACTTCCTACGGATATACTAGCAGATAAGGGTCAGGGATAGAAACTAGTCTAGCGTTTTGTATACCTACCAGCTTATA
CTACCTTGTTCTGATAGAAATATTTAGGACATCTAGCTTATCATGCCGAGACCGGTACCTC

**Brique pEF1a BsaI A (SEQ ID NO : 55)**

GAGGTACCGGTCTCAATGCaaGGAACCAATTCAGTCGACTGGATCCCGATGGCTCCGGTGCCCGTCAGTGGGCAG
AGCGCACATCGCCCACAGTCCCCGAGAAGTTGGGGGGAGGGGTCGGCAATTGAACCGGTGCCTAGAGAAGGTGG
CGCGGGGTAAACTGGGAAAGTGATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAACACAGGTCCGCGGCCCCGAACTAG
GCCTAGGCGTCTGATCACTAGTGACTCTAGTCCTAGTCGACTAGGGATAACAGGGGCCCCGAGACCGGTACCTC

**Brique TK BsaI A (SEQ ID NO : 56)**

GAGGTACCGGTCTCAGCCCATGGCTTCGTACCCCTGCCATCAACACGCGTCTGCGTTCGACCAGGCTGCGCGTTCTC
GCGGCCATAGCAACCGACGTACGGCGTTGCGCCCTCGCCGGCAGCAAGAAGCCACGGAAGTCCGCCTGGAGCAG

AAAATGCCCACGCTACTGCGGGTTTATATAGACGGTCCTCACGGGATGGGGAAAACCACCACCACGCAACTGCTGGTGGCCCTGGGTTCGCGCGACGATATCGTCTACGTACCCGAGCCGATGACTTACTGGCAGGTGCTGGGGGCTTCCGAGACAATCGCGAACATCTACACCACACAACACCGCCTCGACCAGGGTGAGATATCGGCCGGGGACGCGGCGGTGGTAATGACAAGCGCCCAGATAACAATGGGCATGCCTTATGCCGTGACCGACGCCGTTCTGGCTCCTCATATCGGGGGGGAGGCTGGGAGCTCACATGCCCCGCCCCCGGCCCTCACCCTCATCTTCGACCGCCATCCCATCGCCGCCCTCCTGTGCTACCCGGCCGCGCGATACCTTATGGGCAGCATGACCCCCCAGGCCGTGCTGGCGTTCGTGGCCCTCATCCCGCCGACCTTGCCCGGCACAAACATCGTGTTGGGGGCCCTTCCGGAGGACAGACACATCGACCGCCTGGCCAAACGCCAGCGCCCCGGCGAGCGGCTTGACCTGGCTATGCTGGCCGCGATTCGCCGCGTTTACGGGCTGCTTGCCAATACGGTGCGGTATCTGCAGGGCGGCGGGTCGTGGCGGGAGGATTGGGGACAGCTTTCGGGGACGGCCGTGCCGCCCCAGGGTGCCGAGCCCCAGAGCAACGCGGGCCCACGACCCCATATCGGGGACACGTTATTTACCCTGTTTCGGGCCCCCGAGTTGCTGGCCCCCAACGGCGACCTGTACAACGTGTTTGCCTGGGCCTTGGACGTCTTGGCCAAACGCCTCCGTCCCATGCACGTCTTTATCCTGGATTACGACCAATCGCCCGCCGGCTGCCGGGACGCCCTGCTGCAACTTACCTCCGGGATGGTCCAGACCCACGTCACCACCCCCGGCTCCATACCGACGATCTGCGACCTGGCGCGCACGTTTGCCCGGGAGATGGGGGAGGCTAACTGACCGCCGAGACCGGTACCTC

**Brique Tkter BsaI A (SEQ ID NO : 57)**

GAGGTACCGGTCTCACCGCGGGGGAGGCTAACTGAAACACGGAAGGAGACAATACCGGAAGGAACCCGCGCTATGACGGCAATAAAAAGACAGAATAAAACGCACGGTGTTGGGTCGTTTGTTCATAAACGCGGGGTTCGGTCCCAGGGCTGGCACTCTGTCGATACCCCACCGAGGCCCCATTGGGGCCAATACGCCCGCGTTTCTTCCTTTTCCCCACCCCACCCCCCAAGTTCGGGTGAAGGCCCAGGGCTCGCAGCCAACGTCGGGGCGGCAGGCCCTGCCATAGCCTATTCGAGACCGGTACCTC

**V1.3 (SEQ ID NO : 58, exemple de vecteur du groupe V1.3)**

TATTGTAATACGGTTATCCACAGAATCAGGGGATAACGCAGGAAAGAACATGTGAGCAAAAGGCCAGCAAAAGGCCAGGAACCGTAAAAAGGCCGCGTTGCTGGCGTTTTTCCATAGGCTCCGCCCCCCTGACGAGCATCACAAAAATCGACGCTCAAGTCAGAGGTGGCGAAACCCGACAGGACTATAAAGATACCAGGCGTTTCCCCCTGGAAGCTCCCTCGTGCGCTCTCCTGTTCCGACCCTGCCGCTTACCGGATACCTGTCCGCCTTTCTCCCTTCGGGAAGCGTGGCGCTTTCTCATAGCTCACGCTGTAGGTATCTCAGTTCGGTGTAGGTCGTTCGCTCCAAGCTGGGCTGTGTGCACGAACCCCCCGTTCAGCCCGACCGCTGCGCCTTATCCGGTAACTATCGTCTTGAGTCCAACCCGGTAAGACACGACTTATCGCCACTGGCAGCAGCCACTGGTAACAGGATTAGCAGAGCGAGGTATGTAGGCGGTGCTACAGAGTTCTTGAAGTGGTGGCCTAACTACGGCTACACTAGAAGAACAGTATTTGGTATCTGCGCTCTGCTGAAGCCAGTTACCTTCGGAAAAAGAGTTGGTAGCTCTTGATCCGGCAAACAAACCACCGCTGGTAGCGGTTTTTTTGTTTGCAAGCAGCAGATTACGCGCAGAAAAA

AAGGATCTCAAGAAGATCCTTTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGAAGTTTTAAATCAATCTAAAG
TATATATGAGTAAACTTGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACCATCTGGCCCCAGTGCT
GCAATGATACCGCGTGACCCACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACGCTCGTCGTTTGGTATGGCTT
CATTCAGCTCCGGTTCCCAACGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATGGCAGCACTGCATAATTCTCT
TACTGTCATGCCATCCGTAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCAAGGACCCCGCGGCAGGCCCTCCGAGCGTGGTGGAGCCGTTCTGTGAGACAGCCGGG
TACGAGTCGTGACGCTGGAAGGGGCAAGCGGGTGGTGGGCAGGAATGCGGTCCGCCCTGCAGCAACCGGAGGG
GGAGGGAGAAGGGAGCGGAAAAGTCTCCACCGGACGCGGCCATGGCTCGGGGGGGGGGGGGCAGCGGAGGAG
CGCTTCCGGCCGACGTCTCGTCGCTGATTGGCTTCTTTTCCTCCCGCCGTGTGTGAAAACACAAATGGCGTGTTTTG
GTTGGCGTAAGGCGCCTGTCAGTTAACGGCAGCCGGAGTGCGCAGCCGCCGGCAGCCTCGCTCTGCCCACTGGGT
GGGGCGGGAGGTAGGTGGGGTGAGGCGAGCTGGACGTGCGGGCGCGGTCGGCCTCTGGCGGGGCGGGGGAGG
GGAGGGAGGGTCAGCGAAAGTAGCTCGCGCGCGAGCGGCCGCCCACCCTCCCCTTCCTCTGGGGGAGTCGTTTTA
CCCGCCGCCGGCCGGGCCTCGTCGTCTGATTGGCTCTCGGGGCCCAGAAAACTGGCCCTTGCCATTGGCTCGTGTT
CGTGCAAGTTGAGTCCATCCGCCGGCCAGCGGGGGCGGCGAGGAGGCGCTCCCAGGTTCCGGCCCTCCCCTCGGC
CCCGCGCCGCAGAGTCTGGCCGCGCGCCCCTGCGCAACGTGGCAGGAAGCGCGCGCTGGGGGCGGGGACGGGC
AGTAGGGCTGAGCGGCTGCGGGGCGGGTGCAAGCACGTTTCCGACTTGAGTTGCCTCAAGAGGGGCGTGCTGAG
CCAGACCTCCATCGCGCACTCCGGGGAGTGGAGGGAAGGAGCGAGGGCTCAGTTGGGCTGTTTTGGAGGCAGGA
AGCACTTGCTCTCCCAAAGTCGCTCTGAGTTGTTATCAGTAAGGGAGCTGCAGTGGAGTAGGCGGGGAGAAGGCC
GCACCCTTCTCCGGAGGGGGGAGGGGAGTGTTGCAATACCTTTCTGGGAGTTCTCTGCTGCCTCCTGGCTTCTGAG
GACCGCCCTGGGCCTGGGAGAATCCCTTGCCCCCTCTTCCCCTCGTGATCTGCAACTCCAGTCTTACAAACCAATTC
AGTCGACTGGATCCTAGTTATTAATAGTAATCAATTACGGGGTCATTAGTTCATAGCCCATATATGGAGTTCCGCGT
TACATAACTTACGGTAAATGGCCCGCCTGGCTGACCGCCCAACGACCCCCGCCCATTGACGTCAATAATGACGTAT

GTTCCCATAGTAACGCCAATAGGGACTTTCCATTGACGTCAATGGGTGGAGTATTTACGGTAAACTGCCCACTTGG
CAGTACATCAAGTGTATCATATGCCAAGTACGCCCCCTATTGACGTCAATGACGGTAAATGGCCCGCCTGGCATTAT
GCCCAGTACATGACCTTATGGGACTTTCCTACTTGGCAGTACATCTACGTATTAGTCATCGCTATTACCATGGTGAT
GCGGTTTTGGCAGTACATCAATGGGCGTGGATAGCGGTTTGACTCACGGGGATTTCCAAGTCTCCACCCCATTGAC
GTCAATGGGAGTTTGTTTTGGCACCAAAATCAACGGGACTTTCCAAAATGTCGTAACAACTCCGCCCCATTGACGC
AAATGGGCGGTAGGCGTGTACGGTGGGAGGTCTATATAAGCAGAGCTGGTTTAGTGAACCGTCAGATCACTAGTC
GACTAGGGATAACAGGGCACCATGGATCCCCTGGGTGCAGCCAAGCCACAATGGCCATGGCGCCGCTGTCTGGCC
GCACTGCTATTTCAGCTGCTGGTGGCTGTGTGTTTCTTCTCCTACCTGCGTGTGTCCCGAGACGATGCCACTGGATC
CCCTAGGGCTCCCAGTGGGTCCTCCCGACAGGACACCACTCCCACCCGCCCCACCCTCCTGATCCTGCTATGGACAT
GGCCTTTCCACATCCCTGTGGCTCTGTCCCGCTGTTCAGAGATGGTGCCCGGCACAGCCGACTGCCACATCACTGCC
GACCGCAAGGTGTACCCACAGGCAGACACGGTCATCGTGCACCACTGGGATATCATGTCCAACCCTAAGTCACGCC
TCCCACCTTCCCCGAGGCCGCAGGGGCAGCGCTGGATCTGGTTCAACTTGGAGCCACCCCCTAACTGCCAGCACCT
GGAAGCCCTGGACAGATACTTCAATCTCACCATGTCCTACCGCAGCGACTCCGACATCTTCACGCCCTACGGCTGGC
TGGAGCCGTGGTCCGGCCAGCCTGCCCACCCACCGCTCAACCTCTCGGCCAAGACCGAGCTGGTGGCCTGGGCGG
TGTCCAACTGGAAGCCGGACTCAGCCAGGGTGCGCTACTACCAGAGCCTGCAGGCTCATCTCAAGGTGGACGTGT
ACGGACGCTCCCACAAGCCCCTGCCCAAGGGGACCATGATGGAGACGCTGTCCCGGTACAAGTTCTACCTGGCCTT
CGAGAACTCCTTGCACCCCGACTACATCACCGAGAAGCTGTGGAGGAACGCCCTGGAGGCCTGGGCCGTGCCCGT
GGTGCTGGGCCCCAGCAGAAGCAACTACGAGAGGTTCCTGCCACCCGACGCCTTCATCCACGTGGACGACTTCCA
GAGCCCCAAGGACCTGGCCCGGTACCTGCAGGAGCTGGACAAGGACCACGCCCGCTACCTGAGCTACTTTCGCTG
GCGGGAGACGCTGCGGCCTCGCTCCTTCAGCTGGGCACTGGATTTCTGCAAGGCCTGCTGGAAACTGCAGCAGGA
ATCCAGGTACCAGACGGTGCGCAGCATAGCGGCTTGGTTCACCTGATCGACTGTGCCTTCTAGTTGCCAGCCATCT
GTTGTTTGCCCCTCCCCCGTGCCTTCCTTGACCCTGGAAGGTGCCACTCCCACTGTCCTTTCCTAATAAAATGAGGAA
ATTGCATCGCATTGTCTGAGTAGGTGTCATTCTATTCTGGGGGGTGGGGTGGGGCAGGACAGCAAGGGGGAGGA
TTGGGAGGACAATAGCAGGCATGCTGGGGATGCGGTGGGCTCTATGGCTTCGACAGGGTCGACAAGCTTTTCCAA
AAAAAAAGCATGAGGTGCAGTTGCGCCTTTCCTATCTCTTGAATAGGAAAGGCGCAACTGCACCTCATGCTGGATC
CCGCGTCCTTTCCACAAGATATATAAACCCAAGAAATCGAAATACTTTCAAGTTACGGTAAGCATATGATAGTCCAT
TTTAAAACATAATTTTAAAACTGCAAACTACCCAAGAAATTATTACTTTCTACGTCACGTATTTTGTACTAATATCTTT
GTGTTTACAGTCAAATTAATTCTAATTATCTCTCTAACAGCCTTGTATCGTATATGCAAATATGAAGGAATCATGGG
AAATAGGCCCTCTTCCTGCCCGACCTTGGCGCGCGCTCGGCGCGCGGTCACGCTCCGTCACGTGGTGCGTTTTGCC
AGCAGGCAGAAGTATGCAAAGCATGCATCTCAATTAGTCAGCAACCAGGTGTGGAAAGTCCCCAGGCTCCCCAGC
AGGCAGAAGTATGCAAAGCATGCATCTCAATTAGTCAGCAACCATAGTCCCGCCCCTAACTCCGCCCATCCCGCCCC
TAACTCCGCCCAGTTCCGCCCATTCTCCGCCCCATGGCTGACTAATTTTTTTTATTTATGCAGAGGCCGAGGCCGCCT

CTGCCTCTGAGCTATTCCAGAAGTAGTGAGGAGGCTTTTTTGGAGGCCTAGGCTTTTGCAAAAAGCTCCCGGGAGC
TTGTATATCCATTTTCGGATCTGATCAGCACGTGATGAAAAAGCCTGAACTCACCGCGACGTCTGTCGAGAAGTTTC
TGATCGAAAAGTTCGACAGCGTGTCCGACCTGATGCAGCTCTCGGAGGGCGAAGAATCTCGTGCTTTCAGCTTCGA
TGTAGGAGGGCGTGGATATGTCCTGCGGGTAAATAGCTGCGCCGATGGTTTCTACAAAGATCGTTATGTTTATCGG
CACTTTGCATCGGCCGCGCTCCCGATTCCGGAAGTGCTTGACATTGGGGAATTCAGCGAGAGCCTGACCTATTGCA
TCTCCCGCCGTGCACAGGGTGTCACGTTGCAAGACTTGCCTGAAACCGAACTGCCCGCTGTTCTGCAGCCGGTCGC
GGAGGCCATGGATGCGATCGCTGCGGCCGATCTTAGCCAGACGAGCGGGTTCGGCCCATTCGGACCGCAAGGAA
TCGGTCAATACACTACATGGCGTGATTTCATATGCGCGATTGCTGATCCCCATGTGTATCACTGGCAAACTGTGATG
GACGACACCGTCAGTGCGTCCGTCGCGCAGGCTCTCGATGAGCTGATGCTTTGGGCCGAGGACTGCCCCGAAGTC
CGGCACCTCGTGCACGCGGATTTCGGCTCCAACAATGTCCTGACGGACAATGGCCGCATAACAGCGGTCATTGACT
GGAGCGAGGCGATGTTCGGGGATTCCCAATACGAGGTCGCCAACATCTTCTTCTGGAGGCCGTGGTTGGCTTGTAT
GGAGCAGCAGACGCGCTACTTCGAGCGGAGGCATCCGGAGCTTGCAGGATCGCCGCGGCTCCGGGCGTATATGC
TCCGCATTGGTCTTGACCAACTCTATCAGAGCTTGGTTGACGGCAATTTCGATGATGCAGCTTGGGCGCAGGGTCG
ATGCGACGCAATCGTCCGATCCGGAGCCGGGACTGTCGGGCGTACACAAATCGCCCGCAGAAGCGCGGCCGTCTG
GACCGATGGCTGTGTAGAAGTACTCGCCGATAGTGGAAACCGACGCCCCAGCACTCGTCCGAGGGCAAAGGAATA
GCACGTGCTACGAGATTTCGATTCCACCGCCGCCTTCTATGAAAGGTTGGGCTTCGGAATCGTTTTCCGGGACGCC
GGCTGGATGATCCTCCAGCGCGGGGATCTCATGCTGGAGTTCTTCGCCCACCCCAACTTGTTTATTGCAGCTTATAA
TGGTTACAAATAAAGCAATAGCATCACAAATTTCACAAATAAAGCATTTTTTTCACTGCATTCTAGTTGTGGTTTGTC
CAAACTCATCAATGTATCGTAGAGATGGGCGGGAGTCTTCTGGGCAGGCTTAAAGGCTAACCTGGTGTGTGGGCG
TTGTCCTGCAGGGGAATTGAACAGGTGTAAAATTGGAGGGACAAGACTTCCCACAGATTTTCGGTTTTGTCGGGAA
GTTTTTTAATAGGGGCAAATAGGAAAATGGAGGATAGGAGTCATCTGGGGTTTATGCAGCAAAACTACAGGTATA
TTGCTTGTATCCGCCTCGGAGATTTCCATGAGGAGATAAAGACATGTCACCCGAGTTTATACTCTCCTGCTTAGATC
CTACTACAGTATGAAATACAGTGTCGCGAGGTAGACTATGTAAGCAGATTTAATCATTTTAAAGAGCCCAGTACTTC
ATATCCATTTCTCCCGCTCCTTCTGCAGCCTTATCAAAAGGTATTTAGAACACTCATTTTAGCCCCATTTTCATTTATT
ATACTGGCTTATCCAACCCCTAGACAGAGCATTGGCATTTTCCCTTTCCTGATCTTAGAAGTCTGATGACTCATGAAA
CCAGACAGATTAGTTACATACACCACAAATCGAGGCTGTAGCTGGGGCCTCAACACTGCAGTTCTTTTATAACTCCT
TAGTACACTTTTTGTTGATCCTTTGCCTTGATCCTTAATTTTCAGTGTCTATCACCTCTCCCGTCAGGTGGTGTTCCAC
ATTTGGGCCTATTCTCAGTCCAGGGAGTTTTACAACAATAGATGTATTGAGAATCCAACCTAAAGCTTAACTTTCCA
CTCCCATGAATGCCTCTCTCCTTTTTCTCCATTATAACTGAGCTATAACCATTAATGGTTTCAGGTGGATGTCTCCTCC
CCCAATATACCTGATGTATCTACATATTGCCAGGCTGATATTTTAAGACATAAAAGGTATATTTCATTATTGAGCCAC
ATGGTATTGATTACTGCTACTAAAATTTTGTCATTGTACACATCTGTAAAAGGTGGTTCCTTTTGGAATGCAAAGTTC
AGGTGTTTGTTGTCTTTCCTGACCTAAGGTCTTGTGAGCTTGTATTTTTTCTATTTAAGCAGTGCTTTCTCTTGGACTG

GCTTGACTCATGGCATTCTACACGTTATTGCTGGTCTAAATGTGATTTTGCCAAGCTTCTTCAGGACCTATAATTTTG CTTGACTTGTAGCCAAACACAAGTAAAATGATTAAGCAACAAATGTATTTGTGAAGCTTGGTTTTTAGGTTGTTGTG TTGTGTGTGCTTGTGCTCTATAATAATACTATCCAGGGGCTGGAGAGGTGGCTCGGAGTTCAAGAGCACAGACTGC TCTTCCAGAAGTCCTGAGTTCAATTCCCAGCAACCACATGGTGGCTCACAACCATCTGTAATGGGATCTGATGCCCT CTTCTGGTGTGTCTGAAGACCACAAGTGTATTCACATTAAATAAATAATCCTCCTTCTTCTTCTTTTTTTTTTTTTAAA GAGAATACTGTCTCCAGTAGAATTACTGAAGTAATGAAATACTTTGTGTTTGTTCCAATATGGAAGCCAATAATCAA ATACTCTTAAGCACTGGAAATGTACCAAGGAACTATTTTATTTAAGTGAACTGTGGACAGAGGAGCCATAACTGCA GACTTGTGGGATACAGAAGACCAATGCAGACTTAATGTCTTTTCTCTTACACTAAGCAATAAAGAAATAAAAAATTG AACTTCTAGTATCCTATTTGTTAAACTGCTAGCTTTACTAACTTTTGTGCTTCATCTATACAAAGCTGAAAGCTAAGT CTGCAGCCATTACTAAACATGAAAGCAAGTAATGATAATTTTGGATTTCAAAAATGTAGGGCCAGAGTTTAGCCAG CCAGTGGTGGTGCTTGCCTTTATGCCTTAATCCCAGCACTCTGGAGGCAGAGACAGGCAGATCTCTGAGTTTGAGC CCAGCCTGGTCTACACATCAAGTTCTATCTAGGATAGCCAGGAATACACACAGAAACCCTGTTGGGGAGGGGGGC TCTGAGATTTCATAAAATTATAATTGAAGCATTCCCTAATGAGCCACTATGGATGTGGCTAAATCCGTCTACCTTTCT GATGAGATTTGGGTATTATTTTTTCTGTCTCTGCTGTTGGTTGGGTCTTTTGACACTGTGGGCTTTCTTAAAGCCTCC TTCCCTGCCATGTGGACTCTTGTTTGCTACTAACTTCCCATGGCTTAAATGGCATGGCTTTTTGCCTTCTAAGGGCAG CTGCTGAGATTTGCAGCCTGATTTCCAGGGTGGGGTTGGGAAATCTTTCAAACACTAAAATTGTCCTTTAATTTTTTT TTAAAAAAATGGGTTATATAATAAACCTCATAAAATAGTTATGAGGAGTGAGGTGGACTAATATTAATGAGTCCCTC CCCTATAAAAGAGCTATTAAGGCTTTTTGTCTTATACTAACTTTTTTTTTAAATGTGGTATCTTTAGAACCAAGGGTC TTAGAGTTTTAGTATACAGAAACTGTTGCATCGCTTAATCAGATTTTCTAGTTTCAAATCCAGAGAATCCAAATTCTT CACAGCCAAAGTCAAATTAAGAATTTCTGACTTTAATGTTATTTGCTACTGTGAATATAAAATGATAGCTTTTCCTGA GGCAGGGTATCACTATGTATCTCTGCCTGATCTGCAACAAGATATGTAGACTAAAGTTCTGCCTGCTTTTGTCTCCT GAATACTAAGGTTAAAATGTAGTAATACTTTTGGAACTTGCAGGTCAGATTCTTTTATAGGGGACACACTAAGGGA GCTTGGGTGATAGTTGGTAAATGTGTTTAAGTGATGAAAACTTGAATTATTATCACCGCAACCTACTTTTTAAAAAA AAAAGCCAGGCCTGTTAGAGCATGCTAAGGGATCCCTAGGACTTGCTGAGCACACAAGAGTAGTACTTGGCAGGC TCCTGGTGAGAGCATATTTCAAAAAACAAGGCAGACAACCAAGAAACTACAGTAAGGTTACCTGTCTTTAACCATC TGCATATACACAGGGATATTAAAATATTCCAAATAATATTTCATTCAAGTTTTCCCCCATCAAATTGGGACATGGATT TCTCCGGTGAATAGGCAGAGTTGGAAACTAAACAAATGTTGGTTTTGTGATTTGTGAAATTGTTTTCAAGTGATAGT TAAAGCCCATGAGATACAGAACAAAGCTGCTATTTCGAGGTCACTTGGTTATACTCAGAAGCACTTCTTTGGGTTTC CCTGCACTATCCTGATCATGTGCTAGGCCTACCTTAGGCTGATTGTTGTTCAAATAACTTAAGTTTCCTGTCAGGTGA TGTCATATGATTTCATATATCAAGGCAAAACATGTTATATATGTTAAACATTTGGACTTAATGTGAAAGTTAGGTCTT TGTGGGTTTTGATTTTAATTTCAAAACCTGAGCTAAATAAGTCATTTTACATGTCTTACATTTGGTGAATTGTATATT GTGGTTTGCAGGCAAGACTCTCTGACCTAGTAACCCTCCTATAGAGCACTTTGCTGGGTCACAAGTCTAGGAGTCA

AGCATTTCACCTTGAAGTTGAGACGTTTTGTTAGTGTATACTAGTTATATGTTGGAGGACATGTTTATCCAGAAGAT
ATTCAGGACTATTTTTGACTGGGCTAAGGAATTGATTCTGATTAGCACTGTTAGTGAGCATTGAGTGGCCTTTAGGC
TTGAATTGGAGTCACTTGTATATCTCAAATAATGCTGGCCTTTTTTAAAAAGCCCTTGTTCTTTATCACCCTGTTTTCT
ACATAATTTTTGTTCAAAGAAATACTTGTTTGGATCTCCTTTTGACAACAATAGCATGTTTTCAAGCCATATTTTTTTT
CCTTTTTTTTTTTTTTTTTTGGTTTTTCGAGACAGGGTTTCTCTGTATAGCCCTGGCTGTCCTGGAACTCACTTTGTAGA
CCAGGCTGGCCTCGAACTCAGAAATCCGCCTGCCTCTGCCTCCTGAGTGCCGGGATTAAAGGCGTGCACCACCACG
CCTGGCTAAGTTGGATATTTTGTATATAACTATAACCAATACTAACTCCACTGGGTGGATTTTTAATTCAGTCAGTAG
TCTTAAGTGGTCTTTATTGGCCCTTATTAAAATCTACTGTTCACTCTAACAGAGGCTGTTGGACTAGTGGGACTAAG
CAACTTCCTACGGATATACTAGCAGATAAGGGTCAGGGATAGAAACTAGTCTAGCGTTTTGTATACCTACCAGCTTA
TACTACCTTGTTCTGATAGAAATATTTAGGACATCTAGCTTATCATGCAAGGAACCAATTCAGTCGACTGGATCCCG
ATGGCTCCGGTGCCCGTCAGTGGGCAGAGCGCACATCGCCCACAGTCCCCGAGAAGTTGGGGGGAGGGGTCGGC
AATTGAACCGGTGCCTAGAGAAGGTGGCGCGGGGTAAACTGGGAAAGTGATGTCGTGTACTGGCTCCGCCTTTTT
CCCGAGGGTGGGGGAGAACCGTATATAAGTGCAGTAGTCGCCGTGAACGTTCTTTTTCGCAACGGGTTTGCCGCC
AGAACACAGGTCCGCGGCCCCGAACTAGGCCTAGGCGTCTGATCACTAGTGACTCTAGTCCTAGTCGACTAGGGAT
AACAGGGGCCCATGGCTTCGTACCCCTGCCATCAACACGCGTCTGCGTTCGACCAGGCTGCGCGTTCTCGCGGCCA
TAGCAACCGACGTACGGCGTTGCGCCCTCGCCGGCAGCAAGAAGCCACGGAAGTCCGCCTGGAGCAGAAAATGCC
CACGCTACTGCGGGTTTATATAGACGGTCCTCACGGGATGGGGAAAACCACCACCACGCAACTGCTGGTGGCCCT
GGGTTCGCGCGACGATATCGTCTACGTACCCGAGCCGATGACTTACTGGCAGGTGCTGGGGGCTTCCGAGACAAT
CGCGAACATCTACACCACACAACACCGCCTCGACCAGGGTGAGATATCGGCCGGGGACGCGGCGGTGGTAATGAC
AAGCGCCCAGATAACAATGGGCATGCCTTATGCCGTGACCGACGCCGTTCTGGCTCCTCATATCGGGGGGGAGGC
TGGGAGCTCACATGCCCCGCCCCCGGCCCTCACCCTCATCTTCGACCGCCATCCCATCGCCGCCCTCCTGTGCTACCC
GGCCGCGCGATACCTTATGGGCAGCATGACCCCCCAGGCCGTGCTGGCGTTCGTGGCCCTCATCCCGCCGACCTTG
CCCGGCACAAACATCGTGTTGGGGGCCCTTCCGGAGGACAGACACATCGACCGCCTGGCCAAACGCCAGCGCCCC
GGCGAGCGGCTTGACCTGGCTATGCTGGCCGCGATTCGCCGCGTTTACGGGCTGCTTGCCAATACGGTGCGGTATC
TGCAGGGCGGCGGGTCGTGGCGGGAGGATTGGGGACAGCTTTCGGGGACGGCCGTGCCGCCCCAGGGTGCCGA
GCCCCAGAGCAACGCGGGCCCACGACCCCATATCGGGGACACGTTATTTACCCTGTTTCGGGCCCCCGAGTTGCTG
GCCCCCAACGGCGACCTGTACAACGTGTTTGCCTGGGCCTTGGACGTCTTGGCCAAACGCCTCCGTCCCATGCACG
TCTTTATCCTGGATTACGACCAATCGCCCGCCGGCTGCCGGGACGCCCTGCTGCAACTTACCTCCGGGATGGTCCA
GACCCACGTCACCACCCCCGGCTCCATACCGACGATCTGCGACCTGGCGCGCACGTTTGCCCGGGAGATGGGGGA
GGCTAACTGACCGCGGGGGAGGCTAACTGAAACACGGAAGGAGACAATACCGGAAGGAACCCGCGCTATGACGG
CAATAAAAAGACAGAATAAAACGCACGGTGTTGGGTCGTTTGTTCATAAACGCGGGGTTCGGTCCCAGGGCTGGC

ACTCTGTCGATACCCCACCGAGGCCCCATTGGGGCCAATACGCCCGCGTTTCTTCCTTTTCCCCACCCCACCCCCCAA
GTTCGGGTGAAGGCCCAGGGCTCGCAGCCAACGTCGGGGCGGCAGGCCCTGCCATAGCC

L’ analyse de restriction par triple digestion EcoRV, Pvul, Sall de la réalisation 5 est illustrée figure 14.

Vérification fonctionnelle de la réalisation 5 : des cellules eucaryotes (par exemple des cellules cancéreuses mammaires de souris 4T1) sont transfectées avec le vecteur V1.3 selon un protocole usuel (par exemple par lipotransfection ou électroporation). Après transfection, les cellules sont traitées avec de l'hygromycine (50µg-mL-1 pendant 7-14 jours), puis avec du Ganciclovir (3-6 jours 5-40µM). Les cellules sont ensuite collectées et lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel.

Conditions spécifiques de la PCR quantitative:

40 cycles des trois étapes suivantes sont réalisés: dénaturation 94°C-30s ; hybridation 60°C-30s ; extension 72°C-30s.

Amorces utilisés:

**mB3Galt6**

BETA3Galt6ms2-s ACCACTCTGTTGTACCTGGC (SEQ ID NO : 42).
BETA3Galt6ms2-as CACACGTCCTCGGGTCC (SEQ ID NO : 43).

**hFUT3**

FUT35 CACTAGTCGACTAGGGATAACAGG (SEQ ID NO : 44).
FUT33 ATGTCCATAGCAGGATCAGGAG (SEQ ID NO : 45).

**Réalisation 6**

**Groupe de vecteurs V2 : Vecteur permettant d'exprimer un ou plusieurs transgènes de façon inductible.**

$$U1 + U2c + nxU2d + mxU2e$$

U1 : Unité fonctionnelle bactérienne
U2c = gène codant un transactivateur transcriptionelle (exemple protéine TAT).
U2d = gène(s) dont le promoteur est dépendant du transactivateur codé par le gène U2c, n ≥1
U2e = gène(s) dont le promoteur n'est pas dépendant du transactivateur codé par le gène U2c, m ≥0

Exemple : vecteur permettant l'expression inductible de l'enzyme mB3Galt6

| | |
|---|---|
| U1 | ori-Amp |
| U2c | promoteur CMV<br>Teton3G<br>Terminateur BPA |
| U2d | promoteur TRE3G<br>mB3Galt6<br>HSV Tk terminator |

Liste des briques utilisées pour la construction du vecteur V2 (Figure 15)

**Brique Ori-AmpR BsaI B (SEQ ID NO : 36)**
**Brique pCMV BsaI B (SEQ ID NO : 37)**

**Brique TO3G BsaI A (SEQ ID NO : 59)**

GAGGTACCGGTCTCACACCATGTCTAGACTGGACAAGAGCAAAGTCATAAACTCTGCTCTGGAATTACTCAATGGA
GTCGGTATCGAAGGCCTGACGACAAGGAAACTCGCTCAAAAGCTGGGAGTTGAGCAGCCTACCCTGTACTGGCAC
GTGAAGAACAAGCGGGCCCTGCTCGATGCCCTGCCAATCGAGATGCTGGACAGGCATCATACCCACTCCTGCCCCC
TGGAAGGCGAGTCATGGCAAGACTTTCTGCGGAACAACGCCAAGTCATACCGCTGTGCTCTCCTCTCACATCGCGA
CGGGGCTAAAGTGCATCTCGGCACCCGCCCAACAGAGAAACAGTACGAAACCCTGGAAAATCAGCTCGCGTTCCT
GTGTCAGCAAGGCTTCTCCCTGGAGAACGCACTGTACGCTCTGTCCGCCGTGGGCCACTTTACACTGGGCTGCGTA
TTGGAGGAACAGGAGCATCAAGTAGCAAAAGAGGAAAGAGAGACACCTACCACCGATTCTATGCCCCCACTTCTG
AAACAAGCAATTGAGCTGTTCGACCGGCAGGGAGCCGAACCTGCCTTCCTTTTCGGCCTGGAACTAATCATATGTG
GCCTGGAGAAACAGCTAAAGTGCGAAAGCGGCGGGCCGACCGACGCCCTTGACGATTTTGACTTAGACATGCTCC
CAGCCGATGCCCTTGACGACTTTGACCTTGATATGCTGCCTGCTGACGCTCTTGACGATTTTGACCTTGACATGCTCC
CCGGGTAATGATCGAGACCGGTACCTC

**Brique BGHpA BsaI B (SEQ ID NO : 39)**

**Brique pTRE3G BsaI A (SEQ ID NO : 60)**

GAGGTACCGGTCTCATTCGCTTTCGTCTTCAAGAATTCCTGGAGTTTACTCCCTATCAGTGATAGAGAACGTATGAA
GAGTTTACTCCCTATCAGTGATAGAGAACGTATGCAGACTTTACTCCCTATCAGTGATAGAGAACGTATAAGGAGT
TTACTCCCTATCAGTGATAGAGAACGTATGACCAGTTTACTCCCTATCAGTGATAGAGAACGTATCTACAGTTTACT
CCCTATCAGTGATAGAGAACGTATATCCAGTTTACTCCCTATCAGTGATAGAGAACGTATAAGCTTTAGGCGTGTAC
GGTGGGCGCCTATAAAAGCAGAGCTCGTTTAGTGAACCGTCAGATCGCCTGGAGCAATTCCACAACACTTTTGTCT
TATACCAACTTTCCGTACCACTTCCTACCCTCGTAAACCAGCGAGACCGGTACCTC

**Brique mB3Galt6 BsaI A (SEQ ID NO : 61)**

CCAGATGAAGGTATTCCGGCGCGCTTGGCGGCACCGGGTGGCGCTGGGCCTAGGCGGCCTGGCGTTCTGCGGCA
CCACTCTGTTGTACCTGGCGCGCTGCGCTTCCGAGGGCGAGACGCCCTCCGCTTCCGGAGCCGCTCGGCCCCGCGC
TAAGGCCTTCCTGGCGGTGCTGGTGGCCAGTGCGCCCCGCGCGGTCGAGCGCCGCACCGCAGTGCGCAGCACGTG
GCTGGCACCGGAGAGGCGTGGCGGACCCGAGGACGTGTGGGCGCGCTTCGCCGTGGGCACTGGCGGCTTAGGCT
CGGAGGAGCGGCGCGCTCTTGAGCTCGAGCAGGCGCAGCACGGGGACCTGCTGCTGCTGCCCGCCCTGCGCGAC
GCCTACGAGAACCTCACGGCCAAGGTCCTGGCCATGCTGACCTGGCTGGATGAGCGCGTGGACTTCGAGTTCGTG
CTCAAGGCGGACGACGACTCCTTTGCGCGCCTGGACGCTATCCTGGTGGACCTACGCGCACGGGAGCCCGCACGC
CGCCGGCGCCTCTACTGGGGCTTCTTTTCCGGGCGCGGGCGCGTCAAGCCGGGAGGTCGCTGGCGAGAAGCAGCC
TGGCAACTCTGCGACTACTACCTGCCCTACGCGTTGGGCGGTGGCTATGTCCTTTCTGCGGACCTGGTGCATTACCT
GCGCCTCAGCCGCGAGTACCTGCGCGCGTGGCACAGTGAAGACGTATCGCTGGGCACCTGGCTGGCACCAGTGGA
TGTGCAACGGGAGCACGACCCACGCTTCGACACGGAGTACAAATCTCGAGGCTGCAACAATCAGTATCTGGTGAC
ACACAAGCAAAGCCCAGAGGACATGTTGGAGAAGCAACAGATGTTGCTGCATGAGGGCCGGTTGTGCAAGCATG
AGGTGCAGTTGCGCCTTTCCTATGTCTATGACTGGTCAGCTCCACCCTCCCAGTGCTGCCAGCGCAAGGAGGGCGT
TCCCTGACCGC

**Brique Tkter BsaI A (SEQ ID NO : 57)**

**V2 (SEQ ID NO : 62, exemple de vecteur du groupe V2)**

TATTGTAATACGGTTATCCACAGAATCAGGGGATAACGCAGGAAAGAACATGTGAGCAAAAGGCCAGCAAAAGG
CCAGGAACCGTAAAAAGGCCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGAAGCGTGGCGCTTTCTCA
TAGCTCACGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCCACTGGC

AGCAGCCACTGGTAACAGGATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTTTTTTTGTTTGCAAGCAGCAGATTACGCGCAGAAAAA
AAGGATCTCAAGAAGATCCTTTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGAAGTTTTAAATCAATCTAAAG
TATATATGAGTAAACTTGGTCTGACAGTTACCAATGCTTAATCAGTGAGGCACCTATCTCAGCGATCTGTCTATTTC
GTTCATCCATAGTTGCCTGACTCCCCGTCGTGTAGATAACTACGATACGGGAGGGCTTACCATCTGGCCCCAGTGCT
GCAATGATACCGCGTGACCCACGCTCACCGGCTCCAGATTTATCAGCAATAAACCAGCCAGCCGGAAGGGCCGAG
CGCAGAAGTGGTCCTGCAACTTTATCCGCCTCCATCCAGTCTATTAATTGTTGCCGGGAAGCTAGAGTAAGTAGTTC
GCCAGTTAATAGTTTGCGCAACGTTGTTGCCATTGCTACAGGCATCGTGGTGTCACGCTCGTCGTTTGGTATGGCTT
CATTCAGCTCCGGTTCCCAACGATCAAGGCGAGTTACATGATCCCCCATGTTGTGCAAAAAAGCGGTTAGCTCCTTC
GGTCCTCCGATCGTTGTCAGAAGTAAGTTGGCCGCAGTGTTATCACTCATGGTTATGGCAGCACTGCATAATTCTCT
TACTGTCATGCCATCCGTAAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCAAGGAACCAATTCAGTCGACTGGATCCTAGTTATTAATAGTAATCAATTACGGGGTCAT
TAGTTCATAGCCCATATATGGAGTTCCGCGTTACATAACTTACGGTAAATGGCCCGCCTGGCTGACCGCCCAACGA
CCCCCGCCCATTGACGTCAATAATGACGTATGTTCCCATAGTAACGCCAATAGGGACTTTCCATTGACGTCAATGGG
TGGAGTATTTACGGTAAACTGCCCACTTGGCAGTACATCAAGTGTATCATATGCCAAGTACGCCCCCTATTGACGTC
AATGACGGTAAATGGCCCGCCTGGCATTATGCCCAGTACATGACCTTATGGGACTTTCCTACTTGGCAGTACATCTA
CGTATTAGTCATCGCTATTACCATGGTGATGCGGTTTTGGCAGTACATCAATGGGCGTGGATAGCGGTTTGACTCA
CGGGGATTTCCAAGTCTCCACCCCATTGACGTCAATGGGAGTTTGTTTTGGCACCAAAATCAACGGGACTTTCCAAA
ATGTCGTAACAACTCCGCCCCATTGACGCAAATGGGCGGTAGGCGTGTACGGTGGGAGGTCTATATAAGCAGAGC
TGGTTTAGTGAACCGTCAGATCACTAGTCGACTAGGGATAACAGGGCACCATGTCTAGACTGGACAAGAGCAAAG
TCATAAACTCTGCTCTGGAATTACTCAATGGAGTCGGTATCGAAGGCCTGACGACAAGGAAACTCGCTCAAAAGCT
GGGAGTTGAGCAGCCTACCCTGTACTGGCACGTGAAGAACAAGCGGGCCCTGCTCGATGCCCTGCCAATCGAGAT
GCTGGACAGGCATCATACCCACTCCTGCCCCCTGGAAGGCGAGTCATGGCAAGACTTTCTGCGGAACAACGCCAA
GTCATACCGCTGTGCTCTCCTCTCACATCGCGACGGGGCTAAAGTGCATCTCGGCACCCGCCCAACAGAGAAACAG
TACGAAACCCTGGAAAATCAGCTCGCGTTCCTGTGTCAGCAAGGCTTCTCCCTGGAGAACGCACTGTACGCTCTGT

CCGCCGTGGGCCACTTTACACTGGGCTGCGTATTGGAGGAACAGGAGCATCAAGTAGCAAAAGAGGAAAGAGAG
ACACCTACCACCGATTCTATGCCCCCACTTCTGAAACAAGCAATTGAGCTGTTCGACCGGCAGGGAGCCGAACCTG
CCTTCCTTTTCGGCCTGGAACTAATCATATGTGGCCTGGAGAAACAGCTAAAGTGCGAAAGCGGCGGGCCGACCG
ACGCCCTTGACGATTTTGACTTAGACATGCTCCCAGCCGATGCCCTTGACGACTTTGACCTTGATATGCTGCCTGCT
GACGCTCTTGACGATTTTGACCTTGACATGCTCCCCGGGTAATGATCGACTGTGCCTTCTAGTTGCCAGCCATCTGT
TGTTTGCCCCTCCCCCGTGCCTTCCTTGACCCTGGAAGGTGCCACTCCCACTGTCCTTTCCTAATAAAATGAGGAAAT
TGCATCGCATTGTCTGAGTAGGTGTCATTCTATTCTGGGGGGTGGGGTGGGGCAGGACAGCAAGGGGGAGGATT
GGGAGGACAATAGCAGGCATGCTGGGGATGCGGTGGGCTCTATGGCTTCGCTTTCGTCTTCAAGAATTCCTGGAG
TTTACTCCCTATCAGTGATAGAGAACGTATGAAGAGTTTACTCCCTATCAGTGATAGAGAACGTATGCAGACTTTAC
TCCCTATCAGTGATAGAGAACGTATAAGGAGTTTACTCCCTATCAGTGATAGAGAACGTATGACCAGTTTACTCCCT
ATCAGTGATAGAGAACGTATCTACAGTTTACTCCCTATCAGTGATAGAGAACGTATATCCAGTTTACTCCCTATCAG
TGATAGAGAACGTATAAGCTTTAGGCGTGTACGGTGGGCGCCTATAAAAGCAGAGCTCGTTTAGTGAACCGTCAG
ATCGCCTGGAGCAATTCCACAACACTTTTGTCTTATACCAACTTTCCGTACCACTTCCTACCCTCGTAAACCAGAGCA
TGAAGGTATTCCGGCGCGCTTGGCGGCACCGGGTGGCGCTGGGCCTAGGCGGCCTGGCGTTCTGCGGCACCACTC
TGTTGTACCTGGCGCGCTGCGCTTCCGAGGGCGAGACGCCCTCCGCTTCCGGAGCCGCTCGGCCCCGCGCTAAGG
CCTTCCTGGCGGTGCTGGTGGCCAGTGCGCCCCGCGCGGTCGAGCGCCGCACCGCAGTGCGCAGCACGTGGCTGG
CACCGGAGAGGCGTGGCGGACCCGAGGACGTGTGGGCGCGCTTCGCCGTGGGCACTGGCGGCTTAGGCTCGGAG
GAGCGGCGCGCTCTTGAGCTCGAGCAGGCGCAGCACGGGGACCTGCTGCTGCCCGCCCTGCGCGACGCCTAC
GAGAACCTCACGGCCAAGGTCCTGGCCATGCTGACCTGGCTGGATGAGCGCGTGGACTTCGAGTTCGTGCTCAAG
GCGGACGACGACTCCTTTGCGCGCCTGGACGCTATCCTGGTGGACCTACGCGCACGGGAGCCCGCACGCCGCCGG
CGCCTCTACTGGGGCTTCTTTTCCGGGCGCGGGCGCGTCAAGCCGGGAGGTCGCTGGCGAGAAGCAGCCTGGCAA
CTCTGCGACTACTACCTGCCCTACGCGTTGGGCGGTGGCTATGTCCTTTCTGCGGACCTGGTGCATTACCTGCGCCT
CAGCCGCGAGTACCTGCGCGCGTGGCACAGTGAAGACGTATCGCTGGGCACCTGGCTGGCACCAGTGGATGTGCA
ACGGGAGCACGACCCACGCTTCGACACGGAGTACAAATCTCGAGGCTGCAACAATCAGTATCTGGTGACACACAA
GCAAAGCCCAGAGGACATGTTGGAGAAGCAACAGATGTTGCTGCATGAGGGCCGGTTGTGCAAGCATGAGGTGC
AACTTCGCCTTTCCTATGTCTATGACTGGTCAGCTCCACCCTCCCAGTGCTGCCAGCGCAAGGAGGGCGTTCCCTGA
**TGTCA**CCGCGGGGGAGGCTAACTGAAACACGGAAGGAGACAATACCGGAAGGAACCCGCGCTATGACGGCAATA
AAAAGACAGAATAAAACGCACGGTGTTGGGTCGTTTGTTCATAAACGCGGGGTTCGGTCCCAGGGCTGGCACTCT
GTCGATACCCCACCGAGGCCCCATTGGGGCCAATACGCCCGCGTTTCTTCCTTTTCCCCACCCCACCCCCCAAGTTC
GGGTGAAGGCCCAGGGCTCGCAGCCAACGTCGGGGCGGCAGGCCCTGCCATAGCC

L'analyse de restriction par triple digestion NdeI, SalI, XhoI de la réalisation 6 est illustrée figure 16.

Vérification fonctionnelle de la realisation 6 : des cellules eucaryotes (ex : cellules cancéreuses mammaires de souris 4T1) sont transfectées avec le vecteur V2 selon un protocole usuel (exemple lipotransfection ou électroporation). Après transfection les cellules sont traitées avec de 100-1000 ng.mL$^{-1}$ de Doxycycline pendant 24 à 48 heures. Les cellules sont ensuite collectées et lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel.

Conditions spécifiques de la PCR quantitative:

40 cycles des trois étapes suivantes sont réalisés: dénaturation 94°C-30s ; hybridation 60°C-30s ; extension 72°C-30s.

Amorces utilisées:

BETA3Galt6ms2-s ACCACTCTGTTGTACCTGGC (SEQ ID NO : 42).
BETA3Galt6ms2-as CACACGTCCTCGGGTCC (SEQ ID NO : 43).

**Réalisation 7**

**Groupe de vecteurs V3 : Vecteur permettant de réaliser de la complémentation génétique sous contrôle inductible.**

U1 + U2f + U2c +U2g

U1 : Unité fonctionnelle bactérienne
U2f = gène dont le promoteur est un promoteur à ARN polymérase III et dont le produit d'expression est un petit ARN en épingle à cheveux (shRNA) précurseur d'un petit ARN interférant ciblant un gène X.
U2c = gène codant un transactivateur transcriptionel (exemple : protéine TAT du VIH-1 ou-2).
U2g = gène dont le promoteur est dépendant du transactivateur codé par le gène U2c et dont le produit d'expression est une version mutée du produit du gène X de sorte à être insensible au produit du gène U2f

Exemple : Vecteur permettant de réprimer l'expression de l'enzyme mB3Galt6 tout en surexprimant de façon inductible l'expression de cette enzyme (complémentation possible).

| | |
|---|---|
| U1 | ori-Amp |
| U2c | promoteur CMV<br>Teton3G<br>Terminateur BPA |
| U2g | promoteur TRE3G<br>mB3Galt6 - muté<br>HSV Tk terminator |
| U2f | cassette shRNA mB3GALT6 |

Liste des briques utilisées pour la construction du vecteur V3 (figure 17)

**Brique Ori-AmpR BsaI B (SEQ ID NO : 36)**
**Brique pCMV BsaI B (SEQ ID NO : 37)**
**Brique TO3G BsaI A (SEQ ID NO : 59)**
**Brique BGHpA BsaI B (SEQ ID NO : 39)**
**Brique pTRE3G BsaI A (SEQ ID NO : 60)**

**Brique mB3Galt6 BsaI B (SEQ ID NO : 63)**

GAGGTACCGGTCTCACCAGAGCATGAAGGTATTCCGGCGCGCTTGGCGGCACCGGGTGGCGCTGGGCCTAGGCG
GCCTGGCGTTCTGCGGCACCACTCTGTTGTACCTGGCGCGCTGCGCTTCCGAGGGCGAGACGCCCTCCGCTTCCGG
AGCCGCTCGGCCCCGCGCTAAGGCCTTCCTGGCGGTGCTGGTGGCCAGTGCGCCCCGCGCGGTCGAGCGCCGCAC
CGCAGTGCGCAGCACGTGGCTGGCACCGGAGAGGCGTGGCGGACCCGAGGACGTGTGGGCGCGCTTCGCCGTG
GGCACTGGCGGCTTAGGCTCGGAGGAGCGGCGCGCTCTTGAGCTCGAGCAGGCGCAGCACGGGGACCTGCTGCT
GCTGCCCGCCCTGCGCGACGCCTACGAGAACCTCACGGCCAAGGTCCTGGCCATGCTGACCTGGCTGGATGAGCG
CGTGGACTTCGAGTTCGTGCTCAAGGCGGACGACGACTCCTTTGCGCGCCTGGACGCTATCCTGGTGGACCTACGC
GCACGGGAGCCCGCACGCCGCCGGCGCCTCTACTGGGGCTTCTTTTCCGGGCGCGGGCGCGTCAAGCCGGGAGGT
CGCTGGCGAGAAGCAGCCTGGCAACTCTGCGACTACTACCTGCCCTACGCGTTGGGCGGTGGCTATGTCCTTTCTG
CGGACCTGGTGCATTACCTGCGCCTCAGCCGCGAGTACCTGCGCGCGTGGCACAGTGAAGACGTATCGCTGGGCA
CCTGGCTGGCACCAGTGGATGTGCAACGGGAGCACGACCCACGCTTCGACACGGAGTACAAATCTCGAGGCTGCA
ACAATCAGTATCTGGTGACACACAAGCAAAGCCCAGAGGACATGTTGGAGAAGCAACAGATGTTGCTGCATGAGG
GCCGGTTGTGCAAGCATGAGGTGCAACTTCGCCTTTCCTATGTCTATGACTGGTCAGCTCCACCCTCCCAGTGCTGC
CAGCGCAAGGAGGGCGTTCCCTGATGTCACCGCCGAGACCGGTACCTC

**Brique Tkter BsaI B (SEQ ID NO : 64)**

GAGGTACCGGTCTCACCGCGGGGGAGGCTAACTGAAACACGGAAGGAGACAATACCGGAAGGAACCCGCGCTAT
GACGGCAATAAAAAGACAGAATAAAACGCACGGTGTTGGGTCGTTTGTTCATAAACGCGGGGTTCGGTCCCAGGG
CTGGCACTCTGTCGATACCCCACCGAGGCCCCATTGGGGCCAATACGCCCGCGTTTCTTCCTTTTCCCCACCCCACCC
CCCAAGTTCGGGTGAAGGCCCAGGGCTCGCAGCCAACGTCGGGGCGGCAGGCCCTGCCATAGCCACAACGAGAC
CGGTACCTC

**Brique shB3Galt6 BsaI C (SEQ ID NO : 65)**

GAGGTACCGGTCTCAACAAACAGGGTCGACAAGCTTTTCCAAAAAAAAAGCATGAGGTGCAGTTGCGCCTTTCCTA
TCTCTTGAATAGGAAAGGCGCAACTGCACCTCATGCTGGATCCCGCGTCCTTTCCACAAGATATATAAACCCAAGAA
ATCGAAATACTTTCAAGTTACGGTAAGCATATGATAGTCCATTTTAAAACATAATTTTAAAACTGCAAACTACCCAA
GAAATTATTACTTTCTACGTCACGTATTTTGTACTAATATCTTTGTGTTTACAGTCAAATTAATTCTAATTATCTCTCTA
ACAGCCTTGTATCGTATATGCAAATATGAAGGAATCATGGGAAATAGGCCCTCTTCCTGCCCGACCTTGGCGCGCG
CTCGGCGCGCGGTCACGCTCCGTCACGTGGTGCGTTTTGTATTCGAGACCGGTACCTC

**V3 (SEQ ID NO : 66, exemple de vecteur du groupe V3)**

TATTGTAATACGGTTATCCACAGAATCAGGGGATAACGCAGGAAAGAACATGTGAGCAAAAGGCCAGCAAAAGGC
CAGGAACCGTAAAAAGGCCGCGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCCCCTGGAAGCTCCCTCGTGCG
CTCTCCTGTTCCGACCCTGCCGCTTACCGGATACCTGTCCGCCTTTCTCCCTTCGGGAAGCGTGGCGCTTTCTCATAGC
TCACGCTGTAGGTATCTCAGTTCGGTGTAGGTCGTTCGCTCCAAGCTGGGCTGTGTGCACGAACCCCCCGTTCAGCC
CGACCGCTGCGCCTTATCCGGTAACTATCGTCTTGAGTCCAACCCGGTAAGACACGACTTATCGCCACTGGCAGCAG
CCACTGGTAACAGGATTAGCAGAGCGAGGTATGTAGGCGGTGCTACAGAGTTCTTGAAGTGGTGGCCTAACTACGG
CTACACTAGAAGAACAGTATTTGGTATCTGCGCTCTGCTGAAGCCAGTTACCTTCGGAAAAAGAGTTGGTAGCTCTT
GATCCGGCAAACAAACCACCGCTGGTAGCGGTTTTTTTGTTTGCAAGCAGCAGATTACGCGCAGAAAAAAAGGATC
TCAAGAAGATCCTTTGATCTTTTCTACGGGGTCTGACGCTCAGTGGAACGAAAACTCACGTTAAGGGATTTTGGTCA
TGAGATTATCAAAAAGGATCTTCACCTAGATCCTTTTAAATTAAAAATGAAGTTTTAAATCAATCTAAAGTATATATG
AGTAAACTTGGTCTGACAGTTACCAATGCTTAATCAGTGAGGCACCTATCTCAGCGATCTGTCTATTTCGTTCATCCA
TAGTTGCCTGACTCCCCGTCGTGTAGATAACTACGATACGGGAGGGCTTACCATCTGGCCCCAGTGCTGCAATGATA
CCGCGTGACCCACGCTCACCGGCTCCAGATTTATCAGCAATAAACCAGCCAGCCGGAAGGGCCGAGCGCAGAAGTG
GTCCTGCAACTTTATCCGCCTCCATCCAGTCTATTAATTGTTGCCGGGAAGCTAGAGTAAGTAGTTCGCCAGTTAATA

GTTTGCGCAACGTTGTTGCCATTGCTACAGGCATCGTGGTGTCACGCTCGTCGTTTGGTATGGCTTCATTCAGCTCCG
GTTCCCAACGATCAAGGCGAGTTACATGATCCCCCATGTTGTGCAAAAAAGCGGTTAGCTCCTTCGGTCCTCCGATC
GTTGTCAGAAGTAAGTTGGCCGCAGTGTTATCACTCATGGTTATGGCAGCACTGCATAATTCTCTTACTGTCATGCCA
TCCGTAAGATGCTTTTCTGTGACTGGTGAGTACTCAACCAAGTCATTCTGAGAATAGTGTATGCGGCGACCGAGTTG
CTCTTGCCCGGCGTCAATACGGGATAATACCGCGCCACATAGCAGAACTTTAAAAGTGCTCATCATTGGAAAACGTT
CTTCGGGGCGAAAACTCTCAAGGATCTTACCGCTGTTGAGATCCAGTTCGATGTAACCCACTCGTGCACCCAACTGA
TCTTCAGCATCTTTTACTTTCACCAGCGTTTCTGGGTGAGCAAAAACAGGAAGGCAAAATGCCGCAAAAAAGGGAAT
AAGGGCGACACGGAAATGTTGAATACTCATACTCTTCCTTTTTCAATATTATTGAAGCATTTATCAGGGTTATTGTCTC
ATGAGCGGATACATATTTGAATGTATTTAGAAAAATAAACAAATAGGGGTTCCGCGCACATTTCCCCGAAAAGTGCC
AAGGAACCAATTCAGTCGACTGGATCCTAGTTATTAATAGTAATCAATTACGGGGTCATTAGTTCATAGCCCATATAT
GGAGTTCCGCGTTACATAACTTACGGTAAATGGCCCGCCTGGCTGACCGCCCAACGACCCCCGCCCATTGACGTCAA
TAATGACGTATGTTCCCATAGTAACGCCAATAGGGACTTTCCATTGACGTCAATGGGTGGAGTATTTACGGTAAACT
GCCCACTTGGCAGTACATCAAGTGTATCATATGCCAAGTACGCCCCCTATTGACGTCAATGACGGTAAATGGCCCGC
CTGGCATTATGCCCAGTACATGACCTTATGGGACTTTCCTACTTGGCAGTACATCTACGTATTAGTCATCGCTATTACC
ATGGTGATGCGGTTTTGGCAGTACATCAATGGGCGTGGATAGCGGTTTGACTCACGGGGATTTCCAAGTCTCCACCC
CATTGACGTCAATGGGAGTTTGTTTTGGCACCAAAATCAACGGGACTTTCCAAAATGTCGTAACAACTCCGCCCCATT
GACGCAAATGGGCGGTAGGCGTGTACGGTGGGAGGTCTATATAAGCAGAGCTGGTTTAGTGAACCGTCAGATCAC
TAGTCGACTAGGGATAACAGGGCACCATGTCTAGACTGGACAAGAGCAAAGTCATAAACTCTGCTCTGGAATTACTC
AATGGAGTCGGTATCGAAGGCCTGACGACAAGGAAACTCGCTCAAAAGCTGGGAGTTGAGCAGCCTACCCTGTACT
GGCACGTGAAGAACAAGCGGGCCCTGCTCGATGCCCTGCCAATCGAGATGCTGGACAGGCATCATACCCACTCCTG
CCCCCTGGAAGGCGAGTCATGGCAAGACTTTCTGCGGAACAACGCCAAGTCATACCGCTGTGCTCTCCTCTCACATC
GCGACGGGGCTAAAGTGCATCTCGGCACCCGCCCAACAGAGAAACAGTACGAAACCCTGGAAAATCAGCTCGCGTT
CCTGTGTCAGCAAGGCTTCTCCCTGGAGAACGCACTGTACGCTCTGTCCGCCGTGGGCCACTTTACACTGGGCTGCG
TATTGGAGGAACAGGAGCATCAAGTAGCAAAAGAGGAAAGAGAGACACCTACCACCGATTCTATGCCCCCACTTCT
GAAACAAGCAATTGAGCTGTTCGACCGGCAGGGAGCCGAACCTGCCTTCCTTTTCGGCCTGGAACTAATCATATGTG
GCCTGGAGAAACAGCTAAAGTGCGAAAGCGGCGGGCCGACCGACGCCCTTGACGATTTTGACTTAGACATGCTCCC
AGCCGATGCCCTTGACGACTTTGACCTTGATATGCTGCCTGCTGACGCTCTTGACGATTTTGACCTTGACATGCTCCC
CGGGTAATGATCGACTGTGCCTTCTAGTTGCCAGCCATCTGTTGTTTGCCCCTCCCCCGTGCCTTCCTTGACCCTGGA
AGGTGCCACTCCCACTGTCCTTTCCTAATAAAATGAGGAAATTGCATCGCATTGTCTGAGTAGGTGTCATTCTATTCT
GGGGGGTGGGGTGGGGCAGGACAGCAAGGGGGAGGATTGGGAGGACAATAGCAGGCATGCTGGGGATGCGGT
GGGCTCTATGGCTTCGCTTTCGTCTTCAAGAATTCCTGGAGTTTACTCCCTATCAGTGATAGAGAACGTATGAAGAGT
TTACTCCCTATCAGTGATAGAGAACGTATGCAGACTTTACTCCCTATCAGTGATAGAGAACGTATAAGGAGTTTACTC

CCTATCAGTGATAGAGAACGTATGACCAGTTTACTCCCTATCAGTGATAGAGAACGTATCTACAGTTTACTCCCTATC
AGTGATAGAGAACGTATATCCAGTTTACTCCCTATCAGTGATAGAGAACGTATAAGCTTTAGGCGTGTACGGTGGGC
GCCTATAAAAGCAGAGCTCGTTTAGTGAACCGTCAGATCGCCTGGAGCAATTCCACAACACTTTTGTCTTATACCAAC
TTTCCGTACCACTTCCTACCCTCGTAAACCAG**AGC**ATGAAGGTATTCCGGCGCGCTTGGCGGCACCGGGTGGCGCTG
GGCCTAGGCGGCCTGGCGTTCTGCGGCACCACTCTGTTGTACCTGGCGCGCTGCGCTTCCGAGGGCGAGACGCCCT
CCGCTTCCGGAGCCGCTCGGCCCCGCGCTAAGGCCTTCCTGGCGGTGCTGGTGGCCAGTGCGCCCCGCGCGGTCGA
GCGCCGCACCGCAGTGCGCAGCACGTGGCTGGCACCGGAGAGGCGTGGCGGACCCGAGGACGTGTGGGCGCGCT
TCGCCGTGGGCACTGGCGGCTTAGGCTCGGAGGAGCGGCGCGCTCTTGAGCTCGAGCAGGCGCAGCACGGGGACC
TGCTGCTGCTGCCCGCCCTGCGCGACGCCTACGAGAACCTCACGGCCAAGGTCCTGGCCATGCTGACCTGGCTGGAT
GAGCGCGTGGACTTCGAGTTCGTGCTCAAGGCGGACGACGACTCCTTTGCGCGCCTGGACGCTATCCTGGTGGACC
TACGCGCACGGGAGCCCGCACGCCGCCGGCGCCTCTACTGGGGCTTCTTTTCCGGGCGCGGGCGCGTCAAGCCGGG
AGGTCGCTGGCGAGAAGCAGCCTGGCAACTCTGCGACTACTACCTGCCCTACGCGTTGGGCGGTGGCTATGTCCTTT
CTGCGGACCTGGTGCATTACCTGCGCCTCAGCCGCGAGTACCTGCGCGCGTGGCACAGTGAAGACGTATCGCTGGG
CACCTGGCTGGCACCAGTGGATGTGCAACGGGAGCACGACCCACGCTTCGACACGGAGTACAAATCTCGAGGCTGC
AACAATCAGTATCTGGTGACACACAAGCAAAGCCCAGAGGACATGTTGGAGAAGCAACAGATGTTGCTGCATGAG
GGCCGGTTGTGCAAGCATGAGGTGCAACTTCGCCTTTCCTATGTCTATGACTGGTCAGCTCCACCCTCCCAGTGCTGC
CAGCGCAAGGAGGGCGTTCCCTG**ATGTC**ACCGCGGGGGAGGCTAACTGAAACACGGAAGGAGACAATACCGGAA
GGAACCCGCGCTATGACGGCAATAAAAAGACAGAATAAAACGCACGGTGTTGGGTCGTTTGTTCATAAACGCGGG
GTTCGGTCCCAGGGCTGGCACTCTGTCGATACCCCACCGAGGCCCCATTGGGGCCAATACGCCCGCGTTTCTTCCTTT
TCCCCACCCCACCCCCCAAGTTCGGGTGAAGGCCCAGGGCTCGCAGCCAACGTCGGGGCGGCAGGCCCTGCCATAG
CCACAAACAGGGTCGACAAGCTTTTCCAAAAAAAAAGCATGAGGTGCAGTTGCGCCTTTCCTATCTCTTGAATAGGA
AAGGCGCAACTGCACCTCATGCTGGATCCCGCGTCCTTTCCACAAGATATATAAACCCAAGAAATCGAAATACTTTCA
AGTTACGGTAAGCATATGATAGTCCATTTTAAAACATAATTTTAAAACTGCAAACTACCCAAGAAATTATTACTTTCTA
CGTCACGTATTTTGTACTAATATCTTTGTGTTTACAGTCAAATTAATTCTAATTATCTCTCTAACAGCCTTGTATCGTAT
ATGCAAATATGAAGGAATCATGGGAAATAGGCCCTCTTCCTGCCCGACCTTGGCGCGCGCTCGGCGCGCGGTCACG
CTCCGTCACGTGGTGCGTTTTG

L'analyse de restriction par triple digestion NdeI, SalI, XhoI de la realisation 7 est illustrée figure 18.

Vérification fonctionnelle de la construction N°7: des cellules eucaryotes (ex : cellules cancéreuses mammaires de souris 4T1) sont transfectées avec le vecteur V3 selon un protocole usuel (exemple par lipotransfection ou électroporation). Après transfection les cellules sont traitées ou non avec de 100-1000 ng.mL$^{-1}$ de Doxycycline pendant 24 à 48 heures. Les cellules sont ensuite collectées lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel.

Conditions spécifiques de la PCR quantitative:

40 cycles des trois étapes suivantes sont réalisés: dénaturation 94°C-30s ; hybridation 60°C-30s ; extension 72°C-30s.

Amorces utilisées:

BETA3Galt6ms2-s ACCACTCTGTTGTACCTGGC (SEQ ID NO : 42).
BETA3Galt6ms2-as CACACGTCCTCGGGTCC (SEQ ID NO : 43).

**Réalisation 8**

**V2b (appartient au groupe de vecteurs V2) : Vecteur permettant d'exprimer un ou plusieurs transgènes de façon inductible. V2b se distingue de V2 par la structuration de son unité fonctionnelle bactérienne en deux briques au lieu d'une.**

$$U1 + U2c + nxU2d + mxU2e$$

U1 : Unité fonctionnelle bactérienne
U2c = gène codant un transactivateur transcriptionel.
U2d = gène(s) dont le promoteur est dépendant du transactivateur codé par le gène U2c, n ≥1
U2e = gène(s) dont le promoteur n'est pas dépendant du transactivateur codé par le gène U2c, m ≥0

Exemple : vecteur permettant l'expression inductible de l'enzyme mB3Galt6

| | |
|---|---|
| U1 | ori-Amp |
| U2c | promoteur CMV<br>Teton3G<br>Terminateur BPA |
| U2d | promoteur TRE3G<br>mB3Galt6<br>HSV Tk terminator |

Liste des briques utilisées pour la construction du vecteur V2b (figure 19)

**Brique Ori Bsal A (SEQ ID NO : 104)**

GAGGTACCGGTCTCATATTGTAATACGGTTATCCACAGAATCAGGGGATAACGCAGGAAAGAACATGTGAGCAAAAGGCCAGCAAAAGGCCAGGAACCGTAAAAAGGCCGCGTTGCTGGCGTTTTTCCATAGGCTCCGCCCCCCTGACGAGCATCACAAAAATCGACGCTCAAGTCAGAGGTGGCGAAACCCGACAGGACTATAAAGATACCAGGCGTTTCCCCCTGGAAGCTCCCTCGTGCGCTCTCCTGTTCCGACCCTGCCGCTTACCGGATACCTGTCCGCCTTTCTCCCTTCGGGAAGCGTGGCGCTTTCTCATAGCTCACGCTGTAGGTATCTCAGTTCGGTGTAGGTCGTTCGCTCCAAGCTGGGCTGTGTGCACGAACCCCCCGTTCAGCCCGACCGCTGCGCCTTATCCGGTAACTATCGTCTTGAGTCCAACCCGGTAAGACACGACTTATCGCCACTGGCAGCAGCCACTGGTAACAGGATTAGCAGAGCGAGGTATGTAGGCGGTGCTACAGAGTTCTTGAAGTGGTGGCCTAACTACGGCTACACTAGAAGAACAGTATTTGGTATCTGCGCTCTGCTGAAGCCAGTTACCTTCGGAAAAAGAGTTGGTAGCTCTTGATCCGGCAAACAAACCACCGCTGGTAGCGGTTTTTTTGTTTGCAAGCAGCAGATTACGCGCAGAAAAAAAGGATCTCAAGAAGATCCTTTGATCTTTTCTACGGGGTCTGACGCTCAGTGGAACGAAAACTCACGTTAAGGGATTTTGGTCATGAGATTATCAAAAAGGATCTTCACCTAGATCCTTTTAAATTAAAAATGAAGTTTTAAATCAATCTAAAGTATATATGAGTTTTATGAGACCGGTACCTC

**Brique AmpR BsaI A (SEQ ID NO : 105)**

GAGGTACCGGTCTCTTTTATTGGTCTGACAGTTACCAATGCTTAATCAGTGAGGCACCTATCTCAGCGATCTGTCTATTTCGTTCATCCATAGTTGCCTGACTCCCCGTCGTGTAGATAACTACGATACGGGAGGGCTTACCATCTGGCCCCAGTGCTGCAATGATACCGCGTGACCCACGCTCACCGGCTCCAGATTTATCAGCAATAAACCAGCCAGCCGGAAGGGCCGAGCGCAGAAGTGGTCCTGCAACTTTATCCGCCTCCATCCAGTCTATTAATTGTTGCCGGGAAGCTAGAGTAAGTAGTTCGCCAGTTAATAGTTTGCGCAACGTTGTTGCCATTGCTACAGGCATCGTGGTGTCACGCTCGTCGTTTGGTATGGCTTCATTCAGCTCCGGTTCCCAACGATCAAGGCGAGTTACATGATCCCCCATGTTGTGCAAAAAAGCGGTTAGCTCCTTCGGTCCTCCGATCGTTGTCAGAAGTAAGTTGGCCGCAGTGTTATCACTCATGGTTATGGCAGCACTGCATAATTCTCTTACTGTCATGCCATCCGTAAGATGCTTTTCTGTGACTGGTGAGTACTCAACCAAGTCATTCTGAGAATAGTGTATGCGGCGACCGAGTTGCTCTTGCCCGGCGTCAATACGGGATAATACCGCGCCACATAGCAGAACTTTAAAAGTGCTCATCATTGGAAAACGTTCTTCGGGGCGAAAACTCTCAAGGATCTTACCGCTGTTGAGATCCAGTTCGATGTAACCCACTCGTGCACCCAACTGATCTTCAGCATCTTTTACTTTCACCAGCGTTTCTGGGTGAGCAAAAACAGGAAGGCAAAATGCCGCAAAAAAGGGAATAAGGGCGACACGGAAATGTTGAATACTCATACTCTTCCTTTTTCAATATTATTGAAGCATTTATCAGGGTTATTGTCTCATGAGCGGATACATATTTGAATGTATTTAGAAAAATAAACAAATAGGGGTTCCGCGCACATTTCCCCGAAAAGTGCCAAGGACGAGACCGGTACCTC

**Brique pCMV BsaI B (SEQ ID NO : 37)**
**Brique TO3G BsaI A (SEQ ID NO : 59)**
**Brique BGHpA BsaI B (SEQ ID NO : 39)**
**Brique pTRE3G BsaI A (SEQ ID NO : 60)**
**Brique mb3Galt6 BsaI B (SEQ ID NO : 63)**
**Brique Tkter BsaI A (SEQ ID NO : 57)**

**Vecteur V2b (SEQ ID NO : 149,, exemple de vecteur du groupe V2)**

TATTGTAATACGGTTATCCACAGAATCAGGGGATAACGCAGGAAAGAACATGTGAGCAAAAGGCCAGCAAAAGG
CCAGGAACCGTAAAAAGGCCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGAAGCGTGGCGCTTTCTCA
TAGCTCACGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCCACTGGC
AGCAGCCACTGGTAACAGGATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTTTTTTTGTTTGCAAGCAGCAGATTACGCGCAGAAAAA
AAGGATCTCAAGAAGATCCTTTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGAAGTTTTAAATCAATCTAAAG
TATATATGAGTTTTATTGGTCTGACAGTTACCAATGCTTAATCAGTGAGGCACCTATCTCAGCGATCTGTCTATTTCG
TTCATCCATAGTTGCCTGACTCCCCGTCGTGTAGATAACTACGATACGGGAGGGCTTACCATCTGGCCCCAGTGCTG
CAATGATACCGCGTGACCCACGCTCACCGGCTCCAGATTTATCAGCAATAAACCAGCCAGCCGGAAGGGCCGAGC
GCAGAAGTGGTCCTGCAACTTTATCCGCCTCCATCCAGTCTATTAATTGTTGCCGGGAAGCTAGAGTAAGTAGTTCG
CCAGTTAATAGTTTGCGCAACGTTGTTGCCATTGCTACAGGCATCGTGGTGTCACGCTCGTCGTTTGGTATGGCTTC
ATTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT

ATCAGGGTTATTGTCTCATGAGCGGATACATATTTGAATGTATTTAGAAAAATAAACAAATAGGGGTTCCGCGCAC
ATTTCCCCGAAAAGTGCCAAGGAACCAATTCAGTCGACTGGATCCTAGTTATTAATAGTAATCAATTACGGGGTCAT
TAGTTCATAGCCCATATATGGAGTTCCGCGTTACATAACTTACGGTAAATGGCCCGCCTGGCTGACCGCCCAACGA
CCCCCGCCCATTGACGTCAATAATGACGTATGTTCCCATAGTAACGCCAATAGGGACTTTCCATTGACGTCAATGGG
TGGAGTATTTACGGTAAACTGCCCACTTGGCAGTACATCAAGTGTATCATATGCCAAGTACGCCCCCTATTGACGTC
AATGACGGTAAATGGCCCGCCTGGCATTATGCCCAGTACATGACCTTATGGGACTTTCCTACTTGGCAGTACATCTA
CGTATTAGTCATCGCTATTACCATGGTGATGCGGTTTTGGCAGTACATCAATGGGCGTGGATAGCGGTTTGACTCA
CGGGGATTTCCAAGTCTCCACCCCATTGACGTCAATGGGAGTTTGTTTTGGCACCAAAATCAACGGGACTTTCCAAA
ATGTCGTAACAACTCCGCCCCATTGACGCAAATGGGCGGTAGGCGTGTACGGTGGGAGGTCTATATAAGCAGAGC
TGGTTTAGTGAACCGTCAGATCACTAGTCGACTAGGGATAACAGGGCACCATGTCTAGACTGGACAAGAGCAAAG
TCATAAACTCTGCTCTGGAATTACTCAATGGAGTCGGTATCGAAGGCCTGACGACAAGGAAACTCGCTCAAAAGCT
GGGAGTTGAGCAGCCTACCCTGTACTGGCACGTGAAGAACAAGCGGGCCCTGCTCGATGCCCTGCCAATCGAGAT
GCTGGACAGGCATCATACCCACTCCTGCCCCCTGGAAGGCGAGTCATGGCAAGACTTTCTGCGGAACAACGCCAA
GTCATACCGCTGTGCTCTCCTCTCACATCGCGACGGGGCTAAAGTGCATCTCGGCACCCGCCCAACAGAGAAACAG
TACGAAACCCTGGAAAATCAGCTCGCGTTCCTGTGTCAGCAAGGCTTCTCCCTGGAGAACGCACTGTACGCTCTGT
CCGCCGTGGGCCACTTTACACTGGGCTGCGTATTGGAGGAACAGGAGCATCAAGTAGCAAAAGAGGAAAGAGAG
ACACCTACCACCGATTCTATGCCCCCACTTCTGAAACAAGCAATTGAGCTGTTCGACCGGCAGGGAGCCGAACCTG
CCTTCCTTTTCGGCCTGGAACTAATCATATGTGGCCTGGAGAAACAGCTAAAGTGCGAAAGCGGCGGGCCGACCG
ACGCCCTTGACGATTTTGACTTAGACATGCTCCCAGCCGATGCCCTTGACGACTTTGACCTTGATATGCTGCCTGCT
GACGCTCTTGACGATTTTGACCTTGACATGCTCCCCGGGTAATGATCGACTGTGCCTTCTAGTTGCCAGCCATCTGT
TGTTTGCCCCTCCCCCGTGCCTTCCTTGACCCTGGAAGGTGCCACTCCCACTGTCCTTTCCTAATAAAATGAGGAAAT
TGCATCGCATTGTCTGAGTAGGTGTCATTCTATTCTGGGGGGTGGGGTGGGGCAGGACAGCAAGGGGGAGGATT
GGGAGGACAATAGCAGGCATGCTGGGGATGCGGTGGGCTCTATGGCTTCGCTTTCGTCTTCAAGAATTCCTGGAG
TTTACTCCCTATCAGTGATAGAGAACGTATGAAGAGTTTACTCCCTATCAGTGATAGAGAACGTATGCAGACTTTAC
TCCCTATCAGTGATAGAGAACGTATAAGGAGTTTACTCCCTATCAGTGATAGAGAACGTATGACCAGTTTACTCCCT
ATCAGTGATAGAGAACGTATCTACAGTTTACTCCCTATCAGTGATAGAGAACGTATATCCAGTTTACTCCCTATCAG
TGATAGAGAACGTATAAGCTTTAGGCGTGTACGGTGGGCGCCTATAAAAGCAGAGCTCGTTTAGTGAACCGTCAG
ATCGCCTGGAGCAATTCCACAACACTTTTGTCTTATACCAACTTTCCGTACCACTTCCTACCCTCGTAAACCAGAGCA
TGAAGGTATTCCGGCGCGCTTGGCGGCACCGGGTGGCGCTGGGCCTAGGCGGCCTGGCGTTCTGCGGCACCACTC
TGTTGTACCTGGCGCGCTGCGCTTCCGAGGGCGAGACGCCCTCCGCTTCCGGAGCCGCTCGGCCCCGCGCTAAGG
CCTTCCTGGCGGTGCTGGTGGCCAGTGCGCCCCGCGCGGTCGAGCGCCGCACCGCAGTGCGCAGCACGTGGCTGG
CACCGGAGAGGCGTGGCGGACCCGAGGACGTGTGGGCGCGCTTCGCCGTGGGCACTGGCGGCTTAGGCTCGGAG

GAGCGGCGCGCTCTTGAGCTCGAGCAGGCGCAGCACGGGGACCTGCTGCTGCCCGCCCTGCGCGACGCCTAC
GAGAACCTCACGGCCAAGGTCCTGGCCATGCTGACCTGGCTGGATGAGCGCGTGGACTTCGAGTTCGTGCTCAAG
GCGGACGACGACTCCTTTGCGCGCCTGGACGCTATCCTGGTGGACCTACGCGCACGGGAGCCCGCACGCCGCCGG
CGCCTCTACTGGGGCTTCTTTTCCGGGCGCGGGCGCGTCAAGCCGGGAGGTCGCTGGCGAGAAGCAGCCTGGCAA
CTCTGCGACTACTACCTGCCCTACGCGTTGGGCGGTGGCTATGTCCTTTCTGCGGACCTGGTGCATTACCTGCGCCT
CAGCCGCGAGTACCTGCGCGCGTGGCACAGTGAAGACGTATCGCTGGGCACCTGGCTGGCACCAGTGGATGTGCA
ACGGGAGCACGACCCACGCTTCGACACGGAGTACAAATCTCGAGGCTGCAACAATCAGTATCTGGTGACACACAA
GCAAAGCCCAGAGGACATGTTGGAGAAGCAACAGATGTTGCTGCATGAGGGCCGGTTGTGCAAGCATGAGGTGC
AACTTCGCCTTTCCTATGTCTATGACTGGTCAGCTCCACCCTCCCAGTGCTGCCAGCGCAAGGAGGGCGTTCCCTGA
TGTCACCGCGGGGGAGGCTAACTGAAACACGGAAGGAGACAATACCGGAAGGAACCCGCGCTATGACGGCAATA
AAAAGACAGAATAAAACGCACGGTGTTGGGTCGTTTGTTCATAAACGCGGGGTTCGGTCCCAGGGCTGGCACTCT
GTCGATACCCCACCGAGGCCCCATTGGGGCCAATACGCCCGCGTTTCTTCCTTTTCCCCACCCCACCCCCCAAGTTC
GGGTGAAGGCCCAGGGCTCGCAGCCAACGTCGGGGCGGCAGGCCCTGCCATAGCC

L'Empreinte de restriction par triple digestion (validation structurelle), NdeI, SalI, XhoI, de la réalisation 8 est illustrée Figure 20.

Vérification fonctionnelle de la réalisation 8 : des cellules eucaryotes (ex : cellules cancéreuses mammaires de souris 4T1) sont transfectées avec le vecteur V2b selon un protocole usuel (exemple lipotransfection ou électroporation). Après transfection les cellules sont traitées avec 100-1000 ng.mL$^{-1}$ de Doxycycline pendant 24 à 48 heures. Les cellules sont ensuite collectées et lysées pour en extraire les ARN totaux puis générer des ADN complémentaires (ADNc) par transcription inverse selon un protocole usuel. Ces ADNc sont ensuite utilisés comme matrice pour une analyse en PCR quantitative selon un protocole usuel. (Figure 21)
L'expérience montre que l'expression du transcrit mB3Galt6, codé par le vecteur V2b, est augmentée en cellule 4T1 uniquement en présence de doxocycline, démontrant les présences concomitantes d'un transgène inductible et de son co-activateur dans le vecteur V2b.

Conditions spécifiques de la PCR quantitative:

40 cycles des trois étapes suivantes sont réalisés: dénaturation 94°C-30s ; hybridation 60°C-30s ; extension 72°C-30s.

Amorces utilisées:

BETA3Galt6ms2-s ACCACTCTGTTGTACCTGGC (SEQ ID NO : 42).
BETA3Galt6ms2-as CACACGTCCTCGGGTCC (SEQ ID NO : 43).

**Réalisation 9**

**V3b : (appartient au groupe de vecteurs V3) Vecteur permettant de réaliser de la complémentation génétique sous contrôle inductible. V3b se distingue de V3 par la structuration de son unité fonctionnelle bactérienne en deux briques au lieu d'une.**

U1 + U2f + U2c +U2g

U1 : Unité fonctionnelle bactérienne
U2f = gène dont le promoteur est un promoteur à ARN polymérase III et dont le produit d'expression est un petit ARN en épingle à cheveux (shRNA) précurseur d'un petit ARN interférant ciblant un gène X.
U2c = gène codant un transactivateur transcriptionel (exemple : protéine TAT du VIH-1 ou-2).
U2g = gène dont le promoteur est dépendant du transactivateur codé par le gène U2c et dont le produit d'expression est une version mutée du produit du gène X de sorte à être insensible au produit du gène U2f

Exemple : Vecteur permettant de réprimer l'expression de l'enzyme mB3Galt6 tout en surexprimant de façon inductible l'expression de cette enzyme (complémentation possible).

| | |
|---|---|
| U1 | Ori<br>AmpR |
| U2c | promoteur CMV<br>Teton3G<br>Terminateur BPA |
| U2g | promoteur TRE3G<br>mB3Galt6 - muté<br>HSV Tk terminator |
| U2f | cassette shRNA mB3Galt6 |

Liste des briques utilisées pour la construction du vecteur V3b (figure 22)

**Brique Ori BsaI A (SEQ ID NO : 104)**
**Brique AmpR BsaI A (SEQ ID NO : 105)**
**Brique pCMV BsaI B (SEQ ID NO : 37)**
**Brique TO3G BsaI A (SEQ ID NO : 59)**
**Brique BGHpA BsaI B (SEQ ID NO : 39)**
**Brique pTRE3G BsaI A (SEQ ID NO : 60)**
**Brique mb3Galt6 BsaI B (SEQ ID NO : 63)**
**Brique Tkter BsaI B (SEQ ID NO : 64)**
**Brique shB3Galt6 BsaI C (SEQ ID NO : 65)**

**Vecteur V3b (SEQ ID NO : 150, exemple de vecteur du groupe V3)**

TATTGTAATACGGTTATCCACAGAATCAGGGGATAACGCAGGAAAGAACATGTGAGCAAAAGGCCAGCAAAAGG
CCAGGAACCGTAAAAAGGCCGCGTTGCTGGCGTTTTTCCATAGGCTCCGCCCCCCTGACGAGCATCACAAAAATCG
ACGCTCAAGTCAGAGGTGGCGAAACCCGACAGGACTATAAAGATACCAGGCGTTTCCCCCTGGAAGCTCCCTCGT
GCGCTCTCCTGTTCCGACCCTGCCGCTTACCGGATACCTGTCCGCCTTTCTCCCTTCGGGAAGCGTGGCGCTTTCTCA
TAGCTCACGCTGTAGGTATCTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACACGACTTATCGCCACTGGC
AGCAGCCACTGGTAACAGGATTAGCAGAGCGAGGTATGTAGGCGGTGCTACAGAGTTCTTGAAGTGGTGGCCTAA
CTACGGCTACACTAGAAGAACAGTATTTGGTATCTGCGCTCTGCTGAAGCCAGTTACCTTCGGAAAAAGAGTTGGT
AGCTCTTGATCCGGCAAACAAACCACCGCTGGTAGCGGTTTTTTTGTTTGCAAGCAGCAGATTACGCGCAGAAAAA
AAGGATCTCAAGAAGATCCTTTGATCTTTTCTACGGGGTCTGACGCTCAGTGGAACGAAAACTCACGTTAAGGGAT
TTTGGTCATGAGATTATCAAAAAGGATCTTCACCTAGATCCTTTTAAATTAAAAATGAAGTTTTAAATCAATCTAAAG
TATATATGAGTTTTATTGGTCTGACAGTTACCAATGCTTAATCAGTGAGGCACCTATCTCAGCGATCTGTCTATTTCG
TTCATCCATAGTTGCCTGACTCCCCGTCGTGTAGATAACTACGATACGGGAGGGCTTACCATCTGGCCCCAGTGCTG
CAATGATACCGCGTGACCCACGCTCACCGGCTCCAGATTTATCAGCAATAAACCAGCCAGCCGGAAGGGCCGAGC
GCAGAAGTGGTCCTGCAACTTTATCCGCCTCCATCCAGTCTATTAATTGTTGCCGGGAAGCTAGAGTAAGTAGTTCG
CCAGTTAATAGTTTGCGCAACGTTGTTGCCATTGCTACAGGCATCGTGGTGTCACGCTCGTCGTTTGGTATGGCTTC
ATTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC

CGCAAAAAAGGGAATAAGGGCGACACGGAAATGTTGAATACTCATACTCTTCCTTTTTCAATATTATTGAAGCATTT ATCAGGGTTATTGTCTCATGAGCGGATACATATTTGAATGTATTTAGAAAAATAAACAAATAGGGGTTCCGCGCAC ATTTCCCCGAAAAGTGCCAAGGAACCAATTCAGTCGACTGGATCCTAGTTATTAATAGTAATCAATTACGGGGTCAT TAGTTCATAGCCCATATATGGAGTTCCGCGTTACATAACTTACGGTAAATGGCCCGCCTGGCTGACCGCCCAACGA CCCCCGCCCATTGACGTCAATAATGACGTATGTTCCCATAGTAACGCCAATAGGGACTTTCCATTGACGTCAATGGG TGGAGTATTTACGGTAAACTGCCCACTTGGCAGTACATCAAGTGTATCATATGCCAAGTACGCCCCCTATTGACGTC AATGACGGTAAATGGCCCGCCTGGCATTATGCCCAGTACATGACCTTATGGGACTTTCCTACTTGGCAGTACATCTA CGTATTAGTCATCGCTATTACCATGGTGATGCGGTTTTGGCAGTACATCAATGGGCGTGGATAGCGGTTTGACTCA CGGGGATTTCCAAGTCTCCACCCCATTGACGTCAATGGGAGTTTGTTTTGGCACCAAAATCAACGGGACTTTCCAAA ATGTCGTAACAACTCCGCCCCATTGACGCAAATGGGCGGTAGGCGTGTACGGTGGGAGGTCTATATAAGCAGAGC TGGTTTAGTGAACCGTCAGATCACTAGTCGACTAGGGATAACAGGGCACCATGTCTAGACTGGACAAGAGCAAAG TCATAAACTCTGCTCTGGAATTACTCAATGGAGTCGGTATCGAAGGCCTGACGACAAGGAAACTCGCTCAAAAGCT GGGAGTTGAGCAGCCTACCCTGTACTGGCACGTGAAGAACAAGCGGGCCCTGCTCGATGCCCTGCCAATCGAGAT GCTGGACAGGCATCATACCCACTCCTGCCCCCTGGAAGGCGAGTCATGGCAAGACTTTCTGCGGAACAACGCCAA GTCATACCGCTGTGCTCTCCTCTCACATCGCGACGGGGCTAAAGTGCATCTCGGCACCCGCCCAACAGAGAAACAG TACGAAACCCTGGAAAATCAGCTCGCGTTCCTGTGTCAGCAAGGCTTCTCCCTGGAGAACGCACTGTACGCTCTGT CCGCCGTGGGCCACTTTACACTGGGCTGCGTATTGGAGGAACAGGAGCATCAAGTAGCAAAAGAGGAAAGAGAG ACACCTACCACCGATTCTATGCCCCCACTTCTGAAACAAGCAATTGAGCTGTTCGACCGGCAGGGAGCCGAACCTG CCTTCCTTTTCGGCCTGGAACTAATCATATGTGGCCTGGAGAAACAGCTAAAGTGCGAAAGCGGCGGGCCGACCG ACGCCCTTGACGATTTTGACTTAGACATGCTCCCAGCCGATGCCCTTGACGACTTTGACCTTGATATGCTGCCTGCT GACGCTCTTGACGATTTTGACCTTGACATGCTCCCCGGGTAATGATCGACTGTGCCTTCTAGTTGCCAGCCATCTGT TGTTTGCCCCTCCCCCGTGCCTTCCTTGACCCTGGAAGGTGCCACTCCCACTGTCCTTTCCTAATAAAATGAGGAAAT TGCATCGCATTGTCTGAGTAGGTGTCATTCTATTCTGGGGGGTGGGGTGGGGCAGGACAGCAAGGGGGAGGATT GGGAGGACAATAGCAGGCATGCTGGGGATGCGGTGGGCTCTATGGCTTCGCTTTCGTCTTCAAGAATTCCTGGAG TTTACTCCCTATCAGTGATAGAGAACGTATGAAGAGTTTACTCCCTATCAGTGATAGAGAACGTATGCAGACTTTAC TCCCTATCAGTGATAGAGAACGTATAAGGAGTTTACTCCCTATCAGTGATAGAGAACGTATGACCAGTTTACTCCCT ATCAGTGATAGAGAACGTATCTACAGTTTACTCCCTATCAGTGATAGAGAACGTATATCCAGTTTACTCCCTATCAG TGATAGAGAACGTATAAGCTTTAGGCGTGTACGGTGGGCGCCTATAAAAGCAGAGCTCGTTTAGTGAACCGTCAG ATCGCCTGGAGCAATTCCACAACACTTTTGTCTTATACCAACTTTCCGTACCACTTCCTACCCTCGTAAACCAGAGCA TGAAGGTATTCCGGCGCGCTTGGCGGCACCGGGTGGCGCTGGGCCTAGGCGGCCTGGCGTTCTGCGGCACCACTC TGTTGTACCTGGCGCGCTGCGCTTCCGAGGGCGAGACGCCCTCCGCTTCCGGAGCCGCTCGGCCCCGCGCTAAGG CCTTCCTGGCGGTGCTGGTGGCCAGTGCGCCCCGCGCGGTCGAGCGCCGCACCGCAGTGCGCAGCACGTGGCTGG

CACCGGAGAGGCGTGGCGGACCCGAGGACGTGTGGGCGCGCTTCGCCGTGGGCACTGGCGGCTTAGGCTCGGAG
GAGCGGCGCGCTCTTGAGCTCGAGCAGGCGCAGCACGGGGACCTGCTGCTGCCCGCCCTGCGCGACGCCTAC
GAGAACCTCACGGCCAAGGTCCTGGCCATGCTGACCTGGCTGGATGAGCGCGTGGACTTCGAGTTCGTGCTCAAG
GCGGACGACGACTCCTTTGCGCGCCTGGACGCTATCCTGGTGGACCTACGCGCACGGGAGCCCGCACGCCGCCGG
CGCCTCTACTGGGGCTTCTTTTCCGGGCGCGGGCGCGTCAAGCCGGGAGGTCGCTGGCGAGAAGCAGCCTGGCAA
CTCTGCGACTACTACCTGCCCTACGCGTTGGGCGGTGGCTATGTCCTTTCTGCGGACCTGGTGCATTACCTGCGCCT
CAGCCGCGAGTACCTGCGCGCGTGGCACAGTGAAGACGTATCGCTGGGCACCTGGCTGGCACCAGTGGATGTGCA
ACGGGAGCACGACCCACGCTTCGACACGGAGTACAAATCTCGAGGCTGCAACAATCAGTATCTGGTGACACACAA
GCAAAGCCCAGAGGACATGTTGGAGAAGCAACAGATGTTGCTGCATGAGGGCCGGTTGTGCAAGCATGAGGTGC
AACTTCGCCTTTCCTATGTCTATGACTGGTCAGCTCCACCCTCCCAGTGCTGCCAGCGCAAGGAGGGCGTTCCCTGA
TGTCACCGCGGGGGAGGCTAACTGAAACACGGAAGGAGACAATACCGGAAGGAACCCGCGCTATGACGGCAATA
AAAAGACAGAATAAAACGCACGGTGTTGGGTCGTTTGTTCATAAACGCGGGGTTCGGTCCCAGGGCTGGCACTCT
GTCGATACCCCACCGAGGCCCCATTGGGGCCAATACGCCCGCGTTTCTTCCTTTTCCCCACCCCACCCCCCAAGTTC
GGGTGAAGGCCCAGGGCTCGCAGCCAACGTCGGGGCGGCAGGCCCTGCCATAGCCACAAACAGGGTCGACAAGC
TTTTCCAAAAAAAAAGCATGAGGTGCAGTTGCGCCTTTCCTATCTCTTGAATAGGAAAGGCGCAACTGCACCTCAT
GCTGGATCCCGCGTCCTTTCCACAAGATATATAAACCCAAGAAATCGAAATACTTTCAAGTTACGGTAAGCATATGA
TAGTCCATTTTAAAACATAATTTTAAAACTGCAAACTACCCAAGAAATTATTACTTTCTACGTCACGTATTTTGTACTA
ATATCTTTGTGTTTACAGTCAAATTAATTCTAATTATCTCTCTAACAGCCTTGTATCGTATATGCAAATATGAAGGAA
TCATGGGAAATAGGCCCTCTTCCTGCCCGACCTTGGCGCGCGCTCGGCGCGCGGTCACGCTCCGTCACGTGGTGCG
TTTTG

L'Empreinte de restriction par triple digestion (validation structurelle), NdeI, SalI, XhoI, de la réalisation 9 est illustrée Figure 23

**Réalisation 10**

**V1.1b : (appartient au groupe de vecteurs V1.1) Vecteur permettant de sélectionner l'intégration des transgènes par recombinaison non homologue dans le génome cible.**

**Ce vecteur se distingue du vecteur V1.1 par la structuration de son unité fonctionnelle bactérienne en deux briques au lieu d'une.**

U1+ U2 + U3a

U1 : Unité fonctionnelle bactérienne

U2 : nxU2a + mxU2b, n≥0, m≥0 et n+m≥2

U2a : Unité fonctionnelle d'expression dont le promoteur est dépendant de l'ARN polymerase II et dont le produit d'expression est une protéineU3a : cassette de sélection positive

Exemple : Vecteur permettant d'exprimer deux protéines de fusions constituées d'un domaine fluorescent et d'un domaine d'adressage à un compartiment cellulaire spécifique (ici membrane cellulaire et appareil de Golgi).

| | |
|---|---|
| U1 | Ori<br>AmpR |
| U2a-1 | promoteur EFlalpha<br>protéine de fusion EGFP-CAAX<br>terminateur BPA |
| U2a-2 | promoteur CMV<br>domaine N-terminal de protéine de fusion : SiaT<br>domaine C-terminal de protéine de fusion : mCherry<br>terminateur HSV-TK |
| U3a | Hygomicin resistance |

Liste des briques utilisées pour la construction du vecteur V1.1b (Figure 24)

**Brique Ori BsaI B (SEQ ID NO : 106)**

GAGGTACCGGTCTCTATTACGGTAATACGGTTATCCACAGAATCAGGGGATAACGCAGGAAAGAACATGTGAGCA
AAAGGCCAGCAAAAGGCCAGGAACCGTAAAAAGGCCGCGTTGCTGGCGTTTTTCCATAGGCTCCGCCCCCCTGAC
GAGCATCACAAAAATCGACGCTCAAGTCAGAGGTGGCGAAACCCGACAGGACTATAAAGATACCAGGCGTTTCCC
CCTGGAAGCTCCCTCGTGCGCTCTCCTGTTCCGACCCTGCCGCTTACCGGATACCTGTCCGCCTTTCTCCCTTCGGGA
AGCGTGGCGCTTTCTCATAGCTCACGCTGTAGGTATCTCAGTTCGGTGTAGGTCGTTCGCTCCAAGCTGGGCTGTG
TGCACGAACCCCCCGTTCAGCCCGACCGCTGCGCCTTATCCGGTAACTATCGTCTTGAGTCCAACCCGGTAAGACAC
GACTTATCGCCACTGGCAGCAGCCACTGGTAACAGGATTAGCAGAGCGAGGTATGTAGGCGGTGCTACAGAGTTC
TTGAAGTGGTGGCCTAACTACGGCTACACTAGAAGAACAGTATTTGGTATCTGCGCTCTGCTGAAGCCAGTTACCT
TCGGAAAAAGAGTTGGTAGCTCTTGATCCGGCAAACAAACCACCGCTGGTAGCGGTTTTTTTGTTTGCAAGCAGCA
GATTACGCGCAGAAAAAAAGGATCTCAAGAAGATCCTTTGATCTTTTCTACGGGGTCTGACGCTCAGTGGAACGAA
AACTCACGTTAAGGGATTTTGGTCATGAGATTATCAAAAAGGATCTTCACCTAGATCCTTTTAAATTAAAAATGAAG
TTTTAAATCAATCTAAAGTATATATGAGTAAACCGAGACCGGTACCTC

**Brique AmpR BsaI B (SEQ ID NO : 107)**

GAGGTACCGGTCTCAAAACTTGGTCTGACAGTTACCAATGCTTAATCAGTGAGGCACCTATCTCAGCGATCTGTCTA
TTTCGTTCATCCATAGTTGCCTGACTCCCCGTCGTGTAGATAACTACGATACGGGAGGGCTTACCATCTGGCCCCAG
TGCTGCAATGATACCGCGTGACCCACGCTCACCGGCTCCAGATTTATCAGCAATAAACCAGCCAGCCGGAAGGGCC
GAGCGCAGAAGTGGTCCTGCAACTTTATCCGCCTCCATCCAGTCTATTAATTGTTGCCGGGAAGCTAGAGTAAGTA
GTTCGCCAGTTAATAGTTTGCGCAACGTTGTTGCCATTGCTACAGGCATCGTGGTGTCACGCTCGTCGTTTGGTATG
GCTTCATTCAGCTCCGGTTCCCAACGATCAAGGCGAGTTACATGATCCCCCATGTTGTGCAAAAAAGCGGTTAGCTC
CTTCGGTCCTCCGATCGTTGTCAGAAGTAAGTTGGCCGCAGTGTTATCACTCATGGTTATGGCAGCACTGCATAATT
CTCTTACTGTCATGCCATCCGTAAGATGCTTTTCTGTGACTGGTGAGTACTCAACCAAGTCATTCTGAGAATAGTGT
ATGCGGCGACCGAGTTGCTCTTGCCCGGCGTCAATACGGGATAATACCGCGCCACATAGCAGAACTTTAAAAGTGC
TCATCATTGGAAAACGTTCTTCGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGAGCAAAAACAGGAAGGCAAAA
TGCCGCAAAAAAGGGAATAAGGGCGACACGGAAATGTTGAATACTCATACTCTTCCTTTTTCAATATTATTGAAGC
ATTTATCAGGGTTATTGTCTCATGAGCGGATACATATTTGAATGTATTTAGAAAAATAAACAAATAGGGGTTCCGC
GCACATTTCCCCGAAAAGTGCCAATGCTGAGACCGGTACCTC

**Brique pEF1aL BsaI B (SEQ ID NO : 108)**

GAGGTACCGGTCTCAATGCGTGAGGCTCCGGTGCCCGTCAGTGGGCAGAGCGCACATCGCCCACAGTCCCCGAGA
AGTTGGGGGGAGGGGTCGGCAATTGAACCGGTGCCTAGAGAAGGTGGCGCGGGGTAAACTGGGAAAGTGATGT
CGTGTACTGGCTCCGCCTTTTTCCCGAGGGTGGGGGAGAACCGTATATAAGTGCAGTAGTCGCCGTGAACGTTCTT
TTTCGCAACGGGTTTGCCGCCAGAACACAGGTAAGTGCCGTGTGTGGTTCCCGCGGGCCTGGCCTCTTTACGGGTT
ATGGCCCTTGCGTGCCTTGAATTACTTCCACCTGGCTGCAGTACGTGATTCTTGATCCCGAGCTTCGGGTTGGAAGT
GGGTGGGAGAGTTCGAGGCCTTGCGCTTAAGGAGCCCCTTCGCCTCGTGCTTGAGTTGAGGCCTGGCCTGGGCGC
TGGGGCCGCCGCGTGCGAATCTGGTGGCACCTTCGCGCCTGTCTCGCTGCTTTCGATAAGTCTCTAGCCATTTAAAA
TTTTTGATGACCTGCTGCGACGCTTTTTTTCTGGCAAGATAGTCTTGTAAATGCGGGCCAAGATCTGCACACTGGTA
TTTCGGTTTTTGGGGCCGCGGGCGGCGACGGGGCCCGTGCGTCCCAGCGCACATGTTCGGCGAGGCGGGGCCTG
CGAGCGCGGCCACCGAGAATCGGACGGGGGTAGTCTCAAGCTGGCCGGCCTGCTCTGGTGCCTGGCCTCGCGCCG
CCGTGTATCGCCCCGCCCTGGGCGGCAAGGCTGGCCCGGTCGGCACCAGTTGCGTGAGCGGAAAGATGGCCGCTT
CCCGGCCCTGCTGCAGGGAGCTCAAAATGGAGGACGCGGCGCTCGGGAGAGCGGGCGGGTGAGTCACCCACACA
AAGGAAAAGGGCCTTTCCGTCCTCAGCCGTCGCTTCATGTGACTCCACGGAGTACCGGGCGCCGTCCAGGCACCTC
GATTAGTTCTCGAGCTTTTGGAGTACGTCGTCTTTAGGTTGGGGGGAGGGGTTTTATGCGATGGAGTTTCCCCACA

CTGAGTGGGTGGAGACTGAAGTTAGGCCAGCTTGGCACTTGATGTAATTCTCCTTGGAATTTGCCCTTTTTGAGTTT
GGATCTTGGTTCATTCTCAAGCCTCAGACAGTGGTTCAAAGTTTTTTTCTTCCATTTCAGGTGTCGTGATATCCGAGA
CCGGTACCTC

**Brique EGFP-CAAX BsaI A (SEQ ID NO : 109)**

GAGGTACCGGTCTCCTATCATGGTGAGCAAGGGCGAGGAGCTGTTCACCGGGGTGGTGCCCATCCTGGTCGAGCT
GGACGGCGACGTAAACGGCCACAAGTTCAGCGTGTCCGGCGAGGGCGAGGGCGATGCCACCTACGGCAAGCTGA
CCCTGAAGTTCATCTGCACCACCGGCAAGCTGCCCGTGCCCTGGCCCACCCTCGTGACCACCCTGACCTACGGCGT
GCAGTGCTTCAGCCGCTACCCCGACCACATGAAGCAGCACGACTTCTTCAAGTCCGCCATGCCCGAAGGCTACGTC
CAGGAGCGCACCATCTTCTTCAAGGACGACGGCAACTACAAGACCCGCGCCGAGGTGAAGTTCGAGGGCGACACC
CTGGTGAACCGCATCGAGCTGAAGGGCATCGACTTCAAGGAGGACGGCAACATCCTGGGGCACAAGCTGGAGTA
CAACTACAACAGCCACAACGTCTATATCATGGCCGACAAGCAGAAGAACGGCATCAAGGTGAACTTCAAGATCCG
CCACAACATCGAGGACGGCAGCGTGCAGCTCGCCGACCACTACCAGCAGAACACCCCCATCGGCGACGGCCCCGT
GCTGCTGCCCGACAACCACTACCTGAGCACCCAGTCCGCCCTGAGCAAAGACCCCAACGAGAAGCGCGATCACAT
GGTCCTGCTGGAGTTCGTGACCGCCGCCGGGATCACTCTCGGCATGGACGAGCTGTACAAGAAGAAGAAAAAGAA
GTCAAAGACAAAGTGTGTAATTATGTAAGAGTGGAGACCGGTACCTC

**Brique BGHpA BsaI C (SEQ ID NO : 110)**

GAGGTACCGGTCTCAGAGTCGACTGTGCCTTCTAGTTGCCAGCCATCTGTTGTTTGCCCCTCCCCCGTGCCTTCCTTG
ACCCTGGAAGGTGCCACTCCCACTGTCCTTTCCTAATAAAATGAGGAAATTGCATCGCATTGTCTGAGTAGGTGTCA
TTCTATTCTGGGGGGTGGGGTGGGGCAGGACAGCAAGGGGGAGGATTGGGAGGACAATAGCAGGCATGCTGGG
GATGCGGTGGGCTCTATGGGTAGCGAGACCGGTACCTC

**Brique pCMV BsaI D (SEQ ID NO : 111)**

GAGGTACCGGTCTCAGTAGTTATTAATAGTAATCAATTACGGGGTCATTAGTTCATAGCCCATATATGGAGTTCCGC
GTTACATAACTTACGGTAAATGGCCCGCCTGGCTGACCGCCCAACGACCCCCGCCCATTGACGTCAATAATGACGT
ATGTTCCCATAGTAACGCCAATAGGGACTTTCCATTGACGTCAATGGGTGGAGTATTTACGGTAAACTGCCCACTTG
GCAGTACATCAAGTGTATCATATGCCAAGTACGCCCCCTATTGACGTCAATGACGGTAAATGGCCCGCCTGGCATT
ATGCCCAGTACATGACCTTATGGGACTTTCCTACTTGGCAGTACATCTACGTATTAGTCATCGCTATTACCATGGTG
ATGCGGTTTTGGCAGTACATCAATGGGCGTGGATAGCGGTTTGACTCACGGGGATTTCCAAGTCTCCACCCCATTG
ACGTCAATGGGAGTTTGTTTTGGCACCAAAATCAACGGGACTTTCCAAAATGTCGTAACAACTCCGCCCCATTGAC

GCAAATGGGCGGTAGGCGTGTACGGTGGGAGGTCTATATAAGCAGAGCTGGTTTAGTGAACCGTCAGATCTTCGC
GAGACCGGTACCTC

**Brique SiaT BsaI B (SEQ ID NO : 112)**

GAGGTACCGGTCTCCTTCGATGATTCACACCAACCTGAAGAAAAAGTTCAGCTGCTGCGTCCTGGTCTTTCTTCTGTTTGCAGTCATCTGTGTGTGGAAGGAAAAGAAGAAAGGGAGTTACTATGATTCCTTTAAATTGCAAACCAAGGAATTCCAGGTGTTAAAGAGTCTGGGGAAATTGGCCATGGGGTCTGATTCCCAGTCTGTATCCTCAAGCAGCACCCAGGACCCCCACAGGGGCCGCCAGACCCTCGGCAGTCTCAGAGGCCTAGCCAAGGCCAAACCAGAGGCCTCCTTCCAGGTGTGGAACAAGGACAGCTCTTCCAAAAACCTTATCCCTAGGCTGCAAAAGGGGTCGGGGGTGATGAGACCGGTACCTC

**Brique mCherry BsaI B (SEQ ID NO : 113)**

GAGGTACCGGTCTCAGTGAGCAAGGGCGAGGAGGATAACATGGCCATCATCAAGGAGTTCATGCGCTTCAAGGTGCACATGGAGGGCTCCGTGAACGGCCACGAGTTCGAGATCGAGGGCGAGGGCGAGGGCCGCCCCTACGAGGGCACCCAGACCGCCAAGCTGAAGGTGACCAAGGGTGGCCCCCTGCCCTTCGCCTGGGACATCCTGTCCCCTCAGTTCATGTACGGCTCCAAGGCCTACGTGAAGCACCCCGCCGACATCCCCGACTACTTGAAGCTGTCCTTCCCCGAGGGCTTCAAGTGGGAGCGCGTGATGAACTTCGAGGACGGCGGCGTGGTGACCGTGACCCAGGACTCCTCCCTGCAGGACGGCGAGTTCATCTACAAGGTGAAGCTGCGCGGCACCAACTTCCCCTCCGACGGCCCCGTAATGCAGAAGAAAACCATGGGCTGGGAGGCCTCCTCCGAGCGGATGTACCCCGAGGACGGCGCCCTGAAGGGCGAGATCAAGCAGAGGCTGAAGCTGAAGGACGGCGGCCACTACGACGCTGAGGTCAAGACCACCTACAAGGCCAAGAAGCCCGTGCAGCTGCCCGGCGCCTACAACGTCAACATCAAGTTGGACATCACCTCCCACAACGAGGACTACACCATCGTGGAACAGTACGAACGCGCCGAGGGCCGCCACTCCACCGGCGGCATGGACGAGCTGTACAAGTAACCGCTGAGACCGGTACCTC

**Brique TKter BsaI B (SEQ ID NO : 64)**
**Brique HygroR BsaI D (SEQ ID NO : 114)**

GAGGTACCGGTCTCAACAACAGGCAGAAGTATGCAAAGCATGCATCTCAATTAGTCAGCAACCAGGTGTGGAAAGTCCCCAGGCTCCCCAGCAGGCAGAAGTATGCAAAGCATGCATCTCAATTAGTCAGCAACCATAGTCCCGCCCCTAACTCCGCCCATCCCGCCCCTAACTCCGCCCAGTTCCGCCCATTCTCCGCCCCATGGCTGACTAATTTTTTTTATTTATGCAGAGGCCGAGGCCGCCTCTGCCTCTGAGCTATTCCAGAAGTAGTGAGGAGGCTTTTTTGGAGGCCTAGGCTTTTGCAAAAAGCTCCCGGGAGCTTGTATATCCATTTTCGGATCTGATCAGCACGTGATGAAAAAGCCTGAACTCACCGCGACGTCTGTCGAGAAGTTTCTGATCGAAAAGTTCGACAGCGTGTCCGACCTGATGCAGCTCTCGGAGGGCGAAGAAT

CTCGTGCTTTCAGCTTCGATGTAGGAGGGCGTGGATATGTCCTGCGGGTAAATAGCTGCGCCGATGGTTTCTACAA
AGATCGTTATGTTTATCGGCACTTTGCATCGGCCGCGCTCCCGATTCCGGAAGTGCTTGACATTGGGGAATTCAGC
GAGAGCCTGACCTATTGCATCTCCCGCCGTGCACAGGGTGTCACGTTGCAAGACTTGCCTGAAACCGAACTGCCCG
CTGTTCTGCAGCCGGTCGCGGAGGCCATGGATGCGATCGCTGCGGCCGATCTTAGCCAGACGAGCGGGTTCGGCC
CATTCGGACCGCAAGGAATCGGTCAATACACTACATGGCGTGATTTCATATGCGCGATTGCTGATCCCCATGTGTAT
CACTGGCAAACTGTGATGGACGACACCGTCAGTGCGTCCGTCGCGCAGGCTCTCGATGAGCTGATGCTTTGGGCC
GAGGACTGCCCCGAAGTCCGGCACCTCGTGCACGCGGATTTCGGCTCCAACAATGTCCTGACGGACAATGGCCGC
ATAACAGCGGTCATTGACTGGAGCGAGGCGATGTTCGGGGATTCCCAATACGAGGTCGCCAACATCTTCTTCTGGA
GGCCGTGGTTGGCTTGTATGGAGCAGCAGACGCGCTACTTCGAGCGGAGGCATCCGGAGCTTGCAGGATCGCCGC
GGCTCCGGGCGTATATGCTCCGCATTGGTCTTGACCAACTCTATCAGAGCTTGGTTGACGGCAATTTCGATGATGC
AGCTTGGGCGCAGGGTCGATGCGACGCAATCGTCCGATCCGGAGCCGGGACTGTCGGGCGTACACAAATCGCCC
GCAGAAGCGCGGCCGTCTGGACCGATGGCTGTGTAGAAGTACTCGCCGATAGTGGAAACCGACGCCCCAGCACTC
GTCCGAGGGCAAAGGAATAGCACGTGCTACGAGATTTCGATTCCACCGCCGCCTTCTATGAAAGGTTGGGCTTCG
GAATCGTTTTCCGGGACGCCGGCTGGATGATCCTCCAGCGCGGGGATCTCATGCTGGAGTTCTTCGCCCACCCCAA
CTTGTTTATTGCAGCTTATAATGGTTACAAATAAAGCAATAGCATCACAAATTTCACAAATAAAGCATTTTTTTCACT
GCATTCTAGTTGTGGTTTGTCCAAACTCATCAATGTATCATTACGAGACCGGTACCTC

**V1.1b (SEQ ID NO : 151, exemple de vecteur du groupe V1.1)**

CGGTAATACGGTTATCCACAGAATCAGGGGATAACGCAGGAAAGAACATGTGAGCAAAAGGCCAGCAAAAGGCC
AGGAACCGTAAAAAGGCCGCGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCCCCTGGAAGCTCCCTCGTGC
GCTCTCCTGTTCCGACCCTGCCGCTTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACACGACTTATCGCCACTGGCA
GCAGCCACTGGTAACAGGATTAGCAGAGCGAGGTATGTAGGCGGTGCTACAGAGTTCTTGAAGTGGTGGCCTAAC
TACGGCTACACTAGAAGAACAGTATTTGGTATCTGCGCTCTGCTGAAGCCAGTTACCTTCGGAAAAAGAGTTGGTA
GCTCTTGATCCGGCAAACAAACCACCGCTGGTAGCGGTTTTTTTGTTTGCAAGCAGCAGATTACGCGCAGAAAAAA
AGGATCTCAAGAAGATCCTTTGATCTTTTCTACGGGGTCTGACGCTCAGTGGAACGAAAACTCACGTTAAGGGATT
TTGGTCATGAGATTATCAAAAAGGATCTTCACCTAGATCCTTTTAAATTAAAAATGAAGTTTTAAATCAATCTAAAGT
ATATATGAGTAAACTTGGTCTGACAGTTACCAATGCTTAATCAGTGAGGCACCTATCTCAGCGATCTGTCTATTTCG
TTCATCCATAGTTGCCTGACTCCCCGTCGTGTAGATAACTACGATACGGGAGGGCTTACCATCTGGCCCCAGTGCTG
CAATGATACCGCGTGACCCACGCTCACCGGCTCCAGATTTATCAGCAATAAACCAGCCAGCCGGAAGGGCCGAGC

GCAGAAGTGGTCCTGCAACTTTATCCGCCTCCATCCAGTCTATTAATTGTTGCCGGGAAGCTAGAGTAAGTAGTTCG
CCAGTTAATAGTTTGCGCAACGTTGTTGCCATTGCTACAGGCATCGTGGTGTCACGCTCGTCGTTTGGTATGGCTTC
ATTCAGCTCCGGTTCCCAACGATCAAGGCGAGTTACATGATCCCCCATGTTGTGCAAAAAAGCGGTTAGCTCCTTCG
GTCCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATGCG
GCGACCGAGTTGCTCTTGCCCGGCGTCAATACGGGATAATACCGCGCCACATAGCAGAACTTTAAAAGTGCTCATC
ATTGGAAAACGTTCTTCGGGGCGAAAACTCTCAAGGATCTTACCGCTGTTGAGATCCAGTTCGATGTAACCCACTC
GTGCACCCAACTGATCTTCAGCATCTTTTACTTTCACCAGCGTTTCTGGGTGAGCAAAAACAGGAAGGCAAAATGC
CGCAAAAAAGGGAATAAGGGCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACAAATAGGGGTTCCGCGCAC
ATTTCCCCGAAAAGTGCCAATGCGTGAGGCTCCGGTGCCCGTCAGTGGGCAGAGCGCACATCGCCCACAGTCCCC
GAGAAGTTGGGGGGAGGGGTCGGCAATTGAACCGGTGCCTAGAGAAGGTGGCGCGGGGTAAACTGGGAAAGTG
ATGTCGTGTACTGGCTCCGCCTTTTTCCCGAGGGTGGGGGAGAACCGTATATAAGTGCAGTAGTCGCCGTGAACGT
TCTTTTTCGCAACGGGTTTGCCGCCAGAACACAGGTAAGTGCCGTGTGTGGTTCCCGCGGGCCTGGCCTCTTTACG
GGTTATGGCCCTTGCGTGCCTTGAATTACTTCCACCTGGCTGCAGTACGTGATTCTTGATCCCGAGCTTCGGGTTGG
AAGTGGGTGGGAGAGTTCGAGGCCTTGCGCTTAAGGAGCCCCTTCGCCTCGTGCTTGAGTTGAGGCCTGGCCTGG
GCGCTGGGGCCGCCGCGTGCGAATCTGGTGGCACCTTCGCGCCTGTCTCGCTGCTTTCGATAAGTCTCTAGCCATTT
AAAATTTTTGATGACCTGCTGCGACGCTTTTTTTCTGGCAAGATAGTCTTGTAAATGCGGGCCAAGATCTGCACACT
GGTATTTCGGTTTTTGGGGCCGCGGGCGGCGACGGGGCCCGTGCGTCCCAGCGCACATGTTCGGCGAGGCGGGG
CCTGCGAGCGCGGCCACCGAGAATCGGACGGGGGTAGTCTCAAGCTGGCCGGCCTGCTCTGGTGCCTGGCCTCGC
GCCGCCGTGTATCGCCCCGCCCTGGGCGGCAAGGCTGGCCCGGTCGGCACCAGTTGCGTGAGCGGAAAGATGGC
CGCTTCCCGGCCCTGCTGCAGGGAGCTCAAAATGGAGGACGCGGCGCTCGGGAGAGCGGGCGGGTGAGTCACCC
ACACAAAGGAAAAGGGCCTTTCCGTCCTCAGCCGTCGCTTCATGTGACTCCACGGAGTACCGGGCGCCGTCCAGGC
ACCTCGATTAGTTCTCGAGCTTTTGGAGTACGTCGTCTTTAGGTTGGGGGGAGGGGTTTTATGCGATGGAGTTTCC
CCACACTGAGTGGGTGGAGACTGAAGTTAGGCCAGCTTGGCACTTGATGTAATTCTCCTTGGAATTTGCCCTTTTTG
AGTTTGGATCTTGGTTCATTCTCAAGCCTCAGACAGTGGTTCAAAGTTTTTTTCTTCCATTTCAGGTGTCGTGATATC
ATGGTGAGCAAGGGCGAGGAGCTGTTCACCGGGGTGGTGCCCATCCTGGTCGAGCTGGACGGCGACGTAAACGG
CCACAAGTTCAGCGTGTCCGGCGAGGGCGAGGGCGATGCCACCTACGGCAAGCTGACCCTGAAGTTCATCTGCAC
CACCGGCAAGCTGCCCGTGCCCTGGCCCACCCTCGTGACCACCCTGACCTACGGCGTGCAGTGCTTCAGCCGCTAC
CCCGACCACATGAAGCAGCACGACTTCTTCAAGTCCGCCATGCCCGAAGGCTACGTCCAGGAGCGCACCATCTTCT
TCAAGGACGACGGCAACTACAAGACCCGCGCCGAGGTGAAGTTCGAGGGCGACACCCTGGTGAACCGCATCGAG
CTGAAGGGCATCGACTTCAAGGAGGACGGCAACATCCTGGGGCACAAGCTGGAGTACAACTACAACAGCCACAAC

GTCTATATCATGGCCGACAAGCAGAAGAACGGCATCAAGGTGAACTTCAAGATCCGCCACAACATCGAGGACGGC AGCGTGCAGCTCGCCGACCACTACCAGCAGAACACCCCCATCGGCGACGGCCCCGTGCTGCTGCCCGACAACCACT ACCTGAGCACCCAGTCCGCCCTGAGCAAAGACCCCAACGAGAAGCGCGATCACATGGTCCTGCTGGAGTTCGTGA CCGCCGCCGGGATCACTCTCGGCATGGACGAGCTGTACAAGAAGAAGAAAAAGAAGTCAAAGACAAAGTGTGTA ATTATGTAAGAGTCGACTGTGCCTTCTAGTTGCCAGCCATCTGTTGTTTGCCCCTCCCCCGTGCCTTCCTTGACCCTG GAAGGTGCCACTCCCACTGTCCTTTCCTAATAAAATGAGGAAATTGCATCGCATTGTCTGAGTAGGTGTCATTCTAT TCTGGGGGGTGGGGTGGGGCAGGACAGCAAGGGGGAGGATTGGGAGGACAATAGCAGGCATGCTGGGGATGC GGTGGGCTCTATGGGTAGTTATTAATAGTAATCAATTACGGGGTCATTAGTTCATAGCCCATATATGGAGTTCCGC GTTACATAACTTACGGTAAATGGCCCGCCTGGCTGACCGCCCAACGACCCCCGCCCATTGACGTCAATAATGACGT ATGTTCCCATAGTAACGCCAATAGGGACTTTCCATTGACGTCAATGGGTGGAGTATTTACGGTAAACTGCCCACTTG GCAGTACATCAAGTGTATCATATGCCAAGTACGCCCCCTATTGACGTCAATGACGGTAAATGGCCCGCCTGGCATT ATGCCCAGTACATGACCTTATGGGACTTTCCTACTTGGCAGTACATCTACGTATTAGTCATCGCTATTACCATGGTG ATGCGGTTTTGGCAGTACATCAATGGGCGTGGATAGCGGTTTGACTCACGGGGATTTCCAAGTCTCCACCCCATTG ACGTCAATGGGAGTTTGTTTTGGCACCAAAATCAACGGGACTTTCCAAAATGTCGTAACAACTCCGCCCCATTGAC GCAAATGGGCGGTAGGCGTGTACGGTGGGAGGTCTATATAAGCAGAGCTGGTTTAGTGAACCGTCAGATCTTCGA TGATTCACACCAACCTGAAGAAAAAGTTCAGCTGCTGCGTCCTGGTCTTTCTTCTGTTTGCAGTCATCTGTGTGTGG AAGGAAAAGAAGAAAGGGAGTTACTATGATTCCTTTAAATTGCAAACCAAGGAATTCCAGGTGTTAAAGAGTCTG GGGAAATTGGCCATGGGGTCTGATTCCCAGTCTGTATCCTCAAGCAGCACCCAGGACCCCCACAGGGGCCGCCAG ACCCTCGGCAGTCTCAGAGGCCTAGCCAAGGCCAAACCAGAGGCCTCCTTCCAGGTGTGGAACAAGGACAGCTCT TCCAAAAACCTTATCCCTAGGCTGCAAAAGGGGTCGGGGGTGAGCAAGGGCGAGGAGGATAACATGGCCATCAT CAAGGAGTTCATGCGCTTCAAGGTGCACATGGAGGGCTCCGTGAACGGCCACGAGTTCGAGATCGAGGGCGAGG GCGAGGGCCGCCCCTACGAGGGCACCCAGACCGCCAAGCTGAAGGTGACCAAGGGTGGCCCCCTGCCCTTCGCCT GGGACATCCTGTCCCCTCAGTTCATGTACGGCTCCAAGGCCTACGTGAAGCACCCCGCCGACATCCCCGACTACTTG AAGCTGTCCTTCCCCGAGGGCTTCAAGTGGGAGCGCGTGATGAACTTCGAGGACGGCGGCGTGGTGACCGTGACC CAGGACTCCTCCCTGCAGGACGGCGAGTTCATCTACAAGGTGAAGCTGCGCGGCACCAACTTCCCCTCCGACGGCC CCGTAATGCAGAAGAAAACCATGGGCTGGGAGGCCTCCTCCGAGCGGATGTACCCCGAGGACGGCGCCCTGAAG GGCGAGATCAAGCAGAGGCTGAAGCTGAAGGACGGCGGCCACTACGACGCTGAGGTCAAGACCACCTACAAGGC CAAGAAGCCCGTGCAGCTGCCCGGCGCCTACAACGTCAACATCAAGTTGGACATCACCTCCCACAACGAGGACTAC ACCATCGTGGAACAGTACGAACGCGCCGAGGGCCGCCACTCCACCGGCGGCATGGACGAGCTGTACAAGTAACCG CGGGGGAGGCTAACTGAAACACGGAAGGAGACAATACCGGAAGGAACCCGCGCTATGACGGCAATAAAAAGACA GAATAAAACGCACGGTGTTGGGTCGTTTGTTCATAAACGCGGGGTTCGGTCCCAGGGCTGGCACTCTGTCGATACC CCACCGAGGCCCCATTGGGGCCAATACGCCCGCGTTTCTTCCTTTTCCCCACCCCACCCCCCAAGTTCGGGTGAAGG

CCCAGGGCTCGCAGCCAACGTCGGGGCGGCAGGCCCTGCCATAGACAACAGGCAGAAGTATGCAAAGCATGCAT
CTCAATTAGTCAGCAACCAGGTGTGGAAAGTCCCCAGGCTCCCCAGCAGGCAGAAGTATGCAAAGCATGCATCTCA
ATTAGTCAGCAACCATAGTCCCGCCCCTAACTCCGCCCATCCCGCCCCTAACTCCGCCCAGTTCCGCCCATTCTCCGC
CCCATGGCTGACTAATTTTTTTTATTTATGCAGAGGCCGAGGCCGCCTCTGCCTCTGAGCTATTCCAGAAGTAGTGA
GGAGGCTTTTTTGGAGGCCTAGGCTTTTGCAAAAAGCTCCCGGGAGCTTGTATATCCATTTTCGGATCTGATCAGCA
CGTGATGAAAAAGCCTGAACTCACCGCGACGTCTGTCGAGAAGTTTCTGATCGAAAAGTTCGACAGCGTGTCCGAC
CTGATGCAGCTCTCGGAGGGCGAAGAATCTCGTGCTTTCAGCTTCGATGTAGGAGGGCGTGGATATGTCCTGCGG
GTAAATAGCTGCGCCGATGGTTTCTACAAAGATCGTTATGTTTATCGGCACTTTGCATCGGCCGCGCTCCCGATTCC
GGAAGTGCTTGACATTGGGGAATTCAGCGAGAGCCTGACCTATTGCATCTCCCGCCGTGCACAGGGTGTCACGTTG
CAAGACTTGCCTGAAACCGAACTGCCCGCTGTTCTGCAGCCGGTCGCGGAGGCCATGGATGCGATCGCTGCGGCC
GATCTTAGCCAGACGAGCGGGTTCGGCCCATTCGGACCGCAAGGAATCGGTCAATACACTACATGGCGTGATTTCA
TATGCGCGATTGCTGATCCCCATGTGTATCACTGGCAAACTGTGATGGACGACACCGTCAGTGCGTCCGTCGCGCA
GGCTCTCGATGAGCTGATGCTTTGGGCCGAGGACTGCCCCGAAGTCCGGCACCTCGTGCACGCGGATTTCGGCTCC
AACAATGTCCTGACGGACAATGGCCGCATAACAGCGGTCATTGACTGGAGCGAGGCGATGTTCGGGGATTCCCAA
TACGAGGTCGCCAACATCTTCTTCTGGAGGCCGTGGTTGGCTTGTATGGAGCAGCAGACGCGCTACTTCGAGCGG
AGGCATCCGGAGCTTGCAGGATCGCCGCGGCTCCGGGCGTATATGCTCCGCATTGGTCTTGACCAACTCTATCAGA
GCTTGGTTGACGGCAATTTCGATGATGCAGCTTGGGCGCAGGGTCGATGCGACGCAATCGTCCGATCCGGAGCCG
GGACTGTCGGGCGTACACAAATCGCCCGCAGAAGCGCGGCCGTCTGGACCGATGGCTGTGTAGAAGTACTCGCCG
ATAGTGGAAACCGACGCCCCAGCACTCGTCCGAGGGCAAAGGAATAGCACGTGCTACGAGATTTCGATTCCACCG
CCGCCTTCTATGAAAGGTTGGGCTTCGGAATCGTTTTCCGGGACGCCGGCTGGATGATCCTCCAGCGCGGGGATCT
CATGCTGGAGTTCTTCGCCCACCCCAACTTGTTTATTGCAGCTTATAATGGTTACAAATAAAGCAATAGCATCACAA
ATTTCACAAATAAAGCATTTTTTTCACTGCATTCTAGTTGTGGTTTGTCCAAACTCATCAATGTATCATTA

L'Empreinte de restriction par triple digestion (validation structurelle), EcoRV, PstI, ScaI, de la réalisation 10 est illustrée figure 25.

Vérification fonctionnelle de la réalisation 10 : Des cellules eucaryotes (ex : cellules cancéreuses mammaires de souris 4T1) sont transfectées avec le vecteur V1.1b selon un protocole usuel (exemple lipotransfection ou électroporation). Vingt-quatre heures après transfection, les cellules sont observées sous microscope optique à lumiède blanche et fluorescence. (Figure 26)

Les cellules visibles en fluorescence GFP montrent un marquage dessinant les contours membranaires de chaque cellule. Les cellules visibles en fluorescence mCherry montrent un marquage ponctiforme, correspondant à l'appareil de golgi. Lorsque l'on supperpose les marquages GFP et mCherry, toutes les cellules visibles expriment les deux marquages simultanément, les cellules non fluorescentes étant celles qui n'ont pas reçu de vecteur suite à l'électroporation. Ceci démontre bien la co-expression de deux marqueurs fluorescents correctement exprimés dans des compartiments cellulaires distincts suite à l'introduction d'un vecteur unique dans les cellules.

## Réalisation 11

**Groupe de vecteurs V4** : **Vecteurs permettant de sélectionner des cellules dont le génome a été édité par recombinaison homologue ciblée.**

U1 + U3b + U3a + U3c

U1 : Unité fonctionnelle bactérienne
U3a = cassette de sélection positive
U3b = motif 5' d'une séquence de recombinaison homologue X
U3c = motif 3' de la séquence de recombinaison homologue X

Exemple : vecteur permettant de sélectionner une souche de levure *S. cerevisiae* dont le gène MNN10 a été délété

| | |
|---|---|
| U1 | Ori<br>AmpR |
| U3b | MNN10-left |
| U3a | Kanamycin resistance KanMX4 |
| U3c | MNN10-right |

Liste des briques utilisées pour la construction du vecteur V4 (Figure 27)

**Brique Ori-2 Bsal C (SEQ ID NO : 115)**

GAGGTACCGGTCTCTGGGGCGGTAATACGGTTATCCACAGAATCAGGGGATAACGCAGGAAAGAACATGTGAGC
AAAAGGCCAGCAAAAGGCCAGGAACCGTAAAAAGGCCGCGTTGCTGGCGTTTTTCCATAGGCTCCGCCCCCCTGA
CGAGCATCACAAAAATCGACGCTCAAGTCAGAGGTGGCGAAACCCGACAGGACTATAAAGATACCAGGCGTTTCC
CCCTGGAAGCTCCCTCGTGCGCTCTCCTGTTCCGACCCTGCCGCTTACCGGATACCTGTCCGCCTTTCTCCCTTCGGG

AAGCGTGGCGCTTTCTCATAGCTCACGCTGTAGGTATCTCAGTTCGGTGTAGGTCGTTCGCTCCAAGCTGGGCTGT
GTGCACGAACCCCCCGTTCAGCCCGACCGCTGCGCCTTATCCGGTAACTATCGTCTTGAGTCCAACCCGGTAAGAC
ACGACTTATCGCCACTGGCAGCAGCCACTGGTAACAGGATTAGCAGAGCGAGGTATGTAGGCGGTGCTACAGAGT
TCTTGAAGTGGTGGCCTAACTACGGCTACACTAGAAGAACAGTATTTGGTATCTGCGCTCTGCTGAAGCCAGTTAC
CTTCGGAAAAAGAGTTGGTAGCTCTTGATCCGGCAAACAAACCACCGCTGGTAGCGGTTTTTTTGTTTGCAAGCAG
CAGATTACGCGCAGAAAAAAAGGATCTCAAGAAGATCCTTTGATCTTTTCTACGGGGTCTGACGCTCAGTGGAACG
AAAACTCACGTTAAGGGATTTTGGTCATGAGATTATCAAAAAGGATCTTCACCTAGATCCTTTTAAATTAAAAATGA
AGTTTTAAATCAATCTAAAGTATATATGAGTTTAAACTGAGACCGGTACCTC

**Brique AmpR Bsal C (SEQ ID NO : 116)**

GAGGTACCGGTCTCAAAACTTGGTCTGACAGTTACCAATGCTTAATCAGTGAGGCACCTATCTCAGCGATCTGTCTA
TTTCGTTCATCCATAGTTGCCTGACTCCCCGTCGTGTAGATAACTACGATACGGGAGGGCTTACCATCTGGCCCCAG
TGCTGCAATGATACCGCGTGACCCACGCTCACCGGCTCCAGATTTATCAGCAATAAACCAGCCAGCCGGAAGGGCC
GAGCGCAGAAGTGGTCCTGCAACTTTATCCGCCTCCATCCAGTCTATTAATTGTTGCCGGGAAGCTAGAGTAAGTA
GTTCGCCAGTTAATAGTTTGCGCAACGTTGTTGCCATTGCTACAGGCATCGTGGTGTCACGCTCGTCGTTTGGTATG
GCTTCATTCAGCTCCGGTTCCCAACGATCAAGGCGAGTTACATGATCCCCCATGTTGTGCAAAAAAGCGGTTAGCTC
CTTCGGTCCTCCGATCGTTGTCAGAAGTAAGTTGGCCGCAGTGTTATCACTCATGGTTATGGCAGCACTGCATAATT
CTCTTACTGTCATGCCATCCGTAAGATGCTTTTCTGTGACTGGTGAGTACTCAACCAAGTCATTCTGAGAATAGTGT
ATGCGGCGACCGAGTTGCTCTTGCCCGGCGTCAATACGGGATAATACCGCGCCACATAGCAGAACTTTAAAAGTGC
TCATCATTGGAAAACGTTCTTCGGGGCGAAAACTCTCAAGGATCTTACCGCTGTTGAGATCCAGTTCGATGTAACCC
ACTCGTGCACCCAACTGATCTTCAGCATCTTTTACTTTCACCAGCGTTTCTGGGTGAGCAAAAACAGGAAGGCAAAA
TGCCGCAAAAAAGGGAATAAGGGCGACACGGAAATGTTGAATACTCATACTCTTCCTTTTTCAATATTATTGAAGC
ATTTATCAGGGTTATTGTCTCATGAGCGGATACATATTTGAATGTATTTAGAAAAATAAACAAATAGGGGTTCCGC
GCACATTTCCCCGAAAAGTGCCAGTGATGAGACCGGTACCTC

**Brique MNN10-Lrec BsaI A (SEQ ID NO : 117)**

GAGGTACCGGTCTCTGTGAGTTTAAACATGCATTCAAAGGTCATAATTGCTGCTCTATTTACAGTCGTCCATAATGA
CATTTCTCTTTGATTATTTTCTTGTTTTTTCGCTCTTCTCAAGTGGATGTTACATAACAAACAAAACAGAAAAAATTGT
TTAAATATAAAGTTTAAAAGTTATCTTTGATTCCGCACCTGAATTTTTGGATTGAAGGCCAAAGGAGGTTTATCAGG
GAGAGAAAAGCTCTCTATTTATTTTTATAAGGAATAATTGTGCATGTACAACTATACAATTGCGTGAGACCGGTACC
TC

**Brique KanMX BsaI A (SEQ ID NO : 119)**

GAGGTACCGGTCTCGTGCGGTACGCTGCAGGTCGACAACCCTTAATATAACTTCGTATAATGTATGCTATACGAAGTTATTAGGTCTAGAGATCTGTTTAGCTTGCCTCGTCCCCGCCGGGTCACCCGGCCAGCGACATGGAGGCCCAGAATACCCTCCTTGACAGTCTTGACGTGCGCAGCTCAGGGGCATGATGTGACTGTCGCCCGTACATTTAGCCCATACATCCCCATGTATAATCATTTGCATCCATACATTTTGATGGCCGCACGGCGCGAAGCAAAAATTACGGCTCCTCGCTGCAGACCTGCGAGCAGGGAAACGCTCCCCTCACAGACGCGTTGAATTGTCCCCACGCCGCGCCCCTGTAGAGAAATATAAAAGGTTAGGATTTGCCACTGAGGTTCTTCTTTCATATACTTCCTTTTAAAATCTTGCTAGGATACAGTTCTCACATCACATCCGAACATAAACAACCATGGGTAAGGAAAAGACTCACGTTTCGAGGCCGCGATTAAATTCCAACATGGATGCTGATTTATATGGGTATAAATGGGCTCGCGATAATGTCGGGCAATCAGGTGCGACAATCTATCGATTGTATGGGAAGCCCGATGCGCCAGAGTTGTTTCTGAAACATGGCAAAGGTAGCGTTGCCAATGATGTTACAGATGAGATGGTCAGACTAAACTGGCTGACGGAATTTATGCCTCTTCCGACCATCAAGCATTTTATCCGTACTCCTGATGATGCATGGTTACTCACCACTGCGATCCCCGGCAAAACAGCATTCCAGGTATTAGAAGAATATCCTGATTCAGGTGAAAATATTGTTGATGCGCTGGCAGTGTTCCTGCGCCGGTTGCATTCGATTCCTGTTTGTAATTGTCCTTTTAACAGCGATCGCGTATTTCGTCTCGCTCAGGCGCAATCACGAATGAATAACGGTTTGGTTGATGCGAGTGATTTTGATGACGAGCGTAATGGCTGGCCTGTTGAACAAGTCTGGAAAGAAATGCATAAGCTTTTGCCATTCTCACCGGATTCAGTCGTCACTCATGGTGATTTCTCACTTGATAACCTTATTTTTGACGAGGGGAAATTAATAGGTTGTATTGATGTTGGACGAGTCGGAATCGCAGACCGATACCAGGATCTTGCCATCCTATGGAACTGCCTCGGTGAGTTTTCTCCTTCATTACAGAAACGGCTTTTTCAAAAATATGGTATTGATAATCCTGATATGAATAAATTGCAGTTTCATTTGATGCTCGATGAGTTTTTCTAATCAGTACTGACAATAAAAAGATTCTTGTTTTCAAGAACTTGTCATTTGTATAGTTTTTTTATATTGTAGTTGTTCTATTTTAATCAAATGTTAGCGTGATTTATATTTTTTTTCGCCTCGACATCATCTGCCCAGATGCGAAGTTAAGTGCGCAGAAAGTAATATCATGCGTCAATCGTATGTGAATGCTGGTCGCTATACTGCTGTCGATTCGATACTAACGCCGCCATCCAGTGTCGAAAACGAGCTCTCGAGAACCCTTAATATAACTTCGTATAATGTATGCTATACGAAGTTATTAGGTGATATCAGATCCACTAGTGTCGTAGAGACCGGTACCTC

**Brique MNN10-Rrec BsaI A (SEQ ID NO : 118)**

GAGGTACCGGTCTCTTCGTAATGGAAGTTATCAATATTGTAAAGAGAAGCATTTACAAGCTTTTATTTTTCTTTTTAATTTCCACTACTGGTTCTGCTTTAAAATGTTGTTTTATAATTTATGTACATTTAGGCCTATAGAAGATTCTTTCAATAATATGCTACACATTCTTTTATTTTTCCATCATATGTTGGAGTTTATGCCTCCTCGGCAGGAGTTGGGCGGTGCGAAGAGAAGAAAAAGAGTGAAACTAAAAAAAGGAATCTGCCTTTGCATAAGTTCAAAAGTGCAATTTTAGTGTTGGATTTAAACGGGAAAAATTGAAATGGCCATCGAAACAATACTTGTAATAAACAAATCAGGCGGACTAATCTATCAGCGGAATTTTACCAACGACGAACAGAAATTGAACAGCAATGAATACTTAATTCTTGCTAGTACACTGCACGGTGTATTCGCCATCGCGAGCCAGCTGACTCCGAAGGCATTACAGCTAACTCAACAAACGAACATCGAAAATACCATCCCATATATACCTT

ACGTGGGCATGTCCAGCAATAGGAGCGATACAAGAAATGGAGGTGGCAATAACAACAAACACACTAATAATGAAAAACTGGGCAGTTTAAACGGGGAGAGACCGGTACCTC

**Vecteur V4 (SEQ ID NO : 152, exemple de vecteur du groupe V4)**

CGGTAATACGGTTATCCACAGAATCAGGGGATAACGCAGGAAAGAACATGTGAGCAAAAGGCCAGCAAAAGGCC
AGGAACCGTAAAAAGGCCGCGTTGCTGGCGTTTTTCCATAGGCTCCGCCCCCCTGACGAGCATCACAAAAATCGAC
GCTCAAGTCAGAGGTGGCGAAACCCGACAGGACTATAAAGATACCAGGCGTTTCCCCCTGGAAGCTCCCTCGTGC
GCTCTCCTGTTCCGACCCTGCCGCTTACCGGATACCTGTCCGCCTTTCTCCCTTCGGGAAGCGTGGCGCTTTCTCATA
GCTCACGCTGTAGGTATCTCAGTTCGGTGTAGGTCGTTCGCTCCAAGCTGGGCTGTGTGCACGAACCCCCCGTTCA
GCCCGACCGCTGCGCCTTATCCGGTAACTATCGTCTTGAGTCCAACCCGGTAAGACACGACTTATCGCCACTGGCA
GCAGCCACTGGTAACAGGATTAGCAGAGCGAGGTATGTAGGCGGTGCTACAGAGTTCTTGAAGTGGTGGCCTAAC
TACGGCTACACTAGAAGAACAGTATTTGGTATCTGCGCTCTGCTGAAGCCAGTTACCTTCGGAAAAAGAGTTGGTA
GCTCTTGATCCGGCAAACAAACCACCGCTGGTAGCGGTTTTTTTGTTTGCAAGCAGCAGATTACGCGCAGAAAAAA
AGGATCTCAAGAAGATCCTTTGATCTTTTCTACGGGGTCTGACGCTCAGTGGAACGAAAACTCACGTTAAGGGATT
TTGGTCATGAGATTATCAAAAAGGATCTTCACCTAGATCCTTTTAAATTAAAAATGAAGTTTTAAATCAATCTAAAGT
ATATATGAGTAAACTTGGTCTGACAGTTACCAATGCTTAATCAGTGAGGCACCTATCTCAGCGATCTGTCTATTT
CGTTCATCCATAGTTGCCTGACTCCCCGTCGTGTAGATAACTACGATACGGGAGGGCTTACCATCTGGCCCCAGTGC
TGCAATGATACCGCGTGACCCACGCTCACCGGCTCCAGATTTATCAGCAATAAACCAGCCAGCCGGAAGGGCCGA
GCGCAGAAGTGGTCCTGCAACTTTATCCGCCTCCATCCAGTCTATTAATTGTTGCCGGGAAGCTAGAGTAAGTAGTT
CGCCAGTTAATAGTTTGCGCAACGTTGTTGCCATTGCTACAGGCATCGTGGTGTCACGCTCGTCGTTTGGTATGGCT
TCATTCAGCTCCGGTTCCCAACGATCAAGGCGAGTTACATGATCCCCCATGTTGTGCAAAAAAGCGGTTAGCTCCTT
CGGTCCTCCGATCGTTGTCAGAAGTAAGTTGGCCGCAGTGTTATCACTCATGGTTATGGCAGCACTGCATAATTCTC
TTACTGTCATGCCATCCGTAAGATGCTTTTCTGTGACTGGTGAGTACTCAACCAAGTCATTCTGAGAATAGTGTATG
CGGCGACCGAGTTGCTCTTGCCCGGCGTCAATACGGGATAATACCGCGCCACATAGCAGAACTTTAAAAGTGCTCA
TCATTGGAAAACGTTCTTCGGGGCGAAAACTCTCAAGGATCTTACCGCTGTTGAGATCCAGTTCGATGTAACCCACT
CGTGCACCCAACTGATCTTCAGCATCTTTTACTTTCACCAGCGTTTCTGGGTGAGCAAAAACAGGAAGGCAAAATG
CCGCAAAAAAGGGAATAAGGGCGACACGGAAATGTTGAATACTCATACTCTTCCTTTTTCAATATTATTGAAGCATT
TATCAGGGTTATTGTCTCATGAGCGGATACATATTTGAATGTATTTAGAAAAATAAACAAATAGGGGTTCCGCGCA
CATTTCCCCGAAAAGTGCCAGTGAGTTTAAACATGCATTCAAAGGTCATAATTGCTGCTCTATTTACAGTCGTCCAT
AATGACATTTCTCTTTGATTATTTTCTTGTTTTTCGCTCTTCTCAAGTGGATGTTACATAACAAACAAAACAGAAAA
AATTGTTTAAATATAAAGTTTAAAAGTTATCTTTGATTCCGCACCTGAATTTTTGGATTGAAGGCCAAAGGAGGTTT
ATCAGGGAGAGAAAAGCTCTCTATTTATTTTTATAAGGAATAATTGTGCATGTACAACTATACAATTGCGGTACGCT

GCAGGTCGACAACCCTTAATATAACTTCGTATAATGTATGCTATACGAAGTTATTAGGTCTAGAGATCTGTTTAGCT
TGCCTCGTCCCCGCCGGGTCACCCGGCCAGCGACATGGAGGCCCAGAATACCCTCCTTGACAGTCTTGACGTGCGC
AGCTCAGGGGCATGATGTGACTGTCGCCCGTACATTTAGCCCATACATCCCCATGTATAATCATTTGCATCCATACA
TTTTGATGGCCGCACGGCGCGAAGCAAAAATTACGGCTCCTCGCTGCAGACCTGCGAGCAGGGAAACGCTCCCCT
CACAGACGCGTTGAATTGTCCCCACGCCGCGCCCCTGTAGAGAAATATAAAAGGTTAGGATTTGCCACTGAGGTTC
TTCTTTCATATACTTCCTTTTAAAATCTTGCTAGGATACAGTTCTCACATCACATCCGAACATAAACAACCATGGGTA
AGGAAAAGACTCACGTTTCGAGGCCGCGATTAAATTCCAACATGGATGCTGATTTATATGGGTATAAATGGGCTCG
CGATAATGTCGGGCAATCAGGTGCGACAATCTATCGATTGTATGGGAAGCCCGATGCGCCAGAGTTGTTTCTGAAA
CATGGCAAAGGTAGCGTTGCCAATGATGTTACAGATGAGATGGTCAGACTAAACTGGCTGACGGAATTTATGCCTC
TTCCGACCATCAAGCATTTTATCCGTACTCCTGATGATGCATGGTTACTCACCACTGCGATCCCCGGCAAAACAGCA
TTCCAGGTATTAGAAGAATATCCTGATTCAGGTGAAAATATTGTTGATGCGCTGGCAGTGTTCCTGCGCCGGTTGC
ATTCGATTCCTGTTTGTAATTGTCCTTTTAACAGCGATCGCGTATTTCGTCTCGCTCAGGCGCAATCACGAATGAATA
ACGGTTTGGTTGATGCGAGTGATTTTGATGACGAGCGTAATGGCTGGCCTGTTGAACAAGTCTGGAAAGAAATGC
ATAAGCTTTTGCCATTCTCACCGGATTCAGTCGTCACTCATGGTGATTTCTCACTTGATAACCTTATTTTTGACGAGG
GGAAATTAATAGGTTGTATTGATGTTGGACGAGTCGGAATCGCAGACCGATACCAGGATCTTGCCATCCTATGGAA
CTGCCTCGGTGAGTTTTCTCCTTCATTACAGAAACGGCTTTTTCAAAAATATGGTATTGATAATCCTGATATGAATAA
ATTGCAGTTTCATTTGATGCTCGATGAGTTTTTCTAATCAGTACTGACAATAAAAAGATTCTTGTTTTCAAGAACTTG
TCATTTGTATAGTTTTTTTATATTGTAGTTGTTCTATTTTAATCAAATGTTAGCGTGATTTATATTTTTTTTCGCCTCGA
CATCATCTGCCCAGATGCGAAGTTAAGTGCGCAGAAAGTAATATCATGCGTCAATCGTATGTGAATGCTGGTCGCT
ATACTGCTGTCGATTCGATACTAACGCCGCCATCCAGTGTCGAAAACGAGCTCTCGAGAACCCTTAATATAACTTCG
TATAATGTATGCTATACGAAGTTATTAGGTGATATCAGATCCACTAGTGTCGTAATGGAAGTTATCAATATTGTAAA
GAGAAGCATTTACAAGCTTTTATTTTTCTTTTTAATTTCCACTACTGGTTCTGCTTTAAAATGTTGTTTTATAATTTATG
TACATTTAGGCCTATAGAAGATTCTTTCAATAATATGCTACACATTCTTTTATTTTTCCATCATATGTTGGAGTTTATG
CCTCCTCGGCAGGAGTTGGGCGGTGCGAAGAGAAGAAAAAGAGTGAAACTAAAAAAAGGAATCTGCCTTTGCAT
AAGTTCAAAAGTGCAATTTTAGTGTTGGATTTAAACGGGAAAAATTGAAATGGCCATCGAAACAATACTTGTAATA
AACAAATCAGGCGGACTAATCTATCAGCGGAATTTTACCAACGACGAACAGAAATTGAACAGCAATGAATACTTAA
TTCTTGCTAGTACACTGCACGGTGTATTCGCCATCGCGAGCCAGCTGACTCCGAAGGCATTACAGCTAACTCAACAA
ACGAACATCGAAAATACCATCCCATATATACCTTACGTGGGCATGTCCAGCAATAGGAGCGATACAAGAAATGGA
GGTGGCAATAACAACAAACACACTAATAATGAAAAACTGGGCAGTTTAAACGGGG

L'Empreinte de restriction par double digestion (validation structurelle), HindIII et PmeI de la réalisation 11 est illustrée Figure 28.

Vérification fonctionnelle de la réalisation 11 : Le vecteur V4 a été utilisé pour inactiver le gène MNN10 de la levure par recombinaison homologue. Pour cela la cassette de délétion du vecteur a été libérée par digestion avec l'enzyme PmeI et transformée dans une souche de levure BY4741. Des colonies obtenues après 72h de croissance sur milieu sélectif contenant du G418 ont été repiquées et l'invalidation du gène MNN10 a été vérifiée par PCR. Afin de valider la fonctionnalité de la construction, le profil de migration sur gel natif de l'invertase du mutant MNN10 ainsi obtenu a été analysé et comparé à celui d'une souche sauvage et à celui d'une souche mutante pmr1, qui présente un sévère défaut de glycosylation.

Comme le montre la figure 29, le profil de migration de l'invertase du mutant MNN10 (piste C) est nettement différent du profil de migration de l'invertase de la souche sauvage (piste B) révélant un défaut de glycosylation de l'enzyme chez le mutant MNN10. Ce défaut n'est pas aussi sévère que celui observé pour le mutant pmr1 (piste A), conformément à ce qui est connu du rôle des produits de ces gènes dans la N-glycosylation.

Séquences des Matrices utilisées pour la fabrication des différentes briques.

**Matrice eZ-Ori-AmpR (SEQ ID NO : 153)**

GCGTCTTCTAGGGTTAAGGTTAGTGTAGAGAAGCAACCGAAGATTGAGAAGACATGGCGGTAATACGGTTATCCA
CAGAATCAGGGGATAACGCAGGAAAGAACATGTGAGCAAAAGGCCAGCAAAAGGCCAGGAACCGTAAAAAGGC
CGCGTTGCTGGCGTTTTTCCATAGGCTCCGCCCCCCTGACGAGCATCACAAAAATCGACGCTCAAGTCAGAGGTGG
CGAAACCCGACAGGACTATAAAGATACCAGGCGTTTCCCCCTGGAAGCTCCCTCGTGCGCTCTCCTGTTCCGACCCT
GCCGCTTACCGGATACCTGTCCGCCTTTCTCCCTTCGGGAAGCGTGGCGCTTTCTCATAGCTCACGCTGTAGGTATC
TCAGTTCGGTGTAGGTCGTTCGCTCCAAGCTGGGCTGTGTGCACGAACCCCCCGTTCAGCCCGACCGCTGCGCCTT
ATCCGGTAACTATCGTCTTGAGTCCAACCCGGTAAGACACGACTTATCGCCACTGGCAGCAGCCACTGGTAACAGG
ATTAGCAGAGCGAGGTATGTAGGCGGTGCTACAGAGTTCTTGAAGTGGTGGCCTAACTACGGCTACACTAGAAGA
ACAGTATTTGGTATCTGCGCTCTGCTGAAGCCAGTTACCTTCGGAAAAAGAGTTGGTAGCTCTTGATCCGGCAAAC
AAACCACCGCTGGTAGCGGTTTTTTTGTTTGCAAGCAGCAGATTACGCGCAGAAAAAAAGGATCTCAAGAAGATCC
TTTGATCTTTTCTACGGGGTCTGACGCTCAGTGGAACGAAAACTCACGTTAAGGGATTTTGGTCATGAGATTATCAA
AAAGGATCTTCACCTAGATCCTTTTAAATTAAAAATGAAGTTTTAAATCAATCTAAAGTATATATGAGTAAACTTGG
TCTGACAGTTACCAATGCTTAATCAGTGAGGCACCTATCTCAGCGATCTGTCTATTTCGTTCATCCATAGTTGCCTGA
CTCCCCGTCGTGTAGATAACTACGATACGGGAGGGCTTACCATCTGGCCCCAGTGCTGCAATGATACCGCGTGACC
CACGCTCACCGGCTCCAGATTTATCAGCAATAAACCAGCCAGCCGGAAGGGCCGAGCGCAGAAGTGGTCCTGCAA

CTTTATCCGCCTCCATCCAGTCTATTAATTGTTGCCGGGAAGCTAGAGTAAGTAGTTCGCCAGTTAATAGTTTGCGC
AACGTTGTTGCCATTGCTACAGGCATCGTGGTGTCACGCTCGTCGTTTGGTATGGCTTCATTCAGCTCCGGTTCCCA
ACGATCAAGGCGAGTTACATGATCCCCCATGTTGTGCAAAAAAGCGGTTAGCTCCTTCGGTCCTCCGATCGTTGTCA
GAAGTAAGTTGGCCGCAGTGTTATCACTCATGGTTATGGCAGCACTGCATAATTCTCTTACTGTCATGCCATCCGTA
AGATGCTTTTCTGTGACTGGTGAGTACTCAACCAAGTCATTCTGAGAATAGTGTATGCGGCGACCGAGTTGCTCTT
GCCCGGCGTCAATACGGGATAATACCGCGCCACATAGCAGAACTTTAAAAGTGCTCATCATTGGAAAACGTTCTTC
GGGGCGAAAACTCTCAAGGATCTTACCGCTGTTGAGATCCAGTTCGATGTAACCCACTCGTGCACCCAACTGATCTT
CAGCATCTTTTACTTTCACCAGCGTTTCTGGGTGAGCAAAAACAGGAAGGCAAAATGCCGCAAAAAAGGGAATAA
GGGCGACACGGAAATGTTGAATACTCATACTCTTCCTTTTTCAATATTATTGAAGCATTTATCAGGGTTATTGTCTCA
TGAGCGGATACATATTTGAATGTATTTAGAAAAATAAACAAATAGGGGTTCCGCGCACATTTCCCCGAAAAGTGCC
ACCTATGAGACGTGAGGCTAGGGATAGGACGAGAGCATCGGGAACGAGGACTAGCGTCTCA

**Matrice BGH polyA (SEQ ID NO : 154):**

TCGACTGTGCCTTCTAGTTGCCAGCCATCTGTTGTTTGCCCCTCCCCCGTGCCTTCCTTGACCCTGGAAGGTGCCACT
CCCACTGTCCTTTCCTAATAAAATGAGGAAATTGCATCGCATTGTCTGAGTAGGTGTCATTCTATTCTGGGGGGTGG
GGTGGGGCAGGACAGCAAGGGGGAGGATTGGGAGGACAATAGCAGGCATGCTGGGGATGCGGTGGGCTCTATG
GCTTCTG

**Matrice eZ-E1GFP (SEQ ID NO : 155)**

ATGGTGAGCAAGGGCGAGGAGCTGTTCACCGGGGTGGTGCCCATCCTGGTCGAGCTGGACGGCGACGTAAACGG
CCACAAGTTCAGCGTGTCCGGCGAGGGCGAGGGCGATGCCACCTACGGCAAGCTGACCCTGAAGTTCATCTGCAC
CACCGGCAAGCTGCCCGTGCCCTGGCCCACCCTCGTGACCACCCTGTCCTACGGCGTGCAGTGCTTCAGCCGCTAC
CCCGACCACATGAAGCAGCACGACTTCTTCAAGTCCGCCATGCCCGAAGGCTACGTCCAGGAGCGCACCATCTTCT
TCAAGGACGACGGCAACTACAAGACCCGCGCCGAGGTGAAGTTCGAGGGCGACACCCTGGTGAACCGCATCGAG
CTGAAGGGCATCGACTTCAAGGAGGACGGCAACATCCTGGGGCACAAGCTGGAGTACAACTACAACAGCCACAAC
GTCTATATCATGGCCGACAAGCAGAAGAACGGCATCAAGGTGAACTTCAAGATCCGCCACAACATCGAGGACGGC
AGCGTGCAGCTCGCCGACCACTACCAGCAGAACACCCCCATCGGCGACGGCCCCGTGCTGCTGCCCGACAACCACT
ACCTGAGCTACCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTACAAGTAA

**Matrice EGFP (SEQ ID NO : 156)**

ATGGTGAGCAAGGGCGAGGAGCTGTTCACCGGGGTGGTGCCCATCCTGGTCGAGCTGGACGGCGACGTAAACGG
CCACAAGTTCAGCGTGTCCGGCGAGGGCGAGGGCGATGCCACCTACGGCAAGCTGACCCTGAAGTTCATCTGCAC
CACCGGCAAGCTGCCCGTGCCCTGGCCCACCCTCGTGACCACCCTGACCTACGGCGTGCAGTGCTTCAGCCGCTAC
CCCGACCACATGAAGCAGCACGACTTCTTCAAGTCCGCCATGCCCGAAGGCTACGTCCAGGAGCGCACCATCTTCT
TCAAGGACGACGGCAACTACAAGACCCGCGCCGAGGTGAAGTTCGAGGGCGACACCCTGGTGAACCGCATCGAG
CTGAAGGGCATCGACTTCAAGGAGGACGGCAACATCCTGGGGCACAAGCTGGAGTACAACTACAACAGCCACAAC
GTCTATATCATGGCCGACAAGCAGAAGAACGGCATCAAGGTGAACTTCAAGATCCGCCACAACATCGAGGACGGC
AGCGTGCAGCTCGCCGACCACTACCAGCAGAACACCCCCATCGGCGACGGCCCCGTGCTGCTGCCCGACAACCACT
ACCTGAGCACCCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTACAAGTAA

**Matrice ECFP (SEQ ID NO : 157)**

ATGGTGAGCAAGGGCGAGGAGCTGTTCACCGGGGTGGTGCCCATCCTGGTCGAGCTGGACGGCGACGTAAACGG
CCACAAGTTCAGCGTGTCCGGCGAGGGCGAGGGCGATGCCACCTACGGCAAGCTGACCCTGAAGTTCATCTGCAC
CACCGGCAAGCTGCCCGTGCCCTGGCCCACCCTCGTGACCACCCTGACCTGGGGCGTGCAGTGCTTCAGCCGCTAC
CCCGACCACATGAAGCAGCACGACTTCTTCAAGTCCGCCATGCCCGAAGGCTACGTCCAGGAGCGCACCATCTTCT
TCAAGGACGACGGCAACTACAAGACCCGCGCCGAGGTGAAGTTCGAGGGCGACACCCTGGTGAACCGCATCGAG
CTGAAGGGCATCGACTTCAAGGAGGACGGCAACATCCTGGGGCACAAGCTGGAGTACAACTACATCAGCCACAAC
GTCTATATCACCGCCGACAAGCAGAAGAACGGCATCAAGGCCAACTTCAAGATCCGCCACAACATCGAGGACGGC
AGCGTGCAGCTCGCCGACCACTACCAGCAGAACACCCCCATCGGCGACGGCCCCGTGCTGCTGCCCGACAACCACT
ACCTGAGCACCCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTACAAGTCC

**Matrice EYFP (SEQ ID NO : 158)**

ATGGTGAGCAAGGGCGAGGAGCTGTTCACCGGGGTGGTGCCCATCCTGGTCGAGCTGGACGGCGACGTAAACGG
CCACAAGTTCAGCGTGTCCGGCGAGGGCGAGGGCGATGCCACCTACGGCAAGCTGACCCTGAAGTTCATCTGCAC
CACCGGCAAGCTGCCCGTGCCCTGGCCCACCCTCGTGACCACCTTCGGCTACGGCCTGCAGTGCTTCGCCCGCTACC
CCGACCACATGAAGCAGCACGACTTCTTCAAGTCCGCCATGCCCGAAGGCTACGTCCAGGAGCGCACCATCTTCTT
CAAGGACGACGGCAACTACAAGACCCGCGCCGAGGTGAAGTTCGAGGGCGACACCCTGGTGAACCGCATCGAGC
TGAAGGGCATCGACTTCAAGGAGGACGGCAACATCCTGGGGCACAAGCTGGAGTACAACTACAACAGCCACAAC
GTCTATATCATGGCCGACAAGCAGAAGAACGGCATCAAGGTGAACTTCAAGATCCGCCACAACATCGAGGACGGC
AGCGTGCAGCTCGCCGACCACTACCAGCAGAACACCCCCATCGGCGACGGCCCCGTGCTGCTGCCCGACAACCACT
ACCTGAGCTACCAGTCCGCCCTGAGCAAAGACCCCAACGAGAAGCGCGATCACATGGTCCTGCTGGAGTTCGTGA
CCGCCGCCGGGATCACTCTCGGCATGGACGAGCTGTAC

**Matrice mCherry (SEQ ID NO : 159)**

ATGGTCGGGGGTGAGCAAGGGCGAGGAGGATAACATGGCCATCATCAAGGAGTTCATGCGCTTCAAGGTGCACA
TGGAGGGCTCCGTGAACGGCCACGAGTTCGAGATCGAGGGCGAGGGCGAGGGCCGCCCCTACGAGGGCACCCA
GACCGCCAAGCTGAAGGTGACCAAGGGTGGCCCCCTGCCCTTCGCCTGGGACATCCTGTCCCCTCAGTTCATGTAC
GGCTCCAAGGCCTACGTGAAGCACCCCGCCGACATCCCCGACTACTTGAAGCTGTCCTTCCCCGAGGGCTTCAAGT
GGGAGCGCGTGATGAACTTCGAGGACGGCGGCGTGGTGACCGTGACCCAGGACTCCTCCCTGCAGGACGGCGAG
TTCATCTACAAGGTGAAGCTGCGCGGCACCAACTTCCCCTCCGACGGCCCCGTAATGCAGAAGAAAACCATGGGCT
GGGAGGCCTCCTCCGAGCGGATGTACCCCGAGGACGGCGCCCTGAAGGGCGAGATCAAGCAGAGGCTGAAGCTG
AAGGACGGCGGCCACTACGACGCTGAGGTCAAGACCACCTACAAGGCCAAGAAGCCCGTGCAGCTGCCCGGCGC
CTACAACGTCAACATCAAGTTGGACATCACCTCCCACAACGAGGACTACACCATCGTGGAACAGTACGAACGCGCC
GAGGGCCGCCACTCCACCGGCGGCATGGACGAGCTGTACAAGTGA

**Matrice hFUT3 cDNA (SEQ ID NO : 160)**

ATGGATCCCCTGGGTGCAGCCAAGCCACAATGGCCATGGCGCCGCTGTCTGGCCGCACTGCTATTTCAGCTGCTGG
TGGCTGTGTGTTTCTTCTCCTACCTGCGTGTGTCCCGAGACGATGCCACTGGATCCCCTAGGGCTCCCAGTGGGTCC
TCCCGACAGGACACCACTCCCACCCGCCCCACCCTCCTGATCCTGCTATGGACATGGCCTTTCCACATCCCTGTGGCT
CTGTCCCGCTGTTCAGAGATGGTGCCCGGCACAGCCGACTGCCACATCACTGCCGACCGCAAGGTGTACCCACAGG
CAGACACGGTCATCGTGCACCACTGGGATATCATGTCCAACCCTAAGTCACGCCTCCCACCTTCCCCGAGGCCGCA
GGGGCAGCGCTGGATCTGGTTCAACTTGGAGCCACCCCCTAACTGCCAGCACCTGGAAGCCCTGGACAGATACTTC
AATCTCACCATGTCCTACCGCAGCGACTCCGACATCTTCACGCCCTACGGCTGGCTGGAGCCGTGGTCCGGCCAGC
CTGCCCACCCACCGCTCAACCTCTCGGCCAAGACCGAGCTGGTGGCCTGGGCGGTGTCCAACTGGAAGCCGGACT
CAGCCAGGGTGCGCTACTACCAGAGCCTGCAGGCTCATCTCAAGGTGGACGTGTACGGACGCTCCCACAAGCCCC
TGCCCAAGGGGACCATGATGGAGACGCTGTCCCGGTACAAGTTCTACCTGGCCTTCGAGAACTCCTTGCACCCCGA
CTACATCACCGAGAAGCTGTGGAGGAACGCCCTGGAGGCCTGGGCCGTGCCCGTGGTGCTGGGCCCCAGCAGAA
GCAACTACGAGAGGTTCCTGCCACCCGACGCCTTCATCCACGTGGACGACTTCCAGAGCCCCAAGGACCTGGCCCG
GTACCTGCAGGAGCTGGACAAGGACCACGCCCGCTACCTGAGCTACTTTCGCTGGCGGGAGACGCTGCGGCCTCG
CTCCTTCAGCTGGGCACTGGATTTCTGCAAGGCCTGCTGGAAACTGCAGCAGGAATCCAGGTACCAGACGGTGCG
CAGCATAGCGGCTTGGTTCACCTGA

**Matrice eZ-HygromycinR K7 (SEQ ID NO : 161)**

CAGGCAGAAGTATGCAAAGCATGCATCTCAATTAGTCAGCAACCAGGTGTGGAAAGTCCCCAGGCTCCCCAGCAG
GCAGAAGTATGCAAAGCATGCATCTCAATTAGTCAGCAACCATAGTCCCGCCCCTAACTCCGCCCATCCCGCCCCTA
ACTCCGCCCAGTTCCGCCCATTCTCCGCCCCATGGCTGACTAATTTTTTTTATTTATGCAGAGGCCGAGGCCGCCTCT
GCCTCTGAGCTATTCCAGAAGTAGTGAGGAGGCTTTTTTGGAGGCCTAGGCTTTTGCAAAAAGCTCCCGGGAGCTT
GTATATCCATTTTCGGATCTGATCAGCACGTGATGAAAAAGCCTGAACTCACCGCGACGTCTGTCGAGAAGTTTCT
GATCGAAAAGTTCGACAGCGTGTCCGACCTGATGCAGCTCTCGGAGGGCGAAGAATCTCGTGCTTTCAGCTTCGAT
GTAGGAGGGCGTGGATATGTCCTGCGGGTAAATAGCTGCGCCGATGGTTTCTACAAAGATCGTTATGTTTATCGGC
ACTTTGCATCGGCCGCGCTCCCGATTCCGGAAGTGCTTGACATTGGGGAATTCAGCGAGAGCCTGACCTATTGCAT
CTCCCGCCGTGCACAGGGTGTCACGTTGCAAGACTTGCCTGAAACCGAACTGCCCGCTGTTCTGCAGCCGGTCGCG
GAGGCCATGGATGCGATCGCTGCGGCCGATCTTAGCCAGACGAGCGGGTTCGGCCCATTCGGACCGCAAGGAATC
GGTCAATACACTACATGGCGTGATTTCATATGCGCGATTGCTGATCCCCATGTGTATCACTGGCAAACTGTGATGG
ACGACACCGTCAGTGCGTCCGTCGCGCAGGCTCTCGATGAGCTGATGCTTTGGGCCGAGGACTGCCCCGAAGTCC
GGCACCTCGTGCACGCGGATTTCGGCTCCAACAATGTCCTGACGGACAATGGCCGCATAACAGCGGTCATTGACTG
GAGCGAGGCGATGTTCGGGGATTCCCAATACGAGGTCGCCAACATCTTCTTCTGGAGGCCGTGGTTGGCTTGTATG
GAGCAGCAGACGCGCTACTTCGAGCGGAGGCATCCGGAGCTTGCAGGATCGCCGCGGCTCCGGGCGTATATGCTC
CGCATTGGTCTTGACCAACTCTATCAGAGCTTGGTTGACGGCAATTTCGATGATGCAGCTTGGGCGCAGGGTCGAT
GCGACGCAATCGTCCGATCCGGAGCCGGGACTGTCGGGCGTACACAAATCGCCCGCAGAAGCGCGGCCGTCTGG
ACCGATGGCTGTGTAGAAGTACTCGCCGATAGTGGAAACCGACGCCCCAGCACTCGTCCGAGGGCAAAGGAATAG
CACGTGCTACGAGATTTCGATTCCACCGCCGCCTTCTATGAAAGGTTGGGCTTCGGAATCGTTTTCCGGGACGCCG
GCTGGATGATCCTCCAGCGCGGGGATCTCATGCTGGAGTTCTTCGCCCACCCCAACTTGTTTATTGCAGCTTATAAT
GGTTACAAATAAAGCAATAGCATCACAAATTTCACAAATAAAGCATTTTTTTCACTGCATTCTAGTTGTGGTTTGTCC
AAACTCATCAATGTATC

**Matrice KanMX4 K7 (SEQ ID NO : 162)**

GTACGCTGCAGGTCGACAACCCTTAATATAACTTCGTATAATGTATGCTATACGAAGTTATTAGGTCTAGAGATCTG
TTTAGCTTGCCTCGTCCCCGCCGGGTCACCCGGCCAGCGACATGGAGGCCCAGAATACCCTCCTTGACAGTCTTGA
CGTGCGCAGCTCAGGGGCATGATGTGACTGTCGCCCGTACATTTAGCCCATACATCCCCATGTATAATCATTTGCAT
CCATACATTTTGATGGCCGCACGGCGCGAAGCAAAAATTACGGCTCCTCGCTGCAGACCTGCGAGCAGGGAAACG
CTCCCCTCACAGACGCGTTGAATTGTCCCCACGCCGCGCCCCTGTAGAGAAATATAAAAGGTTAGGATTTGCCACT
GAGGTTCTTCTTTCATATACTTCCTTTTAAAATCTTGCTAGGATACAGTTCTCACATCACATCCGAACATAAACAACC
ATGGGTAAGGAAAAGACTCACGTTTCGAGGCCGCGATTAAATTCCAACATGGATGCTGATTTATATGGGTATAAAT

GGGCTCGCGATAATGTCGGGCAATCAGGTGCGACAATCTATCGATTGTATGGGAAGCCCGATGCGCCAGAGTTGT
TTCTGAAACATGGCAAAGGTAGCGTTGCCAATGATGTTACAGATGAGATGGTCAGACTAAACTGGCTGACGGAAT
TTATGCCTCTTCCGACCATCAAGCATTTTATCCGTACTCCTGATGATGCATGGTTACTCACCACTGCGATCCCCGGCA
AAACAGCATTCCAGGTATTAGAAGAATATCCTGATTCAGGTGAAAATATTGTTGATGCGCTGGCAGTGTTCCTGCG
CCGGTTGCATTCGATTCCTGTTTGTAATTGTCCTTTTAACAGCGATCGCGTATTTCGTCTCGCTCAGGCGCAATCACG
AATGAATAACGGTTTGGTTGATGCGAGTGATTTTGATGACGAGCGTAATGGCTGGCCTGTTGAACAAGTCTGGAA
AGAAATGCATAAGCTTTTGCCATTCTCACCGGATTCAGTCGTCACTCATGGTGATTTCTCACTTGATAACCTTATTTT
TGACGAGGGGAAATTAATAGGTTGTATTGATGTTGGACGAGTCGGAATCGCAGACCGATACCAGGATCTTGCCAT
CCTATGGAACTGCCTCGGTGAGTTTTCTCCTTCATTACAGAAACGGCTTTTTCAAAAATATGGTATTGATAATCCTGA
TATGAATAAATTGCAGTTTCATTTGATGCTCGATGAGTTTTTCTAATCAGTACTGACAATAAAAAGATTCTTGTTTTC
AAGAACTTGTCATTTGTATAGTTTTTTTATATTGTAGTTGTTCTATTTTAATCAAATGTTAGCGTGATTTATATTTTTTT
TCGCCTCGACATCATCTGCCCAGATGCGAAGTTAAGTGCGCAGAAAGTAATATCATGCGTCAATCGTATGTGAATG
CTGGTCGCTATACTGCTGTCGATTCGATACTAACGCCGCCATCCAGTGTCGAAAACGAGCTCTCGAGAACCCTTAAT
ATAACTTCGTATAATGTATGCTATACGAAGTTATTAGGTGATATCAGATCCACTAGTG

**Matrice LacZα (SEQ ID NO : 163)**

GCCCAATACGCAAACCGCCTCTCCCCGCGCGTTGGCCGATTCATTAATGCAGCTGGCACGACAGGTTTCCCGACTG
GAAAGCGGGCAGTGAGCGCAACGCAATTAATGTGAGTTAGCTCACTCATTAGGCACCCCAGGCTTTACACTTTATG
CTTCCGGCTCGTATGTTGTGTGGAATTGTGAGCGGATAACAATTTCACACAGGAAACAGCTATGACCATGATTACG
GACAGCCTGGCCGTCGTTTTACAACGTCGTGACTGGGAAAACCCTGGCGTTACCCAACTTAATCGCCTTGCAGCAC
ATCCCCCTTTCGCCAGCTGGCGTAATAGCGAAGAGGCCCGCACCGATCGCCCTTCCCAACAGTTGCGCAGCCTGAA
TGGCGAATGGCGCCTGATGCGGTATTTTCTCCTTACGCATCTGTGCGGTATTTCACACCGCATATGGTGCACTCTCA
GTACAATCTGCTCTGATGCCGCATAGAC

**Matrice mB3Galt6 cDNA (SEQ ID NO : 164)**

ATGAAGGTATTCCGGCGCGCTTGGCGGCACCGGGTGGCGCTGGGCCTAGGCGGCCTGGCGTTCTGCGGCACCACT
CTGTTGTACCTGGCGCGCTGCGCTTCCGAGGGCGAGACGCCCTCCGCTTCCGGAGCCGCTCGGCCCCGCGCTAAG
GCCTTCCTGGCGGTGCTGGTGGCCAGTGCGCCCCGCGCGGTCGAGCGCCGCACCGCAGTGCGCAGCACGTGGCTG
GCACCGGAGAGGCGTGGCGGACCCGAGGACGTGTGGGCGCGCTTCGCCGTGGGCACTGGCGGCTTAGGCTCGGA
GGAGCGGCGCGCTCTTGAGCTCGAGCAGGCGCAGCACGGGGACCTGCTGCTGCTGCCCGCCCTGCGCGACGCCTA
CGAGAACCTCACGGCCAAGGTCCTGGCCATGCTGACCTGGCTGGATGAGCGCGTGGACTTCGAGTTCGTGCTCAA
GGCGGACGACGACTCCTTTGCGCGCCTGGACGCTATCCTGGTGGACCTACGCGCACGGGAGCCCGCACGCCGCCG

GCGCCTCTACTGGGGCTTCTTTTCCGGGCGCGGGCGCGTCAAGCCGGGAGGTCGCTGGCGAGAAGCAGCCTGGCA
ACTCTGCGACTACTACCTGCCCTACGCGTTGGGCGGTGGCTATGTCCTTTCTGCGGACCTGGTGCATTACCTGCGCC
TCAGCCGCGAGTACCTGCGCGCGTGGCACAGTGAAGACGTATCGCTGGGCACCTGGCTGGCACCAGTGGATGTGC
AACGGGAGCACGACCCACGCTTCGACACGGAGTACAAATCTCGAGGCTGCAACAATCAGTATCTGGTGACACACA
AGCAAAGCCCAGAGGACATGTTGGAGAAGCAACAGATGTTGCTGCATGAGGGCCGGTTGTGCAAGCATGAGGTG
CAGTTGCGCCTTTCCTATGTCTATGACTGGTCAGCTCCACCCTCCCAGTGCTGCCAGCGCAAGGAGGGCGTTCCCTG
ATGTCA

**Matrice eZ-mB3Galt6 cDNA shRNA insensible (SEQ ID NO : 165)**

ATGAAGGTATTCCGGCGCGCTTGGCGGCACCGGGTGGCGCTGGGCCTAGGCGGCCTGGCGTTCTGCGGCACCACT
CTGTTGTACCTGGCGCGCTGCGCTTCCGAGGGCGAGACGCCCTCCGCTTCCGGAGCCGCTCGGCCCCGCGCTAAG
GCCTTCCTGGCGGTGCTGGTGGCCAGTGCGCCCCGCGCGGTCGAGCGCCGCACCGCAGTGCGCAGCACGTGGCTG
GCACCGGAGAGGCGTGGCGGACCCGAGGACGTGTGGGCGCGCTTCGCCGTGGGCACTGGCGGCTTAGGCTCGGA
GGAGCGGCGCGCTCTTGAGCTCGAGCAGGCGCAGCACGGGGACCTGCTGCTGCTGCCCGCCCTGCGCGACGCCTA
CGAGAACCTCACGGCCAAGGTCCTGGCCATGCTGACCTGGCTGGATGAGCGCGTGGACTTCGAGTTCGTGCTCAA
GGCGGACGACGACTCCTTTGCGCGCCTGGACGCTATCCTGGTGGACCTACGCGCACGGGAGCCCGCACGCCGCCG
GCGCCTCTACTGGGGCTTCTTTTCCGGGCGCGGGCGCGTCAAGCCGGGAGGTCGCTGGCGAGAAGCAGCCTGGCA
ACTCTGCGACTACTACCTGCCCTACGCGTTGGGCGGTGGCTATGTCCTTTCTGCGGACCTGGTGCATTACCTGCGCC
TCAGCCGCGAGTACCTGCGCGCGTGGCACAGTGAAGACGTATCGCTGGGCACCTGGCTGGCACCAGTGGATGTGC
AACGGGAGCACGACCCACGCTTCGACACGGAGTACAAATCTCGAGGCTGCAACAATCAGTATCTGGTGACACACA
AGCAAAGCCCAGAGGACATGTTGGAGAAGCAACAGATGTTGCTGCATGAGGGCCGGTTGTGCAAGCATGAGGTG
CAACTTCGCCTTTCCTATGTCTATGACTGGTCAGCTCCACCCTCCCAGTGCTGCCAGCGCAAGGAGGGCGTTCCCTG
ATGTCA

**Matrice Yeast *S.cerevisiae* MNN10 gene (SEQ ID NO : 166)**

AAACATGCATTCAAAGGTCATAATTGCTGCTCTATTTACAGTCGTCCATAATGACATTTCTCTTTGATTATTTTCTTGT
TTTTTCGCTCTTCTCAAGTGGATGTTACATAACAAACAAAACAGAAAAAATTGTTTAAATATAAAGTTTAAAAGTTAT
CTTTGATTCCGCACCTGAATTTTTGGATTGAAGGCCAAAGGAGGTTTATCAGGGAGAGAAAAGCTCTCTATTTATTT
TTATAAGGAATAATTGTGCATGTACAACTATACAATATGTCTAGTGTACCTTATAATTCCCAACTTCCTATATCCAAC
CATCTAGAGTACGATGAAGATGAAAAGAAGAGCAGAGGCTCAAAACTAGGCCTGAAATATAAAATGATATACTGG
AGGAAAACTTTATGCAGTTCGCTAGCGAGATGGAGAAAGCTAATACTATTAATATCTTTAGCTTTGTTTTTATTCAT
ATGGATAAGCGATTCCACCATAAGCAGAAATCCATCTACCACAAGTTTTCAAGGCCAAAATAGTAACGATAATAAG

TTGAGTAATACTGGTTCTAGCATCAACTCCAAAAGATATGTACCACCATATTCTAAGAGATCAAGATGGTCGTTTTG
GAATCAAGATCCTAGGATTGTCATTATATTAGCGGCAAACGAAGGTGGTGGTGTATTGAGGTGGAAAAATGAGCA
AGAATGGGCTATCGAAGGCATATCAATAGAAAATAAGAAGGCCTATGCGAAGAGACATGGATATGCGTTGACTAT
CAAGGATTTGACAACGTCCAAAAGATACTCTCACGAATACAGAGAGGGTTGGCAAAAAGTAGATATATTGAGACA
GACGTTCAGGGAGTTTCCTAATGCAGAATGGTTCTGGTGGTTGGACCTGGATACTATGATAATGGAGCCTTCTAAA
TCATTAGAAGAACATATTTTCGACAGATTGGAAACTCTGGCTGACAGAGAATTGAAAAGTTTTAATCCCCTAAACCT
AAGAGACGACATACCCTATGTCGATTATTCAGAGGAAATGGAGTTTCTAATAACACAAGATTGTGGAGGCTTCAAT
TTGGGCTCATTTCTGATAAAAAATAGCGAATGGTCTAAGCTGCTTCTAGATATGTGGTGGGACCCCGTTCTGTATGA
ACAAAAACATATGGTTTGGGAACATAGAGAACAAGATGCGTTAGAGGCATTATATGAAAACGAACCGTGGATTCG
TTCGAGAATAGGATTTTTGCCCTTAAGAACGATCAATGCATTCCCACCGGGAGCATGCTCTGAATACAGTGGTGAC
TCAAGATACTTTTACAGTGAGAAAGACCATGATTTTGTTGTGAATATGGCCGGATGCAATTTTGGCAGAGATTGCT
GGGGCGAGATGCAGTACTACACCACTTTAATGGAAAAACTGAATAGGAAATGGTACACGAGATTTTTCTTCCCATA
AAATGGAAGTTATCAATATTGTAAAGAGAAGCATTTACAAGCTTTTATTTTTCTTTTTAATTTCCACTACTGGTTCTG
CTTTAAAATGTTGTTTTATAATTTATGTACATTTAGGCCTATAGAAGATTCTTTCAATAATATGCTACACATTCTTTTA
TTTTTCCATCATATGTTGGAGTTTATGCCTCCTCGGCAGGAGTTGGGCGGTGCGAAGAGAAGAAAAAGAGTGAAA
CTAAAAAAAGGAATCTGCCTTTGCATAAGTTCAAAAGTGCAATTTTAGTGTTGGATTTAAACGGGAAAAATTGAAA
TGGCCATCGAAACAATACTTGTAATAAACAAATCAGGCGGACTAATCTATCAGCGGAATTTTACCAACGACGAACA
GAAATTGAACAGCAATGAATACTTAATTCTTGCTAGTACACTGCACGGTGTATTCGCCATCGCGAGCCAGCTGACTC
CGAAGGCATTACAGCTAACTCAACAAACGAACATCGAAAATACCATCCCATATATACCTTACGTGGGCATGTCCAG
CAATAGGAGCGATACAAGAAATGGAGGTGGCAATAACAACAAACACACTAATAATGAAAAACTGGGCAGTTT

**Matrice promoteur CMV (SEQ ID NO : 167)**

ACCAATTCAGTCGACTGGATCCTAGTTATTAATAGTAATCAATTACGGGGTCATTAGTTCATAGCCCATATATGGAG
TTCCGCGTTACATAACTTACGGTAAATGGCCCGCCTGGCTGACCGCCCAACGACCCCCGCCCATTGACGTCAATAAT
GACGTATGTTCCCATAGTAACGCCAATAGGGACTTTCCATTGACGTCAATGGGTGGAGTATTTACGGTAAACTGCC
CACTTGGCAGTACATCAAGTGTATCATATGCCAAGTACGCCCCCTATTGACGTCAATGACGGTAAATGGCCCGCCT
GGCATTATGCCCAGTACATGACCTTATGGGACTTTCCTACTTGGCAGTACATCTACGTATTAGTCATCGCTATTACCA
TGGTGATGCGGTTTTGGCAGTACATCAATGGGCGTGGATAGCGGTTTGACTCACGGGGATTTCCAAGTCTCCACCC
CATTGACGTCAATGGGAGTTTGTTTTGGCACCAAAATCAACGGGACTTTCCAAAATGTCGTAACAACTCCGCCCCAT
TGACGCAAATGGGCGGTAGGCGTGTACGGTGGGAGGTCTATATAAGCAGAGCTGGTTTAGTGAACCGTCAGATCA
CTAGTCGACTAGGGATAACAGGGCCGC

**Matrice promoteur EF1alpha, version courte (SEQ ID NO : 168)**

AGGAACCAATTCAGTCGACTGGATCCCGATGGCTCCGGTGCCCGTCAGTGGGCAGAGCGCACATCGCCCACAGTCCCCGAGAAGTTGGGGGGAGGGGTCGGCAATTGAACCGGTGCCTAGAGAAGGTGGCGCGGGGTAAACTGGGAAAGTGATGTCGTGTACTGGCTCCGCCTTTTTCCCGAGGGTGGGGGAGAACCGTATATAAGTGCAGTAGTCGCCGTGAACGTTCTTTTTCGCAACGGGTTTGCCGCCAGAACACAGGTCCGCGGCCCCGAACTAGGCCTAGGCGTCTGATCACTAGTGACTCTAGTCCTAGTCGACTAGGGATAACAGGG

**Matrice promoteur EF1alpha (SEQ ID NO : 169)**

GTGAGGCTCCGGTGCCCGTCAGTGGGCAGAGCGCACATCGCCCACAGTCCCCGAGAAGTTGGGGGGAGGGGTCGGCAATTGAACCGGTGCCTAGAGAAGGTGGCGCGGGGTAAACTGGGAAAGTGATGTCGTGTACTGGCTCCGCCTTTTTCCCGAGGGTGGGGGAGAACCGTATATAAGTGCAGTAGTCGCCGTGAACGTTCTTTTTCGCAACGGGTTTGCCGCCAGAACACAGGTAAGTGCCGTGTGTGGTTCCCGCGGGCCTGGCCTCTTTACGGGTTATGGCCCTTGCGTGCCTTGAATTACTTCCACCTGGCTGCAGTACGTGATTCTTGATCCCGAGCTTCGGGTTGGAAGTGGGTGGGAGAGTTCGAGGCCTTGCGCTTAAGGAGCCCCTTCGCCTCGTGCTTGAGTTGAGGCCTGGCCTGGGCGCTGGGGCCGCCGCGTGCGAATCTGGTGGCACCTTCGCGCCTGTCTCGCTGCTTTCGATAAGTCTCTAGCCATTTAAAATTTTTGATGACCTGCTGCGACGCTTTTTTTCTGGCAAGATAGTCTTGTAAATGCGGGCCAAGATCTGCACACTGGTATTTCGGTTTTTGGGGCCGCGGGCGGCGACGGGGCCCGTGCGTCCCAGCGCACATGTTCGGCGAGGCGGGGCCTGCGAGCGCGGCCACCGAGAATCGGACGGGGGTAGTCTCAAGCTGGCCGGCCTGCTCTGGTGCCTGGCCTCGCGCCGCCGTGTATCGCCCCGCCCTGGGCGGCAAGGCTGGCCCGGTCGGCACCAGTTGCGTGAGCGGAAAGATGGCCGCTTCCCGGCCCTGCTGCAGGGAGCTCAAAATGGAGGACGCGGCGCTCGGGAGAGCGGGCGGGTGAGTCACCCACACAAAGGAAAAGGGCCTTTCCGTCCTCAGCCGTCGCTTCATGTGACTCCACGGAGTACCGGGCGCCGTCCAGGCACCTCGATTAGTTCTCGAGCTTTTGGAGTACGTCGTCTTTAGGTTGGGGGGAGGGGTTTTATGCGATGGAGTTTCCCCACACTGAGTGGGTGGAGACTGAAGTTAGGCCAGCTTGGCACTTGATGTAATTCTCCTTGGAATTTGCCCTTTTTGAGTTTGGATCTTGGTTCATTCTCAAGCCTCAGACAGTGGTTCAAAGTTTTTTTCTTCCATTTCAGGTGTCGTGA

**Matrice promoteur TRE3G (SEQ ID NO : 170)**

CTTTCGTCTTCAAGAATTCCTGGAGTTTACTCCCTATCAGTGATAGAGAACGTATGAAGAGTTTACTCCCTATCAGTGATAGAGAACGTATGCAGACTTTACTCCCTATCAGTGATAGAGAACGTATAAGGAGTTTACTCCCTATCAGTGATAGAGAACGTATGACCAGTTTACTCCCTATCAGTGATAGAGAACGTATCTACAGTTTACTCCCTATCAGTGATAGAGAACGTATATCCAGTTTACTCCCTATCAGTGATAGAGAACGTATAAGCTTTAGGCGTGTACGGTGGGCGCCTATAAAAGCAGAGCTCGTTTAGTGAACCGTCAGATCGCCTGGAGCAATTCCACAACACTTTTGTCTTATACCAACTTTCCGTACCACTTCCTACCCTCGTAAA

**Matrice eZ-Rosa26-3' (SEQ ID NO : 171)**

AGATGGGCGGGAGTCTTCTGGGCAGGCTTAAAGGCTAACCTGGTGTGTGGGCGTTGTCCTGCAGGGGAATTGAAC
AGGTGTAAAATTGGAGGGACAAGACTTCCCACAGATTTTCGGTTTTGTCGGGAAGTTTTTTAATAGGGGCAAATAG
GAAAATGGAGGATAGGAGTCATCTGGGGTTTATGCAGCAAAACTACAGGTATATTGCTTGTATCCGCCTCGGAGA
TTTCCATGAGGAGATAAAGACATGTCACCCGAGTTTATACTCTCCTGCTTAGATCCTACTACAGTATGAAATACAGT
GTCGCGAGGTAGACTATGTAAGCAGATTTAATCATTTTAAAGAGCCCAGTACTTCATATCCATTTCTCCCGCTCCTTC
TGCAGCCTTATCAAAAGGTATTTAGAACACTCATTTTAGCCCCATTTTCATTTATTATACTGGCTTATCCAACCCCTAG
ACAGAGCATTGGCATTTTCCCTTTCCTGATCTTAGAAGTCTGATGACTCATGAAACCAGACAGATTAGTTACATACA
CCACAAATCGAGGCTGTAGCTGGGGCCTCAACACTGCAGTTCTTTTATAACTCCTTAGTACACTTTTTGTTGATCCTT
TGCCTTGATCCTTAATTTTCAGTGTCTATCACCTCTCCCGTCAGGTGGTGTTCCACATTTGGGCCTATTCTCAGTCCA
GGGAGTTTTACAACAATAGATGTATTGAGAATCCAACCTAAAGCTTAACTTTCCACTCCCATGAATGCCTCTCTCCTT
TTTCTCCATTATAACTGAGCTATAACCATTAATGGTTTCAGGTGGATGTCTCCTCCCCCAATATACCTGATGTATCTA
CATATTGCCAGGCTGATATTTTAAGACATAAAAGGTATATTTCATTATTGAGCCACATGGTATTGATTACTGCTACTA
AAATTTTGTCATTGTACACATCTGTAAAAGGTGGTTCCTTTTGGAATGCAAAGTTCAGGTGTTTGTTGTCTTTCCTGA
CCTAAGGTCTTGTGAGCTTGTATTTTTTCTATTTAAGCAGTGCTTTCTCTTGGACTGGCTTGACTCATGGCATTCTAC
ACGTTATTGCTGGTCTAAATGTGATTTTGCCAAGCTTCTTCAGGACCTATAATTTTGCTTGACTTGTAGCCAAACACA
AGTAAAATGATTAAGCAACAAATGTATTTGTGAAGCTTGGTTTTTAGGTTGTTGTGTTGTGTGTGCTTGTGCTCTAT
AATAATACTATCCAGGGGCTGGAGAGGTGGCTCGGAGTTCAAGAGCACAGACTGCTCTTCCAGAAGTCCTGAGTT
CAATTCCCAGCAACCACATGGTGGCTCACAACCATCTGTAATGGGATCTGATGCCCTCTTCTGGTGTGTCTGAAGAC
CACAAGTGTATTCACATTAAATAAATAATCCTCCTTCTTCTTCTTTTTTTTTTTTTAAAGAGAATACTGTCTCCAGTAG
AATTACTGAAGTAATGAAATACTTTGTGTTTGTTCCAATATGGAAGCCAATAATCAAATACTCTTAAGCACTGGAAA
TGTACCAAGGAACTATTTTATTTAAGTGAACTGTGGACAGAGGAGCCATAACTGCAGACTTGTGGGATACAGAAG
ACCAATGCAGACTTAATGTCTTTTCTCTTACACTAAGCAATAAAGAAATAAAAATTGAACTTCTAGTATCCTATTTGT
TAAACTGCTAGCTTTACTAACTTTTGTGCTTCATCTATACAAAGCTGAAAGCTAAGTCTGCAGCCATTACTAAACATG
AAAGCAAGTAATGATAATTTTGGATTTCAAAAATGTAGGGCCAGAGTTTAGCCAGCCAGTGGTGGTGCTTGCCTTT
ATGCCTTAATCCCAGCACTCTGGAGGCAGAGACAGGCAGATCTCTGAGTTTGAGCCCAGCCTGGTCTACACATCAA
GTTCTATCTAGGATAGCCAGGAATACACACAGAAACCCTGTTGGGGAGGGGGGCTCTGAGATTTCATAAAATTATA
ATTGAAGCATTCCCTAATGAGCCACTATGGATGTGGCTAAATCCGTCTACCTTTCTGATGAGATTTGGGTATTATTTT
TTCTGTCTCTGCTGTTGGTTGGGTCTTTTGACACTGTGGGCTTTCTTAAAGCCTCCTTCCCTGCCATGTGGACTCTTG
TTTGCTACTAACTTCCCATGGCTTAAATGGCATGGCTTTTTGCCTTCTAAGGGCAGCTGCTGAGATTTGCAGCCTGA
TTTCCAGGGTGGGGTTGGGAAATCTTTCAAACACTAAAATTGTCCTTTAATTTTTTTTTAAAAAATGGGTTATATAAT

AAACCTCATAAAATAGTTATGAGGAGTGAGGTGGACTAATATTAATGAGTCCCTCCCCTATAAAAGAGCTATTAAGGCTTTTTGTCTTATACTAACTTTTTTTTTAAATGTGGTATCTTTAGAACCAAGGGTCTTAGAGTTTTAGTATACAGAAACTGTTGCATCGCTTAATCAGATTTTCTAGTTTCAAATCCAGAGAATCCAAATTCTTCACAGCCAAAGTCAAATTAAGAATTTCTGACTTTAATGTTATTTGCTACTGTGAATATAAAATGATAGCTTTTCCTGAGGCAGGGTATCACTATGTATCTCTGCCTGATCTGCAACAAGATATGTAGACTAAAGTTCTGCCTGCTTTTGTCTCCTGAATACTAAGGTTAAAATGTAGTAATACTTTTGGAACTTGCAGGTCAGATTCTTTTATAGGGGACACACTAAGGGAGCTTGGGTGATAGTTGGTAAATGTGTTTAAGTGATGAAAACTTGAATTATTATCACCGCAACCTACTTTTTAAAAAAAAAAGCCAGGCCTGTTAGAGCATGCTAAGGGATCCCTAGGACTTGCTGAGCACACAAGAGTAGTACTTGGCAGGCTCCTGGTGAGAGCATATTTCAAAAAACAAGGCAGACAACCAAGAAACTACAGTAAGGTTACCTGTCTTTAACCATCTGCATATACACAGGGATATTAAAATATTCCAAATAATATTTCATTCAAGTTTTCCCCCATCAAATTGGGACATGGATTTCTCCGGTGAATAGGCAGAGTTGGAAACTAAACAAATGTTGGTTTTGTGATTTGTGAAATTGTTTTCAAGTGATAGTTAAAGCCCATGAGATACAGAACAAAGCTGCTATTTCGAGGTCACTTGGTTATACTCAGAAGCACTTCTTTGGGTTTCCCTGCACTATCCTGATCATGTGCTAGGCCTACCTTAGGCTGATTGTTGTTCAAATAACTTAAGTTTCCTGTCAGGTGATGTCATATGATTTCATATATCAAGGCAAAACATGTTATATATGTTAAACATTTGGACTTAATGTGAAAGTTAGGTCTTTGTGGGTTTTGATTTTAATTTCAAAACCTGAGCTAAATAAGTCATTTTACATGTCTTACATTTGGTGAATTGTATATTGTGGTTTGCAGGCAAGACTCTCTGACCTAGTAACCCTCCTATAGAGCACTTTGCTGGGTCACAAGTCTAGGAGTCAAGCATTTCACCTTGAAGTTGAGACGTTTTGTTAGTGTATACTAGTTATATGTTGGAGGACATGTTTATCCAGAAGATATTCAGGACTATTTTTGACTGGGCTAAGGAATTGATTCTGATTAGCACTGTTAGTGAGCATTGAGTGGCCTTTAGGCTTGAATTGGAGTCACTTGTATATCTCAAATAATGCTGGCCTTTTTTAAAAAGCCCTTGTTCTTTATCACCCTGTTTTCTACATAATTTTTGTTCAAAGAAATACTTGTTTGGATCTCCTTTTGACAACAATAGCATGTTTTCAAGCCATATTTTTTTTCCTTTTTTTTTTTTTTTTTGGTTTTTCGAGACAGGGTTTCTCTGTATAGCCCTGGCTGTCCTGGAACTCACTTTGTAGACCAGGCTGGCCTCGAACTCAGAAATCCGCCTGCCTCTGCCTCCTGAGTGCCGGGATTAAAGGCGTGCACCACCACGCCTGGCTAAGTTGGATATTTTGTATATAACTATAACCAATACTAACTCCACTGGGTGGATTTTTAATTCAGTCAGTAGTCTTAAGTGGTCTTTATTGGCCCTTATTAAAATCTACTGTTCACTCTAACAGAGGCTGTTGGACTAGTGGGACTAAGCAACTTCCTACGGATATACTAGCAGATAAGGGTCAGGGATAGAAACTAGTCTAGCGTTTTGTATACCTACCAGCTTATACTACCTTGTTCTGATAGAAATATTTAGGACATCTAGCTTATC

**Matrice eZ-Rosa26-5' (SEQ ID NO : 172)**

CCCCGCGGCAGGCCCTCCGAGCGTGGTGGAGCCGTTCTGTGAGACAGCCGGGTACGAGTCGTGACGCTGGAAGGGGCAAGCGGGTGGTGGGCAGGAATGCGGTCCGCCCTGCAGCAACCGGAGGGGGAGGGAGAAGGGAGCGGAAAAGTCTCCACCGGACGCGGCCATGGCTCGGGGGGGGGGGGGCAGCGGAGGAGCGCTTCCGGCCGACGTCTCGTCGCTGATTGGCTTCTTTTCCTCCCGCCGTGTGTGAAAACACAAATGGCGTGTTTTGGTTGGCGTAAGGCGCCTGTCAGT

TAACGGCAGCCGGAGTGCGCAGCCGCCGGCAGCCTCGCTCTGCCCACTGGGTGGGGCGGGAGGTAGGTGGGGTG
AGGCGAGCTGGACGTGCGGGCGCGGTCGGCCTCTGGCGGGGCGGGGGAGGGGAGGGAGGGTCAGCGAAAGTA
GCTCGCGCGCGAGCGGCCGCCCACCCTCCCCTTCCTCTGGGGGAGTCGTTTTACCCGCCGCCGGCCGGGCCTCGTC
GTCTGATTGGCTCTCGGGGCCCAGAAAACTGGCCCTTGCCATTGGCTCGTGTTCGTGCAAGTTGAGTCCATCCGCC
GGCCAGCGGGGGCGGCGAGGAGGCGCTCCCAGGTTCCGGCCCTCCCCTCGGCCCCGCGCCGCAGAGTCTGGCCG
CGCGCCCCTGCGCAACGTGGCAGGAAGCGCGCGCTGGGGGCGGGGACGGGCAGTAGGGCTGAGCGGCTGCGGG
GCGGGTGCAAGCACGTTTCCGACTTGAGTTGCCTCAAGAGGGGCGTGCTGAGCCAGACCTCCATCGCGCACTCCG
GGGAGTGGAGGGAAGGAGCGAGGGCTCAGTTGGGCTGTTTTGGAGGCAGGAAGCACTTGCTCTCCCAAAGTCGC
TCTGAGTTGTTATCAGTAAGGGAGCTGCAGTGGAGTAGGCGGGGAGAAGGCCGCACCCTTCTCCGGAGGGGGGA
GGGGAGTGTTGCAATACCTTTCTGGGAGTTCTCTGCTGCCTCCTGGCTTCTGAGGACCGCCCTGGGCCTGGGAGAA
TCCCTTGCCCCCTCTTCCCCTCGTGATCTGCAACTCCAGTCTT

**Matrice mB3Galt6 shRNA TR506016D (SEQ ID NO : 173)**

ACAGGGTCGACAAGCTTTTCCAAAAAAAAAGCATGAGGTGCAGTTGCGCCTTTCCTATCTCTTGAATAGGAAAGGC
GCAACTGCACCTCATGCTGGATCCCGCGTCCTTTCCACAAGATATATAAACCCAAGAAATCGAAATACTTTCAAGTT
ACGGTAAGCATATGATAGTCCATTTTAAAACATAATTTTAAAACTGCAAACTACCCAAGAAATTATTACTTTCTACGT
CACGTATTTTGTACTAATATCTTTGTGTTTACAGTCAAATTAATTCTAATTATCTCTCTAACAGCCTTGTATCGTATAT
GCAAATATGAAGGAATCATGGGAAATAGGCCCTCTTCCTGCCCGACCTTGGCGCGCGCTCGGCGCGCGGTCACGC
TCCGTCACGTGGTGCGTTTTG

**Matrice eZ-SiaT-TGS-Hook (SEQ ID NO : 174)**

ATGATTCACACCAACCTGAAGAAAAAGTTCAGCTGCTGCGTCCTGGTCTTTCTTCTGTTTGCAGTCATCTGTGTGTG
GAAGGAAAAGAAGAAAGGGAGTTACTATGATTCCTTTAAATTGCAAACCAAGGAATTCCAGGTGTTAAAGAGTCT
GGGGAAATTGGCCATGGGGTCTGATTCCCAGTCTGTATCCTCAAGCAGCACCCAGGACCCCCACAGGGGCCGCCA
GACCCTCGGCAGTCTCAGAGGCCTAGCCAAGGCCAAACCAGAGGCCTCCTTCCAGGTGTGGAACAAGGACAGCTC
TTCCAAAAACCTTATCCCTAGGCTGCAAAAGGGGTCGGGG

**Matrice TagBFP (SEQ ID NO : 175)**

ATGTCGGGGAGCGAGCTGATTAAGGAGAACATGCACATGAAGCTGTACATGGAGGGCACCGTGGACAACCATCA
CTTCAAGTGCACATCCGAGGGCGAAGGCAAGCCCTACGAGGGCACCCAGACCATGAGAATCAAGGTGGTCGAGG
GCGGCCCTCTCCCCTTCGCCTTCGACATCCTGGCTACTAGCTTCCTCTACGGCAGCAAGACCTTCATCAACCACACCC
AGGGCATCCCCGACTTCTTCAAGCAGTCCTTCCCTGAGGGCTTCACATGGGAGAGAGTCACCACATACGAGGACG

GGGGCGTGCTGACCGCTACCCAGGACACCAGCCTCCAGGACGGCTGCCTCATCTACAACGTCAAGATCAGAGGGGTGAACTTCACATCCAACGGCCCTGTGATGCAGAAGAAAACACTCGGCTGGGAGGCCTTCACCGAAACGCTGTACCCCGCTGACGGCGGCCTGGAAGGCAGAAACGACATGGCCCTGAAGCTCGTGGGCGGGAGCCATCTGATCGCAAACATCAAGACCACATATAGATCCAAGAAACCCGCTAAGAACCTCAAGATGCCTGGCGTCTACTATGTGGACTACAGACTGGAAAGAATCAAGGAGGCCAACAACGAAACCTACGTCGAGCAGCACGAGGTGGCAGTGGCCAGATACTGCGACCTCCCTAGCAAACTGGGGCACAAGCTTAATTCCGGATGA

**Matrice Thymidine Kinase cDNA (SEQ ID NO : 176)**

ATGGCTTCGTACCCCTGCCATCAACACGCGTCTGCGTTCGACCAGGCTGCGCGTTCTCGCGGCCATAGCAACCGACGTACGGCGTTGCGCCCTCGCCGGCAGCAAGAAGCCACGGAAGTCCGCCTGGAGCAGAAAATGCCCACGCTACTGCGGGTTTATATAGACGGTCCTCACGGGATGGGGAAAACCACCACCACGCAACTGCTGGTGGCCCTGGGTTCGCGCGACGATATCGTCTACGTACCCGAGCCGATGACTTACTGGCAGGTGCTGGGGGCTTCCGAGACAATCGCGAACATCTACACCACACAACACCGCCTCGACCAGGGTGAGATATCGGCCGGGGACGCGGCGGTGGTAATGACAAGCGCCCAGATAACAATGGGCATGCCTTATGCCGTGACCGACGCCGTTCTGGCTCCTCATATCGGGGGGGAGGCTGGGAGCTCACATGCCCCGCCCCCGGCCCTCACCCTCATCTTCGACCGCCATCCCATCGCCGCCCTCCTGTGCTACCCGGCCGCGCGATACCTTATGGGCAGCATGACCCCCCAGGCCGTGCTGGCGTTCGTGGCCCTCATCCCGCCGACCTTGCCCGGCACAAACATCGTGTTGGGGGCCCTTCCGGAGGACAGACACATCGACCGCCTGGCCAAACGCCAGCGCCCCGGCGAGCGGCTTGACCTGGCTATGCTGGCCGCGATTCGCCGCGTTTACGGGCTGCTTGCCAATACGGTGCGGTATCTGCAGGGCGGCGGGTCGTGGCGGGAGGATTGGGGACAGCTTTCGGGGACGGCCGTGCCGCCCCAGGGTGCCGAGCCCCAGAGCAACGCGGGCCCACGACCCCATATCGGGGACACGTTATTTACCCTGTTTCGGGCCCCCGAGTTGCTGGCCCCCAACGGCGACCTGTACAACGTGTTTGCCTGGGCCTTGGACGTCTTGGCCAAACGCCTCCGTCCCATGCACGTCTTTATCCTGGATTACGACCAATCGCCCGCCGGCTGCCGGGACGCCCTGCTGCAACTTACCTCCGGGATGGTCCAGACCCACGTCACCACCCCCGGCTCCATACCGACGATCTGCGACCTGGCGCGCACGTTTGCCCGGGAGATGGGGGAGGCTAACTGA

**Matrice TK term (SEQ ID NO : 177)**

GGGGGAGGCTAACTGAAACACGGAAGGAGACAATACCGGAAGGAACCCGCGCTATGACGGCAATAAAAAGACAGAATAAAACGCACGGTGTTGGGTCGTTTGTTCATAAACGCGGGGTTCGGTCCCAGGGCTGGCACTCTGTCGATACCCCACCGAGGCCCCATTGGGGCCAATACGCCCGCGTTTCTTCCTTTTCCCCACCCCACCCCCCAAGTTCGGGTGAAGGCCCAGGGCTCGCAGCCAACGTCGGGGCGGCAGGCCCTGCCATAGCC

**Matrice TetON-3G cDNA (SEQ ID NO : 178)**

ATGTCTAGACTGGACAAGAGCAAAGTCATAAACTCTGCTCTGGAATTACTCAATGGAGTCGGTATCGAAGGCCTGA
CGACAAGGAAACTCGCTCAAAAGCTGGGAGTTGAGCAGCCTACCCTGTACTGGCACGTGAAGAACAAGCGGGCCC
TGCTCGATGCCCTGCCAATCGAGATGCTGGACAGGCATCATACCCACTCCTGCCCCCTGGAAGGCGAGTCATGGCA
AGACTTTCTGCGGAACAACGCCAAGTCATACCGCTGTGCTCTCCTCTCACATCGCGACGGGGCTAAAGTGCATCTC
GGCACCCGCCCAACAGAGAAACAGTACGAAACCCTGGAAAATCAGCTCGCGTTCCTGTGTCAGCAAGGCTTCTCCC
TGGAGAACGCACTGTACGCTCTGTCCGCCGTGGGCCACTTTACACTGGGCTGCGTATTGGAGGAACAGGAGCATC
AAGTAGCAAAAGAGGAAAGAGAGACACCTACCACCGATTCTATGCCCCCACTTCTGAAACAAGCAATTGAGCTGTT
CGACCGGCAGGGAGCCGAACCTGCCTTCCTTTTCGGCCTGGAACTAATCATATGTGGCCTGGAGAAACAGCTAAA
GTGCGAAAGCGGCGGGCCGACCGACGCCCTTGACGATTTTGACTTAGACATGCTCCCAGCCGATGCCCTTGACGAC
TTTGACCTTGATATGCTGCCTGCTGACGCTCTTGACGATTTTGACCTTGACATGCTCCCCGGGTAA

**Revendications**

1. Procédé de fabrication d'un vecteur circulaire d'ADN double brin comportant au moins six séquences d'intérêt, comprenant :

a) une étape de mise en contact simultanée d'un mélange équimolaire d'au moins six briques moléculaires, différentes l'une de l'autre, en présence d'une seule enzyme de restriction de type IIs et d'une ligase, chaque brique moléculaire étant une molécule d'ADN linéaire double brin; et contenant :

i. une séquence d'intérêt dépourvue de site de reconnaissance spécifique de la susdite enzyme de restriction de type IIs,
ii. deux adaptateurs d'ADN double brin, flanquant en amont et en aval ladite séquence d'intérêt, chaque adaptateur d'ADN double brin consistant en une séquence d'au moins 12 nucléotides, laquelle contient un seul et unique site de reconnaissance de la susdite enzyme de restriction de type IIs, le site de reconnaissance de la susdite enzyme de restriction de type IIs de l'adaptateur en amont de ladite séquence d'intérêt et le site de reconnaissance de la susdite enzyme de restriction de type IIs de l'adaptateur en aval de ladite séquence spécifique étant convergents,

b) une étape d'incubation du mélange de l'étape a) à une température de 20 à 55°C pendant une période de 2 à 30 min,
laquelle étape conduit :

- à l'élimination par coupure des sites de reconnaissance de l'enzyme de restriction de type IIs utilisée,
- à la formation d'une suture monobrin cohésive d'au moins 2 nucléotides à chacune des extrémités de ladite séquence d'intérêt, ladite suture monobrin cohésive d'au moins 2 nucléotides en amont d'une des au moins six séquences d'intérêt étant complémentaire à ladite suture monobrin cohésive d'au moins 2 nucléotides en aval d'une autre séquence d'intérêt,
- à l'appariement par complémentarité nucléotidique des susdites sutures monobrins cohésives d'au moins 2 nucléotides et
- au positionnement des séquences d'intérêt, de façon contigüe les unes aux autres, selon un ordre et un sens unique et déterminé,

c) une étape de ligation des susdites sutures monobrins cohésives d'au moins 2 nucléotides, à une température de 10 à 40°C pendant une période de 2 à 30 min pour obtenir un vecteur circulaire d'ADN double brin,

les étapes (b) et (c) étant répétées de 1 à 49 fois,
ledit procédé comprenant en outre,

(d) au moins une étape d'incubation à une température de 41 à 60°C pendant une période de 0,5 à 15 min et, éventuellement,
(e) une étape d'incubation à une température de 61 à 90°C pendant une période de 0,5 à 15 minutes.

**2.** Procédé selon la revendication précédente, dans lequel :

- l'étape a) comprend la mise en contact simultanée de n briques moléculaires, chaque brique moléculaire étant une molécule d'ADN linéaire double brin, et contenant :

i. Une séquence d'intérêt (SI)i dépourvue de site de reconnaissance spécifique de la susdite enzyme de restriction de type IIs et comprenant au moins une unité, ladite unité étant une unité fonctionnelle ou une unité non fonctionnelle, ladite unité comprenant au moins un module, ledit module étant un module fonctionnel ou un module non fonctionnel, et
ii. deux adaptateurs d'ADN double brin A(i-1,i) et A(i,i+1), différents l'un de l'autre, flanqués respectivement en amont et en aval de ladite séquence d'intérêt (SI)i, chaque adaptateur d'ADN double brin consistant en une séquence d'au moins 12 nucléotides,

la séquence d'au moins 12 nucléotides de l'adaptateur d'ADN double brin A(i-1,i) contenant

- un seul et unique site de reconnaissance de la sus-dite enzyme de restriction de type IIs, et
- une suture d'au moins 2 nucléotides, s(i-1, i) en aval du site de reconnaissance de ladite enzyme de restriction de type IIs,

la séquence d'au moins 12 nucléotides de l'adaptateur d'ADN double brin A(i,i+1) contenant

- un seul et unique site de reconnaissance de la susdite enzyme de restriction de type IIs, et
- une suture d'au moins 2 nucléotides, s(i,i+1), en amont du site de reconnaissance de ladite enzyme de restriction de type IIs,

le site de reconnaissance de la susdite enzyme de restriction de type IIs de l'adaptateur d'ADN double brin A(i-1,i) en amont de ladite séquence d'intérêt et le site de reconnaissance de la susdite enzyme de restriction de type IIs de l'adaptateur d'ADN double brin A(i,i+1) en aval de ladite séquence spécifique étant convergents,

(SI)1 étant la séquence d'intérêt (SI)i dans laquelle i=1
(SI)n étant la séquence d'intérêt (SI)i dans laquelle i=n

n étant un nombre entier allant de 6 à 100, i allant de 1 à n, i étant différent de n quand i=1,
et quand i=n alors i+1 est 1,
et lorsque i=1, alors i-1=n, de sorte que la suture monobrin cohésive d'au moins 2 nucléotides générée en amont de (SI)1, si-1, est la séquence complémentaire de la suture monobrin cohésive d'au moins 2 nucléotides générée en aval de (SI)n, sn +1,
et lorsque i=n alors la suture monobrin cohésive d'au moins 2 nucléotides générée en aval de (SI)n, sn+1, est la séquence complémentaire de la suture monobrin cohésive d'au moins 2 nucléotides générée en amont de (SI)1, si-1,
- l'étape b) conduit :

- à l'élimination des sites de reconnaissance de l'enzyme de restriction de type IIs utilisée,
- à la formation d'une extrémité cohésive constituée par une suture monobrin d'au moins 2 nucléotides, à chacune des extrémités amont et aval de chacune des (SI)i et
- à l'appariement par complémentarité nucléotidique de la susdite suture monobrin cohésive d'au moins 2 nucléotides en aval de (SI)i avec la suture monobrin cohésive d'au moins 2 nucléotides en amont de la (SI)i+1, et de la susdite suture monobrin cohésive d'au moins 2 nucléotides en amont de la (SI)i avec la susdite suture monobrin cohésive d'au moins 2 nucléotides en aval de (SI)i-1 et
- au positionnement des susdites (SI)i de façon contigüe les unes aux autres, selon un ordre et un sens unique et déterminé.

**3.** Procédé selon l'une quelconque des revendications précédentes dans lequel l'une des au moins six séquences d'intérêt comprend au moins une unité non fonctionnelle, et/ou dans lequel l'enzyme de restriction de type IIs est une enzyme de restriction de type IIs choisie parmi BsaI, Eco31I, BbsI, BpiI, BsmBI, Esp3I, BspMI, BfuAI et BveI, et/ou dans lequel ladite enzyme de restriction de type IIs coupe l'ADN à une distance allant de 2 à 15 nucléotides, 2 à 14, 2 à 13, 2 à 12, 2 à 11, 2 à 10, 2 à 9, 2 à 8, 2 à 7, 2 à 6, 2 à 5, 2 à 4, 2 à 3 nucléotides du site de reconnaissance spécifique de ladite enzyme de type IIs, et/ou
en ce qu'il comprend, avant l'étape de mise en contact simultanée d'au moins six briques moléculaires, une étape de préparation de chacune des briques moléculaires, par synthèse chimique ou par une étape d'amplification par PCR de la séquence d'intérêt contenue dans une brique à l'aide d'une amorce sens comprenant, de 5' en 3', une séquence correspondant à la séquence de l'adaptateur, et au moins 14 nucléotides de la séquence d'intérêt et d'une amorce anti-sens comprenant de 5' en 3' au moins 14 nucléotides de la séquence d'intérêt et au moins une séquence correspondant à la séquence de l'adaptateur.

**4.** Procédé selon l'une quelconque des revendications précédentes dans lequel l'adaptateur d'ADN double brin en aval ou en amont de ladite séquence d'intérêt, comprend au moins un site de reconnaissance d'une enzyme de restriction de type IIp, avantageusement au moins un site de reconnaissance d'une enzyme de restriction rare telles que NotI, PacI, PmeI, SwaI, SmiI, SgsI, SgrDI, SgrAI, SbfI, FseI, AscI, AsiSI, MreI, MssI et plus avantageusement deux sites de reconnaissance d'enzymes de restriction choisis parmi KpnI et AgeI, EcoRI et BstBI, SalI et MluI.

**5.** Procédé selon l'une quelconque des revendications précédentes dans lequel les adaptateurs d'ADN double brin en amont et en aval de ladite séquence d'intérêt ne comprennent aucun site de reconnaissance d'une enzyme de restriction de type IIs autre que celui de l'enzyme de restriction de type IIs, présente dans l'étape de mise en contact simultanée d'au moins deux briques moléculaires, différentes l'une de l'autre, et/ou
dans lequel la suture monobrin cohésive d'au moins 2 nucléotides à chacune des extrémités amont et aval de la séquence d'intérêt, comprend de 2 à 10 nucléotides, de préférence de 2 à 5 nucléotides, et plus particulièrement 4 nucléotides, et/ou
dans lequel chaque suture monobrin cohésive d'au moins 2 nucléotides générée à partir d'une brique moléculaire s'apparie avec une suture monobrin cohésive d'au moins 2 nucléotides générée à partir d'une autre brique moléculaire.

**6.** Procédé selon l'une quelconque des revendications précédentes dans lequel la suture monobrin cohésive d'au moins 2 nucléotides générée à chacune des extrémités amont et aval de la séquence d'intérêt, comprend une séquence de 4' combinaisons possibles excluant les combinaisons qui résultent en un palindrome d'ADN dans laquelle z est compris de 2 à 10 et z est le nombre de nucléotides de la suture mono brin, et/ou
dans lequel la suture mono brin cohésive d'au moins 2 nucléotides en amont et aval de la séquence d'intérêt, est conçue à l'aide d'une matrice de score, et/ou
dans lequel la séquence complémentaire de la suture monobrin cohésive d'au moins 2 nucléotides générée en amont n'est pas complémentaire de la suture monobrin cohésive d'au moins 2 nucléotides générée en aval, à l'exception de la séquence complémentaire de la suture monobrin cohésive d'au moins 2 nucléotides générée en amont de la (SI)1, (si-1), qui est complémentaire de la suture monobrin cohésive d'au moins 2 nucléotides en aval de la séquence (SI)n, (sn+1).

**7.** Procédé selon l'une quelconque des revendications précédentes dans lequel l'étape (a) comprend la mise en contact simultanée d'un mélange équimolaire d'au moins huit briques moléculaires.

## Patentansprüche

**1.** Verfahren zur Herstellung eines zirkulären doppelsträngigen DNA-Vektors, umfassend mindestens sechs Sequenzen von Interesse, umfassend:

a) einen Schritt eines gleichzeitigen Inkontaktbringens einer äquimolaren Mischung von mindestens sechs molekularen Bausteinen, die sich voneinander unterscheiden, in Gegenwart eines einzelnen Restriktionsenzyms vom Typ IIs und einer Ligase, wobei jeder molekulare Baustein ein lineares, doppelsträngiges DNA-Molekül ist und umfasst:

(i) eine Sequenz von Interesse, der eine spezifische Erkennungsstelle für das vorgenannte Restriktionsenzym vom Typ IIs fehlt,
(ii) zwei doppelsträngige DNA-Adapter, die die Sequenz von Interesse stromaufwärts und stromabwärts flankieren, wobei jeder doppelsträngige DNA-Adapter aus einer Sequenz von mindestens 12 Nukleotiden besteht, die eine einzige und einzigartige Erkennungsstelle für das oben genannten Restriktionsenzym vom Typ IIs enthält, wobei die Erkennungsstelle des vorgenannten Restriktionsenzyms vom Typ IIs des Adapters stromaufwärts der Sequenz von Interesse und die Erkennungsstelle des vorgenannten Restriktionsenzyms vom Typ IIs des Adapters stromabwärts der spezifischen Sequenz konvergent sind,

b) einen Schritt einer Inkubation der Mischung von Schritt a) bei einer Temperatur von 20 bis 55 °C für einen Zeitraum von 2 bis 30 min,
wobei der Schritt

- zur Eliminierung durch Schneiden der Erkennungsstellen des verwendeten Restriktionsenzyms vom Typ IIs,
- zur Bildung einer kohäsiven Einzelstrangnaht von mindestens 2 Nukleotiden an jedem Ende der Sequenz von Interesse, wobei die kohäsive Einzelstrangnaht von mindestens 2 Nukleotiden stromaufwärts einer der mindestens sechs Sequenzen von Interesse komplementär zu der kohäsiven Einzelstrangnaht von mindestens 2 Nukleotiden stromabwärts einer anderen Sequenz von Interesse ist,
- zur Paarung der vorgenannten kohäsiven Einzelstrangnähte von mindestens 2 Nukleotiden durch Nukleotidkomplementarität
- zur Positionierung der Sequenzen von Interesse nebeneinander, in einer einzigartigen und bestimmten Reihenfolge und Richtung,

führt
c) einen Schritt einer Ligation der vorgenannten kohäsiven Einzelstrangnähte von mindestens 2 Nukleotiden, bei einer Temperatur von 10 bis 40 °C für einen Zeitraum von 2 bis 30 min um einen zirkulären doppelsträngigen DNA-Vektor zu erhalten,

wobei die Schritte (b) und (c) 1 bis 49 mal wiederholt werden,
wobei das Verfahren ferner umfasst

(d) mindestens einen Inkubationsschritt bei einer Temperatur von 41 bis 60 °C für einen Zeitraum von 0,5 bis 15 min und gegebenenfalls
(e) einen Inkubationsschritt bei einer Temperatur von 61 bis 90 °C für einen Zeitraum von 0,5 bis 15 Minuten.

**2.** Verfahren nach dem vorhergehenden Anspruch, in dem:

- Schritt (a) das gleichzeitige Inkontaktbringen von n molekularen Bausteinen umfasst, wobei jeder molekulare Baustein ein lineares, doppelsträngiges DNA-Molekül ist, und umfasst

(i) eine Sequenz von Interesse (SI)i, der eine spezifische Erkennungsstelle für das vorgenannte Restriktionsenzym vom Typ IIs fehlt und die mindestens eine Einheit umfasst, wobei die Einheit eine funktionelle Einheit oder eine nicht funktionelle Einheit ist, wobei die Einheit mindestens ein Modul umfasst, wobei das Modul ein funktionelles Modul oder ein nicht funktionelles Modul ist, und
(ii) zwei doppelsträngige DNA-Adapter A(i-1, i) und A(i, i+1), die sich voneinander unterscheiden, die die Sequenz von Interesse (SI)i stromaufwärts beziehungsweise stromabwärts flankieren, wobei jeder doppelsträngige DNA-Adapter aus einer Sequenz von mindestens 12 Nukleotiden besteht,

wobei die Sequenz von mindestens 12 Nukleotiden des doppelsträngigen DNA-Adapters A(i-1, i) umfasst

- eine einzelne und einzigartige Erkennungsstelle des vorgenannten Restriktionsenzyms vom Typ IIs und
- eine Naht von mindestens 2 Nukleotiden, s(i-1, i) stromabwärts der Erkennungsstelle des Restriktionsenzyms vom Typ IIs,

wobei die Sequenz von mindestens 12 Nukleotiden des doppelsträngigen DNA-Adapters A(i, i+1) umfasst

- eine einzelne und einzigartige Erkennungsstelle des vorgenannten Restriktionsenzyms vom Typ IIs und
- eine Naht von mindestens 2 Nukleotiden, s(i, i+1) stromaufwärts der Erkennungsstelle des Restriktionsenzyms vom Typ IIs,

wobei die Erkennungsstelle des vorgenannten Restriktionsenzyms vom Typ IIs des doppelsträngigen DNA-Adapters A(i-1, i) stromaufwärts der Sequenz von Interesse und die Erkennungsstelle des vorgenannten Restriktionsenzyms vom Typ IIs des doppelsträngigen DNA-Adapters A(i, i+1) stromabwärts der spezifischen Sequenz konvergent sind,

wobei (SI)1 die Sequenz von Interesse (SI)i ist, in der i = 1
wobei (SI)n die Sequenz von Interesse (SI)i ist, in der i = n

wobei n eine ganze Zahl im Bereich von 6 bis 100 ist, i von 1 bis n läuft, i sich von n unterscheidet, wenn i = 1, und wenn i = n, i+1 dann 1 ist,
und wenn i = 1, dann i-1 = n ist, so dass die kohäsive Einzelstrangnaht von mindestens 2 Nukleotiden, die stromaufwärts von (SI)1, si-1 erzeugt ist, die komplementäre Sequenz der kohäsiven Einzelstrangnaht von mindestens 2 Nukleotiden ist, die stromabwärts von (SI)n, sn+1 erzeugt ist
und wenn i = n, die kohäsive Einzelstrangnaht von mindestens 2 Nukleotiden, die stromabwärts von (SI)n, sn + 1 erzeugt ist, dann die komplementäre Sequenz der kohäsiven Einzelstrangnaht von mindestens 2 Nukleotiden, die stromaufwärts von (SI)1, si-1 erzeugt ist, ist,
- Schritt b)

- zur Eliminierung der Erkennungsstellen des verwendeten Restriktionsenzyms vom Typ IIs,
- zur Bildung eines kohäsiven Endes, das aus einer Einzelstrangnaht von mindestens 2 Nukleotiden besteht, an jedem der Enden stromaufwärts und stromabwärts von jedem der (SI)i und
- zur Paarung der vorgenannten kohäsiven Einzelstrangnaht von mindestens 2 Nukleotiden stromabwärts von (SI)i mit der kohäsiven Einzelstrangnaht von mindestens 2 Nukleotiden stromaufwärts von (SI)i+1 und der vorgenannten kohäsiven Einzelstrangnaht von mindestens 2 Nukleotiden stromaufwärts von (SI)i mit der vorgenannten kohäsiven Einzelstrangnaht von mindestens 2 Nukleotiden stromabwärts von (SI)i-1 durch Nukleotidkomplementarität und
- zur Positionierung der vorgenannten (SI)i nebeneinander in einer einzigartigen und bestimmten Reihenfolge und Richtung,

führt.

**3.** Verfahren nach einem der vorhergehenden Ansprüche, wobei eine der mindestens sechs Sequenzen von Interesse mindestens eine nicht funktionelle Einheit umfasst und/oder wobei das Restriktionsenzym vom Typ IIs ein Restriktionsenzym vom Typ IIs ist, ausgewählt aus Bsal, Eco31I, Bbsl, Bpil, BsmBI, Esp3I, BspMI, BfuAI und Bvel und/oder wobei das Restriktionsenzym vom Typ IIs die DNA in einem Abstand der von 2 bis 15 Nukleotiden, 2 bis 14, 2 bis 13, 2 bis 12, 2 bis 11, 2 bis 10, 2 bis 9, 2 bis 8, 2 bis 7, 2 bis 6, 2 bis 5, 2 bis 4, 2 bis 3 Nukleotiden von der spezifischen Erkennungssequenz des Enzyms vom Typ IIs reicht, schneidet und/oder
wobei es, vor dem Schritt eines gleichzeitigen Inkontaktbringen von mindestens sechs molekularen Bausteinen, einen Schritt eines Herstellens jedes der molekularen Bausteine durch chemische Synthese oder durch einen Schritt einer Amplifikation mittels PCR der Sequenz von Interesse, die in einem Baustein enthalten ist, unter Verwendung eines Sense-Primers, umfassend von 5’ nach 3’ eine Sequenz, die der Sequenz des Adapters entspricht, und mindestens 14 Nukleotide der Sequenz von Interesse, und eines Antisense-Primers, umfassend von 5’ nach 3’ mindestens 14 Nukleotide der Sequenz von Interesse und mindestens eine Sequenz, die der Sequenz des Adapters entspricht, umfasst.

**4.** Verfahren nach einem der vorhergehenden Ansprüche, wobei der doppelsträngige DNA-Adapter stromabwärts oder stromaufwärts der Sequenz von Interesse mindestens eine Erkennungsstelle eines Restriktionsenzyms vom Tpy IIp umfasst, vorteilhafterweise mindestens eine Erkennungsstelle eines seltenen Restriktionsenzyms wie NotI, PacI, PmeI, SwaI, SmiI, SgsI, SgrDI, SgrAI, SbfI, FseI, AscI, AsiSI, MreI, MssI und noch vorteilhafterweise zwei Erkennungsstellen der Restriktionsenzyme, ausgewählt aus KpnI und AgeI, EcoRI und BstBI, SalI und MluI.

**5.** Verfahren nach einem der vorhergehenden Ansprüche, wobei die doppelsträngigen DNA-Adapter stromaufwärts und stromabwärts der Sequenz von Interesse keine andere Erkennungsstelle eines Restriktionsenzyms vom Typ IIs als die des Restriktionsenzyms vom Typ IIs, die im Schritt des gleichzeitigen Inkontaktbringens von mindestens zwei voneinander unterschiedlichen molekularen Bausteinen vorhanden ist, umfasst, und/oder
wobei die kohäsive Einzelstrangnaht von mindestens 2 Nukleotiden an jedem der Enden stromaufwärts und stromabwärts der Sequenz von Interesse 2 bis 10 Nukleotide, vorzugsweise 2 bis 5 Nukleotide, und insbesondere 4 Nukleotide umfasst und/oder
wobei sich jede kohäsive Einzelstrangnaht von mindestens 2 Nukleotiden, die aus einem molekularen Baustein gebildet ist, mit einer kohäsiven Einzelstrangnaht von mindestens 2 Nukleotiden, die aus einem anderen molekularen Baustein gebildet ist, paart.

**6.** Verfahren nach einem der vorhergehenden Ansprüche, wobei die kohäsive Einzelstrangnaht von mindestens 2 Nukleotiden, die an jedem der Enden stromaufwärts und stromabwärts der Sequenz von Interesse gebildet ist, eine Sequenz von 4' möglichen Kombinationen mit Ausnahme der Kombinationen, die zu einem DNA-Palindrom führen, umfasst, wobei z im Bereich von 2 bis 10 liegt und z die Anzahl der Nukleotide der Einzelstrangnaht ist, und/oder
wobei die kohäsive Einzelstrangnaht von mindestens 2 Nukleotiden stromaufwärts und stromabwärts der Sequenz von Interesse unter Verwendung einer Punktematrix konzipiert ist und/oder
wobei die Sequenz, die zu der kohäsiven Einzelstrangnaht von mindestens 2 Nukleotiden, die stromaufwärts erzeugt ist, komplementär ist, nicht komplementär zu der kohäsiven Einzelstrangnaht von mindestens 2 Nukleotiden, die stromabwärts erzeugt ist, ist, mit Ausnahme der Sequenz, die zu der kohäsiven Einzelstrangnaht von mindestens 2 Nukleotiden komplementär ist, die stromaufwärts von (SI)1, (si-1) erzeugt ist, die zu der kohäsiven Einzelstrangnaht von mindestens 2 Nukleotiden stromabwärts der Sequenz (SI)n, (sn+1) komplementär ist.

**7.** Verfahren nach einem der vorhergehenden Ansprüche, wobei der Schritt (a) das gleichzeitige Inkontaktbringen einer äquimolaren Mischung von mindestens acht molekularen Bausteinen umfasst.

## Claims

**1.** A method for producing a circular double-stranded DNA vector comprising at least six sequences of interest, said method comprising:

(a) a step of simultaneously contacting at equimolar ratio at least six molecular building blocks which are different from one another, in the presence of a single type IIs restriction enzyme and a ligase, each molecular building block being a linear double-stranded DNA molecule and containing:

(i) a sequence of interest with no specific recognition site of the aforementioned type IIs restriction enzyme,
(ii) two double-stranded DNA adapters, flanked upstream and downstream of said sequence of interest, each double-stranded DNA adapter consisting of a sequence of at least 12 nucleotides, which sequence contains: a single and only recognition site of the aforementioned type IIs restriction enzyme, the recognition site of the aforementioned type IIs restriction enzyme of the adapter upstream of said sequence of interest and the recognition site of the aforementioned type IIs restriction enzyme of the adapter downstream of said specific sequence being convergent;

(b) a step of incubation of the mixture obtained in step a) at a temperature ranging from 20°C to a temperature of 55°C, during a period ranging from 2 minutes to a period of 30 minutes, which step leads:

- to the elimination by cleaving of the recognition sites of the type IIs restriction enzyme used,
- to the formation of a cohesive single-stranded suture of at least 2 nucleotides at each of the ends of said sequence of interest, said cohesive single-stranded suture of at least 2 nucleotides upstream of one of the at least six sequences of interest being complementary to said cohesive single-stranded suture of at least 2 nucleotides downstream of another sequence of interest,
- to the pairing by nucleotide complementarity of the aforementioned cohesive single-stranded sutures of at least 2 nucleotides, and
- to the positioning of the sequences of interest contiguously with one another in an order and a single and defined direction;

(c) a step of ligation of the aforementioned cohesive single-stranded sutures of at least 2 nucleotides said step being performed at a temperature ranging from 10°C to 40°C during a period ranging from 2 min to 30 min, to obtain a circular double-stranded DNA vector;

said step (b) and (c) being repeated from 1 to 49 times;
said method also comprising:

(d) at least one step of incubation at a temperature from 41°C to 60°C during a period ranging from 0.5 min to 15 min and, eventually,
(e) a step of incubating at a temperature from 61°C to 90°C during a period ranging from 0.5 min to 15 minutes.

**2.** The method according to the preceding claim, wherein:

- step (a) comprises simultaneously contacting n molecular building blocks, each molecular building block being a linear double-stranded DNA molecule and containing:

(i) a sequence of interest (SI)i with no specific recognition site of the aforementioned type IIs restriction enzyme and comprising at least one unit, said unit being a functional unit or a non-functional unit, said unit comprising at least one module, said module being a functional module or a non-functional module, and
(ii) two double-stranded DNA adapters A(i-1,i) and A(i,i+1), which are different from one another, flanked respectively upstream and downstream of said sequence of interest (SI)i, each double-stranded DNA adapter consisting of a sequence of at least 12 nucleotides,

the sequence of at least 12 nucleotides of the double-stranded DNA adapter A(i-1,i) containing

- a single and only recognition site of the aforementioned type IIs restriction enzyme, and
- a suture of at least 2 nucleotides, s(i-1, i) downstream of the recognition site of said type IIs restriction enzyme,

the sequence of at least 12 nucleotides of the double-stranded DNA adapter A(i,i+1) containing

- a single and only recognition site of the aforementioned type IIs restriction enzyme, and
- a suture of at least 2 nucleotides, s(i,i+1) upstream of the recognition site of said type IIs restriction enzyme,

the recognition site of the aforementioned type IIs restriction enzyme of the double-stranded DNA adapter A(i-1,i) upstream of said sequence of interest and the recognition site of the aforementioned type IIs restriction enzyme of the double-stranded DNA adapter A(i,i+1) downstream of said specific sequence being convergent,

(SI)1 being the sequence of interest (SI)i in which i=1
(SI)n being the sequence of interest (SI)i in which i=n

n being an integer ranging from 6 to 100, i ranging from 1 to n, i being different from n when i=1 and when i=n then i+1 is 1,
and when i=1, then i-1=n, such that the cohesive single-stranded suture of at least 2 nucleotides produced upstream of (SI)1, si-1, is the complementary sequence of the cohesive single-stranded suture of at least 2 nucleotides produced downstream of (SI)n, sn+1,
and when i=n then the cohesive single-stranded suture of at least 2 nucleotides produced downstream of (SI)n, sn+1, is the complementary sequence of the cohesive single-stranded suture of at least 2 nucleotides produced upstream of (SI)1, si-1,
- step (b) leads:

- to the elimination of the recognition sites of the type IIs restriction enzyme used,
- to the formation of a cohesive end formed by a single-stranded suture of at least 2 nucleotides at each of the ends upstream and downstream of each of the (SI)i and,
- to the pairing by nucleotide complementarity of the aforementioned cohesive single-stranded suture of at least 2 nucleotides downstream of (SI)i with the cohesive single-stranded suture of at least 2 nucleotides upstream of the (SI)i+1, and of the aforementioned cohesive single-stranded suture of at least 2 nucleotides upstream of the (SI)i with the aforementioned cohesive single-stranded suture of at least 2 nucleotides downstream of (SI)i-1, and
- to the positioning of the aforementioned (SI)i contiguously with one another in an order and a single and defined direction.

3. The method according to any one of the preceding claims, wherein at least six sequences of interest comprises at least one non-functional unit, and/or wherein the type IIs restriction enzyme is a type IIs restriction enzyme selected from BsaI, Eco31I, BbsI, BpiI, BsmBI, Esp3I, BspMI, BfuAI and BveI, and/or wherein said type IIs restriction enzyme cleaves the DNA at a distance ranging from 2 to 15 nucleotides, 2 to 14, 2 to 13, 2 to 12, 2 to 11, 2 to 10, 2 to 9, 2 to 8, 2 to 7, 2 to 6, 2 to 5, 2 to 4 or 2 to 3 nucleotides from the specific recognition site of said type IIs enzyme, and/or in that it comprises before the step of simultaneously contacting at least six molecular building blocks, a step of preparing each of the molecular building blocks by chemical synthesis or by a step of amplification by PCR of the sequence of interest contained in a building block with the aid of a forward primer comprising, from 5' to 3', a sequence corresponding to the sequence of the adapter and at least 14 nucleotides of the sequence of interest, and a reverse primer comprising, from 5' to 3', at least 14 nucleotides of the sequence of interest and at least one sequence corresponding to the sequence of the adapter.

4. The method according to any one of the preceding claims, wherein the double-stranded DNA adapter, downstream or upstream of said sequence of interest, comprises at least one site of recognition of a type IIp restriction enzyme, advantageously at least one site of recognition of a rare restriction enzyme such as NotI, PacI, PmeI, SwaI, SmiI, SgsI, SgrDI, SgrAI, SbfI, FseI, AscI, AsiSI, MreI, MssI, and more advantageously two sites of recognition of a restriction enzyme chosen from KpnI et AgeI, EcoRI et BstBI, SalI, and MluI.

5. The method according to any one of the preceding claims, wherein the double-stranded DNA adapters upstream and downstream of said sequence of interest do not have a site of recognition of a type IIs restriction enzyme other than that of the type IIs restriction enzyme present in the step of simultaneously contacting at least six molecular building blocks, which are different from one another, and/or
wherein the cohesive single-stranded suture of at least 2 nucleotides at each of the ends upstream and downstream of the sequence of interest comprises 2 to 10 nucleotides, preferably 2 to 5, and more particularly 4 nucleotides, and/or wherein each cohesive single-stranded suture of at least 2 nucleotides produced from a molecular building block pairs with a cohesive single-stranded suture of at least 2 nucleotides produced from another molecular building block.

6. The method according to any one of the preceding claims, wherein the cohesive single-stranded suture of at least 2 nucleotides produced at each of the ends downstream and upstream of the sequence of interest comprises a sequence of $4^z$ possible combinations excluding the combinations which result in a DNA palindrome, in which z is between 2 and 10 and z is the number of nucleotides of the single-stranded suture, and/or
wherein the cohesive single-stranded suture of at least 2 nucleotides downstream and upstream of the sequence of interest is designed using a scoring matrix, and/or
wherein the complementary sequence of the cohesive single-stranded suture of at least 2 nucleotides produced upstream is not complementary to the cohesive single-stranded suture of at least 2 nucleotides produced downstream, with the exception of the complementary sequence of the cohesive single-stranded suture of at least 2 nucleotides produced upstream of the (SI)1, (si-1), which is complementary to the cohesive single-stranded suture of at least 2 nucleotides downstream of the sequence (SI)n, (sn+1).

7. The method according to any one of the preceding claims, wherein step (a) comprises the simultaneously contacting of an equimolar ratio of at least eight molecular building blocks.

A

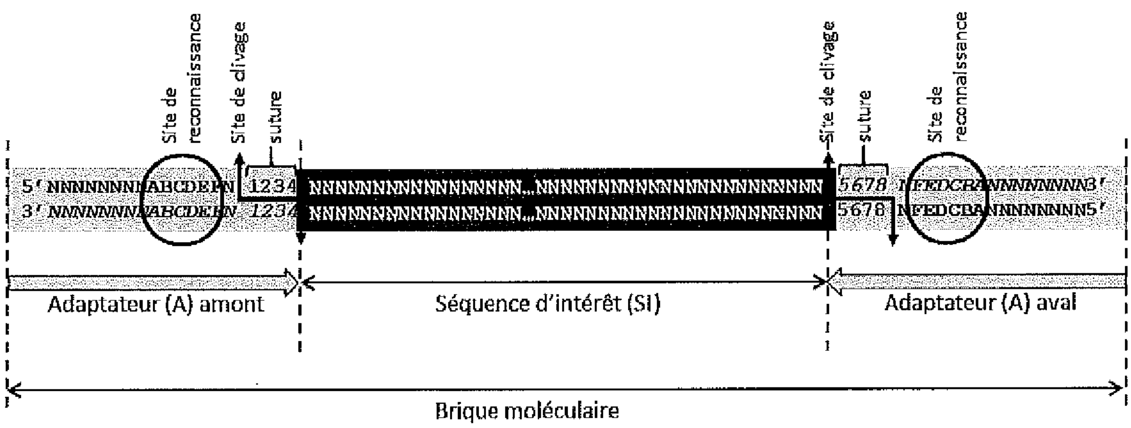
Site de reconnaissance
Site de clivage
suture
5' NNNNNNNNABCDEFN 1234 NNNNNNNNNNNNNNNNNN...NNNNNNNNNNNNNNNNNNNNNNNN 5678 NFEDCBANNNNNNNN3'
3' NNNNNNNNABCDEFN 1234 NNNNNNNNNNNNNNNNNN...NNNNNNNNNNNNNNNNNNNNNNNN 5678 NFEDCBANNNNNNNN5'
Adaptateur (A) amont
Séquence d'intérêt (SI)
Adaptateur (A) aval
Brique moléculaire

B

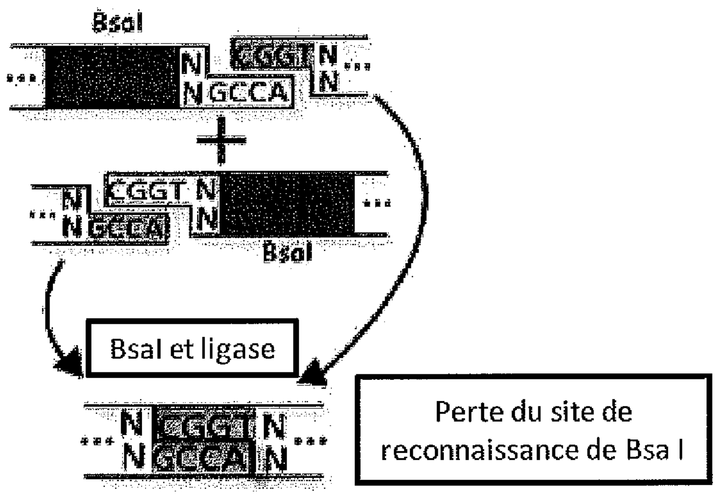
BsaI
CGGT
GCCA
BsaI et ligase
Perte du site de reconnaissance de Bsa I

Figure 1

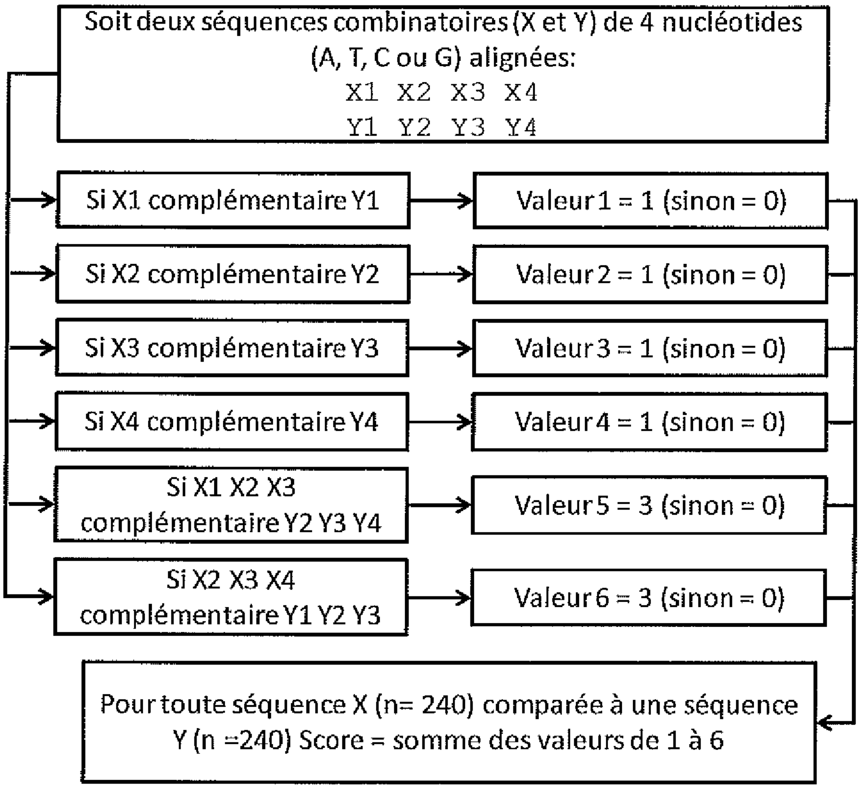


Figure 2

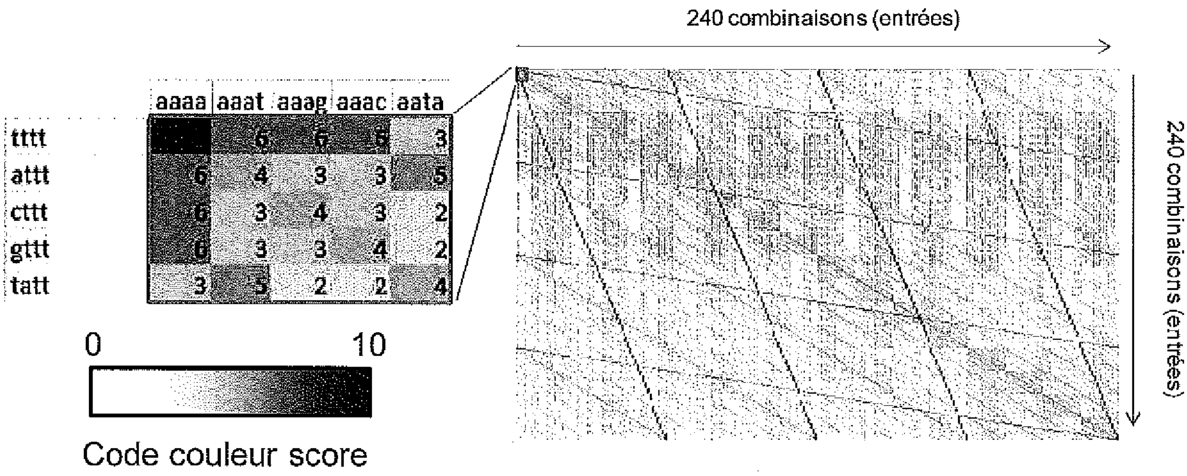


Figure 3

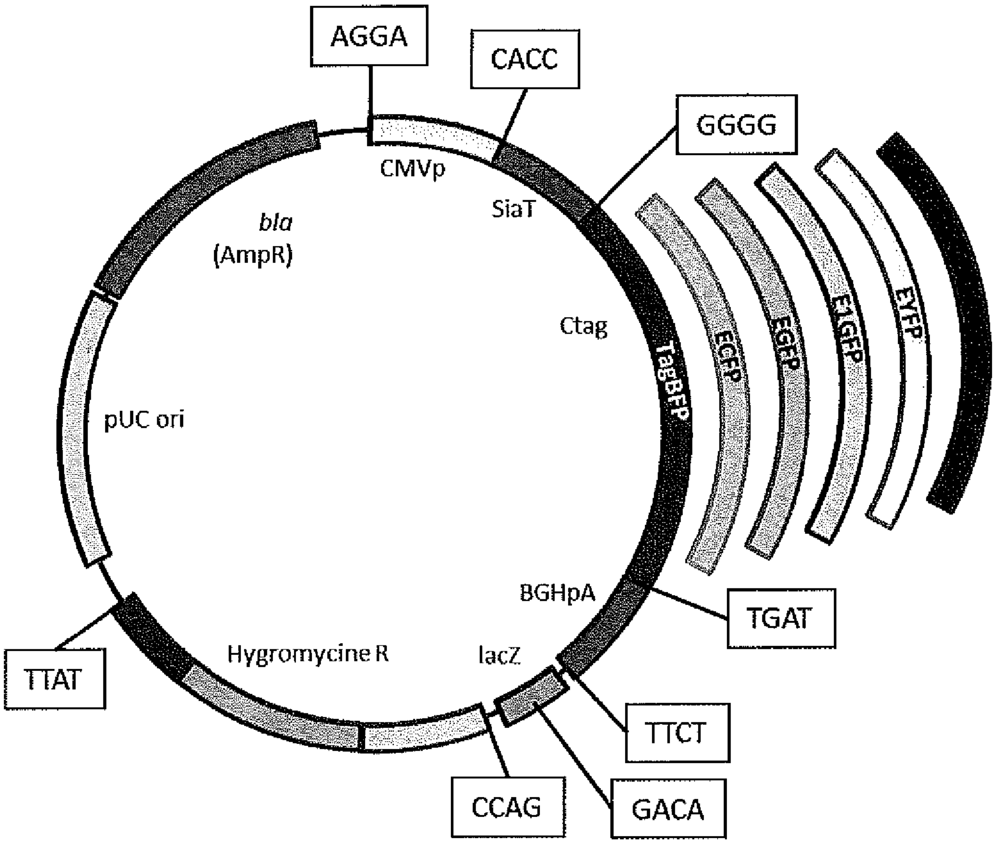


Figure 4

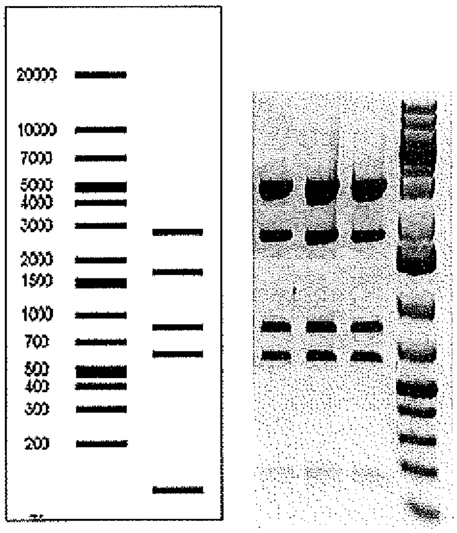


Figure 5

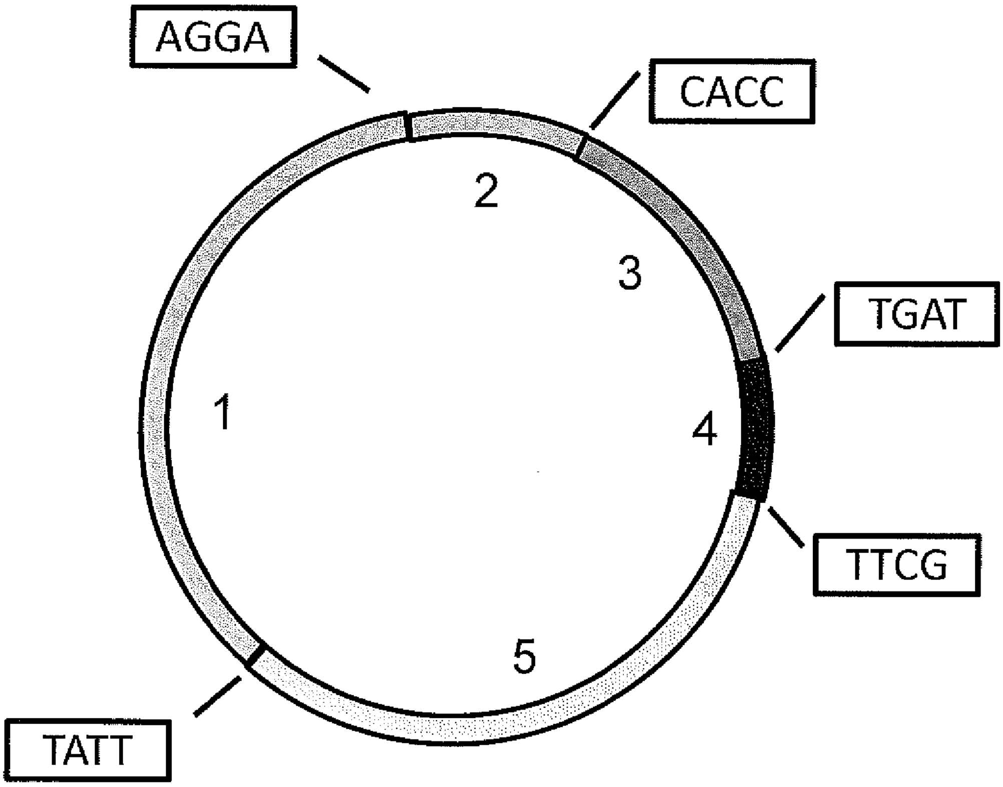


Figure 6

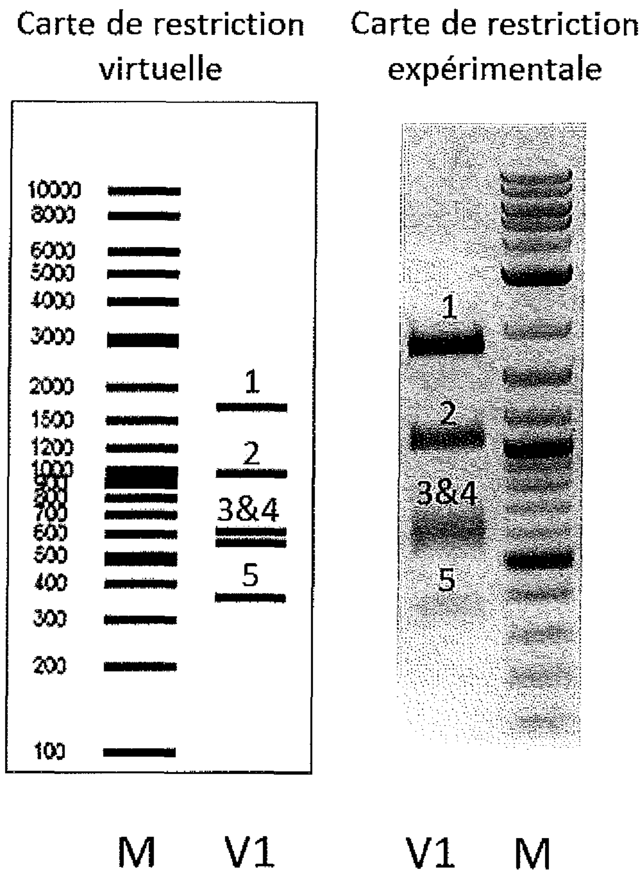
Carte de restriction virtuelle
Carte de restriction expérimentale
10000
8000
6000
5000
4000
3000
2000
1500
1200
1000
700
600
500
400
300
200
100
1
2
3&4
5
M
V1
V1
M

Figure 7

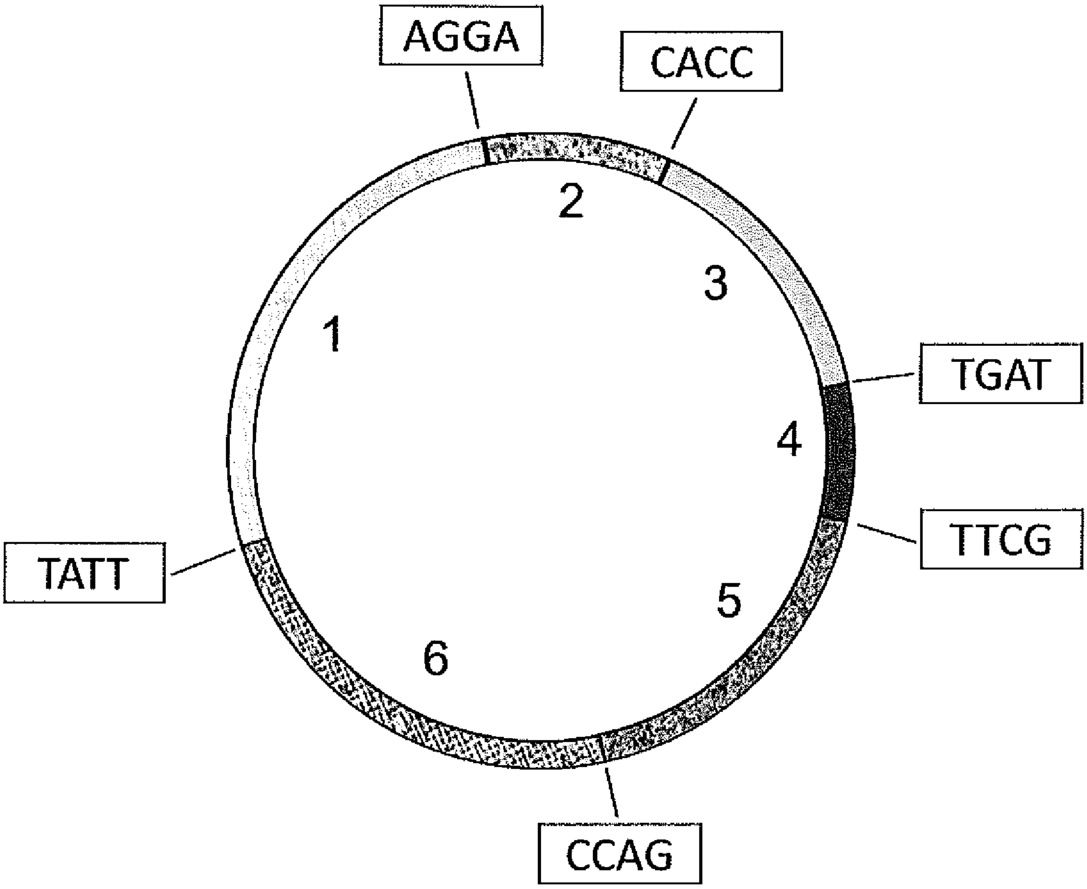


Figure 8

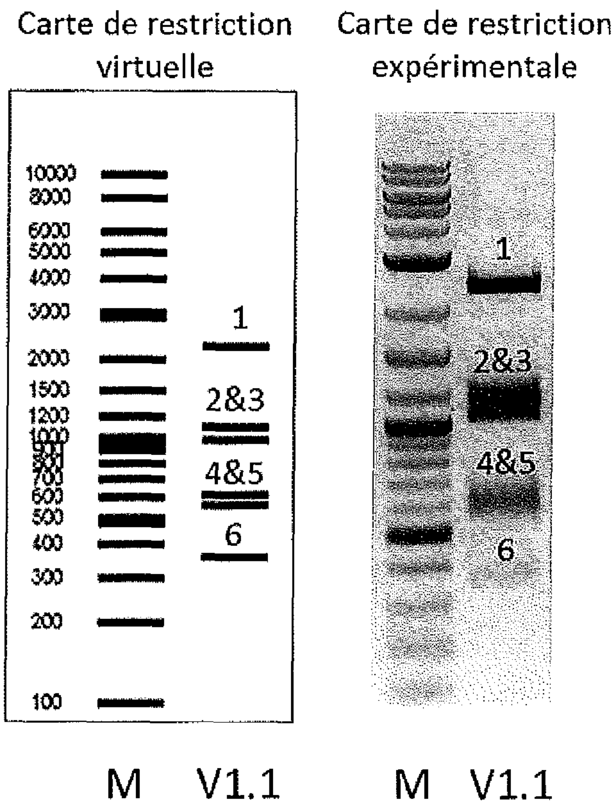


Figure 9

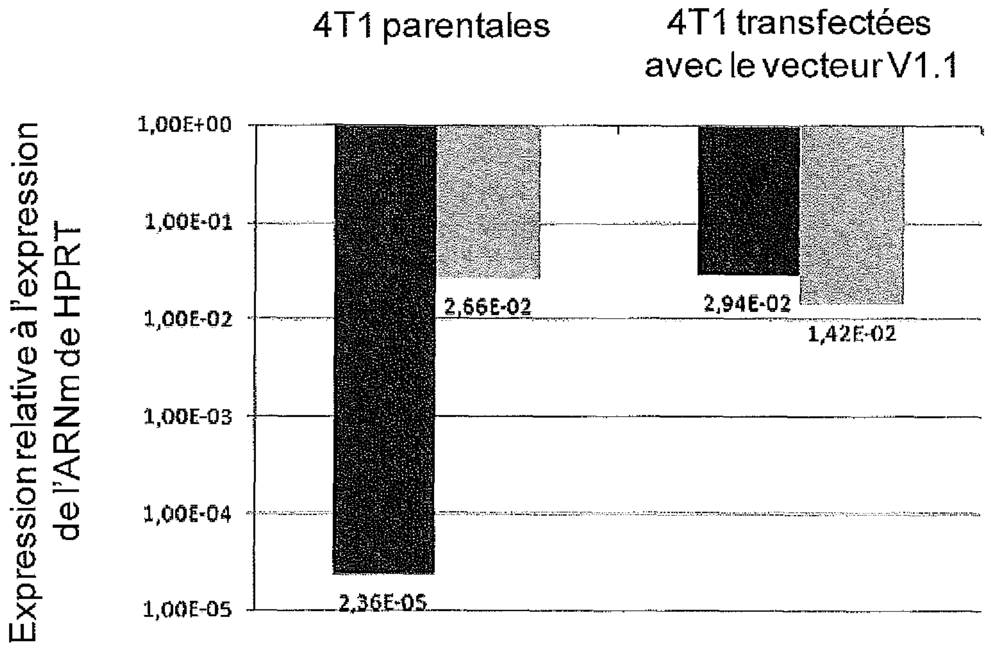


Figure 10

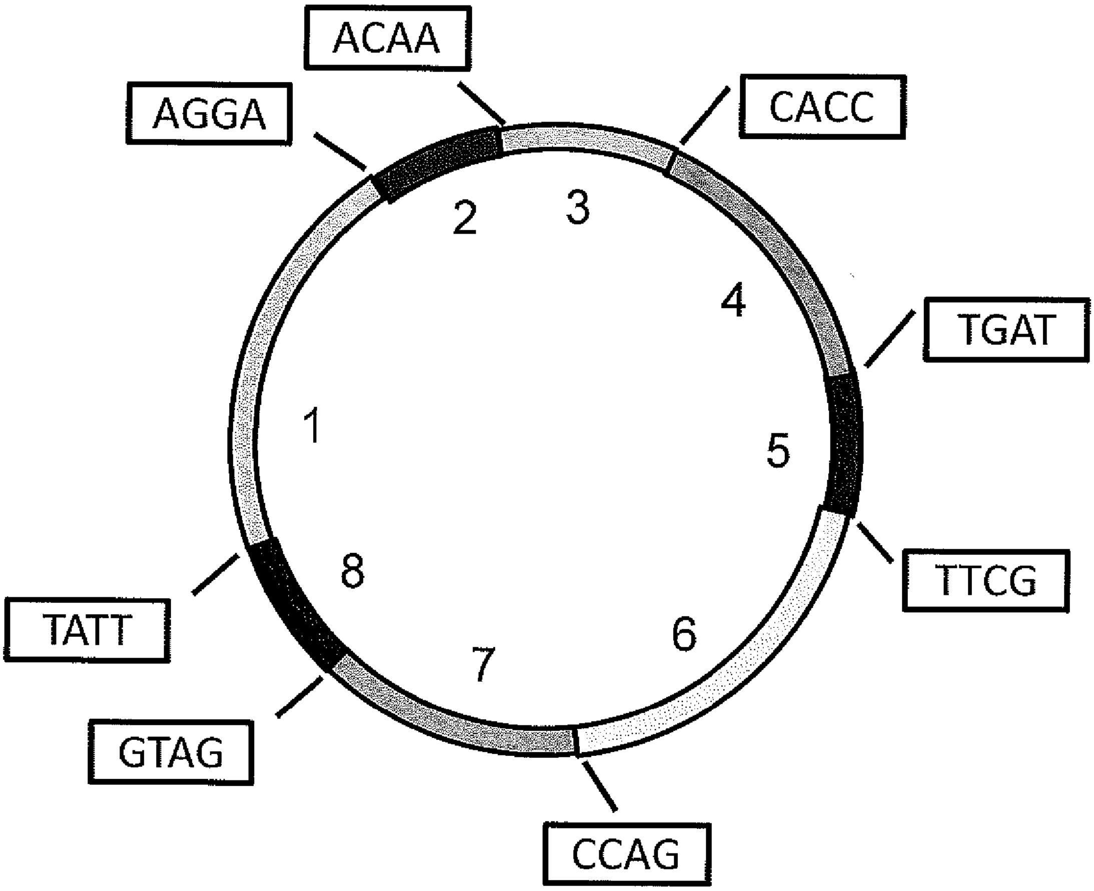


Figure 11

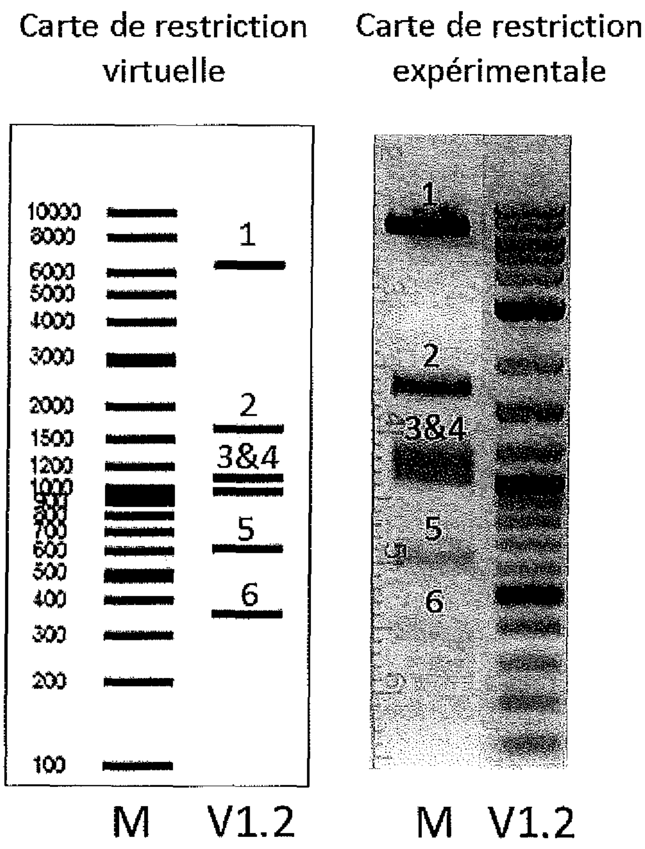
Carte de restriction virtuelle
Carte de restriction expérimentale
10000
8000
6000
5000
4000
3000
2000
1500
1200
1000
900
800
700
600
500
400
300
200
100
1
2
3&4
5
6
M
V1.2
M
V1.2

**Figure 12**

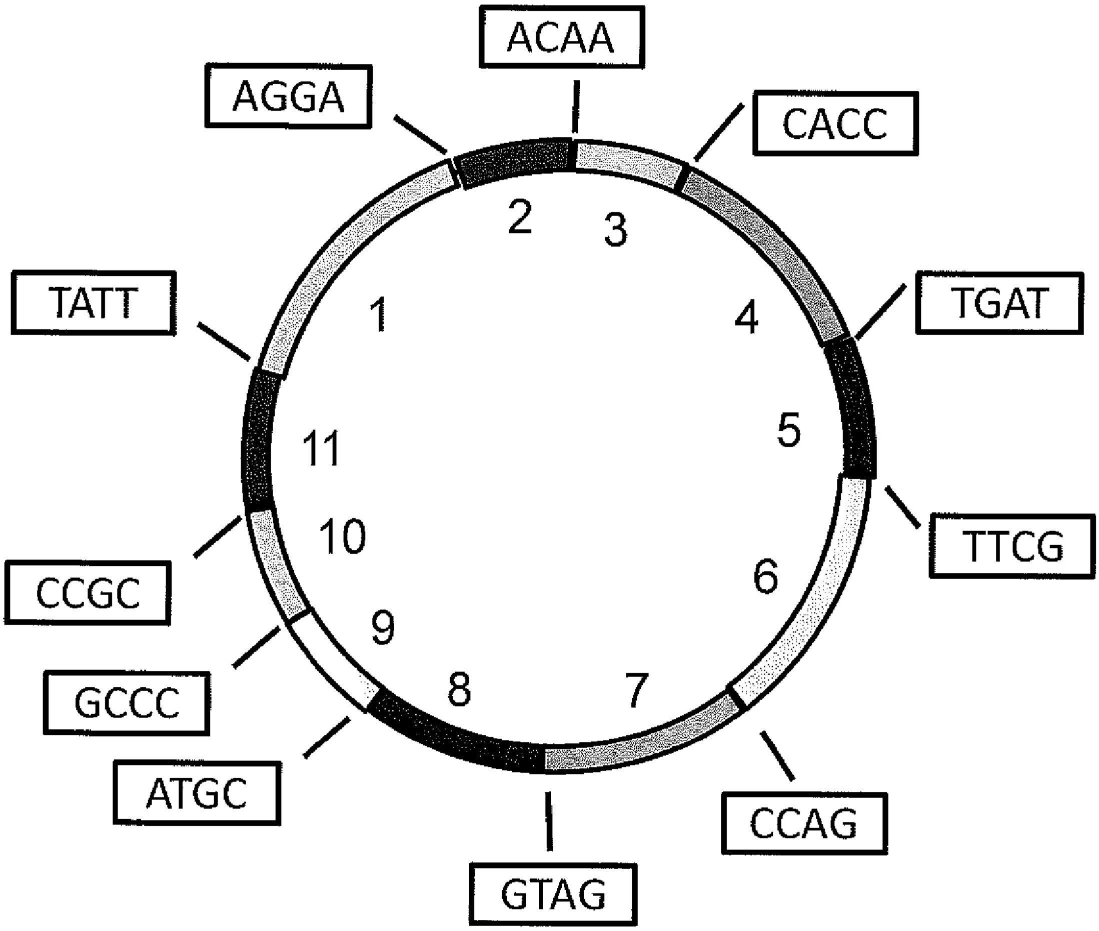
ACAA
CACC
AGGA
2
3
TATT
1
4
TGAT
5
11
TTCG
10
CCGC
6
9
GCCC
8
7
ATGC
CCAG
GTAG

Figure 13

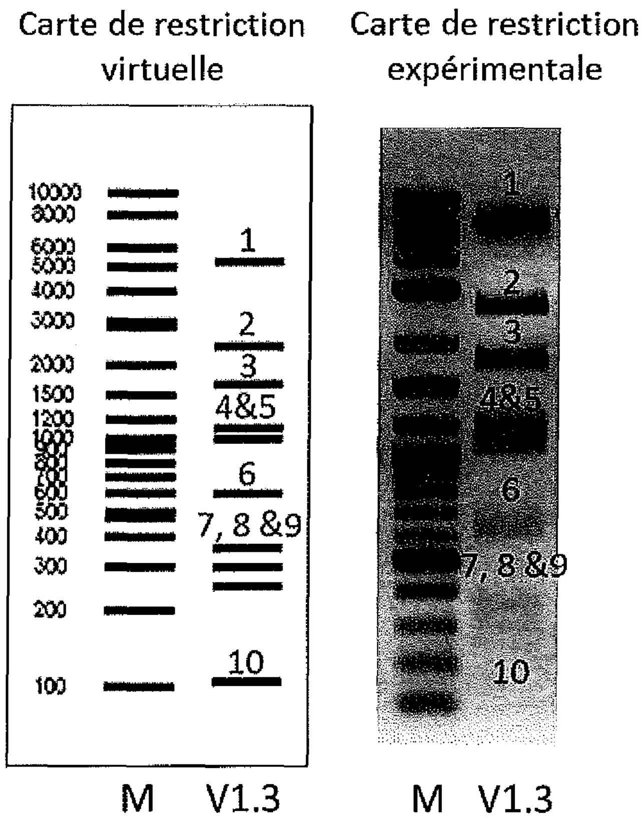
Carte de restriction virtuelle
Carte de restriction expérimentale
10000
8000
6000
5000
4000
3000
2000
1500
1200
1000
400
300
200
100
1
2
3
4&5
6
7, 8 &9
10
M
V1.3
M
V1.3

Figure 14

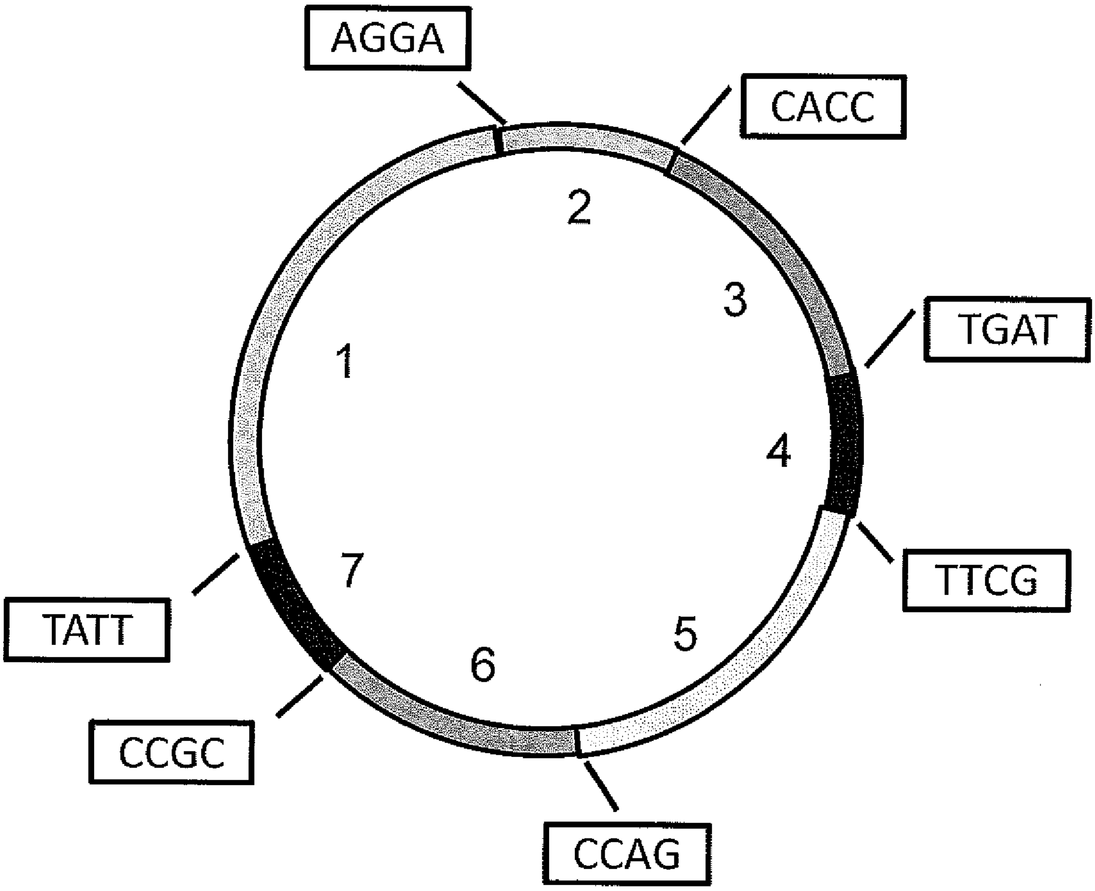


Figure 15

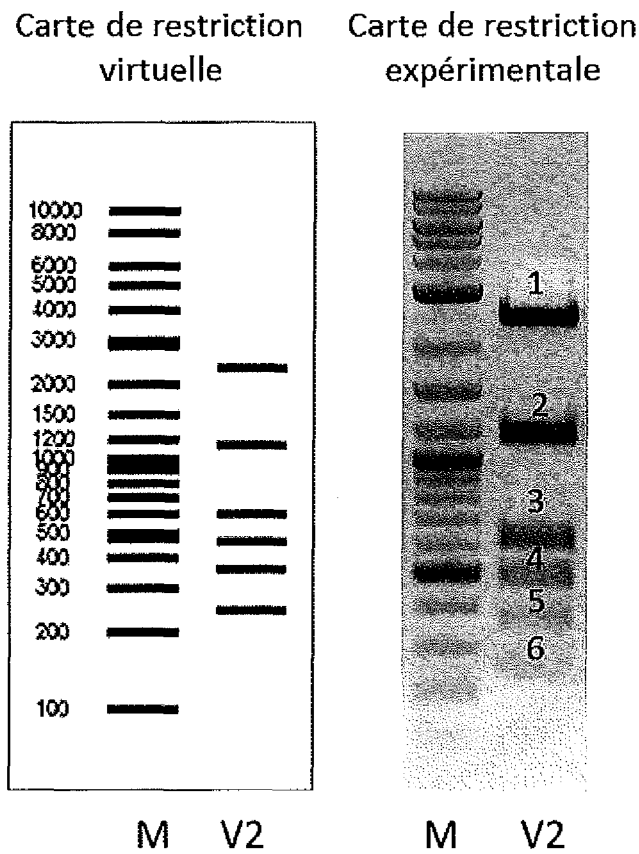
Carte de restriction virtuelle
Carte de restriction expérimentale
10000
8000
6000
5000
4000
3000
2000
1500
1200
1000
500
400
300
200
100
1
2
3
4
5
6
M
V2
M
V2

Figure 16

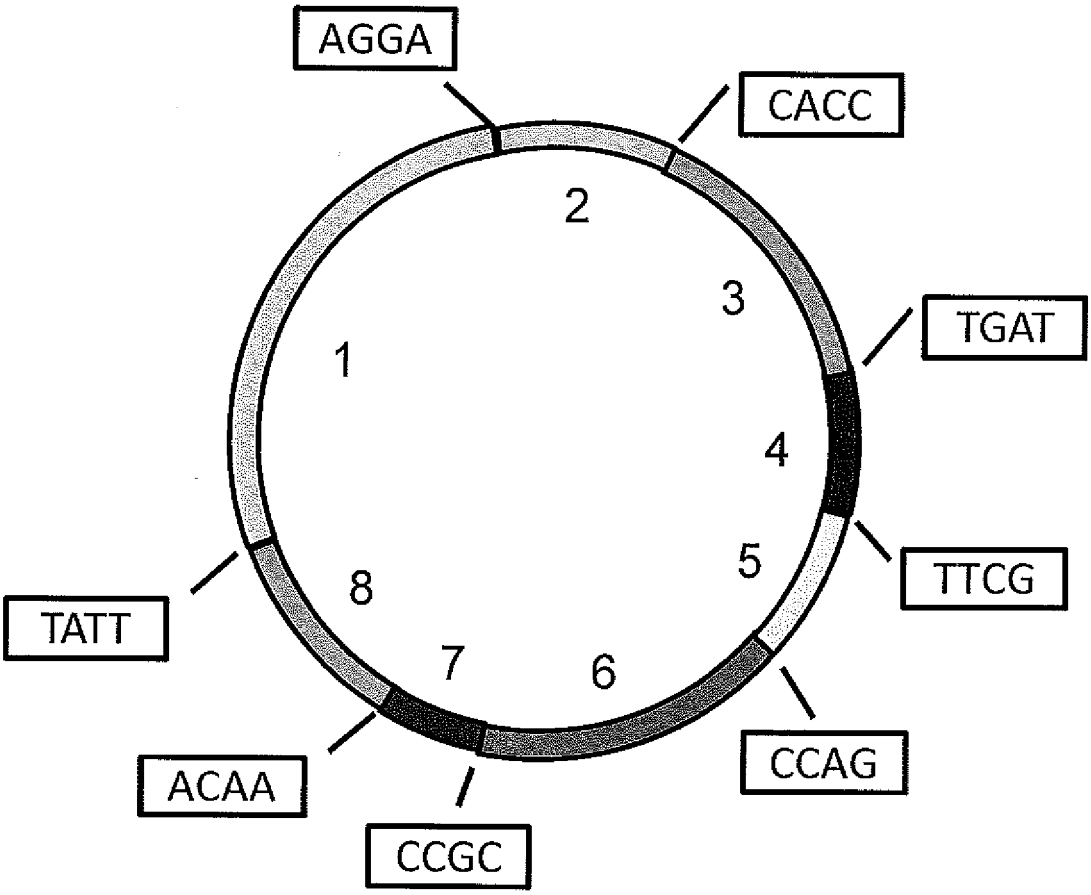


Figure 17

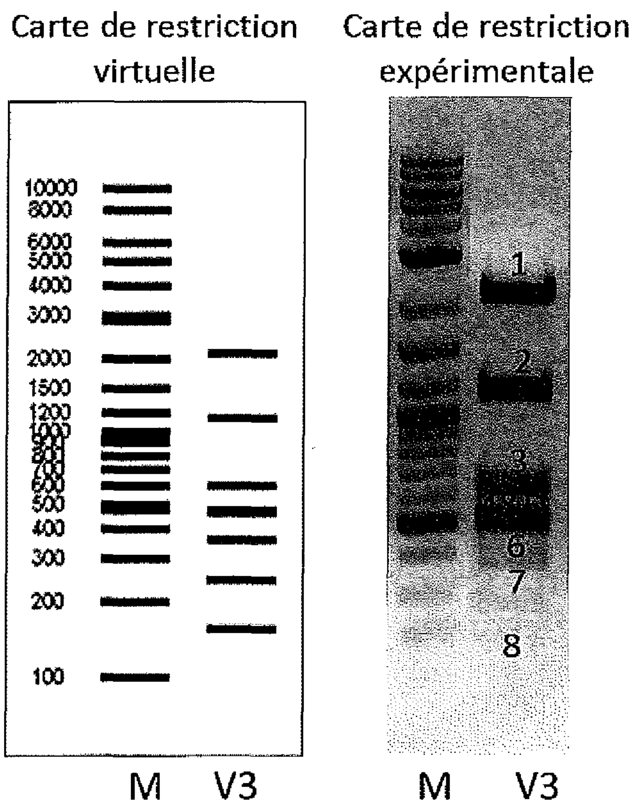
Carte de restriction virtuelle
Carte de restriction expérimentale
10000
8000
6000
5000
4000
3000
2000
1500
1200
1000
300
200
100
1
2
3
6
7
8
M
V3
M
V3

Figure 18

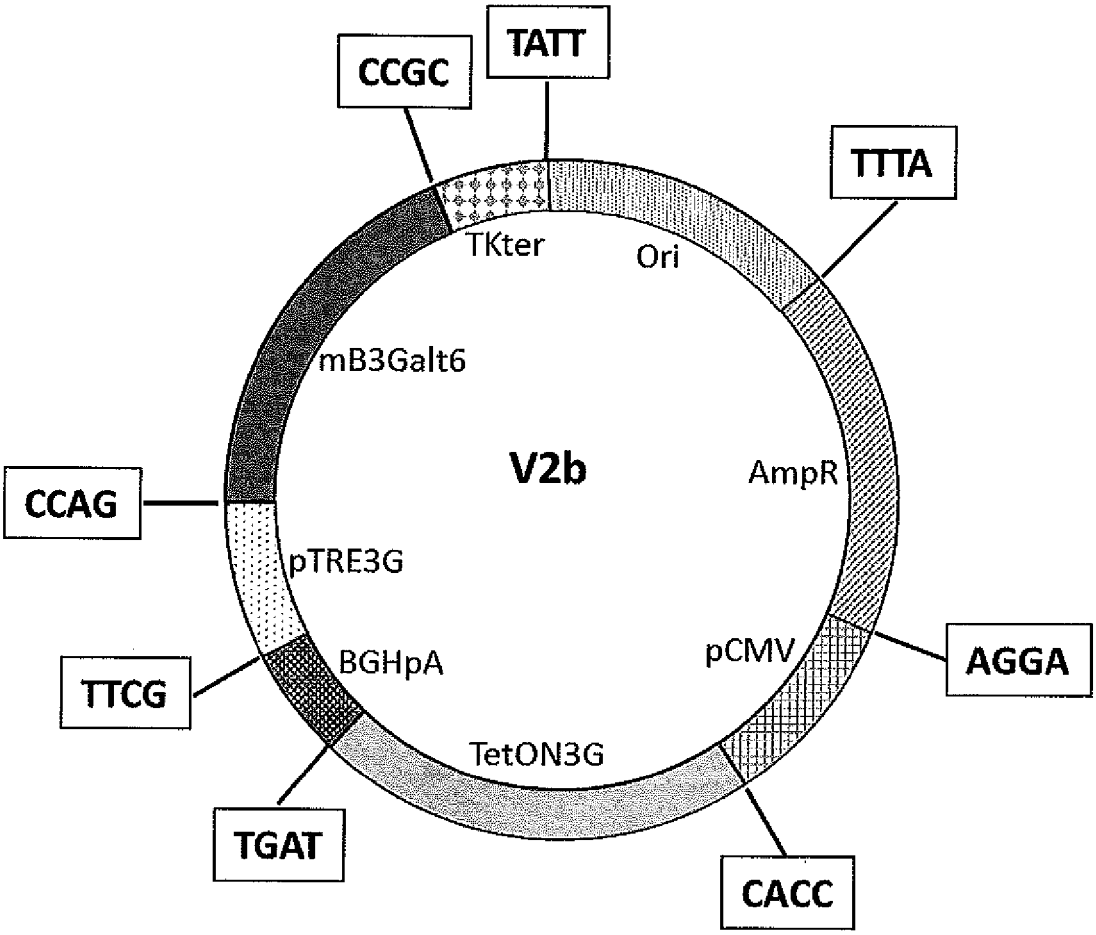
TATT
CCGC
TTTA
TKter
Ori
mB3Galt6
V2b
AmpR
CCAG
pTRE3G
AGGA
pCMV
TTCG
BGHpA
TetON3G
TGAT
CACC

Figure 19

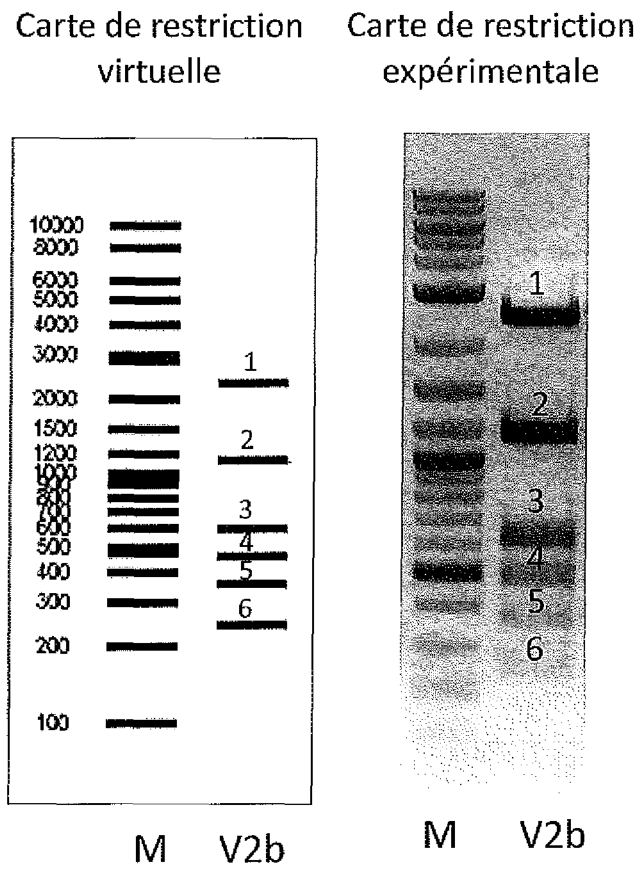


Figure 20

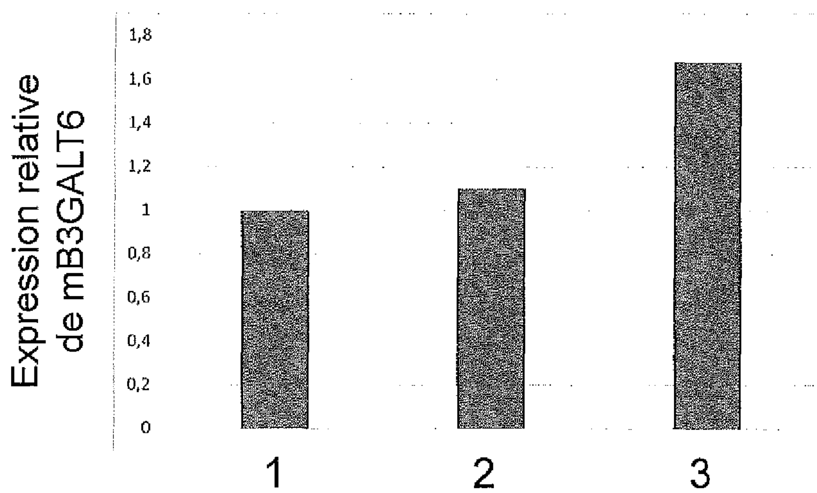


Figure 21

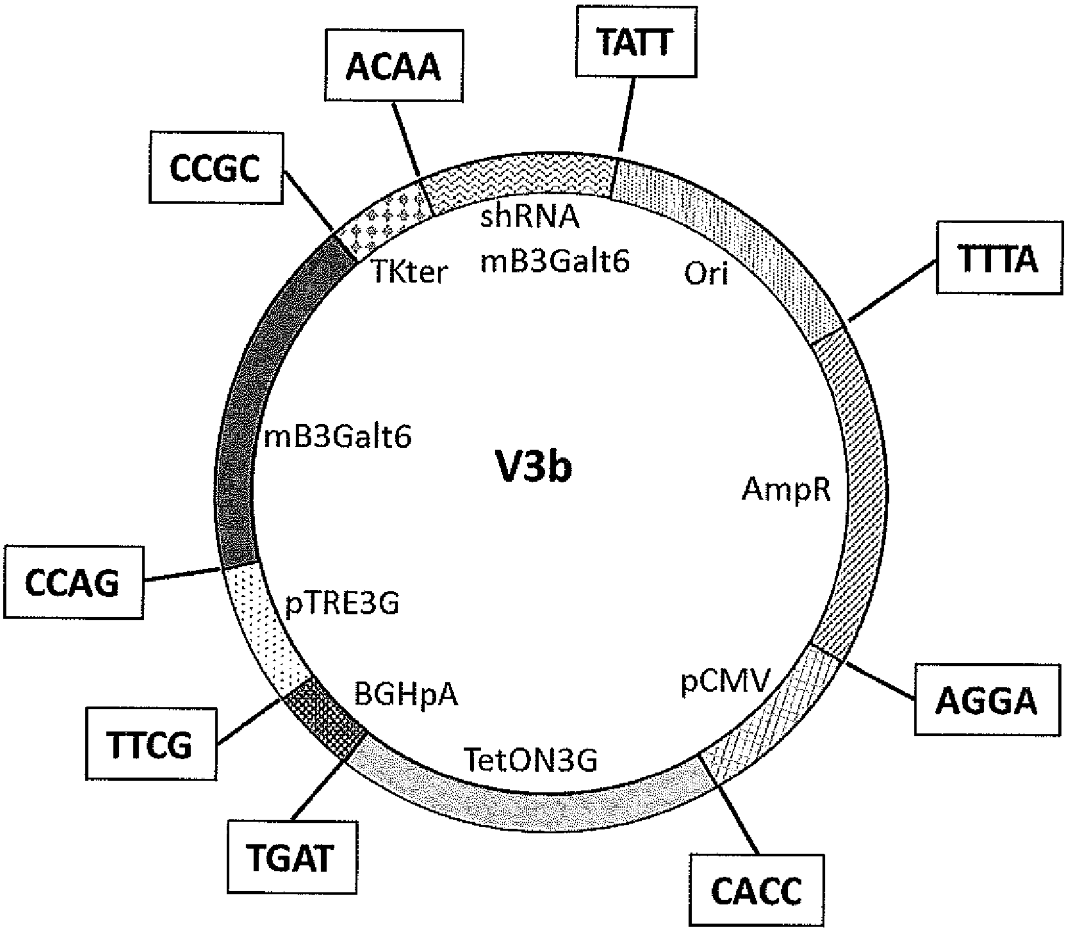
TATT
ACAA
CCGC
TTTA
shRNA
mB3Galt6
TKter
Ori
mB3Galt6
V3b
AmpR
CCAG
pTRE3G
pCMV
AGGA
BGHpA
TTCG
TetON3G
TGAT
CACC

Figure 22

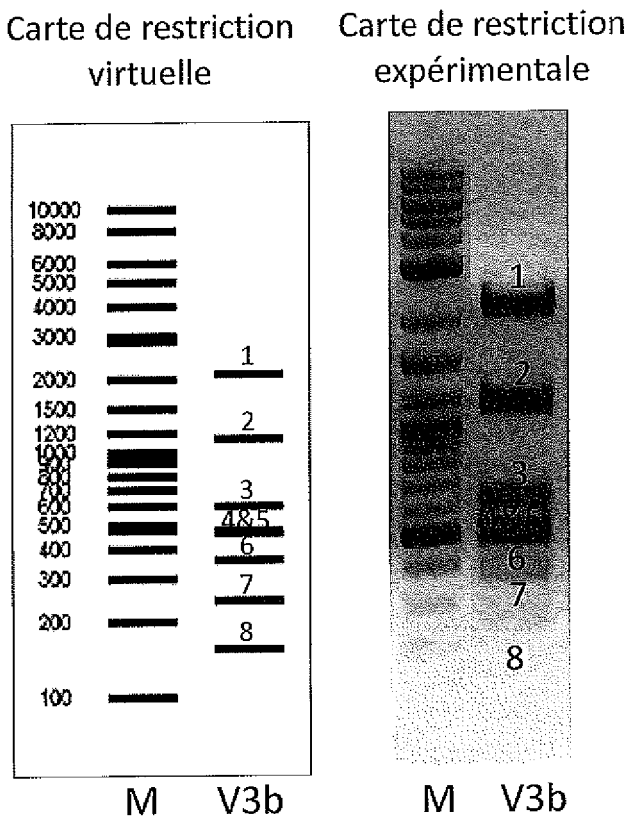
Carte de restriction virtuelle
Carte de restriction expérimentale
10000
8000
6000
5000
4000
3000
2000
1500
1200
1000
400
300
200
100
1
2
3
4&5
6
7
8
M
V3b
M
V3b

Figure 23

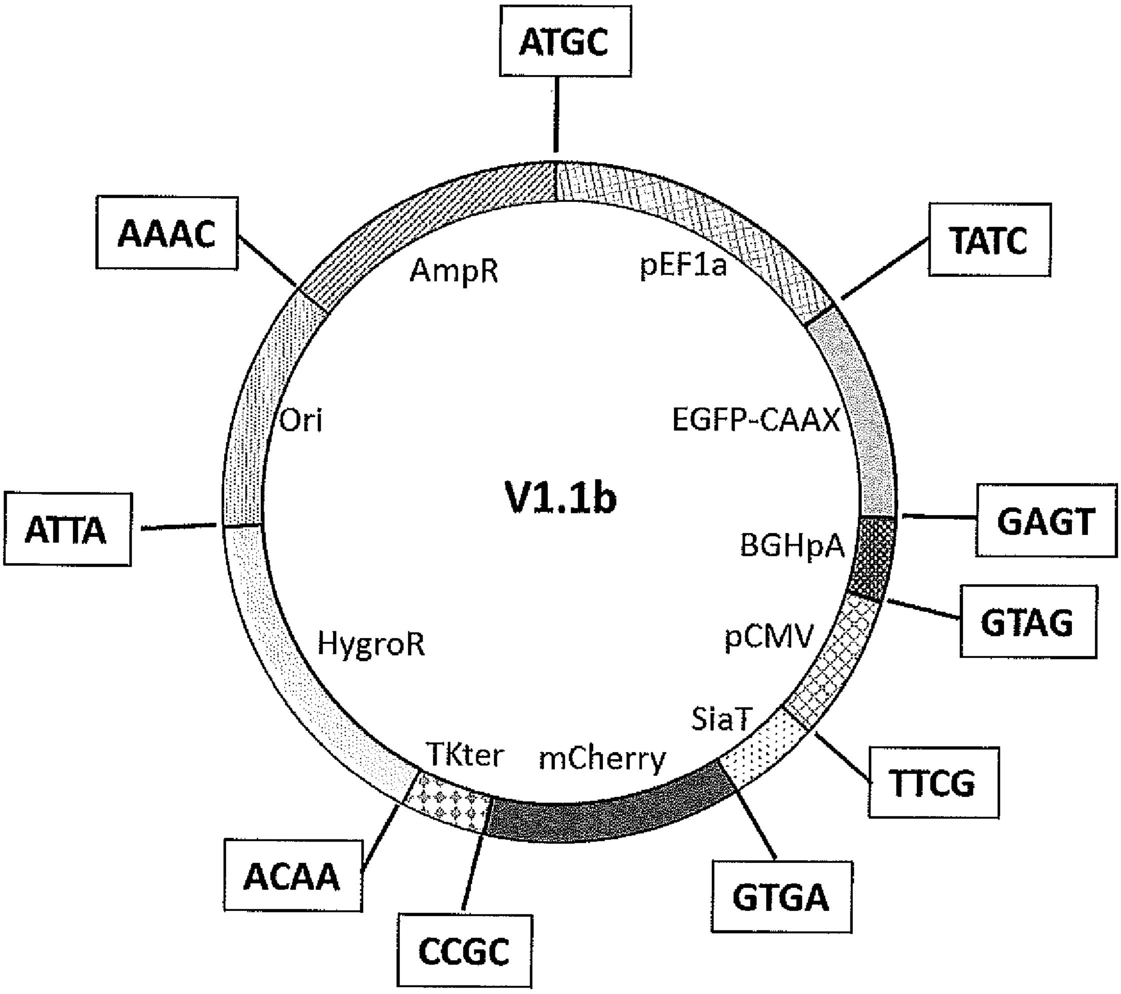
ATGC
TATC
GAGT
GTAG
TTCG
GTGA
CCGC
ACAA
ATTA
AAAC
pEF1a
EGFP-CAAX
BGHpA
pCMV
SiaT
mCherry
TKter
HygroR
Ori
AmpR
V1.1b

Figure 24

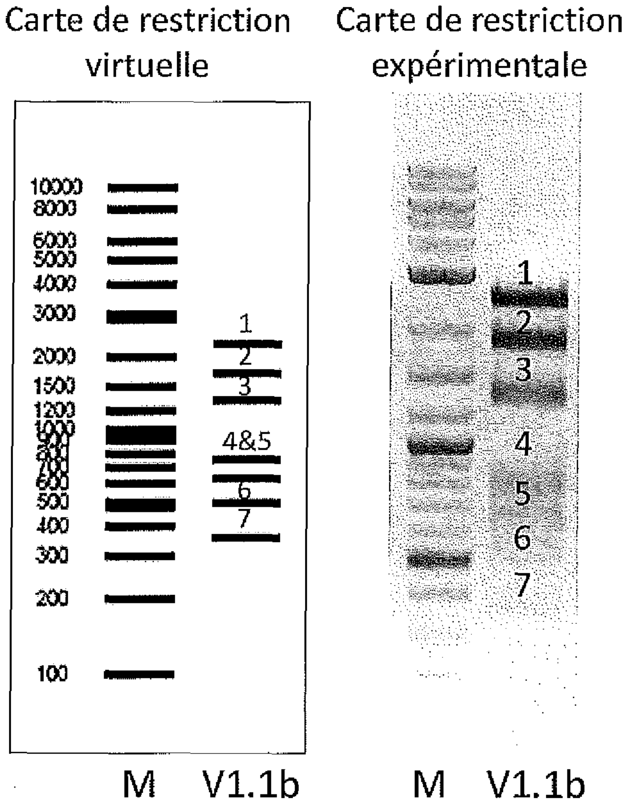


Figure 25

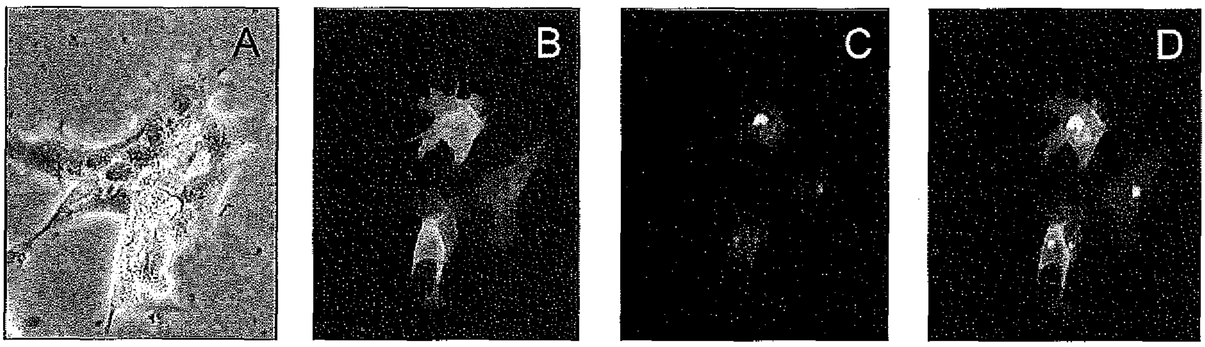


Figure 26

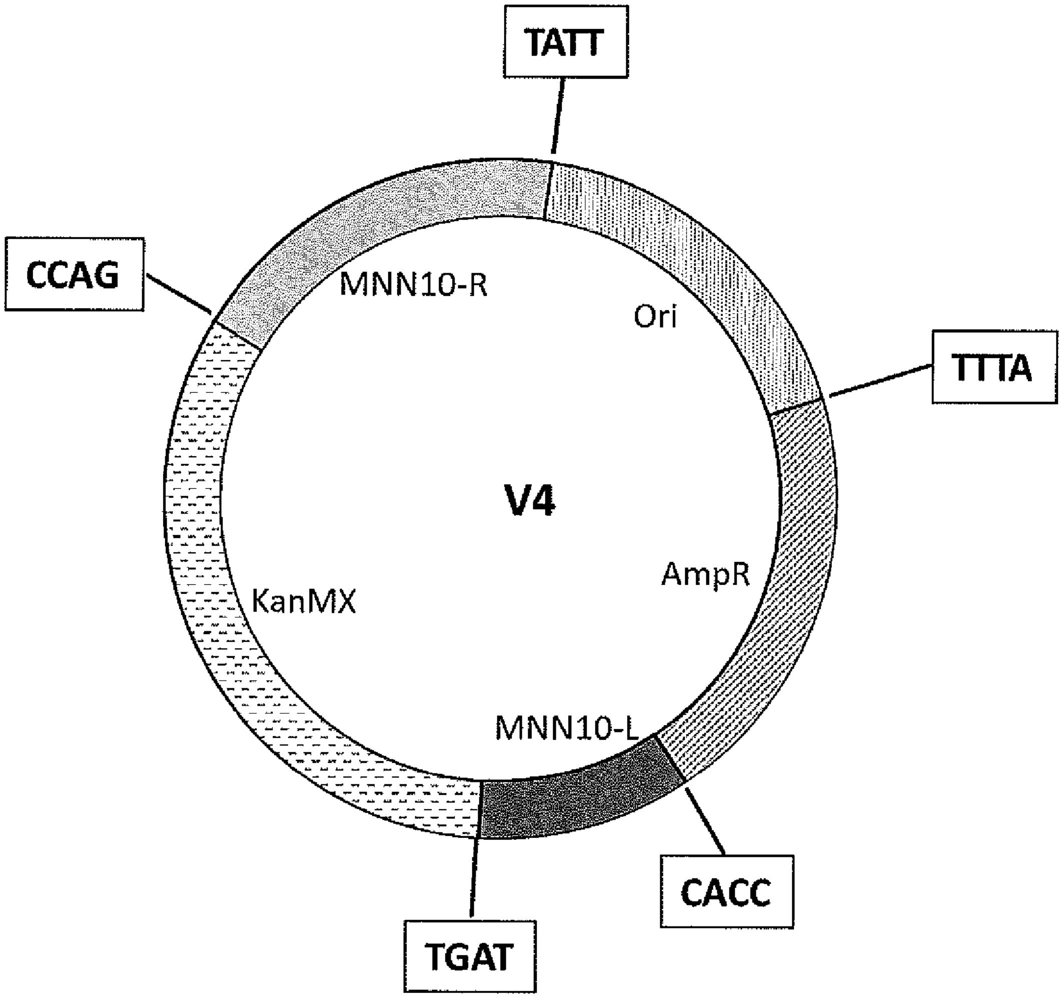


Figure 27

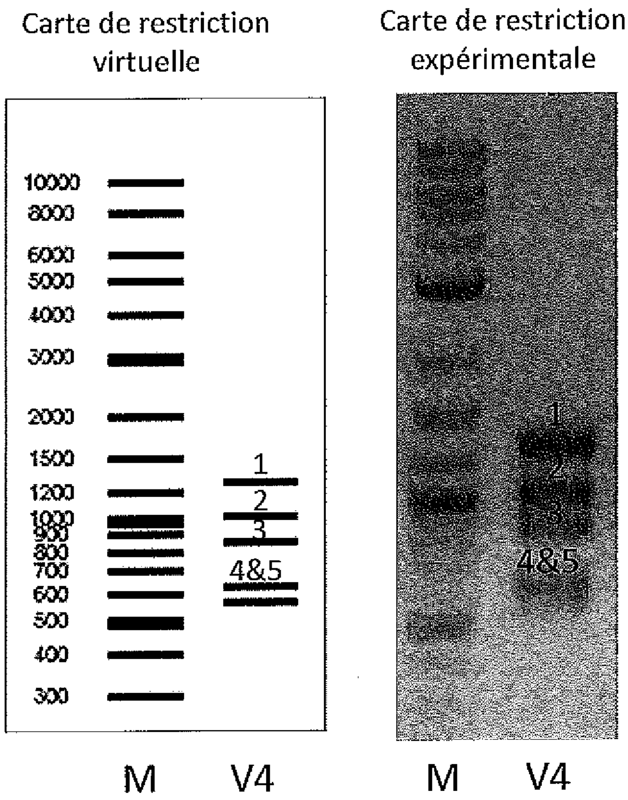


Figure 28

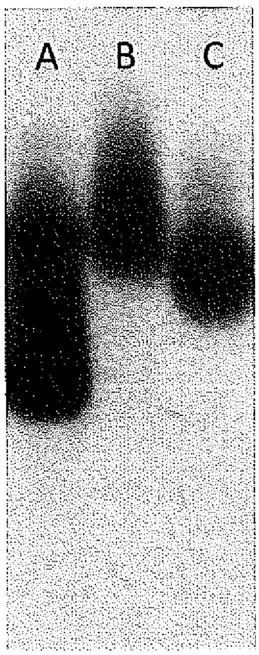


Figure 29

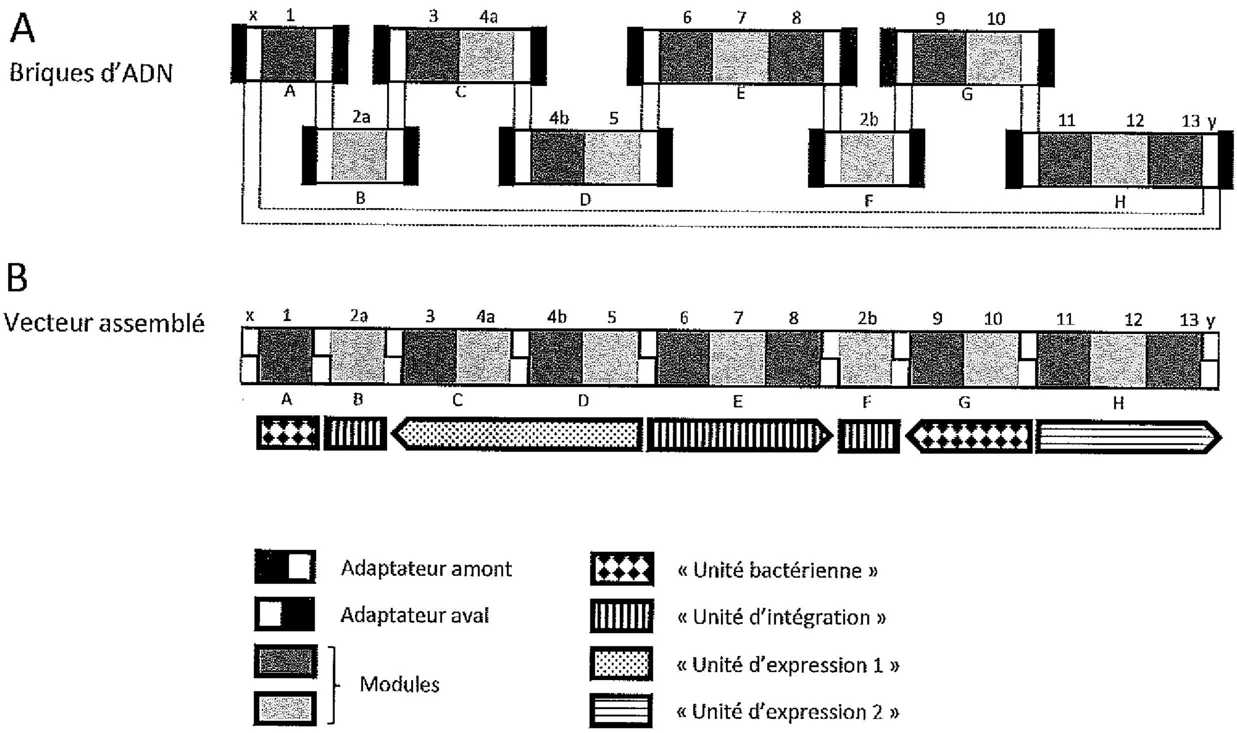
A
Briques d'ADN
x 1
A
3 4a
C
6 7 8
E
9 10
G
2a
B
4b 5
D
2b
F
11 12 13 y
H
B
Vecteur assemblé
x 1 2a 3 4a 4b 5 6 7 8 2b 9 10 11 12 13 y
A B C D E F G H
Adaptateur amont
Adaptateur aval
Modules
« Unité bactérienne »
« Unité d'intégration »
« Unité d'expression 1 »
« Unité d'expression 2 »

Figure 30

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 2395087 A
- WO 2008095927 A
- US 20140038240 A
- WO 9838326 A


### Littérature non-brevet citée dans la description


- **WANG T. et al.** *Appl Microbiol Biotechnol,* 2012, vol. 93, 1853-1863
- **WEBER E.** *PLoS ONE,* 2011, vol. 6 (2), e16765
- **SARRION-PERDIGONES A.** *PLoS ONE,* 2012, vol. 6 (7), e21622
- **D'ENGLER et al.** *PLoS ONE,* 2009, vol. 4, e5553
- **LIPPOW et al.** *Nucleic Acides Res,* 2009, vol. 37, 3061-3073
- **ZHIJUN WANG A ; LI JIN A,B ; ZHENGHONG YUAN C ; GRZEGORZ WE,GRZYN D ; ALICJA WE,GRZYN.** Classification of plasmid vectors using replication origin, selection marker and promoter as criteria. *Plasmid,* 2009, vol. 61, 47-51
- **CORMACK, B.** Directed Mutagenesis Using the Polymerase Chain Reaction. *Current Protocols in Molecular Biology.,* 2001, vol. 37
- **PROBER JM ; TRAINOR GL ; DAM RJ ; HOBBS FW ; ROBERTSON CW ; ZAGURSKY RJ et al.** A system for rapid DNA sequencing with fluorescent chain-terminating dideoxynucleotides. *Science,* 16 Octobre 1987, vol. 238 (4825), 336-4
- **LANGER-SAFER PR ; LEVINE M ; WARD DC.** Immunological method for mapping genes on Drosophila polytene chromosomes. *Proc. Natl. Acad. Sci. U.S.A.,* Juillet 1982, vol. 79 (14), 4381-5
- **PRIGODICH, A. E. ; RANDERIA, P. S. ; BRILEY, W. ; KIM, N. ; DANIEL, W. L. ; GILJOHANN, D. A. ; MIRKIN, C. A.** Multiplexed Nanoflares: mRNA Detection in Live Cells. *Anal. Chem.,* 2012, vol. 84, 2062-2066

SEQUENCE LISTING

<110> Université des Sciences et Technologies de Lille - Lille I Centre National de la Recherche Scientifique

<120> Procédé de fabrication de vecteurs d'ADN à partir de briques moléculaires contenant des séquences d'intérêt

<130> WOB 14 CQ LIL EZYV

<150> FR 15/51075
<151> 2015-02-10

<160> 178

<170> PatentIn version 3.5

<210> 1
<211> 40
<212> DNA
<213> Artificial sequence

<220>
<223> BGHpA TGAT BsaI CW

<400> 1
gaggtaccgg tctcatgatc gactgtgcct tctagttgcc 40

<210> 2
<211> 36
<212> DNA
<213> Artificial sequence

<220>
<223> BGHpA AGAA BsaI CCW

<400> 2
gaggtaccgg tctccagaag ccatagagcc caccgc 36

<210> 3

<211> 50
<212> DNA
<213> Artificial sequence

<220>
<223> lacZ-up TTCT BsaI CW

<400> 3
gaggtaccgg tctcgttctc cctgcaggtg cgcccaatac gcaaaccgcc 50

<210> 4
<211> 44
<212> DNA
<213> Artificial sequence

<220>
<223> lacZ-up TGTC BsaI CCW

<400> 4
gaggtaccgg tctcctgtcc gtaatcatgg tcatagctgt ttcc 44

<210> 5
<211> 41
<212> DNA
<213> Artificial sequence

<220>
<223> lacZ-down GACA BsaI CW

<400> 5
gaggtaccgg tctcggacag cctggccgtc gttttacaac g 41

<210> 6
<211> 55
<212> DNA
<213> Artificial sequence

<220>
<223> lacZ-down CTGG BsaI CCW

<400> 6
gaggtaccgg tctcactggc cctgcaggtc tatgcggcat cagagcagat tgtac 55

<210> 7
<211> 39
<212> DNA
<213> Artificial sequence

<220>
<223> SV40pori CCAG BsaI CW

<400> 7
gaggtaccgg tctccccagc aggcagaagt atgcaaagc 39

<210> 8
<211> 40
<212> DNA
<213> Artificial sequence

<220>
<223> SV40term ATAA BsaI CCW

<400> 8
gaggtaccgg tctcgataag atacattgat gagtttggac 40

<210> 9
<211> 58
<212> DNA
<213> Artificial sequence

<220>
<223> ColE1-ori TTAT BsaI CW

<400> 9
gaggtaccgg tctcattatg cgtcttctag ggttaaggtt agtgtagaga agcaaccg 58

<210> 10
<211> 59
<212> DNA
<213> Artificial sequence

<220>
<223> AmpR TCCT BsaI CCW

<400> 10
gaggtaccgg tctcgtcctt gagacgctag tcctcgttcc cgatgctctc gtcctatcc 59

<210> 11
<211> 37
<212> DNA
<213> Artificial sequence

<220>
<223> pENprom AGGA BsaI CW

<400> 11
gaggtaccgg tctcaaggaa ccaattcagt cgactgg 37

<210> 12
<211> 46
<212> DNA
<213> Artificial sequence

<220>
<223> pENprom GGTG BsaI CCW

<400> 12
gaggtaccgg tctcaggtgg cggccctgtt atccctagtc gactag 46

<210> 13
<211> 40
<212> DNA
<213> Artificial sequence

<220>
<223> SiaT CACC BsaI CW

<400> 13
gaggtaccgg tctcccacca tgattcacac caacctgaag 40

<210> 14
<211> 40
<212> DNA
<213> Artificial sequence

<220>
<223> SiaT CCCC BsaI CCW

<400> 14
gaggtaccgg tctcacccct tttgcagcct agggataagg 40

<210> 15
<211> 43
<212> DNA
<213> Artificial sequence

<220>
<223> XFP-Ctag GGGG BsaI CW

<400> 15
gaggtaccgg tctccggggt cgggggtgag caagggcgag gag 43

<210> 16
<211> 39
<212> DNA
<213> Artificial sequence

<220>
<223> XFP-Ctag ATCA BsaI CCW

<400> 16
gaggtaccgg tctccatcac ttgtacagct cgtccatgc 39

<210> 17
<211> 2005
<212> DNA
<213> Artificial sequence

<220>
<223> Ori-AmpR BsaI A

<400> 17

```
gaggtaccgg tctcattatg cgtcttctag ggttaaggtt agtgtagaga agcaaccgaa      60
gattgagaag acatggcggt aatacggtta tccacagaat caggggataa cgcaggaaag     120
aacatgtgag caaaaggcca gcaaaaggcc aggaaccgta aaaaggccgc gttgctggcg     180
tttttccata ggctccgccc ccctgacgag catcacaaaa atcgacgctc aagtcagagg     240
tggcgaaacc cgacaggact ataaagatac caggcgtttc cccctggaag ctccctcgtg     300
cgctctcctg ttccgaccct gccgcttacc ggatacctgt ccgcctttct cccttcggga     360
agcgtggcgc tttctcatag ctcacgctgt aggtatctca gttcggtgta ggtcgttcgc     420
tccaagctgg gctgtgtgca cgaacccccc gttcagcccg accgctgcgc cttatccggt     480
aactatcgtc ttgagtccaa cccggtaaga cacgacttat cgccactggc agcagccact     540
ggtaacagga ttagcagagc gaggtatgta ggcggtgcta cagagttctt gaagtggtgg     600
cctaactacg gctacactag aagaacagta tttggtatct gcgctctgct gaagccagtt     660
accttcggaa aaagagttgg tagctcttga tccggcaaac aaaccaccgc tggtagcggt     720
ttttttgttt gcaagcagca gattacgcgc agaaaaaaag gatctcaaga agatcctttg     780
atcttttcta cggggtctga cgctcagtgg aacgaaaact cacgttaagg gattttggtc     840
atgagattat caaaaaggat cttcacctag atccttttaa attaaaaatg aagttttaaa     900
tcaatctaaa gtatatatga gtaaacttgg tctgacagtt accaatgctt aatcagtgag     960
gcacctatct cagcgatctg tctatttcgt tcatccatag ttgcctgact ccccgtcgtg    1020
```

tagataacta cgatacggga gggcttacca tctggcccca gtgctgcaat gataccgcgt 1080

gacccacgct caccggctcc agatttatca gcaataaacc agccagccgg aagggccgag 1140

cgcagaagtg gtcctgcaac tttatccgcc tccatccagt ctattaattg ttgccgggaa 1200

gctagagtaa gtagttcgcc agttaatagt ttgcgcaacg ttgttgccat tgctacaggc 1260

atcgtggtgt cacgctcgtc gtttggtatg gcttcattca gctccggttc ccaacgatca 1320

aggcgagtta catgatcccc catgttgtgc aaaaaagcgg ttagctcctt cggtcctccg 1380

atcgttgtca gaagtaagtt ggccgcagtg ttatcactca tggttatggc agcactgcat 1440

aattctctta ctgtcatgcc atccgtaaga tgcttttctg tgactggtga gtactcaacc 1500

aagtcattct gagaatagtg tatgcggcga ccgagttgct cttgcccggc gtcaatacgg 1560

gataataccg cgccacatag cagaacttta aaagtgctca tcattggaaa acgttcttcg 1620

gggcgaaaac tctcaaggat cttaccgctg ttgagatcca gttcgatgta acccactcgt 1680

gcacccaact gatcttcagc atcttttact ttcaccagcg tttctgggtg agcaaaaaca 1740

ggaaggcaaa atgccgcaaa aaagggaata agggcgacac ggaaatgttg aatactcata 1800

ctcttccttt ttcaatatta ttgaagcatt tatcagggtt attgtctcat gagcggatac 1860

atatttgaat gtatttagaa aaataaacaa ataggggttc cgcgcacatt tccccgaaaa 1920

gtgccaccta tgagacgtga ggctagggat aggacgagag catcgggaac gaggactagc 1980

gtctcaagga cgagaccggt acctc 2005

<210> 18
<211> 677
<212> DNA
<213> Artificial sequence

<220>
<223> pCMV BsaI A

<400> 18

```
gaggtaccgg tctcaaggaa ccaattcagt cgactggatc ctagttatta atagtaatca        60
attacggggt cattagttca tagcccatat atggagttcc gcgttacata acttacggta       120
aatggcccgc ctggctgacc gcccaacgac ccccgcccat tgacgtcaat aatgacgtat       180
gttcccatag taacgccaat agggactttc cattgacgtc aatgggtgga gtatttacgg       240
taaactgccc acttggcagt acatcaagtg tatcatatgc caagtacgcc ccctattgac       300
gtcaatgacg gtaaatggcc cgcctggcat tatgcccagt acatgacctt atgggacttt       360
cctacttggc agtacatcta cgtattagtc atcgctatta ccatggtgat gcggttttgg       420
cagtacatca atgggcgtgg atagcggttt gactcacggg gatttccaag tctccacccc       480
attgacgtca atgggagttt gttttggcac caaaatcaac gggactttcc aaaatgtcgt       540
aacaactccg ccccattgac gcaaatgggc ggtaggcgtg tacggtggga ggtctatata       600

agcagagctg gtttagtgaa ccgtcagatc actagtcgac tagggataac agggccgcca       660
cctgagaccg gtacctc                                                      677
```

<210> 19
<211> 370
<212> DNA
<213> Artificial sequence

<220>
<223> SiaT BsaI A

<400> 19

```
gaggtaccgg tctcccacca tgattcacac caacctgaag aaaaagttca gctgctgcgt        60
cctggtcttt cttctgtttg cagtcatctg tgtgtggaag gaaaagaaga aagggagtta       120
ctatgattcc tttaaattgc aaaccaagga attccaggtg ttaaagagtc tggggaaatt       180
ggccatgggg tctgattccc agtctgtatc ctcaagcagc acccaggacc cccacagggg       240
ccgccagacc ctcggcagtc tcagaggcct agccaaggcc aaaccagagg cctccttcca       300
ggtgtggaac aaggacagct cttccaaaaa ccttatccct aggctgcaaa aggggtgaga       360
ccggtacctc                                                              370
```

<210> 20
<211> 758
<212> DNA
<213> Artificial sequence

<220>
<223> E1GFP BsaI A

<400> 20

```
gaggtaccgg tctccggggt cgggggtgag caagggcgag gagctgttca ccggggtggt        60
gcccatcctg gtcgagctgg acggcgacgt aaacggccac aagttcagcg tgtccggcga       120
gggcgagggc gatgccacct acggcaagct gaccctgaag ttcatctgca ccaccggcaa       180
gctgcccgtg ccctggccca ccctcgtgac caccctgtcc tacggcgtgc agtgcttcag       240
ccgctacccc gaccacatga agcagcacga cttcttcaag tccgccatgc ccgaaggcta       300
cgtccaggag cgcaccatct tcttcaagga cgacggcaac tacaagaccc gcgccgaggt       360
gaagttcgag ggcgacaccc tggtgaaccg catcgagctg aagggcatcg acttcaagga       420
ggacggcaac atcctggggc acaagctgga gtacaactac aacagccaca acgtctatat       480
catggccgac aagcagaaga acggcatcaa ggtgaacttc aagatccgcc acaacatcga       540
ggacggcagc gtgcagctcg ccgaccacta ccagcagaac acccccatcg gcgacggccc       600
cgtgctgctg cccgacaacc actacctgag ctaccagtcc gccctgagca aagaccccaa       660
cgagaagcgc gatcacatgg tcctgctgga gttcgtgacc gccgccggga tcactctcgg       720

catggacgag ctgtacaagt gatggagacc ggtacctc                               758
```

<210> 21
<211> 758
<212> DNA
<213> Artificial sequence

<220>
<223> EGFP BsaI A

<400> 21

```
gaggtaccgg tctccggggt cgggggtgag caagggcgag gagctgttca ccggggtggt      60
gcccatcctg gtcgagctgg acggcgacgt aaacggccac aagttcagcg tgtccggcga     120
gggcgagggc gatgccacct acggcaagct gaccctgaag ttcatctgca ccaccggcaa     180
gctgcccgtg ccctggccca ccctcgtgac caccctgacc tacggcgtgc agtgcttcag     240
ccgctacccc gaccacatga agcagcacga cttcttcaag tccgccatgc ccgaaggcta     300
cgtccaggag cgcaccatct tcttcaagga cgacggcaac tacaagaccc gcgccgaggt     360
gaagttcgag ggcgacaccc tggtgaaccg catcgagctg aagggcatcg acttcaagga     420
ggacggcaac atcctggggc acaagctgga gtacaactac aacagccaca acgtctatat     480
catggccgac aagcagaaga acggcatcaa ggtgaacttc aagatccgcc acaacatcga     540
ggacggcagc gtgcagctcg ccgaccacta ccagcagaac acccccatcg gcgacggccc     600
cgtgctgctg cccgacaacc actacctgag cacccagtcc gccctgagca aagaccccaa     660
cgagaagcgc gatcacatgg tcctgctgga gttcgtgacc gccgccggga tcactctcgg     720
catggacgag ctgtacaagt gatggagacc ggtacctc                             758
```

<210> 22
<211> 758
<212> DNA
<213> Artificial sequence

<220>
<223> ECFP BsaI A

<400> 22

```
gaggtaccgg tctccggggt cgggggtgag caagggcgag gagctgttca ccggggtggt      60
gcccatcctg gtcgagctgg acggcgacgt aaacggccac aagttcagcg tgtccggcga     120
gggcgagggc gatgccacct acggcaagct gaccctgaag ttcatctgca ccaccggcaa     180
gctgcccgtg ccctggccca ccctcgtgac caccctgacc tggggcgtgc agtgcttcag     240
ccgctacccc gaccacatga agcagcacga cttcttcaag tccgccatgc ccgaaggcta     300
cgtccaggag cgcaccatct tcttcaagga cgacggcaac tacaagaccc gcgccgaggt     360
gaagttcgag ggcgacaccc tggtgaaccg catcgagctg aagggcatcg acttcaagga     420
ggacggcaac atcctggggc acaagctgga gtacaactac atcagccaca acgtctatat     480
```

caccgccgac aagcagaaga acggcatcaa ggccaacttc aagatccgcc acaacatcga 540

ggacggcagc gtgcagctcg ccgaccacta ccagcagaac acccccatcg gcgacggccc 600

cgtgctgctg cccgacaacc actacctgag cacccagtcc gccctgagca aagaccccaa 660

cgagaagcgc gatcacatgg tcctgctgga gttcgtgacc gccgccggga tcactctcgg 720

catggacgag ctgtacaagt gatggagacc ggtacctc 758

<210> 23
<211> 758
<212> DNA
<213> Artificial sequence

<220>
<223> EYFP BsaI A

<400> 23

gaggtaccgg tctccggggt cgggggtgag caagggcgag gagctgttca ccggggtggt 60

gcccatcctg gtcgagctgg acggcgacgt aaacggccac aagttcagcg tgtccggcga 120

gggcgagggc gatgccacct acggcaagct gaccctgaag ttcatctgca ccaccggcaa 180

gctgcccgtg ccctggccca ccctcgtgac caccttcggc tacggcctgc agtgcttcgc 240

ccgctacccc gaccacatga agcagcacga cttcttcaag tccgccatgc ccgaaggcta 300

cgtccaggag cgcaccatct tcttcaagga cgacggcaac tacaagaccc gcgccgaggt 360

gaagttcgag ggcgacaccc tggtgaaccg catcgagctg aagggcatcg acttcaagga 420

ggacggcaac atcctggggc acaagctgga gtacaactac aacagccaca acgtctatat 480

catggccgac aagcagaaga acggcatcaa ggtgaacttc aagatccgcc acaacatcga 540

ggacggcagc gtgcagctcg ccgaccacta ccagcagaac acccccatcg gcgacggccc 600

cgtgctgctg cccgacaacc actacctgag ctaccagtcc gccctgagca aagaccccaa 660

cgagaagcgc gatcacatgg tcctgctgga gttcgtgacc gccgccggga tcactctcgg 720

catggacgag ctgtacaagt gatggagacc ggtacctc 758

<210> 24
<211> 746
<212> DNA
<213> Artificial sequence

<220>
<223> mCherry BsaI A

<400> 24

```
gaggtaccgg tctccggggt cggggagcga gctgattaag gagaacatgc acatgaagct        60
gtacatggag ggcaccgtgg acaaccatca cttcaagtgc acatccgagg gcgaaggcaa       120
gccctacgag ggcacccaga ccatgagaat caaggtggtc gagggcggcc ctctcccctt       180
cgccttcgac atcctggcta ctagcttcct ctacggcagc aagaccttca tcaaccacac       240
ccagggcatc cccgacttct tcaagcagtc cttccctgag ggcttcacat gggagagagt       300
caccacatac gaggacgggg gcgtgctgac cgctacccag gacaccagcc tccaggacgg       360
ctgcctcatc tacaacgtca agatcagagg ggtgaacttc acatccaacg gccctgtgat       420
gcagaagaaa acactcggct gggaggcctt caccgaaacg ctgtaccccg ctgacggcgg       480
cctggaaggc agaaacgaca tggccctgaa gctcgtgggc gggagccatc tgatcgcaaa       540
catcaagacc acatatagat ccaagaaacc cgctaagaac ctcaagatgc ctggcgtcta       600
ctatgtggac tacagactgg aaagaatcaa ggaggccaac aacgaaacct acgtcgagca       660
gcacgaggtg gcagtggcca gatactgcga cctccctagc aaactggggc acaagcttaa       720
ttccggatga tggagaccgg tacctc                                            746
```

<210> 25
<211> 746
<212> DNA
<213> Artificial sequence

<220>
<223> TagBFP BsaI A

<400> 25

gaggtaccgg tctccggggt cggggagcga gctgattaag gagaacatgc acatgaagct 60

gtacatggag ggcaccgtgg acaaccatca cttcaagtgc acatccgagg gcgaaggcaa 120

gccctacgag ggcacccaga ccatgagaat caaggtggtc gagggcggcc ctctcccctt 180

cgccttcgac atcctggcta ctagcttcct ctacggcagc aagaccttca tcaaccacac 240

ccagggcatc cccgacttct tcaagcagtc cttccctgag ggcttcacat gggagagagt 300

caccacatac gaggacgggg gcgtgctgac cgctacccag gacaccagcc tccaggacgg 360

ctgcctcatc tacaacgtca agatcagagg ggtgaacttc acatccaacg gccctgtgat 420

gcagaagaaa acactcggct gggaggcctt caccgaaacg ctgtaccccg ctgacggcgg 480

cctggaaggc agaaacgaca tggccctgaa gctcgtgggc gggagccatc tgatcgcaaa 540

catcaagacc acatatagat ccaagaaacc cgctaagaac ctcaagatgc ctggcgtcta 600

ctatgtggac tacagactgg aaagaatcaa ggaggccaac aacgaaacct acgtcgagca 660

gcacgaggtg gcagtggcca gatactgcga cctccctagc aaactggggc acaagcttaa 720

ttccggatga tggagaccgg tacctc 746

<210> 26
<211> 267
<212> DNA
<213> Artificial sequence

<220>
<223> BGHpA BsaI A

<400> 26

gaggtaccgg tctcatgatc gactgtgcct tctagttgcc agccatctgt tgtttgcccc 60

tcccccgtgc cttccttgac cctggaaggt gccactccca ctgtcctttc ctaataaaat 120

gaggaaattg catcgcattg tctgagtagg tgtcattcta ttctgggggg tggggtgggg 180

caggacagca agggggagga ttgggaggac aatagcaggc atgctgggga tgcggtgggc 240

tctatggctt ctggagaccg gtacctc 267

<210> 27
<211> 278
<212> DNA
<213> Artificial sequence

<220>
<223> LacZ-up BsaI A

<400> 27

gaggtaccgg tctcgttctc cctgcaggtg cgcccaatac gcaaaccgcc tctccccgcg 60

cgttggccga ttcattaatg cagctggcac gacaggtttc ccgactggaa agcgggcagt 120

gagcgcaacg caattaatgt gagttagctc actcattagg caccccaggc tttacacttt 180

atgcttccgg ctcgtatgtt gtgtggaatt gtgagcggat aacaatttca cacaggaaac 240

agctatgacc atgattacgg acaggagacc ggtacctc 278

<210> 28
<211> 299
<212> DNA
<213> Artificial sequence

<220>
<223> LacZ-down BsaI A

<400> 28

gaggtaccgg tctcggacag cctggccgtc gttttacaac gtcgtgactg ggaaaaccct 60

ggcgttaccc aacttaatcg ccttgcagca catccccctt tcgccagctg gcgtaatagc 120

gaagaggccc gcaccgatcg cccttcccaa cagttgcgca gcctgaatgg cgaatggcgc 180

ctgatgcggt attttctcct tacgcatctg tgcggtattt cacaccgcat atggtgcact 240

ctcagtacaa tctgctctga tgccgcatag acctgcaggg ccagtgagac cggtacctc 299

<210> 29
<211> 1650
<212> DNA
<213> Artificial sequence

<220>
<223> HygroR BsaI A

<400> 29

```
gaggtaccgg tctccccagc aggcagaagt atgcaaagca tgcatctcaa ttagtcagca      60
accaggtgtg gaaagtcccc aggctcccca gcaggcagaa gtatgcaaag catgcatctc     120
aattagtcag caaccatagt cccgccccta actccgccca tcccgcccct aactccgccc     180
agttccgccc attctccgcc ccatggctga ctaatttttt ttatttatgc agaggccgag     240
gccgcctctg cctctgagct attccagaag tagtgaggag gcttttttgg aggcctaggc     300
ttttgcaaaa agctcccggg agcttgtata tccattttcg gatctgatca gcacgtgatg     360
aaaaagcctg aactcaccgc gacgtctgtc gagaagtttc tgatcgaaaa gttcgacagc     420
gtgtccgacc tgatgcagct ctcggagggc gaagaatctc gtgctttcag cttcgatgta     480
ggagggcgtg gatatgtcct gcgggtaaat agctgcgccg atggtttcta caaagatcgt     540
tatgtttatc ggcactttgc atcggccgcg ctcccgattc cggaagtgct tgacattggg     600
gaattcagcg agagcctgac ctattgcatc tcccgccgtg cacagggtgt cacgttgcaa     660
gacttgcctg aaaccgaact gcccgctgtt ctgcagccgg tcgcggaggc catggatgcg     720
atcgctgcgg ccgatcttag ccagacgagc gggttcggcc cattcggacc gcaaggaatc     780
ggtcaataca ctacatggcg tgatttcata tgcgcgattg ctgatcccca tgtgtatcac     840
tggcaaactg tgatggacga caccgtcagt gcgtccgtcg cgcaggctct cgatgagctg     900
atgctttggg ccgaggactg ccccgaagtc cggcacctcg tgcacgcgga tttcggctcc     960
aacaatgtcc tgacggacaa tggccgcata acagcggtca ttgactggag cgaggcgatg    1020
ttcggggatt cccaatacga ggtcgccaac atcttcttct ggaggccgtg gttggcttgt    1080
atggagcagc agacgcgcta cttcgagcgg aggcatccgg agcttgcagg atcgccgcgg    1140
ctccgggcgt atatgctccg cattggtctt gaccaactct atcagagctt ggttgacggc    1200
aatttcgatg atgcagcttg ggcgcagggt cgatgcgacg caatcgtccg atccggagcc    1260
gggactgtcg ggcgtacaca aatcgcccgc agaagcgcgg ccgtctggac cgatggctgt    1320
gtagaagtac tcgccgatag tggaaaccga cgccccagca ctcgtccgag ggcaaaggaa    1380
tagcacgtgc tacgagattt cgattccacc gccgccttct atgaaaggtt gggcttcgga    1440
atcgttttcc gggacgccgg ctggatgatc ctccagcgcg gggatctcat gctggagttc    1500
ttcgcccacc ccaacttgtt tattgcagct tataatggtt acaaataaag caatagcatc    1560
acaaatttca caaataaagc atttttttca ctgcattcta gttgtggttt gtccaaactc    1620
atcaatgtat cttatcgaga ccggtacctc                                     1650
```

<210> 30
<211> 6032
<212> DNA
<213> Artificial sequence

<220>
<223> pHCsiaT-E1GFP

<400> 30

tgaggctagg gataggacga gagcatcggg aacgaggact agcgtctcaa ggaaccaatt 60
cagtcgactg gatcctagtt attaatagta atcaattacg gggtcattag ttcatagccc 120
atatatggag ttccgcgtta cataacttac ggtaaatggc ccgcctggct gaccgcccaa 180
cgacccccgc ccattgacgt caataatgac gtatgttccc atagtaacgc caatagggac 240
tttccattga cgtcaatggg tggagtattt acggtaaact gcccacttgg cagtacatca 300
agtgtatcat atgccaagta cgccccctat tgacgtcaat gacggtaaat ggcccgcctg 360
gcattatgcc cagtacatga ccttatggga ctttcctact tggcagtaca tctacgtatt 420
agtcatcgct attaccatgg tgatgcggtt ttggcagtac atcaatgggc gtggatagcg 480
gtttgactca cggggatttc caagtctcca ccccattgac gtcaatggga gtttgttttg 540
gcaccaaaat caacgggact ttccaaaatg tcgtaacaac tccgccccat tgacgcaaat 600
gggcggtagg cgtgtacggt gggaggtcta tataagcaga gctggtttag tgaaccgtca 660
gatcactagt cgactaggga taacagggcc gccaccatga ttcacaccaa cctgaagaaa 720
aagttcagct gctgcgtcct ggtctttctt ctgtttgcag tcatctgtgt gtggaaggaa 780
aagaagaaag ggagttacta tgattccttt aaattgcaaa ccaaggagtt ccaggtgtta 840
aagagtctgg ggaaattggc catggggtct gattcccagt ctgtatcctc aagcagcacc 900
caggaccccc acaggggccg ccagaccctc ggcagtctca gaggcctagc caaggccaaa 960
ccagaggcct ccttccaggt gtggaacaag gacagctctt ccaaaaacct tatccctagg 1020
ctgcaaaagg ggtcgggggt gagcaagggc gaggagctgt tcaccggggt ggtgcccatc 1080
ctggtcgagc tggacggcga cgtaaacggc cacaagttca gcgtgtccgg cgagggcgag 1140
ggcgatgcca cctacggcaa gctgaccctg aagttcatct gcaccaccgg caagctgccc 1200
gtgccctggc ccaccctcgt gaccaccctg tcctacggcg tgcagtgctt cagccgctac 1260
cccgaccaca tgaagcagca cgacttcttc aagtccgcca tgcccgaagg ctacgtccag 1320
gagcgcacca tcttcttcaa ggacgacggc aactacaaga cccgcgccga ggtgaagttc 1380
gagggcgaca ccctggtgaa ccgcatcgag ctgaagggca tcgacttcaa ggaggacggc 1440
aacatcctgg ggcacaagct ggagtacaac tacaacagcc acaacgtcta tatcatggcc 1500
gacaagcaga agaacggcat caaggtgaac ttcaagatcc gccacaacat cgaggacggc 1560
agcgtgcagc tcgccgacca ctaccagcag aacaccccca tcggcgacgg ccccgtgctg 1620
ctgcccgaca accactacct gagctaccag tccgccctga gcaaagaccc caacgagaag 1680
cgcgatcaca tggtcctgct ggagttcgtg accgccgccg ggatcactct cggcatggac 1740
gagctgtaca agtgatcgac tgtgccttct agttgccagc catctgttgt ttgcccctcc 1800

cccgtgcctt ccttgaccct ggaaggtgcc actcccactg tcctttccta ataaaatgag 1860
gaaattgcat cgcattgtct gagtaggtgt cattctattc tggggggtgg ggtggggcag 1920
gacagcaagg gggaggattg ggaggacaat agcaggcatg ctggggatgc ggtgggctct 1980
atggcttctc cctgcaggtg cgcccaatac gcaaaccgcc tctccccgcg cgttggccga 2040
ttcattaatg cagctggcac gacaggtttc ccgactggaa agcgggcagt gagcgcaacg 2100
caattaatgt gagttagctc actcattagg caccccaggc tttacacttt atgcttccgg 2160
ctcgtatgtt gtgtggaatt gtgagcggat aacaatttca cacaggaaac agctatgacc 2220
atgattacgg acagcctggc cgtcgtttta caacgtcgtg actgggaaaa ccctggcgtt 2280
acccaactta atcgccttgc agcacatccc cctttcgcca gctggcgtaa tagcgaagag 2340
gcccgcaccg atcgcccttc ccaacagttg cgcagcctga atggcgaatg gcgcctgatg 2400
cggtattttc tccttacgca tctgtgcggt atttcacacc gcatatggtg cactctcagt 2460
acaatctgct ctgatgccgc atagacctgc agggccagca ggcagaagta tgcaaagcat 2520
gcatctcaat tagtcagcaa ccaggtgtgg aaagtcccca ggctccccag caggcagaag 2580
tatgcaaagc atgcatctca attagtcagc aaccatagtc ccgcccctaa ctccgcccat 2640
cccgccccta actccgccca gttccgccca ttctccgccc catggctgac taattttttt 2700
tatttatgca gaggccgagg ccgcctctgc ctctgagcta ttccagaagt agtgaggagg 2760
ctttttgga ggcctaggct tttgcaaaaa gctcccggga gcttgtatat ccattttcgg 2820
atctgatcag cacgtgatga aaaagcctga actcaccgcg acgtctgtcg agaagtttct 2880
gatcgaaaag ttcgacagcg tgtccgacct gatgcagctc tcggagggcg aagaatctcg 3000
tgctttcagc ttcgatgtag gagggcgtgg atatgtcctg cgggtaaata gctgcgccga 3000
tggtttctac aaagatcgtt atgtttatcg gcactttgca tcggccgcgc tcccgattcc 3060
ggaagtgctt gacattgggg aattcagcga gagcctgacc tattgcatct cccgccgtgc 3120
acagggtgtc acgttgcaag acttgcctga aaccgaactg cccgctgttc tgcagccggt 3180
cgcggaggcc atggatgcga tcgctgcggc cgatcttagc cagacgagcg ggttcggccc 3240
attcggaccg caaggaatcg gtcaatacac tacatggcgt gatttcatat gcgcgattgc 3300
tgatccccat gtgtatcact ggcaaactgt gatggacgac accgtcagtg cgtccgtcgc 3360
gcaggctctc gatgagctga tgctttgggc cgaggactgc cccgaagtcc ggcacctcgt 3420
gcacgcggat ttcggctcca acaatgtcct gacggacaat ggccgcataa cagcggtcat 3480
tgactggagc gaggcgatgt tcggggattc ccaatacgag gtcgccaaca tcttcttctg 3540
gaggccgtgg ttggcttgta tggagcagca gacgcgctac ttcgagcgga ggcatccgga 3600
gcttgcagga tcgccgcggc tccgggcgta tatgctccgc attggtcttg accaactcta 3660
tcagagcttg gttgacggca atttcgatga tgcagcttgg gcgcagggtc gatgcgacgc 3720

aatcgtccga tccggagccg ggactgtcgg gcgtacacaa atcgcccgca gaagcgcggc 3780
cgtctggacc gatggctgtg tagaagtact cgccgatagt ggaaaccgac gccccagcac 3840
tcgtccgagg gcaaaggaat agcacgtgct acgagatttc gattccaccg ccgccttcta 3900
tgaaaggttg ggcttcggaa tcgttttccg ggacgccggc tggatgatcc tccagcgcgg 3960
ggatctcatg ctggagttct tcgcccaccc caacttgttt attgcagctt ataatggtta 4020
caaataaagc aatagcatca caaatttcac aaataaagca tttttttcac tgcattctag 4080
ttgtggtttg tccaaactca tcaatgtatc ttatgcgtct tctagggtta aggttagtgt 4140
agagaagcaa ccgaagattg agaagacatg gcggtaatac ggttatccac agaatcaggg 4200
gataacgcag gaaagaacat gtgagcaaaa ggccagcaaa aggccaggaa ccgtaaaaag 4260
gccgcgttgc tggcgttttt ccataggctc cgcccccctg acgagcatca caaaaatcga 4320
cgctcaagtc agaggtggcg aaacccgaca ggactataaa gataccaggc gtttccccct 4380
ggaagctccc tcgtgcgctc tcctgttccg accctgccgc ttaccggata cctgtccgcc 4440
tttctccctt cgggaagcgt ggcgctttct catagctcac gctgtaggta tctcagttcg 4500
gtgtaggtcg ttcgctccaa gctgggctgt gtgcacgaac cccccgttca gcccgaccgc 4560
tgcgccttat ccggtaacta tcgtcttgag tccaacccgg taagacacga cttatcgcca 4620
ctggcagcag ccactggtaa caggattagc agagcgaggt atgtaggcgg tgctacagag 4680
ttcttgaagt ggtggcctaa ctacggctac actagaagaa cagtatttgg tatctgcgct 4740
ctgctgaagc cagttacctt cggaaaaaga gttggtagct cttgatccgg caaacaaacc 4800
accgctggta gcggtttttt tgtttgcaag cagcagatta cgcgcagaaa aaaaggatct 4860
caagaagatc ctttgatctt ttctacgggg tctgacgctc agtggaacga aaactcacgt 4920
taagggattt tggtcatgag attatcaaaa aggatcttca cctagatcct tttaaattaa 4980
aaatgaagtt ttaaatcaat ctaaagtata tatgagtaaa cttggtctga cagttaccaa 5040
tgcttaatca gtgaggcacc tatctcagcg atctgtctat ttcgttcatc catagttgcc 5100
tgactccccg tcgtgtagat aactacgata cgggagggct taccatctgg ccccagtgct 5160
gcaatgatac cgcgtgaccc acgctcaccg gctccagatt tatcagcaat aaaccagcca 5220
gccggaaggg ccgagcgcag aagtggtcct gcaactttat ccgcctccat ccagtctatt 5280
aattgttgcc gggaagctag agtaagtagt tcgccagtta atagtttgcg caacgttgtt 5340
gccattgcta caggcatcgt ggtgtcacgc tcgtcgtttg gtatggcttc attcagctcc 5400
ggttcccaac gatcaaggcg agttacatga tcccccatgt tgtgcaaaaa agcggttagc 5460
tccttcggtc ctccgatcgt tgtcagaagt aagttggccg cagtgttatc actcatggtt 5520
atggcagcac tgcataattc tcttactgtc atgccatccg taagatgctt ttctgtgact 5580

ggtgagtact caaccaagtc attctgagaa tagtgtatgc ggcgaccgag ttgctcttgc 5640

ccggcgtcaa tacgggataa taccgcgcca catagcagaa ctttaaaagt gctcatcatt 5700

ggaaaacgtt cttcggggcg aaaactctca aggatcttac cgctgttgag atccagttcg 5760

atgtaaccca ctcgtgcacc caactgatct tcagcatctt ttactttcac cagcgtttct 5820

gggtgagcaa aaacaggaag gcaaaatgcc gcaaaaaagg gaataagggc gacacggaaa 5880

tgttgaatac tcatactctt cctttttcaa tattattgaa gcatttatca gggttattgt 5940

ctcatgagcg gatacatatt tgaatgtatt tagaaaaata aacaaatagg ggttccgcgc 6000

acatttcccc gaaaagtgcc acctatgaga cg 6032

<210> 31
<211> 6032
<212> DNA
<213> Artificial sequence

<220>
<223> pHCsiaT-EGFP

<400> 31

```
tgaggctagg gataggacga gagcatcggg aacgaggact agcgtctcaa ggaaccaatt      60
cagtcgactg gatcctagtt attaatagta atcaattacg gggtcattag ttcatagccc     120
atatatggag ttccgcgtta cataacttac ggtaaatggc ccgcctggct gaccgcccaa     180
cgacccccgc ccattgacgt caataatgac gtatgttccc atagtaacgc caatagggac     240
tttccattga cgtcaatggg tggagtattt acggtaaact gcccacttgg cagtacatca     300
agtgtatcat atgccaagta cgccccctat tgacgtcaat gacggtaaat ggcccgcctg     360
gcattatgcc cagtacatga ccttatggga ctttcctact tggcagtaca tctacgtatt     420
agtcatcgct attaccatgg tgatgcggtt ttggcagtac atcaatgggc gtggatagcg     480
gtttgactca cggggatttc caagtctcca ccccattgac gtcaatggga gtttgttttg     540
gcaccaaaat caacgggact ttccaaaatg tcgtaacaac tccgccccat tgacgcaaat     600
gggcggtagg cgtgtacggt gggaggtcta tataagcaga gctggtttag tgaaccgtca     660
gatcactagt cgactaggga taacagggcc gccaccatga ttcacaccaa cctgaagaaa     720
aagttcagct gctgcgtcct ggtctttctt ctgtttgcag tcatctgtgt gtggaaggaa     780
aagaagaaag ggagttacta tgattccttt aaattgcaaa ccaaggaatt ccaggtgtta     840
aagagtctgg ggaaattggc catggggtct gattcccagt ctgtatcctc aagcagcacc     900
caggaccccc acagggccg ccagaccctc ggcagtctca gaggcctagc caaggccaaa      960
ccagaggcct ccttccaggt gtggaacaag gacagctctt ccaaaaacct tatccctagg    1020
ctgcaaaagg ggtcgggggt gagcaagggc gaggagctgt tcaccggggt ggtgcccatc    1080
ctggtcgagc tggacggcga cgtaaacggc cacaagttca gcgtgtccgg cgagggcgag    1140
```

ggcgatgcca cctacggcaa gctgaccctg aagttcatct gcaccaccgg caagctgccc 1200
gtgccctggc ccaccctcgt gaccaccctg acctacggcg tgcagtgctt cagccgctac 1260
cccgaccaca tgaagcagca cgacttcttc aagtccgcca tgcccgaagg ctacgtccag 1320
gagcgcacca tcttcttcaa ggacgacggc aactacaaga cccgcgccga ggtgaagttc 1380
gagggcgaca ccctggtgaa ccgcatcgag ctgaagggca tcgacttcaa ggaggacggc 1440
aacatcctgg ggcacaagct ggagtacaac tacaacagcc acaacgtcta tatcatggcc 1500
gacaagcaga agaacggcat caaggtgaac ttcaagatcc gccacaacat cgaggacggc 1560
agcgtgcagc tcgccgacca ctaccagcag aacaccccca tcggcgacgg ccccgtgctg 1620
ctgcccgaca accactacct gagcacccag tccgccctga gcaaagaccc caacgagaag 1680
cgcgatcaca tggtcctgct ggagttcgtg accgccgccg ggatcactct cggcatggac 1740
gagctgtaca agtgatcgac tgtgccttct agttgccagc catctgttgt ttgcccctcc 1800
cccgtgcctt ccttgaccct ggaaggtgcc actcccactg tcctttccta ataaaatgag 1860
gaaattgcat cgcattgtct gagtaggtgt cattctattc tggggggtgg ggtggggcag 1920
gacagcaagg gggaggattg ggaggacaat agcaggcatg ctggggatgc ggtgggctct 1980
atggcttctc cctgcaggtg cgcccaatac gcaaaccgcc tctccccgcg cgttggccga 2040
ttcattaatg cagctggcac gacaggtttc ccgactggaa agcgggcagt gagcgcaacg 2100
caattaatgt gagttagctc actcattagg caccccaggc tttacacttt atgcttccgg 2160
ctcgtatgtt gtgtggaatt gtgagcggat aacaatttca cacaggaaac agctatgacc 2220
atgattacgg acagcctggc cgtcgtttta caacgtcgtg actgggaaaa ccctggcgtt 2280
acccaactta atcgccttgc agcacatccc cctttcgcca gctggcgtaa tagcgaagag 2340
gcccgcaccg atcgcccttc ccaacagttg cgcagcctga atggcgaatg gcgcctgatg 2400
cggtattttc tccttacgca tctgtgcggt atttcacacc gcatatggtg cactctcagt 2460
acaatctgct ctgatgccgc atagacctgc agggccagca ggcagaagta tgcaaagcat 2520
gcatctcaat tagtcagcaa ccaggtgtgg aaagtcccca ggctccccag caggcagaag 2580
tatgcaaagc atgcatctca attagtcagc aaccatagtc ccgcccctaa ctccgcccat 2640
cccgccccta actccgccca gttccgccca ttctccgccc catggctgac taattttttt 2700
tatttatgca gaggccgagg ccgcctctgc ctctgagcta ttccagaagt agtgaggagg 2760
cttttttgga ggcctaggct tttgcaaaaa gctcccggga gcttgtatat ccattttcgg 2820
atctgatcag cacgtgatga aaaagcctga actcaccgcg acgtctgtcg agaagtttct 2880
gatcgaaaag ttcgacagcg tgtccgacct gatgcagctc tcggagggcg aagaatctcg 2940
tgctttcagc ttcgatgtag gagggcgtgg atatgtcctg cgggtaaata gctgcgccga 3000

tggtttctac aaagatcgtt atgtttatcg gcactttgca tcggccgcgc tcccgattcc 3060
ggaagtgctt gacattgggg aattcagcga gagcctgacc tattgcatct cccgccgtgc 3120
acagggtgtc acgttgcaag acttgcctga aaccgaactg cccgctgttc tgcagccggt 3180
cgcggaggcc atggatgcga tcgctgcggc cgatcttagc cagacgagcg ggttcggccc 3240
attcggaccg caaggaatcg gtcaatacac tacatggcgt gatttcatat gcgcgattgc 3300
tgatccccat gtgtatcact ggcaaactgt gatggacgac accgtcagtg cgtccgtcgc 3360
gcaggctctc gatgagctga tgctttgggc cgaggactgc cccgaagtcc ggcacctcgt 3420
gcacgcggat ttcggctcca acaatgtcct gacggacaat ggccgcataa cagcggtcat 3480
tgactggagc gaggcgatgt tcggggattc ccaatacgag gtcgccaaca tcttcttctg 3540
gaggccgtgg ttggcttgta tggagcagca gacgcgctac ttcgagcgga ggcatccgga 3600
gcttgcagga tcgccgcggc tccgggcgta tatgctccgc attggtcttg accaactcta 3660
tcagagcttg gttgacggca atttcgatga tgcagcttgg gcgcagggtc gatgcgacgc 3720
aatcgtccga tccggagccg ggactgtcgg gcgtacacaa atcgcccgca gaagcgcggc 3780
cgtctggacc gatggctgtg tagaagtact cgccgatagt ggaaaccgac gccccagcac 3840
tcgtccgagg gcaaaggaat agcacgtgct acgagatttc gattccaccg ccgccttcta 3900
tgaaaggttg ggcttcggaa tcgttttccg ggacgccggc tggatgatcc tccagcgcgg 3960
ggatctcatg ctggagttct tcgcccaccc caacttgttt attgcagctt ataatggtta 4020
caaataaagc aatagcatca caaatttcac aaataaagca tttttttcac tgcattctag 4080
ttgtggtttg tccaaactca tcaatgtatc ttatgcgtct tctagggtta aggttagtgt 4140
agagaagcaa ccgaagattg agaagacatg gcggtaatac ggttatccac agaatcaggg 4200
gataacgcag gaaagaacat gtgagcaaaa ggccagcaaa aggccaggaa ccgtaaaaag 4260
gccgcgttgc tggcgttttt ccataggctc cgcccccctg acgagcatca caaaaatcga 4320
cgctcaagtc agaggtggcg aaacccgaca ggactataaa gataccaggc gtttccccct 4380
ggaagctccc tcgtgcgctc tcctgttccg accctgccgc ttaccggata cctgtccgcc 4440
tttctccctt cgggaagcgt ggcgctttct catagctcac gctgtaggta tctcagttcg 4500
gtgtaggtcg ttcgctccaa gctgggctgt gtgcacgaac cccccgttca gcccgaccgc 4560
tgcgccttat ccggtaacta tcgtcttgag tccaacccgg taagacacga cttatcgcca 4620
ctggcagcag ccactggtaa caggattagc agagcgaggt atgtaggcgg tgctacagag 4680
ttcttgaagt ggtggcctaa ctacggctac actagaagaa cagtatttgg tatctgcgct 4740
ctgctgaagc cagttacctt cggaaaaaga gttggtagct cttgatccgg caaacaaacc 4800
accgctggta gcggtttttt tgtttgcaag cagcagatta cgcgcagaaa aaaggatct 4860
caagaagatc ctttgatctt ttctacgggg tctgacgctc agtggaacga aaactcacgt 4920

taagggattt tggtcatgag attatcaaaa aggatcttca cctagatcct tttaaattaa 4980
aaatgaagtt ttaaatcaat ctaaagtata tatgagtaaa cttggtctga cagttaccaa 5040
tgcttaatca gtgaggcacc tatctcagcg atctgtctat ttcgttcatc catagttgcc 5100
tgactccccg tcgtgtagat aactacgata cgggagggct taccatctgg ccccagtgct 5160
gcaatgatac cgcgtgaccc acgctcaccg gctccagatt tatcagcaat aaaccagcca 5220
gccggaaggg ccgagcgcag aagtggtcct gcaactttat ccgcctccat ccagtctatt 5280
aattgttgcc gggaagctag agtaagtagt tcgccagtta atagtttgcg caacgttgtt 5340
gccattgcta caggcatcgt ggtgtcacgc tcgtcgtttg gtatggcttc attcagctcc 5400
ggttcccaac gatcaaggcg agttacatga tcccccatgt tgtgcaaaaa agcggttagc 5460
tccttcggtc ctccgatcgt tgtcagaagt aagttggccg cagtgttatc actcatggtt 5520
atggcagcac tgcataattc tcttactgtc atgccatccg taagatgctt ttctgtgact 5580
ggtgagtact caaccaagtc attctgagaa tagtgtatgc ggcgaccgag ttgctcttgc 5640
ccggcgtcaa tacgggataa taccgcgcca catagcagaa ctttaaaagt gctcatcatt 5700
ggaaaacgtt cttcggggcg aaaactctca aggatcttac cgctgttgag atccagttcg 5760
atgtaaccca ctcgtgcacc caactgatct tcagcatctt ttactttcac cagcgtttct 5820
gggtgagcaa aaacaggaag gcaaaatgcc gcaaaaaagg gaataagggc gacacggaaa 5880
tgttgaatac tcatactctt cctttttcaa tattattgaa gcatttatca gggttattgt 5940
ctcatgagcg gatacatatt tgaatgtatt tagaaaaata aacaaatagg ggttccgcgc 6000
acatttcccc gaaaagtgcc acctatgaga cg 6032

<210> 32
<211> 6032
<212> DNA
<213> Artificial sequence

<220>
<223> pHCsiaT-ECFP

<400> 32

```
tgaggctagg gataggacga gagcatcggg aacgaggact agcgtctcaa ggaaccaatt      60
cagtcgactg gatcctagtt attaatagta atcaattacg gggtcattag ttcatagccc     120
atatatggag ttccgcgtta cataacttac ggtaaatggc ccgcctggct gaccgcccaa     180
cgacccccgc ccattgacgt caataatgac gtatgttccc atagtaacgc caatagggac     240
tttccattga cgtcaatggg tggagtattt acggtaaact gcccacttgg cagtacatca     300
agtgtatcat atgccaagta cgccccctat tgacgtcaat gacggtaaat ggcccgcctg     360
gcattatgcc cagtacatga ccttatggga ctttcctact tggcagtaca tctacgtatt     420
```

agtcatcgct attaccatgg tgatgcggtt ttggcagtac atcaatgggc gtggatagcg 480
gtttgactca cggggatttc caagtctcca ccccattgac gtcaatggga gtttgttttg 540
gcaccaaaat caacgggact ttccaaaatg tcgtaacaac tccgccccat tgacgcaaat 600
gggcggtagg cgtgtacggt gggaggtcta tataagcaga gctggtttag tgaaccgtca 660
gatcactagt cgactaggga taacagggcc gccaccatga ttcacaccaa cctgaagaaa 720
aagttcagct gctgcgtcct ggtctttctt ctgtttgcag tcatctgtgt gtggaaggaa 780
aagaagaaag ggagttacta tgattccttt aaattgcaaa ccaaggaatt ccaggtgtta 840
aagagtctgg ggaaattggc catggggtct gattcccagt ctgtatcctc aagcagcacc 900
caggaccccc acaggggccg ccagaccctc ggcagtctca gaggcctagc caaggccaaa 960
ccagaggcct ccttccaggt gtggaacaag gacagctctt ccaaaaacct tatccctagg 1020
ctgcaaaagg ggtcgggggt gagcaagggc gaggagctgt tcaccggggt ggtgcccatc 1080
ctggtcgagc tggacggcga cgtaaacggc cacaagttca gcgtgtccgg cgagggcgag 1140
ggcgatgcca cctacggcaa gctgaccctg aagttcatct gcaccaccgg caagctgccc 1200
gtgccctggc ccaccctcgt gaccaccctg acctggggcg tgcagtgctt cagccgctac 1260
cccgaccaca tgaagcagca cgacttcttc aagtccgcca tgcccgaagg ctacgtccag 1320
gagcgcacca tcttcttcaa ggacgacggc aactacaaga cccgcgccga ggtgaagttc 1380
gagggcgaca ccctggtgaa ccgcatcgag ctgaagggca tcgacttcaa ggaggacggc 1440
aacatcctgg ggcacaagct ggagtacaac tacatcagcc acaacgtcta tatcaccgcc 1500
gacaagcaga agaacggcat caaggccaac ttcaagatcc gccacaacat cgaggacggc 1560
agcgtgcagc tcgccgacca ctaccagcag aacaccccca tcggcgacgg ccccgtgctg 1620
ctgcccgaca accactacct gagcacccag tccgccctga gcaaagaccc caacgagaag 1680
cgcgatcaca tggtcctgct ggagttcgtg accgccgccg ggatcactct cggcatggac 1740
gagctgtaca agtgatcgac tgtgccttct agttgccagc catctgttgt ttgcccctcc 1800
cccgtgcctt ccttgaccct ggaaggtgcc actcccactg tcctttccta ataaaatgag 1860
gaaattgcat cgcattgtct gagtaggtgt cattctattc tggggggtgg ggtggggcag 1920
gacagcaagg gggaggattg ggaggacaat agcaggcatg ctggggatgc ggtgggctct 1980
atggcttctc cctgcaggtg cgcccaatac gcaaaccgcc tctccccgcg cgttggccga 2040
ttcattaatg cagctggcac gacaggtttc ccgactggaa agcgggcagt gagcgcaacg 2100
caattaatgt gagttagctc actcattagg caccccaggc tttacacttt atgcttccgg 2160
ctcgtatgtt gtgtggaatt gtgagcggat aacaatttca cacaggaaac agctatgacc 2220
atgattacgg acagcctggc cgtcgtttta caacgtcgtg actgggaaaa ccctggcgtt 2280
acccaactta atcgccttgc agcacatccc cctttcgcca gctggcgtaa tagcgaagag 2340

gcccgcaccg atcgcccttc ccaacagttg cgcagcctga atggcgaatg gcgcctgatg 2400
cggtattttc tccttacgca tctgtgcggt atttcacacc gcatatggtg cactctcagt 2460
acaatctgct ctgatgccgc atagacctgc agggccagca ggcagaagta tgcaaagcat 2520
gcatctcaat tagtcagcaa ccaggtgtgg aaagtcccca ggctccccag caggcagaag 2580
tatgcaaagc atgcatctca attagtcagc aaccatagtc ccgcccctaa ctccgcccat 2640
cccgccccta actccgccca gttccgccca ttctccgccc catggctgac taattttttt 2700
tatttatgca gaggccgagg ccgcctctgc ctctgagcta ttccagaagt agtgaggagg 2760
cttttttgga ggcctaggct tttgcaaaaa gctcccggga gcttgtatat ccattttcgg 2820
atctgatcag cacgtgatga aaaagcctga actcaccgcg acgtctgtcg agaagtttct 2880
gatcgaaaag ttcgacagcg tgtccgacct gatgcagctc tcggagggcg aagaatctcg 2940
tgctttcagc ttcgatgtag gagggcgtgg atatgtcctg cgggtaaata gctgcgccga 3000
tggtttctac aaagatcgtt atgtttatcg gcactttgca tcggccgcgc tcccgattcc 3060
ggaagtgctt gacattgggg aattcagcga gagcctgacc tattgcatct cccgccgtgc 3120
acagggtgtc acgttgcaag acttgcctga aaccgaactg cccgctgttc tgcagccggt 3180
cgcggaggcc atggatgcga tcgctgcggc cgatcttagc cagacgagcg ggttcggccc 3240
attcggaccg caaggaatcg gtcaatacac tacatggcgt gatttcatat gcgcgattgc 3300
tgatccccat gtgtatcact ggcaaactgt gatggacgac accgtcagtg cgtccgtcgc 3360
gcaggctctc gatgagctga tgctttgggc cgaggactgc cccgaagtcc ggcacctcgt 3420
gcacgcggat ttcggctcca acaatgtcct gacggacaat ggccgcataa cagcggtcat 3480
tgactggagc gaggcgatgt tcggggattc ccaatacgag gtcgccaaca tcttcttctg 3540
gaggccgtgg ttggcttgta tggagcagca gacgcgctac ttcgagcgga ggcatccgga 3600
gcttgcagga tcgccgcggc tccgggcgta tatgctccgc attggtcttg accaactcta 3660
tcagagcttg gttgacggca atttcgatga tgcagcttgg gcgcagggtc gatgcgacgc 3720
aatcgtccga tccggagccg ggactgtcgg gcgtacacaa atcgcccgca gaagcgcggc 3780
cgtctggacc gatggctgtg tagaagtact cgccgatagt ggaaaccgac gccccagcac 3840
tcgtccgagg gcaaaggaat agcacgtgct acgagatttc gattccaccg ccgccttcta 3900
tgaaaggttg ggcttcggaa tcgttttccg ggacgccggc tggatgatcc tccagcgcgg 3960
ggatctcatg ctggagttct tcgcccaccc caacttgttt attgcagctt ataatggtta 4020
caaataaagc aatagcatca caaatttcac aaataaagca tttttttcac tgcattctag 4080
ttgtggtttg tccaaactca tcaatgtatc ttatgcgtct tctagggtta aggttagtgt 4140
agagaagcaa ccgaagattg agaagacatg gcggtaatac ggttatccac agaatcaggg 4200

gataacgcag gaaagaacat gtgagcaaaa ggccagcaaa aggccaggaa ccgtaaaaag 4260
gccgcgttgc tggcgttttt ccataggctc cgcccccctg acgagcatca caaaaatcga 4320
cgctcaagtc agaggtggcg aaacccgaca ggactataaa gataccaggc gtttccccct 4380
ggaagctccc tcgtgcgctc tcctgttccg accctgccgc ttaccggata cctgtccgcc 4440
tttctccctt cgggaagcgt ggcgctttct catagctcac gctgtaggta tctcagttcg 4500
gtgtaggtcg ttcgctccaa gctgggctgt gtgcacgaac cccccgttca gcccgaccgc 4560
tgcgccttat ccggtaacta tcgtcttgag tccaacccgg taagacacga cttatcgcca 4620
ctggcagcag ccactggtaa caggattagc agagcgaggt atgtaggcgg tgctacagag 4680
ttcttgaagt ggtggcctaa ctacggctac actagaagaa cagtatttgg tatctgcgct 4740
ctgctgaagc cagttacctt cggaaaaaga gttggtagct cttgatccgg caaacaaacc 4800
accgctggta gcggtttttt tgtttgcaag cagcagatta cgcgcagaaa aaaaggatct 4860
caagaagatc ctttgatctt ttctacgggg tctgacgctc agtggaacga aaactcacgt 4920
taagggattt tggtcatgag attatcaaaa aggatcttca cctagatcct tttaaattaa 4980
aaatgaagtt ttaaatcaat ctaaagtata tatgagtaaa cttggtctga cagttaccaa 5040
tgcttaatca gtgaggcacc tatctcagcg atctgtctat ttcgttcatc catagttgcc 5100
tgactccccg tcgtgtagat aactacgata cgggagggct taccatctgg ccccagtgct 5160
gcaatgatac cgcgtgaccc acgctcaccg gctccagatt tatcagcaat aaaccagcca 5220
gccggaaggg ccgagcgcag aagtggtcct gcaactttat ccgcctccat ccagtctatt 5280
aattgttgcc gggaagctag agtaagtagt tcgccagtta atagtttgcg caacgttgtt 5340
gccattgcta caggcatcgt ggtgtcacgc tcgtcgtttg gtatggcttc attcagctcc 5400
ggttcccaac gatcaaggcg agttacatga tcccccatgt tgtgcaaaaa agcggttagc 5460
tccttcggtc ctccgatcgt tgtcagaagt aagttggccg cagtgttatc actcatggtt 5520
atggcagcac tgcataattc tcttactgtc atgccatccg taagatgctt ttctgtgact 5580
ggtgagtact caaccaagtc attctgagaa tagtgtatgc ggcgaccgag ttgctcttgc 5640
ccggcgtcaa tacgggataa taccgcgcca catagcagaa ctttaaaagt gctcatcatt 5700
ggaaaacgtt cttcggggcg aaaactctca aggatcttac cgctgttgag atccagttcg 5760
atgtaaccca ctcgtgcacc caactgatct tcagcatctt ttactttcac cagcgtttct 5820
gggtgagcaa aaacaggaag gcaaaatgcc gcaaaaaagg gaataagggc gacacggaaa 5880
tgttgaatac tcatactctt cctttttcaa tattattgaa gcatttatca gggttattgt 5940
ctcatgagcg gatacatatt tgaatgtatt tagaaaaata aacaaatagg ggttccgcgc 6000
acatttcccc gaaaagtgcc acctatgaga cg 6032

<210> 33
<211> 6032
<212> DNA
<213> Artificial sequence

<220>
<223> pHCsiaT-EYFP

<400> 33

```
tgaggctagg gataggacga gagcatcggg aacgaggact agcgtctcaa ggaaccaatt      60
cagtcgactg gatcctagtt attaatagta atcaattacg gggtcattag ttcatagccc     120
atatatggag ttccgcgtta cataacttac ggtaaatggc ccgcctggct gaccgcccaa     180
cgacccccgc ccattgacgt caataatgac gtatgttccc atagtaacgc caatagggac     240
tttccattga cgtcaatggg tggagtattt acggtaaact gcccacttgg cagtacatca     300
agtgtatcat atgccaagta cgccccctat tgacgtcaat gacggtaaat ggcccgcctg     360
gcattatgcc cagtacatga ccttatggga ctttcctact tggcagtaca tctacgtatt     420
agtcatcgct attaccatgg tgatgcggtt ttggcagtac atcaatgggc gtggatagcg     480
gtttgactca cggggatttc caagtctcca ccccattgac gtcaatggga gtttgttttg     540
gcaccaaaat caacgggact ttccaaaatg tcgtaacaac tccgccccat tgacgcaaat     600
gggcggtagg cgtgtacggt gggaggtcta tataagcaga gctggtttag tgaaccgtca     660
gatcactagt cgactaggga taacagggcc gccaccatga ttcacaccaa cctgaagaaa     720
aagttcagct gctgcgtcct ggtctttctt ctgtttgcag tcatctgtgt gtggaaggaa     780
aagaagaaag ggagttacta tgattccttt aaattgcaaa ccaaggaatt ccaggtgtta     840
aagagtctgg ggaaattggc catggggtct gattcccagt ctgtatcctc aagcagcacc     900
caggaccccc acaggggccg ccagaccctc ggcagtctca gaggcctagc caaggccaaa     960
ccagaggcct ccttccaggt gtggaacaag gacagctctt ccaaaaacct tatccctagg    1020
ctgcaaaagg ggtcgggggt gagcaagggc gaggagctgt tcaccggggt ggtgcccatc    1080
ctggtcgagc tggacggcga cgtaaacggc cacaagttca gcgtgtccgg cgagggcgag    1140
ggcgatgcca cctacggcaa gctgaccctg aagttcatct gcaccaccgg caagctgccc    1200
gtgccctggc ccaccctcgt gaccaccttc ggctacggcc tgcagtgctt cgcccgctac    1260
cccgaccaca tgaagcagca cgacttcttc aagtccgcca tgcccgaagg ctacgtccag    1320
gagcgcacca tcttcttcaa ggacgacggc aactacaaga cccgcgccga ggtgaagttc    1380
gagggcgaca ccctggtgaa ccgcatcgag ctgaagggca tcgacttcaa ggaggacggc    1440
aacatcctgg ggcacaagct ggagtacaac tacaacagcc acaacgtcta tatcatggcc    1500
gacaagcaga agaacggcat caaggtgaac ttcaagatcc gccacaacat cgaggacggc    1560
agcgtgcagc tcgccgacca ctaccagcag aacaccccca tcggcgacgg ccccgtgctg    1620
```

ctgcccgaca accactacct gagctaccag tccgccctga gcaaagaccc caacgagaag 1680

cgcgatcaca tggtcctgct ggagttcgtg accgccgccg ggatcactct cggcatggac 1740

gagctgtaca agtgatcgac tgtgccttct agttgccagc catctgttgt ttgcccctcc 1800

cccgtgcctt ccttgaccct ggaaggtgcc actcccactg tcctttccta ataaaatgag 1860

gaaattgcat cgcattgtct gagtaggtgt cattctattc tggggggtgg ggtggggcag 1920

gacagcaagg gggaggattg ggaggacaat agcaggcatg ctggggatgc ggtgggctct 1980

atggcttctc cctgcaggtg cgcccaatac gcaaaccgcc tctccccgcg cgttggccga 2040

ttcattaatg cagctggcac gacaggtttc ccgactggaa agcgggcagt gagcgcaacg 2100

caattaatgt gagttagctc actcattagg caccccaggc tttacacttt atgcttccgg 2160

ctcgtatgtt gtgtggaatt gtgagcggat aacaatttca cacaggaaac agctatgacc 2220

atgattacgg acagcctggc cgtcgtttta caacgtcgtg actgggaaaa ccctggcgtt 2280

acccaactta atcgccttgc agcacatccc cctttcgcca gctggcgtaa tagcgaagag 2340

gcccgcaccg atcgcccttc ccaacagttg cgcagcctga atggcgaatg gcgcctgatg 2400

cggtattttc tccttacgca tctgtgcggt atttcacacc gcatatggtg cactctcagt 2460

acaatctgct ctgatgccgc atagacctgc agggccagca ggcagaagta tgcaaagcat 2520

gcatctcaat tagtcagcaa ccaggtgtgg aaagtcccca ggctccccag caggcagaag 2580

tatgcaaagc atgcatctca attagtcagc aaccatagtc ccgcccctaa ctccgcccat 2640

cccgccccta actccgccca gttccgccca ttctccgccc catggctgac taattttttt 2700

tatttatgca gaggccgagg ccgcctctgc ctctgagcta ttccagaagt agtgaggagg 2760

cttttttgga ggcctaggct tttgcaaaaa gctcccggga gcttgtatat ccattttcgg 2820

atctgatcag cacgtgatga aaaagcctga actcaccgcg acgtctgtcg agaagtttct 2880

gatcgaaaag ttcgacagcg tgtccgacct gatgcagctc tcggagggcg aagaatctcg 2940

tgctttcagc ttcgatgtag gagggcgtgg atatgtcctg cgggtaaata gctgcgccga 3000

tggtttctac aaagatcgtt atgtttatcg gcactttgca tcggccgcgc tcccgattcc 3060

ggaagtgctt gacattgggg aattcagcga gagcctgacc tattgcatct cccgccgtgc 3120

acagggtgtc acgttgcaag acttgcctga aaccgaactg cccgctgttc tgcagccggt 3180

cgcggaggcc atggatgcga tcgctgcggc cgatcttagc cagacgagcg ggttcggccc 3240

attcggaccg caaggaatcg gtcaatacac tacatggcgt gatttcatat gcgcgattgc 3300

tgatccccat gtgtatcact ggcaaactgt gatggacgac accgtcagtg cgtccgtcgc 3360

gcaggctctc gatgagctga tgctttgggc cgaggactgc cccgaagtcc ggcacctcgt 3420

gcacgcggat ttcggctcca acaatgtcct gacggacaat ggccgcataa cagcggtcat 3480

tgactggagc gaggcgatgt tcggggattc ccaatacgag gtcgccaaca tcttcttctg 3540

gaggccgtgg ttggcttgta tggagcagca gacgcgctac ttcgagcgga ggcatccgga 3600
gcttgcagga tcgccgcggc tccgggcgta tatgctccgc attggtcttg accaactcta 3660
tcagagcttg gttgacggca atttcgatga tgcagcttgg gcgcagggtc gatgcgacgc 3720
aatcgtccga tccggagccg ggactgtcgg gcgtacacaa atcgcccgca gaagcgcggc 3780
cgtctggacc gatggctgtg tagaagtact cgccgatagt ggaaaccgac gccccagcac 3840
tcgtccgagg gcaaaggaat agcacgtgct acgagatttc gattccaccg ccgccttcta 3900
tgaaaggttg ggcttcggaa tcgttttccg ggacgccggc tggatgatcc tccagcgcgg 3960
ggatctcatg ctggagttct tcgcccaccc caacttgttt attgcagctt ataatggtta 4020
caaataaagc aatagcatca caaatttcac aaataaagca tttttttcac tgcattctag 4080
ttgtggtttg tccaaactca tcaatgtatc ttatgcgtct tctagggtta aggttagtgt 4140
agagaagcaa ccgaagattg agaagacatg gcggtaatac ggttatccac agaatcaggg 4200
gataacgcag gaaagaacat gtgagcaaaa ggccagcaaa aggccaggaa ccgtaaaaag 4260
gccgcgttgc tggcgttttt ccataggctc cgcccccctg acgagcatca caaaaatcga 4320
cgctcaagtc agaggtggcg aaacccgaca ggactataaa gataccaggc gtttccccct 4380
ggaagctccc tcgtgcgctc tcctgttccg accctgccgc ttaccggata cctgtccgcc 4440
tttctccctt cgggaagcgt ggcgctttct catagctcac gctgtaggta tctcagttcg 4500
gtgtaggtcg ttcgctccaa gctgggctgt gtgcacgaac cccccgttca gcccgaccgc 4560
tgcgccttat ccggtaacta tcgtcttgag tccaacccgg taagacacga cttatcgcca 4620
ctggcagcag ccactggtaa caggattagc agagcgaggt atgtaggcgg tgctacagag 4680
ttcttgaagt ggtggcctaa ctacggctac actagaagaa cagtatttgg tatctgcgct 4740
ctgctgaagc cagttacctt cggaaaaaga gttggtagct cttgatccgg caaacaaacc 4800
accgctggta gcggtttttt tgtttgcaag cagcagatta cgcgcagaaa aaaaggatct 4860
caagaagatc ctttgatctt ttctacgggg tctgacgctc agtggaacga aaactcacgt 4920
taagggattt tggtcatgag attatcaaaa aggatcttca cctagatcct tttaaattaa 4980
aaatgaagtt ttaaatcaat ctaaagtata tatgagtaaa cttggtctga cagttaccaa 5040
tgcttaatca gtgaggcacc tatctcagcg atctgtctat ttcgttcatc catagttgcc 5100
tgactccccg tcgtgtagat aactacgata cgggagggct taccatctgg ccccagtgct 5160
gcaatgatac cgcgtgaccc acgctcaccg gctccagatt tatcagcaat aaaccagcca 5220
gccggaaggg ccgagcgcag aagtggtcct gcaactttat ccgcctccat ccagtctatt 5280
aattgttgcc gggaagctag agtaagtagt tcgccagtta atagtttgcg caacgttgtt 5340
gccattgcta caggcatcgt ggtgtcacgc tcgtcgtttg gtatggcttc attcagctcc 5400

ggttcccaac gatcaaggcg agttacatga tcccccatgt tgtgcaaaaa agcggttagc 5460
tccttcggtc ctccgatcgt tgtcagaagt aagttggccg cagtgttatc actcatggtt 5520
atggcagcac tgcataattc tcttactgtc atgccatccg taagatgctt ttctgtgact 5580
ggtgagtact caaccaagtc attctgagaa tagtgtatgc ggcgaccgag ttgctcttgc 5640
ccggcgtcaa tacgggataa taccgcgcca catagcagaa ctttaaaagt gctcatcatt 5700
ggaaaacgtt cttcggggcg aaaactctca aggatcttac cgctgttgag atccagttcg 5760
atgtaaccca ctcgtgcacc caactgatct tcagcatctt ttactttcac cagcgtttct 5820
gggtgagcaa aaacaggaag gcaaaatgcc gcaaaaaagg gaataagggc gacacggaaa 5880
tgttgaatac tcatactctt cctttttcaa tattattgaa gcatttatca gggttattgt 5940
ctcatgagcg gatacatatt tgaatgtatt tagaaaaata aacaaatagg ggttccgcgc 6000
acatttcccc gaaaagtgcc acctatgaga cg 6032

<210> 34
<211> 6023
<212> DNA
<213> Artificial sequence

<220>
<223> pHCsiaT-mCherry

<400> 34

tgaggctagg gataggacga gagcatcggg aacgaggact agcgtctcaa ggaaccaatt 60

cagtcgactg gatcctagtt attaatagta atcaattacg gggtcattag ttcatagccc 120

atatatggag ttccgcgtta cataacttac ggtaaatggc ccgcctggct gaccgcccaa 180

cgacccccgc ccattgacgt caataatgac gtatgttccc atagtaacgc caatagggac 240

tttccattga cgtcaatggg tggagtattt acggtaaact gcccacttgg cagtacatca 300

agtgtatcat atgccaagta cgccccctat tgacgtcaat gacggtaaat ggcccgcctg 360

gcattatgcc cagtacatga ccttatggga ctttcctact tggcagtaca tctacgtatt 420

agtcatcgct attaccatgg tgatgcggtt ttggcagtac atcaatgggc gtggatagcg 480

gtttgactca cggggatttc caagtctcca ccccattgac gtcaatggga gtttgttttg 540

gcaccaaaat caacgggact ttccaaaatg tcgtaacaac tccgccccat tgacgcaaat 600

gggcggtagg cgtgtacggt gggaggtcta tataagcaga gctggtttag tgaaccgtca 660

gatcactagt cgactaggga taacagggcc gccaccatga ttcacaccaa cctgaagaaa 720

aagttcagct gctgcgtcct ggtctttctt ctgtttgcag tcatctgtgt gtggaaggaa 780

aagaagaaag ggagttacta tgattccttt aaattgcaaa ccaaggaatt ccaggtgtta 840

aagagtctgg ggaaattggc catggggtct gattcccagt ctgtatcctc aagcagcacc 900

caggaccccc acagggccg ccagaccctc ggcagtctca gaggcctagc caaggccaaa 960

ccagaggcct ccttccaggt gtggaacaag gacagctctt ccaaaaacct tatccctagg 1020
ctgcaaaagg ggtcgggggt gagcaagggc gaggaggata acatggccat catcaaggag 1080
ttcatgcgct tcaaggtgca catggagggc tccgtgaacg gccacgagtt cgagatcgag 1140
ggcgagggcg agggccgccc ctacgagggc acccagaccg ccaagctgaa ggtgaccaag 1200
ggtggccccc tgcccttcgc ctgggacatc ctgtcccctc agttcatgta cggctccaag 1260
gcctacgtga agcaccccgc cgacatcccc gactacttga agctgtcctt ccccgagggc 1320
ttcaagtggg agcgcgtgat gaacttcgag gacggcggcg tggtgaccgt gacccaggac 1380
tcctccctgc aggacggcga gttcatctac aaggtgaagc tgcgcggcac caacttcccc 1440
tccgacggcc ccgtaatgca gaagaaaacc atgggctggg aggcctcctc cgagcggatg 1500
taccccgagg acggcgccct gaagggcgag atcaagcaga ggctgaagct gaaggacggc 1560
ggccactacg acgctgaggt caagaccacc tacaaggcca agaagcccgt gcagctgccc 1620
ggcgcctaca acgtcaacat caagttggac atcacctccc acaacgagga ctacaccatc 1680
gtggaacagt acgaacgcgc cgagggccgc cactccaccg gcggcatgga cgagctgtac 1740
aagtgatcga ctgtgccttc tagttgccag ccatctgttg tttgcccctc ccccgtgcct 1800
tccttgaccc tggaaggtgc cactcccact gtcctttcct aataaaatga ggaaattgca 1860
tcgcattgtc tgagtaggtg tcattctatt ctggggggtg gggtggggca ggacagcaag 1920
ggggaggatt gggaggacaa tagcaggcat gctggggatg cggtgggctc tatggcttct 1980
ccctgcaggt gcgcccaata cgcaaaccgc ctctccccgc gcgttggccg attcattaat 2040
gcagctggca cgacaggttt cccgactgga aagcgggcag tgagcgcaac gcaattaatg 2100
tgagttagct cactcattag gcaccccagg ctttacactt tatgcttccg gctcgtatgt 2160
tgtgtggaat tgtgagcgga taacaatttc acacaggaaa cagctatgac catgattacg 2220
gacagcctgg ccgtcgtttt acaacgtcgt gactgggaaa accctggcgt tacccaactt 2280
aatcgccttg cagcacatcc ccctttcgcc agctggcgta atagcgaaga ggcccgcacc 2340
gatcgccctt cccaacagtt gcgcagcctg aatggcgaat ggcgcctgat gcggtatttt 2400
ctccttacgc atctgtgcgg tatttcacac cgcatatggt gcactctcag tacaatctgc 2460
tctgatgccg catagacctg cagggccagc aggcagaagt atgcaaagca tgcatctcaa 2520
ttagtcagca accaggtgtg gaaagtcccc aggctcccca gcaggcagaa gtatgcaaag 2580
catgcatctc aattagtcag caaccatagt cccgccccta actccgccca tcccgcccct 2640
aactccgccc agttccgccc attctccgcc ccatggctga ctaatttttt ttatttatgc 2700
agaggccgag gccgcctctg cctctgagct attccagaag tagtgaggag gcttttttgg 2760
aggcctaggc ttttgcaaaa agctcccggg agcttgtata tccattttcg gatctgatca 2820

gcacgtgatg aaaaagcctg aactcaccgc gacgtctgtc gagaagtttc tgatcgaaaa 2880

gttcgacagc gtgtccgacc tgatgcagct ctcggagggc gaagaatctc gtgctttcag 2940

cttcgatgta ggagggcgtg gatatgtcct gcgggtaaat agctgcgccg atggtttcta 3000

caaagatcgt tatgtttatc ggcactttgc atcggccgcg ctcccgattc cggaagtgct 3060

tgacattggg gaattcagcg agagcctgac ctattgcatc tcccgccgtg cacagggtgt 3120

cacgttgcaa gacttgcctg aaaccgaact gcccgctgtt ctgcagccgg tcgcggaggc 3180

catggatgcg atcgctgcgg ccgatcttag ccagacgagc gggttcggcc cattcggacc 3240

gcaaggaatc ggtcaataca ctacatggcg tgatttcata tgcgcgattg ctgatcccca 3300

tgtgtatcac tggcaaactg tgatggacga caccgtcagt gcgtccgtcg cgcaggctct 3360

cgatgagctg atgctttggg ccgaggactg ccccgaagtc cggcacctcg tgcacgcgga 3420

tttcggctcc aacaatgtcc tgacggacaa tggccgcata acagcggtca ttgactggag 3480

cgaggcgatg ttcggggatt cccaatacga ggtcgccaac atcttcttct ggaggccgtg 3540

gttggcttgt atggagcagc agacgcgcta cttcgagcgg aggcatccgg agcttgcagg 3600

atcgccgcgg ctccgggcgt atatgctccg cattggtctt gaccaactct atcagagctt 3660

ggttgacggc aatttcgatg atgcagcttg ggcgcagggt cgatgcgacg caatcgtccg 3720

atccggagcc gggactgtcg ggcgtacaca aatcgcccgc agaagcgcgg ccgtctggac 3780

cgatggctgt gtagaagtac tcgccgatag tggaaaccga cgccccagca ctcgtccgag 3840

ggcaaaggaa tagcacgtgc tacgagattt cgattccacc gccgccttct atgaaaggtt 3900

gggcttcgga atcgttttcc gggacgccgg ctggatgatc ctccagcgcg gggatctcat 3960

gctggagttc ttcgcccacc ccaacttgtt tattgcagct tataatggtt acaaataaag 4020

caatagcatc acaaatttca caaataaagc atttttttca ctgcattcta gttgtggttt 4080

gtccaaactc atcaatgtat cttatgcgtc ttctagggtt aaggttagtg tagagaagca 4140

accgaagatt gagaagacat ggcggtaata cggttatcca cagaatcagg ggataacgca 4200

ggaaagaaca tgtgagcaaa aggccagcaa aaggccagga accgtaaaaa ggccgcgttg 4260

ctggcgtttt tccataggct ccgcccccct gacgagcatc acaaaaatcg acgctcaagt 4320

cagaggtggc gaaacccgac aggactataa agataccagg cgtttccccc tggaagctcc 4380

ctcgtgcgct ctcctgttcc gaccctgccg cttaccggat acctgtccgc ctttctccct 4440

tcgggaagcg tggcgctttc tcatagctca cgctgtaggt atctcagttc ggtgtaggtc 4500

gttcgctcca agctgggctg tgtgcacgaa ccccccgttc agcccgaccg ctgcgcctta 4560

tccggtaact atcgtcttga gtccaacccg gtaagacacg acttatcgcc actggcagca 4620

gccactggta acaggattag cagagcgagg tatgtaggcg gtgctacaga gttcttgaag 4680

tggtggccta actacggcta cactagaaga acagtatttg gtatctgcgc tctgctgaag 4740

ccagttacct tcggaaaaag agttggtagc tcttgatccg gcaaacaaac caccgctggt 4800
agcggttttt ttgtttgcaa gcagcagatt acgcgcagaa aaaaaggatc tcaagaagat 4860
cctttgatct tttctacggg gtctgacgct cagtggaacg aaaactcacg ttaagggatt 4920
ttggtcatga gattatcaaa aaggatcttc acctagatcc ttttaaatta aaaatgaagt 4980
tttaaatcaa tctaaagtat atatgagtaa acttggtctg acagttacca atgcttaatc 5040
agtgaggcac ctatctcagc gatctgtcta tttcgttcat ccatagttgc ctgactcccc 5100
gtcgtgtaga taactacgat acgggagggc ttaccatctg gccccagtgc tgcaatgata 5160
ccgcgtgacc cacgctcacc ggctccagat ttatcagcaa taaaccagcc agccggaagg 5220
gccgagcgca gaagtggtcc tgcaacttta tccgcctcca tccagtctat taattgttgc 5280
cgggaagcta gagtaagtag ttcgccagtt aatagtttgc gcaacgttgt tgccattgct 5340
acaggcatcg tggtgtcacg ctcgtcgttt ggtatggctt cattcagctc cggttcccaa 5400
cgatcaaggc gagttacatg atcccccatg ttgtgcaaaa aagcggttag ctccttcggt 5460
cctccgatcg ttgtcagaag taagttggcc gcagtgttat cactcatggt tatggcagca 5520
ctgcataatt ctcttactgt catgccatcc gtaagatgct tttctgtgac tggtgagtac 5580
tcaaccaagt cattctgaga atagtgtatg cggcgaccga gttgctcttg cccggcgtca 5640
atacgggata ataccgcgcc acatagcaga actttaaaag tgctcatcat tggaaaacgt 5700
tcttcggggc gaaaactctc aaggatctta ccgctgttga gatccagttc gatgtaaccc 5760
actcgtgcac ccaactgatc ttcagcatct tttactttca ccagcgtttc tgggtgagca 5820
aaaacaggaa ggcaaaatgc cgcaaaaaag ggaataaggg cgacacggaa atgttgaata 5880
ctcatactct tcctttttca atattattga agcatttatc agggttattg tctcatgagc 5940
ggatacatat ttgaatgtat ttagaaaaat aaacaaatag gggttccgcg cacatttccc 6000
cgaaaagtgc cacctatgag acg 6023

<210> 35
<211> 6020
<212> DNA
<213> Artificial sequence

<220>
<223> pHCsiaT-TagBFP

<400> 35

```
tgaggctagg gataggacga gagcatcggg aacgaggact agcgtctcaa ggaaccaatt        60
cagtcgactg gatcctagtt attaatagta atcaattacg gggtcattag ttcatagccc       120
atatatggag ttccgcgtta cataacttac ggtaaatggc ccgcctggct gaccgcccaa       180
cgacccccgc ccattgacgt caataatgac gtatgttccc atagtaacgc caatagggac       240
```

tttccattga cgtcaatggg tggagtattt acggtaaact gcccacttgg cagtacatca 300
agtgtatcat atgccaagta cgccccctat tgacgtcaat gacggtaaat ggcccgcctg 360
gcattatgcc cagtacatga ccttatggga ctttcctact tggcagtaca tctacgtatt 420
agtcatcgct attaccatgg tgatgcggtt ttggcagtac atcaatgggc gtggatagcg 480
gtttgactca cggggatttc caagtctcca ccccattgac gtcaatggga gtttgttttg 540
gcaccaaaat caacgggact ttccaaaatg tcgtaacaac tccgccccat tgacgcaaat 600
gggcggtagg cgtgtacggt gggaggtcta tataagcaga gctggtttag tgaaccgtca 660
gatcactagt cgactaggga taacagggcc gccaccatga ttcacaccaa cctgaagaaa 720
aagttcagct gctgcgtcct ggtctttctt ctgtttgcag tcatctgtgt gtggaaggaa 780
aagaagaaag ggagttacta tgattccttt aaattgcaaa ccaaggaatt ccaggtgtta 840
aagagtctgg ggaaattggc catggggtct gattcccagt ctgtatcctc aagcagcacc 900
caggaccccc acaggggccg ccagaccctc ggcagtctca gaggcctagc caaggccaaa 960
ccagaggcct ccttccaggt gtggaacaag gacagctctt ccaaaaacct tatccctagg 1020
ctgcaaaagg ggtcggggag cgagctgatt aaggagaaca tgcacatgaa gctgtacatg 1080
gagggcaccg tggacaacca tcacttcaag tgcacatccg agggcgaagg caagccctac 1140
gagggcaccc agaccatgag aatcaaggtg gtcgagggcg gccctctccc cttcgccttc 1200
gacatcctgg ctactagctt cctctacggc agcaagacct tcatcaacca cacccagggc 1260
atccccgact tcttcaagca gtccttccct gagggcttca catgggagag agtcaccaca 1320
tacgaggacg gggcgtgct gaccgctacc caggacacca gcctccagga cggctgcctc 1380
atctacaacg tcaagatcag aggggtgaac ttcacatcca acggccctgt gatgcagaag 1440
aaaacactcg gctgggaggc cttcaccgaa acgctgtacc ccgctgacgg cggcctggaa 1500
ggcagaaacg acatggccct gaagctcgtg ggcgggagcc atctgatcgc aaacatcaag 1560
accacatata gatccaagaa acccgctaag aacctcaaga tgcctggcgt ctactatgtg 1620
gactacagac tggaaagaat caaggaggcc aacaacgaaa cctacgtcga gcagcacgag 1680
gtggcagtgg ccagatactg cgacctccct agcaaactgg ggcacaagct taattccgga 1740
tgatcgactg tgccttctag ttgccagcca tctgttgttt gcccctcccc cgtgccttcc 1800
ttgaccctgg aaggtgccac tcccactgtc ctttcctaat aaaatgagga aattgcatcg 1860
cattgtctga gtaggtgtca ttctattctg gggggtgggg tggggcagga cagcaagggg 1920
gaggattggg aggacaatag caggcatgct ggggatgcgg tgggctctat ggcttctccc 1980
tgcaggtgcg cccaatacgc aaaccgcctc tccccgcgcg ttggccgatt cattaatgca 2040
gctggcacga caggtttccc gactggaaag cgggcagtga gcgcaacgca attaatgtga 2100
gttagctcac tcattaggca ccccaggctt tacactttat gcttccggct cgtatgttgt 2160

gtggaattgt gagcggataa caatttcaca caggaaacag ctatgaccat gattacggac 2220
agcctggccg tcgttttaca acgtcgtgac tgggaaaacc ctggcgttac ccaacttaat 2280
cgccttgcag cacatccccc tttcgccagc tggcgtaata gcgaagaggc ccgcaccgat 2340
cgcccttccc aacagttgcg cagcctgaat ggcgaatggc gcctgatgcg gtattttctc 2400
cttacgcatc tgtgcggtat ttcacaccgc atatggtgca ctctcagtac aatctgctct 2460
gatgccgcat agacctgcag ggccagcagg cagaagtatg caaagcatgc atctcaatta 2520
gtcagcaacc aggtgtggaa agtccccagg ctccccagca ggcagaagta tgcaaagcat 2580
gcatctcaat tagtcagcaa ccatagtccc gcccctaact ccgcccatcc cgcccctaac 2640
tccgcccagt tccgcccatt ctccgcccca tggctgacta atttttttta tttatgcaga 2700
ggccgaggcc gcctctgcct ctgagctatt ccagaagtag tgaggaggct tttttggagg 2760
cctaggcttt tgcaaaaagc tcccgggagc ttgtatatcc attttcggat ctgatcagca 2820
cgtgatgaaa aagcctgaac tcaccgcgac gtctgtcgag aagtttctga tcgaaaagtt 2880
cgacagcgtg tccgacctga tgcagctctc ggagggcgaa gaatctcgtg ctttcagctt 2940
cgatgtagga gggcgtggat atgtcctgcg ggtaaatagc tgcgccgatg gtttctacaa 3000
agatcgttat gtttatcggc actttgcatc ggccgcgctc ccgattccgg aagtgcttga 3060
cattggggaa ttcagcgaga gcctgaccta ttgcatctcc cgccgtgcac agggtgtcac 3120
gttgcaagac ttgcctgaaa ccgaactgcc cgctgttctg cagccggtcg cggaggccat 3180
ggatgcgatc gctgcggccg atcttagcca gacgagcggg ttcggcccat tcggaccgca 3240
aggaatcggt caatacacta catggcgtga tttcatatgc gcgattgctg atccccatgt 3300
gtatcactgg caaactgtga tggacgacac cgtcagtgcg tccgtcgcgc aggctctcga 3360
tgagctgatg ctttgggccg aggactgccc cgaagtccgg cacctcgtgc acgcggattt 3420
cggctccaac aatgtcctga cggacaatgg ccgcataaca gcggtcattg actggagcga 3480
ggcgatgttc ggggattccc aatacgaggt cgccaacatc ttcttctgga ggccgtggtt 3540
ggcttgtatg gagcagcaga cgcgctactt cgagcggagg catccggagc ttgcaggatc 3600
gccgcggctc cgggcgtata tgctccgcat tggtcttgac caactctatc agagcttggt 3660
tgacggcaat ttcgatgatg cagcttgggc gcagggtcga tgcgacgcaa tcgtccgatc 3720
cggagccggg actgtcgggc gtacacaaat cgcccgcaga agcgcggccg tctggaccga 3780
tggctgtgta gaagtactcg ccgatagtgg aaaccgacgc cccagcactc gtccgagggc 3840
aaaggaatag cacgtgctac gagatttcga ttccaccgcc gccttctatg aaaggttggg 3900
cttcggaatc gttttccggg acgccggctg gatgatcctc cagcgcgggg atctcatgct 3960
ggagttcttc gcccacccca acttgtttat tgcagcttat aatggttaca aataaagcaa 4020

tagcatcaca aatttcacaa ataaagcatt tttttcactg cattctagtt gtggtttgtc 4080
caaactcatc aatgtatctt atgcgtcttc tagggttaag gttagtgtag agaagcaacc 4140
gaagattgag aagacatggc ggtaatacgg ttatccacag aatcagggga taacgcagga 4200
aagaacatgt gagcaaaagg ccagcaaaag gccaggaacc gtaaaaaggc cgcgttgctg 4260
gcgtttttcc ataggctccg cccccctgac gagcatcaca aaaatcgacg ctcaagtcag 4320
aggtggcgaa acccgacagg actataaaga taccaggcgt ttccccctgg aagctccctc 4380
gtgcgctctc ctgttccgac cctgccgctt accggatacc tgtccgcctt tctcccttcg 4440
ggaagcgtgg cgctttctca tagctcacgc tgtaggtatc tcagttcggt gtaggtcgtt 4500
cgctccaagc tgggctgtgt gcacgaaccc cccgttcagc ccgaccgctg cgccttatcc 4560
ggtaactatc gtcttgagtc caacccggta agacacgact tatcgccact ggcagcagcc 4620
actggtaaca ggattagcag agcgaggtat gtaggcggtg ctacagagtt cttgaagtgg 4680
tggcctaact acggctacac tagaagaaca gtatttggta tctgcgctct gctgaagcca 4740
gttaccttcg gaaaaagagt tggtagctct tgatccggca aacaaaccac cgctggtagc 4800
ggtttttttg tttgcaagca gcagattacg cgcagaaaaa aaggatctca agaagatcct 4860
ttgatctttt ctacggggtc tgacgctcag tggaacgaaa actcacgtta agggattttg 4920
gtcatgagat tatcaaaaag gatcttcacc tagatccttt taaattaaaa atgaagtttt 4980
aaatcaatct aaagtatata tgagtaaact tggtctgaca gttaccaatg cttaatcagt 5040
gaggcaccta tctcagcgat ctgtctattt cgttcatcca tagttgcctg actccccgtc 5100
gtgtagataa ctacgatacg ggagggctta ccatctggcc ccagtgctgc aatgataccg 5160
cgtgacccac gctcaccggc tccagattta tcagcaataa accagccagc cggaagggcc 5220
gagcgcagaa gtggtcctgc aactttatcc gcctccatcc agtctattaa ttgttgccgg 5280
gaagctagag taagtagttc gccagttaat agtttgcgca acgttgttgc cattgctaca 5340
ggcatcgtgg tgtcacgctc gtcgtttggt atggcttcat tcagctccgg ttcccaacga 5400
tcaaggcgag ttacatgatc ccccatgttg tgcaaaaaag cggttagctc cttcggtcct 5460
ccgatcgttg tcagaagtaa gttggccgca gtgttatcac tcatggttat ggcagcactg 5520
cataattctc ttactgtcat gccatccgta agatgctttt ctgtgactgg tgagtactca 5580
accaagtcat tctgagaata gtgtatgcgg cgaccgagtt gctcttgccc ggcgtcaata 5640
cgggataata ccgcgccaca tagcagaact ttaaaagtgc tcatcattgg aaaacgttct 5700
tcggggcgaa aactctcaag gatcttaccg ctgttgagat ccagttcgat gtaacccact 5760
cgtgcaccca actgatcttc agcatctttt actttcacca gcgtttctgg gtgagcaaaa 5820
acaggaaggc aaaatgccgc aaaaaaggga ataagggcga cacggaaatg ttgaatactc 5880
atactcttcc tttttcaata ttattgaagc atttatcagg gttattgtct catgagcgga 5940

tacatatttg aatgtattta gaaaaataaa caaatagggg ttccgcgcac atttccccga 6000

aaagtgccac ctatgagacg 6020

<210> 36
<211> 1886
<212> DNA
<213> Artificial sequence

<220>
<223> Ori-AmpR BsaI B

<400> 36

gaggtaccgg tctcatattg taatacggtt atccacagaa tcaggggata acgcaggaaa 60
gaacatgtga gcaaaaggcc agcaaaaggc caggaaccgt aaaaaggccg cgttgctggc 120
gtttttccat aggctccgcc cccctgacga gcatcacaaa aatcgacgct caagtcagag 180
gtggcgaaac ccgacaggac tataaagata ccaggcgttt ccccctggaa gctccctcgt 240
gcgctctcct gttccgaccc tgccgcttac cggatacctg tccgcctttc tcccttcggg 300
aagcgtggcg ctttctcata gctcacgctg taggtatctc agttcggtgt aggtcgttcg 360
ctccaagctg ggctgtgtgc acgaaccccc cgttcagccc gaccgctgcg ccttatccgg 420
taactatcgt cttgagtcca acccggtaag acacgactta tcgccactgg cagcagccac 480
tggtaacagg attagcagag cgaggtatgt aggcggtgct acagagttct tgaagtggtg 540
gcctaactac ggctacacta gaagaacagt atttggtatc tgcgctctgc tgaagccagt 600
taccttcgga aaaagagttg gtagctcttg atccggcaaa caaaccaccg ctggtagcgg 660
tttttttgtt tgcaagcagc agattacgcg cagaaaaaaa ggatctcaag aagatccttt 720
gatcttttct acggggtctg acgctcagtg gaacgaaaac tcacgttaag ggattttggt 780
catgagatta tcaaaaagga tcttcaccta gatcctttta aattaaaaat gaagttttaa 840
atcaatctaa agtatatatg agtaaacttg gtctgacagt taccaatgct taatcagtga 900
ggcacctatc tcagcgatct gtctatttcg ttcatccata gttgcctgac tccccgtcgt 960
gtagataact acgatacggg agggcttacc atctggcccc agtgctgcaa tgataccgcg 1020
tgacccacgc tcaccggctc cagatttatc agcaataaac cagccagccg gaagggccga 1080
gcgcagaagt ggtcctgcaa ctttatccgc ctccatccag tctattaatt gttgccggga 1140
agctagagta agtagttcgc cagttaatag tttgcgcaac gttgttgcca ttgctacagg 1200
catcgtggtg tcacgctcgt cgtttggtat ggcttcattc agctccggtt cccaacgatc 1260
aaggcgagtt acatgatccc ccatgttgtg caaaaaagcg gttagctcct tcggtcctcc 1320
gatcgttgtc agaagtaagt tggccgcagt gttatcactc atggttatgg cagcactgca 1380
taattctctt actgtcatgc catccgtaag atgcttttct gtgactggtg agtactcaac 1440

caagtcattc tgagaatagt gtatgcggcg accgagttgc tcttgcccgg cgtcaatacg 1500
ggataatacc gcgccacata gcagaacttt aaaagtgctc atcattggaa aacgttcttc 1560
ggggcgaaaa ctctcaagga tcttaccgct gttgagatcc agttcgatgt aacccactcg 1620
tgcacccaac tgatcttcag catcttttac tttcaccagc gtttctgggt gagcaaaaac 1680
aggaaggcaa aatgccgcaa aaaagggaat aagggcgaca cggaaatgtt gaatactcat 1740
actcttcctt tttcaatatt attgaagcat ttatcagggt tattgtctca tgagcggata 1800
catatttgaa tgtatttaga aaaataaaca aataggggtt ccgcgcacat ttccccgaaa 1860
agtgccaagg acgagaccgg tacctc 1886

<210> 37
<211> 673
<212> DNA
<213> Artificial sequence

<220>
<223> pCMV BsaI B

<400> 37

gaggtaccgg tctcaaggaa ccaattcagt cgactggatc ctagttatta atagtaatca 60
attacggggt cattagttca tagcccatat atggagttcc gcgttacata acttacggta 120
aatggcccgc ctggctgacc gcccaacgac ccccgcccat tgacgtcaat aatgacgtat 180
gttcccatag taacgccaat agggactttc cattgacgtc aatgggtgga gtatttacgg 240
taaactgccc acttggcagt acatcaagtg tatcatatgc caagtacgcc ccctattgac 300
gtcaatgacg gtaaatggcc cgcctggcat tatgcccagt acatgacctt atgggacttt 360
cctacttggc agtacatcta cgtattagtc atcgctatta ccatggtgat gcggttttgg 420
cagtacatca atgggcgtgg atagcggttt gactcacggg gatttccaag tctccacccc 480
attgacgtca atgggagttt gttttggcac caaaatcaac gggactttcc aaaatgtcgt 540
aacaactccg ccccattgac gcaaatgggc ggtaggcgtg tacggtggga ggtctatata 600
agcagagctg gtttagtgaa ccgtcagatc actagtcgac tagggataac agggcacccg 660
agaccggtac ctc 673

<210> 38
<211> 1121
<212> DNA
<213> Artificial sequence

<220>
<223> hFUT3 BsaI A

<400> 38

```
gaggtaccgg tctcacacca tggatcccct gggtgcagcc aagccacaat ggccatggcg        60
ccgctgtctg gccgcactgc tatttcagct gctggtggct gtgtgtttct tctcctacct       120
gcgtgtgtcc cgagacgatg ccactggatc ccctagggct cccagtgggt cctcccgaca       180
ggacaccact cccacccgcc ccaccctcct gatcctgcta tggacatggc ctttccacat       240
ccctgtggct ctgtcccgct gttcagagat ggtgcccggc acagccgact gccacatcac       300
tgccgaccgc aaggtgtacc cacaggcaga cacggtcatc gtgcaccact gggatatcat       360
gtccaaccct aagtcacgcc tcccaccttc cccgaggccg caggggcagc gctggatctg       420
gttcaacttg gagccacccc ctaactgcca gcacctggaa gccctggaca gatacttcaa       480
tctcaccatg tcctaccgca gcgactccga catcttcacg ccctacggct ggctggagcc       540
gtggtccggc cagcctgccc acccaccgct caacctctcg gccaagaccg agctggtggc       600
ctgggcggtg tccaactgga agccggactc agccagggtg cgctactacc agagcctgca       660
ggctcatctc aaggtggacg tgtacggacg ctcccacaag cccctgccca aggggaccat       720
gatggagacg ctgtcccggt acaagttcta cctggccttc gagaactcct tgcaccccga       780
ctacatcacc gagaagctgt ggaggaacgc cctggaggcc tgggccgtgc ccgtggtgct       840
gggccccagc agaagcaact acgagaggtt cctgccaccc gacgccttca tccacgtgga       900
cgacttccag agccccaagg acctggcccg gtacctgcag gagctggaca aggaccacgc       960
ccgctacctg agctactttc gctggcggga gacgctgcgg cctcgctcct tcagctgggc      1020
actggatttc tgcaaggcct gctggaaact gcagcaggaa tccaggtacc agacggtgcg      1080
cagcatagcg gcttggttca cctgatcgag accggtacct c                           1121
```

<210> 39
<211> 267
<212> DNA
<213> Artificial sequence

<220>
<223> BGHpA BsaI B

<400> 39

```
gaggtaccgg tctcatgatc gactgtgcct tctagttgcc agccatctgt tgtttgcccc        60
tcccccgtgc cttccttgac cctggaaggt gccactccca ctgtcctttc ctaataaaat       120
gaggaaattg catcgcattg tctgagtagg tgtcattcta ttctgggggg tggggtgggg       180
caggacagca agggggagga ttgggaggac aatagcaggc atgctgggga tgcggtgggc       240
tctatggctt cgcgagaccg gtacctc                                           267
```

<210> 40
<211> 444
<212> DNA
<213> Artificial sequence

<220>
<223> shB3Galt6 BsaI A

<400> 40

```
gaggtaccgg tctcattcga cagggtcgac aagcttttcc aaaaaaaaag catgaggtgc      60
agttgcgcct ttcctatctc ttgaatagga aaggcgcaac tgcacctcat gctggatccc     120
gcgtcctttc cacaagatat ataaacccaa gaaatcgaaa tactttcaag ttacggtaag     180
catatgatag tccattttaa aacataattt taaaactgca aactacccaa gaaattatta     240
ctttctacgt cacgtatttt gtactaatat ctttgtgttt acagtcaaat taattctaat     300
tatctctcta acagccttgt atcgtatatg caaatatgaa ggaatcatgg gaaataggcc     360
ctcttcctgc ccgaccttgg cgcgcgctcg gcgcgcggtc acgctccgtc acgtggtgcg     420
ttttgtattc gagaccggta cctc                                            444
```

<210> 41
<211> 4221
<212> DNA
<213> Artificial sequence

<220>
<223> V1

<400> 41

```
tattgtaata cggttatcca cagaatcagg ggataacgca ggaaagaaca tgtgagcaaa      60
aggccagcaa aaggccagga accgtaaaaa ggccgcgttg ctggcgtttt tccataggct     120
ccgcccccct gacgagcatc acaaaaatcg acgctcaagt cagaggtggc gaaacccgac     180
aggactataa agataccagg cgtttccccc tggaagctcc ctcgtgcgct ctcctgttcc     240
gaccctgccg cttaccggat acctgtccgc ctttctccct tcgggaagcg tggcgctttc     300
tcatagctca cgctgtaggt atctcagttc ggtgtaggtc gttcgctcca agctgggctg     360
tgtgcacgaa ccccccgttc agcccgaccg ctgcgcctta tccggtaact atcgtcttga     420
gtccaacccg gtaagacacg acttatcgcc actggcagca gccactggta acaggattag     480
cagagcgagg tatgtaggcg gtgctacaga gttcttgaag tggtggccta actacggcta     540
cactagaaga acagtatttg gtatctgcgc tctgctgaag ccagttacct tcggaaaaag     600
agttggtagc tcttgatccg gcaaacaaac caccgctggt agcggttttt ttgtttgcaa     660
gcagcagatt acgcgcagaa aaaaaggatc tcaagaagat cctttgatct tttctacggg     720
gtctgacgct cagtggaacg aaaactcacg ttaagggatt ttggtcatga gattatcaaa     780
aaggatcttc acctagatcc ttttaaatta aaaatgaagt tttaaatcaa tctaaagtat     840
atatgagtaa acttggtctg acagttacca atgcttaatc agtgaggcac ctatctcagc     900
gatctgtcta tttcgttcat ccatagttgc ctgactcccc gtcgtgtaga taactacgat     960
acgggagggc ttaccatctg gccccagtgc tgcaatgata ccgcgtgacc cacgctcacc    1020
ggctccagat ttatcagcaa taaaccagcc agccggaagg gccgagcgca gaagtggtcc    1080
```

tgcaacttta tccgcctcca tccagtctat taattgttgc cgggaagcta gagtaagtag 1140
ttcgccagtt aatagtttgc gcaacgttgt tgccattgct acaggcatcg tggtgtcacg 1200
ctcgtcgttt ggtatggctt cattcagctc cggttcccaa cgatcaaggc gagttacatg 1260
atcccccatg ttgtgcaaaa aagcggttag ctccttcggt cctccgatcg ttgtcagaag 1320
taagttggcc gcagtgttat cactcatggt tatggcagca ctgcataatt ctcttactgt 1380
catgccatcc gtaagatgct tttctgtgac tggtgagtac tcaaccaagt cattctgaga 1440
atagtgtatg cggcgaccga gttgctcttg cccggcgtca atacgggata ataccgcgcc 1500
acatagcaga actttaaaag tgctcatcat tggaaaacgt tcttcggggc gaaaactctc 1560
aaggatctta ccgctgttga gatccagttc gatgtaaccc actcgtgcac ccaactgatc 1620
ttcagcatct tttactttca ccagcgtttc tgggtgagca aaaacaggaa ggcaaaatgc 1680
cgcaaaaaag ggaataaggg cgacacggaa atgttgaata ctcatactct tcctttttca 1740
atattattga agcatttatc agggttattg tctcatgagc ggatacatat ttgaatgtat 1800
ttagaaaaat aaacaaatag gggttccgcg cacatttccc cgaaaagtgc caaggaacca 1860
attcagtcga ctggatccta gttattaata gtaatcaatt acggggtcat tagttcatag 1920
cccatatatg gagttccgcg ttacataact tacggtaaat ggcccgcctg gctgaccgcc 1980
caacgacccc cgcccattga cgtcaataat gacgtatgtt cccatagtaa cgccaatagg 2040
gactttccat tgacgtcaat gggtggagta tttacggtaa actgcccact tggcagtaca 2100
tcaagtgtat catatgccaa gtacgccccc tattgacgtc aatgacggta aatggcccgc 2160
ctggcattat gcccagtaca tgaccttatg ggactttcct acttggcagt acatctacgt 2220
attagtcatc gctattacca tggtgatgcg gttttggcag tacatcaatg ggcgtggata 2280
gcggtttgac tcacggggat ttccaagtct ccaccccatt gacgtcaatg ggagtttgtt 2340
ttggcaccaa aatcaacggg actttccaaa atgtcgtaac aactccgccc cattgacgca 2400
aatgggcggt aggcgtgtac ggtgggaggt ctatataagc agagctggtt tagtgaaccg 2460
tcagatcact agtcgactag ggataacagg gcaccatgga tcccctgggt gcagccaagc 2520
cacaatggcc atggcgccgc tgtctggccg cactgctatt tcagctgctg gtggctgtgt 2580
gtttcttctc ctacctgcgt gtgtcccgag acgatgccac tggatcccct agggctccca 2640
gtgggtcctc ccgacaggac accactccca cccgccccac cctcctgatc ctgctatgga 2700
catggccttt ccacatccct gtggctctgt cccgctgttc agagatggtg cccggcacag 2760
ccgactgcca catcactgcc gaccgcaagg tgtacccaca ggcagacacg gtcatcgtgc 2820
accactggga tatcatgtcc aaccctaagt cacgcctccc accttccccg aggccgcagg 2880
ggcagcgctg gatctggttc aacttggagc caccccctaa ctgccagcac ctggaagccc 2940

```
tggacagata cttcaatctc accatgtcct accgcagcga ctccgacatc ttcacgccct     3000
acggctggct ggagccgtgg tccggccagc ctgcccaccc accgctcaac ctctcggcca     3060
agaccgagct ggtggcctgg gcggtgtcca actggaagcc ggactcagcc agggtgcgct     3120
actaccagag cctgcaggct catctcaagg tggacgtgta cggacgctcc cacaagcccc     3180
tgcccaaggg gaccatgatg gagacgctgt cccggtacaa gttctacctg gccttcgaga     3240
actccttgca ccccgactac atcaccgaga agctgtggag gaacgccctg gaggcctggg     3300
ccgtgcccgt ggtgctgggc cccagcagaa gcaactacga gaggttcctg ccacccgacg     3360
ccttcatcca cgtggacgac ttccagagcc ccaaggacct ggcccggtac ctgcaggagc     3420
tggacaagga ccacgcccgc tacctgagct actttcgctg gcgggagacg ctgcggcctc     3480
gctccttcag ctgggcactg gatttctgca aggcctgctg gaaactgcag caggaatcca     3540
ggtaccagac ggtgcgcagc atagcggctt ggttcacctg atcgactgtg ccttctagtt     3600
gccagccatc tgttgtttgc ccctcccccg tgccttcctt gaccctggaa ggtgccactc     3660
ccactgtcct ttcctaataa aatgaggaaa ttgcatcgca ttgtctgagt aggtgtcatt     3720
ctattctggg gggtggggtg gggcaggaca gcaaggggga ggattgggag gacaatagca     3780
ggcatgctgg ggatgcggtg ggctctatgg cttcgacagg gtcgacaagc ttttccaaaa     3840
aaaaagcatg aggtgcagtt gcgcctttcc tatctcttga ataggaaagg cgcaactgca     3900
cctcatgctg gatcccgcgt cctttccaca agatatataa acccaagaaa tcgaaatact     3960
ttcaagttac ggtaagcata tgatagtcca ttttaaaaca taattttaaa actgcaaact     4020
acccaagaaa ttattacttt ctacgtcacg tattttgtac taatatcttt gtgtttacag     4080
tcaaattaat tctaattatc tctctaacag ccttgtatcg tatatgcaaa tatgaaggaa     4140
tcatgggaaa taggccctct tcctgcccga ccttggcgcg cgctcggcgc gcggtcacgc     4200
tccgtcacgt ggtgcgtttt g                                               4221
```

<210> 42
<211> 20
<212> DNA
<213> Artificial sequence

<220>
<223> BETA3Galt6ms2-s

<400> 42
accactctgt tgtacctggc 20

<210> 43
<211> 17
<212> DNA
<213> Artificial sequence

<220>
<223> BETA3Galt6ms2-as

<400> 43
cacacgtcct cgggtcc 17

<210> 44
<211> 24
<212> DNA
<213> Artificial sequence

<220>
<223> FUT35

<400> 44
cactagtcga ctagggataa cagg 24

<210> 45
<211> 22
<212> DNA
<213> Artificial sequence

<220>
<223> FUT33

<400> 45
atgtccatag caggatcagg ag 22

<210> 46
<211> 444
<212> DNA
<213> Artificial sequence

<220>
<223> shB3Galt6 BsaI B

<400> 46

gaggtaccgg tctcattcga cagggtcgac aagcttttcc aaaaaaaaag catgaggtgc 60
agttgcgcct ttcctatctc ttgaatagga aaggcgcaac tgcacctcat gctggatccc 120
gcgtcctttc cacaagatat ataaacccaa gaaatcgaaa tactttcaag ttacggtaag 180
catatgatag tccattttaa aacataattt taaaactgca aactacccaa gaaattatta 240
ctttctacgt cacgtatttt gtactaatat ctttgtgttt acagtcaaat taattctaat 300
tatctctcta acagccttgt atcgtatatg caaatatgaa ggaatcatgg gaaataggcc 360
ctcttcctgc ccgaccttgg cgcgcgctcg gcgcgcggtc acgctccgtc acgtggtgcg 420
ttttgccagc gagaccggta cctc 444

<210> 47
<211> 1650
<212> DNA
<213> Artificial sequence

<220>
<223> HygroR BsaI B

<400> 47

gaggtaccgg tctcaccagc aggcagaagt atgcaaagca tgcatctcaa ttagtcagca 60
accaggtgtg gaaagtcccc aggctcccca gcaggcagaa gtatgcaaag catgcatctc 120
aattagtcag caaccatagt cccgccccta actccgccca tcccgcccct aactccgccc 180
agttccgccc attctccgcc ccatggctga ctaatttttt ttatttatgc agaggccgag 240
gccgcctctg cctctgagct attccagaag tagtgaggag gcttttttgg aggcctaggc 300
ttttgcaaaa agctcccggg agcttgtata tccattttcg gatctgatca gcacgtgatg 360
aaaaagcctg aactcaccgc gacgtctgtc gagaagtttc tgatcgaaaa gttcgacagc 420
gtgtccgacc tgatgcagct ctcggagggc gaagaatctc gtgctttcag cttcgatgta 480
ggagggcgtg gatatgtcct gcgggtaaat agctgcgccg atggtttcta caaagatcgt 540
tatgtttatc ggcactttgc atcggccgcg ctcccgattc cggaagtgct tgacattggg 600
gaattcagcg agagcctgac ctattgcatc tcccgccgtg cacagggtgt cacgttgcaa 660
gacttgcctg aaaccgaact gcccgctgtt ctgcagccgg tcgcggaggc catggatgcg 720
atcgctgcgg ccgatcttag ccagacgagc gggttcggcc cattcggacc gcaaggaatc 780
ggtcaataca ctacatggcg tgatttcata tgcgcgattg ctgatcccca tgtgtatcac 840
tggcaaactg tgatggacga caccgtcagt gcgtccgtcg cgcaggctct cgatgagctg 900
atgctttggg ccgaggactg ccccgaagtc cggcacctcg tgcacgcgga tttcggctcc 960
aacaatgtcc tgacggacaa tggccgcata acagcggtca ttgactggag cgaggcgatg 1020
ttcggggatt cccaatacga ggtcgccaac atcttcttct ggaggccgtg gttggcttgt 1080
atggagcagc agacgcgcta cttcgagcgg aggcatccgg agcttgcagg atcgccgcgg 1140
ctccgggcgt atatgctccg cattggtctt gaccaactct atcagagctt ggttgacggc 1200
aatttcgatg atgcagcttg ggcgcagggt cgatgcgacg caatcgtccg atccggagcc 1260
gggactgtcg ggcgtacaca aatcgcccgc agaagcgcgg ccgtctggac cgatggctgt 1320
gtagaagtac tcgccgatag tggaaaccga cgccccagca ctcgtccgag ggcaaaggaa 1380
tagcacgtgc tacgagattt cgattccacc gccgccttct atgaaaggtt gggcttcgga 1440
atcgttttcc gggacgccgg ctggatgatc ctccagcgcg gggatctcat gctggagttc 1500
ttcgcccacc ccaacttgtt tattgcagct tataatggtt acaaataaag caatagcatc 1560
acaaatttca caaataaagc atttttttca ctgcattcta gttgtggttt gtccaaactc 1620
atcaatgtat ctattcgaga ccggtacctc 1650

<210> 48
<211> 5837
<212> DNA
<213> Artificial sequence

<220>
<223> V1.1

<400> 48

tattgtaata cggttatcca cagaatcagg ggataacgca ggaaagaaca tgtgagcaaa 60
aggccagcaa aaggccagga accgtaaaaa ggccgcgttg ctggcgtttt tccataggct 120
ccgcccccct gacgagcatc acaaaaatcg acgctcaagt cagaggtggc gaaacccgac 180
aggactataa agataccagg cgtttccccc tggaagctcc ctcgtgcgct ctcctgttcc 240
gaccctgccg cttaccggat acctgtccgc ctttctccct tcgggaagcg tggcgctttc 300
tcatagctca cgctgtaggt atctcagttc ggtgtaggtc gttcgctcca agctgggctg 360
tgtgcacgaa ccccccgttc agcccgaccg ctgcgcctta tccggtaact atcgtcttga 420
gtccaacccg gtaagacacg acttatcgcc actggcagca gccactggta acaggattag 480
cagagcgagg tatgtaggcg gtgctacaga gttcttgaag tggtggccta actacggcta 540
cactagaaga acagtatttg gtatctgcgc tctgctgaag ccagttacct tcggaaaaag 600
agttggtagc tcttgatccg gcaaacaaac caccgctggt agcggttttt ttgtttgcaa 660
gcagcagatt acgcgcagaa aaaaaggatc tcaagaagat cctttgatct tttctacggg 720
gtctgacgct cagtggaacg aaaactcacg ttaagggatt ttggtcatga gattatcaaa 780
aaggatcttc acctagatcc ttttaaatta aaaatgaagt tttaaatcaa tctaaagtat 840
atatgagtaa acttggtctg acagttacca atgcttaatc agtgaggcac ctatctcagc 900
gatctgtcta tttcgttcat ccatagttgc ctgactcccc gtcgtgtaga taactacgat 960
acgggagggc ttaccatctg gccccagtgc tgcaatgata ccgcgtgacc cacgctcacc 1020
ggctccagat ttatcagcaa taaaccagcc agccggaagg gccgagcgca gaagtggtcc 1080
tgcaacttta tccgcctcca tccagtctat taattgttgc cgggaagcta gagtaagtag 1140
ttcgccagtt aatagtttgc gcaacgttgt tgccattgct acaggcatcg tggtgtcacg 1200
ctcgtcgttt ggtatggctt cattcagctc cggttcccaa cgatcaaggc gagttacatg 1260
atcccccatg ttgtgcaaaa aagcggttag ctccttcggt cctccgatcg ttgtcagaag 1320
taagttggcc gcagtgttat cactcatggt tatggcagca ctgcataatt ctcttactgt 1380
catgccatcc gtaagatgct tttctgtgac tggtgagtac tcaaccaagt cattctgaga 1440
atagtgtatg cggcgaccga gttgctcttg cccggcgtca atacgggata ataccgcgcc 1500
acatagcaga actttaaaag tgctcatcat tggaaaacgt tcttcggggc gaaaactctc 1560
aaggatctta ccgctgttga gatccagttc gatgtaaccc actcgtgcac ccaactgatc 1620
ttcagcatct tttactttca ccagcgtttc tgggtgagca aaaacaggaa ggcaaaatgc 1680
cgcaaaaaag ggaataaggg cgacacggaa atgttgaata ctcatactct tcctttttca 1740

atattattga agcatttatc agggttattg tctcatgagc ggatacatat ttgaatgtat 1800
ttagaaaaat aaacaaatag gggttccgcg cacatttccc cgaaaagtgc caaggaacca 1860
attcagtcga ctggatccta gttattaata gtaatcaatt acggggtcat tagttcatag 1920
cccatatatg gagttccgcg ttacataact tacggtaaat ggcccgcctg gctgaccgcc 1980
caacgacccc cgcccattga cgtcaataat gacgtatgtt cccatagtaa cgccaatagg 2040
gactttccat tgacgtcaat gggtggagta tttacggtaa actgcccact tggcagtaca 2100
tcaagtgtat catatgccaa gtacgccccc tattgacgtc aatgacggta aatggcccgc 2160
ctggcattat gcccagtaca tgaccttatg ggactttcct acttggcagt acatctacgt 2220
attagtcatc gctattacca tggtgatgcg gttttggcag tacatcaatg ggcgtggata 2280
gcggtttgac tcacggggat ttccaagtct ccaccccatt gacgtcaatg ggagtttgtt 2340
ttggcaccaa aatcaacggg actttccaaa atgtcgtaac aactccgccc cattgacgca 2400
aatgggcggt aggcgtgtac ggtgggaggt ctatataagc agagctggtt tagtgaaccg 2460
tcagatcact agtcgactag ggataacagg gcaccatgga tcccctgggt gcagccaagc 2520
cacaatggcc atggcgccgc tgtctggccg cactgctatt tcagctgctg gtggctgtgt 2580
gtttcttctc ctacctgcgt gtgtcccgag acgatgccac tggatcccct agggctccca 2640
gtgggtcctc ccgacaggac accactccca cccgccccac cctcctgatc ctgctatgga 2700
catggccttt ccacatccct gtggctctgt cccgctgttc agagatggtg cccggcacag 2760
ccgactgcca catcactgcc gaccgcaagg tgtacccaca ggcagacacg gtcatcgtgc 2820
accactggga tatcatgtcc aaccctaagt cacgcctccc accttccccg aggccgcagg 2880
ggcagcgctg gatctggttc aacttggagc caccccctaa ctgccagcac ctggaagccc 2940
tggacagata cttcaatctc accatgtcct accgcagcga ctccgacatc ttcacgccct 3000
acggctggct ggagccgtgg tccggccagc ctgcccaccc accgctcaac ctctcggcca 3060
agaccgagct ggtggcctgg gcggtgtcca actggaagcc ggactcagcc agggtgcgct 3120
actaccagag cctgcaggct catctcaagg tggacgtgta cggacgctcc cacaagcccc 3180
tgcccaaggg gaccatgatg gagacgctgt cccggtacaa gttctacctg gccttcgaga 3240
actccttgca ccccgactac atcaccgaga agctgtggag gaacgccctg gaggcctggg 3300
ccgtgcccgt ggtgctgggc cccagcagaa gcaactacga gaggttcctg ccacccgacg 3360
ccttcatcca cgtggacgac ttccagagcc cccaggacct ggcccggtac ctgcaggagc 3420
tggacaagga ccacgcccgc tacctgagct actttcgctg gcgggagacg ctgcggcctc 3480
gctccttcag ctgggcactg gatttctgca aggcctgctg gaaactgcag caggaatcca 3540
ggtaccagac ggtgcgcagc atagcggctt ggttcacctg atcgactgtg ccttctagtt 3600
gccagccatc tgttgtttgc ccctcccccg tgccttcctt gaccctggaa ggtgccactc 3660

ccactgtcct ttcctaataa aatgaggaaa ttgcatcgca ttgtctgagt aggtgtcatt 3720
ctattctggg gggtggggtg gggcaggaca gcaaggggga ggattgggag gacaatagca 3780
ggcatgctgg ggatgcggtg ggctctatgg cttcgacagg gtcgacaagc ttttccaaaa 3840
aaaaagcatg aggtgcagtt gcgcctttcc tatctcttga ataggaaagg cgcaactgca 3900
cctcatgctg gatcccgcgt cctttccaca agatatataa acccaagaaa tcgaaatact 3960
ttcaagttac ggtaagcata tgatagtcca ttttaaaaca taattttaaa actgcaaact 4020
acccaagaaa ttattacttt ctacgtcacg tattttgtac taatatcttt gtgtttacag 4080
tcaaattaat tctaattatc tctctaacag ccttgtatcg tatatgcaaa tatgaaggaa 4140
tcatgggaaa taggccctct tcctgcccga ccttggcgcg cgctcggcgc gcggtcacgc 4200
tccgtcacgt ggtgcgtttt gccagcaggc agaagtatgc aaagcatgca tctcaattag 4260
tcagcaacca ggtgtggaaa gtccccaggc tccccagcag gcagaagtat gcaaagcatg 4320
catctcaatt agtcagcaac catagtcccg cccctaactc cgcccatccc gcccctaact 4380
ccgcccagtt ccgcccattc tccgccccat ggctgactaa ttttttttat ttatgcagag 4440
gccgaggccg cctctgcctc tgagctattc cagaagtagt gaggaggctt ttttggaggc 4500
ctaggctttt gcaaaaagct cccgggagct tgtatatcca ttttcggatc tgatcagcac 4560
gtgatgaaaa agcctgaact caccgcgacg tctgtcgaga agtttctgat cgaaaagttc 4620
gacagcgtgt ccgacctgat gcagctctcg gagggcgaag aatctcgtgc tttcagcttc 4680
gatgtaggag ggcgtggata tgtcctgcgg gtaaatagct gcgccgatgg tttctacaaa 4740
gatcgttatg tttatcggca ctttgcatcg gccgcgctcc cgattccgga agtgcttgac 4800
attggggaat tcagcgagag cctgacctat tgcatctccc gccgtgcaca gggtgtcacg 4860
ttgcaagact tgcctgaaac cgaactgccc gctgttctgc agccggtcgc ggaggccatg 4920
gatgcgatcg ctgcggccga tcttagccag acgagcgggt tcggcccatt cggaccgcaa 4980
ggaatcggtc aatacactac atggcgtgat ttcatatgcg cgattgctga tccccatgtg 5040
tatcactggc aaactgtgat ggacgacacc gtcagtgcgt ccgtcgcgca ggctctcgat 5100
gagctgatgc tttgggccga ggactgcccc gaagtccggc acctcgtgca cgcggatttc 5160
ggctccaaca atgtcctgac ggacaatggc cgcataacag cggtcattga ctggagcgag 5220
gcgatgttcg gggattccca atacgaggtc gccaacatct tcttctggag gccgtggttg 5280
gcttgtatgg agcagcagac gcgctacttc gagcggaggc atccggagct tgcaggatcg 5340
ccgcggctcc gggcgtatat gctccgcatt ggtcttgacc aactctatca gagcttggtt 5400
gacggcaatt tcgatgatgc agcttgggcg cagggtcgat gcgacgcaat cgtccgatcc 5460
ggagccggga ctgtcgggcg tacacaaatc gcccgcagaa gcgcggccgt ctggaccgat 5520

ggctgtgtag aagtactcgc cgatagtgga aaccgacgcc ccagcactcg tccgagggca 5580

aaggaatagc acgtgctacg agatttcgat tccaccgccg ccttctatga aaggttgggc 5640

ttcggaatcg ttttccggga cgccggctgg atgatcctcc agcgcgggga tctcatgctg 5700

gagttcttcg cccaccccaa cttgtttatt gcagcttata atggttacaa ataaagcaat 5760

agcatcacaa atttcacaaa taaagcattt ttttcactgc attctagttg tggtttgtcc 5820

aaactcatca atgtatc 5837

<210> 49
<211> 1122
<212> DNA
<213> Artificial sequence

<220>
<223> rosa26-5' BsaI A

<400> 49

gaggtaccgg tctcaaggac cccgcggcag gccctccgag cgtggtggag ccgttctgtg 60

agacagccgg gtacgagtcg tgacgctgga aggggcaagc gggtggtggg caggaatgcg 120

gtccgccctg cagcaaccgg agggggaggg agaagggagc ggaaaagtct ccaccggacg 180

cggccatggc tcgggggggg gggggcagcg gaggagcgct tccggccgac gtctcgtcgc 240

tgattggctt cttttcctcc cgccgtgtgt gaaaacacaa atggcgtgtt ttggttggcg 300

taaggcgcct gtcagttaac ggcagccgga gtgcgcagcc gccggcagcc tcgctctgcc 360

cactgggtgg ggcgggaggt aggtggggtg aggcgagctg gacgtgcggg cgcggtcggc 420

ctctggcggg gcgggggagg ggagggaggg tcagcgaaag tagctcgcgc gcgagcggcc 480

gcccaccctc cccttcctct gggggagtcg ttttacccgc cgccggccgg gcctcgtcgt 540

ctgattggct ctcggggccc agaaaactgg cccttgccat tggctcgtgt tcgtgcaagt 600

tgagtccatc cgccggccag cgggggcggc gaggaggcgc tcccaggttc cggccctccc 660

ctcggccccg cgccgcagag tctggccgcg cgcccctgcg caacgtggca ggaagcgcgc 720

gctgggggcg gggacgggca gtagggctga gcggctgcgg ggcgggtgca agcacgtttc 780

cgacttgagt tgcctcaaga ggggcgtgct gagccagacc tccatcgcgc actccgggga 840

gtggagggaa ggagcgaggg ctcagttggg ctgttttgga ggcaggaagc acttgctctc 900

ccaaagtcgc tctgagttgt tatcagtaag ggagctgcag tggagtaggc ggggagaagg 960

ccgcaccctt ctccggaggg gggaggggag tgttgcaata cctttctggg agttctctgc 1020

tgcctcctgg cttctgagga ccgccctggg cctgggagaa tcccttgccc cctcttcccc 1080

tcgtgatctg caactccagt cttacaacga gaccggtacc tc 1122

<210> 50
<211> 673
<212> DNA
<213> Artificial sequence

<220>
<223> pCMV BsaI C

<400> 50

```
gaggtaccgg tctcaacaaa ccaattcagt cgactggatc ctagttatta atagtaatca      60
attacggggt cattagttca tagcccatat atggagttcc gcgttacata acttacggta     120
aatggcccgc ctggctgacc gcccaacgac ccccgcccat tgacgtcaat aatgacgtat     180
gttcccatag taacgccaat agggactttc cattgacgtc aatgggtgga gtatttacgg     240
taaactgccc acttggcagt acatcaagtg tatcatatgc caagtacgcc ccctattgac     300
gtcaatgacg gtaaatggcc cgcctggcat tatgcccagt acatgacctt atgggacttt     360
cctacttggc agtacatcta cgtattagtc atcgctatta ccatggtgat gcggttttgg     420
cagtacatca atgggcgtgg atagcggttt gactcacggg gatttccaag tctccacccc     480
attgacgtca atgggagttt gttttggcac caaaatcaac gggactttcc aaaatgtcgt     540
aacaactccg ccccattgac gcaaatgggc ggtaggcgtg tacggtggga ggtctatata     600
agcagagctg gtttagtgaa ccgtcagatc actagtcgac tagggataac agggcacccg     660
agaccggtac ctc                                                        673
```

<210> 51
<211> 1650
<212> DNA
<213> Artificial sequence

<220>
<223> HygroR BsaI C

<400> 51

```
gaggtaccgg tctcaccagc aggcagaagt atgcaaagca tgcatctcaa ttagtcagca        60
accaggtgtg gaaagtcccc aggctcccca gcaggcagaa gtatgcaaag catgcatctc       120
aattagtcag caaccatagt cccgccccta actccgccca tcccgcccct aactccgccc       180
agttccgccc attctccgcc ccatggctga ctaatttttt ttatttatgc agaggccgag       240
gccgcctctg cctctgagct attccagaag tagtgaggag gcttttttgg aggcctaggc       300
ttttgcaaaa agctcccggg agcttgtata tccattttcg gatctgatca gcacgtgatg       360
aaaaagcctg aactcaccgc gacgtctgtc gagaagtttc tgatcgaaaa gttcgacagc       420
gtgtccgacc tgatgcagct ctcggagggc gaagaatctc gtgctttcag cttcgatgta       480
ggagggcgtg gatatgtcct gcgggtaaat agctgcgccg atggtttcta caaagatcgt       540
tatgtttatc ggcactttgc atcggccgcg ctcccgattc cggaagtgct tgacattggg       600
gaattcagcg agagcctgac ctattgcatc tcccgccgtg cacagggtgt cacgttgcaa       660

gacttgcctg aaaccgaact gcccgctgtt ctgcagccgg tcgcggaggc catggatgcg       720
atcgctgcgg ccgatcttag ccagacgagc gggttcggcc cattcggacc gcaaggaatc       780
ggtcaataca ctacatggcg tgatttcata tgcgcgattg ctgatcccca tgtgtatcac       840
tggcaaactg tgatggacga caccgtcagt gcgtccgtcg cgcaggctct cgatgagctg       900
atgctttggg ccgaggactg ccccgaagtc cggcacctcg tgcacgcgga tttcggctcc       960
aacaatgtcc tgacggacaa tggccgcata acagcggtca ttgactggag cgaggcgatg      1020
ttcggggatt cccaatacga ggtcgccaac atcttcttct ggaggccgtg gttggcttgt      1080
atggagcagc agacgcgcta cttcgagcgg aggcatccgg agcttgcagg atcgccgcgg      1140
ctccgggcgt atatgctccg cattggtctt gaccaactct atcagagctt ggttgacggc      1200
aatttcgatg atgcagcttg ggcgcagggt cgatgcgacg caatcgtccg atccggagcc      1260
gggactgtcg ggcgtacaca aatcgcccgc agaagcgcgg ccgtctggac cgatggctgt      1320
gtagaagtac tcgccgatag tggaaaccga cgccccagca ctcgtccgag ggcaaaggaa      1380
tagcacgtgc tacgagattt cgattccacc gccgccttct atgaaaggtt gggcttcgga      1440
atcgttttcc gggacgccgg ctggatgatc ctccagcgcg gggatctcat gctggagttc      1500
ttcgcccacc ccaacttgtt tattgcagct tataatggtt acaaataaag caatagcatc      1560
acaaatttca caaataaagc atttttttca ctgcattcta gttgtggttt gtccaaactc      1620
atcaatgtat cgtagcgaga ccggtacctc                                       1650
```

<210> 52
<211> 4309
<212> DNA
<213> Artificial sequence

<220>
<223> rosa26-3' BsaI A

<400> 52

```
gaggtaccgg tctcagtaga gatgggcggg agtcttctgg gcaggcttaa aggctaacct        60
ggtgtgtggg cgttgtcctg caggggaatt gaacaggtgt aaaattggag ggacaagact       120
tcccacagat tttcggtttt gtcgggaagt tttttaatag gggcaaatag gaaaatggag       180
gataggagtc atctggggtt tatgcagcaa aactacaggt atattgcttg tatccgcctc       240
ggagatttcc atgaggagat aaagacatgt cacccgagtt tatactctcc tgcttagatc       300
ctactacagt atgaaataca gtgtcgcgag gtagactatg taagcagatt taatcatttt       360
aaagagccca gtacttcata tccatttctc ccgctccttc tgcagcctta tcaaaaggta       420
tttagaacac tcattttagc cccattttca tttattatac tggcttatcc aacccctaga       480
cagagcattg gcattttccc tttcctgatc ttagaagtct gatgactcat gaaaccagac       540
agattagtta catacaccac aaatcgaggc tgtagctggg gcctcaacac tgcagttctt       600
```

ttataactcc ttagtacact ttttgttgat cctttgcctt gatccttaat tttcagtgtc 660
tatcacctct cccgtcaggt ggtgttccac atttgggcct attctcagtc cagggagttt 720
tacaacaata gatgtattga gaatccaacc taaagcttaa ctttccactc ccatgaatgc 780
ctctctcctt tttctccatt ataactgagc tataaccatt aatggtttca ggtggatgtc 840
tcctccccca atatacctga tgtatctaca tattgccagg ctgatatttt aagacataaa 900
aggtatattt cattattgag ccacatggta ttgattactg ctactaaaat tttgtcattg 960
tacacatctg taaaaggtgg ttccttttgg aatgcaaagt tcaggtgttt gttgtctttc 1020
ctgacctaag gtcttgtgag cttgtatttt ttctatttaa gcagtgcttt ctcttggact 1080
ggcttgactc atggcattct acacgttatt gctggtctaa atgtgatttt gccaagcttc 1140
ttcaggacct ataattttgc ttgacttgta gccaaacaca agtaaaatga ttaagcaaca 1200
aatgtatttg tgaagcttgg tttttaggtt gttgtgttgt gtgtgcttgt gctctataat 1260
aatactatcc aggggctgga gaggtggctc ggagttcaag agcacagact gctcttccag 1320
aagtcctgag ttcaattccc agcaaccaca tggtggctca caaccatctg taatgggatc 1380
tgatgccctc ttctggtgtg tctgaagacc acaagtgtat tcacattaaa taaataatcc 1440
tccttcttct tctttttttt tttttaaaga gaatactgtc tccagtagaa ttactgaagt 1500
aatgaaatac tttgtgtttg ttccaatatg gaagccaata atcaaatact cttaagcact 1560
ggaaatgtac caaggaacta ttttatttaa gtgaactgtg gacagaggag ccataactgc 1620
agacttgtgg gatacagaag accaatgcag acttaatgtc ttttctctta cactaagcaa 1680
taaagaaata aaaattgaac ttctagtatc ctatttgtta aactgctagc tttactaact 1740
tttgtgcttc atctatacaa agctgaaagc taagtctgca gccattacta aacatgaaag 1800
caagtaatga taattttgga tttcaaaaat gtagggccag agtttagcca gccagtggtg 1860
gtgcttgcct ttatgcctta atcccagcac tctggaggca gagacaggca gatctctgag 1920
tttgagccca gcctggtcta cacatcaagt tctatctagg atagccagga atacacacag 1980
aaaccctgtt ggggaggggg gctctgagat ttcataaaat tataattgaa gcattcccta 2040
atgagccact atggatgtgg ctaaatccgt ctacctttct gatgagattt gggtattatt 2100
ttttctgtct ctgctgttgg ttgggtcttt tgacactgtg ggctttctta aagcctcctt 2160
ccctgccatg tggactcttg tttgctacta acttcccatg gcttaaatgg catggctttt 2220
tgccttctaa gggcagctgc tgagatttgc agcctgattt ccagggtggg gttgggaaat 2280
ctttcaaaca ctaaaattgt cctttaattt ttttttaaaa aatgggttat ataataaacc 2340
tcataaaata gttatgagga gtgaggtgga ctaatattaa tgagtccctc ccctataaaa 2400
gagctattaa ggctttttgt cttatactaa cttttttttt aaatgtggta tctttagaac 2460

caagggtctt agagttttag tatacagaaa ctgttgcatc gcttaatcag attttctagt 2520
ttcaaatcca gagaatccaa attcttcaca gccaaagtca aattaagaat ttctgacttt 2580
aatgttattt gctactgtga atataaaatg atagcttttc ctgaggcagg gtatcactat 2640
gtatctctgc ctgatctgca acaagatatg tagactaaag ttctgcctgc ttttgtctcc 2700
tgaatactaa ggttaaaatg tagtaatact tttggaactt gcaggtcaga ttcttttata 2760
ggggacacac taagggagct tgggtgatag ttggtaaatg tgtttaagtg atgaaaactt 2820
gaattattat caccgcaacc tactttttaa aaaaaaaagc caggcctgtt agagcatgct 2880
aagggatccc taggacttgc tgagcacaca agagtagtac ttggcaggct cctggtgaga 2940
gcatatttca aaaaacaagg cagacaacca agaaactaca gtaaggttac ctgtctttaa 3000
ccatctgcat atacacaggg atattaaaat attccaaata atatttcatt caagttttcc 3060
cccatcaaat tgggacatgg atttctccgg tgaataggca gagttggaaa ctaaacaaat 3120
gttggttttg tgatttgtga aattgttttc aagtgatagt taaagcccat gagatacaga 3180
acaaagctgc tatttcgagg tcacttggtt atactcagaa gcacttcttt gggtttccct 3240
gcactatcct gatcatgtgc taggcctacc ttaggctgat tgttgttcaa ataacttaag 3300
tttcctgtca ggtgatgtca tatgatttca tatatcaagg caaaacatgt tatatatgtt 3360
aaacatttgg acttaatgtg aaagttaggt ctttgtgggt tttgatttta atttcaaaac 3420
ctgagctaaa taagtcattt tacatgtctt acatttggtg aattgtatat tgtggtttgc 3480
aggcaagact ctctgaccta gtaaccctcc tatagagcac tttgctgggt cacaagtcta 3540
ggagtcaagc atttcacctt gaagttgaga cgttttgtta gtgtatacta gttatatgtt 3600
ggaggacatg tttatccaga agatattcag gactattttt gactgggcta aggaattgat 3660
tctgattagc actgttagtg agcattgagt ggcctttagg cttgaattgg agtcacttgt 3720
atatctcaaa taatgctggc ctttttttaaa aagcccttgt tctttatcac cctgttttct 3780
acataatttt tgttcaaaga aatacttgtt tggatctcct tttgacaaca atagcatgtt 3840
ttcaagccat atttttttttc cttttttttt tttttttgg tttttcgaga cagggtttct 3900
ctgtatagcc ctggctgtcc tggaactcac tttgtagacc aggctggcct cgaactcaga 3960
aatccgcctg cctctgcctc ctgagtgccg ggattaaagg cgtgcaccac cacgcctggc 4020
taagttggat attttgtata taactataac caatactaac tccactgggt ggatttttaa 4080
ttcagtcagt agtcttaagt ggtctttatt ggcccttatt aaaatctact gttcactcta 4140
acagaggctg ttggactagt gggactaagc aacttcctac ggatatacta gcagataagg 4200
gtcagggata gaaactagtc tagcgttttg tatacctacc agcttatact accttgttct 4260
gatagaaata tttaggacat ctagcttatc tattcgagac cggtacctc 4309

<210> 53
<211> 11200
<212> DNA
<213> Artificial sequence

<220>
<223> V1.2

<400> 53

gtaatacggt tatccacaga atcaggggat aacgcaggaa agaacatgtg agcaaaaggc 60
cagcaaaagg ccaggaaccg taaaaaggcc gcgttgctgg cgtttttcca taggctccgc 120
cccccctgacg agcatcacaa aaatcgacgc tcaagtcaga ggtggcgaaa cccgacagga 180
ctataaagat accaggcgtt tccccctgga agctccctcg tgcgctctcc tgttccgacc 240
ctgccgctta ccggatacct gtccgccttt ctcccttcgg gaagcgtggc gctttctcat 300
agctcacgct gtaggtatct cagttcggtg taggtcgttc gctccaagct gggctgtgtg 360
cacgaacccc ccgttcagcc cgaccgctgc gccttatccg gtaactatcg tcttgagtcc 420
aacccggtaa gacacgactt atcgccactg gcagcagcca ctggtaacag gattagcaga 480
gcgaggtatg taggcggtgc tacagagttc ttgaagtggt ggcctaacta cggctacact 540
agaagaacag tatttggtat ctgcgctctg ctgaagccag ttaccttcgg aaaaagagtt 600
ggtagctctt gatccggcaa acaaaccacc gctggtagcg gtttttttgt ttgcaagcag 660
cagattacgc gcagaaaaaa aggatctcaa gaagatcctt tgatcttttc tacggggtct 720
gacgctcagt ggaacgaaaa ctcacgttaa gggattttgg tcatgagatt atcaaaaagg 780
atcttcacct agatcctttt aaattaaaaa tgaagtttta aatcaatcta aagtatatat 840
gagtaaactt ggtctgacag ttaccaatgc ttaatcagtg aggcacctat ctcagcgatc 900
tgtctatttc gttcatccat agttgcctga ctccccgtcg tgtagataac tacgatacgg 960
gagggcttac catctggccc cagtgctgca atgataccgc gtgacccacg ctcaccggct 1020
ccagatttat cagcaataaa ccagccagcc ggaagggccg agcgcagaag tggtcctgca 1080
actttatccg cctccatcca gtctattaat tgttgccggg aagctagagt aagtagttcg 1140
ccagttaata gtttgcgcaa cgttgttgcc attgctacag gcatcgtggt gtcacgctcg 1200
tcgtttggta tggcttcatt cagctccggt tcccaacgat caaggcgagt tacatgatcc 1260
cccatgttgt gcaaaaaagc ggttagctcc ttcggtcctc cgatcgttgt cagaagtaag 1320
ttggccgcag tgttatcact catggttatg gcagcactgc ataattctct tactgtcatg 1380
ccatccgtaa gatgcttttc tgtgactggt gagtactcaa ccaagtcatt ctgagaatag 1440
tgtatgcggc gaccgagttg ctcttgcccg gcgtcaatac gggataatac cgcgccacat 1500
agcagaactt taaaagtgct catcattgga aaacgttctt cggggcgaaa actctcaagg 1560
atcttaccgc tgttgagatc cagttcgatg taacccactc gtgcacccaa ctgatcttca 1620

gcatctttta ctttcaccag cgtttctggg tgagcaaaaa caggaaggca aaatgccgca 1680
aaaaagggaa taagggcgac acggaaatgt tgaatactca tactcttcct ttttcaatat 1740
tattgaagca tttatcaggg ttattgtctc atgagcggat acatatttga atgtatttag 1800
aaaaataaac aaataggggt tccgcgcaca tttccccgaa aagtgccaag gaccccgcgg 1860
caggccctcc gagcgtggtg gagccgttct gtgagacagc cgggtacgag tcgtgacgct 1920
ggaaggggca agcgggtggt gggcaggaat gcggtccgcc ctgcagcaac cggaggggga 1980
gggagaaggg agcggaaaag tctccaccgg acgcggccat ggctcggggg ggggggggca 2040
gcggaggagc gcttccggcc gacgtctcgt cgctgattgg cttcttttcc tcccgccgtg 2100
tgtgaaaaca caaatggcgt gttttggttg gcgtaaggcg cctgtcagtt aacggcagcc 2160
ggagtgcgca gccgccggca gcctcgctct gcccactggg tggggcggga ggtaggtggg 2220
gtgaggcgag ctggacgtgc gggcgcggtc ggcctctggc ggggcggggg aggggaggga 2280
gggtcagcga aagtagctcg cgcgcgagcg gccgcccacc ctccccttcc tctgggggag 2340
tcgttttacc cgccgccggc cgggcctcgt cgtctgattg gctctcgggg cccagaaaac 2400
tggcccttgc cattggctcg tgttcgtgca agttgagtcc atccgccggc cagcgggggc 2460
ggcgaggagg cgctcccagg ttccggccct cccctcggcc ccgcgccgca gagtctggcc 2520
gcgcgcccct gcgcaacgtg gcaggaagcg cgcgctgggg gcggggacgg gcagtagggc 2580
tgagcggctg cggggcgggt gcaagcacgt ttccgacttg agttgcctca agaggggcgt 2640
gctgagccag acctccatcg cgcactccgg ggagtggagg gaaggagcga gggctcagtt 2700
gggctgtttt ggaggcagga agcacttgct ctcccaaagt cgctctgagt tgttatcagt 2760
aagggagctg cagtggagta ggcggggaga aggccgcacc cttctccgga gggggaggg 2820
gagtgttgca ataccttctt gggagttctc tgctgcctcc tggcttctga ggaccgccct 2880
gggcctggga gaatcccttg ccccctcttc cctcgtgat ctgcaactcc agtcttacaa 2940
accaattcag tcgactggat cctagttatt aatagtaatc aattacgggg tcattagttc 3000
atagcccata tatggagttc cgcgttacat aacttacggt aaatggcccg cctggctgac 3060
cgcccaacga cccccgccca ttgacgtcaa taatgacgta tgttcccata gtaacgccaa 3120
tagggacttt ccattgacgt caatgggtgg agtatttacg gtaaactgcc cacttggcag 3180
tacatcaagt gtatcatatg ccaagtacgc cccctattga cgtcaatgac ggtaaatggc 3240
ccgcctggca ttatgcccag tacatgacct tatgggactt tcctacttgg cagtacatct 3300
acgtattagt catcgctatt accatggtga tgcggttttg gcagtacatc aatgggcgtg 3360
gatagcggtt tgactcacgg ggatttccaa gtctccaccc cattgacgtc aatgggagtt 3420
tgttttggca ccaaaatcaa cgggactttc caaaatgtcg taacaactcc gccccattga 3480
cgcaaatggg cggtaggcgt gtacggtggg aggtctatat aagcagagct ggtttagtga 3540

accgtcagat cactagtcga ctagggataa cagggcacca tggatcccct gggtgcagcc 3600
aagccacaat ggccatggcg ccgctgtctg gccgcactgc tatttcagct gctggtggct 3660
gtgtgtttct tctcctacct gcgtgtgtcc cgagacgatg ccactggatc ccctagggct 3720
cccagtgggt cctcccgaca ggacaccact cccacccgcc ccaccctcct gatcctgcta 3780
tggacatggc ctttccacat ccctgtggct ctgtcccgct gttcagagat ggtgcccggc 3840
acagccgact gccacatcac tgccgaccgc aaggtgtacc cacaggcaga cacggtcatc 3900
gtgcaccact gggatatcat gtccaaccct aagtcacgcc tcccaccttc cccgaggccg 3960
caggggcagc gctggatctg gttcaacttg gagccacccc ctaactgcca gcacctggaa 4020
gccctggaca gatacttcaa tctcaccatg tcctaccgca gcgactccga catcttcacg 4080
ccctacggct ggctggagcc gtggtccggc cagcctgccc acccaccgct caacctctcg 4140
gccaagaccg agctggtggc ctgggcggtg tccaactgga agccggactc agccagggtg 4200
cgctactacc agagcctgca ggctcatctc aaggtggacg tgtacggacg ctcccacaag 4260
cccctgccca aggggaccat gatggagacg ctgtcccggt acaagttcta cctggccttc 4320
gagaactcct tgcaccccga ctacatcacc gagaagctgt ggaggaacgc cctggaggcc 4380
tgggccgtgc ccgtggtgct gggccccagc agaagcaact acgagaggtt cctgccaccc 4440
gacgccttca tccacgtgga cgacttccag agccccaagg acctggcccg gtacctgcag 4500
gagctggaca aggaccacgc ccgctacctg agctactttc gctggcggga gacgctgcgg 4560
cctcgctcct tcagctgggc actggatttc tgcaaggcct gctggaaact gcagcaggaa 4620
tccaggtacc agacggtgcg cagcatagcg gcttggttca cctgatcgac tgtgccttct 4680
agttgccagc catctgttgt ttgcccctcc cccgtgcctt ccttgaccct ggaaggtgcc 4740
actcccactg tcctttccta ataaaatgag gaaattgcat cgcattgtct gagtaggtgt 4800
cattctattc tggggggtgg ggtggggcag gacagcaagg gggaggattg ggaggacaat 4860
agcaggcatg ctggggatgc ggtgggctct atggcttcga cagggtcgac aagcttttcc 4920
aaaaaaaaag catgaggtgc agttgcgcct ttcctatctc ttgaatagga aaggcgcaac 4980
tgcacctcat gctggatccc gcgtcctttc cacaagatat ataaacccaa gaaatcgaaa 5040
tactttcaag ttacggtaag catatgatag tccattttaa aacataattt taaaactgca 5100
aactacccaa gaaattatta ctttctacgt cacgtatttt gtactaatat ctttgtgttt 5160
acagtcaaat taattctaat tatctctcta acagccttgt atcgtatatg caaatatgaa 5220
ggaatcatgg gaaataggcc ctcttcctgc ccgaccttgg cgcgcgctcg gcgcgcggtc 5280
acgctccgtc acgtggtgcg ttttgccagc aggcagaagt atgcaaagca tgcatctcaa 5340
ttagtcagca accaggtgtg gaaagtcccc aggctcccca gcaggcagaa gtatgcaaag 5400

catgcatctc aattagtcag caaccatagt cccgccccta actccgccca tcccgcccct 5460
aactccgccc agttccgccc attctccgcc ccatggctga ctaatttttt ttatttatgc 5520
agaggccgag gccgcctctg cctctgagct attccagaag tagtgaggag gcttttttgg 5580
aggcctaggc ttttgcaaaa agctcccggg agcttgtata tccattttcg gatctgatca 5640
gcacgtgatg aaaaagcctg aactcaccgc gacgtctgtc gagaagtttc tgatcgaaaa 5700
gttcgacagc gtgtccgacc tgatgcagct ctcggagggc gaagaatctc gtgctttcag 5760
cttcgatgta ggagggcgtg gatatgtcct gcgggtaaat agctgcgccg atggtttcta 5820
caaagatcgt tatgtttatc ggcactttgc atcggccgcg ctcccgattc cggaagtgct 5880
tgacattggg gaattcagcg agagcctgac ctattgcatc tcccgccgtg cacagggtgt 5940
cacgttgcaa gacttgcctg aaaccgaact gcccgctgtt ctgcagccgg tcgcggaggc 6000
catggatgcg atcgctgcgg ccgatcttag ccagacgagc gggttcggcc cattcggacc 6060
gcaaggaatc ggtcaataca ctacatggcg tgatttcata tgcgcgattg ctgatcccca 6120
tgtgtatcac tggcaaactg tgatggacga caccgtcagt gcgtccgtcg cgcaggctct 6180
cgatgagctg atgctttggg ccgaggactg ccccgaagtc cggcacctcg tgcacgcgga 6240
tttcggctcc aacaatgtcc tgacggacaa tggccgcata acagcggtca ttgactggag 6300
cgaggcgatg ttcggggatt cccaatacga ggtcgccaac atcttcttct ggaggccgtg 6360
gttggcttgt atggagcagc agacgcgcta cttcgagcgg aggcatccgg agcttgcagg 6420
atcgccgcgg ctccgggcgt atatgctccg cattggtctt gaccaactct atcagagctt 6480
ggttgacggc aatttcgatg atgcagcttg ggcgcagggt cgatgcgacg caatcgtccg 6540
atccggagcc gggactgtcg ggcgtacaca aatcgcccgc agaagcgcgg ccgtctggac 6600
cgatggctgt gtagaagtac tcgccgatag tggaaaccga cgccccagca ctcgtccgag 6660
ggcaaaggaa tagcacgtgc tacgagattt cgattccacc gccgccttct atgaaaggtt 6720
gggcttcgga atcgttttcc gggacgccgg ctggatgatc ctccagcgcg gggatctcat 6780
gctggagttc ttcgcccacc ccaacttgtt tattgcagct tataatggtt acaaataaag 6840
caatagcatc acaaatttca caaataaagc atttttttca ctgcattcta gttgtggttt 6900
gtccaaactc atcaatgtat cgtagagatg ggcgggagtc ttctgggcag gcttaaaggc 6960
taacctggtg tgtgggcgtt gtcctgcagg ggaattgaac aggtgtaaaa ttggagggac 7020
aagacttccc acagattttc ggttttgtcg ggaagttttt taataggggc aaataggaaa 7080
atggaggata ggagtcatct ggggtttatg cagcaaaact acaggtatat tgcttgtatc 7140
cgcctcggag atttccatga ggagataaag acatgtcacc cgagtttata ctctcctgct 7200
tagatcctac tacagtatga aatacagtgt cgcgaggtag actatgtaag cagatttaat 7260
cattttaaag agcccagtac ttcatatcca tttctcccgc tccttctgca gccttatcaa 7320

aaggtattta gaacactcat tttagcccca ttttcattta ttatactggc ttatccaacc 7380
cctagacaga gcattggcat tttccctttc ctgatcttag aagtctgatg actcatgaaa 7440
ccagacagat tagttacata caccacaaat cgaggctgta gctggggcct caacactgca 7500
gttcttttat aactccttag tacacttttt gttgatcctt tgccttgatc cttaattttc 7560
agtgtctatc acctctcccg tcaggtggtg ttccacattt gggcctattc tcagtccagg 7620
gagttttaca acaatagatg tattgagaat ccaacctaaa gcttaacttt ccactcccat 7680
gaatgcctct ctcctttttc tccattataa ctgagctata accattaatg gtttcaggtg 7740
gatgtctcct cccccaatat acctgatgta tctacatatt gccaggctga tattttaaga 7800
cataaaaggt atatttcatt attgagccac atggtattga ttactgctac taaaattttg 7860
tcattgtaca catctgtaaa aggtggttcc ttttggaatg caaagttcag gtgtttgttg 7920
tctttcctga cctaaggtct tgtgagcttg tatttttttct atttaagcag tgctttctct 7980
tggactggct tgactcatgg cattctacac gttattgctg gtctaaatgt gattttgcca 8040
agcttcttca ggacctataa ttttgcttga cttgtagcca aacacaagta aaatgattaa 8100
gcaacaaatg tatttgtgaa gcttggtttt taggttgttg tgttgtgtgt gcttgtgctc 8160
tataataata ctatccaggg gctggagagg tggctcggag ttcaagagca cagactgctc 8220
ttccagaagt cctgagttca attcccagca accacatggt ggctcacaac catctgtaat 8280
gggatctgat gccctcttct ggtgtgtctg aagaccacaa gtgtattcac attaaataaa 8340
taatcctcct tcttcttctt tttttttttt taaagagaat actgtctcca gtagaattac 8400
tgaagtaatg aaatactttg tgtttgttcc aatatggaag ccaataatca aatactctta 8460
agcactggaa atgtaccaag gaactatttt atttaagtga actgtggaca gaggagccat 8520
aactgcagac ttgtgggata cagaagacca atgcagactt aatgtctttt ctcttacact 8580
aagcaataaa gaaataaaaa ttgaacttct agtatcctat ttgttaaact gctagcttta 8640
ctaacttttg tgcttcatct atacaaagct gaaagctaag tctgcagcca ttactaaaca 8700
tgaaagcaag taatgataat tttggatttc aaaaatgtag ggccagagtt tagccagcca 8760
gtggtggtgc ttgcctttat gccttaatcc cagcactctg gaggcagaga caggcagatc 8820
tctgagtttg agcccagcct ggtctacaca tcaagttcta tctaggatag ccaggaatac 8880
acacagaaac cctgttgggg aggggggctc tgagatttca taaaattata attgaagcat 8940
tccctaatga gccactatgg atgtggctaa atccgtctac ctttctgatg agatttgggt 9000
attatttttt ctgtctctgc tgttggttgg gtcttttgac actgtgggct ttcttaaagc 9060
ctccttccct gccatgtgga ctcttgtttg ctactaactt cccatggctt aaatggcatg 9120
gctttttgcc ttctaagggc agctgctgag atttgcagcc tgatttccag ggtggggttg 9180

```
ggaaatcttt caaacactaa aattgtcctt taatttttt ttaaaaaatg ggttatataa     9240
taaacctcat aaaatagtta tgaggagtga ggtggactaa tattaatgag tccctcccct     9300
ataaaagagc tattaaggct ttttgtctta tactaacttt ttttttaaat gtggtatctt     9360
tagaaccaag ggtcttagag ttttagtata cagaaactgt tgcatcgctt aatcagattt     9420
tctagtttca aatccagaga atccaaattc ttcacagcca aagtcaaatt aagaatttct     9480
gactttaatg ttatttgcta ctgtgaatat aaaatgatag cttttcctga ggcagggtat     9540
cactatgtat ctctgcctga tctgcaacaa gatatgtaga ctaaagttct gcctgctttt     9600
gtctcctgaa tactaaggtt aaaatgtagt aatacttttg gaacttgcag gtcagattct     9660
tttatagggg acacactaag ggagcttggg tgatagttgg taaatgtgtt taagtgatga     9720
aaacttgaat tattatcacc gcaacctact ttttaaaaaa aaaagccagg cctgttagag     9780
catgctaagg gatccctagg acttgctgag cacacaagag tagtacttgg caggctcctg     9840
gtgagagcat atttcaaaaa acaaggcaga caaccaagaa actacagtaa ggttacctgt     9900
ctttaaccat ctgcatatac acagggatat taaaatattc caaataatat ttcattcaag     9960
ttttcccccca tcaaattggg acatggattt ctccggtgaa taggcagagt tggaaactaa    10020
acaaatgttg gttttgtgat ttgtgaaatt gttttcaagt gatagttaaa gcccatgaga    10080
tacagaacaa agctgctatt tcgaggtcac ttggttatac tcagaagcac ttctttgggt    10140
ttccctgcac tatcctgatc atgtgctagg cctaccttag gctgattgtt gttcaaataa    10200
cttaagtttc ctgtcaggtg atgtcatatg atttcatata tcaaggcaaa acatgttata    10260
tatgttaaac atttggactt aatgtgaaag ttaggtcttt gtgggttttg attttaattt    10320
caaaacctga gctaaataag tcattttaca tgtcttacat ttggtgaatt gtatattgtg    10380
gtttgcaggc aagactctct gacctagtaa ccctcctata gagcactttg ctgggtcaca    10440
agtctaggag tcaagcattt caccttgaag ttgagacgtt ttgttagtgt atactagtta    10500
tatgttggag gacatgttta tccagaagat attcaggact atttttgact gggctaagga    10560
attgattctg attagcactg ttagtgagca ttgagtggcc tttaggcttg aattggagtc    10620
acttgtatat ctcaaataat gctggccttt tttaaaaagc ccttgttctt tatcaccctg    10680
ttttctacat aatttttgtt caaagaaata cttgtttgga tctccttttg acaacaatag    10740
catgttttca agccatattt tttttccttt tttttttttt ttttggtttt tcgagacagg    10800
gtttctctgt atagccctgg ctgtcctgga actcactttg tagaccaggc tggcctcgaa    10860
ctcagaaatc cgcctgcctc tgcctcctga gtgccgggat taaaggcgtg caccaccacg    10920
cctggctaag ttggatattt tgtatataac tataaccaat actaactcca ctgggtggat    10980
ttttaattca gtcagtagtc ttaagtggtc tttattggcc cttattaaaa tctactgttc    11040
actctaacag aggctgttgg actagtggga ctaagcaact tcctacggat atactagcag    11100
```

ataagggtca gggatagaaa ctagtctagc gttttgtata cctaccagct tatactacct 11160

tgttctgata gaaatattta ggacatctag cttatctatt 11200

<210> 54
<211> 4309
<212> DNA
<213> Artificial sequence

<220>
<223> rosa26-3’ BsaI B

<400> 54

gaggtaccgg tctcagtaga gatgggcggg agtcttctgg gcaggcttaa aggctaacct 60
ggtgtgtggg cgttgtcctg caggggaatt gaacaggtgt aaaattggag ggacaagact 120
tcccacagat tttcggtttt gtcgggaagt tttttaatag gggcaaatag gaaaatggag 180
gataggagtc atctggggtt tatgcagcaa aactacaggt atattgcttg tatccgcctc 240
ggagatttcc atgaggagat aaagacatgt cacccgagtt tatactctcc tgcttagatc 300
ctactacagt atgaaataca gtgtcgcgag gtagactatg taagcagatt taatcatttt 360
aaagagccca gtacttcata tccatttctc ccgctccttc tgcagcctta tcaaaaggta 420
tttagaacac tcattttagc cccattttca tttattatac tggcttatcc aacccctaga 480
cagagcattg gcattttccc tttcctgatc ttagaagtct gatgactcat gaaaccagac 540
agattagtta catacaccac aaatcgaggc tgtagctggg gcctcaacac tgcagttctt 600
ttataactcc ttagtacact ttttgttgat cctttgcctt gatccttaat tttcagtgtc 660
tatcacctct cccgtcaggt ggtgttccac atttgggcct attctcagtc cagggagttt 720
tacaacaata gatgtattga gaatccaacc taaagcttaa ctttccactc ccatgaatgc 780
ctctctcctt tttctccatt ataactgagc tataaccatt aatggtttca ggtggatgtc 840
tcctccccca atatacctga tgtatctaca tattgccagg ctgatatttt aagacataaa 900
aggtatattt cattattgag ccacatggta ttgattactg ctactaaaat tttgtcattg 960
tacacatctg taaaaggtgg ttccttttgg aatgcaaagt tcaggtgttt gttgtctttc 1020
ctgacctaag gtcttgtgag cttgtatttt ttctatttaa gcagtgcttt ctcttggact 1080
ggcttgactc atggcattct acacgttatt gctggtctaa atgtgatttt gccaagcttc 1140
ttcaggacct ataattttgc ttgacttgta gccaaacaca agtaaaatga ttaagcaaca 1200
aatgtatttg tgaagcttgg tttttaggtt gttgtgttgt gtgtgcttgt gctctataat 1260
aatactatcc aggggctgga gaggtggctc ggagttcaag agcacagact gctcttccag 1320
aagtcctgag ttcaattccc agcaaccaca tggtggctca caaccatctg taatgggatc 1380
tgatgccctc ttctggtgtg tctgaagacc acaagtgtat tcacattaaa taaataatcc 1440

tccttcttct tctttttttt tttttaaaga gaatactgtc tccagtagaa ttactgaagt 1500
aatgaaatac tttgtgtttg ttccaatatg gaagccaata atcaaatact cttaagcact 1560
ggaaatgtac caaggaacta ttttatttaa gtgaactgtg gacagaggag ccataactgc 1620
agacttgtgg gatacagaag accaatgcag acttaatgtc ttttctctta cactaagcaa 1680
taaagaaata aaaattgaac ttctagtatc ctatttgtta aactgctagc tttactaact 1740
tttgtgcttc atctatacaa agctgaaagc taagtctgca gccattacta aacatgaaag 1800
caagtaatga taattttgga tttcaaaaat gtagggccag agtttagcca gccagtggtg 1860
gtgcttgcct ttatgcctta atcccagcac tctggaggca gagacaggca gatctctgag 1920
tttgagccca gcctggtcta cacatcaagt tctatctagg atagccagga atacacacag 1980
aaaccctgtt ggggaggggg gctctgagat ttcataaaat tataattgaa gcattcccta 2040
atgagccact atggatgtgg ctaaatccgt ctacctttct gatgagattt gggtattatt 2100
ttttctgtct ctgctgttgg ttgggtcttt tgacactgtg ggctttctta aagcctcctt 2160
ccctgccatg tggactcttg tttgctacta acttcccatg gcttaaatgg catggctttt 2220
tgccttctaa gggcagctgc tgagatttgc agcctgattt ccagggtggg gttgggaaat 2280
ctttcaaaca ctaaaattgt cctttaattt ttttttaaaa aatgggttat ataataaacc 2340
tcataaaata gttatgagga gtgaggtgga ctaatattaa tgagtccctc ccctataaaa 2400
gagctattaa ggctttttgt cttatactaa cttttttttt aaatgtggta tctttagaac 2460
caagggtctt agagttttag tatacagaaa ctgttgcatc gcttaatcag attttctagt 2520
ttcaaatcca gagaatccaa attcttcaca gccaaagtca aattaagaat ttctgacttt 2580
aatgttattt gctactgtga atataaaatg atagcttttc ctgaggcagg gtatcactat 2640
gtatctctgc ctgatctgca acaagatatg tagactaaag ttctgcctgc ttttgtctcc 2700
tgaatactaa ggttaaaatg tagtaatact tttggaactt gcaggtcaga ttcttttata 2760
ggggacacac taagggagct tgggtgatag ttggtaaatg tgtttaagtg atgaaaactt 2820
gaattattat caccgcaacc tactttttaa aaaaaaaagc caggcctgtt agagcatgct 2880
aagggatccc taggacttgc tgagcacaca agagtagtac ttggcaggct cctggtgaga 2940
gcatatttca aaaaacaagg cagacaacca agaaactaca gtaaggttac ctgtctttaa 3000
ccatctgcat atacacaggg atattaaaat attccaaata atatttcatt caagttttcc 3060
cccatcaaat tgggacatgg atttctccgg tgaataggca gagttggaaa ctaaacaaat 3120
gttggttttg tgatttgtga aattgttttc aagtgatagt taaagcccat gagatacaga 3180
acaaagctgc tatttcgagg tcacttggtt atactcagaa gcacttcttt gggtttccct 3240
gcactatcct gatcatgtgc taggcctacc ttaggctgat tgttgttcaa ataacttaag 3300
tttcctgtca ggtgatgtca tatgatttca tatatcaagg caaaacatgt tatatatgtt 3360

aaacatttgg acttaatgtg aaagttaggt ctttgtgggt tttgatttta atttcaaaac 3420

ctgagctaaa taagtcattt tacatgtctt acatttggtg aattgtatat tgtggtttgc 3480

aggcaagact ctctgaccta gtaaccctcc tatagagcac tttgctgggt cacaagtcta 3540

ggagtcaagc atttcacctt gaagttgaga cgttttgtta gtgtatacta gttatatgtt 3600

ggaggacatg tttatccaga agatattcag gactattttt gactgggcta aggaattgat 3660

tctgattagc actgttagtg agcattgagt ggcctttagg cttgaattgg agtcacttgt 3720

atatctcaaa taatgctggc ctttttttaaa aagcccttgt tctttatcac cctgttttct 3780

acataatttt tgttcaaaga aatacttgtt tggatctcct tttgacaaca atagcatgtt 3840

ttcaagccat atttttttttc cttttttttt tttttttgg tttttcgaga cagggtttct 3900

ctgtatagcc ctggctgtcc tggaactcac tttgtagacc aggctggcct cgaactcaga 3960

aatccgcctg cctctgcctc ctgagtgccg ggattaaagg cgtgcaccac cacgcctggc 4020

taagttggat attttgtata taactataac caatactaac tccactgggt ggatttttaa 4080

ttcagtcagt agtcttaagt ggtctttatt ggcccttatt aaaatctact gttcactcta 4140

acagaggctg ttggactagt gggactaagc aacttcctac ggatatacta gcagataagg 4200

gtcagggata gaaactagtc tagcgttttg tatacctacc agcttatact accttgttct 4260

gatagaaata tttaggacat ctagcttatc atgccgagac cggtacctc 4309

<210> 55
<211> 374
<212> DNA
<213> Artificial sequence

<220>
<223> pEF1a BsaI A

<400> 55

gaggtaccgg tctcaatgca aggaaccaat tcagtcgact ggatcccgat ggctccggtg 60

cccgtcagtg ggcagagcgc acatcgccca cagtccccga gaagttgggg ggaggggtcg 120

gcaattgaac cggtgcctag agaaggtggc gcggggtaaa ctgggaaagt gatgtcgtgt 180

actggctccg cctttttccc gagggtgggg gagaaccgta tataagtgca gtagtcgccg 240

tgaacgttct ttttcgcaac gggtttgccg ccagaacaca ggtccgcggc cccgaactag 300

gcctaggcgt ctgatcacta gtgactctag tcctagtcga ctagggataa caggggcccc 360

gagaccggta cctc 374

<210> 56
<211> 1169
<212> DNA

<213> Artificial sequence

<220>
<223> TK BsaI A

<400> 56

```
gaggtaccgg tctcagccca tggcttcgta cccctgccat caacacgcgt ctgcgttcga      60
ccaggctgcg cgttctcgcg gccatagcaa ccgacgtacg gcgttgcgcc ctcgccggca     120
gcaagaagcc acggaagtcc gcctggagca gaaaatgccc acgctactgc gggtttatat     180
agacggtcct cacgggatgg ggaaaaccac caccacgcaa ctgctggtgg ccctgggttc     240
gcgcgacgat atcgtctacg tacccgagcc gatgacttac tggcaggtgc tgggggcttc     300
cgagacaatc gcgaacatct acaccacaca acaccgcctc gaccagggtg agatatcggc     360
cggggacgcg gcggtggtaa tgacaagcgc ccagataaca atgggcatgc cttatgccgt     420
gaccgacgcc gttctggctc ctcatatcgg gggggaggct gggagctcac atgccccgcc     480
cccggccctc accctcatct tcgaccgcca tcccatcgcc gccctcctgt gctacccggc     540
cgcgcgatac cttatgggca gcatgacccc ccaggccgtg ctggcgttcg tggccctcat     600
cccgccgacc ttgcccggca caaacatcgt gttgggggcc cttccggagg acagacacat     660
cgaccgcctg gccaaacgcc agcgccccgg cgagcggctt gacctggcta tgctggccgc     720
gattcgccgc gtttacgggc tgcttgccaa tacggtgcgg tatctgcagg gcggcgggtc     780
gtggcgggag gattggggac agctttcggg gacggccgtg ccgcccccagg gtgccgagcc     840
ccagagcaac gcgggcccac gaccccatat cggggacacg ttatttaccc tgtttcgggc     900
ccccgagttg ctggccccca acggcgacct gtacaacgtg tttgcctggg ccttggacgt     960
cttggccaaa cgcctccgtc ccatgcacgt ctttatcctg gattacgacc aatcgcccgc    1020
cggctgccgg gacgccctgc tgcaacttac ctccgggatg gtccagaccc acgtcaccac    1080
cccccggctcc ataccgacga tctgcgacct ggcgcgcacg tttgcccggg agatggggga    1140
ggctaactga ccgccgagac cggtacctc                                      1169
```

<210> 57
<211> 310
<212> DNA
<213> Artificial sequence

<220>
<223> Tkter BsaI A

<400> 57

gaggtaccgg tctcaccgcg ggggaggcta actgaaacac ggaaggagac aataccggaa 60

ggaacccgcg ctatgacggc aataaaaaga cagaataaaa cgcacggtgt tgggtcgttt 120

gttcataaac gcggggttcg gtcccagggc tggcactctg tcgatacccc accgaggccc 180

cattggggcc aatacgcccg cgtttcttcc ttttccccac cccacccccc aagttcgggt 240

gaaggcccag ggctcgcagc caacgtcggg gcggcaggcc ctgccatagc ctattcgaga 300

ccggtacctc 310

<210> 58
<211> 12951
<212> DNA
<213> Artificial sequence

<220>
<223> V1.3

<400> 58

tattgtaata cggttatcca cagaatcagg ggataacgca ggaaagaaca tgtgagcaaa 60
aggccagcaa aaggccagga accgtaaaaa ggccgcgttg ctggcgtttt tccataggct 120
ccgcccccct gacgagcatc acaaaaatcg acgctcaagt cagaggtggc gaaacccgac 180
aggactataa agataccagg cgtttccccc tggaagctcc ctcgtgcgct ctcctgttcc 240
gaccctgccg cttaccggat acctgtccgc ctttctccct tcgggaagcg tggcgctttc 300
tcatagctca cgctgtaggt atctcagttc ggtgtaggtc gttcgctcca agctgggctg 360
tgtgcacgaa ccccccgttc agcccgaccg ctgcgcctta tccggtaact atcgtcttga 420
gtccaacccg gtaagacacg acttatcgcc actggcagca gccactggta acaggattag 480
cagagcgagg tatgtaggcg gtgctacaga gttcttgaag tggtggccta actacggcta 540
cactagaaga acagtatttg gtatctgcgc tctgctgaag ccagttacct tcggaaaaag 600
agttggtagc tcttgatccg gcaaacaaac caccgctggt agcggttttt ttgtttgcaa 660
gcagcagatt acgcgcagaa aaaaaggatc tcaagaagat cctttgatct tttctacggg 720
gtctgacgct cagtggaacg aaaactcacg ttaagggatt ttggtcatga gattatcaaa 780
aaggatcttc acctagatcc ttttaaatta aaaatgaagt tttaaatcaa tctaaagtat 840
atatgagtaa acttggtctg acagttacca atgcttaatc agtgaggcac ctatctcagc 900
gatctgtcta tttcgttcat ccatagttgc ctgactcccc gtcgtgtaga taactacgat 960
acgggagggc ttaccatctg gccccagtgc tgcaatgata ccgcgtgacc cacgctcacc 1020
ggctccagat ttatcagcaa taaaccagcc agccggaagg gccgagcgca gaagtggtcc 1080
tgcaacttta tccgcctcca tccagtctat taattgttgc cgggaagcta gagtaagtag 1140
ttcgccagtt aatagtttgc gcaacgttgt tgccattgct acaggcatcg tggtgtcacg 1200
ctcgtcgttt ggtatggctt cattcagctc cggttcccaa cgatcaaggc gagttacatg 1260
atcccccatg ttgtgcaaaa aagcggttag ctccttcggt cctccgatcg ttgtcagaag 1320
taagttggcc gcagtgttat cactcatggt tatggcagca ctgcataatt ctcttactgt 1380
catgccatcc gtaagatgct tttctgtgac tggtgagtac tcaaccaagt cattctgaga 1440
atagtgtatg cggcgaccga gttgctcttg cccggcgtca atacgggata ataccgcgcc 1500

acatagcaga actttaaaag tgctcatcat tggaaaacgt tcttcggggc gaaaactctc 1560
aaggatctta ccgctgttga gatccagttc gatgtaaccc actcgtgcac ccaactgatc 1620
ttcagcatct tttactttca ccagcgtttc tgggtgagca aaaacaggaa ggcaaaatgc 1680
cgcaaaaaag ggaataaggg cgacacggaa atgttgaata ctcatactct tcctttttca 1740
atattattga agcatttatc agggttattg tctcatgagc ggatacatat ttgaatgtat 1800
ttagaaaaat aaacaaatag gggttccgcg cacatttccc cgaaaagtgc caaggacccc 1860
gcggcaggcc ctccgagcgt ggtggagccg ttctgtgaga cagccgggta cgagtcgtga 1920
cgctggaagg ggcaagcggg tggtgggcag gaatgcggtc cgccctgcag caaccggagg 1980
gggagggaga agggagcgga aaagtctcca ccggacgcgg ccatggctcg gggggggggg 2040
ggcagcggag gagcgcttcc ggccgacgtc tcgtcgctga ttggcttctt ttcctcccgc 2100
cgtgtgtgaa aacacaaatg gcgtgttttg gttggcgtaa ggcgcctgtc agttaacggc 2160
agccggagtg cgcagccgcc ggcagcctcg ctctgcccac tgggtggggc gggaggtagg 2220
tggggtgagg cgagctggac gtgcgggcgc ggtcggcctc tggcggggcg gggagggga 2280
gggagggtca gcgaaagtag ctcgcgcgcg agcggccgcc caccctcccc ttcctctggg 2340
ggagtcgttt tacccgccgc cggccgggcc tcgtcgtctg attggctctc ggggcccaga 2400
aaactggccc ttgccattgg ctcgtgttcg tgcaagttga gtccatccgc cggccagcgg 2460
gggcggcgag gaggcgctcc caggttccgg ccctcccctc ggccccgcgc cgcagagtct 2520
ggccgcgcgc ccctgcgcaa cgtggcagga agcgcgcgct gggggcgggg acgggcagta 2580
gggctgagcg gctgcggggc gggtgcaagc acgtttccga cttgagttgc ctcaagaggg 2640
gcgtgctgag ccagacctcc atcgcgcact ccggggagtg gagggaagga gcgagggctc 2700
agttgggctg ttttggaggc aggaagcact tgctctccca aagtcgctct gagttgttat 2760
cagtaaggga gctgcagtgg agtaggcggg gagaaggccg cacccttctc cggagggggg 2820
aggggagtgt tgcaatacct ttctgggagt tctctgctgc ctcctggctt ctgaggaccg 2880
ccctgggcct gggagaatcc cttgcccccct cttcccctcg tgatctgcaa ctccagtctt 2940
acaaaccaat tcagtcgact ggatcctagt tattaatagt aatcaattac ggggtcatta 3000
gttcatagcc catatatgga gttccgcgtt acataactta cggtaaatgg cccgcctggc 3060
tgaccgccca acgacccccg cccattgacg tcaataatga cgtatgttcc catagtaacg 3120
ccaataggga ctttccattg acgtcaatgg gtggagtatt tacggtaaac tgcccacttg 3180
gcagtacatc aagtgtatca tatgccaagt acgcccccta ttgacgtcaa tgacggtaaa 3240
tggcccgcct ggcattatgc ccagtacatg accttatggg actttcctac ttggcagtac 3300
atctacgtat tagtcatcgc tattaccatg gtgatgcggt tttggcagta catcaatggg 3360
cgtggatagc ggtttgactc acggggattt ccaagtctcc accccattga cgtcaatggg 3420

agtttgtttt ggcaccaaaa tcaacgggac tttccaaaat gtcgtaacaa ctccgcccca 3480

ttgacgcaaa tgggcggtag gcgtgtacgg tgggaggtct atataagcag agctggttta 3540

gtgaaccgtc agatcactag tcgactaggg ataacagggc accatggatc ccctgggtgc 3600

agccaagcca caatggccat ggcgccgctg tctggccgca ctgctatttc agctgctggt 3660

ggctgtgtgt ttcttctcct acctgcgtgt gtcccgagac gatgccactg gatcccctag 3720

ggctcccagt gggtcctccc gacaggacac cactcccacc cgccccaccc tcctgatcct 3780

gctatggaca tggcctttcc acatccctgt ggctctgtcc cgctgttcag agatggtgcc 3840

cggcacagcc gactgccaca tcactgccga ccgcaaggtg tacccacagg cagacacggt 3900

catcgtgcac cactgggata tcatgtccaa ccctaagtca cgcctcccac cttccccgag 3960

gccgcagggg cagcgctgga tctggttcaa cttggagcca ccccctaact gccagcacct 4020

ggaagccctg gacagatact tcaatctcac catgtcctac cgcagcgact ccgacatctt 4080

cacgccctac ggctggctgg agccgtggtc cggccagcct gcccacccac cgctcaacct 4140

ctcggccaag accgagctgg tggcctgggc ggtgtccaac tggaagccgg actcagccag 4200

ggtgcgctac taccagagcc tgcaggctca tctcaaggtg gacgtgtacg gacgctccca 4260

caagcccctg cccaagggga ccatgatgga gacgctgtcc cggtacaagt tctacctggc 4320

cttcgagaac tccttgcacc ccgactacat caccgagaag ctgtggagga acgccctgga 4380

ggcctgggcc gtgcccgtgg tgctgggccc cagcagaagc aactacgaga ggttcctgcc 4440

acccgacgcc ttcatccacg tggacgactt ccagagcccc aaggacctgg cccggtacct 4500

gcaggagctg gacaaggacc acgcccgcta cctgagctac tttcgctggc gggagacgct 4560

gcggcctcgc tccttcagct gggcactgga tttctgcaag gcctgctgga aactgcagca 4620

ggaatccagg taccagacgg tgcgcagcat agcggcttgg ttcacctgat cgactgtgcc 4680

ttctagttgc cagccatctg ttgtttgccc ctcccccgtg ccttccttga ccctggaagg 4740

tgccactccc actgtccttt cctaataaaa tgaggaaatt gcatcgcatt gtctgagtag 4800

gtgtcattct attctggggg gtggggtggg gcaggacagc aagggggagg attgggagga 4860

caatagcagg catgctgggg atgcggtggg ctctatggct tcgacagggt cgacaagctt 4920

ttccaaaaaa aaagcatgag gtgcagttgc gcctttccta tctcttgaat aggaaaggcg 4980

caactgcacc tcatgctgga tcccgcgtcc tttccacaag atatataaac ccaagaaatc 5040

gaaatacttt caagttacgg taagcatatg atagtccatt ttaaaacata attttaaaac 5100

tgcaaactac ccaagaaatt attactttct acgtcacgta ttttgtacta atatctttgt 5160

gtttacagtc aaattaattc taattatctc tctaacagcc ttgtatcgta tatgcaaata 5220

tgaaggaatc atgggaaata ggccctcttc ctgcccgacc ttggcgcgcg ctcggcgcgc 5280

ggtcacgctc cgtcacgtgg tgcgttttgc cagcaggcag aagtatgcaa agcatgcatc 5340
tcaattagtc agcaaccagg tgtggaaagt ccccaggctc cccagcaggc agaagtatgc 5400
aaagcatgca tctcaattag tcagcaacca tagtcccgcc cctaactccg cccatcccgc 5460
ccctaactcc gcccagttcc gcccattctc cgccccatgg ctgactaatt ttttttattt 5520
atgcagaggc cgaggccgcc tctgcctctg agctattcca gaagtagtga ggaggctttt 5580
ttggaggcct aggcttttgc aaaaagctcc cgggagcttg tatatccatt ttcggatctg 5640
atcagcacgt gatgaaaaag cctgaactca ccgcgacgtc tgtcgagaag tttctgatcg 5700
aaaagttcga cagcgtgtcc gacctgatgc agctctcgga gggcgaagaa tctcgtgctt 5760
tcagcttcga tgtaggaggg cgtggatatg tcctgcgggt aaatagctgc gccgatggtt 5820
tctacaaaga tcgttatgtt tatcggcact ttgcatcggc cgcgctcccg attccggaag 5880
tgcttgacat tggggaattc agcgagagcc tgacctattg catctcccgc cgtgcacagg 5940
gtgtcacgtt gcaagacttg cctgaaaccg aactgcccgc tgttctgcag ccggtcgcgg 6000
aggccatgga tgcgatcgct gcggccgatc ttagccagac gagcgggttc ggcccattcg 6060
gaccgcaagg aatcggtcaa tacactacat ggcgtgattt catatgcgcg attgctgatc 6120
cccatgtgta tcactggcaa actgtgatgg acgacaccgt cagtgcgtcc gtcgcgcagg 6180
ctctcgatga gctgatgctt tgggccgagg actgccccga agtccggcac ctcgtgcacg 6240
cggatttcgg ctccaacaat gtcctgacgg acaatggccg cataacagcg gtcattgact 6300
ggagcgaggc gatgttcggg gattcccaat acgaggtcgc caacatcttc ttctggaggc 6360
cgtggttggc ttgtatggag cagcagacgc gctacttcga gcggaggcat ccggagcttg 6420
caggatcgcc gcggctccgg gcgtatatgc tccgcattgg tcttgaccaa ctctatcaga 6480
gcttggttga cggcaatttc gatgatgcag cttgggcgca gggtcgatgc gacgcaatcg 6540
tccgatccgg agccgggact gtcgggcgta cacaaatcgc ccgcagaagc gcggccgtct 6600
ggaccgatgg ctgtgtagaa gtactcgccg atagtggaaa ccgacgcccc agcactcgtc 6660
cgagggcaaa ggaatagcac gtgctacgag atttcgattc caccgccgcc ttctatgaaa 6720
ggttgggctt cggaatcgtt ttccgggacg ccggctggat gatcctccag cgcggggatc 6780
tcatgctgga gttcttcgcc caccccaact tgtttattgc agcttataat ggttacaaat 6840
aaagcaatag catcacaaat ttcacaaata aagcattttt ttcactgcat tctagttgtg 6900
gtttgtccaa actcatcaat gtatcgtaga gatgggcggg agtcttctgg gcaggcttaa 6960
aggctaacct ggtgtgtggg cgttgtcctg caggggaatt gaacaggtgt aaaattggag 7020
ggacaagact tcccacagat tttcggtttt gtcgggaagt tttttaatag gggcaaatag 7080
gaaaatggag gataggagtc atctggggtt tatgcagcaa aactacaggt atattgcttg 7140
tatccgcctc ggagatttcc atgaggagat aaagacatgt cacccgagtt tatactctcc 7200

tgcttagatc ctactacagt atgaaataca gtgtcgcgag gtagactatg taagcagatt 7260
taatcatttt aaagagccca gtacttcata tccatttctc ccgctccttc tgcagcctta 7320
tcaaaaggta tttagaacac tcattttagc cccattttca tttattatac tggcttatcc 7380
aacccctaga cagagcattg gcattttccc tttcctgatc ttagaagtct gatgactcat 7440
gaaaccagac agattagtta catacaccac aaatcgaggc tgtagctggg gcctcaacac 7500
tgcagttctt ttataactcc ttagtacact ttttgttgat cctttgcctt gatccttaat 7560
tttcagtgtc tatcacctct cccgtcaggt ggtgttccac atttgggcct attctcagtc 7620
cagggagttt tacaacaata gatgtattga gaatccaacc taaagcttaa ctttccactc 7680
ccatgaatgc ctctctcctt tttctccatt ataactgagc tataaccatt aatggtttca 7740
ggtggatgtc tcctccccca atatacctga tgtatctaca tattgccagg ctgatatttt 7800
aagacataaa aggtatattt cattattgag ccacatggta ttgattactg ctactaaaat 7860
tttgtcattg tacacatctg taaaaggtgg ttccttttgg aatgcaaagt tcaggtgttt 7920
gttgtctttc ctgacctaag gtcttgtgag cttgtatttt ttctatttaa gcagtgcttt 7980
ctcttggact ggcttgactc atggcattct acacgttatt gctggtctaa atgtgatttt 8040
gccaagcttc ttcaggacct ataattttgc ttgacttgta gccaaacaca agtaaaatga 8100
ttaagcaaca aatgtatttg tgaagcttgg tttttaggtt gttgtgttgt gtgtgcttgt 8160
gctctataat aatactatcc aggggctgga gaggtggctc ggagttcaag agcacagact 8220
gctcttccag aagtcctgag ttcaattccc agcaaccaca tggtggctca caaccatctg 8280
taatgggatc tgatgccctc ttctggtgtg tctgaagacc acaagtgtat tcacattaaa 8340
taaataatcc tccttcttct tctttttttt tttttaaaga gaatactgtc tccagtagaa 8400
ttactgaagt aatgaaatac tttgtgtttg ttccaatatg gaagccaata atcaaatact 8460
cttaagcact ggaaatgtac caaggaacta ttttatttaa gtgaactgtg gacagaggag 8520
ccataactgc agacttgtgg gatacagaag accaatgcag acttaatgtc ttttctctta 8580
cactaagcaa taaagaaata aaaattgaac ttctagtatc ctatttgtta aactgctagc 8640
tttactaact tttgtgcttc atctatacaa agctgaaagc taagtctgca gccattacta 8700
aacatgaaag caagtaatga taattttgga tttcaaaaat gtagggccag agtttagcca 8760
gccagtggtg gtgcttgcct ttatgcctta atcccagcac tctggaggca gagacaggca 8820
gatctctgag tttgagccca gcctggtcta cacatcaagt tctatctagg atagccagga 8880
atacacacag aaaccctgtt ggggaggggg gctctgagat ttcataaaat tataattgaa 8940
gcattcccta atgagccact atggatgtgg ctaaatccgt ctacctttct gatgagattt 9000
gggtattatt ttttctgtct ctgctgttgg ttgggtcttt tgacactgtg ggctttctta 9060

aagcctcctt ccctgccatg tggactcttg tttgctacta acttcccatg gcttaaatgg 9120

catggctttt tgccttctaa gggcagctgc tgagatttgc agcctgattt ccagggtggg 9180

gttgggaaat ctttcaaaca ctaaaattgt cctttaattt ttttttaaaa aatgggttat 9240

ataataaacc tcataaaata gttatgagga gtgaggtgga ctaatattaa tgagtccctc 9300

ccctataaaa gagctattaa ggctttttgt cttatactaa cttttttttt aaatgtggta 9360

tctttagaac caagggtctt agagttttag tatacagaaa ctgttgcatc gcttaatcag 9420

attttctagt ttcaaatcca gagaatccaa attcttcaca gccaaagtca aattaagaat 9480

ttctgacttt aatgttattt gctactgtga atataaaatg atagcttttc ctgaggcagg 9540

gtatcactat gtatctctgc ctgatctgca acaagatatg tagactaaag ttctgcctgc 9600

ttttgtctcc tgaatactaa ggttaaaatg tagtaatact tttggaactt gcaggtcaga 9660

ttcttttata ggggacacac taagggagct tgggtgatag ttggtaaatg tgtttaagtg 9720

atgaaaactt gaattattat caccgcaacc tacttttttaa aaaaaaaagc caggcctgtt 9780

agagcatgct aagggatccc taggacttgc tgagcacaca agagtagtac ttggcaggct 9840

cctggtgaga gcatatttca aaaaacaagg cagacaacca agaaactaca gtaaggttac 9900

ctgtctttaa ccatctgcat atacacaggg atattaaaat attccaaata atatttcatt 9960

caagttttcc cccatcaaat tgggacatgg atttctccgg tgaataggca gagttggaaa 10020

ctaaacaaat gttggttttg tgatttgtga aattgttttc aagtgatagt taaagcccat 10080

gagatacaga acaaagctgc tatttcgagg tcacttggtt atactcagaa gcacttcttt 10140

gggtttccct gcactatcct gatcatgtgc taggcctacc ttaggctgat tgttgttcaa 10200

ataacttaag tttcctgtca ggtgatgtca tatgatttca tatatcaagg caaaacatgt 10260

tatatatgtt aaacatttgg acttaatgtg aaagttaggt ctttgtgggt tttgatttta 10320

atttcaaaac ctgagctaaa taagtcattt tacatgtctt acatttggtg aattgtatat 10380

tgtggtttgc aggcaagact ctctgaccta gtaaccctcc tatagagcac tttgctgggt 10440

cacaagtcta ggagtcaagc atttcacctt gaagttgaga cgttttgtta gtgtatacta 10500

gttatatgtt ggaggacatg tttatccaga agatattcag gactattttt gactgggcta 10560

aggaattgat tctgattagc actgttagtg agcattgagt ggcctttagg cttgaattgg 10620

agtcacttgt atatctcaaa taatgctggc ctttttttaaa aagcccttgt tctttatcac 10680

cctgttttct acataatttt tgttcaaaga aatacttgtt tggatctcct tttgacaaca 10740

atagcatgtt ttcaagccat atttttttttc cttttttttt tttttttgg tttttcgaga 10800

cagggtttct ctgtatagcc ctggctgtcc tggaactcac tttgtagacc aggctggcct 10860

cgaactcaga aatccgcctg cctctgcctc ctgagtgccg ggattaaagg cgtgcaccac 10920

cacgcctggc taagttggat attttgtata taactataac caatactaac tccactgggt 10980

ggatttttaa ttcagtcagt agtcttaagt ggtctttatt ggcccttatt aaaatctact 11040
gttcactcta acagaggctg ttggactagt gggactaagc aacttcctac ggatatacta 11100
gcagataagg gtcagggata gaaactagtc tagcgttttg tatacctacc agcttatact 11160
accttgttct gatagaaata tttaggacat ctagcttatc atgcaaggaa ccaattcagt 11220
cgactggatc ccgatggctc cggtgcccgt cagtgggcag agcgcacatc gcccacagtc 11280
cccgagaagt tggggggagg ggtcggcaat tgaaccggtg cctagagaag gtggcgcggg 11340
gtaaactggg aaagtgatgt cgtgtactgg ctccgccttt ttcccgaggg tgggggagaa 11400
ccgtatataa gtgcagtagt cgccgtgaac gttctttttc gcaacgggtt tgccgccaga 11460
acacaggtcc gcggccccga actaggccta ggcgtctgat cactagtgac tctagtccta 11520
gtcgactagg gataacaggg gcccatggct tcgtacccct gccatcaaca cgcgtctgcg 11580
ttcgaccagg ctgcgcgttc tcgcggccat agcaaccgac gtacggcgtt gcgccctcgc 11640
cggcagcaag aagccacgga agtccgcctg gagcagaaaa tgcccacgct actgcgggtt 11700
tatatagacg gtcctcacgg gatggggaaa accaccacca cgcaactgct ggtggccctg 11760
ggttcgcgcg acgatatcgt ctacgtaccc gagccgatga cttactggca ggtgctgggg 11820
gcttccgaga caatcgcgaa catctacacc acacaacacc gcctcgacca gggtgagata 11880
tcggccgggg acgcggcggt ggtaatgaca agcgcccaga taacaatggg catgccttat 11940
gccgtgaccg acgccgttct ggctcctcat atcgggggg aggctgggag ctcacatgcc 12000
ccgcccccgg ccctcaccct catcttcgac cgccatccca tcgccgccct cctgtgctac 12060
ccggccgcgc gataccttat gggcagcatg accccccagg ccgtgctggc gttcgtggcc 12120
ctcatcccgc cgaccttgcc cggcacaaac atcgtgttgg gggcccttcc ggaggacaga 12180
cacatcgacc gcctggccaa acgccagcgc cccggcgagc ggcttgacct ggctatgctg 12240
gccgcgattc gccgcgttta cgggctgctt gccaatacgg tgcggtatct gcagggcggc 12300
gggtcgtggc gggaggattg gggacagctt tcggggacgg ccgtgccgcc ccagggtgcc 12360
gagccccaga gcaacgcggg cccacgaccc catatcgggg acacgttatt taccctgttt 12420
cgggcccccg agttgctggc ccccaacggc gacctgtaca acgtgtttgc ctgggccttg 12480
gacgtcttgg ccaaacgcct ccgtcccatg cacgtcttta tcctggatta cgaccaatcg 12540
cccgccggct gccgggacgc cctgctgcaa cttacctccg ggatggtcca gacccacgtc 12600
accacccccg gctccatacc gacgatctgc gacctggcgc gcacgtttgc ccgggagatg 12660
ggggaggcta actgaccgcg gggaggcta actgaaacac ggaaggagac aataccggaa 12720
ggaacccgcg ctatgacggc aataaaaaga cagaataaaa cgcacggtgt tgggtcgttt 12780
gttcataaac gcggggttcg gtcccagggc tggcactctg tcgatacccc accgaggccc 12840

cattggggcc aatacgcccg cgtttcttcc ttttccccac cccacccccc aagttcgggt 12900

gaaggcccag ggctcgcagc caacgtcggg gcggcaggcc ctgccatagc c 12951

<210> 59
<211> 785
<212> DNA
<213> Artificial sequence

<220>
<223> TO3G BsaI A

<400> 59

gaggtaccgg tctcacacca tgtctagact ggacaagagc aaagtcataa actctgctct 60

ggaattactc aatggagtcg gtatcgaagg cctgacgaca aggaaactcg ctcaaaagct 120

gggagttgag cagcctaccc tgtactggca cgtgaagaac aagcgggccc tgctcgatgc 180

cctgccaatc gagatgctgg acaggcatca tacccactcc tgcccccctgg aaggcgagtc 240

atggcaagac tttctgcgga acaacgccaa gtcataccgc tgtgctctcc tctcacatcg 300

cgacggggct aaagtgcatc tcggcacccg cccaacagag aaacagtacg aaaccctgga 360

aaatcagctc gcgttcctgt gtcagcaagg cttctccctg gagaacgcac tgtacgctct 420

gtccgccgtg ggccacttta cactgggctg cgtattggag gaacaggagc atcaagtagc 480

aaaagaggaa agagagacac ctaccaccga ttctatgccc ccacttctga aacaagcaat 540

tgagctgttc gaccggcagg gagccgaacc tgccttcctt ttcggcctgg aactaatcat 600

atgtggcctg gagaaacagc taaagtgcga aagcggcggg ccgaccgacg cccttgacga 660

ttttgactta gacatgctcc cagccgatgc ccttgacgac tttgaccttg atatgctgcc 720

tgctgacgct cttgacgatt ttgaccttga catgctcccc gggtaatgat cgagaccggt 780

acctc 785

<210> 60
<211> 439
<212> DNA
<213> Artificial sequence

<220>
<223> pTRE3G BsaI A

<400> 60

```
gaggtaccgg tctcattcgc tttcgtcttc aagaattcct ggagtttact ccctatcagt        60
gatagagaac gtatgaagag tttactccct atcagtgata gagaacgtat gcagacttta       120
ctccctatca gtgatagaga acgtataagg agtttactcc ctatcagtga tagagaacgt       180
atgaccagtt tactccctat cagtgataga gaacgtatct acagtttact ccctatcagt       240
gatagagaac gtatatccag tttactccct atcagtgata gagaacgtat aagctttagg       300
cgtgtacggt gggcgcctat aaaagcagag ctcgtttagt gaaccgtcag atcgcctgga       360

gcaattccac aacacttttg tcttatacca actttccgta ccacttccta ccctcgtaaa       420
ccagcgagac cggtacctc                                                     439
```

<210> 61
<211> 986
<212> DNA
<213> Artificial sequence

<220>
<223> mB3Galt6 BsaI A

<400> 61

```
ccagatgaag gtattccggc gcgcttggcg gcaccgggtg gcgctgggcc taggcggcct        60
ggcgttctgc ggcaccactc tgttgtacct ggcgcgctgc gcttccgagg gcgagacgcc       120
ctccgcttcc ggagccgctc ggccccgcgc taaggccttc ctggcggtgc tggtggccag       180
tgcgccccgc gcggtcgagc gccgcaccgc agtgcgcagc acgtggctgg caccggagag       240
gcgtggcgga cccgaggacg tgtgggcgcg cttcgccgtg ggcactggcg gcttaggctc       300
ggaggagcgg cgcgctcttg agctcgagca ggcgcagcac ggggacctgc tgctgctgcc       360
cgccctgcgc gacgcctacg agaacctcac ggccaaggtc ctggccatgc tgacctggct       420
ggatgagcgc gtggacttcg agttcgtgct caaggcggac gacgactcct ttgcgcgcct       480
ggacgctatc ctggtggacc tacgcgcacg ggagcccgca cgccgccggc gcctctactg       540
gggcttcttt tccgggcgcg ggcgcgtcaa gccgggaggt cgctggcgag aagcagcctg       600
gcaactctgc gactactacc tgccctacgc gttgggcggt ggctatgtcc tttctgcgga       660
cctggtgcat tacctgcgcc tcagccgcga gtacctgcgc gcgtggcaca gtgaagacgt       720
atcgctgggc acctggctgg caccagtgga tgtgcaacgg gagcacgacc cacgcttcga       780
cacggagtac aaatctcgag gctgcaacaa tcagtatctg gtgacacaca agcaaagccc       840
agaggacatg ttggagaagc aacagatgtt gctgcatgag ggccggttgt gcaagcatga       900
ggtgcagttg cgcctttcct atgtctatga ctggtcagct ccaccctccc agtgctgcca       960
gcgcaaggag ggcgttccct gaccgc                                            986
```

<210> 62
<211> 5146
<212> DNA
<213> Artificial sequence

<220>
<223> V2

<400> 62

```
tattgtaata cggttatcca cagaatcagg ggataacgca ggaaagaaca tgtgagcaaa        60
aggccagcaa aaggccagga accgtaaaaa ggccgcgttg ctggcgtttt tccataggct       120
```

ccgcccccct gacgagcatc acaaaaatcg acgctcaagt cagaggtggc gaaacccgac 180
aggactataa agataccagg cgtttccccc tggaagctcc ctcgtgcgct ctcctgttcc 240
gaccctgccg cttaccggat acctgtccgc ctttctccct tcgggaagcg tggcgctttc 300
tcatagctca cgctgtaggt atctcagttc ggtgtaggtc gttcgctcca agctgggctg 360
tgtgcacgaa ccccccgttc agcccgaccg ctgcgcctta tccggtaact atcgtcttga 420
gtccaacccg gtaagacacg acttatcgcc actggcagca gccactggta acaggattag 480
cagagcgagg tatgtaggcg gtgctacaga gttcttgaag tggtggccta actacggcta 540
cactagaaga acagtatttg gtatctgcgc tctgctgaag ccagttacct tcggaaaaag 600
agttggtagc tcttgatccg gcaaacaaac caccgctggt agcggttttt ttgtttgcaa 660
gcagcagatt acgcgcagaa aaaaaggatc tcaagaagat cctttgatct tttctacggg 720
gtctgacgct cagtggaacg aaaactcacg ttaagggatt ttggtcatga gattatcaaa 780
aaggatcttc acctagatcc ttttaaatta aaaatgaagt tttaaatcaa tctaaagtat 840
atatgagtaa acttggtctg acagttacca atgcttaatc agtgaggcac ctatctcagc 900
gatctgtcta tttcgttcat ccatagttgc ctgactcccc gtcgtgtaga taactacgat 960
acgggagggc ttaccatctg gccccagtgc tgcaatgata ccgcgtgacc cacgctcacc 1020
ggctccagat ttatcagcaa taaaccagcc agccggaagg gccgagcgca gaagtggtcc 1080
tgcaacttta tccgcctcca tccagtctat taattgttgc cgggaagcta gagtaagtag 1140
ttcgccagtt aatagtttgc gcaacgttgt tgccattgct acaggcatcg tggtgtcacg 1200
ctcgtcgttt ggtatggctt cattcagctc cggttcccaa cgatcaaggc gagttacatg 1260
atcccccatg ttgtgcaaaa aagcggttag ctccttcggt cctccgatcg ttgtcagaag 1320
taagttggcc gcagtgttat cactcatggt tatggcagca ctgcataatt ctcttactgt 1380
catgccatcc gtaagatgct tttctgtgac tggtgagtac tcaaccaagt cattctgaga 1440
atagtgtatg cggcgaccga gttgctcttg cccggcgtca atacgggata ataccgcgcc 1500
acatagcaga actttaaaag tgctcatcat tggaaaacgt tcttcggggc gaaaactctc 1560
aaggatctta ccgctgttga gatccagttc gatgtaaccc actcgtgcac ccaactgatc 1620
ttcagcatct tttactttca ccagcgtttc tgggtgagca aaaacaggaa ggcaaaatgc 1680
cgcaaaaaag ggaataaggg cgacacggaa atgttgaata ctcatactct tcctttttca 1740
atattattga agcatttatc agggttattg tctcatgagc ggatacatat ttgaatgtat 1800
ttagaaaaat aaacaaatag gggttccgcg cacatttccc cgaaaagtgc caaggaacca 1860
attcagtcga ctggatccta gttattaata gtaatcaatt acggggtcat tagttcatag 1920
cccatatatg gagttccgcg ttacataact tacggtaaat ggcccgcctg gctgaccgcc 1980
caacgacccc cgcccattga cgtcaataat gacgtatgtt cccatagtaa cgccaatagg 2040

gactttccat tgacgtcaat gggtggagta tttacggtaa actgcccact tggcagtaca 2100
tcaagtgtat catatgccaa gtacgccccc tattgacgtc aatgacggta aatggcccgc 2160
ctggcattat gcccagtaca tgaccttatg ggactttcct acttggcagt acatctacgt 2220
attagtcatc gctattacca tggtgatgcg gttttggcag tacatcaatg ggcgtggata 2280
gcggtttgac tcacggggat ttccaagtct ccaccccatt gacgtcaatg ggagtttgtt 2340
ttggcaccaa aatcaacggg actttccaaa atgtcgtaac aactccgccc cattgacgca 2400
aatgggcggt aggcgtgtac ggtgggaggt ctatataagc agagctggtt tagtgaaccg 2460
tcagatcact agtcgactag ggataacagg gcaccatgtc tagactggac aagagcaaag 2520
tcataaactc tgctctggaa ttactcaatg gagtcggtat cgaaggcctg acgacaagga 2580
aactcgctca aaagctggga gttgagcagc ctaccctgta ctggcacgtg aagaacaagc 2640
gggccctgct cgatgccctg ccaatcgaga tgctggacag gcatcatacc cactcctgcc 2700
ccctggaagg cgagtcatgg caagactttc tgcggaacaa cgccaagtca taccgctgtg 2760
ctctcctctc acatcgcgac ggggctaaag tgcatctcgg cacccgccca acagagaaac 2820
agtacgaaac cctggaaaat cagctcgcgt tcctgtgtca gcaaggcttc tccctggaga 2880
acgcactgta cgctctgtcc gccgtgggcc actttacact gggctgcgta ttggaggaac 2940
aggagcatca agtagcaaaa gaggaaagag agacacctac caccgattct atgcccccac 3000
ttctgaaaca agcaattgag ctgttcgacc ggcagggagc cgaacctgcc ttccttttcg 3060
gcctggaact aatcatatgt ggcctggaga aacagctaaa gtgcgaaagc ggcgggccga 3120
ccgacgccct tgacgatttt gacttagaca tgctcccagc cgatgccctt gacgactttg 3180
accttgatat gctgcctgct gacgctcttg acgattttga ccttgacatg ctccccgggt 3240
aatgatcgac tgtgccttct agttgccagc catctgttgt ttgcccctcc cccgtgcctt 3300
ccttgaccct ggaaggtgcc actcccactg tcctttccta ataaaatgag gaaattgcat 3360
cgcattgtct gagtaggtgt cattctattc tggggggtgg ggtggggcag gacagcaagg 3420
gggaggattg ggaggacaat agcaggcatg ctggggatgc ggtgggctct atggcttcgc 3480
tttcgtcttc aagaattcct ggagtttact ccctatcagt gatagagaac gtatgaagag 3540
tttactccct atcagtgata gagaacgtat gcagacttta ctccctatca gtgatagaga 3600
acgtataagg agtttactcc ctatcagtga tagagaacgt atgaccagtt tactccctat 3660
cagtgataga gaacgtatct acagtttact ccctatcagt gatagagaac gtatatccag 3720
tttactccct atcagtgata gagaacgtat aagctttagg cgtgtacggt gggcgcctat 3780
aaaagcagag ctcgtttagt gaaccgtcag atcgcctgga gcaattccac aacacttttg 3840
tcttatacca actttccgta ccacttccta ccctcgtaaa ccagagcatg aaggtattcc 3900

ggcgcgcttg gcggcaccgg gtggcgctgg gcctaggcgg cctggcgttc tgcggcacca 3960
ctctgttgta cctggcgcgc tgcgcttccg agggcgagac gccctccgct tccggagccg 4020
ctcggccccg cgctaaggcc ttcctggcgg tgctggtggc cagtgcgccc cgcgcggtcg 4080
agcgccgcac cgcagtgcgc agcacgtggc tggcaccgga gaggcgtggc ggacccgagg 4140
acgtgtgggc gcgcttcgcc gtgggcactg gcggcttagg ctcggaggag cggcgcgctc 4200
ttgagctcga gcaggcgcag cacggggacc tgctgctgct gcccgccctg cgcgacgcct 4260
acgagaacct cacggccaag gtcctggcca tgctgacctg gctggatgag cgcgtggact 4320
tcgagttcgt gctcaaggcg gacgacgact cctttgcgcg cctggacgct atcctggtgg 4380
acctacgcgc acgggagccc gcacgccgcc ggcgcctcta ctggggcttc ttttccgggc 4440
gcgggcgcgt caagccggga ggtcgctggc gagaagcagc ctggcaactc tgcgactact 4500
acctgcccta cgcgttgggc ggtggctatg tcctttctgc ggacctggtg cattacctgc 4560
gcctcagccg cgagtacctg cgcgcgtggc acagtgaaga cgtatcgctg ggcacctggc 4620
tggcaccagt ggatgtgcaa cgggagcacg acccacgctt cgacacggag tacaaatctc 4680
gaggctgcaa caatcagtat ctggtgacac acaagcaaag cccagaggac atgttggaga 4740
agcaacagat gttgctgcat gagggccggt tgtgcaagca tgaggtgcaa cttcgccttt 4800
cctatgtcta tgactggtca gctccaccct cccagtgctg ccagcgcaag gagggcgttc 4860
cctgatgtca ccgcgggggga ggctaactga aacacggaag gagacaatac cggaaggaac 4920
ccgcgctatg acggcaataa aaagacagaa taaaacgcac ggtgttgggt cgtttgttca 4980
taaacgcggg gttcggtccc agggctggca ctctgtcgat accccaccga ggccccattg 5040
gggccaatac gcccgcgttt cttcctttc cccaccccac cccccaagtt cgggtgaagg 5100
cccagggctc gcagccaacg tcggggcggc aggccctgcc atagcc 5146

<210> 63
<211> 1024
<212> DNA
<213> Artificial sequence

<220>
<223> mB3Galt6 BsaI B

<400> 63

gaggtaccgg tctcaccaga gcatgaaggt attccggcgc gcttggcggc accgggtggc 60
gctgggccta ggcggcctgg cgttctgcgg caccactctg ttgtacctgg cgcgctgcgc 120
ttccgagggc gagacgccct ccgcttccgg agccgctcgg ccccgcgcta aggccttcct 180
ggcggtgctg gtggccagtg cgccccgcgc ggtcgagcgc cgcaccgcag tgcgcagcac 240
gtggctggca ccggagaggc gtggcggacc cgaggacgtg tgggcgcgct tcgccgtggg 300
cactggcggc ttaggctcgg aggagcggcg cgctcttgag ctcgagcagg cgcagcacgg 360
ggacctgctg ctgctgcccg ccctgcgcga cgcctacgag aacctcacgg ccaaggtcct 420
ggccatgctg acctggctgg atgagcgcgt ggacttcgag ttcgtgctca aggcggacga 480
cgactccttt gcgcgcctgg acgctatcct ggtggaccta cgcgcacggg agcccgcacg 540
ccgccggcgc ctctactggg gcttcttttc cgggcgcggg cgcgtcaagc cgggaggtcg 600
ctggcgagaa gcagcctggc aactctgcga ctactacctg ccctacgcgt tgggcggtgg 660
ctatgtcctt tctgcggacc tggtgcatta cctgcgcctc agccgcgagt acctgcgcgc 720
gtggcacagt gaagacgtat cgctgggcac ctggctggca ccagtggatg tgcaacggga 780
gcacgaccca cgcttcgaca cggagtacaa atctcgaggc tgcaacaatc agtatctggt 840
gacacacaag caaagcccag aggacatgtt ggagaagcaa cagatgttgc tgcatgaggg 900
ccggttgtgc aagcatgagg tgcaacttcg cctttcctat gtctatgact ggtcagctcc 960
accctcccag tgctgccagc gcaaggaggg cgttccctga tgtcaccgcc gagaccggta 1020
cctc 1024

<210> 64
<211> 310
<212> DNA
<213> Artificial sequence

<220>
<223> Tkter BsaI B

<400> 64

gaggtaccgg tctcaccgcg ggggaggcta actgaaacac ggaaggagac aataccggaa 60
ggaacccgcg ctatgacggc aataaaaaga cagaataaaa cgcacggtgt tgggtcgttt 120
gttcataaac gcggggttcg gtcccagggc tggcactctg tcgatacccc accgaggccc 180
cattggggcc aatacgcccg cgtttcttcc ttttccccac cccacccccc aagttcgggt 240
gaaggcccag ggctcgcagc caacgtcggg gcggcaggcc ctgccatagc cacaacgaga 300
ccggtacctc 310

<210> 65

<211> 444
<212> DNA
<213> Artificial sequence

<220>
<223> shB3Galt6 BsaI C

<400> 65

```
gaggtaccgg tctcaacaaa cagggtcgac aagcttttcc aaaaaaaaag catgaggtgc        60
agttgcgcct ttcctatctc ttgaatagga aaggcgcaac tgcacctcat gctggatccc       120
gcgtcctttc cacaagatat ataaacccaa gaaatcgaaa tactttcaag ttacggtaag       180
catatgatag tccattttaa aacataattt taaaactgca aactacccaa gaaattatta       240
ctttctacgt cacgtatttt gtactaatat ctttgtgttt acagtcaaat taattctaat       300
tatctctcta acagccttgt atcgtatatg caaatatgaa ggaatcatgg gaaataggcc       360
ctcttcctgc ccgaccttgg cgcgcgctcg gcgcgcggtc acgctccgtc acgtggtgcg       420
ttttgtattc gagaccggta cctc                                              444
```

<210> 66
<211> 5556
<212> DNA
<213> Artificial sequence

<220>
<223> V3

<400> 66

```
tattgtaata cggttatcca cagaatcagg ggataacgca ggaaagaaca tgtgagcaaa      60
aggccagcaa aaggccagga accgtaaaaa ggccgcgttg ctggcgtttt tccataggct     120
ccgcccccct gacgagcatc acaaaaatcg acgctcaagt cagaggtggc gaaacccgac     180
aggactataa agataccagg cgtttccccc tggaagctcc ctcgtgcgct ctcctgttcc     240
gaccctgccg cttaccggat acctgtccgc ctttctccct tcgggaagcg tggcgctttc     300
tcatagctca cgctgtaggt atctcagttc ggtgtaggtc gttcgctcca agctgggctg     360
tgtgcacgaa ccccccgttc agcccgaccg ctgcgcctta tccggtaact atcgtcttga     420
gtccaacccg gtaagacacg acttatcgcc actggcagca gccactggta acaggattag     480
cagagcgagg tatgtaggcg gtgctacaga gttcttgaag tggtggccta actacggcta     540
cactagaaga acagtatttg gtatctgcgc tctgctgaag ccagttacct tcggaaaaag     600
agttggtagc tcttgatccg gcaaacaaac caccgctggt agcggttttt ttgtttgcaa     660
gcagcagatt acgcgcagaa aaaaaggatc tcaagaagat cctttgatct tttctacggg     720
gtctgacgct cagtggaacg aaaactcacg ttaagggatt ttggtcatga gattatcaaa     780
aaggatcttc acctagatcc ttttaaatta aaaatgaagt tttaaatcaa tctaaagtat     840
atatgagtaa acttggtctg acagttacca atgcttaatc agtgaggcac ctatctcagc     900
gatctgtcta tttcgttcat ccatagttgc ctgactcccc gtcgtgtaga taactacgat     960
acgggagggc ttaccatctg gccccagtgc tgcaatgata ccgcgtgacc cacgctcacc    1020
ggctccagat ttatcagcaa taaaccagcc agccggaagg gccgagcgca gaagtggtcc    1080
tgcaacttta tccgcctcca tccagtctat taattgttgc cgggaagcta gagtaagtag    1140
ttcgccagtt aatagtttgc gcaacgttgt tgccattgct acaggcatcg tggtgtcacg    1200
ctcgtcgttt ggtatggctt cattcagctc cggttcccaa cgatcaaggc gagttacatg    1260
atcccccatg ttgtgcaaaa aagcggttag ctccttcggt cctccgatcg ttgtcagaag    1320
```

taagttggcc gcagtgttat cactcatggt tatggcagca ctgcataatt ctcttactgt 1380

catgccatcc gtaagatgct tttctgtgac tggtgagtac tcaaccaagt cattctgaga 1440

atagtgtatg cggcgaccga gttgctcttg cccggcgtca atacgggata ataccgcgcc 1500

acatagcaga actttaaaag tgctcatcat tggaaaacgt tcttcggggc gaaaactctc 1560

aaggatctta ccgctgttga gatccagttc gatgtaaccc actcgtgcac ccaactgatc 1620

ttcagcatct tttactttca ccagcgtttc tgggtgagca aaaacaggaa ggcaaaatgc 1680

cgcaaaaaag ggaataaggg cgacacggaa atgttgaata ctcatactct tcctttttca 1740

atattattga agcatttatc agggttattg tctcatgagc ggatacatat ttgaatgtat 1800

ttagaaaaat aaacaaatag gggttccgcg cacatttccc cgaaaagtgc caaggaacca 1860

attcagtcga ctggatccta gttattaata gtaatcaatt acggggtcat tagttcatag 1920

cccatatatg gagttccgcg ttacataact tacggtaaat ggcccgcctg gctgaccgcc 1980

caacgacccc cgcccattga cgtcaataat gacgtatgtt cccatagtaa cgccaatagg 2040

gactttccat tgacgtcaat gggtggagta tttacggtaa actgcccact tggcagtaca 2100

tcaagtgtat catatgccaa gtacgccccc tattgacgtc aatgacggta aatggcccgc 2160

ctggcattat gcccagtaca tgaccttatg ggactttcct acttggcagt acatctacgt 2220

attagtcatc gctattacca tggtgatgcg gttttggcag tacatcaatg ggcgtggata 2280

gcggtttgac tcacggggat ttccaagtct ccaccccatt gacgtcaatg ggagtttgtt 2340

ttggcaccaa aatcaacggg actttccaaa atgtcgtaac aactccgccc cattgacgca 2400

aatgggcggt aggcgtgtac ggtgggaggt ctatataagc agagctggtt tagtgaaccg 2460

tcagatcact agtcgactag ggataacagg gcaccatgtc tagactggac aagagcaaag 2520

tcataaactc tgctctggaa ttactcaatg gagtcggtat cgaaggcctg acgacaagga 2580

aactcgctca aaagctggga gttgagcagc ctaccctgta ctggcacgtg aagaacaagc 2640

gggccctgct cgatgccctg ccaatcgaga tgctggacag gcatcatacc cactcctgcc 2700

ccctggaagg cgagtcatgg caagactttc tgcggaacaa cgccaagtca taccgctgtg 2760

ctctcctctc acatcgcgac ggggctaaag tgcatctcgg cacccgccca acagagaaac 2820

agtacgaaac cctggaaaat cagctcgcgt tcctgtgtca gcaaggcttc tccctggaga 2880

acgcactgta cgctctgtcc gccgtgggcc actttacact gggctgcgta ttggaggaac 2940

aggagcatca agtagcaaaa gaggaaagag agacacctac caccgattct atgcccccac 3000

ttctgaaaca agcaattgag ctgttcgacc ggcagggagc cgaacctgcc ttccttttcg 3060

gcctggaact aatcatatgt ggcctggaga aacagctaaa gtgcgaaagc ggcgggccga 3120

ccgacgccct tgacgatttt gacttagaca tgctcccagc cgatgccctt gacgactttg 3180

acettgatat gctgcctgct gacgctcttg acgattttga ccttgacatg ctccccgggt 3240

aatgatcgac tgtgccttct agttgccagc catctgttgt ttgcccctcc cccgtgcctt 3300

ccttgaccct ggaaggtgcc actcccactg tcctttccta ataaaatgag gaaattgcat 3360

cgcattgtct gagtaggtgt cattctattc tggggggtgg ggtggggcag gacagcaagg 3420

gggaggattg ggaggacaat agcaggcatg ctggggatgc ggtgggctct atggcttcgc 3480

tttcgtcttc aagaattcct ggagtttact ccctatcagt gatagagaac gtatgaagag 3540

tttactccct atcagtgata gagaacgtat gcagacttta ctccctatca gtgatagaga 3600

acgtataagg agtttactcc ctatcagtga tagagaacgt atgaccagtt tactccctat 3660

cagtgataga gaacgtatct acagtttact ccctatcagt gatagagaac gtatatccag 3720

tttactccct atcagtgata gagaacgtat aagctttagg cgtgtacggt gggcgcctat 3780

aaaagcagag ctcgtttagt gaaccgtcag atcgcctgga gcaattccac aacacttttg 3840

tcttatacca actttccgta ccacttccta ccctcgtaaa ccagagcatg aaggtattcc 3900

ggcgcgcttg gcggcaccgg gtggcgctgg gcctaggcgg cctggcgttc tgcggcacca 3960

ctctgttgta cctggcgcgc tgcgcttccg agggcgagac gccctccgct tccggagccg 4020

ctcggccccg cgctaaggcc ttcctggcgg tgctggtggc cagtgcgccc cgcgcggtcg 4080

agcgccgcac cgcagtgcgc agcacgtggc tggcaccgga gaggcgtggc ggacccgagg 4140

acgtgtgggc gcgcttcgcc gtgggcactg gcggcttagg ctcggaggag cggcgcgctc 4200

ttgagctcga gcaggcgcag cacggggacc tgctgctgct gcccgccctg cgcgacgcct 4260

acgagaacct cacggccaag gtcctggcca tgctgacctg gctggatgag cgcgtggact 4320

tcgagttcgt gctcaaggcg gacgacgact cctttgcgcg cctggacgct atcctggtgg 4380

acctacgcgc acgggagccc gcacgccgcc ggcgcctcta ctggggcttc ttttccgggc 4440

gcgggcgcgt caagccggga ggtcgctggc gagaagcagc ctggcaactc tgcgactact 4500

acctgcccta cgcgttgggc ggtggctatg tcctttctgc ggacctggtg cattacctgc 4560

gcctcagccg cgagtacctg cgcgcgtggc acagtgaaga cgtatcgctg ggcacctggc 4620

tggcaccagt ggatgtgcaa cgggagcacg acccacgctt cgacacggag tacaaatctc 4680

gaggctgcaa caatcagtat ctggtgacac acaagcaaag cccagaggac atgttggaga 4740

agcaacagat gttgctgcat gagggccggt tgtgcaagca tgaggtgcaa cttcgccttt 4800

cctatgtcta tgactggtca gctccaccct cccagtgctg ccagcgcaag gagggcgttc 4860

cctgatgtca ccgcgggggga ggctaactga aacacggaag gagacaatac cggaaggaac 4920

ccgcgctatg acggcaataa aaagacagaa taaaacgcac ggtgttgggt cgtttgttca 4980

taaacgcggg gttcggtccc agggctggca ctctgtcgat accccaccga ggccccattg 5040

gggccaatac gcccgcgttt cttccttttc cccaccccac cccccaagtt cgggtgaagg 5100

cccagggctc gcagccaacg tcggggcggc aggccctgcc atagccacaa acagggtcga 5160
caagcttttc caaaaaaaaa gcatgaggtg cagttgcgcc tttcctatct cttgaatagg 5220
aaaggcgcaa ctgcacctca tgctggatcc cgcgtccttt ccacaagata tataaaccca 5280
agaaatcgaa atactttcaa gttacggtaa gcatatgata gtccatttta aaacataatt 5340
ttaaaactgc aaactaccca agaaattatt actttctacg tcacgtattt tgtactaata 5400
tctttgtgtt tacagtcaaa ttaattctaa ttatctctct aacagccttg tatcgtatat 5460
gcaaatatga aggaatcatg ggaaataggc cctcttcctg cccgaccttg gcgcgcgctc 5520
ggcgcgcggt cacgctccgt cacgtggtgc gttttg 5556

<210> 67
<211> 34
<212> DNA
<213> Artificial sequence

<220>
<223> LoxP1

<220>
<221> misc_feature
<222> (14)..(16)
<223> n = a, t, c or g

<220>
<221> misc_feature
<222> (19)..(21)
<223> n = a, t, c or g

<400> 67
ataacttcgt atannntann ntatacgaag ttat 34

<210> 68
<211> 34
<212> DNA
<213> Artificial sequence

<220>
<223> FRT Flp-FRT

<400> 68
gaagttccta ttctctagaa agtataggaa cttc 34

<210> 69
<211> 50
<212> DNA
<213> Artificial sequence

<220>
<223> tagBFP-Ctag GGGG BsaI CW

<400> 69
gaggtaccgg tctccggggt cggggagcga gctgattaag gagaacatgc 50

<210> 70
<211> 43
<212> DNA
<213> Artificial sequence

<220>
<223> tagBFP-Ctag ATCA BsaI CCW

<400> 70
gaggtaccgg tctccatcat ccggaattaa gcttgtgccc cag 43

<210> 71
<211> 45
<212> DNA
<213> Artificial sequence

<220>
<223> ColE1-ori-2 TATT BsaI CW

<400> 71
gaggtaccgg tctcgtattg taatacggtt atccacagaa tcagg 45

<210> 72
<211> 42
<212> DNA
<213> Artificial sequence

<220>
<223> AmpR-2 TCCT BsaI CCW

<400> 72
gaggtaccgg tctcgtcctt ggcacttttc ggggaaatgt gc 42

<210> 73
<211> 37
<212> DNA
<213> Artificial sequence

<220>
<223> CMVp AGGA BsaI CW

<400> 73
gaggtaccgg tctcaaggaa ccaattcagt cgactgg 37

<210> 74
<211> 42
<212> DNA
<213> Artificial sequence

<220>
<223> CMVp GGTG BsaI CCW

<400> 74
gaggtaccgg tctcgggtgc cctgttatcc ctagtcgact ag 42

<210> 75
<211> 39
<212> DNA

<213> Artificial sequence

<220>
<223> hFUT3 CACC BsaI CW

<400> 75
gaggtaccgg tctcacacca tggatcccct gggtgcagc 39

<210> 76
<211> 41
<212> DNA
<213> Artificial sequence

<220>
<223> hFUT3 ATCA BsaI CCW

<400> 76
gaggtaccgg tctcgatcag gtgaaccaag ccgctatgct g 41

<210> 77
<211> 36
<212> DNA
<213> Artificial sequence

<220>
<223> BGH polyA CGAA BsaI CCW

<400> 77
gaggtaccgg tctcgcgaag ccatagagcc caccgc 36

<210> 78
<211> 40
<212> DNA
<213> Artificial sequence

<220>
<223> shB3Galt6 TTCG BsaI CW

<400> 78
gaggtaccgg tctcattcga cagggtcgac aagcttttcc 40

<210> 79
<211> 38
<212> DNA
<213> Artificial sequence

<220>
<223> shB3Galt6 AATA BsaI CCW

<400> 79
gaggtaccgg tctcgaatac aaaacgcacc acgtgacg 38

<210> 80
<211> 38
<212> DNA
<213> Artificial sequence

<220>

<223> shB3Galt6 CTGG BsaI CCW

<400> 80
gaggtaccgg tctcgctggc aaaacgcacc acgtgacg 38

<210> 81
<211> 39
<212> DNA
<213> Artificial sequence

<220>
<223> Hygro CCAG BsaI CW

<400> 81
gaggtaccgg tctcaccagc aggcagaagt atgcaaagc 39

<210> 82
<211> 47
<212> DNA
<213> Artificial sequence

<220>
<223> Hygro AATA BsaI CCW

<400> 82
gaggtaccgg tctcgaatag atacattgat gagtttggac aaaccac 47

<210> 83
<211> 37
<212> DNA
<213> Artificial sequence

<220>
<223> rosa26-5' AGGA BsaI CW

<400> 83
gaggtaccgg tctcaaggac cccgcggcag gccctcc 37

<210> 84
<211> 43
<212> DNA
<213> Artificial sequence

<220>
<223> rosa26-5' TTGT BsaI CCW

<400> 84
gaggtaccgg tctcgttgta agactggagt tgcagatcac gag 43

<210> 85
<211> 37
<212> DNA
<213> Artificial sequence

<220>
<223> CMVp ACAA BsaI CW

<400> 85

gaggtaccgg tctcaacaaa ccaattcagt cgactgg 37

<210> 86
<211> 47
<212> DNA
<213> Artificial sequence

<220>
<223> Hygro CTAC BsaI CCW

<400> 86
gaggtaccgg tctcgctacg atacattgat gagtttggac aaaccac 47

<210> 87
<211> 39
<212> DNA
<213> Artificial sequence

<220>
<223> rosa26-3' GTAG BsaI CW

<400> 87
gaggtaccgg tctcagtaga gatgggcggg agtcttctg 39

<210> 88
<211> 49
<212> DNA
<213> Artificial sequence

<220>
<223> rosa26-3' AATA BsaI CCW

<400> 88
gaggtaccgg tctcgaatag ataagctaga tgtcctaaat atttctatc 49

<210> 89
<211> 49
<212> DNA
<213> Artificial sequence

<220>
<223> rosa26-3' GCAT BsaI CCW

<400> 89
gaggtaccgg tctcggcatg ataagctaga tgtcctaaat atttctatc 49

<210> 90
<211> 45
<212> DNA
<213> Artificial sequence

<220>
<223> EF1a ATGC BsaI CW

<400> 90
gaggtaccgg tctcaatgca aggaaccaat tcagtcgact ggatc 45

<210> 91

<211> 42
<212> DNA
<213> Artificial sequence

<220>
<223> EF1a GGGC BsaI CCW

<400> 91
gaggtaccgg tctcggggcc cctgttatcc ctagtcgact ag 42

<210> 92
<211> 37
<212> DNA
<213> Artificial sequence

<220>
<223> TK GCCC BsaI CW

<400> 92
gaggtaccgg tctcagccca tggcttcgta cccctgc 37

<210> 93
<211> 41
<212> DNA
<213> Artificial sequence

<220>
<223> TK GCGG BsaI CCW

<400> 93
gaggtaccgg tctcggcggt cagttagcct cccccatctc c 41

<210> 94
<211> 40
<212> DNA
<213> Artificial sequence

<220>
<223> HSVTKterm CCGC BsaI CW

<400> 94
gaggtaccgg tctcaccgcg ggggaggcta actgaaacac 40

<210> 95
<211> 37
<212> DNA
<213> Artificial sequence

<220>
<223> HSVTKterm AATA BsaI CCW

<400> 95
gaggtaccgg tctcgaatag gctatggcag ggcctgc 37

<210> 96
<211> 44
<212> DNA
<213> Artificial sequence

<220>
<223> TetOn3G CACC BsaI CW

<400> 96
gaggtaccgg tctcacacca tgtctagact ggacaagagc aaag 44

<210> 97
<211> 40
<212> DNA
<213> Artificial sequence

<220>
<223> TetOn3G ATCA BsaI CCW

<400> 97
gaggtaccgg tctcaatcat tacccgggga gcatgtcaag 40

<210> 98
<211> 46
<212> DNA
<213> Artificial sequence

<220>
<223> pTet3G TTCG BsaI CW

<400> 98
gaggtaccgg tctcattcgt cttcaagaat tcctcgagtt tactcc 46

<210> 99
<211> 43
<212> DNA
<213> Artificial sequence

<220>
<223> pTet3G CTGG BsaI CCW

<400> 99
gaggtaccgg tctcgctggt ttacgagggt aggaagtggt acg 43

<210> 100
<211> 45
<212> DNA
<213> Artificial sequence

<220>
<223> mB3Galt6 CCAG BsaI CW

<400> 100
gaggtaccgg tctcaccaga gcatgaaggt attccggcgc gcttg 45

<210> 101
<211> 43
<212> DNA
<213> Artificial sequence

<220>
<223> mB3Galt6 GCGG BsaI CCW

<400> 101
gaggtaccgg tctcggcggt gacatcaggg aacgccctcc ttg 43

<210> 102
<211> 37
<212> DNA
<213> Artificial sequence

<220>
<223> HSVTKterm TTGT BsaI CCW

<400> 102
gaggtaccgg tctcgttgtg gctatggcag ggcctgc 37

<210> 103
<211> 40
<212> DNA
<213> Artificial sequence

<220>
<223> shB3Galt6 ACAA BsaI CW

<400> 103
gaggtaccgg tctcaacaaa cagggtcgac aagcttttcc 40

<210> 104
<211> 882
<212> DNA
<213> Artificial sequence

<220>
<223> Ori BsaI A

<400> 104

```
gaggtaccgg tctcatattg taatacggtt atccacagaa tcaggggata acgcaggaaa      60
gaacatgtga gcaaaaggcc agcaaaaggc caggaaccgt aaaaaggccg cgttgctggc     120
gtttttccat aggctccgcc cccctgacga gcatcacaaa aatcgacgct caagtcagag     180
gtggcgaaac ccgacaggac tataaagata ccaggcgttt ccccctggaa gctccctcgt     240
gcgctctcct gttccgaccc tgccgcttac cggatacctg tccgcctttc tcccttcggg     300
aagcgtggcg ctttctcata gctcacgctg taggtatctc agttcggtgt aggtcgttcg     360
ctccaagctg ggctgtgtgc acgaaccccc cgttcagccc gaccgctgcg ccttatccgg     420
taactatcgt cttgagtcca acccggtaag acacgactta tcgccactgg cagcagccac     480
tggtaacagg attagcagag cgaggtatgt aggcggtgct acagagttct tgaagtggtg     540
gcctaactac ggctacacta gaagaacagt atttggtatc tgcgctctgc tgaagccagt     600
taccttcgga aaaagagttg gtagctcttg atccggcaaa caaaccaccg ctggtagcgg     660
```

```
tttttttgtt tgcaagcagc agattacgcg cagaaaaaaa ggatctcaag aagatccttt      720
gatcttttct acggggtctg acgctcagtg gaacgaaaac tcacgttaag ggattttggt      780
catgagatta tcaaaaagga tcttcaccta gatcctttta aattaaaaat gaagttttaa      840
atcaatctaa agtatatatg agtttatga gaccggtacc tc                          882
```

<210> 105
<211> 1038
<212> DNA
<213> Artificial sequence

<220>
<223> AmpR BsaI A

<400> 105

```
gaggtaccgg tctcttttat tggtctgaca gttaccaatg cttaatcagt gaggcaccta       60
tctcagcgat ctgtctattt cgttcatcca tagttgcctg actccccgtc gtgtagataa      120
ctacgatacg ggagggctta ccatctggcc ccagtgctgc aatgataccg cgtgacccac      180
gctcaccggc tccagattta tcagcaataa accagccagc cggaagggcc gagcgcagaa      240
gtggtcctgc aactttatcc gcctccatcc agtctattaa ttgttgccgg gaagctagag      300
taagtagttc gccagttaat agtttgcgca acgttgttgc cattgctaca ggcatcgtgg      360
tgtcacgctc gtcgtttggt atggcttcat tcagctccgg ttcccaacga tcaaggcgag      420
ttacatgatc ccccatgttg tgcaaaaaag cggttagctc cttcggtcct ccgatcgttg      480
tcagaagtaa gttggccgca gtgttatcac tcatggttat ggcagcactg cataattctc      540
ttactgtcat gccatccgta agatgctttt ctgtgactgg tgagtactca accaagtcat      600
tctgagaata gtgtatgcgg cgaccgagtt gctcttgccc ggcgtcaata cgggataata      660
ccgcgccaca tagcagaact ttaaaagtgc tcatcattgg aaaacgttct tcggggcgaa      720
aactctcaag gatcttaccg ctgttgagat ccagttcgat gtaacccact cgtgcaccca      780
actgatcttc agcatctttt actttcacca gcgtttctgg gtgagcaaaa acaggaaggc      840
aaaatgccgc aaaaaaggga ataagggcga cacggaaatg ttgaatactc atactcttcc      900
tttttcaata ttattgaagc atttatcagg gttattgtct catgagcgga tacatatttg      960
aatgtattta gaaaaataaa caaatagggg ttccgcgcac atttccccga aaagtgccaa     1020
ggacgagacc ggtacctc                                                   1038
```

<210> 106
<211> 884
<212> DNA
<213> Artificial sequence

<220>

<223> Ori BsaI B

<400> 106

```
gaggtaccgg tctctattac ggtaatacgg ttatccacag aatcagggga taacgcagga      60
aagaacatgt gagcaaaagg ccagcaaaag gccaggaacc gtaaaaaggc cgcgttgctg     120
gcgtttttcc ataggctccg cccccctgac gagcatcaca aaaatcgacg ctcaagtcag     180
aggtggcgaa acccgacagg actataaaga taccaggcgt ttccccctgg aagctccctc     240
gtgcgctctc ctgttccgac cctgccgctt accggatacc tgtccgcctt tctcccttcg     300
ggaagcgtgg cgctttctca tagctcacgc tgtaggtatc tcagttcggt gtaggtcgtt     360
cgctccaagc tgggctgtgt gcacgaaccc cccgttcagc ccgaccgctg cgccttatcc     420
ggtaactatc gtcttgagtc caacccggta agacacgact tatcgccact ggcagcagcc     480
actggtaaca ggattagcag agcgaggtat gtaggcggtg ctacagagtt cttgaagtgg     540
tggcctaact acggctacac tagaagaaca gtatttggta tctgcgctct gctgaagcca     600
gttaccttcg gaaaaagagt tggtagctct tgatccggca aacaaaccac cgctggtagc     660
ggtttttttg tttgcaagca gcagattacg cgcagaaaaa aaggatctca agaagatcct     720
ttgatctttt ctacggggtc tgacgctcag tggaacgaaa actcacgtta agggattttg     780
gtcatgagat tatcaaaaag gatcttcacc tagatccttt taaattaaaa atgaagtttt     840
aaatcaatct aaagtatata tgagtaaacc gagaccggta cctc                      884
```

<210> 107
<211> 1038
<212> DNA
<213> Artificial sequence

<220>
<223> AmpR BsaI B

<400> 107

gaggtaccgg tctcaaaact tggtctgaca gttaccaatg cttaatcagt gaggcaccta 60

tctcagcgat ctgtctattt cgttcatcca tagttgcctg actccccgtc gtgtagataa 120

ctacgatacg ggagggctta ccatctggcc ccagtgctgc aatgataccg cgtgacccac 180

gctcaccggc tccagattta tcagcaataa accagccagc cggaagggcc gagcgcagaa 240

gtggtcctgc aactttatcc gcctccatcc agtctattaa ttgttgccgg gaagctagag 300

taagtagttc gccagttaat agtttgcgca acgttgttgc cattgctaca ggcatcgtgg 360

tgtcacgctc gtcgtttggt atggcttcat tcagctccgg ttcccaacga tcaaggcgag 420

ttacatgatc ccccatgttg tgcaaaaaag cggttagctc cttcggtcct ccgatcgttg 480

tcagaagtaa gttggccgca gtgttatcac tcatggttat ggcagcactg cataattctc 540

ttactgtcat gccatccgta agatgctttt ctgtgactgg tgagtactca accaagtcat 600

tctgagaata gtgtatgcgg cgaccgagtt gctcttgccc ggcgtcaata cgggataata 660

ccgcgccaca tagcagaact ttaaaagtgc tcatcattgg aaaacgttct tcggggcgaa 720

aactctcaag gatcttaccg ctgttgagat ccagttcgat gtaacccact cgtgcaccca 780

actgatcttc agcatctttt actttcacca gcgtttctgg gtgagcaaaa acaggaaggc 840

aaaatgccgc aaaaaaggga ataagggcga cacggaaatg ttgaatactc atactcttcc 900

tttttcaata ttattgaagc atttatcagg gttattgtct catgagcgga tacatatttg 960

aatgtattta gaaaaataaa caaatagggg ttccgcgcac atttccccga aaagtgccaa 1020

tgctgagacc ggtacctc 1038

<210> 108
<211> 1221
<212> DNA
<213> Artificial sequence

<220>
<223> pEF1aL BsaI B

<400> 108

gaggtaccgg tctcaatgcg tgaggctccg gtgcccgtca gtgggcagag cgcacatcgc 60
ccacagtccc cgagaagttg gggggagggg tcggcaattg aaccggtgcc tagagaaggt 120
ggcgcggggt aaactgggaa agtgatgtcg tgtactggct ccgccttttt cccgagggtg 180
ggggagaacc gtatataagt gcagtagtcg ccgtgaacgt tctttttcgc aacgggtttg 240
ccgccagaac acaggtaagt gccgtgtgtg gttcccgcgg gcctggcctc tttacgggtt 300
atggcccttg cgtgccttga attacttcca cctggctgca gtacgtgatt cttgatcccg 360
agcttcgggt tggaagtggg tgggagagtt cgaggccttg cgcttaagga gccccttcgc 420
ctcgtgcttg agttgaggcc tggcctgggc gctggggccg ccgcgtgcga atctggtggc 480
accttcgcgc ctgtctcgct gctttcgata agtctctagc catttaaaat ttttgatgac 540
ctgctgcgac gctttttttc tggcaagata gtcttgtaaa tgcgggccaa gatctgcaca 600
ctggtatttc ggtttttggg gccgcgggcg gcgacggggc ccgtgcgtcc cagcgcacat 660
gttcggcgag gcggggcctg cgagcgcggc caccgagaat cggacggggg tagtctcaag 720
ctggccggcc tgctctggtg cctggcctcg cgccgccgtg tatcgccccg ccctgggcgg 780
caaggctggc ccggtcggca ccagttgcgt gagcggaaag atggccgctt cccggccctg 840
ctgcagggag ctcaaaatgg aggacgcggc gctcgggaga gcgggcgggt gagtcaccca 900
cacaaaggaa aagggccttt ccgtcctcag ccgtcgcttc atgtgactcc acggagtacc 960
gggcgccgtc caggcacctc gattagttct cgagcttttg gagtacgtcg tctttaggtt 1020
ggggggaggg gttttatgcg atggagtttc cccacactga gtgggtggag actgaagtta 1080
ggccagcttg gcacttgatg taattctcct tggaatttgc cctttttgag tttggatctt 1140
ggttcattct caagcctcag acagtggttc aaagtttttt tcttccattt caggtgtcgt 1200

gatatccgag accggtacct c 1221

<210> 109
<211> 797
<212> DNA
<213> Artificial sequence

<220>
<223> EGFP-CAAX BsaI A

<400> 109

gaggtaccgg tctcctatca tggtgagcaa gggcgaggag ctgttcaccg gggtggtgcc 60
catcctggtc gagctggacg gcgacgtaaa cggccacaag ttcagcgtgt ccggcgaggg 120
cgagggcgat gccacctacg gcaagctgac cctgaagttc atctgcacca ccggcaagct 180
gcccgtgccc tggcccaccc tcgtgaccac cctgacctac ggcgtgcagt gcttcagccg 240
ctaccccgac cacatgaagc agcacgactt cttcaagtcc gccatgcccg aaggctacgt 300
ccaggagcgc accatcttct tcaaggacga cggcaactac aagacccgcg ccgaggtgaa 360
gttcgagggc gacaccctgg tgaaccgcat cgagctgaag ggcatcgact tcaaggagga 420
cggcaacatc ctggggcaca agctggagta caactacaac agccacaacg tctatatcat 480
ggccgacaag cagaagaacg gcatcaaggt gaacttcaag atccgccaca acatcgagga 540
cggcagcgtg cagctcgccg accactacca gcagaacacc cccatcggcg acggccccgt 600
gctgctgccc gacaaccact acctgagcac ccagtccgcc ctgagcaaag accccaacga 660
gaagcgcgat cacatggtcc tgctggagtt cgtgaccgcc gccgggatca ctctcggcat 720
ggacgagctg tacaagaaga agaaaaagaa gtcaaagaca aagtgtgtaa ttatgtaaga 780
gtggagaccg gtacctc 797

<210> 110
<211> 266
<212> DNA
<213> Artificial sequence

<220>
<223> BGHpA BsaI C

<400> 110

gaggtaccgg tctcagagtc gactgtgcct tctagttgcc agccatctgt tgtttgcccc 60
tcccccgtgc cttccttgac cctggaaggt gccactccca ctgtcctttc ctaataaaat 120
gaggaaattg catcgcattg tctgagtagg tgtcattcta ttctgggggg tggggtgggg 180
caggacagca agggggagga ttgggaggac aatagcaggc atgctgggga tgcggtgggc 240
tctatgggta gcgagaccgg tacctc 266

<210> 111
<211> 624
<212> DNA
<213> Artificial sequence

<220>
<223> pCMV BsaI D

<400> 111

gaggtaccgg tctcagtagt tattaatagt aatcaattac ggggtcatta gttcatagcc 60
catatatgga gttccgcgtt acataactta cggtaaatgg cccgcctggc tgaccgccca 120
acgacccccg cccattgacg tcaataatga cgtatgttcc catagtaacg ccaataggga 180
ctttccattg acgtcaatgg gtggagtatt tacggtaaac tgcccacttg gcagtacatc 240
aagtgtatca tatgccaagt acgcccccta ttgacgtcaa tgacggtaaa tggcccgcct 300
ggcattatgc ccagtacatg accttatggg actttcctac ttggcagtac atctacgtat 360
tagtcatcgc tattaccatg gtgatgcggt tttggcagta catcaatggg cgtggatagc 420
ggtttgactc acggggattt ccaagtctcc accccattga cgtcaatggg agtttgtttt 480
ggcaccaaaa tcaacgggac tttccaaaat gtcgtaacaa ctccgcccca ttgacgcaaa 540
tgggcggtag gcgtgtacgg tgggaggtct atataagcag agctggttta gtgaaccgtc 600
agatcttcgc gagaccggta cctc 624

<210> 112
<211> 380
<212> DNA
<213> Artificial sequence

<220>
<223> SiaT BsaI B

<400> 112

gaggtaccgg tctccttcga tgattcacac caacctgaag aaaaagttca gctgctgcgt 60
cctggtcttt cttctgtttg cagtcatctg tgtgtggaag gaaaagaaga aagggagtta 120
ctatgattcc tttaaattgc aaaccaagga attccaggtg ttaaagagtc tggggaaatt 180
ggccatgggg tctgattccc agtctgtatc ctcaagcagc acccaggacc cccacagggg 240
ccgccagacc ctcggcagtc tcagaggcct agccaaggcc aaaccagagg cctccttcca 300
ggtgtggaac aaggacagct cttccaaaaa ccttatccct aggctgcaaa aggggtcggg 360
ggtgatgaga ccggtacctc 380

<210> 113
<211> 742
<212> DNA
<213> Artificial sequence

<220>
<223> mCherry BsaI B

<400> 113

```
gaggtaccgg tctcagtgag caagggcgag gaggataaca tggccatcat caaggagttc      60
atgcgcttca aggtgcacat ggagggctcc gtgaacggcc acgagttcga gatcgagggc     120
gagggcgagg gccgccccta cgagggcacc cagaccgcca agctgaaggt gaccaagggt     180
ggcccccctgc ccttcgcctg ggacatcctg tcccctcagt tcatgtacgg ctccaaggcc    240
tacgtgaagc accccgccga catccccgac tacttgaagc tgtccttccc cgagggcttc     300
aagtgggagc gcgtgatgaa cttcgaggac ggcggcgtgg tgaccgtgac ccaggactcc     360
tccctgcagg acggcgagtt catctacaag gtgaagctgc gcggcaccaa cttcccctcc     420
gacggccccg taatgcagaa gaaaaccatg ggctgggagg cctcctccga gcggatgtac     480
cccgaggacg gcgccctgaa gggcgagatc aagcagaggc tgaagctgaa ggacggcggc     540
cactacgacg ctgaggtcaa gaccacctac aaggccaaga agcccgtgca gctgcccggc     600
gcctacaacg tcaacatcaa gttggacatc acctcccaca acgaggacta caccatcgtg     660
gaacagtacg aacgcgccga gggccgccac tccaccggcg gcatggacga gctgtacaag     720
taaccgctga gaccggtacc tc                                               742
```

<210> 114
<211> 1650
<212> DNA
<213> Artificial sequence

<220>
<223> HygroR BsaI D

<400> 114

gaggtaccgg tctcaacaac aggcagaagt atgcaaagca tgcatctcaa ttagtcagca 60
accaggtgtg gaaagtcccc aggctcccca gcaggcagaa gtatgcaaag catgcatctc 120
aattagtcag caaccatagt cccgccccta actccgccca tcccgcccct aactccgccc 180
agttccgccc attctccgcc ccatggctga ctaatttttt ttatttatgc agaggccgag 240
gccgcctctg cctctgagct attccagaag tagtgaggag gcttttttgg aggcctaggc 300
ttttgcaaaa agctcccggg agcttgtata tccattttcg gatctgatca gcacgtgatg 360
aaaaagcctg aactcaccgc gacgtctgtc gagaagtttc tgatcgaaaa gttcgacagc 420
gtgtccgacc tgatgcagct ctcggagggc gaagaatctc gtgctttcag cttcgatgta 480
ggagggcgtg gatatgtcct gcgggtaaat agctgcgccg atggtttcta caaagatcgt 540
tatgtttatc ggcactttgc atcggccgcg ctcccgattc cggaagtgct tgacattggg 600
gaattcagcg agagcctgac ctattgcatc tcccgccgtg cacagggtgt cacgttgcaa 660
gacttgcctg aaaccgaact gcccgctgtt ctgcagccgg tcgcggaggc catggatgcg 720
atcgctgcgg ccgatcttag ccagacgagc gggttcggcc cattcggacc gcaaggaatc 780

ggtcaataca ctacatggcg tgatttcata tgcgcgattg ctgatcccca tgtgtatcac 840
tggcaaactg tgatggacga caccgtcagt gcgtccgtcg cgcaggctct cgatgagctg 900
atgctttggg ccgaggactg ccccgaagtc cggcacctcg tgcacgcgga tttcggctcc 960
aacaatgtcc tgacggacaa tggccgcata acagcggtca ttgactggag cgaggcgatg 1020
ttcggggatt cccaatacga ggtcgccaac atcttcttct ggaggccgtg gttggcttgt 1080
atggagcagc agacgcgcta cttcgagcgg aggcatccgg agcttgcagg atcgccgcgg 1140
ctccgggcgt atatgctccg cattggtctt gaccaactct atcagagctt ggttgacggc 1200
aatttcgatg atgcagcttg ggcgcagggt cgatgcgacg caatcgtccg atccggagcc 1260
gggactgtcg ggcgtacaca aatcgcccgc agaagcgcgg ccgtctggac cgatggctgt 1320
gtagaagtac tcgccgatag tggaaaccga cgccccagca ctcgtccgag ggcaaaggaa 1380
tagcacgtgc tacgagattt cgattccacc gccgccttct atgaaaggtt gggcttcgga 1440
atcgttttcc gggacgccgg ctggatgatc ctccagcgcg gggatctcat gctggagttc 1500
ttcgcccacc ccaacttgtt tattgcagct tataatggtt acaaataaag caatagcatc 1560
acaaatttca caaataaagc atttttttca ctgcattcta gttgtggttt gtccaaactc 1620
atcaatgtat cattacgaga ccggtacctc 1650

<210> 115
<211> 886
<212> DNA
<213> Artificial sequence

<220>
<223> Ori-2 BsaI C

<400> 115

```
gaggtaccgg tctctggggc ggtaatacgg ttatccacag aatcagggga taacgcagga      60
aagaacatgt gagcaaaagg ccagcaaaag gccaggaacc gtaaaaaggc cgcgttgctg     120
gcgtttttcc ataggctccg cccccctgac gagcatcaca aaaatcgacg ctcaagtcag     180
aggtggcgaa acccgacagg actataaaga taccaggcgt ttccccctgg aagctccctc     240
gtgcgctctc ctgttccgac cctgccgctt accggatacc tgtccgcctt tctcccttcg     300
ggaagcgtgg cgctttctca tagctcacgc tgtaggtatc tcagttcggt gtaggtcgtt     360
cgctccaagc tgggctgtgt gcacgaaccc cccgttcagc ccgaccgctg cgccttatcc     420
ggtaactatc gtcttgagtc caacccggta agacacgact tatcgccact ggcagcagcc     480
actggtaaca ggattagcag agcgaggtat gtaggcggtg ctacagagtt cttgaagtgg     540
tggcctaact acggctacac tagaagaaca gtatttggta tctgcgctct gctgaagcca     600
gttaccttcg gaaaaagagt tggtagctct tgatccggca aacaaaccac cgctggtagc     660
ggtttttttg tttgcaagca gcagattacg cgcagaaaaa aaggatctca agaagatcct     720
ttgatctttt ctacggggtc tgacgctcag tggaacgaaa actcacgtta agggattttg     780
gtcatgagat tatcaaaaag gatcttcacc tagatccttt taaattaaaa atgaagtttt     840
aaatcaatct aaagtatata tgagtttaaa ctgagaccgg tacctc                    886
```

<210> 116
<211> 1038
<212> DNA
<213> Artificial sequence

<220>
<223> AmpR BsaI C

<400> 116

```
gaggtaccgg tctcaaaact tggtctgaca gttaccaatg cttaatcagt gaggcaccta      60
tctcagcgat ctgtctattt cgttcatcca tagttgcctg actccccgtc gtgtagataa     120
ctacgatacg ggagggctta ccatctggcc ccagtgctgc aatgataccg cgtgacccac     180
gctcaccggc tccagattta tcagcaataa accagccagc cggaagggcc gagcgcagaa     240
gtggtcctgc aactttatcc gcctccatcc agtctattaa ttgttgccgg gaagctagag     300
taagtagttc gccagttaat agtttgcgca acgttgttgc cattgctaca ggcatcgtgg     360
tgtcacgctc gtcgtttggt atggcttcat tcagctccgg ttcccaacga tcaaggcgag     420
ttacatgatc ccccatgttg tgcaaaaaag cggttagctc cttcggtcct ccgatcgttg     480
tcagaagtaa gttggccgca gtgttatcac tcatggttat ggcagcactg cataattctc     540
ttactgtcat gccatccgta agatgctttt ctgtgactgg tgagtactca accaagtcat     600
tctgagaata gtgtatgcgg cgaccgagtt gctcttgccc ggcgtcaata cgggataata     660
ccgcgccaca tagcagaact ttaaaagtgc tcatcattgg aaaacgttct tcggggcgaa     720
aactctcaag gatcttaccg ctgttgagat ccagttcgat gtaacccact cgtgcaccca     780
actgatcttc agcatctttt actttcacca gcgtttctgg gtgagcaaaa acaggaaggc     840
aaaatgccgc aaaaaaggga ataagggcga cacggaaatg ttgaatactc atactcttcc     900
tttttcaata ttattgaagc atttatcagg gttattgtct catgagcgga tacatatttg     960
aatgtattta gaaaaataaa caaatagggg ttccgcgcac atttccccga aaagtgccag    1020
tgatgagacc ggtacctc                                                  1038
```

<210> 117
<211> 312
<212> DNA
<213> Artificial sequence

<220>
<223> MNN10-Lrec BsaI A

<400> 117

```
gaggtaccgg tctctgtgag tttaaacatg cattcaaagg tcataattgc tgctctattt      60
acagtcgtcc ataatgacat ttctctttga ttattttctt gtttttttcgc tcttctcaag   120
tggatgttac ataacaaaca aaacagaaaa aattgtttaa atataaagtt taaaagttat     180
ctttgattcc gcacctgaat ttttggattg aaggccaaag gaggtttatc agggagagaa     240
aagctctcta tttattttta taaggaataa ttgtgcatgt acaactatac aattgcgtga     300
gaccggtacc tc                                                         312
```

<210> 118
<211> 650

<212> DNA
<213> Artificial sequence

<220>
<223> MNN10-Rrec BsaI A

<400> 118

```
gaggtaccgg tctcttcgta atggaagtta tcaatattgt aaagagaagc atttacaagc       60
ttttattttt ctttttaatt tccactactg gttctgcttt aaaatgttgt tttataattt      120
atgtacattt aggcctatag aagattcttt caataatatg ctacacattc ttttattttt      180
ccatcatatg ttggagttta tgcctcctcg gcaggagttg ggcggtgcga agagaagaaa      240
aagagtgaaa ctaaaaaaag gaatctgcct ttgcataagt tcaaaagtgc aattttagtg      300
ttggatttaa acgggaaaaa ttgaaatggc catcgaaaca atacttgtaa taaacaaatc      360
aggcggacta atctatcagc ggaattttac caacgacgaa cagaaattga acagcaatga      420
atacttaatt cttgctagta cactgcacgg tgtattcgcc atcgcgagcc agctgactcc      480
gaaggcatta cagctaactc aacaaacgaa catcgaaaat accatcccat atataccta       540
cgtgggcatg tccagcaata ggagcgatac aagaaatgga ggtggcaata acaacaaaca      600
cactaataat gaaaaactgg gcagtttaaa cggggagaga ccggtacctc                 650
```

<210> 119
<211> 1630
<212> DNA
<213> Artificial sequence

<220>
<223> KanMX BsaI A

<400> 119

```
gaggtaccgg tctcgtgcgg tacgctgcag gtcgacaacc cttaatataa cttcgtataa       60
tgtatgctat acgaagttat taggtctaga gatctgttta gcttgcctcg tccccgccgg      120
gtcacccggc cagcgacatg gaggcccaga ataccctcct tgacagtctt gacgtgcgca      180
gctcaggggc atgatgtgac tgtcgcccgt acatttagcc catacatccc catgtataat      240
catttgcatc catacatttt gatggccgca cggcgcgaag caaaaattac ggctcctcgc      300
```

tgcagacctg cgagcaggga aacgctcccc tcacagacgc gttgaattgt ccccacgccg 360
cgcccctgta gagaaatata aaaggttagg atttgccact gaggttcttc tttcatatac 420
ttccttttaa aatcttgcta ggatacagtt ctcacatcac atccgaacat aaacaaccat 480
gggtaaggaa aagactcacg tttcgaggcc gcgattaaat tccaacatgg atgctgattt 540
atatgggtat aaatgggctc gcgataatgt cgggcaatca ggtgcgacaa tctatcgatt 600
gtatgggaag cccgatgcgc cagagttgtt tctgaaacat ggcaaaggta gcgttgccaa 660
tgatgttaca gatgagatgg tcagactaaa ctggctgacg gaatttatgc ctcttccgac 720
catcaagcat tttatccgta ctcctgatga tgcatggtta ctcaccactg cgatccccgg 780
caaaacagca ttccaggtat tagaagaata tcctgattca ggtgaaaata ttgttgatgc 840
gctggcagtg ttcctgcgcc ggttgcattc gattcctgtt tgtaattgtc cttttaacag 900
cgatcgcgta tttcgtctcg ctcaggcgca atcacgaatg aataacggtt tggttgatgc 960
gagtgatttt gatgacgagc gtaatggctg gcctgttgaa caagtctgga aagaaatgca 1020
taagcttttg ccattctcac cggattcagt cgtcactcat ggtgatttct cacttgataa 1080
ccttattttt gacgagggga aattaatagg ttgtattgat gttggacgag tcggaatcgc 1140
agaccgatac caggatcttg ccatcctatg gaactgcctc ggtgagtttt ctccttcatt 1200
acagaaacgg ctttttcaaa aatatggtat tgataatcct gatatgaata aattgcagtt 1260
tcatttgatg ctcgatgagt ttttctaatc agtactgaca ataaaaagat tcttgttttc 1320
aagaacttgt catttgtata gtttttttat attgtagttg ttctatttta atcaaatgtt 1380
agcgtgattt atattttttt tcgcctcgac atcatctgcc cagatgcgaa gttaagtgcg 1440
cagaaagtaa tatcatgcgt caatcgtatg tgaatgctgg tcgctatact gctgtcgatt 1500
cgatactaac gccgccatcc agtgtcgaaa acgagctctc gagaaccctt aatataactt 1560
cgtataatgt atgctatacg aagttattag gtgatatcag atccactagt gtcgtagaga 1620
ccggtacctc 1630

<210> 120
<211> 49
<212> DNA
<213> Artificial sequence

<220>
<223> ColE1-ori TAAA BsaI CCW

<400> 120
gaggtaccgg tctcataaaa ctcatatata ctttagattg atttaaaac 49

<210> 121
<211> 47
<212> DNA

<213> Artificial sequence

<220>
<223> AmpR TTTA BsaI CW

<400> 121
gaggtaccgg tctcttttat tggtctgaca gttaccaatg cttaatc 47

<210> 122
<211> 49
<212> DNA
<213> Artificial sequence

<220>
<223> ColE1-ori GTTT BsaI CCW

<400> 122
gaggtaccgg tctcggttta ctcatatata ctttagattg atttaaaac 49

<210> 123
<211> 40
<212> DNA
<213> Artificial sequence

<220>
<223> ColE1-ori 2 ATTA BsaI CW

<400> 123
gaggtaccgg tctctattac ggtaatacgg ttatccacag 40

<210> 124
<211> 42
<212> DNA
<213> Artificial sequence

<220>
<223> AmpR 2 GCAT BsaI CCW

<400> 124
gaggtaccgg tctcagcatt ggcacttttc ggggaaatgt gc 42

<210> 125
<211> 47
<212> DNA
<213> Artificial sequence

<220>
<223> AmpR AAAC BsaI CW

<400> 125
gaggtaccgg tctcaaaact tggtctgaca gttaccaatg cttaatc 47

<210> 126
<211> 40
<212> DNA
<213> Artificial sequence

<220>

<223> EF1ap GATA BsaI CCW

<400> 126
gaggtaccgg tctcggatat cacgacacct gaaatggaag 40

<210> 127
<211> 39
<212> DNA
<213> Artificial sequence

<220>
<223> EF1apL ATGC BsaI CW

<400> 127
gaggtaccgg tctcaatgcg tgaggctccg gtgcccgtc 39

<210> 128
<211> 81
<212> DNA
<213> Artificial sequence

<220>
<223> EGFP-CAAX ACTC BsaI CCW

<400> 128

**gaggtaccgg tctccactct tacataatta cacactttgt ctttgacttc tttttcttct 60**

**tcttgtacag ctcgtccatg c 81**

<210> 129
<211> 38
<212> DNA
<213> Artificial sequence

<220>
<223> EGFP TATC BsaI CW

<400> 129
gaggtaccgg tctcctatca tggtgagcaa gggcgagg 38

<210> 130
<211> 39
<212> DNA
<213> Artificial sequence

<220>
<223> BGHpA CTAC BsaI CCW 2

<400> 130
gaggtaccgg tctcgctacc catagagccc accgcatcc 39

<210> 131
<211> 40
<212> DNA
<213> Artificial sequence

<220>
<223> BGHpA GAGT BsaI CW

<400> 131
gaggtaccgg tctcagagtc gactgtgcct tctagttgcc 40

<210> 132
<211> 42
<212> DNA
<213> Artificial sequence

<220>
<223> CMVp CGAA BsaI CCW

<400> 132
gaggtaccgg tctcgcgaag atctgacggt tcactaaacc ag 42

<210> 133
<211> 46
<212> DNA
<213> Artificial sequence

<220>
<223> CMVp GTAG BsaI CW

<400> 133
gaggtaccgg tctcagtagt tattaatagt aatcaattac ggggtc 46

<210> 134
<211> 37
<212> DNA
<213> Artificial sequence

<220>
<223> SiaT TCAC BsaI CCW

<400> 134
gaggtaccgg tctcatcacc cccgacccct tttgcag 37

<210> 135
<211> 46
<212> DNA
<213> Artificial sequence

<220>
<223> SiaT TTCG BsaI CW

<400> 135
gaggtaccgg tctccttcga tgattcacac caacctgaag aaaaag 46

<210> 136
<211> 42
<212> DNA
<213> Artificial sequence

<220>
<223> XFP-Ctag GCGG BsaI CCW

<400> 136
gaggtaccgg tctcagcggt tacttgtaca gctcgtccat gc 42

<210> 137
<211> 33
<212> DNA
<213> Artificial sequence

<220>
<223> XFP-Ctag GTGA BsaI CW

<400> 137
gaggtaccgg tctcagtgag caagggcgag gag 33

<210> 138
<211> 47
<212> DNA
<213> Artificial sequence

<220>
<223> Hygro TAAT BsaI CCW

<400> 138
gaggtaccgg tctcgtaatg atacattgat gagtttggac aaaccac 47

<210> 139
<211> 39
<212> DNA
<213> Artificial sequence

<220>
<223> Hygro ACAA BsaI CW

<400> 139
gaggtaccgg tctcaacaac aggcagaagt atgcaaagc 39

<210> 140
<211> 51
<212> DNA
<213> Artificial sequence

<220>
<223> ColE1-ori 2 GTTT BsaI CCW

<400> 140
gaggtaccgg tctcagttta aactcatata tactttagat tgatttaaaa c 51

<210> 141
<211> 40
<212> DNA
<213> Artificial sequence

<220>
<223> ColE1-ori 2 GGGG BsaI CW

<400> 141
gaggtaccgg tctctggggc ggtaatacgg ttatccacag 40

<210> 142
<211> 42
<212> DNA
<213> Artificial sequence

<220>
<223> AmpR 2 TCAC BsaI CCW

<400> 142
gaggtaccgg tctcatcact ggcacttttc ggggaaatgt gc 42

<210> 143
<211> 49
<212> DNA
<213> Artificial sequence

<220>
<223> MNNI10rec CGCA BsaI CCW

<400> 143
gaggtaccgg tctcacgcaa ttgtatagtt gtacatgcac aattattcc 49

<210> 144
<211> 52
<212> DNA
<213> Artificial sequence

<220>
<223> MNN10Lrec GTGA BsaI CW

<400> 144
gaggtaccgg tctctgtgag tttaaacatg cattcaaagg tcataattgc tg 52

<210> 145
<211> 52
<212> DNA
<213> Artificial sequence

<220>
<223> MNN10Rrec CCCC BsaI CCW

<400> 145
gaggtaccgg tctctccccg tttaaactgc ccagtttttc attattagtg tg 52

<210> 146
<211> 50
<212> DNA
<213> Artificial sequence

<220>
<223> MNN10Rrec TCGT BsaI CW

<400> 146
gaggtaccgg tctcttcgta atggaagtta tcaatattgt aaagagaagc 50

<210> 147
<211> 41
<212> DNA

<213> Artificial sequence

<220>
<223> KanMX ACGA BsaI CCW

<400> 147
gaggtaccgg tctctacgac actagtggat ctgatatcac c 41

<210> 148
<211> 40
<212> DNA
<213> Artificial sequence

<220>
<223> KanMX TGCG BsaI CW

<400> 148
gaggtaccgg tctcgtgcgg tacgctgcag gtcgacaacc 40

<210> 149
<211> 5146
<212> DNA
<213> Artificial sequence

<220>
<223> V2b

<400> 149

tattgtaata cggttatcca cagaatcagg ggataacgca ggaaagaaca tgtgagcaaa 60
aggccagcaa aaggccagga accgtaaaaa ggccgcgttg ctggcgtttt tccataggct 120
ccgcccccct gacgagcatc acaaaaatcg acgctcaagt cagaggtggc gaaacccgac 180
aggactataa agataccagg cgtttccccc tggaagctcc ctcgtgcgct ctcctgttcc 240
gaccctgccg cttaccggat acctgtccgc ctttctccct tcgggaagcg tggcgctttc 300
tcatagctca cgctgtaggt atctcagttc ggtgtaggtc gttcgctcca agctgggctg 360
tgtgcacgaa ccccccgttc agcccgaccg ctgcgcctta tccggtaact atcgtcttga 420
gtccaacccg gtaagacacg acttatcgcc actggcagca gccactggta acaggattag 480
cagagcgagg tatgtaggcg gtgctacaga gttcttgaag tggtggccta actacggcta 540
cactagaaga acagtatttg gtatctgcgc tctgctgaag ccagttacct tcggaaaaag 600
agttggtagc tcttgatccg gcaaacaaac caccgctggt agcggttttt ttgtttgcaa 660
gcagcagatt acgcgcagaa aaaaaggatc tcaagaagat cctttgatct tttctacggg 720
gtctgacgct cagtggaacg aaaactcacg ttaagggatt ttggtcatga gattatcaaa 780
aaggatcttc acctagatcc ttttaaatta aaaatgaagt tttaaatcaa tctaaagtat 840
atatgagttt tattggtctg acagttacca atgcttaatc agtgaggcac ctatctcagc 900
gatctgtcta tttcgttcat ccatagttgc ctgactcccc gtcgtgtaga taactacgat 960
acgggagggc ttaccatctg gccccagtgc tgcaatgata ccgcgtgacc cacgctcacc 1020

ggctccagat ttatcagcaa taaaccagcc agccggaagg gccgagcgca gaagtggtcc 1080
tgcaacttta tccgcctcca tccagtctat taattgttgc cgggaagcta gagtaagtag 1140
ttcgccagtt aatagtttgc gcaacgttgt tgccattgct acaggcatcg tggtgtcacg 1200
ctcgtcgttt ggtatggctt cattcagctc cggttcccaa cgatcaaggc gagttacatg 1260
atcccccatg ttgtgcaaaa aagcggttag ctccttcggt cctccgatcg ttgtcagaag 1320
taagttggcc gcagtgttat cactcatggt tatggcagca ctgcataatt ctcttactgt 1380
catgccatcc gtaagatgct tttctgtgac tggtgagtac tcaaccaagt cattctgaga 1440
atagtgtatg cggcgaccga gttgctcttg cccggcgtca atacgggata ataccgcgcc 1500
acatagcaga actttaaaag tgctcatcat tggaaaacgt tcttcggggc gaaaactctc 1560
aaggatctta ccgctgttga gatccagttc gatgtaaccc actcgtgcac ccaactgatc 1620
ttcagcatct tttactttca ccagcgtttc tgggtgagca aaaacaggaa ggcaaaatgc 1680
cgcaaaaaag ggaataaggg cgacacggaa atgttgaata ctcatactct tcctttttca 1740
atattattga agcatttatc agggttattg tctcatgagc ggatacatat ttgaatgtat 1800
ttagaaaaat aaacaaatag gggttccgcg cacatttccc cgaaaagtgc caaggaacca 1860
attcagtcga ctggatccta gttattaata gtaatcaatt acggggtcat tagttcatag 1920
cccatatatg gagttccgcg ttacataact tacggtaaat ggcccgcctg gctgaccgcc 1980
caacgacccc cgcccattga cgtcaataat gacgtatgtt cccatagtaa cgccaatagg 2040
gactttccat tgacgtcaat gggtggagta tttacggtaa actgcccact tggcagtaca 2100
tcaagtgtat catatgccaa gtacgccccc tattgacgtc aatgacggta aatggcccgc 2160
ctggcattat gcccagtaca tgaccttatg ggactttcct acttggcagt acatctacgt 2220
attagtcatc gctattacca tggtgatgcg gttttggcag tacatcaatg ggcgtggata 2280
gcggtttgac tcacggggat ttccaagtct ccaccccatt gacgtcaatg ggagtttgtt 2340
ttggcaccaa aatcaacggg actttccaaa atgtcgtaac aactccgccc cattgacgca 2400
aatgggcggt aggcgtgtac ggtgggaggt ctatataagc agagctggtt tagtgaaccg 2460
tcagatcact agtcgactag ggataacagg gcaccatgtc tagactggac aagagcaaag 2520
tcataaactc tgctctggaa ttactcaatg gagtcggtat cgaaggcctg acgacaagga 2580
aactcgctca aaagctggga gttgagcagc ctaccctgta ctggcacgtg aagaacaagc 2640
gggccctgct cgatgccctg ccaatcgaga tgctggacag gcatcatacc cactcctgcc 2700
ccctggaagg cgagtcatgg caagactttc tgcggaacaa cgccaagtca taccgctgtg 2760
ctctcctctc acatcgcgac ggggctaaag tgcatctcgg cacccgccca acagagaaac 2820
agtacgaaac cctggaaaat cagctcgcgt tcctgtgtca gcaaggcttc tccctggaga 2880
acgcactgta cgctctgtcc gccgtgggcc actttacact gggctgcgta ttggaggaac 2940

aggagcatca agtagcaaaa gaggaaagag agacacctac caccgattct atgcccccac 3000
ttctgaaaca agcaattgag ctgttcgacc ggcagggagc cgaacctgcc ttccttttcg 3060
gcctggaact aatcatatgt ggcctggaga aacagctaaa gtgcgaaagc ggcgggccga 3120
ccgacgccct tgacgatttt gacttagaca tgctcccagc cgatgccctt gacgactttg 3180
accttgatat gctgcctgct gacgctcttg acgattttga ccttgacatg ctccccgggt 3240
aatgatcgac tgtgccttct agttgccagc catctgttgt ttgcccctcc cccgtgcctt 3300
ccttgaccct ggaaggtgcc actcccactg tcctttccta ataaaatgag gaaattgcat 3360
cgcattgtct gagtaggtgt cattctattc tggggggtgg ggtggggcag gacagcaagg 3420
gggaggattg ggaggacaat agcaggcatg ctggggatgc ggtgggctct atggcttcgc 3480
tttcgtcttc aagaattcct ggagtttact ccctatcagt gatagagaac gtatgaagag 3540
tttactccct atcagtgata gagaacgtat gcagacttta ctccctatca gtgatagaga 3600
acgtataagg agtttactcc ctatcagtga tagagaacgt atgaccagtt tactccctat 3660
cagtgataga gaacgtatct acagtttact ccctatcagt gatagagaac gtatatccag 3720
tttactccct atcagtgata gagaacgtat aagctttagg cgtgtacggt gggcgcctat 3780
aaaagcagag ctcgtttagt gaaccgtcag atcgcctgga gcaattccac aacacttttg 3840
tcttatacca actttccgta ccacttccta ccctcgtaaa ccagagcatg aaggtattcc 3900
ggcgcgcttg gcggcaccgg gtggcgctgg gcctaggcgg cctggcgttc tgcggcacca 3960
ctctgttgta cctggcgcgc tgcgcttccg agggcgagac gccctccgct tccggagccg 4020
ctcggccccg cgctaaggcc ttcctggcgg tgctggtggc cagtgcgccc cgcgcggtcg 4080
agcgccgcac cgcagtgcgc agcacgtggc tggcaccgga gaggcgtggc ggacccgagg 4140
acgtgtgggc gcgcttcgcc gtgggcactg gcggcttagg ctcggaggag cggcgcgctc 4200
ttgagctcga gcaggcgcag cacggggacc tgctgctgct gcccgccctg cgcgacgcct 4260
acgagaacct cacggccaag gtcctggcca tgctgacctg gctggatgag cgcgtggact 4320
tcgagttcgt gctcaaggcg gacgacgact cctttgcgcg cctggacgct atcctggtgg 4380
acctacgcgc acgggagccc gcacgccgcc ggcgcctcta ctggggcttc ttttccgggc 4440
gcgggcgcgt caagccggga ggtcgctggc gagaagcagc ctggcaactc tgcgactact 4500
acctgcccta cgcgttgggc ggtggctatg tcctttctgc ggacctggtg cattacctgc 4560
gcctcagccg cgagtacctg cgcgcgtggc acagtgaaga cgtatcgctg ggcacctggc 4620
tggcaccagt ggatgtgcaa cgggagcacg acccacgctt cgacacggag tacaaatctc 4680
gaggctgcaa caatcagtat ctggtgacac acaagcaaag cccagaggac atgttggaga 4740
agcaacagat gttgctgcat gagggccggt tgtgcaagca tgaggtgcaa cttcgccttt 4800

cctatgtcta tgactggtca gctccaccct cccagtgctg ccagcgcaag gagggcgttc 4860

cctgatgtca ccgcggggga ggctaactga aacacggaag gagacaatac cggaaggaac 4920

ccgcgctatg acggcaataa aaagacagaa taaaacgcac ggtgttgggt cgtttgttca 4980

taaacgcggg gttcggtccc agggctggca ctctgtcgat accccaccga ggccccattg 5040

gggccaatac gcccgcgttt cttccttttc cccaccccac cccccaagtt cgggtgaagg 5100

cccagggctc gcagccaacg tcggggcggc aggccctgcc atagcc 5146

<210> 150
<211> 5556
<212> DNA
<213> Artificial sequence

<220>
<223> V3b

<400> 150

```
tattgtaata cggttatcca cagaatcagg ggataacgca ggaaagaaca tgtgagcaaa      60
aggccagcaa aaggccagga accgtaaaaa ggccgcgttg ctggcgtttt tccataggct     120
ccgcccccct gacgagcatc acaaaaatcg acgctcaagt cagaggtggc gaaacccgac     180
aggactataa agataccagg cgtttccccc tggaagctcc ctcgtgcgct ctcctgttcc     240
gaccctgccg cttaccggat acctgtccgc ctttctccct tcgggaagcg tggcgctttc     300
tcatagctca cgctgtaggt atctcagttc ggtgtaggtc gttcgctcca agctgggctg     360
tgtgcacgaa ccccccgttc agcccgaccg ctgcgcctta tccggtaact atcgtcttga     420
gtccaacccg gtaagacacg acttatcgcc actggcagca gccactggta acaggattag     480
cagagcgagg tatgtaggcg gtgctacaga gttcttgaag tggtggccta actacggcta     540
cactagaaga acagtatttg gtatctgcgc tctgctgaag ccagttacct tcggaaaaag     600
agttggtagc tcttgatccg gcaaacaaac caccgctggt agcggttttt ttgtttgcaa     660
gcagcagatt acgcgcagaa aaaaaggatc tcaagaagat cctttgatct tttctacggg     720
gtctgacgct cagtggaacg aaaactcacg ttaagggatt ttggtcatga gattatcaaa     780
aaggatcttc acctagatcc ttttaaatta aaaatgaagt tttaaatcaa tctaaagtat     840
atatgagttt tattggtctg acagttacca atgcttaatc agtgaggcac ctatctcagc     900
gatctgtcta tttcgttcat ccatagttgc ctgactcccc gtcgtgtaga taactacgat     960
acgggagggc ttaccatctg gccccagtgc tgcaatgata ccgcgtgacc cacgctcacc    1020
ggctccagat ttatcagcaa taaaccagcc agccggaagg gccgagcgca gaagtggtcc    1080
tgcaacttta tccgcctcca tccagtctat taattgttgc cgggaagcta gagtaagtag    1140
ttcgccagtt aatagtttgc gcaacgttgt tgccattgct acaggcatcg tggtgtcacg    1200
ctcgtcgttt ggtatggctt cattcagctc cggttcccaa cgatcaaggc gagttacatg    1260
```

atcccccatg ttgtgcaaaa aagcggttag ctccttcggt cctccgatcg ttgtcagaag 1320

taagttggcc gcagtgttat cactcatggt tatggcagca ctgcataatt ctcttactgt 1380

catgccatcc gtaagatgct tttctgtgac tggtgagtac tcaaccaagt cattctgaga 1440

atagtgtatg cggcgaccga gttgctcttg cccggcgtca atacgggata ataccgcgcc 1500

acatagcaga actttaaaag tgctcatcat tggaaaacgt tcttcggggc gaaaactctc 1560

aaggatctta ccgctgttga gatccagttc gatgtaaccc actcgtgcac ccaactgatc 1620

ttcagcatct tttactttca ccagcgtttc tgggtgagca aaaacaggaa ggcaaaatgc 1680

cgcaaaaaag ggaataaggg cgacacggaa atgttgaata ctcatactct tcctttttca 1740

atattattga agcatttatc agggttattg tctcatgagc ggatacatat ttgaatgtat 1800

ttagaaaaat aaacaaatag gggttccgcg cacatttccc cgaaaagtgc caaggaacca 1860

attcagtcga ctggatccta gttattaata gtaatcaatt acggggtcat tagttcatag 1920

cccatatatg gagttccgcg ttacataact tacggtaaat ggcccgcctg gctgaccgcc 1980

caacgacccc cgcccattga cgtcaataat gacgtatgtt cccatagtaa cgccaatagg 2040

gactttccat tgacgtcaat gggtggagta tttacggtaa actgcccact tggcagtaca 2100

tcaagtgtat catatgccaa gtacgccccc tattgacgtc aatgacggta aatggcccgc 2160

ctggcattat gcccagtaca tgaccttatg ggactttcct acttggcagt acatctacgt 2220

attagtcatc gctattacca tggtgatgcg gttttggcag tacatcaatg ggcgtggata 2280

gcggtttgac tcacggggat ttccaagtct ccaccccatt gacgtcaatg ggagtttgtt 2340

ttggcaccaa aatcaacggg actttccaaa atgtcgtaac aactccgccc cattgacgca 2400

aatgggcggt aggcgtgtac ggtgggaggt ctatataagc agagctggtt tagtgaaccg 2460

tcagatcact agtcgactag ggataacagg gcaccatgtc tagactggac aagagcaaag 2520

tcataaactc tgctctggaa ttactcaatg gagtcggtat cgaaggcctg acgacaagga 2580

aactcgctca aaagctggga gttgagcagc ctaccctgta ctggcacgtg aagaacaagc 2640

gggccctgct cgatgccctg ccaatcgaga tgctggacag gcatcatacc cactcctgcc 2700

ccctggaagg cgagtcatgg caagactttc tgcggaacaa cgccaagtca taccgctgtg 2760

ctctcctctc acatcgcgac ggggctaaag tgcatctcgg cacccgccca acagagaaac 2820

agtacgaaac cctggaaaat cagctcgcgt tcctgtgtca gcaaggcttc tccctggaga 2880

acgcactgta cgctctgtcc gccgtgggcc actttacact gggctgcgta ttggaggaac 2940

aggagcatca agtagcaaaa gaggaaagag agacacctac caccgattct atgcccccac 3000

ttctgaaaca agcaattgag ctgttcgacc ggcagggagc cgaacctgcc ttccttttcg 3060

gcctggaact aatcatatgt ggcctggaga aacagctaaa gtgcgaaagc ggcgggccga 3120

ccgacgccct tgacgatttt gacttagaca tgctcccagc cgatgccctt gacgactttg 3180
accttgatat gctgcctgct gacgctcttg acgattttga ccttgacatg ctccccgggt 3240
aatgatcgac tgtgccttct agttgccagc catctgttgt ttgcccctcc cccgtgcctt 3300
ccttgaccct ggaaggtgcc actcccactg tcctttccta ataaaatgag gaaattgcat 3360
cgcattgtct gagtaggtgt cattctattc tggggggtgg ggtggggcag gacagcaagg 3420
gggaggattg ggaggacaat agcaggcatg ctggggatgc ggtgggctct atggcttcgc 3480
tttcgtcttc aagaattcct ggagtttact ccctatcagt gatagagaac gtatgaagag 3540
tttactccct atcagtgata gagaacgtat gcagacttta ctccctatca gtgatagaga 3600
acgtataagg agtttactcc ctatcagtga tagagaacgt atgaccagtt tactccctat 3660
cagtgataga gaacgtatct acagtttact ccctatcagt gatagagaac gtatatccag 3720
tttactccct atcagtgata gagaacgtat aagctttagg cgtgtacggt gggcgcctat 3780
aaaagcagag ctcgtttagt gaaccgtcag atcgcctgga gcaattccac aacacttttg 3840
tcttatacca actttccgta ccacttccta ccctcgtaaa ccagagcatg aaggtattcc 3900
ggcgcgcttg gcggcaccgg gtggcgctgg gcctaggcgg cctggcgttc tgcggcacca 3960
ctctgttgta cctggcgcgc tgcgcttccg agggcgagac gccctccgct tccggagccg 4020
ctcggccccg cgctaaggcc ttcctggcgg tgctggtggc cagtgcgccc cgcgcggtcg 4080
agcgccgcac cgcagtgcgc agcacgtggc tggcaccgga gaggcgtggc ggacccgagg 4140
acgtgtgggc gcgcttcgcc gtgggcactg gcggcttagg ctcggaggag cggcgcgctc 4200
ttgagctcga gcaggcgcag cacggggacc tgctgctgct gcccgccctg cgcgacgcct 4260
acgagaacct cacggccaag gtcctggcca tgctgacctg gctggatgag cgcgtggact 4320
tcgagttcgt gctcaaggcg gacgacgact cctttgcgcg cctggacgct atcctggtgg 4380
acctacgcgc acgggagccc gcacgccgcc ggcgcctcta ctggggcttc ttttccgggc 4440
gcgggcgcgt caagccggga ggtcgctggc gagaagcagc ctggcaactc tgcgactact 4500
acctgcccta cgcgttgggc ggtggctatg tcctttctgc ggacctggtg cattacctgc 4560
gcctcagccg cgagtacctg cgcgcgtggc acagtgaaga cgtatcgctg ggcacctggc 4620
tggcaccagt ggatgtgcaa cgggagcacg acccacgctt cgacacggag tacaaatctc 4680
gaggctgcaa caatcagtat ctggtgacac acaagcaaag cccagaggac atgttggaga 4740
agcaacagat gttgctgcat gagggccggt tgtgcaagca tgaggtgcaa cttcgccttt 4800
cctatgtcta tgactggtca gctccaccct cccagtgctg ccagcgcaag gagggcgttc 4860
cctgatgtca ccgcggggga ggctaactga aacacggaag gagacaatac cggaaggaac 4920
ccgcgctatg acggcaataa aaagacagaa taaaacgcac ggtgttgggt cgtttgttca 4980
taaacgcggg gttcggtccc agggctggca ctctgtcgat accccaccga ggccccattg 5040

gggccaatac gcccgcgttt cttccttttc cccaccccac cccccaagtt cgggtgaagg 5100
cccagggctc gcagccaacg tcggggcggc aggccctgcc atagccacaa acagggtcga 5160
caagcttttc caaaaaaaaa gcatgaggtg cagttgcgcc tttcctatct cttgaatagg 5220
aaaggcgcaa ctgcacctca tgctggatcc cgcgtccttt ccacaagata tataaaccca 5280
agaaatcgaa atactttcaa gttacggtaa gcatatgata gtccatttta aaacataatt 5340
ttaaaactgc aaactaccca agaaattatt actttctacg tcacgtattt tgtactaata 5400
tctttgtgtt tacagtcaaa ttaattctaa ttatctctct aacagccttg tatcgtatat 5460
gcaaatatga aggaatcatg ggaaataggc cctcttcctg cccgaccttg gcgcgcgctc 5520
ggcgcgcggt cacgctccgt cacgtggtgc gttttg 5556

<210> 151
<211> 7570
<212> DNA
<213> Artificial sequence

<220>
<223> V1.1b

<400> 151

```
cggtaatacg gttatccaca gaatcagggg ataacgcagg aaagaacatg tgagcaaaag      60
gccagcaaaa ggccaggaac cgtaaaaagg ccgcgttgct ggcgtttttc cataggctcc     120
gcccccctga cgagcatcac aaaaatcgac gctcaagtca gaggtggcga aacccgacag     180
gactataaag ataccaggcg tttccccctg gaagctccct cgtgcgctct cctgttccga     240
ccctgccgct taccggatac ctgtccgcct ttctcccttc gggaagcgtg gcgctttctc     300
atagctcacg ctgtaggtat ctcagttcgg tgtaggtcgt tcgctccaag ctgggctgtg     360
tgcacgaacc ccccgttcag cccgaccgct gcgccttatc cggtaactat cgtcttgagt     420
ccaacccggt aagacacgac ttatcgccac tggcagcagc cactggtaac aggattagca     480
gagcgaggta tgtaggcggt gctacagagt tcttgaagtg gtggcctaac tacggctaca     540
ctagaagaac agtatttggt atctgcgctc tgctgaagcc agttaccttc ggaaaaagag     600
ttggtagctc ttgatccggc aaacaaacca ccgctggtag cggttttttt gtttgcaagc     660
agcagattac gcgcagaaaa aaaggatctc aagaagatcc tttgatcttt tctacggggt     720
ctgacgctca gtggaacgaa aactcacgtt aagggatttt ggtcatgaga ttatcaaaaa     780
ggatcttcac ctagatcctt ttaaattaaa aatgaagttt taaatcaatc taaagtatat     840
atgagtaaac ttggtctgac agttaccaat gcttaatcag tgaggcacct atctcagcga     900
tctgtctatt tcgttcatcc atagttgcct gactccccgt cgtgtagata actacgatac     960
gggagggctt accatctggc cccagtgctg caatgatacc gcgtgaccca cgctcaccgg    1020
```

ctccagattt atcagcaata aaccagccag ccggaagggc cgagcgcaga agtggtcctg 1080
caactttatc cgcctccatc cagtctatta attgttgccg ggaagctaga gtaagtagtt 1140
cgccagttaa tagtttgcgc aacgttgttg ccattgctac aggcatcgtg gtgtcacgct 1200
cgtcgtttgg tatggcttca ttcagctccg gttcccaacg atcaaggcga gttacatgat 1260
cccccatgtt gtgcaaaaaa gcggttagct ccttcggtcc tccgatcgtt gtcagaagta 1320
agttggccgc agtgttatca ctcatggtta tggcagcact gcataattct cttactgtca 1380
tgccatccgt aagatgcttt tctgtgactg gtgagtactc aaccaagtca ttctgagaat 1440
agtgtatgcg gcgaccgagt tgctcttgcc cggcgtcaat acgggataat accgcgccac 1500
atagcagaac tttaaaagtg ctcatcattg gaaaacgttc ttcggggcga aaactctcaa 1560
ggatcttacc gctgttgaga tccagttcga tgtaacccac tcgtgcaccc aactgatctt 1620
cagcatcttt tactttcacc agcgtttctg ggtgagcaaa aacaggaagg caaaatgccg 1680
caaaaaaggg aataagggcg acacggaaat gttgaatact catactcttc ctttttcaat 1740
attattgaag catttatcag ggttattgtc tcatgagcgg atacatattt gaatgtattt 1800
agaaaaataa acaaataggg gttccgcgca catttccccg aaaagtgcca atgcgtgagg 1860
ctccggtgcc cgtcagtggg cagagcgcac atcgcccaca gtccccgaga agttgggggg 1920
aggggtcggc aattgaaccg gtgcctagag aaggtggcgc ggggtaaact gggaaagtga 1980
tgtcgtgtac tggctccgcc tttttcccga gggtggggga gaaccgtata taagtgcagt 2040
agtcgccgtg aacgttcttt ttcgcaacgg gtttgccgcc agaacacagg taagtgccgt 2100
gtgtggttcc cgcgggcctg gcctctttac gggttatggc ccttgcgtgc cttgaattac 2160
ttccacctgg ctgcagtacg tgattcttga tcccgagctt cgggttggaa gtgggtggga 2220
gagttcgagg ccttgcgctt aaggagcccc ttcgcctcgt gcttgagttg aggcctggcc 2280
tgggcgctgg ggccgccgcg tgcgaatctg gtggcacctt cgcgcctgtc tcgctgcttt 2340
cgataagtct ctagccattt aaaatttttg atgacctgct gcgacgcttt ttttctggca 2400
agatagtctt gtaaatgcgg gccaagatct gcacactggt atttcggttt ttggggccgc 2460
gggcggcgac ggggcccgtg cgtcccagcg cacatgttcg gcgaggcggg gcctgcgagc 2520
gcggccaccg agaatcggac gggggtagtc tcaagctggc cggcctgctc tggtgcctgg 2580
cctcgcgccg ccgtgtatcg ccccgccctg ggcggcaagg ctggcccggt cggcaccagt 2640
tgcgtgagcg gaaagatggc cgcttcccgg ccctgctgca gggagctcaa aatggaggac 2700
gcggcgctcg ggagagcggg cgggtgagtc acccacacaa aggaaaaggg cctttccgtc 2760
ctcagccgtc gcttcatgtg actccacgga gtaccgggcg ccgtccaggc acctcgatta 2820
gttctcgagc ttttggagta cgtcgtcttt aggttggggg gaggggtttt atgcgatgga 2880
gtttccccac actgagtggg tggagactga agttaggcca gcttggcact tgatgtaatt 2940

ctccttggaa tttgcccttt ttgagtttgg atcttggttc attctcaagc ctcagacagt 3000
ggttcaaagt ttttttcttc catttcaggt gtcgtgatat catggtgagc aagggcgagg 3060
agctgttcac cggggtggtg cccatcctgg tcgagctgga cggcgacgta aacggccaca 3120
agttcagcgt gtccggcgag ggcgagggcg atgccaccta cggcaagctg accctgaagt 3180
tcatctgcac caccggcaag ctgcccgtgc cctggcccac cctcgtgacc accctgacct 3240
acggcgtgca gtgcttcagc cgctaccccg accacatgaa gcagcacgac ttcttcaagt 3300
ccgccatgcc cgaaggctac gtccaggagc gcaccatctt cttcaaggac gacggcaact 3360
acaagacccg cgccgaggtg aagttcgagg gcgacaccct ggtgaaccgc atcgagctga 3420
agggcatcga cttcaaggag gacggcaaca tcctggggca caagctggag tacaactaca 3480
acagccacaa cgtctatatc atggccgaca agcagaagaa cggcatcaag gtgaacttca 3540
agatccgcca caacatcgag gacggcagcg tgcagctcgc cgaccactac cagcagaaca 3600
cccccatcgg cgacggcccc gtgctgctgc ccgacaacca ctacctgagc acccagtccg 3660
ccctgagcaa agaccccaac gagaagcgcg atcacatggt cctgctggag ttcgtgaccg 3720
ccgccgggat cactctcggc atggacgagc tgtacaagaa gaagaaaaag aagtcaaaga 3780
caaagtgtgt aattatgtaa gagtcgactg tgccttctag ttgccagcca tctgttgttt 3840
gcccctcccc cgtgccttcc ttgaccctgg aaggtgccac tcccactgtc ctttcctaat 3900
aaaatgagga aattgcatcg cattgtctga gtaggtgtca ttctattctg gggggtgggg 3960
tggggcagga cagcaagggg gaggattggg aggacaatag caggcatgct ggggatgcgg 4020
tgggctctat gggtagttat taatagtaat caattacggg gtcattagtt catagcccat 4080
atatggagtt ccgcgttaca taacttacgg taaatggccc gcctggctga ccgcccaacg 4140
acccccgccc attgacgtca ataatgacgt atgttcccat agtaacgcca atagggactt 4200
tccattgacg tcaatgggtg gagtatttac ggtaaactgc ccacttggca gtacatcaag 4260
tgtatcatat gccaagtacg ccccctattg acgtcaatga cggtaaatgg cccgcctggc 4320
attatgccca gtacatgacc ttatgggact ttcctacttg gcagtacatc tacgtattag 4380
tcatcgctat taccatggtg atgcggtttt ggcagtacat caatgggcgt ggatagcggt 4440
ttgactcacg gggatttcca agtctccacc ccattgacgt caatgggagt ttgttttggc 4500
accaaaatca acgggacttt ccaaaatgtc gtaacaactc cgccccattg acgcaaatgg 4560
gcggtaggcg tgtacggtgg gaggtctata taagcagagc tggtttagtg aaccgtcaga 4620
tcttcgatga ttcacaccaa cctgaagaaa aagttcagct gctgcgtcct ggtctttctt 4680
ctgtttgcag tcatctgtgt gtggaaggaa aagaagaaag ggagttacta tgattccttt 4740
aaattgcaaa ccaaggaatt ccaggtgtta aagagtctgg ggaaattggc catggggtct 4800

gattcccagt ctgtatcctc aagcagcacc caggaccccc acaggggccg ccagaccctc 4860

ggcagtctca gaggcctagc caaggccaaa ccagaggcct ccttccaggt gtggaacaag 4920

gacagctctt ccaaaaacct tatccctagg ctgcaaaagg ggtcgggggt gagcaagggc 4980

gaggaggata acatggccat catcaaggag ttcatgcgct tcaaggtgca catggagggc 5040

tccgtgaacg gccacgagtt cgagatcgag ggcgagggcg agggccgccc ctacgagggc 5100

acccagaccg ccaagctgaa ggtgaccaag ggtggccccc tgcccttcgc ctgggacatc 5160

ctgtcccctc agttcatgta cggctccaag gcctacgtga agcaccccgc cgacatcccc 5220

gactacttga agctgtcctt ccccgagggc ttcaagtggg agcgcgtgat gaacttcgag 5280

gacggcggcg tggtgaccgt gacccaggac tcctccctgc aggacggcga gttcatctac 5340

aaggtgaagc tgcgcggcac caacttcccc tccgacggcc ccgtaatgca gaagaaaacc 5400

atgggctggg aggcctcctc cgagcggatg taccccgagg acggcgccct gaagggcgag 5460

atcaagcaga ggctgaagct gaaggacggc ggccactacg acgctgaggt caagaccacc 5520

tacaaggcca agaagcccgt gcagctgccc ggcgcctaca acgtcaacat caagttggac 5580

atcacctccc acaacgagga ctacaccatc gtggaacagt acgaacgcgc cgagggccgc 5640

cactccaccg gcggcatgga cgagctgtac aagtaaccgc gggggaggct aactgaaaca 5700

cggaaggaga caataccgga aggaacccgc gctatgacgg caataaaaag acagaataaa 5760

acgcacggtg ttgggtcgtt tgttcataaa cgcggggttc ggtcccaggg ctggcactct 5820

gtcgataccc caccgaggcc ccattggggc caatacgccc gcgtttcttc cttttcccca 5880

ccccaccccc caagttcggg tgaaggccca gggctcgcag ccaacgtcgg ggcggcaggc 5940

cctgccatag acaacaggca gaagtatgca aagcatgcat ctcaattagt cagcaaccag 6000

gtgtggaaag tccccaggct ccccagcagg cagaagtatg caaagcatgc atctcaatta 6060

gtcagcaacc atagtcccgc ccctaactcc gcccatcccg cccctaactc cgcccagttc 6120

cgcccattct ccgccccatg gctgactaat tttttttatt tatgcagagg ccgaggccgc 6180

ctctgcctct gagctattcc agaagtagtg aggaggcttt tttggaggcc taggcttttg 6240

caaaaagctc ccgggagctt gtatatccat tttcggatct gatcagcacg tgatgaaaaa 6300

gcctgaactc accgcgacgt ctgtcgagaa gtttctgatc gaaaagttcg acagcgtgtc 6360

cgacctgatg cagctctcgg agggcgaaga atctcgtgct ttcagcttcg atgtaggagg 6420

gcgtggatat gtcctgcggg taaatagctg cgccgatggt ttctacaaag atcgttatgt 6480

ttatcggcac tttgcatcgg ccgcgctccc gattccggaa gtgcttgaca ttggggaatt 6540

cagcgagagc ctgacctatt gcatctcccg ccgtgcacag ggtgtcacgt tgcaagactt 6600

gcctgaaacc gaactgcccg ctgttctgca gccggtcgcg gaggccatgg atgcgatcgc 6660

tgcggccgat cttagccaga cgagcgggtt cggcccattc ggaccgcaag gaatcggtca 6720

atacactaca tggcgtgatt tcatatgcgc gattgctgat ccccatgtgt atcactggca 6780
aactgtgatg gacgacaccg tcagtgcgtc cgtcgcgcag gctctcgatg agctgatgct 6840
ttgggccgag gactgccccg aagtccggca cctcgtgcac gcggatttcg gctccaacaa 6900
tgtcctgacg gacaatggcc gcataacagc ggtcattgac tggagcgagg cgatgttcgg 6960
ggattcccaa tacgaggtcg ccaacatctt cttctggagg ccgtggttgg cttgtatgga 7020
gcagcagacg cgctacttcg agcggaggca tccggagctt gcaggatcgc cgcggctccg 7080
ggcgtatatg ctccgcattg gtcttgacca actctatcag agcttggttg acggcaattt 7140
cgatgatgca gcttgggcgc agggtcgatg cgacgcaatc gtccgatccg gagccgggac 7200
tgtcgggcgt acacaaatcg cccgcagaag cgcggccgtc tggaccgatg gctgtgtaga 7260
agtactcgcc gatagtggaa accgacgccc cagcactcgt ccgagggcaa aggaatagca 7320
cgtgctacga gatttcgatt ccaccgccgc cttctatgaa aggttgggct tcggaatcgt 7380
tttccgggac gccggctgga tgatcctcca gcgcggggat ctcatgctgg agttcttcgc 7440
ccaccccaac ttgtttattg cagcttataa tggttacaaa taaagcaata gcatcacaaa 7500
tttcacaaat aaagcatttt tttcactgca ttctagttgt ggtttgtcca aactcatcaa 7560
tgtatcatta 7570

<210> 152
<211> 4346
<212> DNA
<213> Artificial sequence

<220>
<223> V4

<400> 152

```
cggtaatacg gttatccaca gaatcagggg ataacgcagg aaagaacatg tgagcaaaag        60
gccagcaaaa ggccaggaac cgtaaaaagg ccgcgttgct ggcgtttttc cataggctcc       120
gcccccctga cgagcatcac aaaaatcgac gctcaagtca gaggtggcga aacccgacag       180
gactataaag ataccaggcg tttccccctg gaagctccct cgtgcgctct cctgttccga       240
ccctgccgct taccggatac ctgtccgcct ttctcccttc gggaagcgtg gcgctttctc       300
atagctcacg ctgtaggtat ctcagttcgg tgtaggtcgt tcgctccaag ctgggctgtg       360
tgcacgaacc ccccgttcag cccgaccgct gcgccttatc cggtaactat cgtcttgagt       420
ccaacccggt aagacacgac ttatcgccac tggcagcagc cactggtaac aggattagca       480
gagcgaggta tgtaggcggt gctacagagt tcttgaagtg gtggcctaac tacggctaca       540
ctagaagaac agtatttggt atctgcgctc tgctgaagcc agttaccttc ggaaaaagag       600
ttggtagctc ttgatccggc aaacaaacca ccgctggtag cggttttttt gtttgcaagc       660
```

agcagattac gcgcagaaaa aaaggatctc aagaagatcc tttgatcttt tctacggggt 720
ctgacgctca gtggaacgaa aactcacgtt aagggatttt ggtcatgaga ttatcaaaaa 780
ggatcttcac ctagatcctt ttaaattaaa aatgaagttt taaatcaatc taaagtatat 840
atgagttaaa acttggtctg acagttacca atgcttaatc agtgaggcac ctatctcagc 900
gatctgtcta tttcgttcat ccatagttgc ctgactcccc gtcgtgtaga taactacgat 960
acgggagggc ttaccatctg gccccagtgc tgcaatgata ccgcgtgacc cacgctcacc 1020
ggctccagat ttatcagcaa taaaccagcc agccggaagg gccgagcgca gaagtggtcc 1080
tgcaacttta tccgcctcca tccagtctat taattgttgc cgggaagcta gagtaagtag 1140
ttcgccagtt aatagtttgc gcaacgttgt tgccattgct acaggcatcg tggtgtcacg 1200
ctcgtcgttt ggtatggctt cattcagctc cggttcccaa cgatcaaggc gagttacatg 1260
atcccccatg ttgtgcaaaa aagcggttag ctccttcggt cctccgatcg ttgtcagaag 1320
taagttggcc gcagtgttat cactcatggt tatggcagca ctgcataatt ctcttactgt 1380
catgccatcc gtaagatgct tttctgtgac tggtgagtac tcaaccaagt cattctgaga 1440
atagtgtatg cggcgaccga gttgctcttg cccggcgtca atacgggata ataccgcgcc 1500
acatagcaga actttaaaag tgctcatcat tggaaaacgt tcttcggggc gaaaactctc 1560
aaggatctta ccgctgttga gatccagttc gatgtaaccc actcgtgcac ccaactgatc 1620
ttcagcatct tttactttca ccagcgtttc tgggtgagca aaaacaggaa ggcaaaatgc 1680
cgcaaaaaag ggaataaggg cgacacggaa atgttgaata ctcatactct tcctttttca 1740
atattattga agcatttatc agggttattg tctcatgagc ggatacatat ttgaatgtat 1800
ttagaaaaat aaacaaatag gggttccgcg cacatttccc cgaaaagtgc cagtgagttt 1860
aaacatgcat tcaaaggtca taattgctgc tctatttaca gtcgtccata atgacatttc 1920
tctttgatta ttttcttgtt ttttcgctct tctcaagtgg atgttacata acaaacaaaa 1980
cagaaaaaat tgtttaaata taaagtttaa aagttatctt tgattccgca cctgaatttt 2040
tggattgaag gccaaaggag gtttatcagg gagagaaaag ctctctattt atttttataa 2100
ggaataattg tgcatgtaca actatacaat tgcggtacgc tgcaggtcga caacccttaa 2160
tataacttcg tataatgtat gctatacgaa gttattaggt ctagagatct gtttagcttg 2220
cctcgtcccc gccgggtcac ccggccagcg acatggaggc ccagaatacc ctccttgaca 2280
gtcttgacgt gcgcagctca ggggcatgat gtgactgtcg cccgtacatt tagcccatac 2340
atccccatgt ataatcattt gcatccatac attttgatgg ccgcacggcg cgaagcaaaa 2400
attacggctc ctcgctgcag acctgcgagc agggaaacgc tcccctcaca gacgcgttga 2460
attgtcccca cgccgcgccc ctgtagagaa atataaaagg ttaggatttg ccactgaggt 2520
tcttctttca tatacttcct tttaaaatct tgctaggata cagttctcac atcacatccg 2580

aacataaaca accatgggta aggaaaagac tcacgtttcg aggccgcgat taaattccaa 2640
catggatgct gatttatatg ggtataaatg ggctcgcgat aatgtcgggc aatcaggtgc 2700
gacaatctat cgattgtatg ggaagcccga tgcgccagag ttgtttctga aacatggcaa 2760
aggtagcgtt gccaatgatg ttacagatga gatggtcaga ctaaactggc tgacggaatt 2820
tatgcctctt ccgaccatca agcattttat ccgtactcct gatgatgcat ggttactcac 2880
cactgcgatc cccggcaaaa cagcattcca ggtattagaa gaatatcctg attcaggtga 2940
aaatattgtt gatgcgctgg cagtgttcct gcgccggttg cattcgattc ctgtttgtaa 3000
ttgtcctttt aacagcgatc gcgtatttcg tctcgctcag gcgcaatcac gaatgaataa 3060
cggtttggtt gatgcgagtg attttgatga cgagcgtaat ggctggcctg ttgaacaagt 3120
ctggaaagaa atgcataagc ttttgccatt ctcaccggat tcagtcgtca ctcatggtga 3180
tttctcactt gataacctta tttttgacga ggggaaatta ataggttgta ttgatgttgg 3240
acgagtcgga atcgcagacc gataccagga tcttgccatc ctatggaact gcctcggtga 3300
gttttctcct tcattacaga aacggctttt tcaaaaatat ggtattgata atcctgatat 3360
gaataaattg cagtttcatt tgatgctcga tgagtttttc taatcagtac tgacaataaa 3420
aagattcttg ttttcaagaa cttgtcattt gtatagtttt tttatattgt agttgttcta 3480
ttttaatcaa atgttagcgt gatttatatt ttttttcgcc tcgacatcat ctgcccagat 3540
gcgaagttaa gtgcgcagaa agtaatatca tgcgtcaatc gtatgtgaat gctggtcgct 3600
atactgctgt cgattcgata ctaacgccgc catccagtgt cgaaaacgag ctctcgagaa 3660
cccttaatat aacttcgtat aatgtatgct atacgaagtt attaggtgat atcagatcca 3720
ctagtgtcgt aatggaagtt atcaatattg taaagagaag catttacaag cttttatttt 3780
tctttttaat ttccactact ggttctgctt taaaatgttg ttttataatt tatgtacatt 3840
taggcctata gaagattctt tcaataatat gctacacatt cttttatttt tccatcatat 3900
gttggagttt atgcctcctc ggcaggagtt gggcggtgcg aagagaagaa aaagagtgaa 3960
actaaaaaaa ggaatctgcc tttgcataag ttcaaaagtg caattttagt gttggattta 4020
aacgggaaaa attgaaatgg ccatcgaaac aatacttgta ataaacaaat caggcggact 4080
aatctatcag cggaatttta ccaacgacga acagaaattg aacagcaatg aatacttaat 4140
tcttgctagt acactgcacg gtgtattcgc catcgcgagc cagctgactc cgaaggcatt 4200
acagctaact caacaaacga acatcgaaaa taccatccca tatatacctt acgtgggcat 4260
gtccagcaat aggagcgata caagaaatgg aggtggcaat aacaacaaac acactaataa 4320
tgaaaaactg ggcagtttaa acgggg 4346

<210> 153

<211> 1967
<212> DNA
<213> Artificial sequence

<220>
<223> Matrice eZ-Ori-AmpR

<400> 153

gcgtcttcta gggttaaggt tagtgtagag aagcaaccga agattgagaa gacatggcgg 60
taatacggtt atccacagaa tcaggggata acgcaggaaa gaacatgtga gcaaaaggcc 120
agcaaaaggc caggaaccgt aaaaaggccg cgttgctggc gtttttccat aggctccgcc 180
cccctgacga gcatcacaaa aatcgacgct caagtcagag gtggcgaaac ccgacaggac 240
tataaagata ccaggcgttt ccccctggaa gctccctcgt gcgctctcct gttccgaccc 300
tgccgcttac cggatacctg tccgcctttc tcccttcggg aagcgtggcg ctttctcata 360
gctcacgctg taggtatctc agttcggtgt aggtcgttcg ctccaagctg ggctgtgtgc 420
acgaaccccc cgttcagccc gaccgctgcg ccttatccgg taactatcgt cttgagtcca 480
acccggtaag acacgactta tcgccactgg cagcagccac tggtaacagg attagcagag 540
cgaggtatgt aggcggtgct acagagttct tgaagtggtg gcctaactac ggctacacta 600
gaagaacagt atttggtatc tgcgctctgc tgaagccagt taccttcgga aaaagagttg 660
gtagctcttg atccggcaaa caaaccaccg ctggtagcgg tttttttgtt tgcaagcagc 720
agattacgcg cagaaaaaaa ggatctcaag aagatccttt gatcttttct acggggtctg 780
acgctcagtg gaacgaaaac tcacgttaag ggattttggt catgagatta tcaaaaagga 840
tcttcaccta gatcctttta aattaaaaat gaagttttaa atcaatctaa agtatatatg 900
agtaaacttg gtctgacagt taccaatgct taatcagtga ggcacctatc tcagcgatct 960
gtctatttcg ttcatccata gttgcctgac tccccgtcgt gtagataact acgatacggg 1020
agggcttacc atctggcccc agtgctgcaa tgataccgcg tgacccacgc tcaccggctc 1080
cagatttatc agcaataaac cagccagccg gaagggccga gcgcagaagt ggtcctgcaa 1140
ctttatccgc ctccatccag tctattaatt gttgccggga agctagagta agtagttcgc 1200
cagttaatag tttgcgcaac gttgttgcca ttgctacagg catcgtggtg tcacgctcgt 1260
cgtttggtat ggcttcattc agctccggtt cccaacgatc aaggcgagtt acatgatccc 1320
ccatgttgtg caaaaaagcg gttagctcct tcggtcctcc gatcgttgtc agaagtaagt 1380
tggccgcagt gttatcactc atggttatgg cagcactgca taattctctt actgtcatgc 1440
catccgtaag atgcttttct gtgactggtg agtactcaac caagtcattc tgagaatagt 1500
gtatgcggcg accgagttgc tcttgcccgg cgtcaatacg ggataatacc gcgccacata 1560
gcagaacttt aaaagtgctc atcattggaa aacgttcttc ggggcgaaaa ctctcaagga 1620
tcttaccgct gttgagatcc agttcgatgt aacccactcg tgcacccaac tgatcttcag 1680

catcttttac tttcaccagc gtttctgggt gagcaaaaac aggaaggcaa aatgccgcaa 1740

aaaagggaat aagggcgaca cggaaatgtt gaatactcat actcttcctt tttcaatatt 1800

attgaagcat ttatcagggt tattgtctca tgagcggata catatttgaa tgtatttaga 1860

aaaataaaca aataggggtt ccgcgcacat ttccccgaaa agtgccacct atgagacgtg 1920

aggctaggga taggacgaga gcatcgggaa cgaggactag cgtctca 1967

<210> 154
<211> 235
<212> DNA
<213> Artificial sequence

<220>
<223> Matrice BGH polyA

<400> 154

tcgactgtgc cttctagttg ccagccatct gttgtttgcc cctcccccgt gccttccttg 60

accctggaag gtgccactcc cactgtcctt tcctaataaa atgaggaaat tgcatcgcat 120

tgtctgagta ggtgtcattc tattctgggg ggtggggtgg ggcaggacag caagggggag 180

gattgggagg acaatagcag gcatgctggg gatgcggtgg gctctatggc ttctg 235

<210> 155
<211> 720
<212> DNA
<213> Artificial sequence

<220>
<223> Matrice eZ-E1GFP

<400> 155

```
atggtgagca agggcgagga gctgttcacc ggggtggtgc ccatcctggt cgagctggac      60
ggcgacgtaa acggccacaa gttcagcgtg tccggcgagg gcgagggcga tgccacctac     120
ggcaagctga ccctgaagtt catctgcacc accggcaagc tgcccgtgcc ctggcccacc     180
ctcgtgacca ccctgtccta cggcgtgcag tgcttcagcc gctaccccga ccacatgaag     240
cagcacgact tcttcaagtc cgccatgccc gaaggctacg tccaggagcg caccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacaa cagccacaac gtctatatca tggccgacaa gcagaagaac     480
ggcatcaagg tgaacttcaa gatccgccac aacatcgagg acggcagcgt gcagctcgcc     540
gaccactacc agcagaacac ccccatcggc gacggccccg tgctgctgcc cgacaaccac     600
tacctgagct accagtccgc cctgagcaaa gaccccaacg agaagcgcga tcacatggtc     660
ctgctggagt tcgtgaccgc cgccgggatc actctcggca tggacgagct gtacaagtaa     720
```

<210> 156
<211> 720
<212> DNA
<213> Artificial sequence

<220>
<223> Matrice EGFP

<400> 156

```
atggtgagca agggcgagga gctgttcacc ggggtggtgc ccatcctggt cgagctggac      60
ggcgacgtaa acggccacaa gttcagcgtg tccggcgagg gcgagggcga tgccacctac     120
ggcaagctga ccctgaagtt catctgcacc accggcaagc tgcccgtgcc ctggcccacc     180
ctcgtgacca ccctgaccta cggcgtgcag tgcttcagcc gctaccccga ccacatgaag     240
cagcacgact tcttcaagtc cgccatgccc gaaggctacg tccaggagcg caccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacaa cagccacaac gtctatatca tggccgacaa gcagaagaac     480
ggcatcaagg tgaacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgccgggatc actctcggca tggacgagct gtacaagtaa     720
```

<210> 157
<211> 720

<212> DNA
<213> Artificial sequence

<220>
<223> Matrice ECFP

<400> 157

```
atggtgagca agggcgagga gctgttcacc ggggtggtgc ccatcctggt cgagctggac        60
ggcgacgtaa acggccacaa gttcagcgtg tccggcgagg gcgagggcga tgccacctac       120
ggcaagctga ccctgaagtt catctgcacc accggcaagc tgcccgtgcc ctggcccacc       180
ctcgtgacca ccctgacctg gggcgtgcag tgcttcagcc gctaccccga ccacatgaag       240
cagcacgact tcttcaagtc cgccatgccc gaaggctacg tccaggagcg caccatcttc       300
ttcaaggacg acggcaacta caagacccgc gccgaggtga agttcgaggg cgacaccctg       360
gtgaaccgca tcgagctgaa gggcatcgac ttcaaggagg acggcaacat cctggggcac       420
aagctggagt acaactacat cagccacaac gtctatatca ccgccgacaa gcagaagaac       480
ggcatcaagg ccaacttcaa gatccgccac aacatcgagg acggcagcgt gcagctcgcc       540
gaccactacc agcagaacac ccccatcggc gacggccccg tgctgctgcc cgacaaccac       600
tacctgagca cccagtccgc cctgagcaaa gaccccaacg agaagcgcga tcacatggtc       660
ctgctggagt tcgtgaccgc cgccgggatc actctcggca tggacgagct gtacaagtcc       720
```

<210> 158
<211> 714
<212> DNA
<213> Artificial sequence

<220>
<223> Matrice EYFP

<400> 158

atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccttcggcta cggcctgcag tgcttcgccc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagct accagtccgc cctgagcaaa gaccccaacg agaagcgcga tcacatggtc 660
ctgctggagt tcgtgaccgc cgccgggatc actctcggca tggacgagct gtac 714

<210> 159
<211> 718
<212> DNA
<213> Artificial sequence

<220>
<223> Matrice mCherry

<400> 159

atggtcgggg gtgagcaagg gcgaggagga taacatggcc atcatcaagg agttcatgcg 60
cttcaaggtg cacatggagg gctccgtgaa cggccacgag ttcgagatcg agggcgaggg 120
cgagggccgc ccctacgagg gcacccagac cgccaagctg aaggtgacca agggtggccc 180
cctgcccttc gcctgggaca tcctgtcccc tcagttcatg tacggctcca aggcctacgt 240
gaagcacccc gccgacatcc ccgactactt gaagctgtcc ttccccgagg gcttcaagtg 300
ggagcgcgtg atgaacttcg aggacggcgg cgtggtgacc gtgacccagg actcctccct 360
gcaggacggc gagttcatct acaaggtgaa gctgcgcggc accaacttcc cctccgacgg 420
ccccgtaatg cagaagaaaa ccatgggctg ggaggcctcc tccgagcgga tgtaccccga 480
ggacggcgcc ctgaagggcg agatcaagca gaggctgaag ctgaaggacg gcggccacta 540
cgacgctgag gtcaagacca cctacaaggc caagaagccc gtgcagctgc ccggcgccta 600
caacgtcaac atcaagttgg acatcacctc ccacaacgag gactacacca tcgtggaaca 660
gtacgaacgc gccgagggcc gccactccac cggcggcatg gacgagctgt acaagtga 718

<210> 160

<211> 1086
<212> DNA
<213> Artificial sequence

<220>
<223> Matrice hFUT3 cDNA

<400> 160

```
atggatcccc tgggtgcagc caagccacaa tggccatggc gccgctgtct ggccgcactg        60
ctatttcagc tgctggtggc tgtgtgtttc ttctcctacc tgcgtgtgtc ccgagacgat       120
gccactggat cccctagggc tcccagtggg tcctcccgac aggacaccac tcccacccgc       180
cccaccctcc tgatcctgct atggacatgg cctttccaca tccctgtggc tctgtcccgc       240
tgttcagaga tggtgcccgg cacagccgac tgccacatca ctgccgaccg caaggtgtac       300
ccacaggcag acacggtcat cgtgcaccac tgggatatca tgtccaaccc taagtcacgc       360
ctcccacctt ccccgaggcc gcaggggcag cgctggatct ggttcaactt ggagccaccc       420
cctaactgcc agcacctgga agccctggac agatacttca atctcaccat gtcctaccgc       480
agcgactccg acatcttcac gccctacggc tggctggagc cgtggtccgg ccagcctgcc       540
cacccaccgc tcaacctctc ggccaagacc gagctggtgg cctgggcggt gtccaactgg       600
aagccggact cagccagggt gcgctactac cagagcctgc aggctcatct caaggtggac       660
gtgtacggac gctcccacaa gcccctgccc aaggggacca tgatggagac gctgtcccgg       720
tacaagttct acctggcctt cgagaactcc ttgcaccccg actacatcac cgagaagctg       780
tggaggaacg ccctggaggc ctgggccgtg cccgtggtgc tgggccccag cagaagcaac       840
tacgagaggt tcctgccacc cgacgccttc atccacgtgg acgacttcca gagccccaag       900
gacctggccc ggtacctgca ggagctggac aaggaccacg cccgctacct gagctacttt       960
cgctggcggg agacgctgcg gcctcgctcc ttcagctggg cactggattt ctgcaaggcc      1020
tgctggaaac tgcagcagga atccaggtac cagacggtgc gcagcatagc ggcttggttc      1080
acctga                                                                 1086
```

<210> 161
<211> 1612
<212> DNA
<213> Artificial sequence

<220>
<223> Matrice eZ-HygromycinR K7

<400> 161

```
caggcagaag tatgcaaagc atgcatctca attagtcagc aaccaggtgt ggaaagtccc       60
caggctcccc agcaggcaga agtatgcaaa gcatgcatct caattagtca gcaaccatag      120
tcccgcccct aactccgccc atcccgcccc taactccgcc cagttccgcc cattctccgc      180
cccatggctg actaattttt tttatttatg cagaggccga ggccgcctct gcctctgagc      240
tattccagaa gtagtgagga ggcttttttg gaggcctagg cttttgcaaa aagctcccgg      300
gagcttgtat atccattttc ggatctgatc agcacgtgat gaaaaagcct gaactcaccg      360
cgacgtctgt cgagaagttt ctgatcgaaa agttcgacag cgtgtccgac ctgatgcagc      420
tctcggaggg cgaagaatct cgtgctttca gcttcgatgt aggagggcgt ggatatgtcc      480
tgcgggtaaa tagctgcgcc gatggtttct acaaagatcg ttatgtttat cggcactttg      540
catcggccgc gctcccgatt ccggaagtgc ttgacattgg ggaattcagc gagagcctga      600
cctattgcat ctcccgccgt gcacagggtg tcacgttgca agacttgcct gaaaccgaac      660
tgcccgctgt tctgcagccg gtcgcggagg ccatggatgc gatcgctgcg gccgatctta      720
gccagacgag cgggttcggc ccattcggac cgcaaggaat cggtcaatac actacatggc      780
gtgatttcat atgcgcgatt gctgatcccc atgtgtatca ctggcaaact gtgatggacg      840
acaccgtcag tgcgtccgtc gcgcaggctc tcgatgagct gatgctttgg gccgaggact      900
gccccgaagt ccggcacctc gtgcacgcgg atttcggctc caacaatgtc ctgacggaca      960
atggccgcat aacagcggtc attgactgga gcgaggcgat gttcggggat tcccaatacg     1020
aggtcgccaa catcttcttc tggaggccgt ggttggcttg tatggagcag cagacgcgct     1080
acttcgagcg gaggcatccg gagcttgcag gatcgccgcg gctccgggcg tatatgctcc     1140
gcattggtct tgaccaactc tatcagagct tggttgacgg caatttcgat gatgcagctt     1200
gggcgcaggg tcgatgcgac gcaatcgtcc gatccggagc cgggactgtc gggcgtacac     1260
aaatcgcccg cagaagcgcg gccgtctgga ccgatggctg tgtagaagta ctcgccgata     1320
gtggaaaccg acgccccagc actcgtccga gggcaaagga atagcacgtg ctacgagatt     1380
tcgattccac cgccgccttc tatgaaaggt tgggcttcgg aatcgttttc cgggacgccg     1440
gctggatgat cctccagcgc ggggatctca tgctggagtt cttcgcccac cccaacttgt     1500
ttattgcagc ttataatggt tacaaataaa gcaatagcat cacaaatttc acaaataaag     1560
catttttttc actgcattct agttgtggtt tgtccaaact catcaatgta tc             1612
```

<210> 162
<211> 1592
<212> **DNA**
<213> Artificial sequence

<220>
<223> Matrice KanMX4 K7

<400> 162

```
gtacgctgca ggtcgacaac ccttaatata acttcgtata atgtatgcta tacgaagtta      60
ttaggtctag agatctgttt agcttgcctc gtccccgccg ggtcacccgg ccagcgacat     120
ggaggcccag aataccctcc ttgacagtct tgacgtgcgc agctcagggg catgatgtga     180
ctgtcgcccg tacatttagc ccatacatcc ccatgtataa tcatttgcat ccatacattt     240
tgatggccgc acggcgcgaa gcaaaaatta cggctcctcg ctgcagacct gcgagcaggg     300
aaacgctccc ctcacagacg cgttgaattg tccccacgcc gcgcccctgt agagaaatat     360
aaaaggttag gatttgccac tgaggttctt ctttcatata cttcctttta aaatcttgct     420
aggatacagt tctcacatca catccgaaca taaacaacca tgggtaagga aaagactcac     480
gtttcgaggc cgcgattaaa ttccaacatg gatgctgatt tatatgggta taaatgggct     540
cgcgataatg tcgggcaatc aggtgcgaca atctatcgat tgtatgggaa gcccgatgcg     600
ccagagttgt ttctgaaaca tggcaaaggt agcgttgcca atgatgttac agatgagatg     660
gtcagactaa actggctgac ggaatttatg cctcttccga ccatcaagca ttttatccgt     720
actcctgatg atgcatggtt actcaccact gcgatccccg gcaaaacagc attccaggta     780
ttagaagaat atcctgattc aggtgaaaat attgttgatg cgctggcagt gttcctgcgc     840
cggttgcatt cgattcctgt ttgtaattgt ccttttaaca gcgatcgcgt atttcgtctc     900
gctcaggcgc aatcacgaat gaataacggt ttggttgatg cgagtgattt tgatgacgag     960
cgtaatggct ggcctgttga acaagtctgg aaagaaatgc ataagctttt gccattctca    1020
ccggattcag tcgtcactca tggtgatttc tcacttgata accttatttt tgacgagggg    1080
aaattaatag gttgtattga tgttggacga gtcggaatcg cagaccgata ccaggatctt    1140
gccatcctat ggaactgcct cggtgagttt tctccttcat tacagaaacg gctttttcaa    1200
aaatatggta ttgataatcc tgatatgaat aaattgcagt ttcatttgat gctcgatgag    1260
tttttctaat cagtactgac aataaaaaga ttcttgtttt caagaacttg tcatttgtat    1320
agttttttta tattgtagtt gttctatttt aatcaaatgt tagcgtgatt tatatttttt    1380
ttcgcctcga catcatctgc ccagatgcga agttaagtgc gcagaaagta atatcatgcg    1440
tcaatcgtat gtgaatgctg gtcgctatac tgctgtcgat tcgatactaa cgccgccatc    1500
cagtgtcgaa aacgagctct cgagaaccct taatataact tcgtataatg tatgctatac    1560
gaagttatta ggtgatatca gatccactag tg                                  1592
```

<210> 163
<211> 485
<212> DNA
<213> Artificial sequence

<220>
<223> Matrice LacZ

<400> 163

gcccaatacg caaaccgcct ctccccgcgc gttggccgat tcattaatgc agctggcacg 60
acaggtttcc cgactggaaa gcgggcagtg agcgcaacgc aattaatgtg agttagctca 120
ctcattaggc accccaggct ttacacttta tgcttccggc tcgtatgttg tgtggaattg 180
tgagcggata acaatttcac acaggaaaca gctatgacca tgattacgga cagcctggcc 240
gtcgttttac aacgtcgtga ctgggaaaac cctggcgtta cccaacttaa tcgccttgca 300
gcacatcccc ctttcgccag ctggcgtaat agcgaagagg cccgcaccga tcgcccttcc 360
caacagttgc gcagcctgaa tggcgaatgg cgcctgatgc ggtattttct ccttacgcat 420
ctgtgcggta tttcacaccg catatggtgc actctcagta caatctgctc tgatgccgca 480
tagac 485

<210> 164
<211> 983
<212> DNA
<213> Artificial sequence

<220>
<223> Matrice mB3Galt6 cDNA

<400> 164

atgaaggtat tccggcgcgc ttggcggcac cgggtggcgc tgggcctagg cggcctggcg 60
ttctgcggca ccactctgtt gtacctggcg cgctgcgctt ccgagggcga gacgccctcc 120
gcttccggag ccgctcggcc ccgcgctaag gccttcctgg cggtgctggt ggccagtgcg 180
ccccgcgcgg tcgagcgccg caccgcagtg cgcagcacgt ggctggcacc ggagaggcgt 240
ggcggacccg aggacgtgtg ggcgcgcttc gccgtgggca ctggcggctt aggctcggag 300
gagcggcgcg ctcttgagct cgagcaggcg cagcacgggg acctgctgct gctgcccgcc 360
ctgcgcgacg cctacgagaa cctcacggcc aaggtcctgg ccatgctgac ctggctggat 420
gagcgcgtgg acttcgagtt cgtgctcaag gcggacgacg actcctttgc gcgcctggac 480
gctatcctgg tggacctacg cgcacgggag cccgcacgcc gccggcgcct ctactggggc 540
ttcttttccg ggcgcgggcg cgtcaagccg ggaggtcgct ggcgagaagc agcctggcaa 600
ctctgcgact actacctgcc ctacgcgttg ggcggtggct atgtcctttc tgcggacctg 660
gtgcattacc tgcgcctcag ccgcgagtac ctgcgcgcgt ggcacagtga agacgtatcg 720
ctgggcacct ggctggcacc agtggatgtg caacgggagc acgacccacg cttcgacacg 780
gagtacaaat ctcgaggctg caacaatcag tatctggtga cacacaagca aagcccagag 840
gacatgttgg agaagcaaca gatgttgctg catgagggcc ggttgtgcaa gcatgaggtg 900
cagttgcgcc tttcctatgt ctatgactgg tcagctccac cctcccagtg ctgccagcgc 960
aaggagggcg ttccctgatg tca 983

<210> 165
<211> 983
<212> DNA
<213> Artificial sequence

<220>
<223> Matrice eZ-mB3Galt6 cDNA shRNA insensible

<400> 165

atgaaggtat tccggcgcgc ttggcggcac cgggtggcgc tgggcctagg cggcctggcg 60

ttctgcggca ccactctgtt gtacctggcg cgctgcgctt ccgagggcga gacgccctcc 120

gcttccggag ccgctcggcc ccgcgctaag gccttcctgg cggtgctggt ggccagtgcg 180

ccccgcgcgg tcgagcgccg caccgcagtg cgcagcacgt ggctggcacc ggagaggcgt 240

ggcggacccg aggacgtgtg ggcgcgcttc gccgtgggca ctggcggctt aggctcggag 300

gagcggcgcg ctcttgagct cgagcaggcg cagcacgggg acctgctgct gctgcccgcc 360

ctgcgcgacg cctacgagaa cctcacggcc aaggtcctgg ccatgctgac ctggctggat 420

gagcgcgtgg acttcgagtt cgtgctcaag gcggacgacg actcctttgc gcgcctggac 480

gctatcctgg tggacctacg cgcacgggag cccgcacgcc gccggcgcct ctactggggc 540

ttcttttccg ggcgcgggcg cgtcaagccg ggaggtcgct ggcgagaagc agcctggcaa 600

ctctgcgact actacctgcc ctacgcgttg ggcggtggct atgtcctttc tgcggacctg 660

gtgcattacc tgcgcctcag ccgcgagtac ctgcgcgcgt ggcacagtga agacgtatcg 720

ctgggcacct ggctggcacc agtggatgtg caacgggagc acgacccacg cttcgacacg 780

gagtacaaat ctcgaggctg caacaatcag tatctggtga cacacaagca aagcccagag 840

gacatgttgg agaagcaaca gatgttgctg catgagggcc ggttgtgcaa gcatgaggtg 900

caacttcgcc tttcctatgt ctatgactgg tcagctccac cctcccagtg ctgccagcgc 960

aaggagggcg ttccctgatg tca 983

<210> 166
<211> 2060
<212> DNA
<213> Artificial sequence

<220>
<223> Matrice Yeast S.cerevisiae MNN10 gene

<400> 166

aaacatgcat tcaaaggtca taattgctgc tctatttaca gtcgtccata atgacatttc 60

tctttgatta ttttcttgtt ttttcgctct tctcaagtgg atgttacata acaaacaaaa 120

cagaaaaaat tgtttaaata taaagtttaa aagttatctt tgattccgca cctgaatttt 180
tggattgaag gccaaaggag gtttatcagg gagagaaaag ctctctattt atttttataa 240
ggaataattg tgcatgtaca actatacaat atgtctagtg taccttataa ttcccaactt 300
cctatatcca accatctaga gtacgatgaa gatgaaaaga agagcagagg ctcaaaacta 360
ggcctgaaat ataaaatgat atactggagg aaaactttat gcagttcgct agcgagatgg 420
agaaagctaa tactattaat atctttagct ttgtttttat tcatatggat aagcgattcc 480
accataagca gaaatccatc taccacaagt tttcaaggcc aaaatagtaa cgataataag 540
ttgagtaata ctggttctag catcaactcc aaaagatatg taccaccata ttctaagaga 600
tcaagatggt cgttttggaa tcaagatcct aggattgtca ttatattagc ggcaaacgaa 660
ggtggtggtg tattgaggtg gaaaaatgag caagaatggg ctatcgaagg catatcaata 720
gaaaataaga aggcctatgc gaagagacat ggatatgcgt tgactatcaa ggatttgaca 780
acgtccaaaa gatactctca cgaatacaga gagggttggc aaaaagtaga tatattgaga 840
cagacgttca gggagtttcc taatgcagaa tggttctggt ggttggacct ggatactatg 900
ataatggagc cttctaaatc attagaagaa catattttcg acagattgga aactctggct 960
gacagagaat tgaaaagttt taatccccta aacctaagag acgacatacc ctatgtcgat 1020
tattcagagg aaatggagtt tctaataaca caagattgtg gaggcttcaa tttgggctca 1080
tttctgataa aaaatagcga atggtctaag ctgcttctag atatgtggtg ggaccccgtt 1140
ctgtatgaac aaaaacatat ggtttgggaa catagagaac aagatgcgtt agaggcatta 1200
tatgaaaacg aaccgtggat tcgttcgaga ataggatttt tgcccttaag aacgatcaat 1260
gcattcccac cgggagcatg ctctgaatac agtggtgact caagatactt ttacagtgag 1320
aaagaccatg attttgttgt gaatatggcc ggatgcaatt ttggcagaga ttgctggggc 1380
gagatgcagt actacaccac tttaatggaa aaactgaata ggaaatggta cacgagattt 1440
ttcttcccat aaaatggaag ttatcaatat tgtaaagaga agcatttaca agcttttatt 1500
tttcttttta atttccacta ctggttctgc tttaaaatgt tgttttataa tttatgtaca 1560
tttaggccta tagaagattc tttcaataat atgctacaca ttcttttatt tttccatcat 1620
atgttggagt ttatgcctcc tcggcaggag ttgggcggtg cgaagagaag aaaaagagtg 1680
aaactaaaaa aaggaatctg cctttgcata agttcaaaag tgcaatttta gtgttggatt 1740
taaacgggaa aaattgaaat ggccatcgaa acaatacttg taataaacaa atcaggcgga 1800
ctaatctatc agcggaattt taccaacgac gaacagaaat tgaacagcaa tgaatactta 1860
attcttgcta gtacactgca cggtgtattc gccatcgcga gccagctgac tccgaaggca 1920
ttacagctaa ctcaacaaac gaacatcgaa aataccatcc catatatacc ttacgtgggc 1980

atgtccagca ataggagcga tacaagaaat ggaggtggca ataacaacaa acacactaat 2040

aatgaaaaac tgggcagttt 2060

<210> 167
<211> 639
<212> DNA
<213> Artificial sequence

<220>
<223> Matrice promoteur CMV

<400> 167

accaattcag tcgactggat cctagttatt aatagtaatc aattacgggg tcattagttc 60

atagcccata tatggagttc cgcgttacat aacttacggt aaatggcccg cctggctgac 120

cgcccaacga cccccgccca ttgacgtcaa taatgacgta tgttcccata gtaacgccaa 180

tagggacttt ccattgacgt caatgggtgg agtatttacg gtaaactgcc cacttggcag 240

tacatcaagt gtatcatatg ccaagtacgc cccctattga cgtcaatgac ggtaaatggc 300

ccgcctggca ttatgcccag tacatgacct tatgggactt tcctacttgg cagtacatct 360

acgtattagt catcgctatt accatggtga tgcggttttg gcagtacatc aatgggcgtg 420

gatagcggtt tgactcacgg ggatttccaa gtctccaccc cattgacgtc aatgggagtt 480

tgttttggca ccaaaatcaa cgggactttc caaaatgtcg taacaactcc gccccattga 540

cgcaaatggg cggtaggcgt gtacggtggg aggtctatat aagcagagct ggtttagtga 600

accgtcagat cactagtcga ctagggataa cagggccgc 639

<210> 168
<211> 335
<212> DNA
<213> Artificial sequence

<220>
<223> Matrice promoteur EF1alpha, version courte

<400> 168

aggaaccaat tcagtcgact ggatcccgat ggctccggtg cccgtcagtg ggcagagcgc 60

acatcgccca cagtccccga gaagttgggg ggaggggtcg gcaattgaac cggtgcctag 120

agaaggtggc gcggggtaaa ctgggaaagt gatgtcgtgt actggctccg cctttttccc 180

gagggtgggg gagaaccgta tataagtgca gtagtcgccg tgaacgttct ttttcgcaac 240

gggtttgccg ccagaacaca ggtccgcggc cccgaactag gcctaggcgt ctgatcacta 300

gtgactctag tcctagtcga ctagggataa caggg 335

<210> 169
<211> 1183
<212> DNA
<213> Artificial sequence

<220>
<223> Matrice promoteur EF1alpha

<400> 169

```
gtgaggctcc ggtgcccgtc agtgggcaga gcgcacatcg cccacagtcc ccgagaagtt      60
ggggggaggg gtcggcaatt gaaccggtgc ctagagaagg tggcgcgggg taaactggga     120
aagtgatgtc gtgtactggc tccgcctttt tcccgagggt gggggagaac cgtatataag     180
tgcagtagtc gccgtgaacg ttcttttttcg caacgggttt gccgccagaa cacaggtaag     240
tgccgtgtgt ggttcccgcg ggcctggcct ctttacgggt tatggccctt gcgtgccttg     300
aattacttcc acctggctgc agtacgtgat tcttgatccc gagcttcggg ttggaagtgg     360
gtgggagagt tcgaggcctt gcgcttaagg agccccttcg cctcgtgctt gagttgaggc     420
ctggcctggg cgctggggcc gccgcgtgcg aatctggtgg caccttcgcg cctgtctcgc     480
tgctttcgat aagtctctag ccatttaaaa tttttgatga cctgctgcga cgcttttttt     540
ctggcaagat agtcttgtaa atgcgggcca agatctgcac actggtattt cggtttttgg     600
ggccgcgggc ggcgacgggg cccgtgcgtc ccagcgcaca tgttcggcga ggcggggcct     660
gcgagcgcgg ccaccgagaa tcggacgggg gtagtctcaa gctggccggc ctgctctggt     720
gcctggcctc gcgccgccgt gtatcgcccc gccctgggcg gcaaggctgg cccggtcggc     780
accagttgcg tgagcggaaa gatggccgct tcccggccct gctgcaggga gctcaaaatg     840
gaggacgcgg cgctcgggag agcgggcggg tgagtcaccc acacaaagga aaagggcctt     900
tccgtcctca gccgtcgctt catgtgactc cacggagtac cgggcgccgt ccaggcacct     960
cgattagttc tcgagctttt ggagtacgtc gtctttaggt tggggggagg ggttttatgc    1020
gatggagttt ccccacactg agtgggtgga gactgaagtt aggccagctt ggcacttgat    1080
gtaattctcc ttggaatttg cccttttttga gtttggatct tggttcattc tcaagcctca    1140
gacagtggtt caaagttttt ttcttccatt tcaggtgtcg tga                      1183
```

<210> 170
<211> 401
<212> DNA
<213> Artificial sequence

<220>
<223> Matrice promoteur TRE3G

<400> 170

```
ctttcgtctt caagaattcc tggagtttac tccctatcag tgatagagaa cgtatgaaga        60
gtttactccc tatcagtgat agagaacgta tgcagacttt actccctatc agtgatagag       120
aacgtataag gagtttactc cctatcagtg atagagaacg tatgaccagt ttactcccta       180
tcagtgatag agaacgtatc tacagtttac tccctatcag tgatagagaa cgtatatcca       240
gtttactccc tatcagtgat agagaacgta taagctttag gcgtgtacgg tgggcgccta       300
taaaagcaga gctcgtttag tgaaccgtca gatcgcctgg agcaattcca caacactttt       360
gtcttatacc aactttccgt accacttcct accctcgtaa a                           401
```

<210> 171
<211> 4271
<212> DNA
<213> Artificial sequence

<220>
<223> Matrice eZ-Rosa26-3’

<400> 171

```
agatgggcgg gagtcttctg ggcaggctta aaggctaacc tggtgtgtgg gcgttgtcct      60
gcaggggaat tgaacaggtg taaaattgga gggacaagac ttcccacaga ttttcggttt     120
tgtcgggaag ttttttaata ggggcaaata ggaaaatgga ggataggagt catctggggt     180
ttatgcagca aaactacagg tatattgctt gtatccgcct cggagatttc catgaggaga     240
taaagacatg tcacccgagt ttatactctc ctgcttagat cctactacag tatgaaatac     300
agtgtcgcga ggtagactat gtaagcagat ttaatcattt taaagagccc agtacttcat     360
atccatttct cccgctcctt ctgcagcctt atcaaaaggt atttagaaca ctcattttag     420
ccccattttc atttattata ctggcttatc caacccctag acagagcatt ggcattttcc     480
ctttcctgat cttagaagtc tgatgactca tgaaaccaga cagattagtt acatacacca     540
caaatcgagg ctgtagctgg ggcctcaaca ctgcagttct tttataactc cttagtacac     600
tttttgttga tcctttgcct tgatccttaa ttttcagtgt ctatcacctc tcccgtcagg     660
tggtgttcca catttgggcc tattctcagt ccagggagtt ttacaacaat agatgtattg     720
agaatccaac ctaaagctta actttccact cccatgaatg cctctctcct ttttctccat     780
tataactgag ctataaccat taatggtttc aggtggatgt ctcctccccc aatatacctg     840
atgtatctac atattgccag gctgatattt taagacataa aaggtatatt tcattattga     900
gccacatggt attgattact gctactaaaa ttttgtcatt gtacacatct gtaaaaggtg     960
gttccttttg gaatgcaaag ttcaggtgtt tgttgtcttt cctgacctaa ggtcttgtga    1020
gcttgtattt tttctattta agcagtgctt tctcttggac tggcttgact catggcattc    1080
tacacgttat tgctggtcta aatgtgattt tgccaagctt cttcaggacc tataattttg    1140
cttgacttgt agccaaacac aagtaaaatg attaagcaac aaatgtattt gtgaagcttg    1200
gtttttaggt tgttgtgttg tgtgtgcttg tgctctataa taatactatc caggggctgg    1260
agaggtggct cggagttcaa gagcacagac tgctcttcca gaagtcctga gttcaattcc    1320
cagcaaccac atggtggctc acaaccatct gtaatgggat ctgatgccct cttctggtgt    1380
gtctgaagac cacaagtgta ttcacattaa ataaataatc ctccttcttc ttcttttttt    1440
```

tttttaaag agaatactgt ctccagtaga attactgaag taatgaaata ctttgtgttt 1500

gttccaatat ggaagccaat aatcaaatac tcttaagcac tggaaatgta ccaaggaact 1560

attttattta agtgaactgt ggacagagga gccataactg cagacttgtg ggatacagaa 1620

gaccaatgca gacttaatgt cttttctctt acactaagca ataaagaaat aaaaattgaa 1680

cttctagtat cctatttgtt aaactgctag ctttactaac ttttgtgctt catctataca 1740

aagctgaaag ctaagtctgc agccattact aaacatgaaa gcaagtaatg ataattttgg 1800

atttcaaaaa tgtagggcca gagtttagcc agccagtggt ggtgcttgcc tttatgcctt 1860

aatcccagca ctctggaggc agagacaggc agatctctga gtttgagccc agcctggtct 1920

acacatcaag ttctatctag gatagccagg aatacacaca gaaaccctgt tggggagggg 1980

ggctctgaga tttcataaaa ttataattga agcattccct aatgagccac tatggatgtg 2040

gctaaatccg tctacctttc tgatgagatt tgggtattat tttttctgtc tctgctgttg 2100

gttgggtctt ttgacactgt gggctttctt aaagcctcct tccctgccat gtggactctt 2160

gtttgctact aacttcccat ggcttaaatg gcatggcttt ttgccttcta agggcagctg 2220

ctgagatttg cagcctgatt tccagggtgg ggttgggaaa tctttcaaac actaaaattg 2280

tcctttaatt ttttttaaa aaatgggtta tataataaac ctcataaaat agttatgagg 2340

agtgaggtgg actaatatta atgagtccct cccctataaa agagctatta aggctttttg 2400

tcttatacta acttttttt taaatgtggt atctttagaa ccaagggtct tagagtttta 2460

gtatacagaa actgttgcat cgcttaatca gattttctag tttcaaatcc agagaatcca 2520

aattcttcac agccaaagtc aaattaagaa tttctgactt taatgttatt tgctactgtg 2580

aatataaaat gatagctttt cctgaggcag ggtatcacta tgtatctctg cctgatctgc 2640

aacaagatat gtagactaaa gttctgcctg cttttgtctc ctgaatacta aggttaaaat 2700

gtagtaatac ttttggaact tgcaggtcag attcttttat aggggacaca ctaagggagc 2760

ttgggtgata gttggtaaat gtgtttaagt gatgaaaact tgaattatta tcaccgcaac 2820

ctacttttta aaaaaaaaag ccaggcctgt tagagcatgc taagggatcc ctaggacttg 2880

ctgagcacac aagagtagta cttggcaggc tcctggtgag agcatatttc aaaaaacaag 2940

gcagacaacc aagaaactac agtaaggtta cctgtcttta accatctgca tatacacagg 3000

gatattaaaa tattccaaat aatatttcat tcaagttttc cccatcaaa ttgggacatg 3060

gatttctccg gtgaataggc agagttggaa actaaacaaa tgttggtttt gtgatttgtg 3120

aaattgtttt caagtgatag ttaaagccca tgagatacag aacaaagctg ctatttcgag 3180

gtcacttggt tatactcaga agcacttctt tgggtttccc tgcactatcc tgatcatgtg 3240

ctaggcctac cttaggctga ttgttgttca aataacttaa gtttcctgtc aggtgatgtc 3300

atatgatttc atatatcaag gcaaaacatg ttatatatgt taaacatttg gacttaatgt 3360
gaaagttagg tctttgtggg ttttgatttt aatttcaaaa cctgagctaa ataagtcatt 3420
ttacatgtct tacatttggt gaattgtata ttgtggtttg caggcaagac tctctgacct 3480
agtaaccctc ctatagagca ctttgctggg tcacaagtct aggagtcaag catttcacct 3540
tgaagttgag acgttttgtt agtgtatact agttatatgt tggaggacat gtttatccag 3600
aagatattca ggactatttt tgactgggct aaggaattga ttctgattag cactgttagt 3660
gagcattgag tggcctttag gcttgaattg gagtcacttg tatatctcaa ataatgctgg 3720
cctttttaa aaagcccttg ttctttatca ccctgttttc tacataattt ttgttcaaag 3780
aaatacttgt ttggatctcc ttttgacaac aatagcatgt tttcaagcca tattttttt 3840
cctttttttt ttttttttg gtttttcgag acagggtttc tctgtatagc cctggctgtc 3900
ctggaactca ctttgtagac caggctggcc tcgaactcag aaatccgcct gcctctgcct 3960
cctgagtgcc gggattaaag gcgtgcacca ccacgcctgg ctaagttgga tattttgtat 4020
ataactataa ccaatactaa ctccactggg tggattttta attcagtcag tagtcttaag 4080
tggtctttat tggcccttat taaaatctac tgttcactct aacagaggct gttggactag 4140
tgggactaag caacttccta cggatatact agcagataag ggtcagggat agaaactagt 4200
ctagcgtttt gtatacctac cagcttatac taccttgttc tgatagaaat atttaggaca 4260
tctagcttat c 4271

<210> 172
<211> 1084
<212> DNA
<213> Artificial sequence

<220>
<223> Matrice eZ-Rosa26-5’

<400> 172

```
ccccgcggca ggccctccga gcgtggtgga gccgttctgt gagacagccg ggtacgagtc        60
gtgacgctgg aaggggcaag cgggtggtgg gcaggaatgc ggtccgccct gcagcaaccg       120
gagggggagg gagaagggag cggaaaagtc tccaccggac gcggccatgg ctcggggggg       180
gggggcagc ggaggagcgc ttccggccga cgtctcgtcg ctgattggct tcttttcctc        240
ccgccgtgtg tgaaaacaca aatggcgtgt tttggttggc gtaaggcgcc tgtcagttaa       300
cggcagccgg agtgcgcagc cgccggcagc ctcgctctgc ccactgggtg gggcgggagg       360
taggtggggt gaggcgagct ggacgtgcgg gcgcggtcgg cctctggcgg ggcgggggag       420
gggagggagg gtcagcgaaa gtagctcgcg cgcgagcggc cgcccaccct ccccttcctc       480
tgggggagtc gttttacccg ccgccggccg ggcctcgtcg tctgattggc tctcggggcc       540
cagaaaactg gcccttgcca ttggctcgtg ttcgtgcaag ttgagtccat ccgccggcca       600

gcgggggcgg cgaggaggcg ctcccaggtt ccggccctcc cctcggcccc gcgccgcaga       660
gtctggccgc gcgcccctgc gcaacgtggc aggaagcgcg cgctgggggc ggggacgggc       720
agtagggctg agcggctgcg gggcgggtgc aagcacgttt ccgacttgag ttgcctcaag       780
aggggcgtgc tgagccagac ctccatcgcg cactccgggg agtggaggga aggagcgagg       840
gctcagttgg gctgttttgg aggcaggaag cacttgctct cccaaagtcg ctctgagttg       900
ttatcagtaa gggagctgca gtggagtagg cggggagaag gccgcaccct tctccggagg       960
ggggaggga gtgttgcaat acctttctgg gagttctctg ctgcctcctg gcttctgagg      1020
accgccctgg gcctgggaga atcccttgcc ccctcttccc ctcgtgatct gcaactccag      1080
tctt                                                                  1084
```

<210> 173
<211> 406
<212> DNA
<213> Artificial sequence

<220>
<223> mB3Galt6 shRNA TR506016D

<400> 173

acagggtcga caagcttttc caaaaaaaaa gcatgaggtg cagttgcgcc tttcctatct 60
cttgaatagg aaaggcgcaa ctgcacctca tgctggatcc cgcgtccttt ccacaagata 120
tataaaccca agaaatcgaa atactttcaa gttacggtaa gcatatgata gtccatttta 180
aaacataatt ttaaaactgc aaactaccca agaaattatt actttctacg tcacgtattt 240
tgtactaata tctttgtgtt tacagtcaaa ttaattctaa ttatctctct aacagccttg 300
tatcgtatat gcaaatatga aggaatcatg ggaaataggc cctcttcctg cccgaccttg 360
gcgcgcgctc ggcgcgcggt cacgctccgt cacgtggtgc gttttg 406

<210> 174
<211> 342
<212> DNA
<213> Artificial sequence

<220>
<223> Matrice eZ-SiaT-TGS-Hook

<400> 174

atgattcaca ccaacctgaa gaaaaagttc agctgctgcg tcctggtctt tcttctgttt 60
gcagtcatct gtgtgtggaa ggaaaagaag aaagggagtt actatgattc ctttaaattg 120
caaaccaagg aattccaggt gttaaagagt ctggggaaat tggccatggg gtctgattcc 180
cagtctgtat cctcaagcag cacccaggac ccccacaggg gccgccagac cctcggcagt 240
ctcagaggcc tagccaaggc caaaccagag gcctccttcc aggtgtggaa caaggacagc 300
tcttccaaaa accttatccc taggctgcaa aaggggtcgg gg 342

<210> 175
<211> 714
<212> DNA
<213> Artificial sequence

<220>
<223> Matrice TagBFP

<400> 175

atgtcgggga gcgagctgat taaggagaac atgcacatga agctgtacat ggagggcacc 60
gtggacaacc atcacttcaa gtgcacatcc gagggcgaag gcaagcccta cgagggcacc 120
cagaccatga gaatcaaggt ggtcgagggc ggccctctcc ccttcgcctt cgacatcctg 180
gctactagct tcctctacgg cagcaagacc ttcatcaacc acacccaggg catccccgac 240
ttcttcaagc agtccttccc tgagggcttc acatgggaga gagtcaccac atacgaggac 300
gggggcgtgc tgaccgctac ccaggacacc agcctccagg acggctgcct catctacaac 360
gtcaagatca gaggggtgaa cttcacatcc aacggccctg tgatgcagaa gaaaacactc 420
ggctgggagg ccttcaccga aacgctgtac cccgctgacg gcggcctgga aggcagaaac 480
gacatggccc tgaagctcgt gggcgggagc catctgatcg caaacatcaa gaccacatat 540
agatccaaga aacccgctaa gaacctcaag atgcctggcg tctactatgt ggactacaga 600
ctggaaagaa tcaaggaggc caacaacgaa acctacgtcg agcagcacga ggtggcagtg 660
gccagatact gcgacctccc tagcaaactg ggcacaagc ttaattccgg atga 714

<210> 176
<211> 1131
<212> DNA
<213> Artificial sequence

<220>
<223> Matrice Thymidine Kinase cDNA

<400> 176

atggcttcgt accccctgcca tcaacacgcg tctgcgttcg accaggctgc gcgttctcgc 60
ggccatagca accgacgtac ggcgttgcgc cctcgccggc agcaagaagc cacggaagtc 120
cgcctggagc agaaaatgcc cacgctactg cgggtttata tagacggtcc tcacgggatg 180
gggaaaacca ccaccacgca actgctggtg gccctgggtt cgcgcgacga tatcgtctac 240
gtacccgagc cgatgactta ctggcaggtg ctgggggctt ccgagacaat cgcgaacatc 300
tacaccacac aacaccgcct cgaccagggt gagatatcgg ccggggacgc ggcggtggta 360
atgacaagcg cccagataac aatgggcatg ccttatgccg tgaccgacgc cgttctggct 420
cctcatatcg gggggggaggc tgggagctca catgccccgc cccggccct caccctcatc 480
ttcgaccgcc atcccatcgc cgccctcctg tgctacccgg ccgcgcgata ccttatgggc 540

agcatgaccc cccaggccgt gctggcgttc gtggccctca tcccgccgac cttgcccggc 600

acaaacatcg tgttgggggc ccttccggag gacagacaca tcgaccgcct ggccaaacgc 660

cagcgccccg gcgagcggct tgacctggct atgctggccg cgattcgccg cgtttacggg 720

ctgcttgcca atacggtgcg gtatctgcag ggcggcgggt cgtggcggga ggattgggga 780

cagctttcgg ggacggccgt gccgccccag ggtgccgagc cccagagcaa cgcgggccca 840

cgaccccata tcggggacac gttatttacc ctgtttcggg cccccgagtt gctggccccc 900

aacggcgacc tgtacaacgt gtttgcctgg gccttggacg tcttggccaa acgcctccgt 960

cccatgcacg tctttatcct ggattacgac caatcgcccg ccggctgccg ggacgccctg 1020

ctgcaactta cctccgggat ggtccagacc cacgtcacca cccccggctc cataccgacg 1080

atctgcgacc tggcgcgcac gtttgcccgg gagatggggg aggctaactg a 1131

<210> 177
<211> 272
<212> DNA
<213> Artificial sequence

<220>
<223> Matrice TK term

<400> 177

gggggaggct aactgaaaca cggaaggaga caataccgga aggaacccgc gctatgacgg 60

caataaaaag acagaataaa acgcacggtg ttgggtcgtt tgttcataaa cgcggggttc 120

ggtcccaggg ctggcactct gtcgataccc caccgaggcc ccattggggc caatacgccc 180

gcgtttcttc cttttcccca ccccaccccc caagttcggg tgaaggccca gggctcgcag 240

ccaacgtcgg ggcggcaggc cctgccatag cc 272

<210> 178
<211> 747
<212> DNA
<213> Artificial sequence

<220>
<223> Matrice TetON-3G cDNA

<400> 178

```
atgtctagac tggacaagag caaagtcata aactctgctc tggaattact caatggagtc      60
ggtatcgaag gcctgacgac aaggaaactc gctcaaaagc tgggagttga gcagcctacc     120
ctgtactggc acgtgaagaa caagcgggcc ctgctcgatg ccctgccaat cgagatgctg     180
gacaggcatc atacccactc ctgcccccctg gaaggcgagt catggcaaga ctttctgcgg     240
aacaacgcca agtcataccg ctgtgctctc ctctcacatc gcgacggggc taaagtgcat     300
ctcggcaccc gcccaacaga gaaacagtac gaaaccctgg aaaatcagct cgcgttcctg     360

tgtcagcaag gcttctccct ggagaacgca ctgtacgctc tgtccgccgt gggccacttt     420
acactgggct gcgtattgga ggaacaggag catcaagtag caaaagagga aagagagaca     480
cctaccaccg attctatgcc cccacttctg aaacaagcaa ttgagctgtt cgaccggcag     540
ggagccgaac ctgccttcct tttcggcctg gaactaatca tatgtggcct ggagaaacag     600
ctaaagtgcg aaagcggcgg gccgaccgac gcccttgacg attttgactt agacatgctc     660
ccagccgatg cccttgacga ctttgacctt gatatgctgc ctgctgacgc tcttgacgat     720
tttgaccttg acatgctccc cgggtaa                                         747
```